# EUROPEAN PATENT SPECIFICATION

(11) **EP 2 570 484 B1**
(45) Date of publication and mention of the grant of the patent: **10.09.2014**
(21) Application number: 12181077.4
(22) Date of filing: 02.07.2009
(51) Int. Cl.: C12N 15/82, A61K 39/145, A61P 31/16, A61P 37/04, C07K 14/11, C12N 15/44, C12N 7/01, C12N 7/02, C12N 15/29

(54) **Influenza virus-like particles (VLPS) comprising hemagglutinin**
Influenza-Virus-Like-Partikel (VLPS) mit Hämagglutinin
Particules de type virus (VLP) de la grippe comprenant de l'hémagglutinine

(30) Priority: 11.07.2008 WO PCT/CA2008/001281; 12.01.2009 WO PCT/CA2009/000032
(43) Date of publication of application: 20.03.2013
(62) Divisional of application: 09793741.1
(73) Proprietor: Medicago Inc., Québec, Québec G1V 3V9 (CA)
(72) Inventor: D'Aoust, Marc-Andre, Québec, Québec G1X 4N4 (CA); Couture, Manon, St.-Augustin-de-Desmaures, Québec G3A 2A5 (CA); Ors, Frédéric, Québec, Québec G1Y 2J8 (CA); Trepanier, Sonia, St-Nicolas, Québec G1Y 2J8 (CA); Lavoie, Pierre-Olivier, Quebec, Québec G1N 2L7 (CA); Dargis, Michèle, Quebec, Québec G2C 1M4 (CA); Vezina, Louis-Philippe, Neuville, Québec G0A 2R0 (CA); Landry, Nathalie, St-Romauld, Québec G6W 0C1 (CA)
(74) Representative: Murphy, Colm Damien

(56) References cited:
- WO-A2-2004/098533
- REBECCA J GARTEN ET AL: "Antigenic and Genetic Characteristics of Swine-Origin 2009 A(H1N1) Influenza Viruses Circulating in Humans", SCIENCE, AMERICAN ASSOCIATION FOR THE ADVANCEMENT OF SCIENCE, WASHINGTON, DC; US, vol. 325, no. 5937, 1 July 2009 (2009-07-01), pages 197-201, XP002637951, ISSN: 0036-8075, DOI: 10.1126/SCIENCE.1176225
- D'AOUST MARC-ANDRÉ ET AL: "Influenza virus-like particles produced by transient expression in Nicotiana benthamiana induce a protective immune response against a lethal viral challenge in mice.", PLANT BIOTECHNOLOGY JOURNAL DEC 2008 LNKD- PUBMED:19076615, vol. 6, no. 9, December 2008 (2008-12), pages 930-940, XP002598510, ISSN: 1467-7644
- MUSIYCHUK KONSTANTIN ET AL: "A launch vector for the production of vaccine antigens in plants", INFLUENZA AND OTHER RESPIRATORY VIRUSES, BLACKWELL PUBLISHING LTD, UK LNKD- DOI:10.1111/J.1750-2659.2006.00005.X, vol. 1, no. 1, 1 January 2007 (2007-01-01) , pages 19-25, XP009058889, ISSN: 1750-2640 [retrieved on 2007-01-19]
- PUSHKO ET AL: "Influenza virus-like particles comprised of the HA, NA, and M1 proteins of H9N2 influenza virus induce protective immune responses in BALB/c mice", VACCINE, ELSEVIER LTD, GB LNKD- DOI:10.1016/J.VACCINE.2005.07.098, vol. 23, no. 50, 30 December 2005 (2005-12-30), pages 5751-5759, XP005180698, ISSN: 0264-410X
- GRGACIC E V L ET AL: "Virus-like particles: Passport to immune recognition", METHODS : A COMPANION TO METHODS IN ENZYMOLOGY, ACADEMIC PRESS INC., NEW YORK, NY, US LNKD- DOI:10.1016/J.YMETH.2006.07.018, vol. 40, no. 1, 1 September 2006 (2006-09-01), pages 60-65, XP024908472, ISSN: 1046-2023 [retrieved on 2006-09-01]
- SAINT-JORE-DUPAS ET AL: "From planta to pharma with glycosylation in the toolbox", TRENDS IN BIOTECHNOLOGY, ELSEVIER PUBLICATIONS, CAMBRIDGE, GB, vol. 25, no. 7, 1 July 2007 (2007-07-01), pages 317-323, XP022116342, ISSN: 0167-7799
- D'AOUST MARC-ANDRE ET AL: "The production of hemagglutinin-based virus-like particles in plants: a rapid, efficient and safe response to pandemic influenza", PLANT BIOTECHNOLOGY JOURNAL, vol. 8, no. 5, June 2010 (2010-06), pages 607-619, XP002598511, ISSN: 1467-7644

## Description

This application is a Continuation-in-Part of PCT Application No. PCT/CA2009/000032, filed January 12, 2009, which is a Continuation-in-Part of, and claims priority from PCT Application No. PCT/CA2008/001281, filed July 11, 2008; which claims priority from Canadian Application No. 2,615,372 filed January 21, 2008; United States Application No. 61/022,775 filed January 22, 2008; U.S. Application No. 60/959,414 filed July 13, 2007; U.S. Application No. 60/990,603 filed November 27, 2007; and U.S. Application No. 61/013,272 filed December 12, 2007.

### FIELD OF INVENTION

The present invention relates to the production of virus-like particles. More specifically, the present invention is directed to the production of virus-like particles comprising influenza antigens.

### BACKGROUND OF THE INVENTION

Influenza is the leading cause of death in humans due to a respiratory virus. Common symptoms include fever, sore throat, shortness of breath, and muscle soreness, among others. During flu season, influenza viruses infect 10-20% of the population worldwide, leading to 250-500,000 deaths annually

Influenza viruses are enveloped viruses that bud from the plasma membrane of infected mammalian and avian cells. They are classified into types A, B, or C, based on the nucleoproteins and matrix protein antigens present. Influenza type A viruses may be further divided into subtypes according to the combination of hemagglutinin (HA) and neuraminidase (NA) surface glycoproteins presented. HA governs the ability of the virus to bind to and penetrate the host cell. NA removes terminal sialic acid residues from glycan chains on host cell and viral surface proteins, which prevents viral aggregation and facilitates virus mobility. Currently, 16 HA (H1-H16) and 9 NA (N1-N9) subtypes are recognized. Each type A influenza virus presents one type of HA and one type of NA glycoprotein. Generally, each subtype exhibits species specificity; for example, all HA and NA subtypes are known to infect birds, while only subtypes H1, H2, H3, H5, H7, H9, H10, N1, N2, N3 and N7 have been shown to infect humans (Horimoto 2006; Suzuki 2005). Influenza viruses comprising H5, H7 and H9 are considered the most highly pathogenic forms of influenza A viruses, and are most likely to cause future pandemics.

Influenza pandemics are usually caused by highly transmittable and virulent influenza viruses, and can lead to elevated levels of illness and death globally. The emergence of new influenza A subtypes resulted in 4 major pandemics in the 20^{th} century. The Spanish flu, caused by an H1N1 virus, in 1918-1919 led to the deaths of over 50 million people worldwide between 1917 and 1920. Presently, the risk of the emergence of a new subtype, or of the transmission to humans of a subtype endemic in animals, is always present. Of particular concern is a highly virulent form of avian influenza (also called "bird flu"), outbreaks of which have been reported in several countries around the world. In many cases, this bird flu can result in mortality rates approaching 100% within 48 hours. The spread of the avian influenza virus (H5N1), first identified in Hong Kong in 1997, to other Asian countries and Europe has been postulated to be linked to the migratory patterns of wild birds.

The current method of combating influenza in humans is by annual vaccination. The vaccine is usually a combination of several strains that are predicted to be the dominant strains for the coming "flu-season". The prediction is coordinated by the World Health Organization. Generally, the number of vaccine doses produced each year is not sufficient to vaccinate the world's population. For example, Canada and the United-States obtain enough vaccines doses to immunize about one third of their population, while only 17% of the population of the European Union can be vaccinated. It is evident that current worldwide production of influenza vaccine would be insufficient in the face of a worldwide flu pandemic. Even if the necessary annual production could somehow be met in a given year, the dominant strains change from year to year, thus stockpiling at low-need times in the year is not practical. Economical, large scale production of an effective influenza vaccine is of significant interest to government and private industry alike.

The viral stocks for use in vaccines are produced in fertilized eggs. The virus particles are harvested, and for an inactivated viral vaccine, disrupted by detergent to inactivate. Live attenuated vaccines are made of influenza viruses that were adapted for growth at low temperature which means that at normal body temperature, the vaccine is attenuated. Such a vaccine is licensed in USA for use in individuals from 5 to 49 years of age. Inactivated whole virus vaccines are rendered harmless by inactivation with chemical agents and they have been produced in embryonic eggs or mammalian cell culture. All these types of vaccine show some specific advantages and disadvantages. One advantage of vaccines derived from whole viruses is the type of immunity induced by such vaccines. In general, split vaccines induce a strong antibody response while vaccines made of whole viruses induce both an antibody (humoral) and cellular response. Even though a functional antibody response is a criterion for licensure that correlates with protection induced by a vaccine, there is increasing evidence that a T-cell response is also important in influenza immunity - this may also provide better protection in the elderly.

In order to induce a cellular immune response, vaccines made of whole viruses were developed. Due to the high pathogenicity of the influenza strain (e.g. H5N1), these vaccines are produced in BL3+ facility. For highly pathogenic influenza strains such as H5N1, some manufacturers have modified the hemagglutinin gene sequence in order to reduce the pathogenicity of the influenza strain and to make it avirulent and more easily produced in embryonic eggs or mammalian cell culture. Others also use reassortant influenza strains in which the genetic sequences for the hemagglutinin and neuraminidase proteins are cloned in a high-yielding low pathogenic influenza donor strain (A/PR/8/34; Quan F-S et al, 2007). While these methods may produce useful vaccines, they do not provide a solution to the need for high-volume, low cost and fast production of vaccines in the scale necessary to meet the global need in a normal year, and would almost certainly be insufficient in the face of a pandemic.

Using this reverse genetic technology, one might also need to mutate the genetic sequence of the HA protein to make it avirulent. For highly pathogenic influenza strains, the production of whole virus vaccines either requires confinement procedures or the resulting vaccines do not exactly match the genetic sequence of the circulating virus. In the case of live-attenuated vaccines, there is still a risk that the administered vaccine can recombine with an influenza virus from the host, leading to a new influenza virus.

While this method maintains the antigenic epitope and post-translational modifications, there are a number of drawbacks to this method, including the risk of contamination due to the use of whole virus and variable yields depending on virus strain. Sub-optimal levels of protection may result from genetic heterogeneity in the virus due to its introduction into eggs. Other disadvantages includes extensive planning for obtaining eggs, contamination risks due to chemicals used in purification, and long production times. Also, persons hypersensitive to egg proteins may not be eligible candidates for receiving the vaccine.

In the case of a pandemic, split vaccine production is limited by the need to adapt the strain for growth in eggs and the variable production yields achieved. Although this technology has been used for years for the production of seasonal vaccines, it can hardly respond in a reasonable timeframe to a pandemic and worldwide manufacturing capacity is limited.

To avoid the use of eggs, influenza viruses have also been produced in mammalian cell culture, for example in MDCK or PERC.6 cells, or the like. Another approach is reverse genetics, in which viruses are produced by cell transformation with viral genes. These methods, however, also requires the use of whole virus as well as elaborate methods and specific culture environments.

Several recombinant products have been developed as recombinant influenza vaccine candidates. These approaches have focused on the expression, production, and purification of influenza type A HA and NA proteins, including expression of these proteins using baculovirus infected insect cells (Crawford et al, 1999; Johansson, 1999), viral vectors, and DNA vaccine constructs (Olsen et al., 1997).

Of recent concern is the outbreak of "swine flu" (strain A/California/04/09). An initial outbreak in Mexico brought this viral strain to the world attention in a few days, and has been detected in countries around the world, as a testament to the rapidity by which influenza may be transmitted, as well as a test for quarantine, antiviral production, infection control and ultimately, vaccine production.

Specifics of an influenza virus infection are well known. Briefly , the infectious cycle is initiated by the attachment of the virion surface HA protein to a sialic acid-containing cellular receptor (glycoproteins and glycolipids). The NA protein mediates processing of the sialic acid receptor, and virus penetration into the cell depends on HA-dependent receptor-mediated endocytosis. In the acidic confines of internalized endosomes containing an influenza virion, the HA protein undergoes conformational changes that lead to fusion of viral and cell membranes and virus uncoating and M2-mediated release of MI proteins from nucleocapsid-associated ribonucleoproteins (RNPs), which migrate into the cell nucleus for viral RNA synthesis. Antibodies to HA proteins prevent virus infection by neutralizing virus infectivity, whereas antibodies to NA proteins mediate their effect on the early steps of viral replication.

Crawford et al. (1999) disclose expression of influenza HA in baculovirus infected insect cells. The expressed proteins are described as being capable of preventing lethal influenza disease caused by avian H5 and H7 influenza subtypes. Johansson et al. (1999) teach that baculovirus-expressed influenza HA and NA proteins induce immune responses in animals superior to those induced by a conventional vaccine. Immunogenicity and efficacy of baculovirus- expressed hemagglutinin of equine influenza virus was compared to a homologous DNA vaccine candidate (Olsen et al., 1997). Collectively, these data demonstrate that a high degree of protection against influenza virus challenge can be induced with recombinant HA or NA proteins, using various experimental approaches and in different animal models.

Since previous research has shown that the surface influenza glycoproteins, HA and NA, are the primary targets for elicitation of protective immunity against influenza virus and that M1 provides a conserved target for cellular immunity to influenza, a new vaccine candidate may include these viral antigens as a protein macromolecular particle, such as virus-like particles (VLPs). As vaccine products, VLPs offer the advantage of being more immunogenic than subunit or recombinant antigens and are able to stimulate both humoral and cellular immune response (Grgacic and Anderson, 2006). Further, the particle with these influenza antigens may display conformational epitopes that elicit neutralizing antibodies to multiple strains of influenza viruses.

Production of a non-infectious influenza virus strain for vaccine purposes is one way to avoid inadvertent infection. Alternatively, virus-like particles (VLPs) as substitutes for the cultured virus have been investigated. VLPs mimic the structure of the viral capsid, but lack a genome, and thus cannot replicate or provide a means for a secondary infection.

Several studies have demonstrated that recombinant influenza proteins self- assemble into VLPs in cell culture using mammalian expression plasmids or baculovirus vectors (Gomez-Puertas et al., 1999; Neumann et al., 2000; Latham and Galarza, 2001). Gomez-Puertas et al.

(1999) discloses that efficient formation of influenza VLP depends on the expression levels of several viral proteins. Neumann et al. (2000) established a mammalian expression plasmid-based system for generating infectious influenza virus-like particles entirely from cloned cDNAs. Latham and Galarza (2001) reported the formation of influenza VLPs in insect cells infected with recombinant baculovirus co-expressing HA, NA, M1, and M2 genes. These studies demonstrated that influenza virion proteins may self-assemble upon co-expression in eukaryotic cells.

Gomez-Puertas et al.(2000) teach that, in addition to the hemagglutinin (HA), the matrix protein (M1) of the influenza virus is essential for VLP budding from insect cells. However, Chen et al. (2007) teach that M1 might not be required for VLP formation, and observed that efficient release of M1 and VLPs required the presence of HA and sialidase activity provided by NA. The NA cleaves the sialic acids of the glycoproteins at the surface of the cells producing the VLPs, and releasing the VLPs in the medium.

Quan et al 2007 teaches that a VLP vaccine produced in a baculovirus expression system (insect cell) induces a protective immunity against some strains of influenza virus (A/PR8/34 (H1N1)). The VLPs studied by Quan were observed to bud from the plasma membrane, and were considered to be of the correct size and morphology, similar to those obtained in a mammalian system (MDCK cells).

PCT Publications WO 2004/098530 and WO 2004/098533 teach expression of Newcastle Disease Virus HN or Avian Influenza A/turkey/Wisconsin/68 (H5N9) in transformed NT-1 (tobacco) cells in culture. Compositions comprising the plant cell culture-expressed polypeptides elicit varying immune responses in rabbits and chickens.

Enveloped viruses may obtain their lipid envelope when 'budding' out of the infected cell and obtain the membrane from the plasma membrane, or from that of an internal organelle. Influenza virus particles and VLPs bud from the plasma membrane of the host cell. In mammalian or baculovirus cell systems, for example, influenza buds from the plasma membrane (Quan et al 2007). Only a few enveloped viruses are known to infect plants (for example, members of the Topoviruses and Rhabdoviruses). Of the known plant enveloped viruses, they are characterized by budding from internal membranes of the host cell, and not from the plasma membrane. Although a small number of recombinant VLPs have been produced in plant hosts, none were derived from the plasma membrane, raising the question whether plasma membrane-derived VLPs, including influenza VLPs can be produced in plants.

Current influenza VLP production technologies rely on the co-expression of multiple viral proteins, and this dependence represents a drawback of these technologies since in case of a pandemic and of yearly epidemics, response time is crucial for vaccination. A simpler VLP production system, for example, one that relies on the expression of only one or a few viral proteins without requiring expression of non-structural viral proteins is desirable to accelerate the development of vaccines.

In order to protect the world population from influenza and to stave off future pandemics, vaccine manufacturers will need to develop effective, rapid methods producing vaccine doses. The current use of fertilized eggs to produce vaccines is insufficient and involves a lengthy process.

### SUMMARY OF THE INVENTION

It is an object of the invention to provide improved influenza virus like particles (VLPs).

According to the present invention there is provided a nucleic acid comprising a nucleotide sequence encoding an antigen from an enveloped virus operatively linked to a regulatory region active in a plant, the antigen is an influenza hemagglutinin (HA). Preferably, the antigen is an HA from influenza A/California/04/09.

The HA may comprise a native, or a non-native signal peptide; the non-native signal peptide may be a protein disulfide isomerase signal peptide.

The HA encoded by the nucleic acid may be a type A influenza, a type B influenza, or is a subtype of type A influenza, selected from the group comprising H1, H2, H3, H4, H5, H6, H7, H8, H9, H10, H11, H12, H13, H14, H15, and H16. In some aspects of the invention, the HA encoded by the nucleic acid may be from a type A influenza, and selected from the group comprising H1, H2, H3, H5, H6, H7 and H9. Preferably, the influenza HA is from strain A/California/04/09.

The present invention also provides a method of producing influenza virus like particles (VLPs) in a plant comprising:
a) introducing a nucleic acid encoding an antigen from an enveloped virus, for example an influenza hemagglutinin (HA) from strain A/California/04/09, operatively linked to a regulatory region active in the plant, into the plant, or portion thereof, and
b) incubating the plant or a portion therefore under conditions that permit the expression of the nucleic acid, thereby producing the VLPs.

The method may further comprise the steps of harvesting the plant and purifying or separating the VLPs from the plant tissue.

The method may further comprise, in the step of introducing (step a), a nucleic acid comprising a nucleotide sequence encoding one or more than one chaperon protein.

The one or more than one chaperone proteins may be selected from the group comprising Hsp40 and Hsp70.

The present invention includes the above method wherein, in the step of introducing (step a), the nucleic acid may be either transiently expressed in the plant, or stably expressed in the plant. Furthermore, the VLPs may be purified using size exclusion chromatography.

According to another aspect of the present invention, there is provided a method of producing influenza virus like particles (VLPs) in a plant comprising providing a plant, or a portion of a plant, comprising a nucleic acid comprising a nucleotide sequence encoding an HA from influenza A/California/04/09 operatively linked to a regulatory region active in a plant., and incubating the plant or portion of the plant under conditions that permit the expression of the nucleic acid, thereby producing the VLPs.

The method may further comprise the steps of harvesting the plant and purifying or separating the VLPs from the plant tissue.

The present invention includes the above method, wherein following the step of providing, a nucleic acid comprising a nucleotide sequence encoding one or more than one chaperone protein operatively linked to a regulatory region active in a plant is introduced, and the plant or portion of the plant incubated under conditions that permit expression of the nucleic acid, thereby producing the VLPs.

The one or more than one chaperone proteins may be selected from the group comprising Hsp40 and Hsp70.

The present invention includes the above method wherein, in the step of introducing (step a), the nucleic acid encoding the HA from influenza A/California/04/09 is stably expressed in the plant. Furthermore, the VLPs may be purified using size exclusion chromatography.

The present invention also provides a virus like particle (VLP) comprising an influenza virus HA protein, from strain A/California/04/09, and one or more than one lipid derived from a plant.

The HA protein of the VLP may be of a type A influenza, a type B influenza, or is a subtype of type A influenza HA selected from the group consisting of H1, H2, H3, H4, H5, H6, H7, H8, H9, H10, H11, H12, H13, H14, H15, and H16. In some aspects of the invention, the HA is from a type A influenza, selected from the group comprising H1, H2, H3, H5, H6, H7 and H9.

Also included in the present invention is a composition comprising an effective dose of a VLP, the VLP comprising an influenza virus HA protein, one or more than one plant lipid, and a pharmaceutically acceptable carrier.

The present invention also contemplates fragments or portions of HA proteins that form VLPs in a plant.

The present invention also pertains to a VLP comprising an influenza virus HA bearing plant-specific N-glycans, or modified N-glycans. The HA protein of the VLP may be of a type A influenza, a type B influenza, or is a subtype of type A influenza HA selected from the group consisting of H1, H2, H3, H4, H5, H6, H7, H8, H9, H10, H11, H12, H13, H14, H15, and H16. In some aspects of the invention, the HA is from a type A influenza, selected from the group comprising H1, H2, H3, H5, H6, H7 and H9.

The VLP may comprise an HA protein of one, or more than one subtype, including H1, H2, H3, H4, H5, H6, H7, H8, H9, H10, H11, H12, H13, H14, H15 or H16 or fragment or portion thereof. Examples of subtypes comprising such HA proteins include A/New Caledonia/20/99 (H1N1)A/Indonesia/5/2006 (H5N1), A/chicken/New York/1995, A/herring gull/DE/677/88 (H2N8), A/Texas/32/2003, A/mallard/MN/33/00, A/duck/Shanghai/1/2000, A/northern pintail/TX/828189/02, A/Turkey/Ontario/6118/68(H8N4), A/shoveler/Iran/G54/03, A/chicken/Germany/N/1949(H10N7), A/duck/England/56(H11N6), A/duck/Alberta/60/76(H12N5), A/Gull/Maryland/704/77(H13N6), A/Mallard/Gurjev/263/82, A/duck/Australia/341/83 (H15N8), A/black-headed gull/Sweden/5/99(H16N3), B/Lee/40, C/Johannesburg/66, A/PuertoRico/8/34 (H1N1), A/Brisbane/59/2007 (H1N1), A/Solomon Islands 3/2006 (H1N1), A/Brisbane 10/2007 (H3N2), A/Wisconsin/67/2005 (H3N2), B/Malaysia/2506/2004, B/Florida/4/2006, A/Singapore/1/57 (H2N2), A/Anhui/1/2005 (H5N1), A/Vietnam/1194/2004 (H5N1), A/Teal/HongKong/W312/97 (H6N1), A/Equine/Prague/56 (H7N7), A/HongKong/1073/99 (H9N2), A/California/04/09 (H1N1).

In an aspect of the invention, the HA protein may be an H1, H2, H3, H5, H6, H7 or H9 subtype. In an another aspect, the H1 protein may be from the A/New Caledonia/20/99 (H1N1), A/PuertoRico/8/34 (H1N1), A/Brisbane/59/2007 (H1N1), A/Solomon Islands 3/2006 (H1N1) or A/California/04/09 (H1N1) strain. The H3 protein may also be from the A/Brisbane 10/2007 (H3N2) or A/Wisconsin/67/2005 (H3N2) strain. In a further aspect of the invention, the H2 protein may be from the A/Singapore/1/57 (H2N2) strain. The H5 protein may be from the A/Anhui/1/2005 (H5N1), A/Vietnam/1194/2004 (H5N1), or A/Indonesia/5/2005 strain. In an aspect of the invention, the H6 protein may be from the A/Teal/HongKong/W312/97 (H6N1) strain. The H7 protein may be from the A/Equine/Prague/56 (H7N7) strain. In an aspect of the invention, the H9 protein is from the A/HongKong/1073/99 (H9N2) strain. In a further aspect of the invention, the HA protein may be from an influenza virus may be a type B virus, including B/Malaysia/2506/2004 or B/Florida/4/2006. Examples of amino acid sequences of the HA proteins from H1, H2, H3, H5, H6, H7, H9 or B subtypes include SEQ ID NOs: 48-59 and 128.

The influenza virus HA protein may be H5 from strain A/Indonesia/05/05 (H5N1) or H1 from strain A/California/04/09 (H1N1).

The present invention also provides nucleic acid molecules comprising sequences encoding an HA protein. The nucleic acid molecules may further comprise one or more regulatory regions operatively linked to the sequence encoding an HA protein. The nucleic acid molecules may comprise a sequence encoding an H1, H2, H3, H4, H5, H6, H7, H8, H9, H10, H11, H12, H13, H14, H15, H16, B or C. In another aspect of the invention, the HA protein encoded by the nucleic acid molecule may be an H1, H2, H3, H5, H6, H7, H9, or B subtype. The H1 protein encoded by the nucleic acid molecule is from the A/New Caledonia/20/99 (H1N1), A/PuertoRico/8/34 (H1N1), A/Brisbane/59/2007 (H1N1), A/Solomon Islands 3/2006 (H1N1) or A/California/04/09 (H1N1) strain. In an aspect of the invention, the H3 protein encoded by the nucleic acid molecule may be from the A/Brisbane 10/2007 (H3N2), or A/Wisconsin/67/2005 (H3N2) strain. In a further aspect of the invention, the H2 protein encoded by the nucleic acid molecule may be from the A/Singapore/1/57 (H2N2) strain. The H5 protein encoded by the nucleic acid molecule may also be from the A/Anhui/1/2005 (H5N1), A/Vietnam/1194/2004 (H5N1), or A/Indonesia/5/2005 strain. In an aspect of the invention, the H6 protein encoded by the nucleic acid molecule may be from the A/Teal/HongKong/W312/97 (H6N1) strain. The H7 protein encoded by the nucleic acid molecule may also be from the A/Equine/Prague/56 (H7N7) strain. Additionally, the H9 protein encoded by the nucleic acid molecule may be from the A/HongKong/1073/99 (H9N2) strain. The HA protein from B subtype encoded by the nucleic acid may be from the B/Florida/4/2006, or B/Malaysia/2506/2004 strain. Examples of sequences of nucleic acid molecules encoding such HA proteins from H1, H2, H3, H5, H6, H7, H9 or B subtypes include SEQ ID NOs: 36-47 and 60-73 and 127.

The nucleic acid sequence may encode the influenza virus HA protein from strain A/Indonesia/05/05 (H5N1) or from strain A/California/04/09 (H1N1).

Regulatory regions that may be operatively linked to a sequence encoding an HA protein include those that are operative in a plant cell, an insect cell or a yeast cell. Such regulatory regions may include a plastocyanin regulatory region, a regulatory region of Ribulose 1,5-bisphosphate carboxylase/oxygenase (RuBisCO), chlorophyll a/b binding protein (CAB), ST-LS 1, a polyhedrin regulatory region, or a gp64 regulatory region. Other regulatory regions include a 5' UTR, 3' UTR or terminator sequences. The plastocyanin regulatory region may be an alfalfa plastocyanin regulatory region; the 5' UTR, 3'UTR or terminator sequences may also be alfalfa sequences.

A method of inducing immunity to an influenza virus infection in a subject, is also provided, the method comprising administering the virus like particle comprising an influenza virus HA protein, one or more than one plant lipid, and a pharmaceutically acceptable carrier. The virus like particle may be administered to a subject orally, intradermally, intranasally, intramuscularly, intraperitoneally, intravenously, or subcutaneously.

The present invention also pertains to a virus like particle (VLP) comprising one or more than one protein derived from a virus selected from the group consisting of Influenza, Measles, Ebola, Marburg, and HIV, and one or more than one lipid derived from a non-sialylating host production cell. The HIV protein may be p24, gp120 or gp41; the Ebolavirus protein may be VP30 or VP35; the Marburg virus protein may be Gp/SGP; the Measles virus protein may be H-protein or F-protein.

Additionally the present invention relates to a virus like particle (VLP) comprising an influenza virus HA protein and one or more than one host lipid. For example if the host is insect, then the virus like particle (VLP) may comprise an influenza virus HA protein and one or more than one insect lipid, or if the host is a yeast, then the virus like particle (VLP) may comprise an influenza virus HA protein and one or more than one yeast lipid.

The present invention also relates to compositions comprising VLPs of two or more strains or subtypes of influenza. The two or more subtypes or strains may be selected from the group comprising: A/New Caledonia/20/99 (H1N1)A/Indonesia/5/2006 (H5N1), A/chicken/New York/1995, A/herring gull/DE/677/88 (H2N8), A/Texas/32/2003, A/mallard/MN/33/00, A/duck/Shanghai/1/2000, A/northern pintail/TX/828189/02, A/Turkey/Ontario/6118/68(H8N4), A/shoveler/Iran/G54/03, A/chicken/Germany/N/1949(H10N7), A/duck/England/56(H11N6), A/duck/Alberta/60/76(H12N5), A/Gull/Maryland/704/77(H13N6), A/Mallard/Gurjev/263/82, A/duck/Australia/341/83 (H15N8), A/black-headed gull/Sweden/5/99(H16N3), B/Lee/40, C/Johannesburg/66, A/PuertoRico/8/34 (H1N1), A/Brisbane/59/2007 (H1N1), A/Solomon Islands 3/2006 (H1N1), A/Brisbane 10/2007 (H3N2), A/Wisconsin/67/2005 (H3N2), B/Malaysia/2506/2004, B/Florida/4/2006, A/Singapore/1/57 (H2N2), A/Anhui/1/2005 (H5N1), A/Vietnam/1194/2004 (H5N1), A/Teal/HongKong/W312/97 (H6N1), A/Equine/Prague/56 (H7N7), A/HongKong/1073/99 (H9N2), or A/California/04/09 (H1N1). The two or more subtypes or strains of VLPs may be present in about equivalent quantities; alternately one or more of the subtypes or strains may be the majority of the strains or subtypes represented.

The present invention pertains to a method for inducing immunity to influenza virus infection in an animal or target organism comprising administering an effective dose of a vaccine comprising one or more than one VLP, the VLP produced using a non-sialyating host, for example a plant host, an insect host, or a yeast host. The vaccine may be administered orally, intradermally, intranasally, intramusclarly, intraperitoneally, intravenously, or subcutaneously. The target organism may be selected from the group comprising humans, primates, horses, pigs, birds (avian) water fowl, migratory birds, quail, duck, geese, poultry, chicken, camel, canine, dogs, feline, cats, tiger, leopard, civet, mink, stone marten, ferrets, house pets, livestock, mice, rats, seal, whales and the like.

The present invention provides a method for producing VLPs containing hemagglutinin (HA) from different influenza strains in a suitable host capable of producing a VLP, for example, a plant, insect, or yeast. VLPs that are produced in plants contain lipids of plant origin, VLPs produced in insect cells comprise lipids from the plasma membrane of insect cells (generally referred to as "insect lipids"), and VLPs produced in yeast comprise lipids from the plasma membrane of yeast cells (generally referred to as "yeast lipids").

The present invention also pertains to a plant, plant tissue or plant cell comprising a nucleic acid comprising a nucleotide sequence encoding an antigen from an enveloped virus operatively linked to a regulatory region active in a plant. The antigen may be an influenza hemagglutinin (HA). Preferably, the antigen is an HA from influenza A/California/04/09.

The plant may further comprise a nucleic acid comprising a nucleotide sequence encoding one or more than one chaperone proteins operatively linked to a regulatory region active in a plant. The one or more than one chaperon proteins may be selected from the group comprising Hsp40 and Hsp70.

The production of VLPs in plants presents several advantages over the production of these particles in insect cell culture. Plant lipids can stimulate specific immune cells and enhance the immune response induced. Plant membranes are made of lipids, phosphatidylcholine (PC) and phosphatidylethanolamine (PE), and also contain glycosphingolipids that are unique to plants and some bacteria and protozoa. Sphingolipids are unusual in that they are not esters of glycerol like PC or PE but rather consist of a long chain amino alcohol that forms an amide linkage to a fatty acid chain containing more than 18 carbons. PC and PE as well as glycosphingolipids can bind to CD1 molecules expressed by mammalian immune cells such as antigen-presenting cells (APCs) like dentritic cells and macrophages and other cells including B and T lymphocytes in the thymus and liver (Tsuji M,. 2006). Furthermore, in addition to the potential adjuvant effect of the presence of plant lipids, the ability of plant N-glycans to facilitate the capture of glycoprotein antigens by antigen presenting cells (Saint-Jore-Dupas, 2007), may be advantageous of the production of VLPs in plants.

Without wishing to be bound by theory, it is anticipated that plant-made VLPs will induce a stronger immune reaction than VLPs made in other manufacturing systems and that the immune reaction induced by these plant-made VLPs will be stronger when compared to the immune reaction induced by live or attenuated whole virus vaccines.

Contrary to vaccines made of whole viruses, VLPs provide the advantage as they are non-infectious, thus restrictive biological containment is not as significant an issue as it would be working with a whole, infectious virus, and is not required for production. Plant-made VLPs provide a further advantage again by allowing the expression system to be grown in a greenhouse or field, thus being significantly more economical and suitable for scale-up.

Additionally, plants do not comprise the enzymes involved in synthesizing and adding sialic acid residues to proteins. VLPs may be produced in the absence of neuraminidase (NA), and there is no need to co-express NA, or to treat the producing cells or extract with sialidase (neuraminidase), to ensure VLP production in plants.

The VLPs produced in accordance with the present invention do not comprise M1 protein which is known to bind RNA. RNA is a contaminant of the VLP preparation and is undesired when obtaining regulatory approval for the VLP product.

This summary of the invention does not necessarily describe all features of the invention.

### BRIEF DESCRIPTION OF THE DRAWINGS

These and other features of the invention will become more apparent from the following description in which reference is made to the appended drawings wherein:

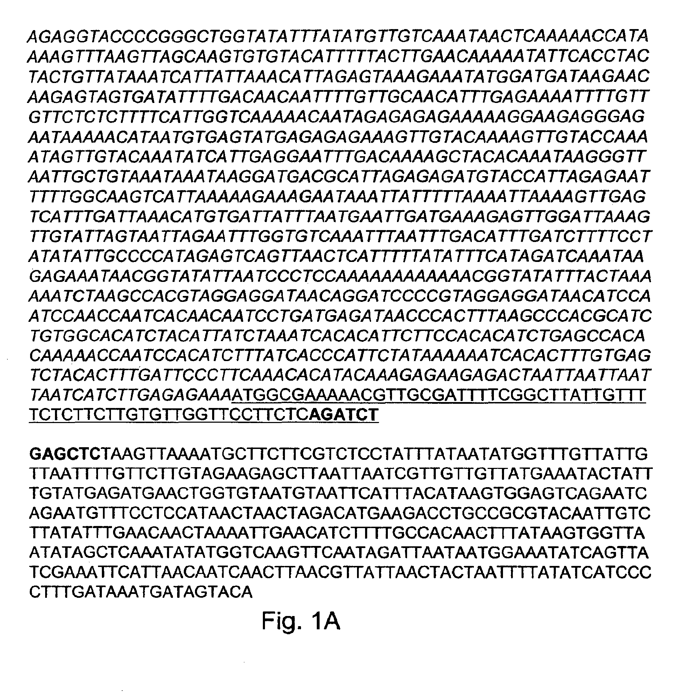

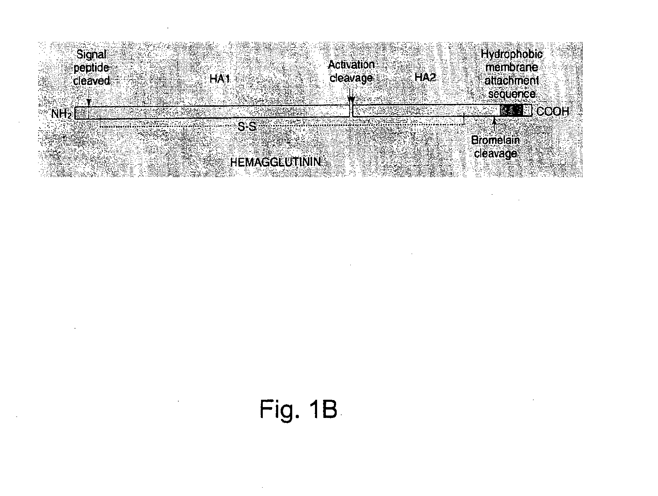

**FIGURE 1A** shows a sequence of an alfalfa plastocyanin-based expression cassette used for the expression of H1 from strain A/New Caledonia/20/99 (H1N1) in accordance with an embodiment of the present invention (SEQ ID NO:8). Protein disulfide isomerase (PDI) signal peptide is underlined. Bg1II (AGATCT) and SacI (GAGCTC) restriction sites used for cloning are shown in bold. Figure 1B shows a schematic diagram of functional domains of influenza hemagglutinin. After cleavage of HA0, HA1 and HA2 fragments remain bound together by a disulfide bridge.

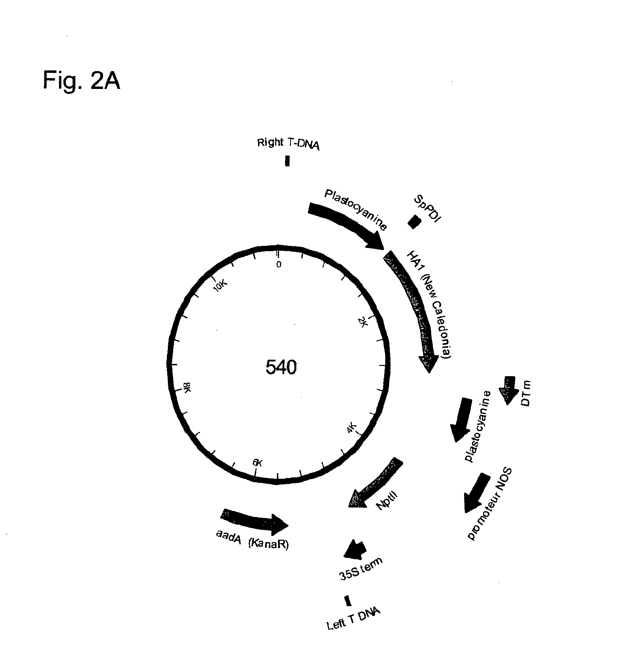

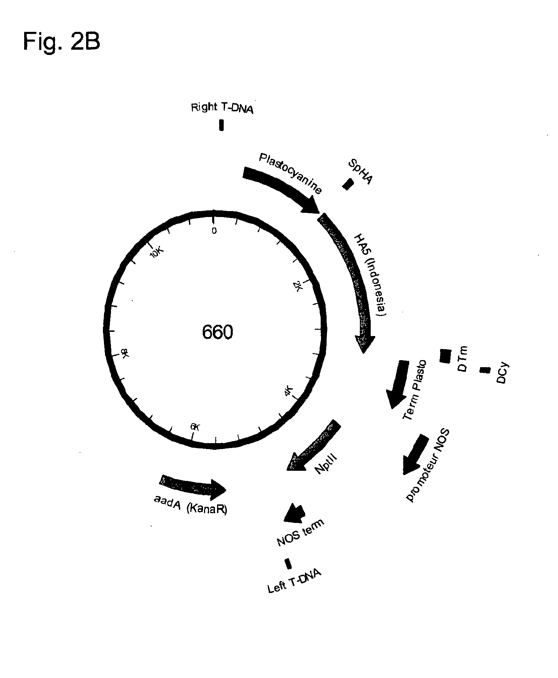

**FIGURE 2A** shows a representation of plasmid 540 assembled for the expression of HA subtype H1 from strain A/New Caledonia/20/99 (H1N1). FIGURE 2B shows a representation of plasmid 660 assembled for the expression of HA subtype H5 from strain A/Indonesia/5/2005 (H5N1).

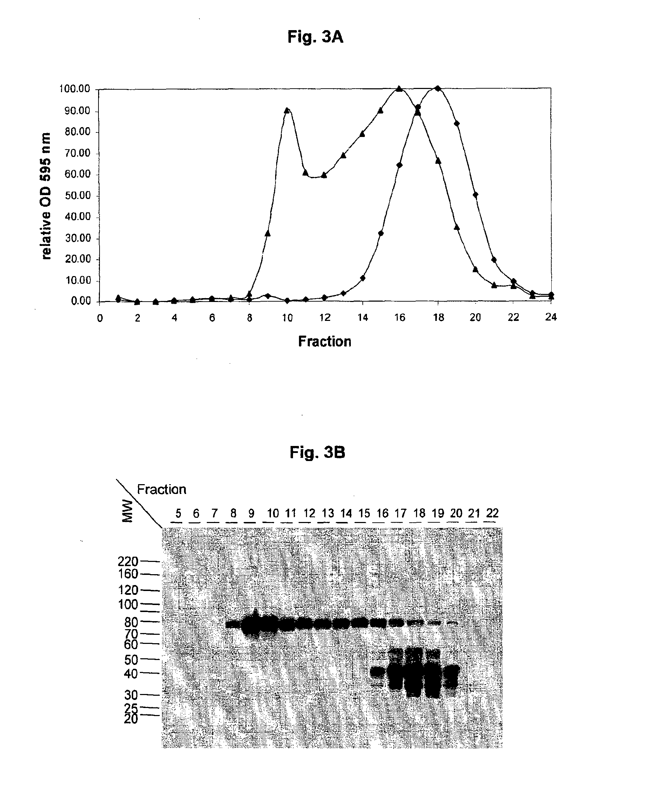

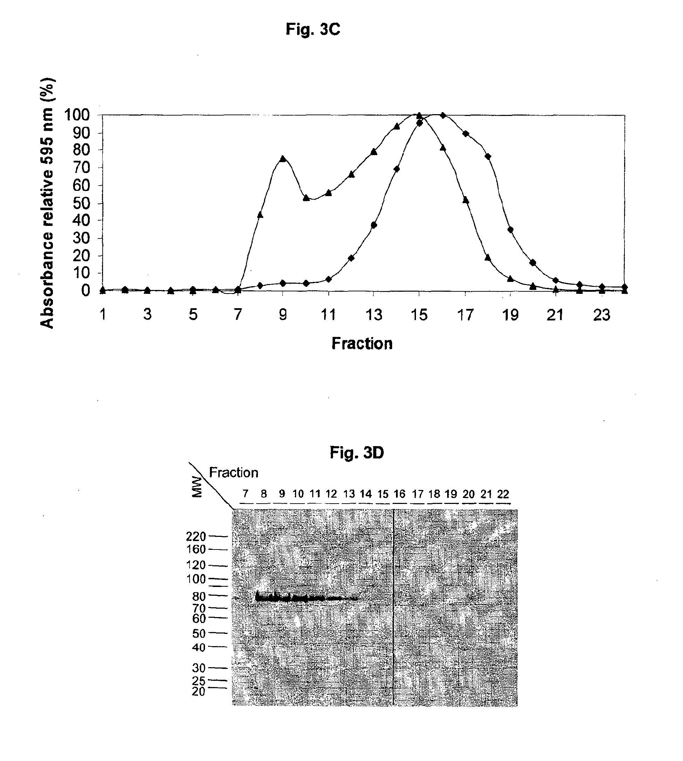

**FIGURE 3** shows a size exclusion chromatography of protein extracts from leaves producing hemagglutinin H1 or H5. FIGURE 3A show the elution profile of Blue Dextran 2000 (triangles) and proteins (diamonds). FIGURE 3B shows immunodetection (western blot; anti H1) of H1 (A/New Caledonia/20/99 (H1N1)) elution fractions following size exclusion chromatography (S500HR beads). FIGURE 3C show the elution profile of H5; Blue Dextran 2000 (triangles) and proteins (diamonds). FIGURE 3D shows immunodetection (western blot; anti H5) of H5 (A/Indonesia/5/2005 (H5N1)) elution fractions following size exclusion chromatography (S500HR beads).

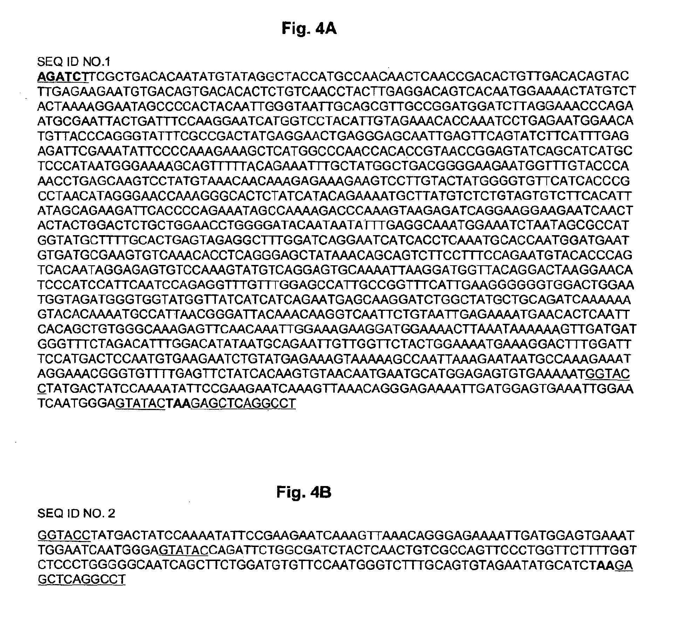

**FIGURE 4A** shows the sequence encoding the N terminal fragment of H1 (A/New Caledonia/20/99 (H1N1)) (SEQ ID NO:1). FIGURE 4B shows the sequence encoding the C terminal fragment of H1 (A/New Caledonia/20/99 (H1N1)) (SEQ ID NO:2).

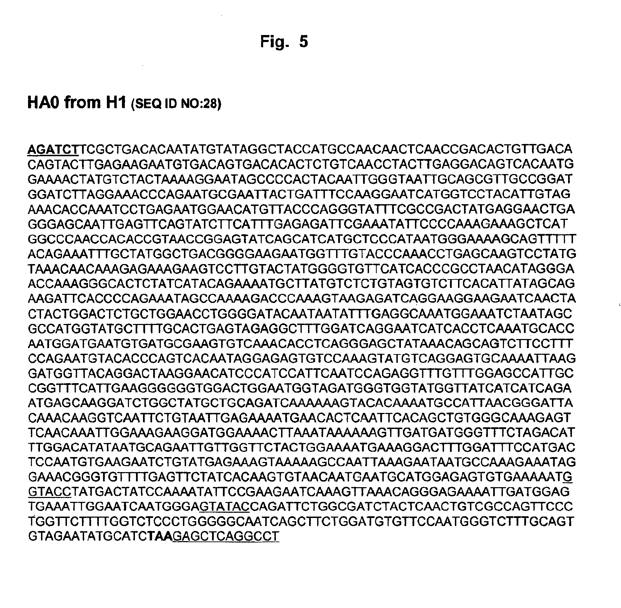

**FIGURE 5** shows the complete sequence encoding HA0 of H1 (A/New Caledonia/20/99 (H1N1)) (SEQ ID NO:28).

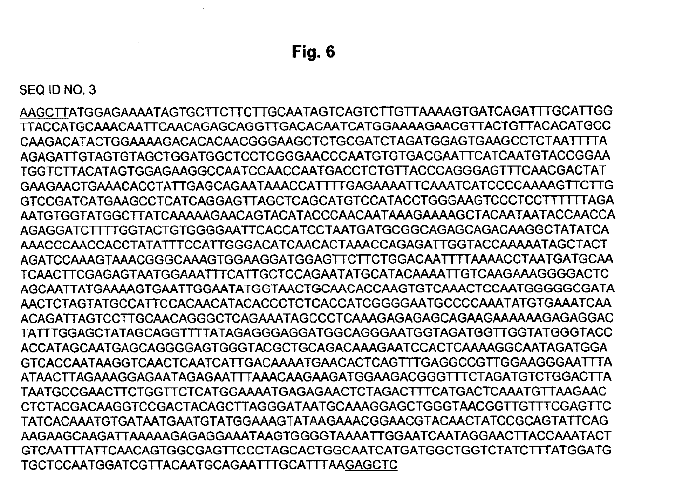

**FIGURE 6** shows the sequence encoding H5 (A/Indonesia/5/2005 (H5N1)) flanked by a HindIII site immediately upstream of the initial ATG, and a SacI site immediately downstream of the stop (TAA) codon (SEQ ID NO:3)

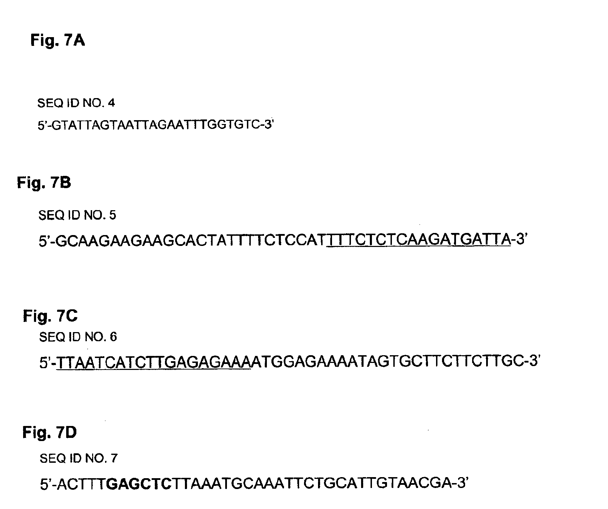

**FIGURE 7A** shows the sequence of the primer Plasto-443c (SEQ ID NO:4). FIGURE 7B shows the sequence of primer SpHA(Ind)-Plasto.r (SEQ ID NO:5). FIGURE 7C shows the sequence of primer SpHA(Ind)-Plasto.r (SEQ ID NO:6). FIGURE 7D shows the sequence of primer HA(Ind)-Sac.r (SEQ ID NO:7).

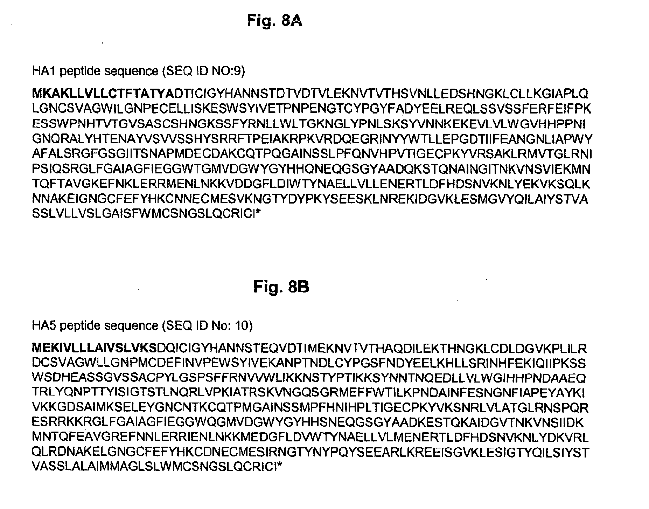

**FIGURE 8A** shows the amino acid sequence of the H1 (A/New Caledonia/20/99 (H1N1); SEQ ID NO:9). FIGURE 8B shows the amino acid sequence of H5 (A/Indonesia/5/2005 (H5N1); SEQ ID NO:10). Native signal peptide is indicated in bold.

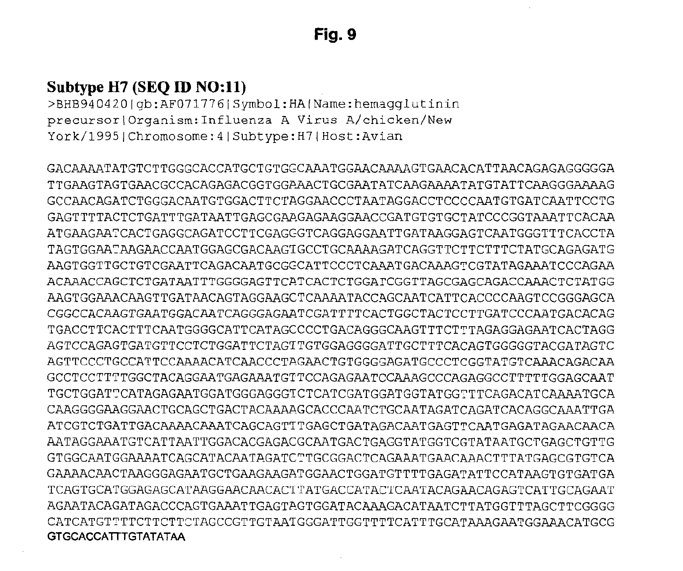

**FIGURE 9** shows the nucleotide sequence of HA of influenza A subtype H7 (SEQ ID No: 11).

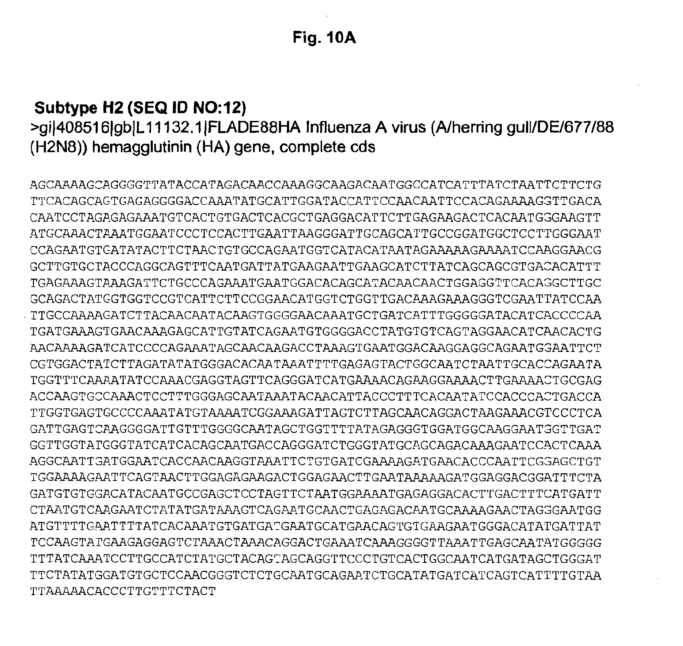

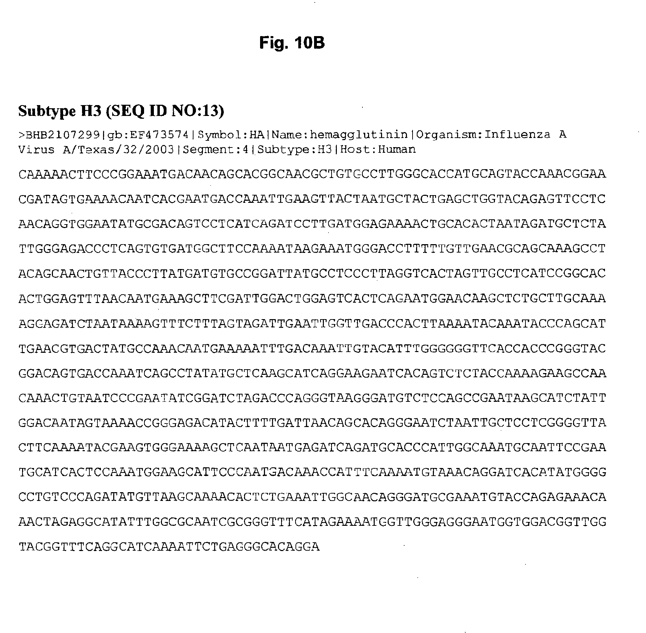

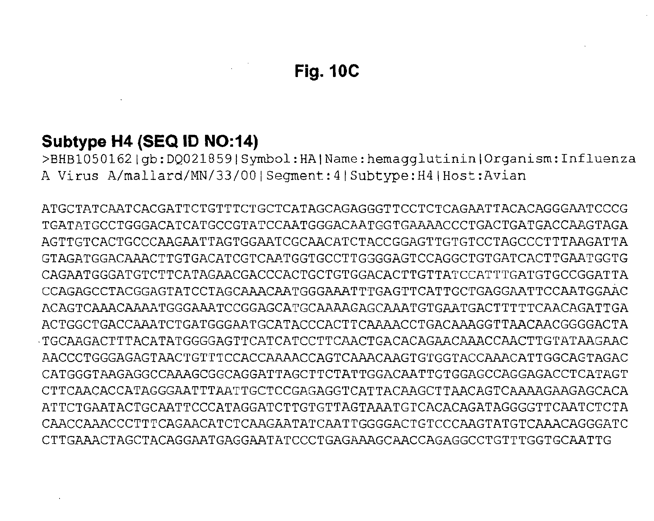

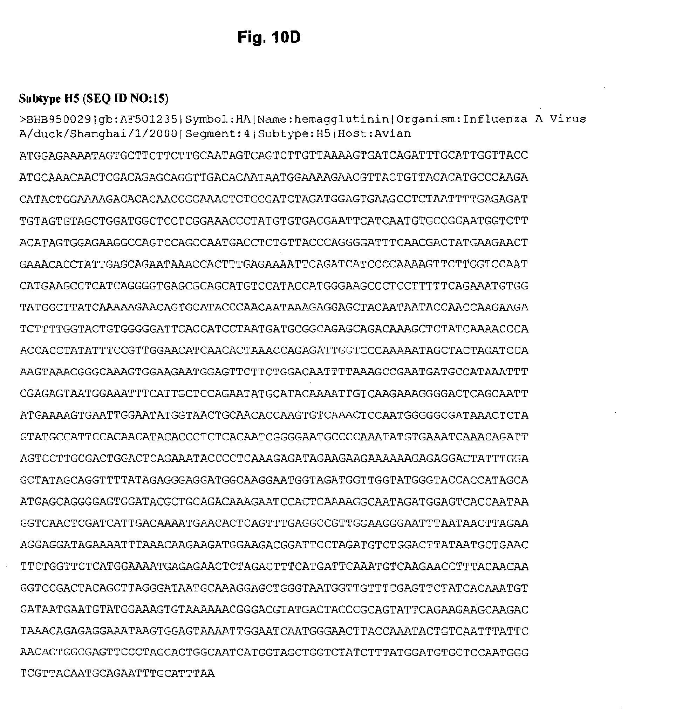

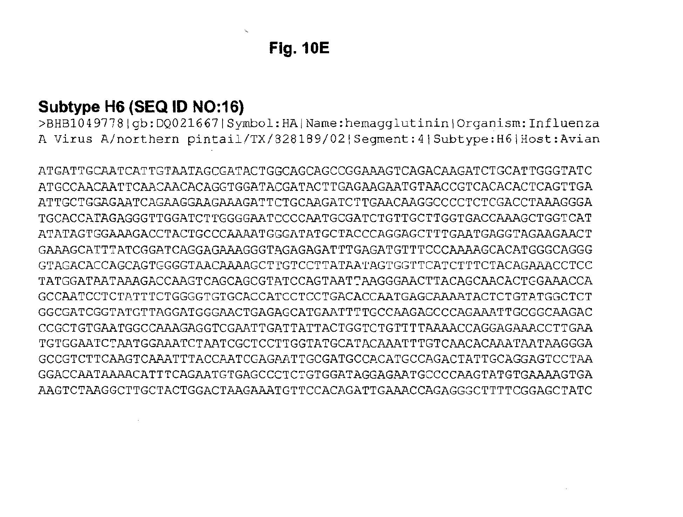

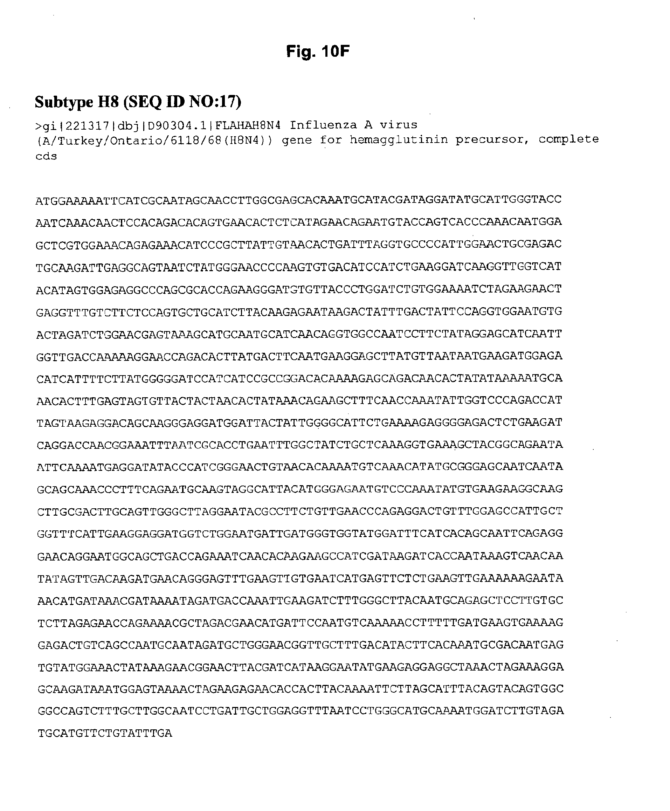

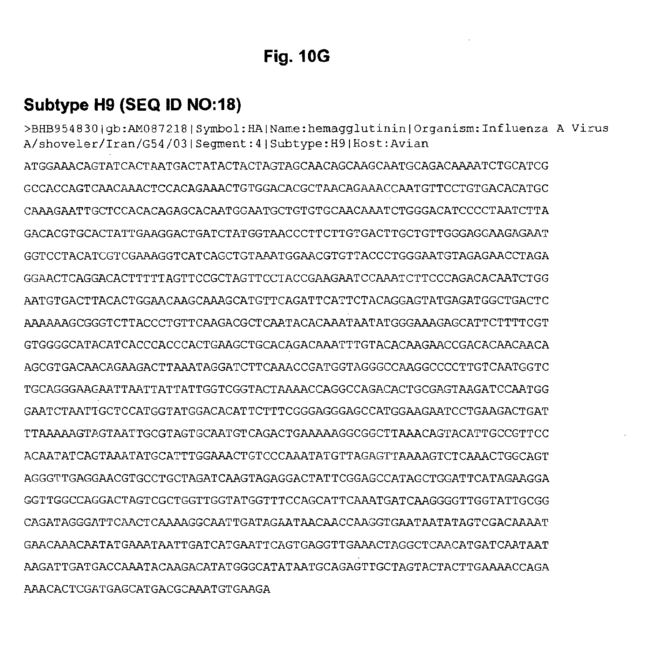

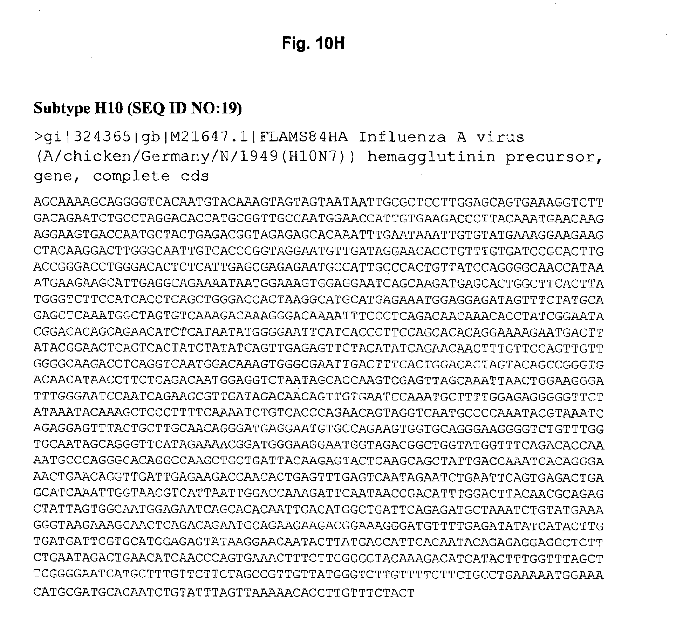

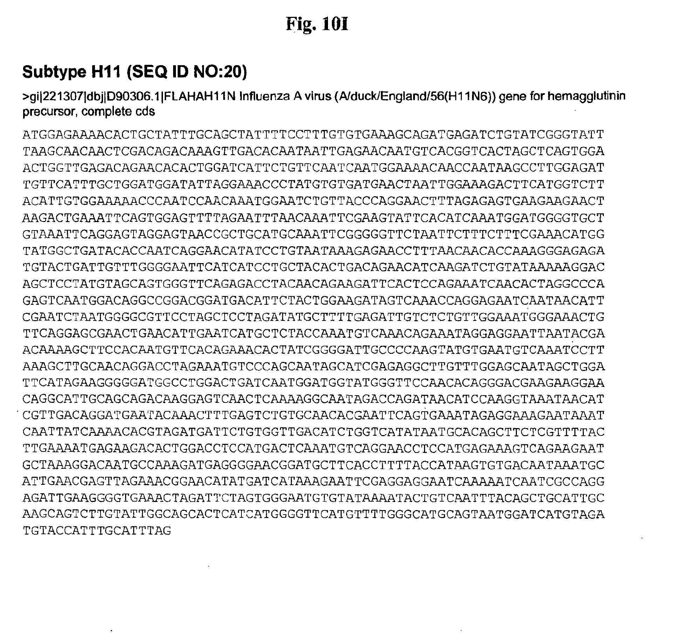

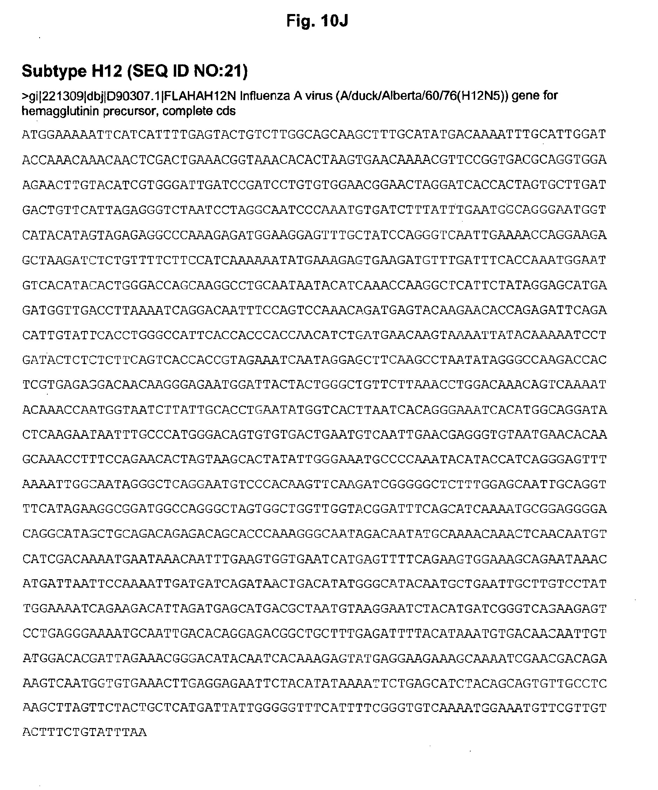

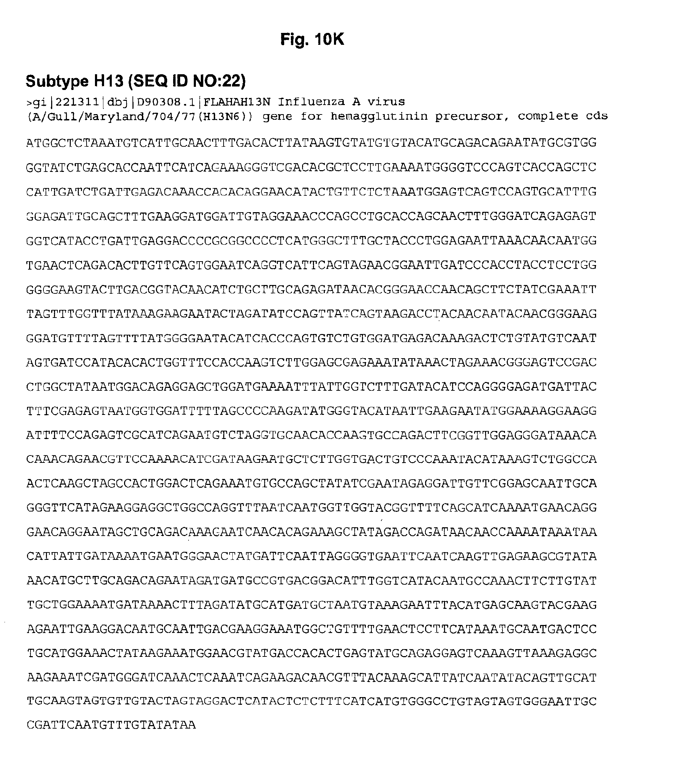

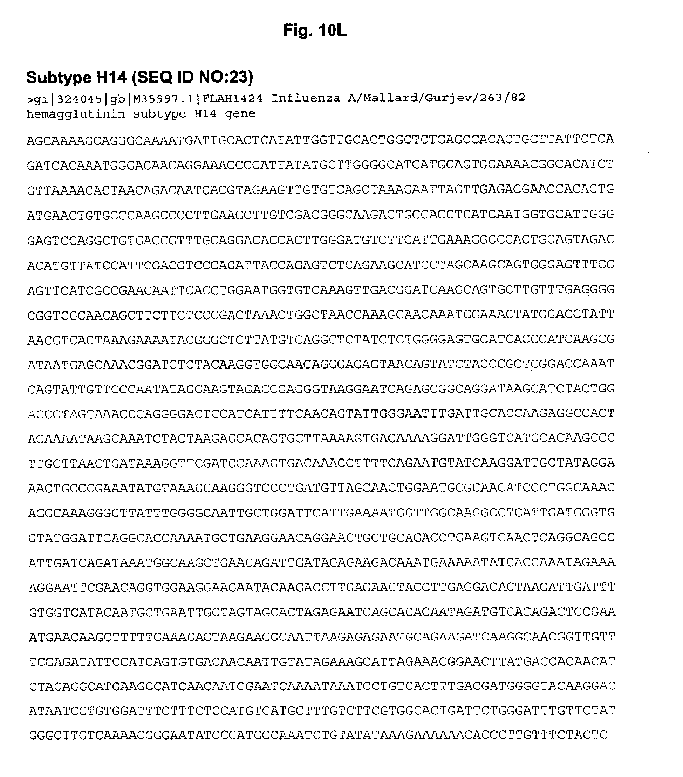

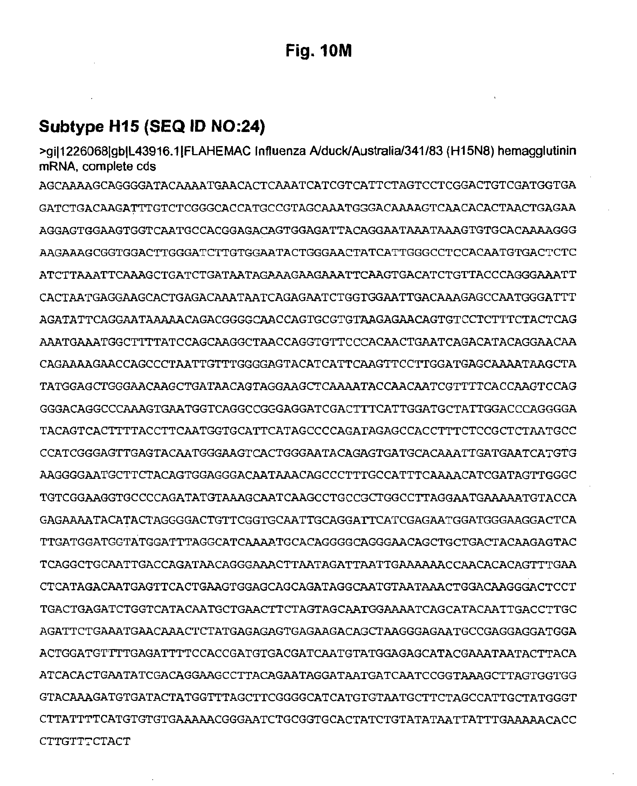

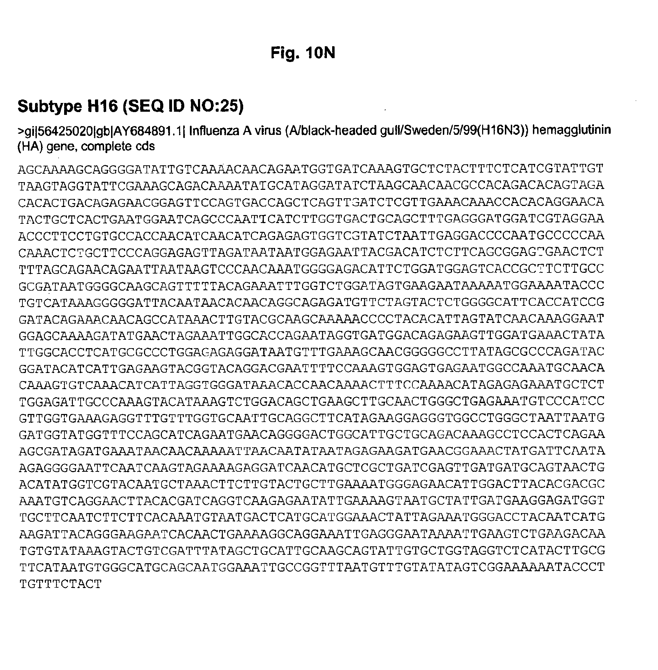

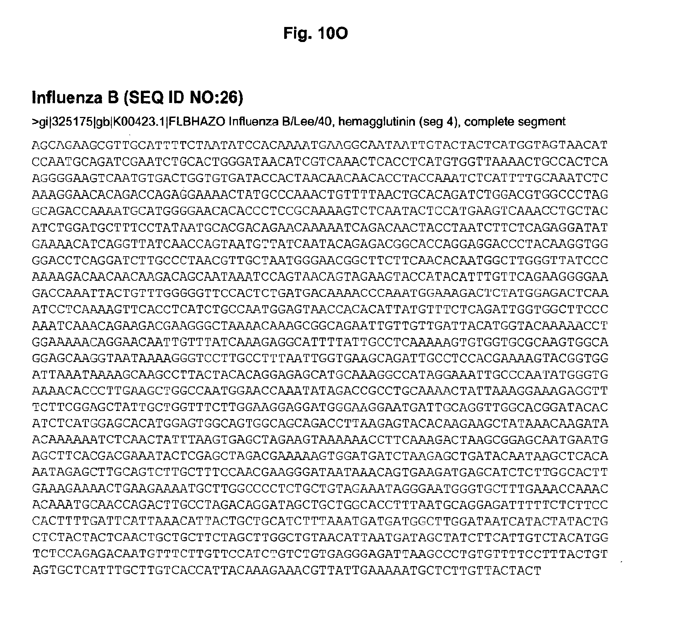

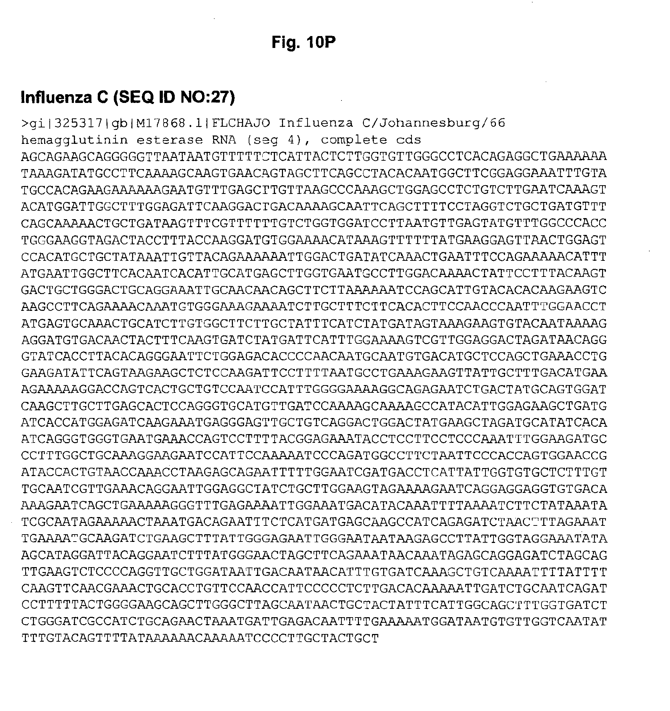

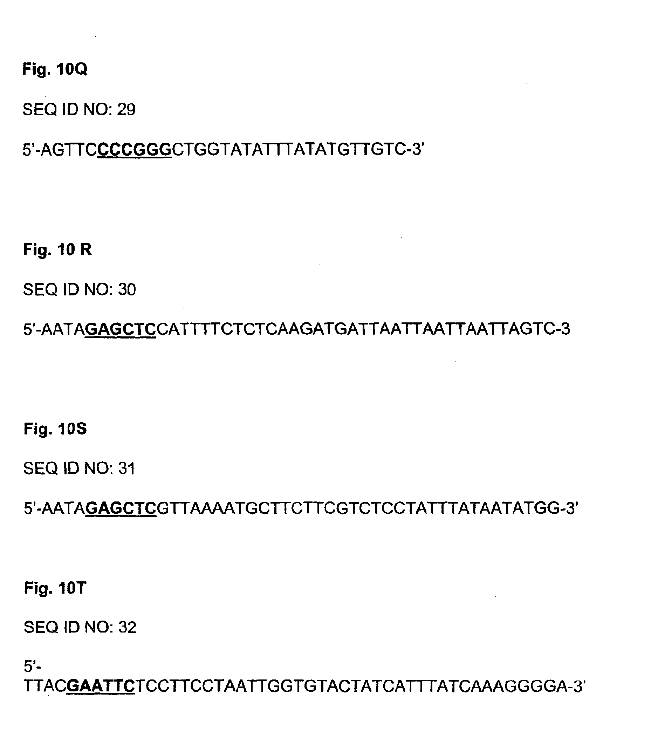

**FIGURE 10A** shows the nucleotide sequence of Influenza A HA, subtype H2 (SEQ ID NO:12). FIGURE 10B shows the nucleotide sequence of Influenza A HA subtype H3 (SEQ ID NO: 13). FIGURE 10C shows the nucleotide sequence of Influenza A HA subtype H4 (SEQ ID NO:14). FIGURE 10D shows the nucleotide sequence of Influenza A HA subtype H5 (SEQ ID NO:15). FIGURE 10E shows the nucleotide sequence of Influenza A HA subtype H6 (SEQ ID NO:16). FIGURE 10F shows the nucleotide sequence of Influenza A HA subtype H8 (SEQ ID NO: 17). FIGURE 10G shows the nucleotide sequence of Influenza A HA subtype H9 (SEQ ID NO:18). FIGURE 10H shows the nucleotide sequence of Influenza A HA subtype H10 (SEQ ID NO:19). FIGURE 10I shows the nucleotide sequence of Influenza A HA subtype H11 (SEQ ID NO:20). FIGURE 10J shows the nucleotide sequence of Influenza A HA subtype H12 (SEQ ID NO:21). FIGURE 10K shows the nucleotide sequence of Influenza A HA subtype H13 (SEQ ID NO:22). FIGURE 10L shows the nucleotide sequence of Influenza A HA subtype H14 (SEQ ID NO:23). FIGURE 10M shows the nucleotide sequence of Influenza A HA subtype H15 (SEQ ID NO:24). FIGURE 10N shows the nucleotide sequence of Influenza A HA subtype H16 (SEQ ID NO:25). FIGURE 10O shows the nucleotide sequence of Influenza B HA (SEQ ID NO:26). FIGURE 10P shows the nucleotide sequence of Influenza C HA (SEQ ID NO:27). FIGURE 10Q shows the nucleotide sequence of primer XmaI-pPlas.c (SEQ ID NO: 29). FIGURE 10R shows the nucleotide sequence of primer SacI-ATG-pPlas.r (SEQ ID NO: 30). FIGURE 10S shows the nucleotide sequence of primer SacI-PlasTer.c (SEQ ID NO: 31). FIGURE 10T shows the nucleotide sequence of primer EcoRI-PlasTer.r (SEQ ID NO: 32).

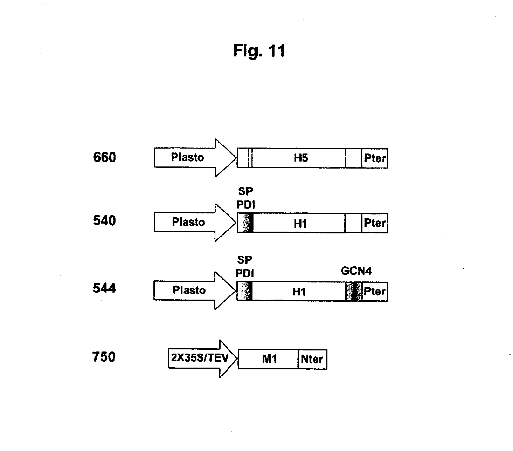

**FIGURE 11** shows a schematic representation of several constructs as used herein. Construct 660 comprises the nucleotide sequence to encode the HA subtype H5 (A/Indonesia/5/2005 (H5N1)) under operatively linked to the plastocyanin promoter (plasto) and terminator (Pter); construct 540 comprises the nucleotide sequence to encode the HA subtype H1 (A/New Caledonia/20/99 (H1N1)) in combination with an alfalfa protein disulfide isomerase signal peptide (SP PDI), and is operatively linked to a plastocyanin promoter (Plasto) and terminator (Pter); construct 544 assembled for the expression of HA subtype H1 (A/New Caledonia/20/99 (H1N1)), the nucleotide sequence encoding H1 is combined with an alfalfa protein disulfide isomerase signal peptide (SP PDI) and an GCN4pII leucine zipper (in place of the transmembrane domain and cytoplasmic tail of HI) and operatively linked to the plastocyanin promoter (Plasto) and terminator (Pter); and construct 750 for the expression of M1 coding region from influenza A/PR/8/34 is combined to the tobacco etch virus (TEV) 5'UTR, and operatively linked with the double 35S promoter and Nos terminator.

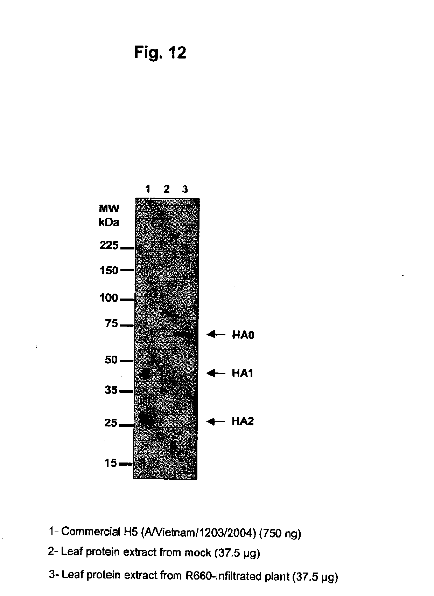

**FIGURE 12** shows immunodetection of H5 (A/Indonesia/5/2005 (H5N1)), using anti-H5 (Vietnam) antibodies, in protein extracts from *N. benthamiana* leaves transformed with construct 660 (lane 3). Commercial H5 from influenza A/Vietnam/1203/2004 was used as positive control of detection (lane 1), and a protein extract from leaves transformed with an empty vector were used as negative control (lane 2).

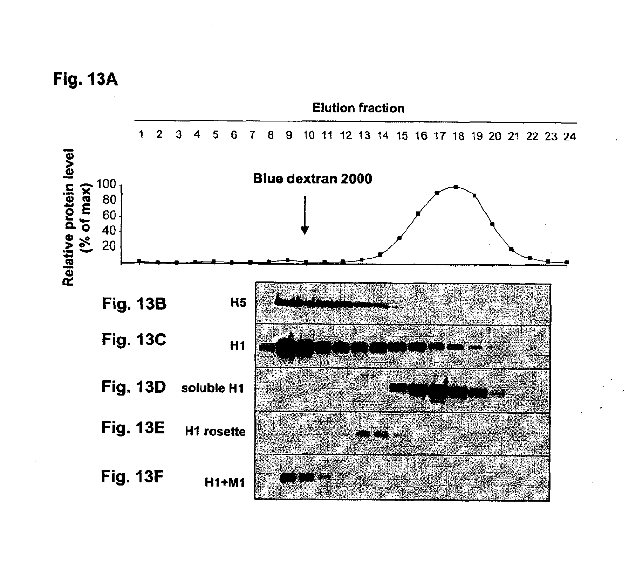

**FIGURE 13** shows characterization of hemagglutinin structures by size exclusion chromatography. Protein extract from separate biomasses producing H5 (A/Indonesia/5/2005 (H5N1)), H1 (A/New Caledonia/20/99 (H1N1)), soluble H1, or H1 and M1 were separated by gel filtration on S-500 HR. Commercial H1 (A/New Caledonia/20/99 (H1N1)) in the form of rosettes was also fractionated (H1 rosette). FIGURE 13A shows elution fractions analyzed for relative protein content (Relative Protein Level - a standard protein elution profile of a biomass fractionation is shown). Blue Dextran 2000 (2 MDa reference standard) elution peak is indicated. FIGURE 13B shows elution fractions analyzed for the presence of hemagglutinin by immunoblotting with anti-H5 (Vietnam) antibodies (for H5). FIGURE 13C shows elution fractions analyzed for anti-influenza A antibodies for H1. FIGURE 13D shows elution fractions analyzed for anti-influenza A antibodies for soluble H1. FIGURE 13E shows elution fractions analyzed for anti-influenza A antibodies for H1 rosette. FIGURE 13F shows elution fractions analyzed for anti-influenza A antibodies for H1+M1.

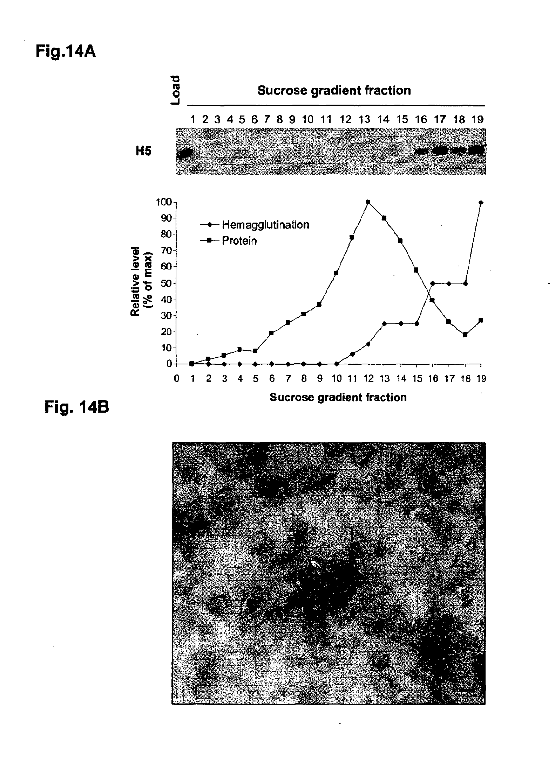

**FIGURE 14** shows concentration of influenza H5 (A/Indonesia/5/2005 (H5N1)) structures by sucrose gradient centrifugation and electron microscopy examination of hemagglutinin-concentrated fractions. FIGURE 14A shows characterization of fractions from sucrose density gradient centrifugation. Each fraction was analyzed for the presence of H5 by immunoblotting using anti-H5 (Vietnam) antibodies (upper panel), and for their relative protein content and hemagglutination capacity (graph). FIGURE 14B shows negative staining transmission electron microscopy examination of pooled fractions 17, 18 and 19 from sucrose gradient centrifugation. The bar represents 100 nm.

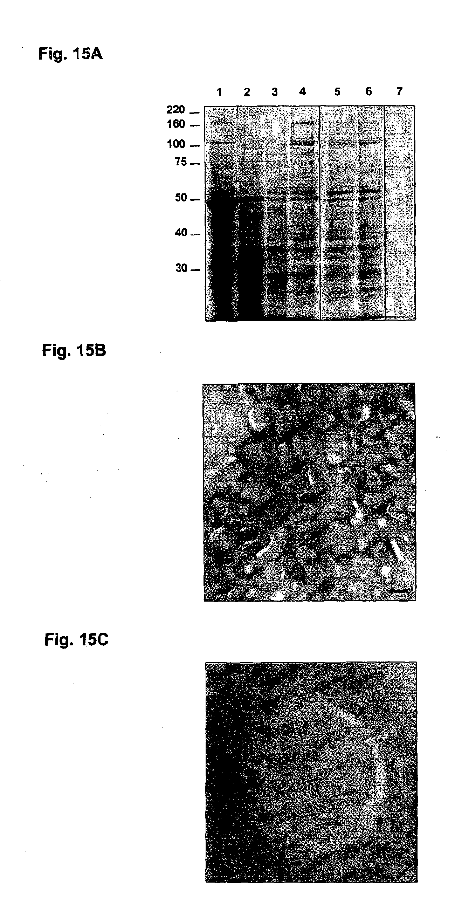

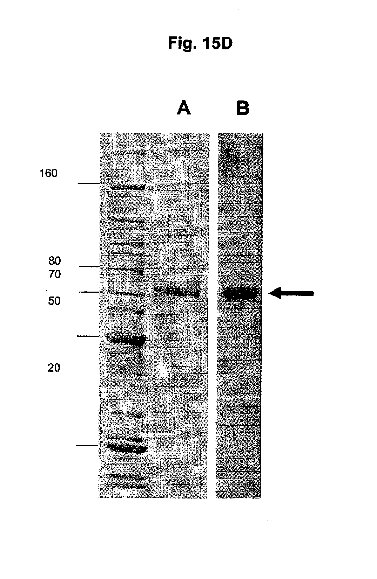

**FIGURE 15** shows purification of influenza H5 VLPs. FIGURE 15A shows Coomassie Blue stained SDS-PAGE analysis of protein content in the clarification steps - lane 1, crude extract; lane 2, pH 6-adjusted extract; lane 3, heat-treated extract; lane 4, DE-filtrated extract; the fetuin affinity purification steps: lane 5, load; lane 6, flowthrough; lane 7, elution (10X concentrated). FIGURE 15B shows negative staining transmission electron microscopy examination of the purified H5 VLP sample. The bar represents 100 nm. FIGURE 15 C shows isolated H5 VLP enlarged to show details of the structure. FIGURE 15D shows the H5 VLP product on a Coomassie-stained reducing SDS-PAGE (lane A) and Western blot (lane B) using rabbit polyclonal antibody raised against HA from strain A/Vietnam/1203/2004 (H5N1).

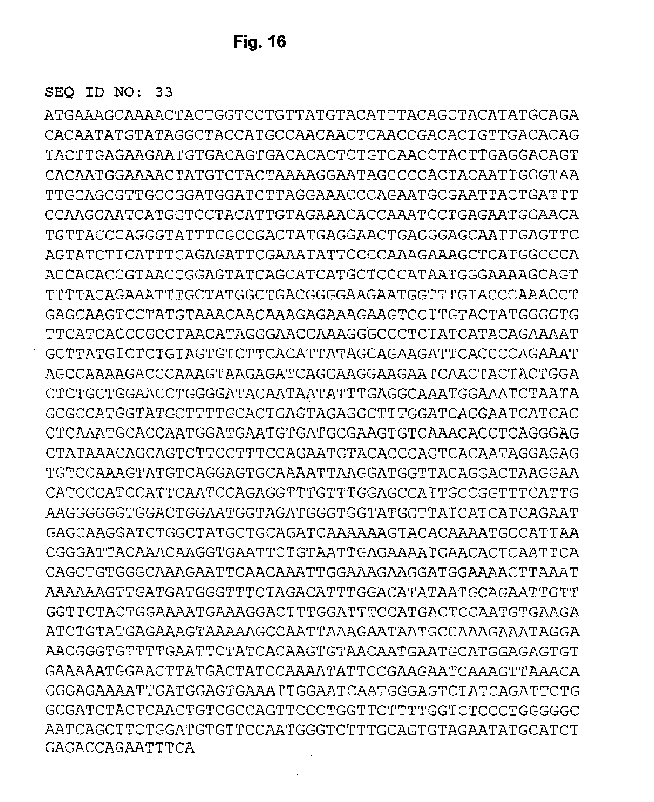

**FIGURE 16** shows a nucleotide sequence for Influenza A virus (A/New Caledonia/20/99(H1N1)) hemagglutinin (HA) gene, complete cds. GenBank Accession No. AY289929 (SEQ ID NO: 33)

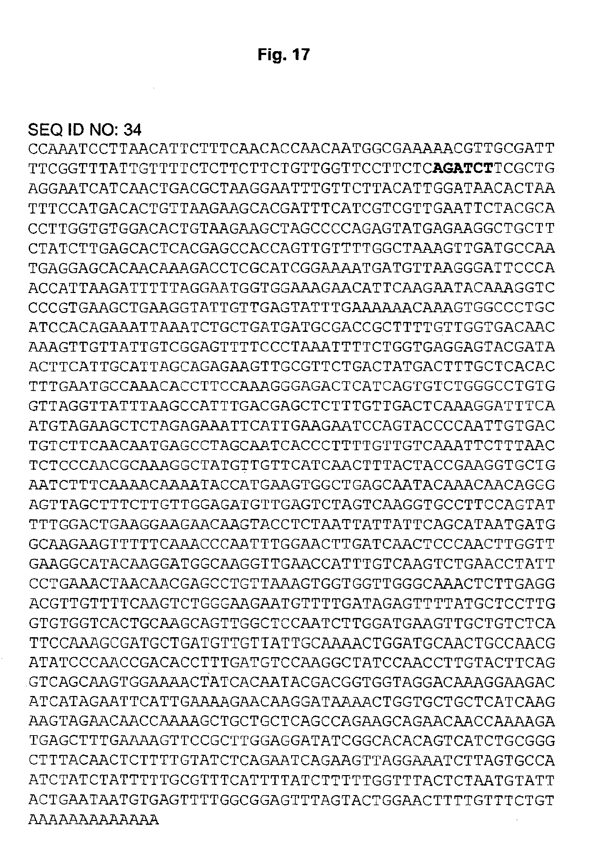

**FIGURE 17** shows a nucleotide sequence for *Medicago sativa* mRNA for protein disulfide isomerase. GenBank Accession No. Z11499 (SEQ ID NO: 34).

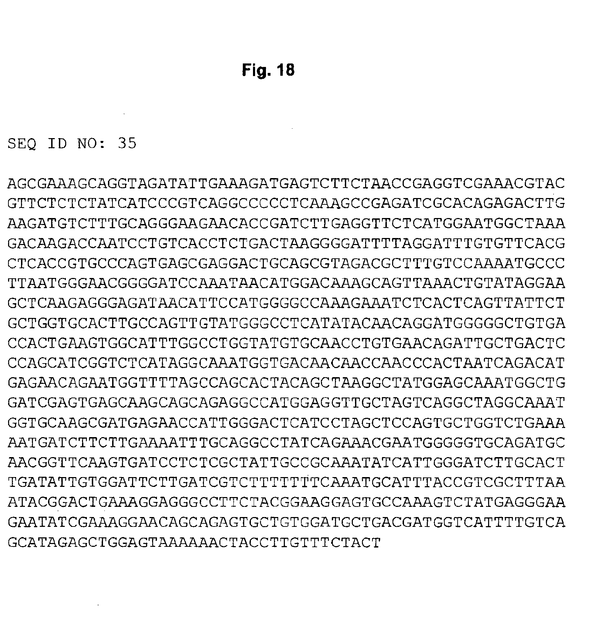

**FIGURE 18** shows a nucleotide sequence for Influenza A virus (A/Puerto Rico/8/34(HlNl)) segment 7, complete sequence. GenBank Accession No. NC_002016.1 (SEQ ID NO: 35).

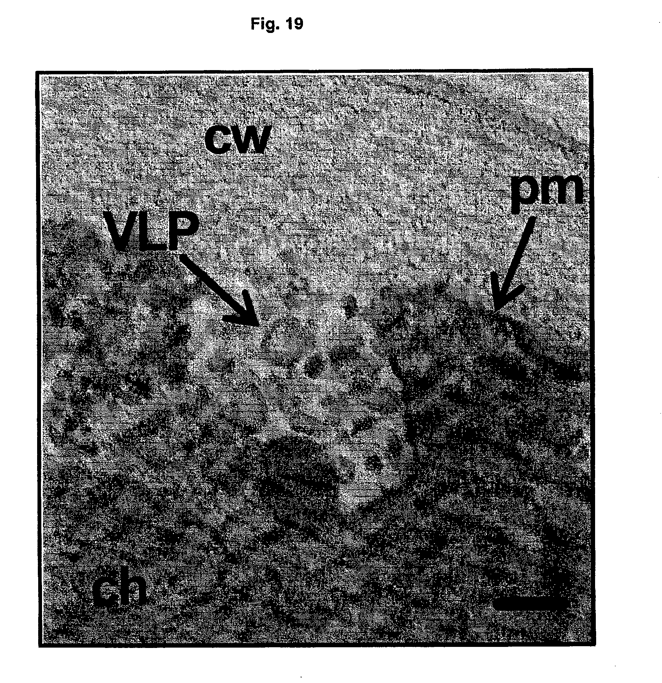

**FIGURE 19** shows localization of VLP accumulation by positive staining transmission electron microscopy observation of H5 producing tissue. CW: cell wall, ch: chloroplast, pm: plasma membrane, VLP: virus-like particle. The bar represents 100 nm.

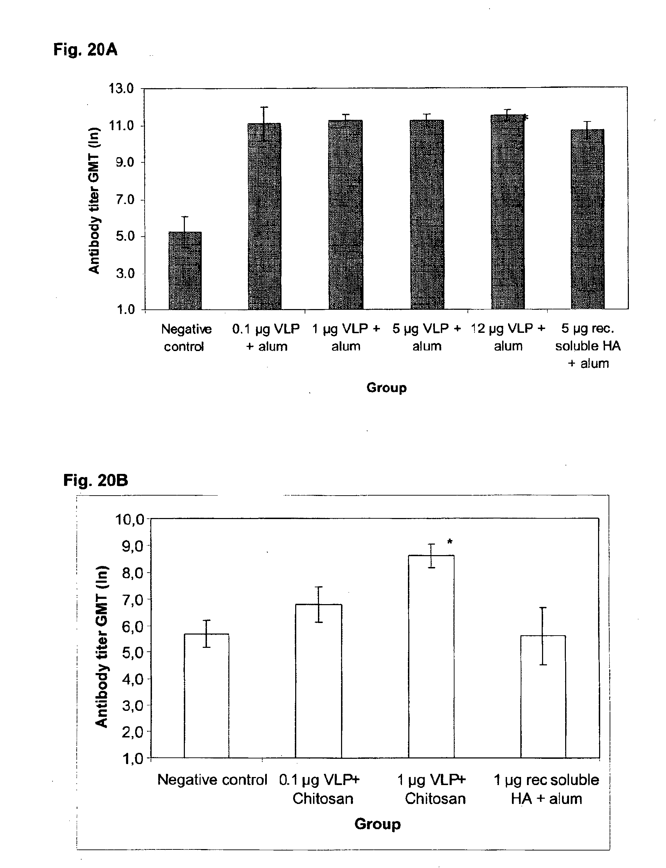

**FIGURE 20** shows induction of serum antibody responses 14 days after boost in Balb/c mice vaccinated with plant-made influenza H5 VLP (A/Indonesia/5/2005 (H5N1)) or recombinant soluble H5 (A/Indonesia/5/2005 (H5N1)). FIGURE 20(A) Antibody responses of mice immunized through intramuscular injection. FIGURE 20(B) Antibody responses of mice immunized through intranasal administration. Antibody responses were measured against inactivated whole H5N1 viruses (A/Indonesia/5/05). GMT: geometric mean titer. Values are the GMT (ln) of reciprocal end-point titers of five mice per group. Bars represent *mean deviation.* * *p<* 0.05 compared to recombinant soluble H5.

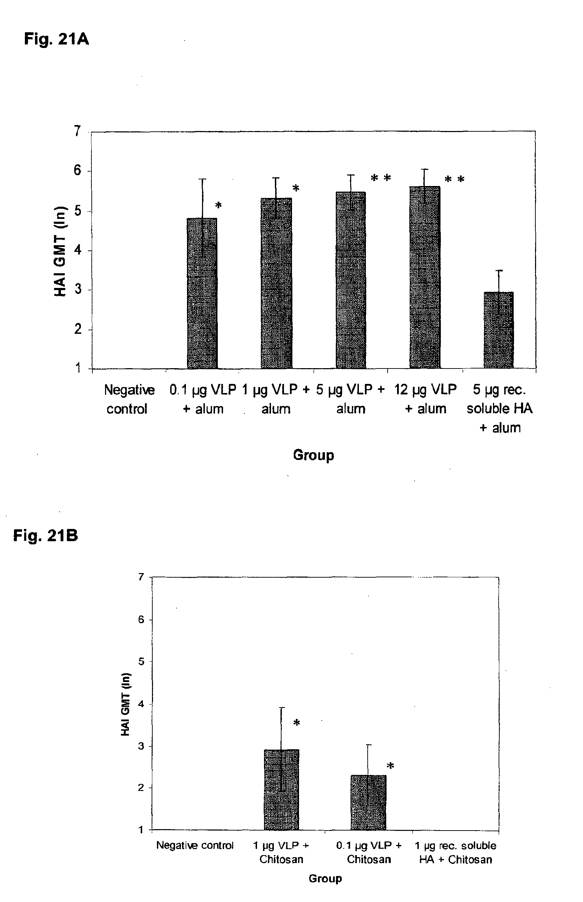

**FIGURE 21** shows hemagglutination inhibition antibody response (HAI) 14 days after boost in Balb/c mice vaccinated with plant-made influenza H5 VLP (A/Indonesia/5/2005 (H5N1)) or recombinant soluble H5 (A/Indonesia/5/2005 (H5N1)). FIGURE 21(A) Antibody responses of mice immunized through intramuscular injection. FIGURE 21 (B) Antibody responses of mice immunized through intranasal administration. HAI antibody responses were measured using inactivated whole H5N1 viruses (A/Indonesia/5/05). GMT: geometric mean titer. Values are the GMT (ln) of reciprocal end-point titers of five mice per group. Bars represent *mean deviation.* * *p<* 0.05 and ** *p*< 0.01 compared to recombinant soluble H5.

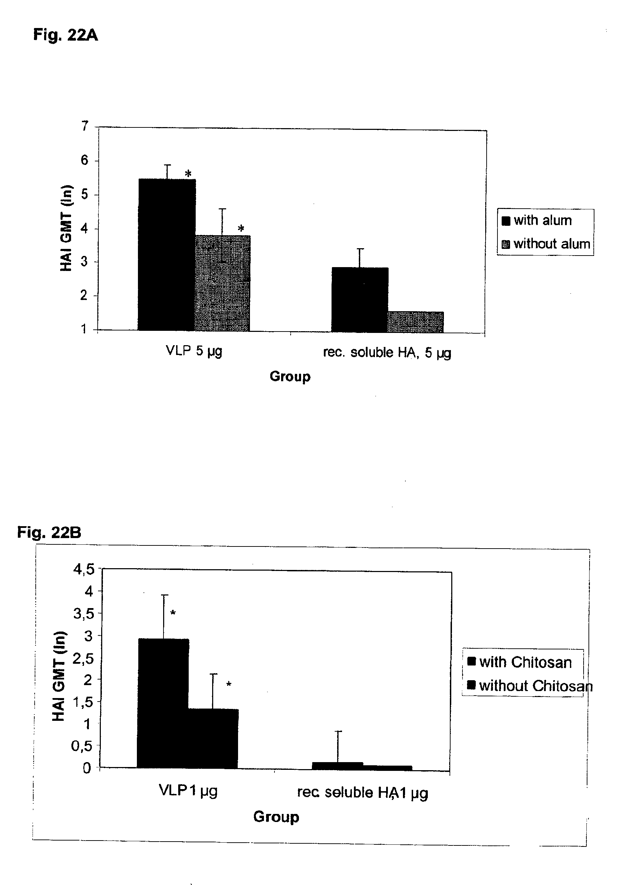

**FIGURE 22** shows the effect of adjuvant on immunogenicity of the VLPs in Balb/c mice. FIGURE 22(A) Effect of alum on mice immunized through intramuscular injection. FIGURE 22(B) Effect of Chitosan on mice immunized through intranasal administration. HAI antibody responses were measured using inactivated whole H5N1 viruses (A/Indonesia/5/05). GMT: geometric mean titer. Values are the GMT (In) of reciprocal end-point titers of five mice per group. Bars represent *mean deviation.* * *p<* 0.05 compared to the corresponding recombinant soluble H5.

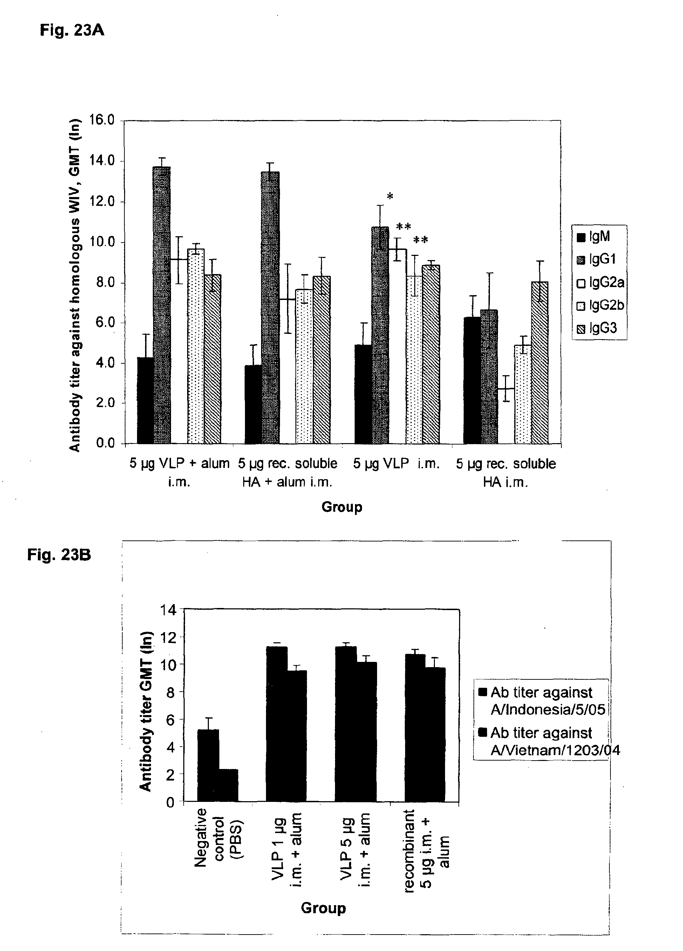

**FIGURE 23** shows antibody response toH5 VLP (A/Indonesia/5/2005 (H5N1)) administration. FIGURE 23(A) Anti-Indonesia/5/05 immunoglobulin isotype in mice immunized through intramuscular administration, 30 days after boost. Values are the GMT (log₂) of reciprocal end-point titers of five mice per group. ELISA performed using whole inactivated H5N1 (A/Indonesia/5/2005) viruses as the coating agent. Bars represent *mean deviation.* * *p<* 0.05, ** *p<* 0.001 compared to the corresponding recombinant soluble H5 (A/Indonesia/5/2005 (H5N1)). FIGURE 23(B) Antibody titers against whole inactivated viruses (A/Indonesia/5/2005 (H5N1) and (A/Vietnam/1194/04 (H5N1))). All groups are statistically different to negative control.

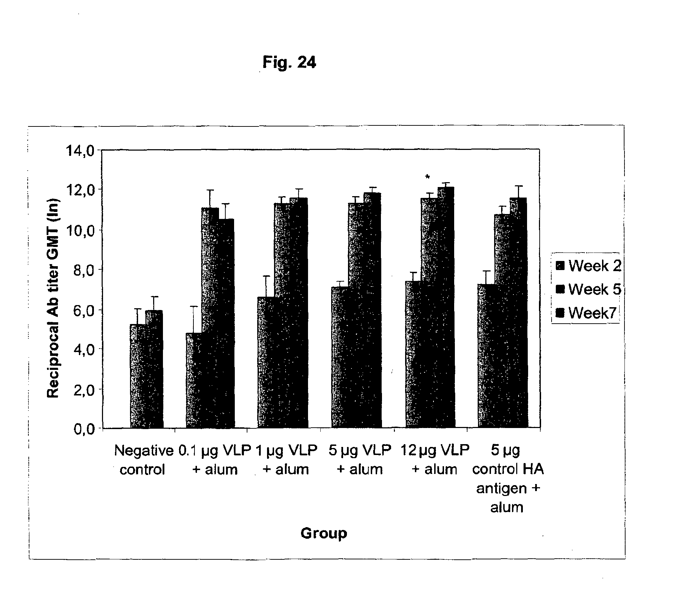

**FIGURE 24** shows antibody titer against homologous whole inactivated viruses (A/Indonesia/5/05), 14 days weeks after first dose (week 2), 14 days after boost (week 5) or 30 days after boost (week 7) from Balb/c mice immunized with H5 VLP (A/Indonesia/5/2005 (H5N1)). GMT: geometric mean titer. Values are the GMT (ln) of reciprocal end-point titers of five mice per group. * *p<* 0.05 compared to recombinant soluble H5.

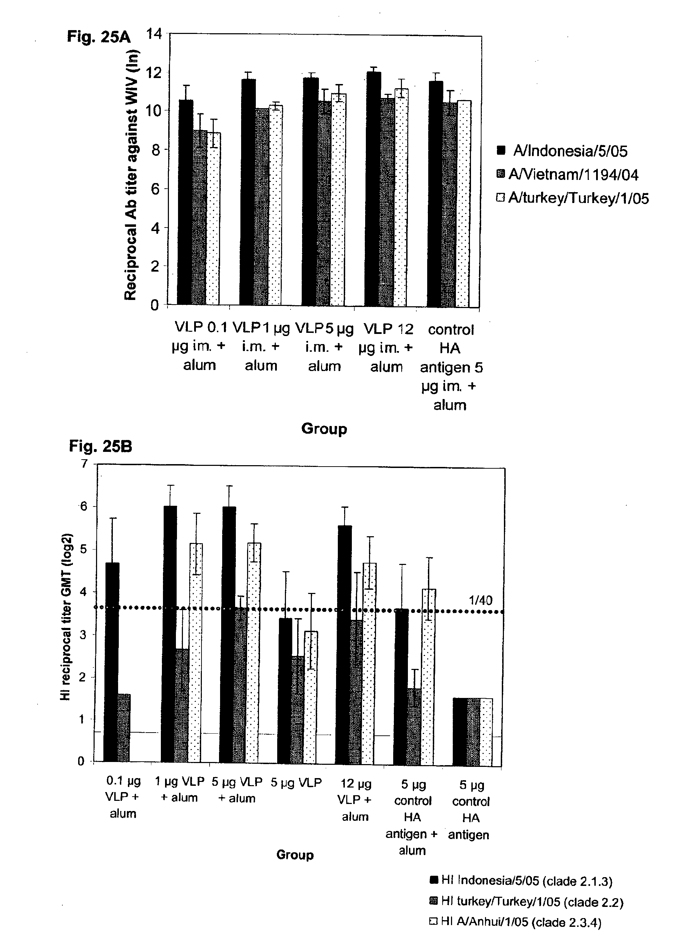

**FIGURE 25** shows *in vitro* cross-reactivity of serum antibodies from Balb/c mice immunized with H5 VLP (A/Indonesia/5/2005 (H5N1)) 30 days after boost. (A) Antibody titers whole inactivated viruses. (B) Hemagglutination-inhibition titers against various whole inactivated viruses. Values are the GMT (ln) of reciprocal end-point titers of five mice per group. Bars represent *mean deviation.* All groups are statistically different to negative control. * *p<* 0.05 compared to the corresponding recombinant soluble H5. All values less than 10 were given an arbitrary value of 5 (1.6 for In) and are considered negative.

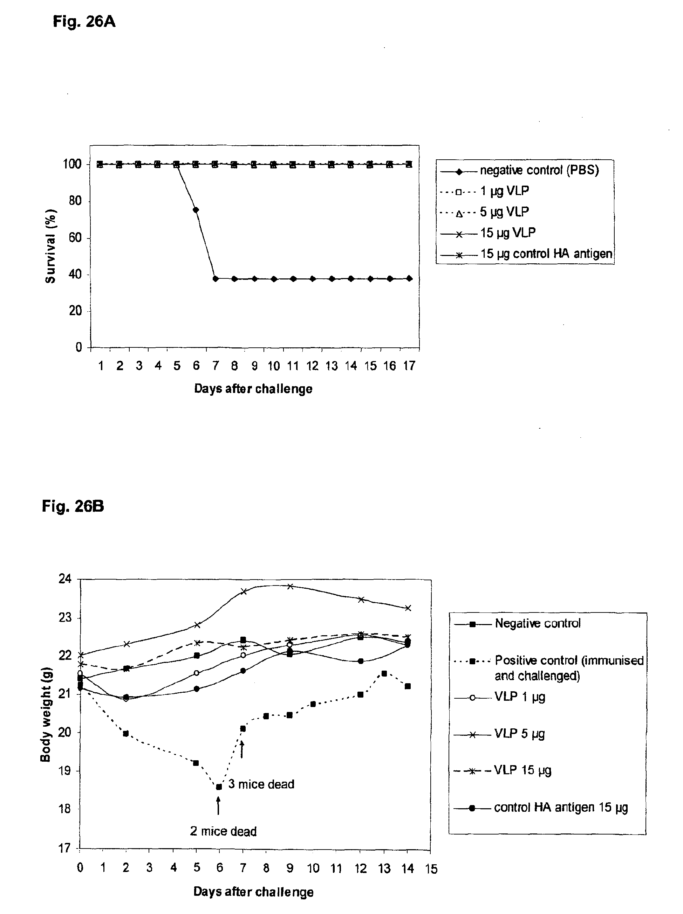

**FIGURE 26** shows efficacy of the plant made H5 VLP (A/Indonesia/5/2005 (H5N1)). (A) Survival rate of mice after challenge with 1000 LD₅₀ (4.09x10⁶ CCID₅₀) of the influenza strain A/Turkey/582/06 (H5N1) (B) Body weight of immunised mice after challenge. Values are the mean body weight of surviving mice

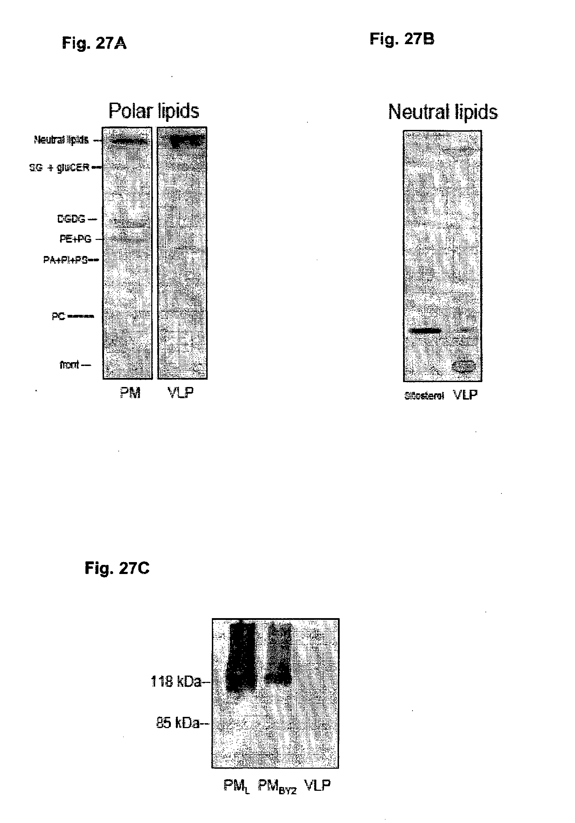

**FIGURE 27** shows origin of plant-derived influenza VLPs. **(A)** Polar lipid composition of purified influenza VLPs. Lipids contained in an equivalent of 40 µg of proteins, were extracted from VLP as described, separated by HP-TLC, and compared to the migration profile of lipids isolated from highly purified tobacco plasma membrane (PM). Lipid abbreviations are as following: DGDG, Digalactosyldiacylglycerol; gluCER, glucosyl-ceramide; PA, phosphatic acid; PC, phosphatidylcholine; PE, phosphatidylethanolamine; PG, phosphatidylglycerol; PI, phosphatidylinositol; PS, phosphatidylserine; SG, Steryl-glycoside. **(B)** Neutral lipid composition of purified influenza VLPs. Lipids contained in an equivalent of 20 µg of proteins were extracted from VLP as described, separated by HP-TLC and compared to the migration of sitosterol. (C) Immunodetection of the plasma membrane marker proton pump ATPase (PMA) in purified VLPs and highly-purified PM from tobacco leaves (PM_{L}) and BY2 tobacco cells (PM_{BY2}). Eighteen micrograms of protein were loaded in each lane.

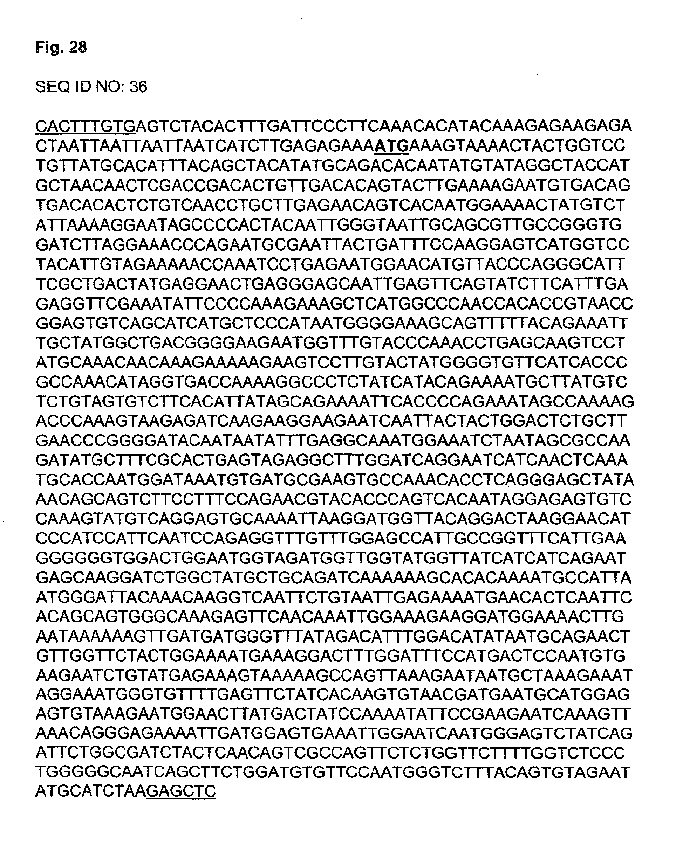

**FIGURE 28** shows the sequence spanning from DraIII to SacI sites of clone 774 - nucleotide sequence of ABrisbane/59/2007 (H1N1) (SEQ ID NO: 36). The coding sequence is flanked by a plastocyanin regulatory region, starting with a DraIII restriction site at the 5' end and by a stop codon and a SacI site at the 3' end. Restriction sites are underlined; ATG is in bold and underlined.

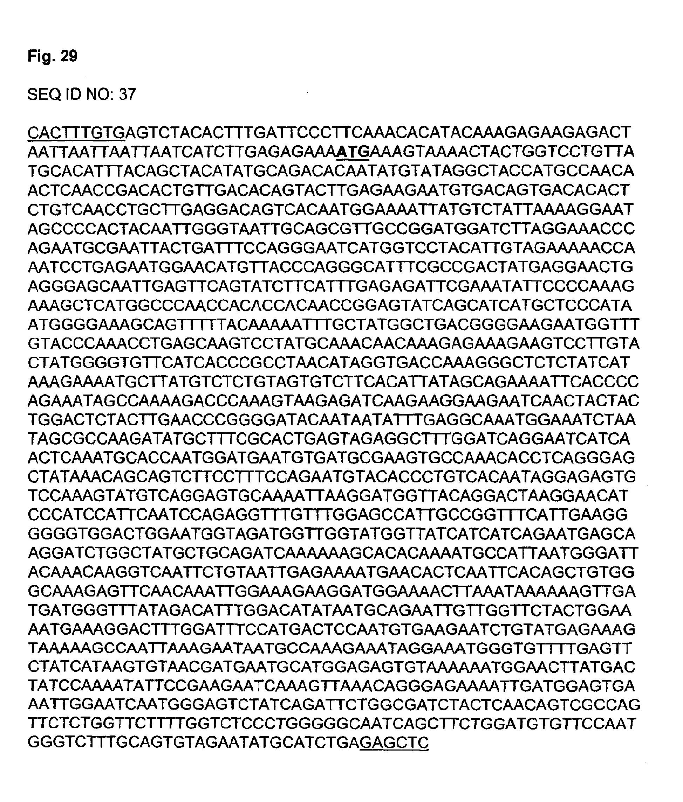

**FIGURE 29** shows the sequence spanning from DraIII to SacI sites of clone 775-nucleotide sequence of A/Solomon Islands 3/2006 (H1N1) (SEQ ID NO: 37). The coding sequence is flanked by a plastocyanin regulatory region, starting with a DraIII restriction site at the 5' end and by a stop codon and a SacI site at the 3' end. Restriction sites are underlined; ATG is in bold and underlined.

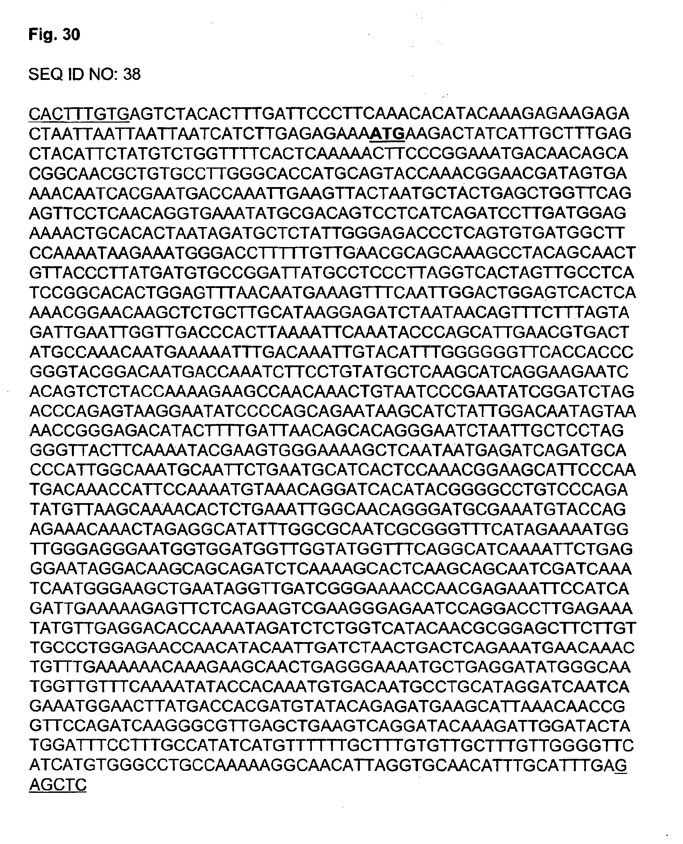

**FIGURE 30** shows the sequence spanning from DraIII to SacI sites of clone 776-nucleotide sequence of A/Brisbane 10/2007 (H3N2) (SEQ ID NO: 38). The coding sequence is flanked by a plastocyanin regulatory region, starting with a DraIII restriction site at the 5' end and by a stop codon and a SacI site at the 3' end. Restriction sites are underlined; ATG is in bold and underlined.

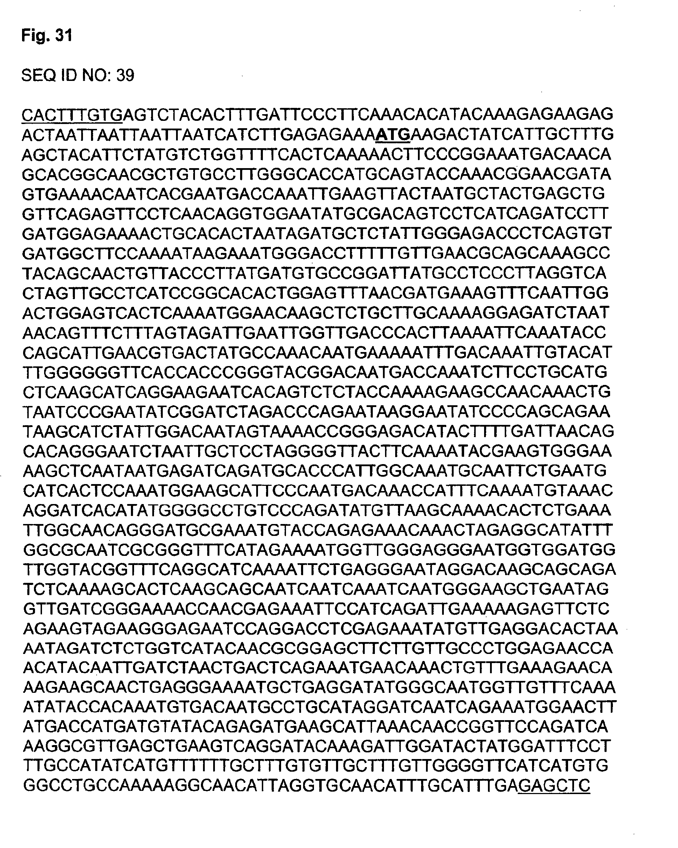

**FIGURE 31** shows the sequence spanning from DraIII to SacI sites of clone 777 - nucleotide sequence of A/Wisconsin/67/2005 (H3N2) (SEQ ID NO: 39). The coding sequence is flanked by a plastocyanin regulatory region, starting with a DraIII restriction site at the 5' end and by a stop codon and a SacI site at the 3' end. Restriction sites are underlined; ATG is in bold and underlined.

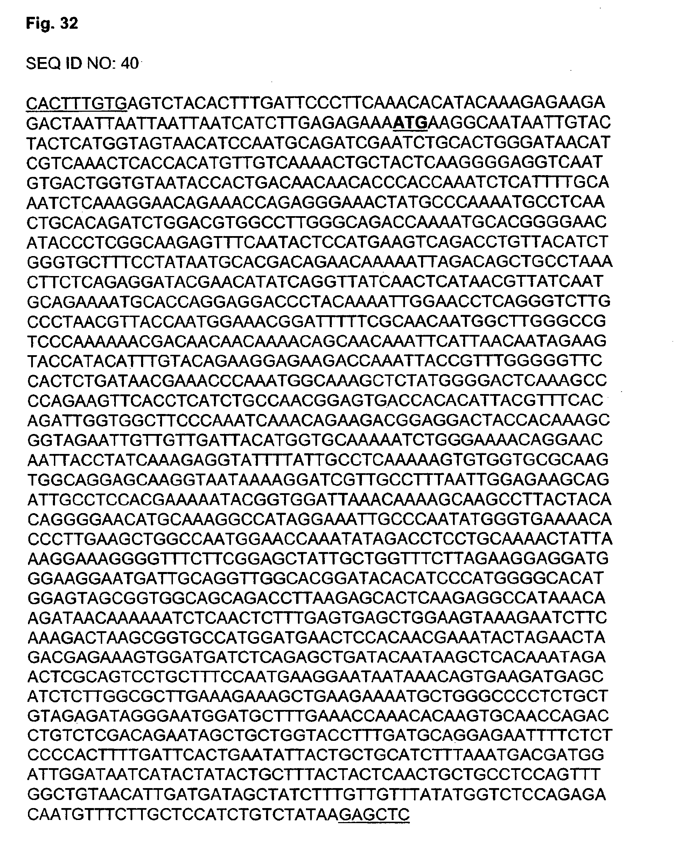

**FIGURE 32** shows the sequence spanning from DraIII to SacI sites of clone 778 - nucleotide sequence of B/Malaysia/2506/2004 (SEQ ID NO: 40). The coding sequence is flanked by a plastocyanin regulatory region, starting with a DraIII restriction site at the 5' end and by a stop codon and a SacI site at the 3' end. Restriction sites are underlined; ATG is in bold and underlined.

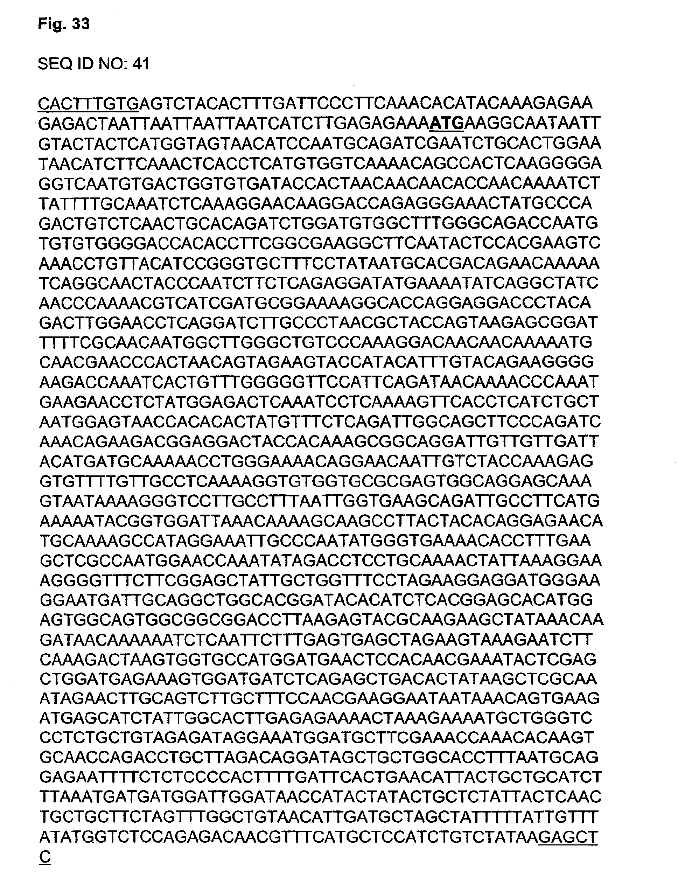

**FIGURE 33** shows the sequence spanning from DraIII to SacI sites of clone 779-nucleotide sequence of B/Florida/4/2006 (SEQ ID NO: 41). The coding sequence is flanked by a plastocyanin regulatory region, starting with a DraIII restriction site at the 5' end and by a stop codon and a SacI site at the 3' end. Restriction sites are underlined; ATG is in bold and underlined.

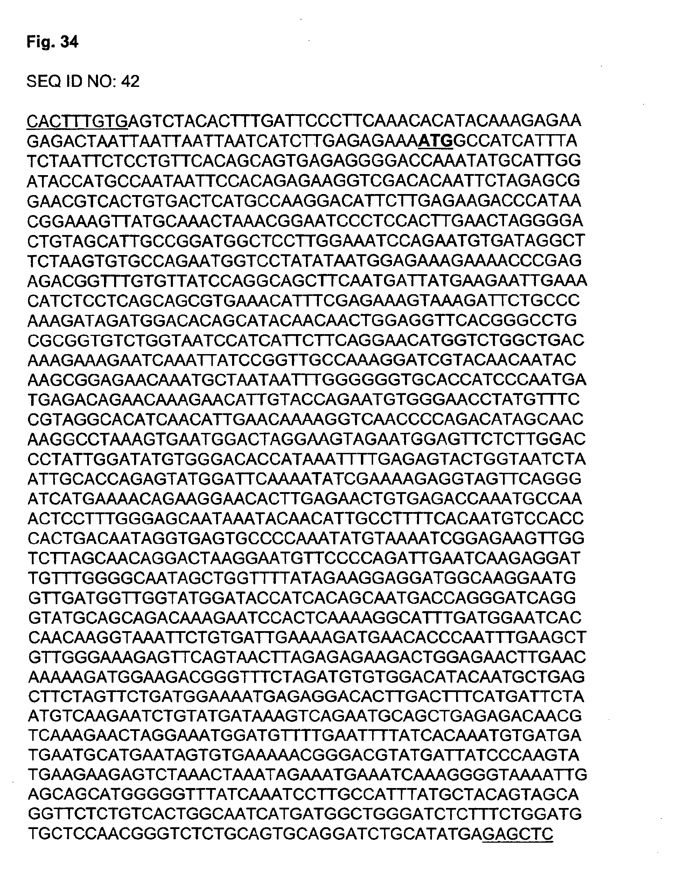

**FIGURE 34** shows the sequence spanning from DraIII to SacI sites of clone 780 - nucleotide sequence of A/Singapore/1/57 (H2N2) (SEQ ID NO: 42). The coding sequence is flanked by a plastocyanin regulatory region, starting with a DraIII restriction site at the 5' end and by a stop codon and a SacI site at the 3' end. Restriction sites are underlined; ATG is in bold and underlined.

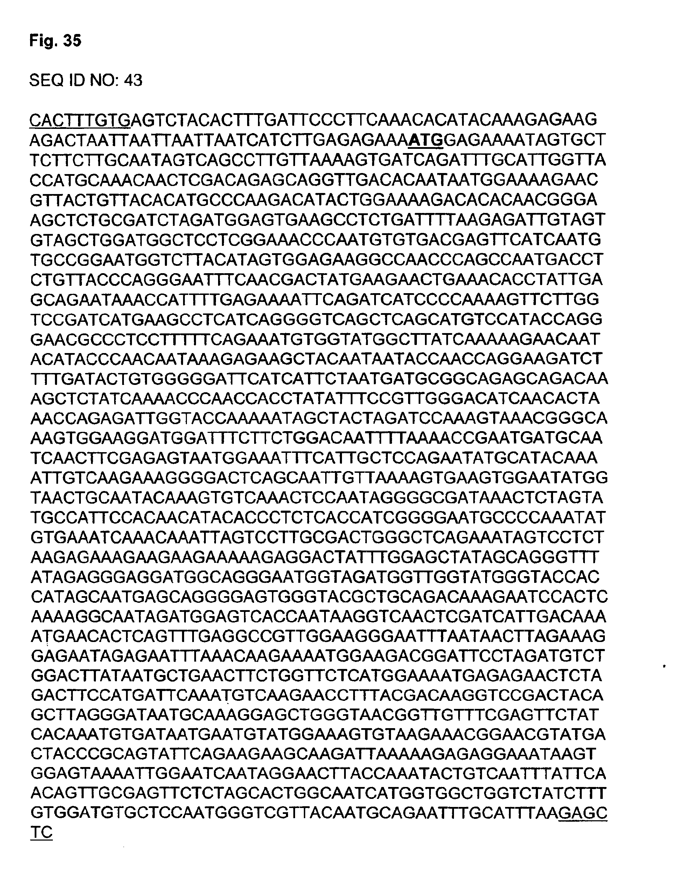

**FIGURE 35** shows the sequence spanning from DraIII to SacI sites of clone 781 - nucleotide sequence of A/Anhui/1/2005 (H5N1) (SEQ ID NO: 43). The coding sequence is flanked by a plastocyanin regulatory region, starting with a DraIII restriction site at the 5' end and by a stop codon and a SacI site at the 3' end. Restriction sites are underlined; ATG is in bold and underlined.

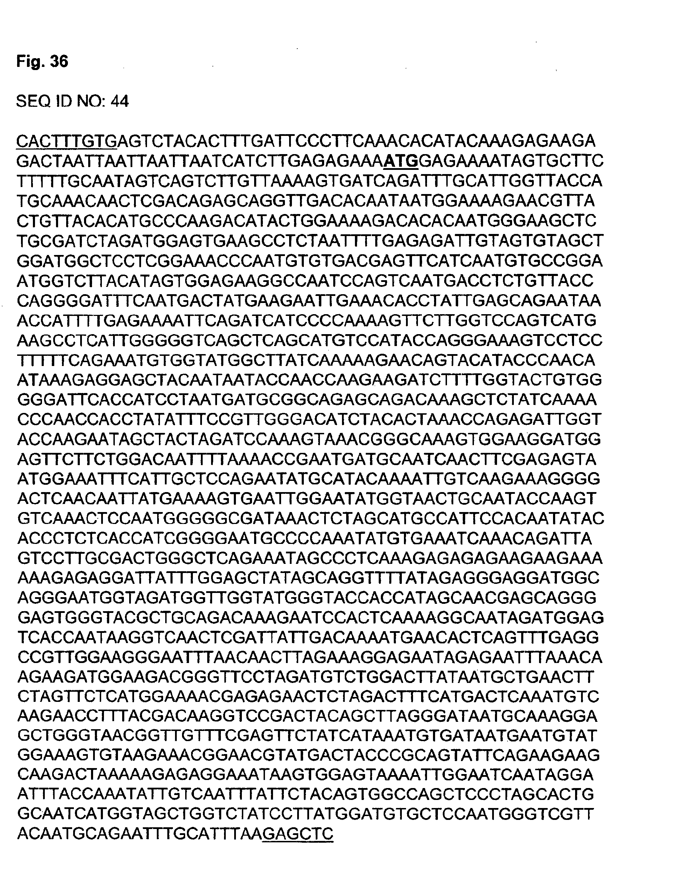

**FIGURE 36** shows the sequence spanning from DraIII to SacI sites of clone 782 - nucleotide sequence of A/Vietnam/1194/2004 (H5N1) (SEQ ID NO: 44). The coding sequence is flanked by a plastocyanin regulatory region, starting with a DraIII restriction site at the 5' end and by a stop codon and a SacI site at the 3' end. Restriction sites are underlined; ATG is in bold and underlined.

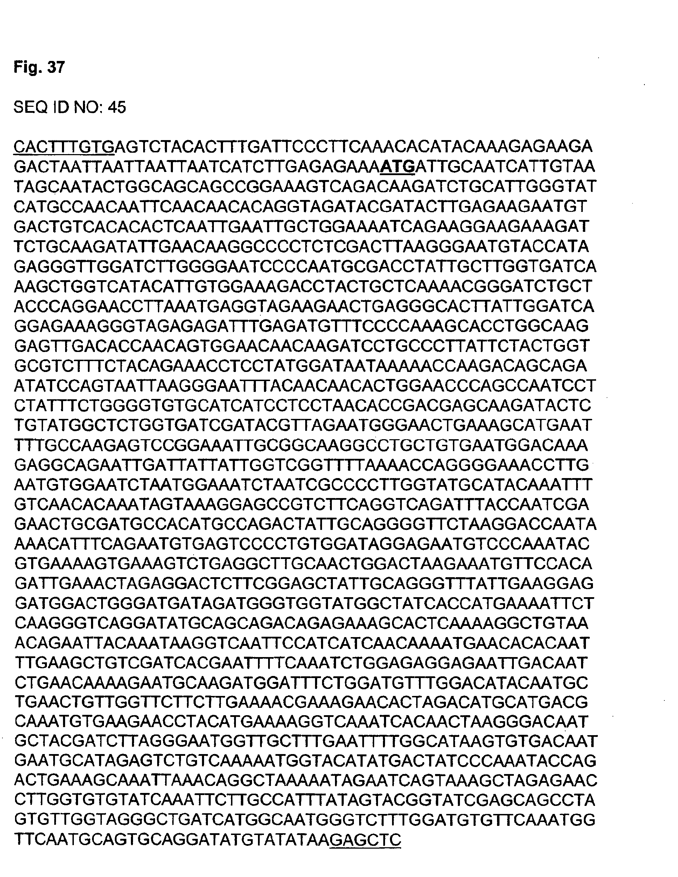

**FIGURE 37** shows the sequence spanning from DraIII to SacI sites of clone 783 - nucleotide sequence of A/Teal/HongKong/W312/97 (H6N1) (SEQ ID NO: 45). The coding sequence is flanked by a plastocyanin regulatory region, starting with a DraIII restriction site at the 5' end and by a stop codon and a SacI site at the 3' end. Restriction sites are underlined; ATG is in bold and underlined.

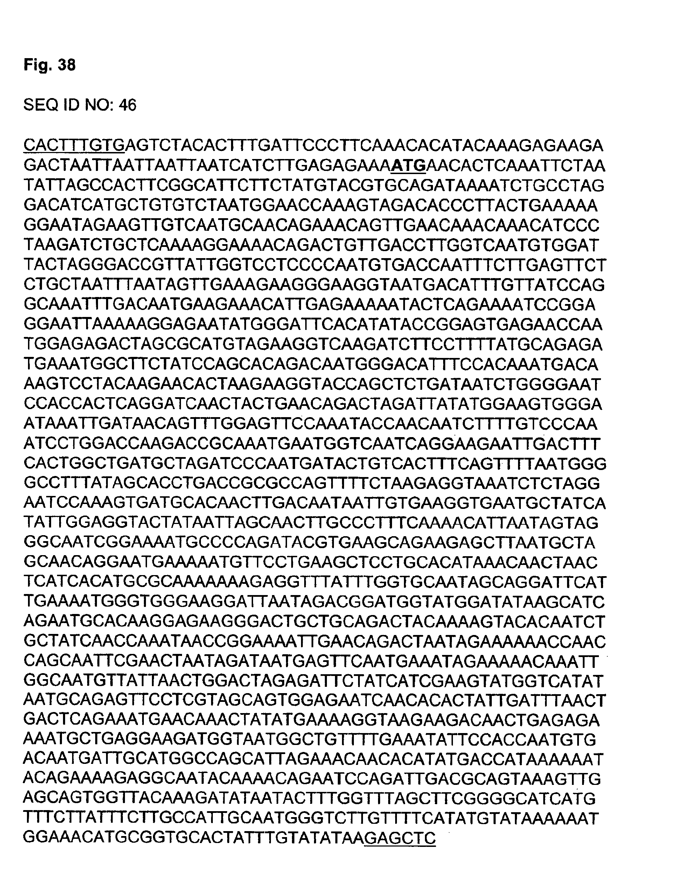

**FIGURE 38** shows the sequence spanning from DraIII to SacI sites of clone 784 - nucleotide sequence of A/Equine/Prague/56 (H7N7) (SEQ ID NO: 46). The coding sequence is flanked by a plastocyanin regulatory region, starting with a DraIII restriction site at the 5' end and by a stop codon and a SacI site at the 3' end. Restriction sites are underlined; ATG is in bold and underlined.

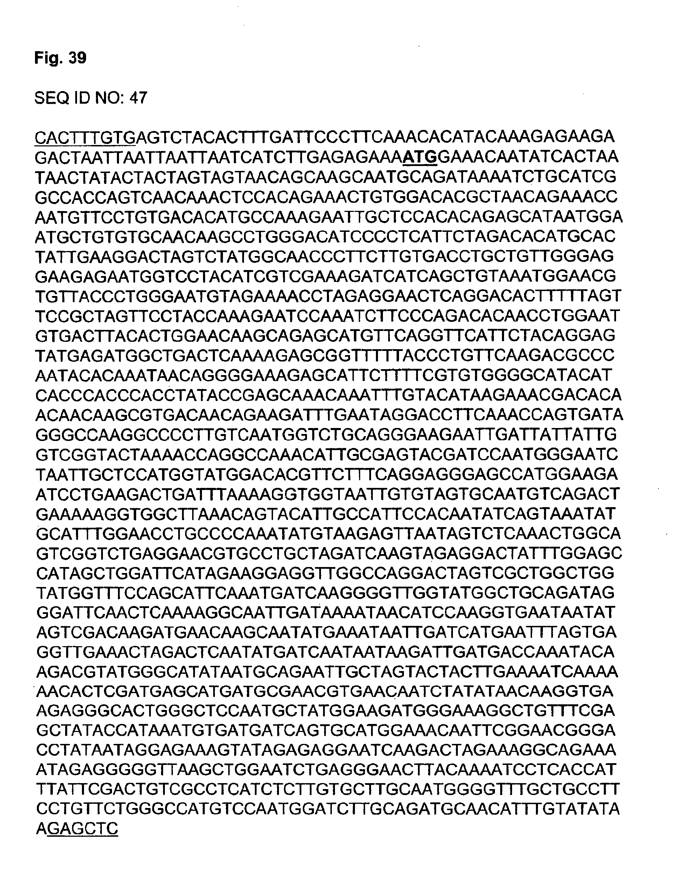

**FIGURE 39** shows the sequence spanning from DraIII to SacI sites of clone 785 - nucleotide sequence of A/HongKong/1073/99 (H9N2) (SEQ ID NO: 47). The coding sequence is flanked by a plastocyanin regulatory region, starting with a DraIII restriction site at the 5' end and by a stop codon and a SacI site at the 3' end. Restriction sites are underlined; ATG is in bold and underlined.

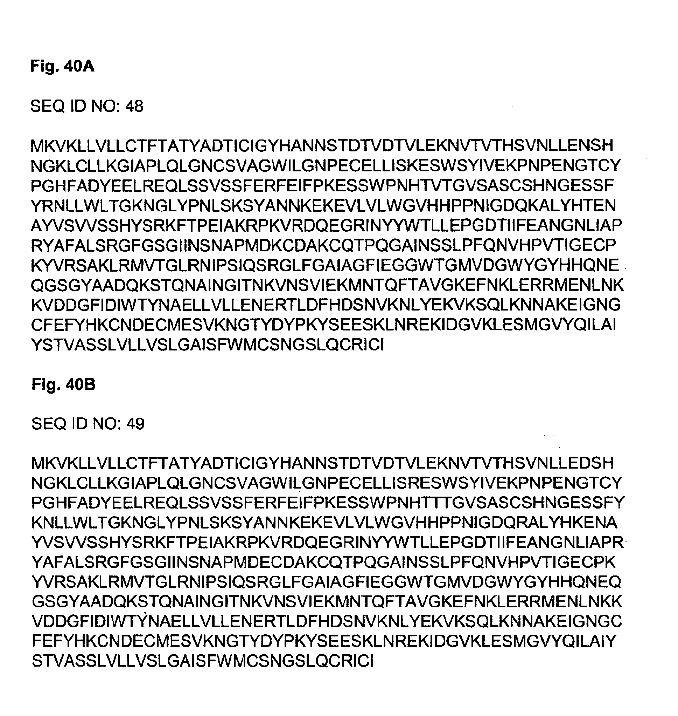

**FIGURE 40A** shows the amino acid sequence (SEQ ID NO: 48) of the polypeptide translated from clone 774 (A/Brisbane/5 9/2007 (H1N1)). The open reading frame of clone 774 starts with the ATG indicated in Figure 28. Figure 40B shows the amino acid sequence (SEQ ID NO: 49) of the polypeptide translated from clone 775 (A/Solomon Islands 3/2006 (H1N1)). The open reading frame of clone 775 starts with the ATG indicated in Figure 29.

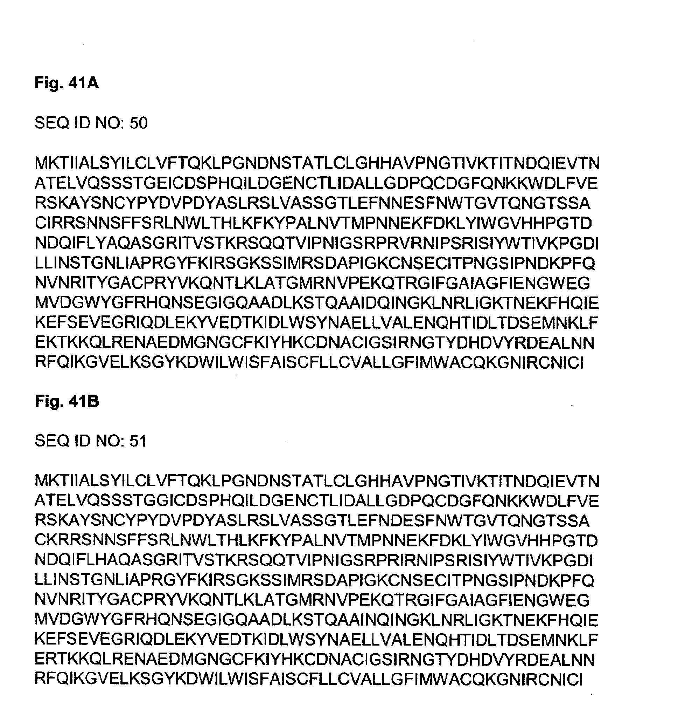

**FIGURE 41A** shows the amino acid sequence (SEQ ID NO: 50) of the polypeptide translated from clone 776 (A/Brisbane/10/2007 (H3N2)). The open reading frame of clone 776 starts with the ATG indicated in Figure 30. Figure 41B shows the amino acid sequence (SEQ ID NO: 51) of the polypeptide translated from clone 777 (A/Wisconsin/67/2005 (H3N2)). The open reading frame of clone 777 starts with the ATG indicated in Figure 31.

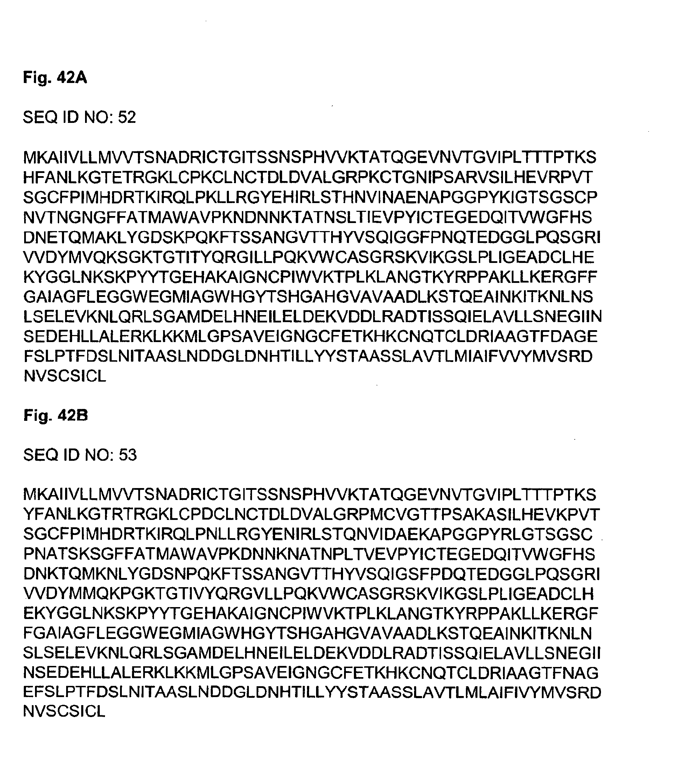

**FIGURE 42A** shows the amino acid sequence (SEQ ID NO: 52) of the polypeptide translated from clone 778 (B/Malaysia/2506/2004). The open reading frame of clone 778 starts with the ATG indicated in Figure 32. Figure 42B shows the amino acid sequence (SEQ ID NO: 53) of the polypeptide translated from clone 779 (B/Florida/4/2006). The open reading frame of clone 779 starts with the ATG indicated in Figure 33.

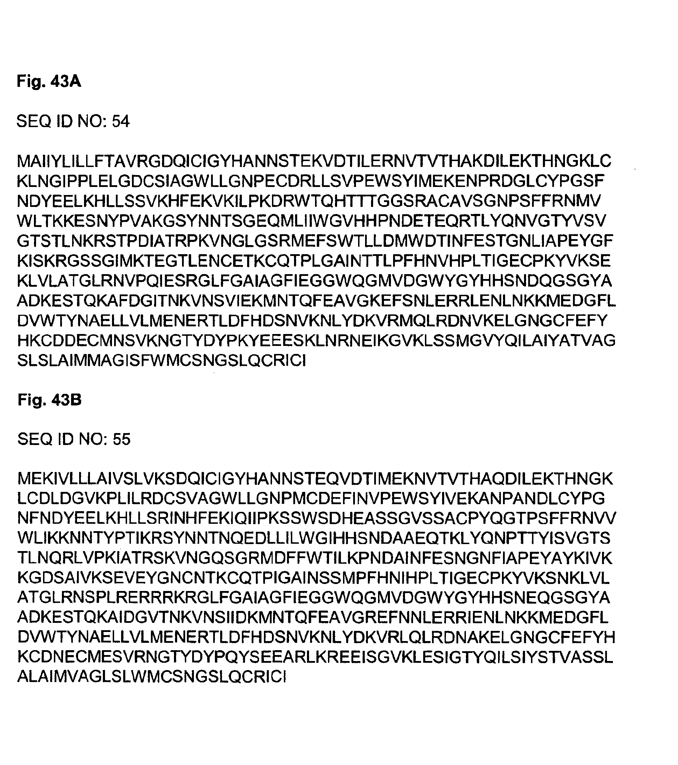

**FIGURE 43A** shows the amino acid sequence (SEQ ID NO: 54) of the polypeptide translated from clone 780 (A/Singapore/1/57 (H2N2)). The open reading frame of clone 780 starts with the ATG indicated in Figure 34. Figure 43B shows the amino acid sequence (SEQ ID NO: 55) of the polypeptide translated from clone 781 (A/Anhui/1/2005 (H5N1)). The open reading frame of clone 781 starts with the ATG indicated in Figure 35.

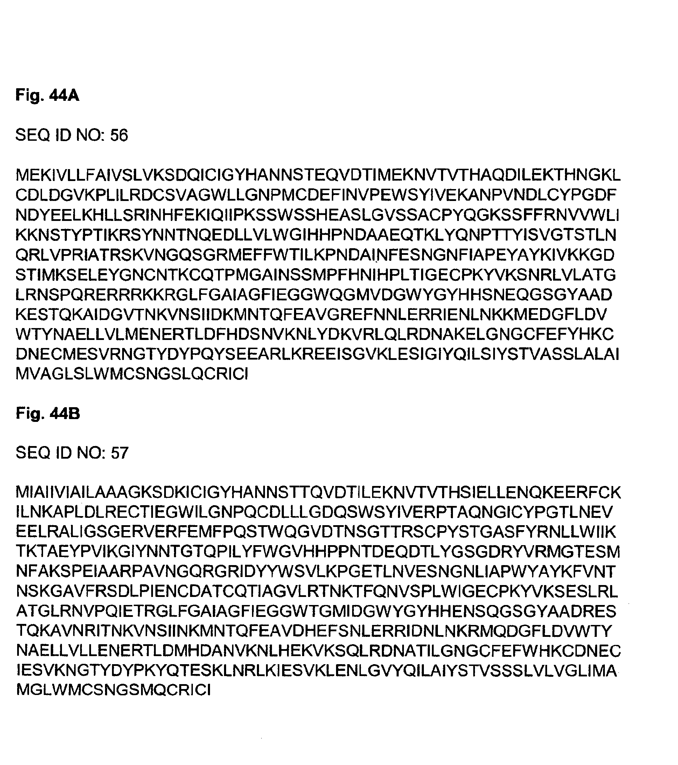

**FIGURE 44A** shows the amino acid sequence (SEQ ID NO: 56) of the polypeptide translated from clone 782 (A/Vietnam/1194/2004 (H5N1)). The open reading frame of clone 782 starts with the ATG indicated in Figure 36. Figure 44B shows the amino acid sequence (SEQ ID NO: 57) of the polypeptide translated from clone 783 (A/Teal/HongKong/W312/97 (H6N1)). The open reading frame of clone 783 starts with the ATG indicated in Figure 37.

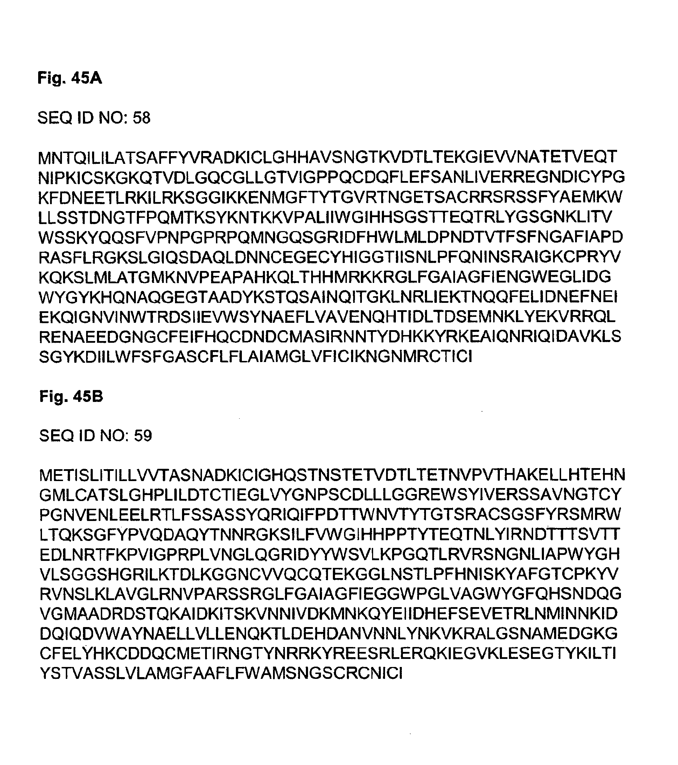

**FIGURE 45A** shows the amino acid sequence (SEQ ID NO: 58) of the polypeptide translated from clone 784 (A/Equine/Prague/56 (H7N7)). The open reading frame of clone 784 starts with the ATG indicated in Figure 38. Figure 45B shows the amino acid sequence (SEQ ID NO: 59) of the polypeptide translated from clone 785 (A/HongKong/1073/99 (H9N2)). The open reading frame of clone 785 starts with the ATG indicated in Figure 39.

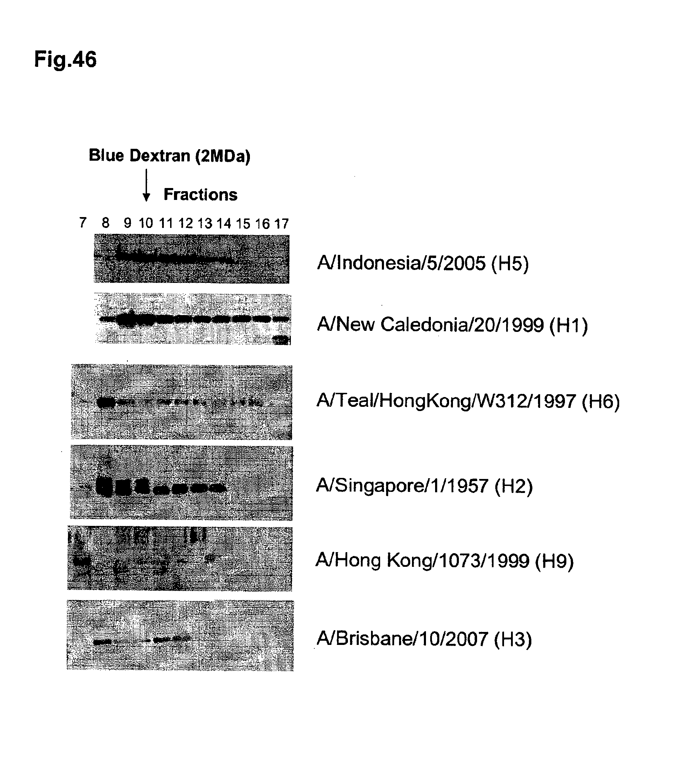

**FIGURE 46** shows immunodetection (western blot) of elution fractions 7-17 of plant-produced VLPs, following size exclusion chromatography. The elution peak (fraction 10) of BlueDextran is indicated by the arrow. Hemagglutinin subtypes H1, H2, H3, H5, H6 and H9 are shown. Hemagglutinin is detected in fractions 7-14, corresponding to the elution of VLPs.

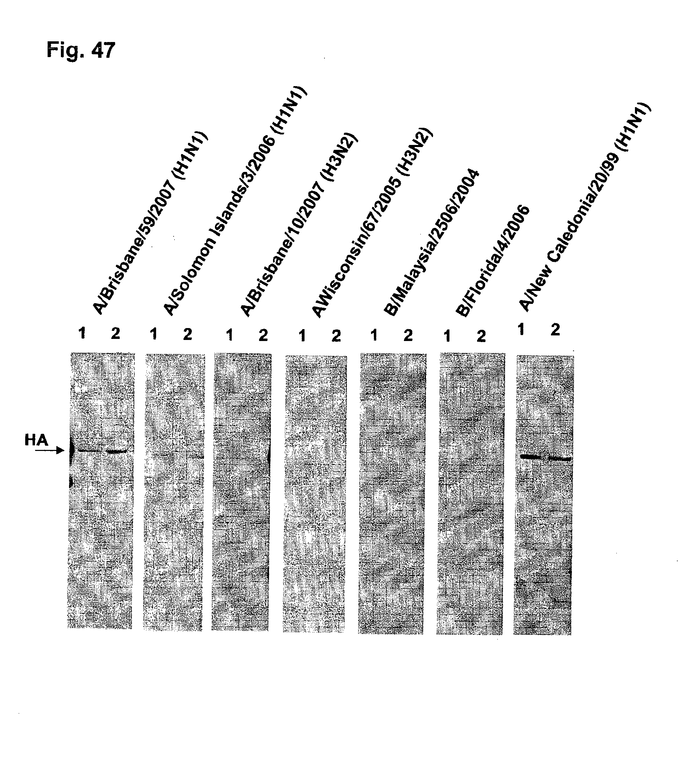

**FIGURE 47** shows an immunoblot analysis of expression of a series of hemagglutinin from annual epidemic strains. Ten and twenty micrograms of leaf protein extracts were loaded in lanes 1 and 2, respectively, for plants expressing HA from various influenza strains (indicated at the top of the immunoblots).

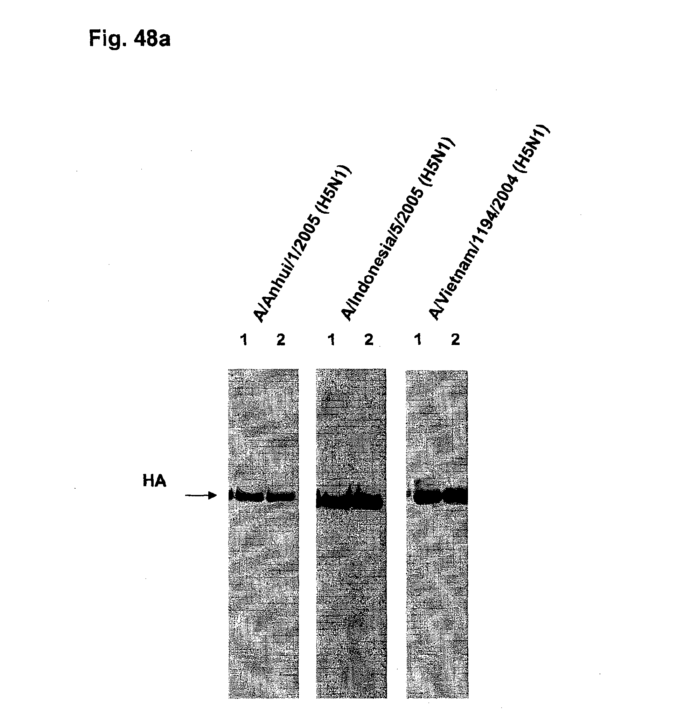

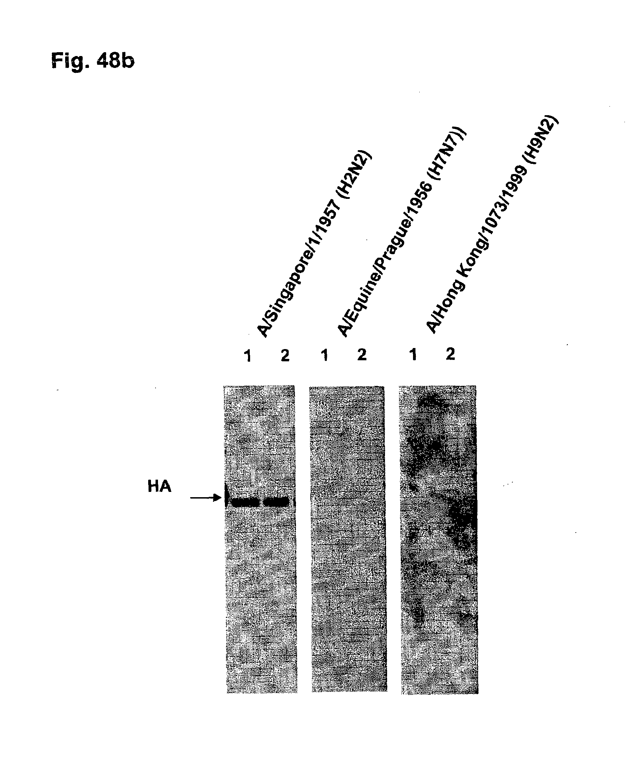

**FIGURE 48A** shows an immunoblot analysis of expression of a series of H5 hemagglutinins from potential pandemic strains. Ten and twenty micrograms of protein extracts were loaded in lanes 1 and 2, respectively. FIGURE 48B shows an immunoblot analysis of expression of H2, H7 and H9 hemagglutinin from selected influenza strains. Ten and twenty micrograms of protein extracts were loaded in lanes 1 and 2, respectively.

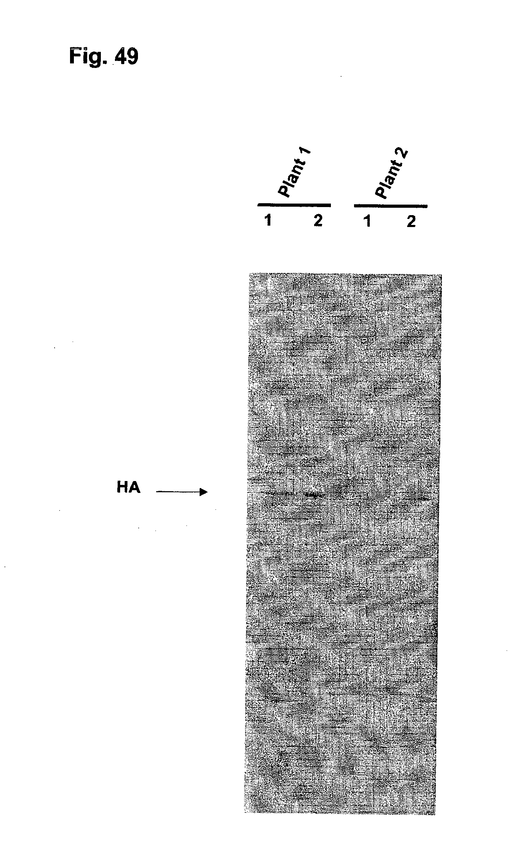

**FIGURE 49** shows an immunoblot of H5 from strain A/Indonesia/5/2005 in protein extracts from *Nicotiana tabacum* leaves, agroinfiltrated with AGL1/660. Two plants (plant 1 and plant 2) were infiltrated and 10 and 20 µg of soluble protein extracted from each plant were loaded in lanes 1 and 2, respectively.

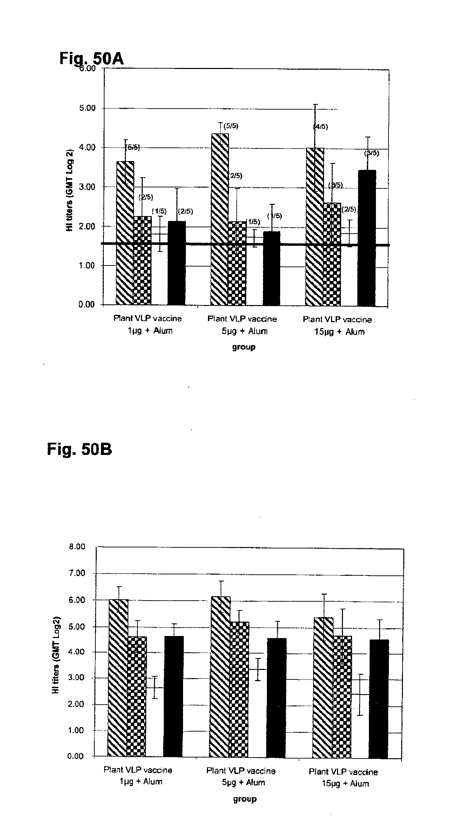

**FIGURE 50** shows the *in vitro* cross-reactivity of serum antibodies. Hemagglutination-inhibition (HI) titers in ferret sera, 14 days (A) after 1^{st} immunization and (B) after 2nd boost with plant-made influenza H5 VLP (A/Indonesia/5/2005 (H5N1)). HAI antibody responses were measured using the following inactivated whole H5N1 viruses: A/turkey/Turkey/1/05, A/Vietnam/1194/04, A/Anhui/5/05 and the homologous strain A/Indonesia/5/05. Values are the GMT (log₂) of reciprocal end-point titers of five ferrets per group.Diagonal stripe - A/Indonesia/6/06 (clade 2.1.3); checked - A/turkey/Turkey/1/05 (clade 2.2); white bar - A/Vietnam/1194/04 (clade 1); black bar A/Anhui/5/05. Responders are indicated. Bars represent *mean deviation.*

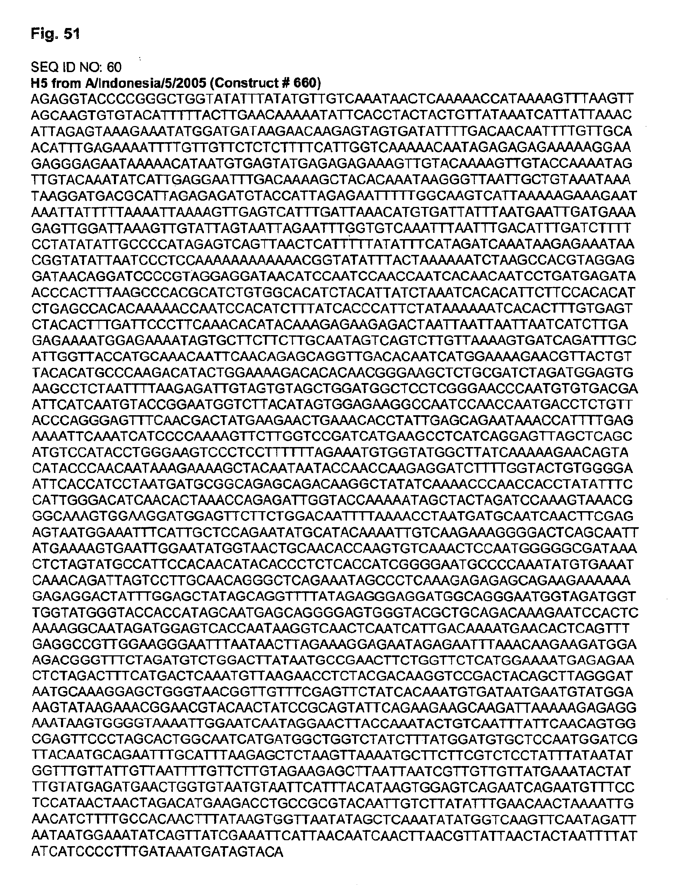

**FIGURE 51** shows the nucleic acid sequence (SEQ ID NO: 60) of an HA expression cassette comprising alfalfa plastocyanin promoter and 5' UTR, hemagglutinin coding sequence of H5 from A/Indonesia/5/2005 (Construct # 660), alfalfa plastocyanin 3' UTR and terminator sequences

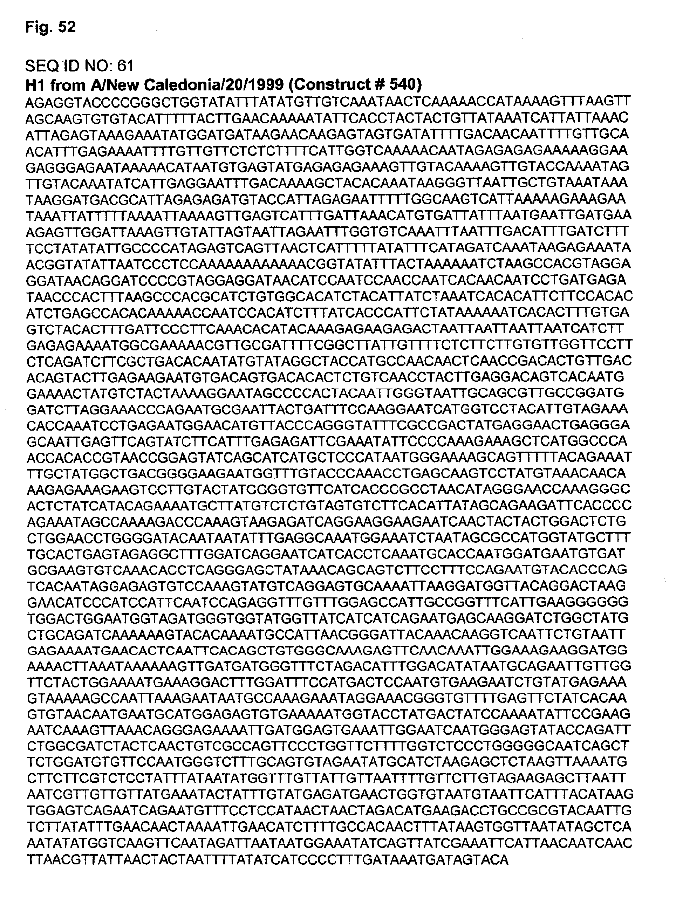

**FIGURE 52** shows the nucleic acid sequence (SEQ ID NO: 61) of an HA expression cassette comprising alfalfa plastocyanin promoter and 5' UTR, hemagglutinin coding sequence of H1 from A/New Caledonia/20/1999 (Construct # 540), alfalfa plastocyanin 3' UTR and terminator sequences

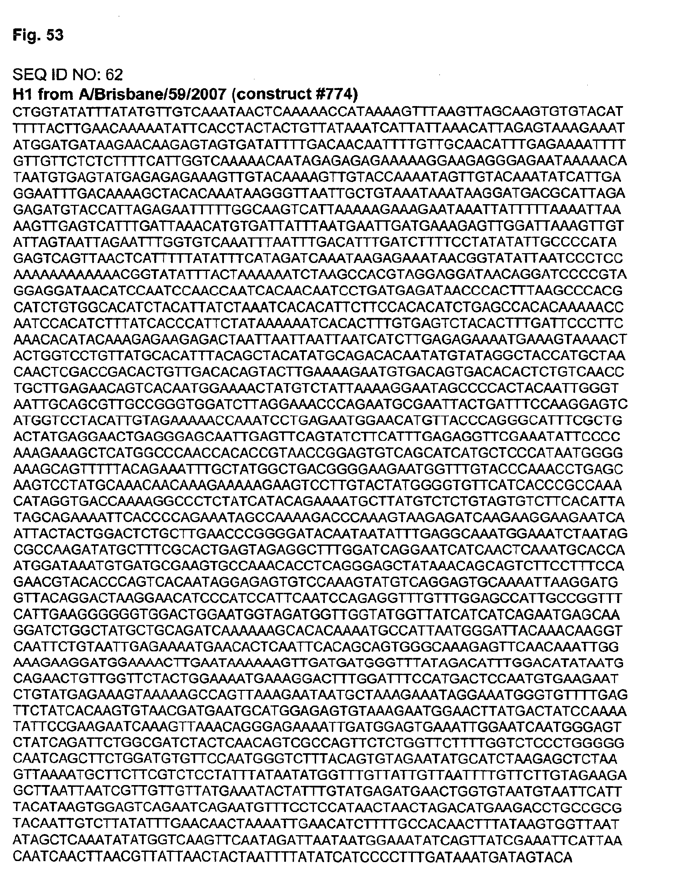

**FIGURE 53** shows the nucleic acid sequence (SEQ ID NO: 62) of an HA expression cassette comprising alfalfa plastocyanin promoter and 5' UTR, hemagglutinin coding sequence of H1 from A/Brisbane/59/2007 (construct #774), alfalfa plastocyanin 3' UTR and terminator sequences.

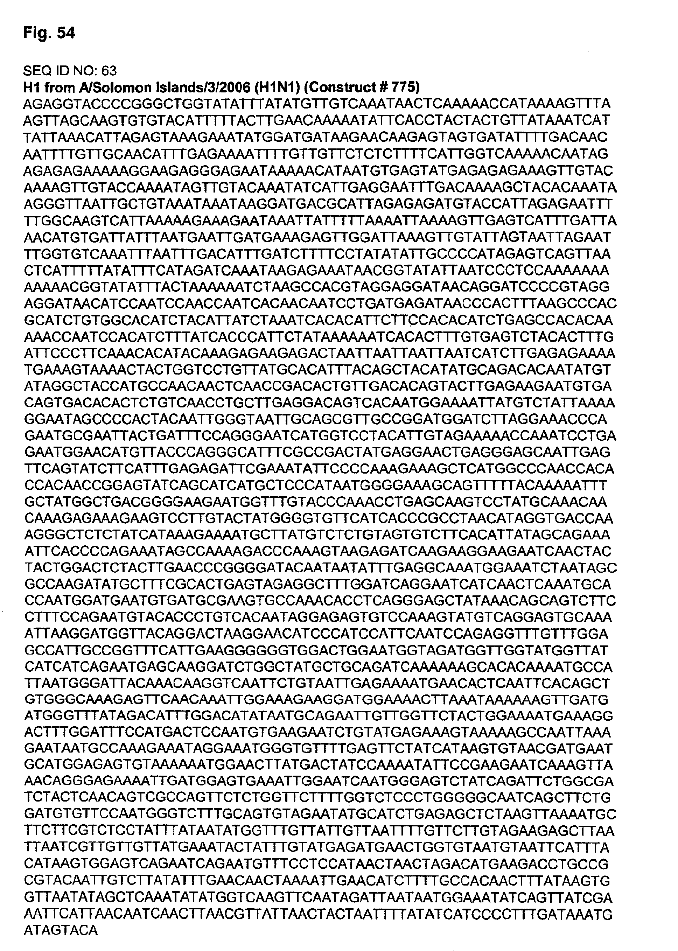

**FIGURE 54** shows the nucleic acid sequence (SEQ ID NO: 63) of an HA expression cassette comprising alfalfa plastocyanin promoter and 5' UTR, hemagglutinin coding sequence of H1 from A/Solomon Islands/3/2006 (H1N1) (construct #775), alfalfa plastocyanin 3' UTR and terminator sequences.

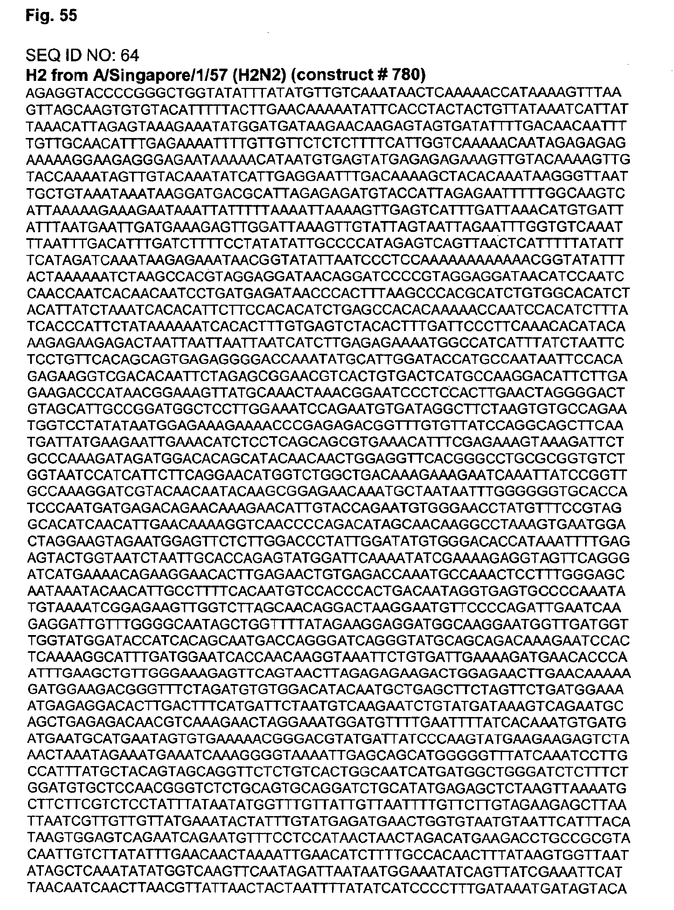

**FIGURE 55** shows the nucleic acid sequence (SEQ ID NO: 64)of an HA expression cassette comprising alfalfa plastocyanin promoter and 5' UTR, hemagglutinin coding sequence of H2 from A/Singapore/1/57 (H2N2) (construct # 780), alfalfa plastocyanin 3' UTR and terminator sequences.

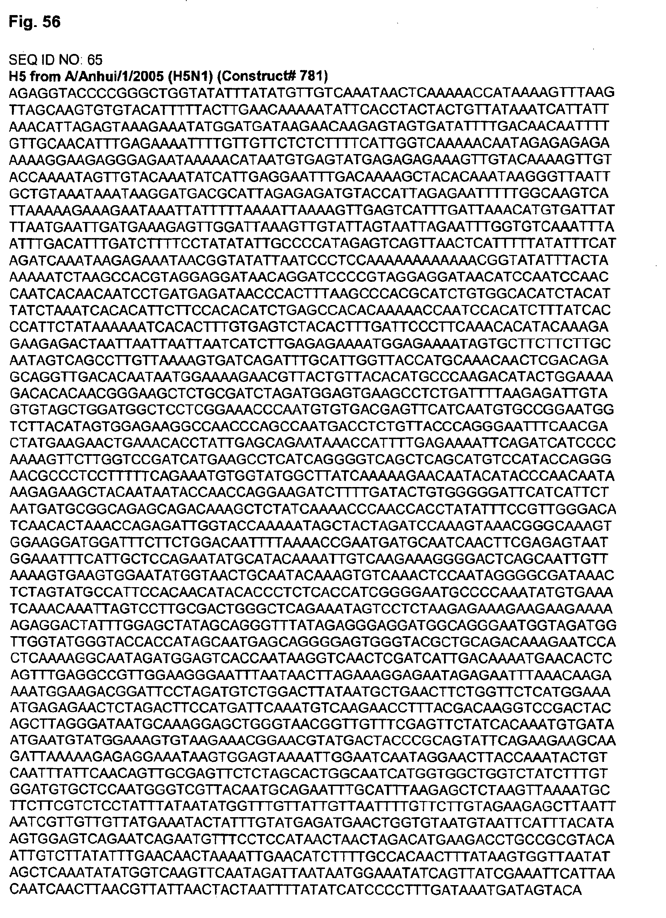

**FIGURE 56** shows the nucleic acid sequence (SEQ ID NO: 65) of an HA expression cassette comprising alfalfa plastocyanin promoter and 5' UTR, hemagglutinin coding sequence of H5 from A/Anhui/1/2005 (H5N1) (Construct# 781), alfalfa plastocyanin 3' UTR and terminator sequences.

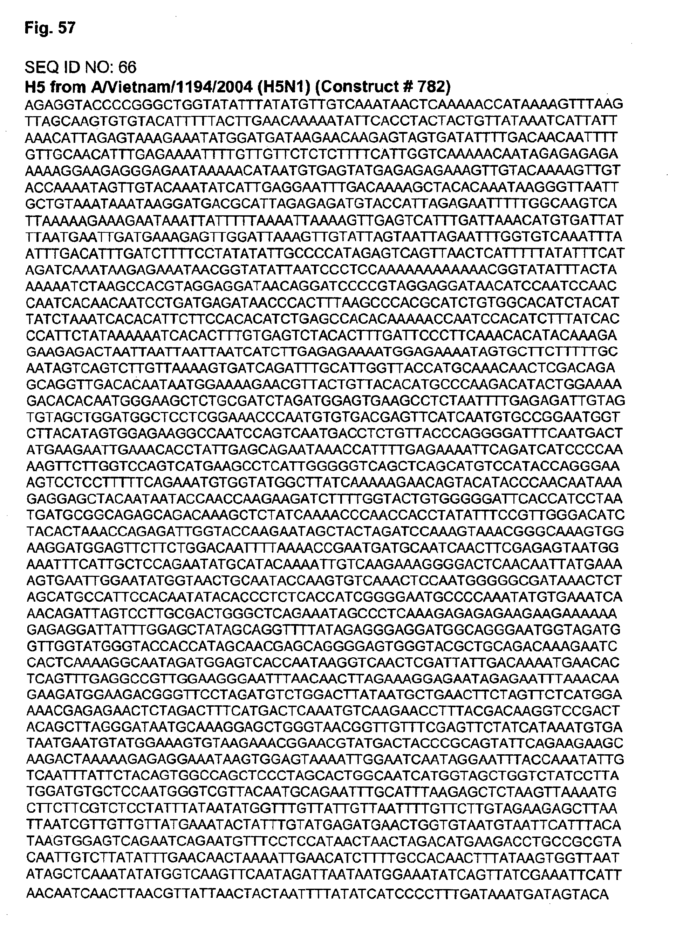

**FIGURE 57** shows the nucleic acid sequence (SEQ ID NO: 66) of an HA expression cassette comprising alfalfa plastocyanin promoter and 5' UTR, hemagglutinin coding sequence of H5 from A/Vietnam/1194/2004 (H5N1) (Construct # 782), alfalfa plastocyanin 3' UTR and terminator sequences.

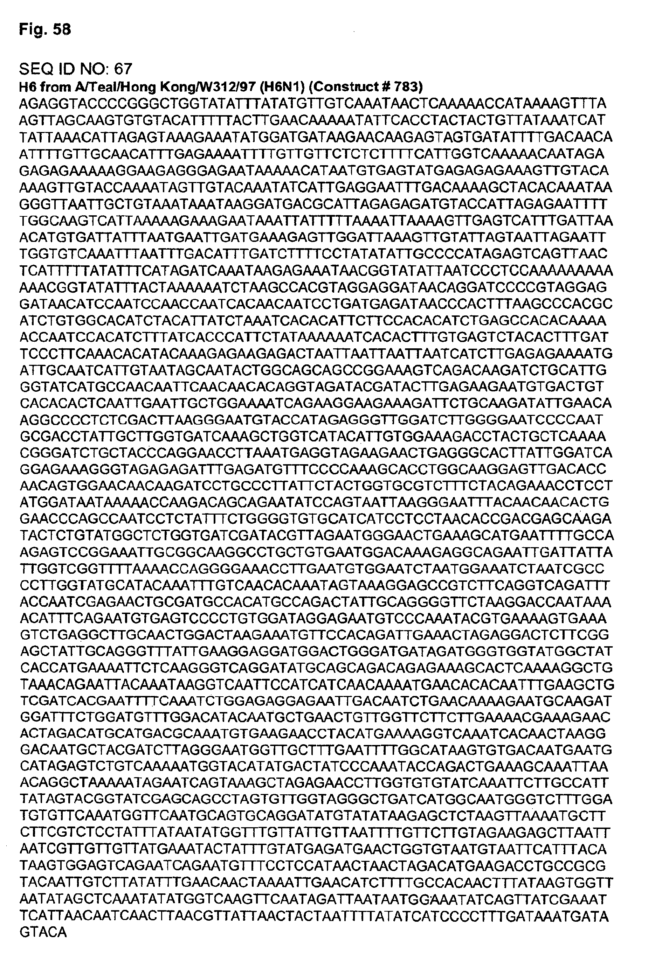

**FIGURE 58** shows the nucleic acid sequence (SEQ ID NO: 67) of an HA expression cassette comprising alfalfa plastocyanin promoter and 5' UTR, hemagglutinin coding sequence of H6 from A/Teal/Hong Kong/W312/97 (H6N1) (Construct # 783), alfalfa plastocyanin 3' UTR and terminator sequences.

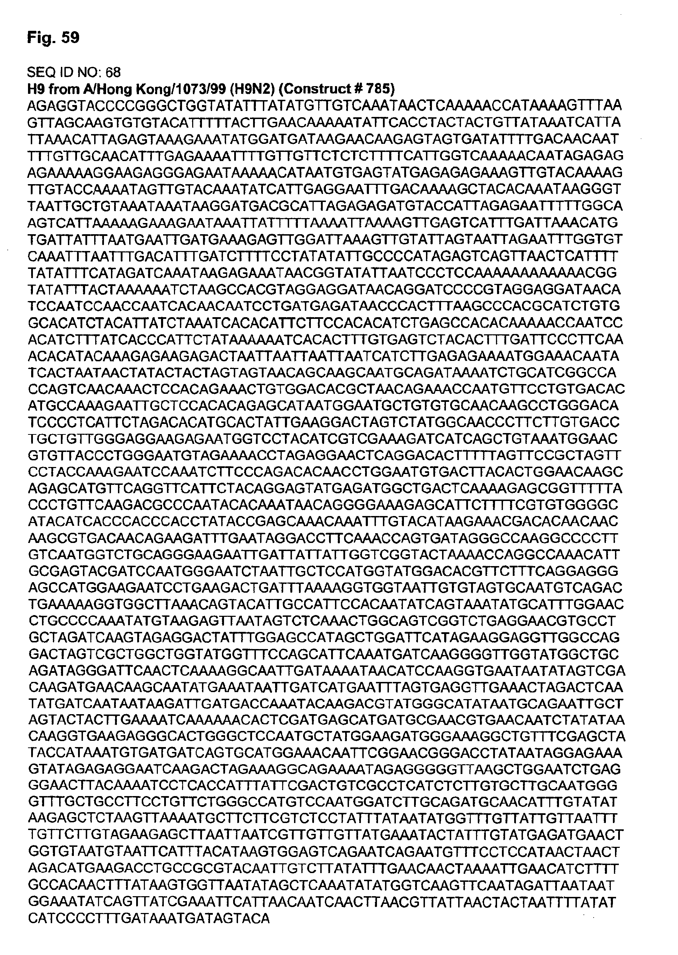

**FIGURE 59** shows the nucleic acid sequence (SEQ ID NO: 68) of an HA expression cassette comprising alfalfa plastocyanin promoter and 5' UTR, hemagglutinin coding sequence of H9 from A/Hong Kong/1073/99 (H9N2) (Construct # 785), alfalfa plastocyanin 3' UTR and terminator sequences.

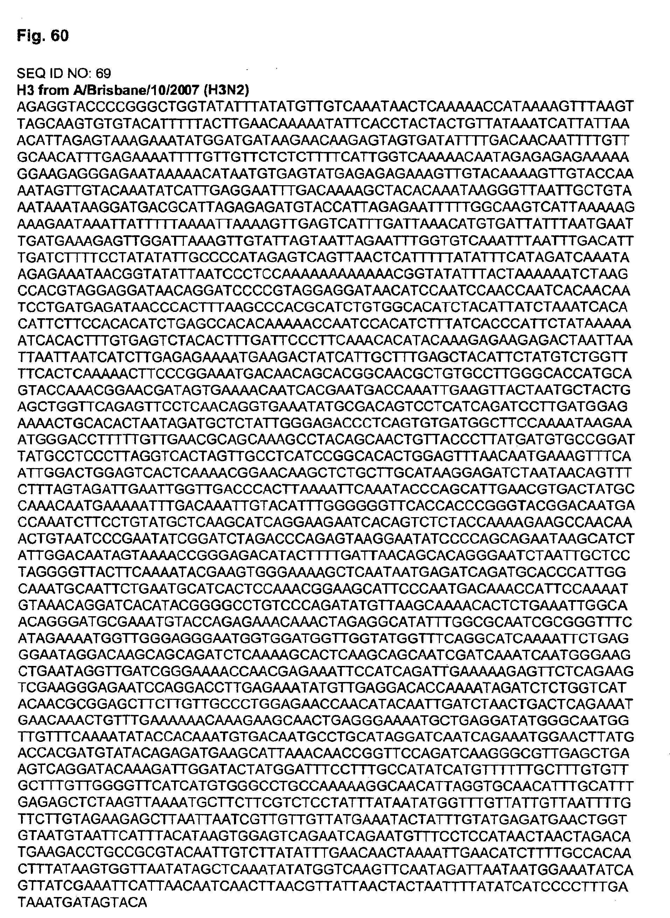

**FIGURE 60** shows the nucleic acid sequence (SEQ ID NO: 69) of an HA expression cassette comprising alfalfa plastocyanin promoter and 5' UTR, hemagglutinin coding sequence of H3 from A/Brisbane/10/2007 (H3N2), alfalfa plastocyanin 3' UTR and terminator sequences.

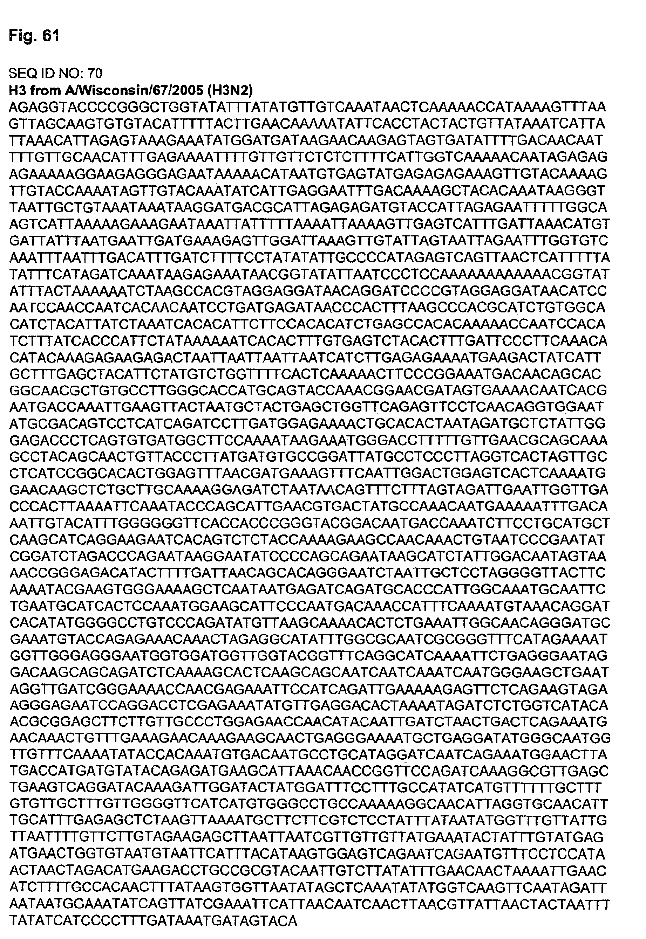

**FIGURE 61** shows the nucleic acid sequence (SEQ ID NO: 70) of an HA expression cassette comprising alfalfa plastocyanin promoter and 5' UTR, hemagglutinin coding sequence of H3 from A/Wisconsin/67/2005 (H3N2), alfalfa plastocyanin 3' UTR and terminator sequences.

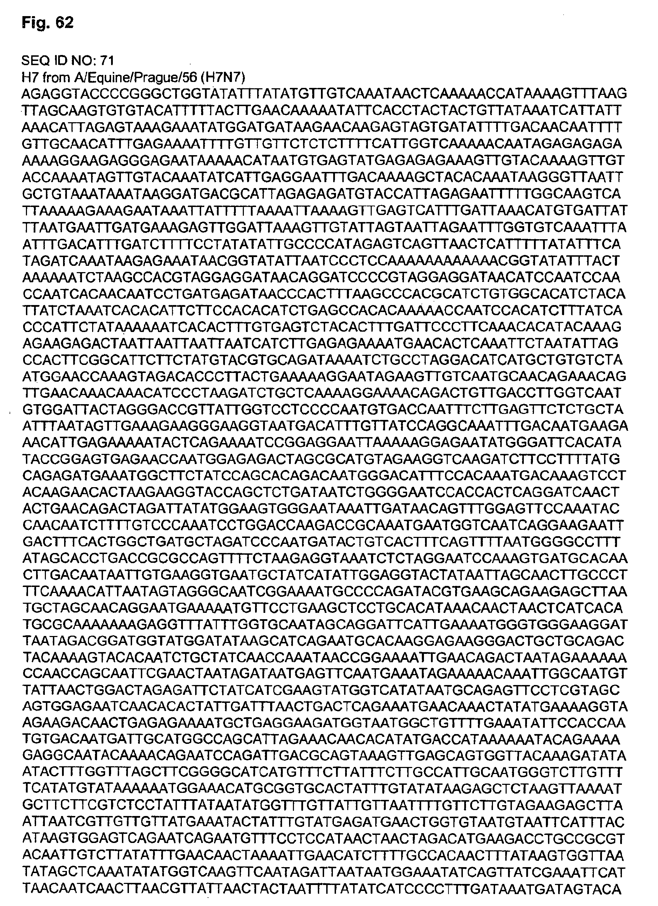

**FIGURE 62** shows the nucleic acid sequence (SEQ ID NO: 71) of an HA expression cassette comprising alfalfa plastocyanin promoter and 5' UTR, hemagglutinin coding sequence of H7 from A/Equine/Prague/56 (H7N7), alfalfa plastocyanin 3' UTR and terminator sequences.

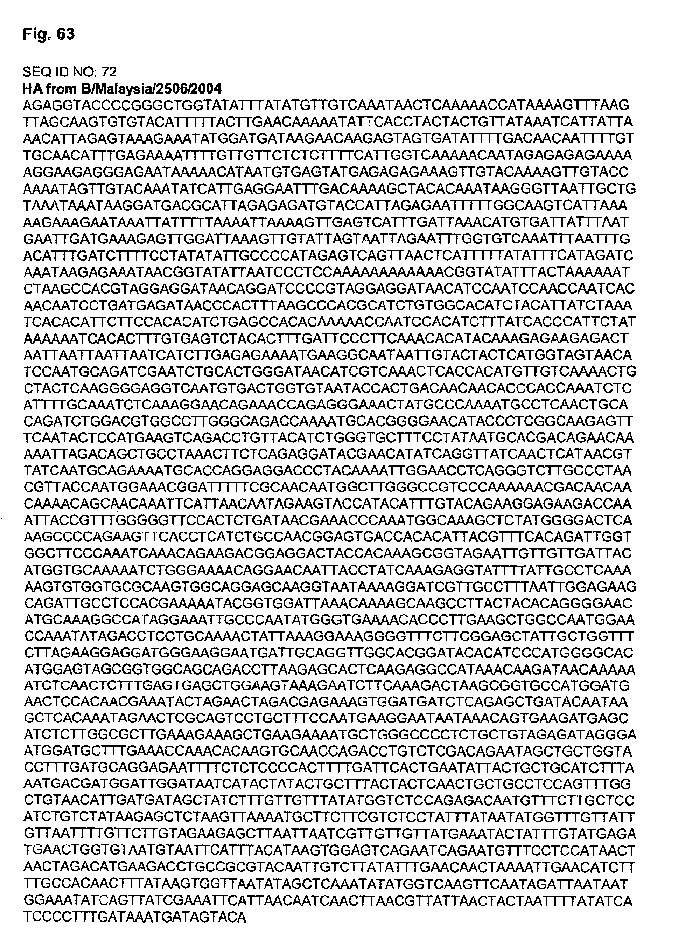

**FIGURE 63** shows the nucleic acid sequence (SEQ ID NO: 72) of an HA expression cassette comprising alfalfa plastocyanin promoter and 5' UTR, hemagglutinin coding sequence of HA from B/Malaysia/2506/2004, alfalfa plastocyanin 3' UTR and terminator sequences.

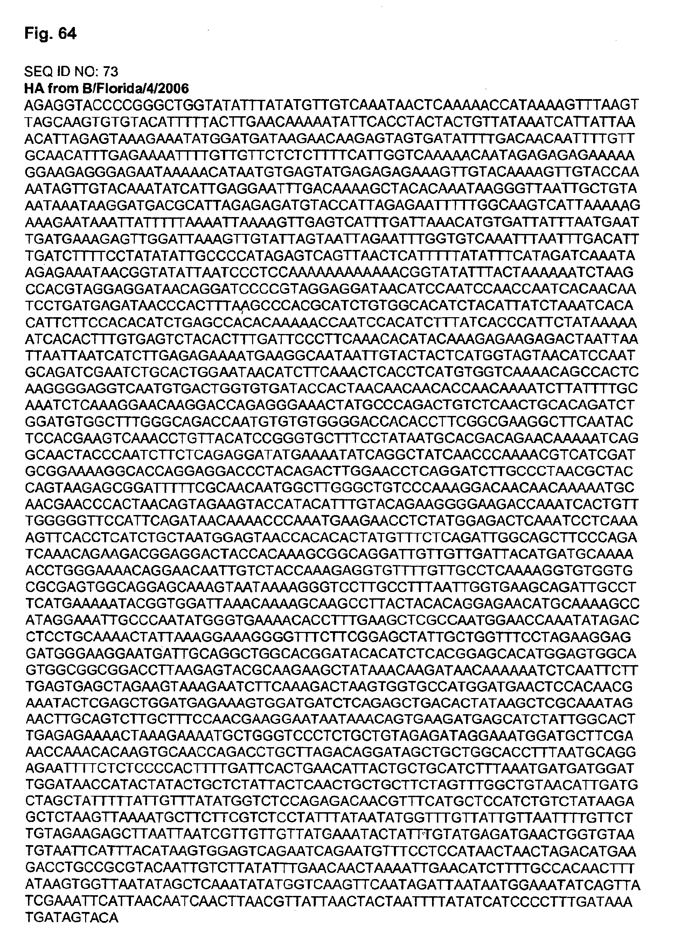

**FIGURE 64** shows the nucleic acid sequence (SEQ ID NO: 73) of an HA expression cassette comprising alfalfa plastocyanin promoter and 5' UTR, hemagglutinin coding sequence of HA from B/Florida/4/2006, alfalfa plastocyanin 3' UTR and terminator sequences.

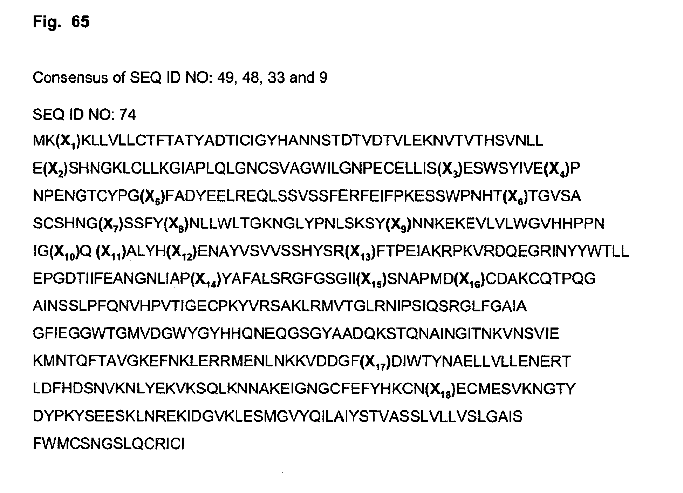

**FIGURE 65** shows a consensus amino acid sequence (SEQ ID NO: 74) for HA of A/New Caledonia/20/99 (H1N1) (encoded by SEQ ID NO: 33), A/Brisbane/59/2007 (H1N1) (SEQ ID NO: 48), A/Solomon Islands/3/2006 (H1N1) (SEQ ID NO: 49) and SEQ ID NO: 9. X1 (position 3) is A or V; X2 (position 52) is D or N; X3 (position 90) is K or R; X4 (position 99) is K or T; X5 (position 111) is Y or H; X6 (position 145) is V or T; X7 (position 154) is E or K; X8 (position 161) is R or K; X9 (position 181) is V or A; X10 (position 203) is D or N; X11 (position 205) is R or K; X12 (position 210) is T or K; X13 (position 225) is R or K; X14 (position 268) is W or R; X15 (position 283) is T or N; X16 (position 290) is E or K; X17 (position 432) is I or L; X18 (position 489) is N or D.

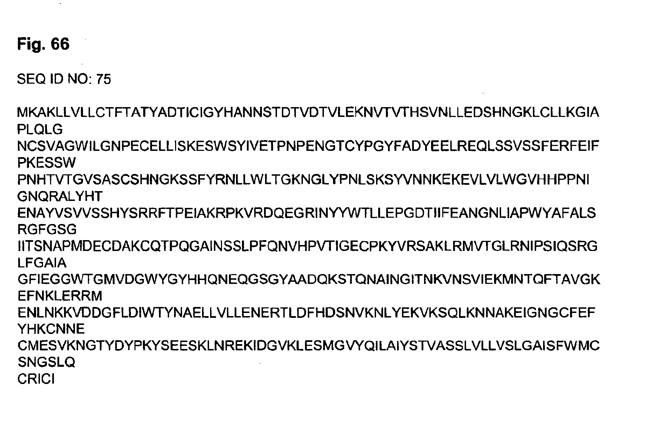

**FIGURE 66** shows amino acid sequence (SEQ ID NO: 75) of H1 New Caledonia (AAP34324.1) encoded by SEQ ID NO: 33.

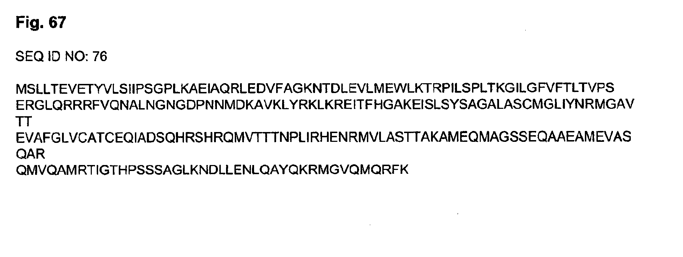

**FIGURE 67** shows the amino acid sequence (SEQ ID NO: 76) of H1 Puerto Rico (NC_0409878.1) encoded by SEQ ID NO: 35

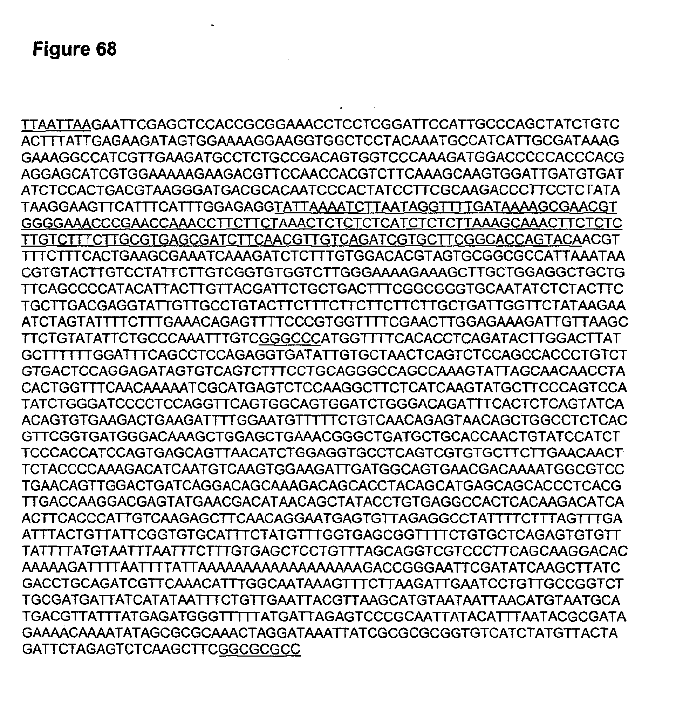

**FIGURE 68** shows the nucleic acid sequence of a portion of expression cassette number 828, from PacI (upstream promoter) to AscI (immediately downstream NOS terminator). CPMV HT 5'UTR sequence underlined with mutated ATG. ApaI restriction site (immediately upstream of ATG of protein coding sequence to be express, in this case C5-1 kappa light chain.)

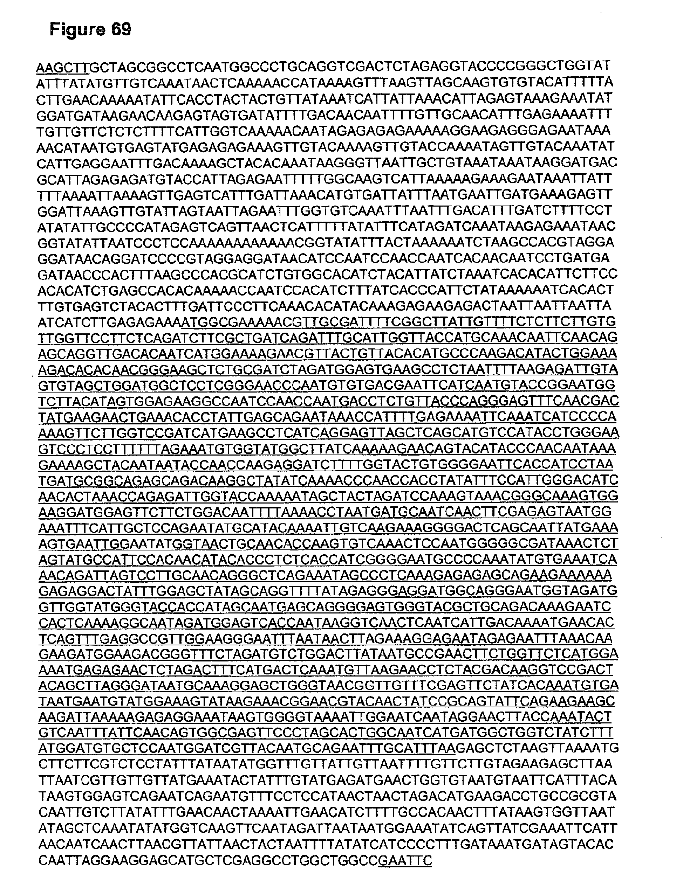

**FIGURE 69** shows the nucleic acid sequence of a portion of construct number 663, from HindIII (in the multiple cloning site, upstream of the plastocyanin promoter) to EcoRI (immediately downstream of the plastocyanin terminator). H5 (from A/Indonesia/5/2005) coding sequence in fusion with PDI SP is underlined.

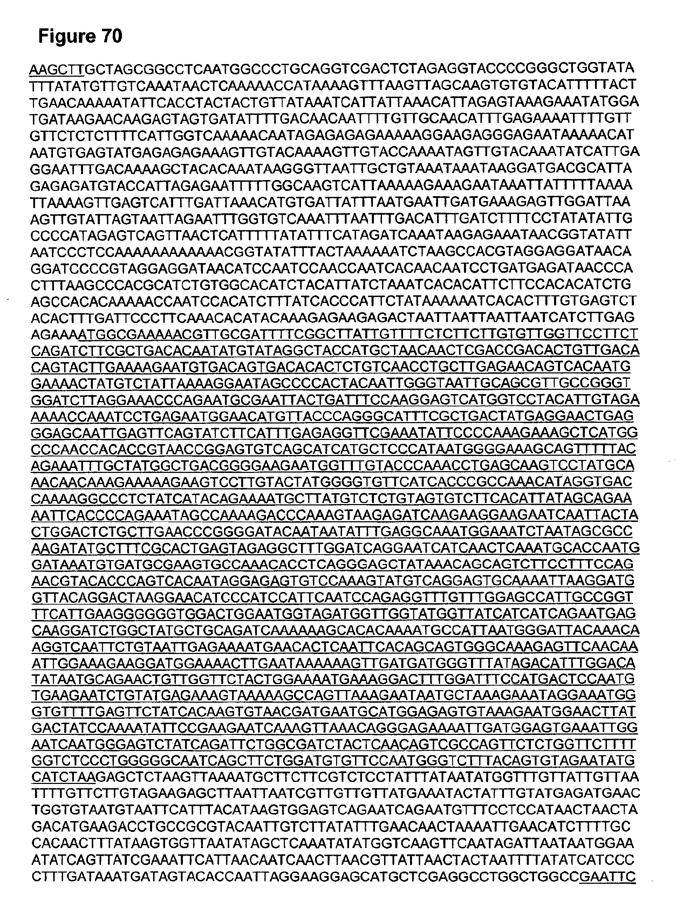

**FIGURE 70** shows the nucleic acid sequence of a portion of construct number 787, from HindIII (in the multiple cloning site, upstream of the plastocyanin promoter) to EcoRI (immediately downstream of the plastocyanin terminator). H1 (from A/Brisbane/59/2007) coding sequence in fusion with PDI SP is underlined.

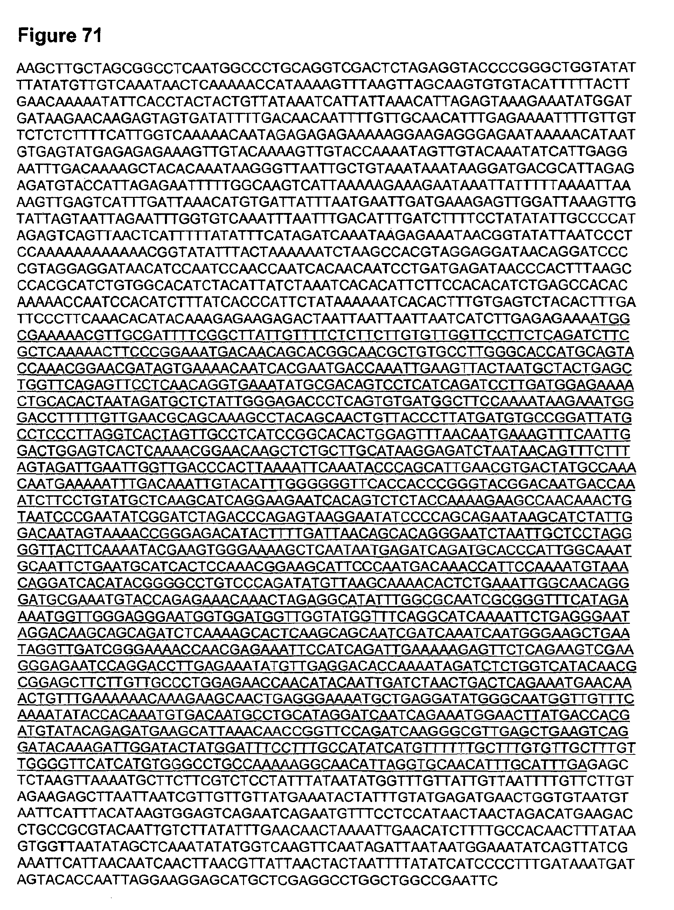

**FIGURE 71** shows the nucleic acid sequence of a portion of construct number 790, from HindIII (in the multiple cloning site, upstream of the plastocyanin promoter) to EcoRI (immediately downstream of the plastocyanin terminator). H3 (from ABrisbane/10/2007) coding sequence in fusion with PDI SP is underlined.

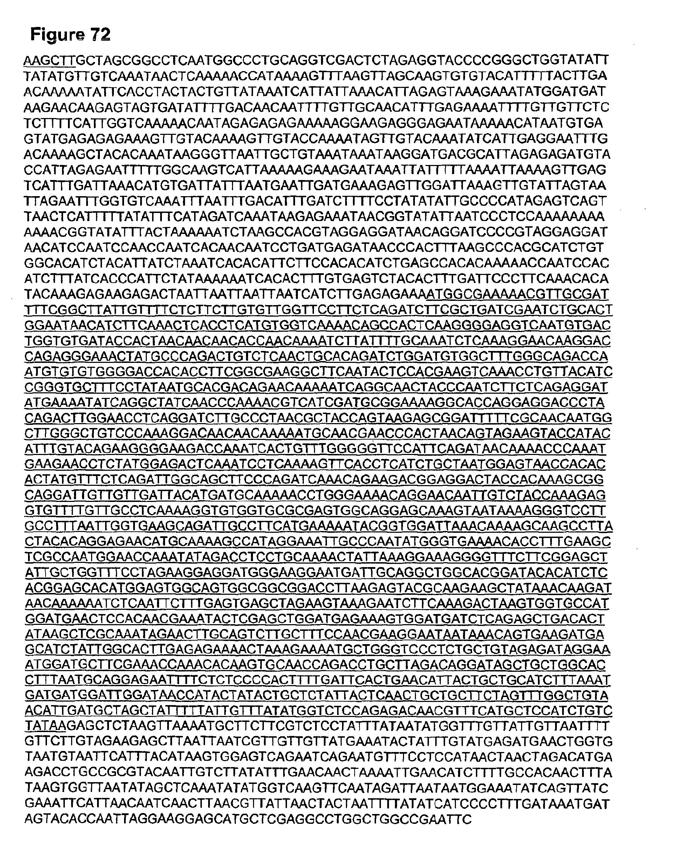

**FIGURE 72** shows the nucleic acid sequence of a portion of construct number 798, from HindIII (in the multiple cloning site, upstream of the plastocyanin promoter) to EcoRI (immediately downstream of the plastocyanin terminator). HA from B/Florida/4/2006 coding sequence in fusion with PDI SP is underlined.

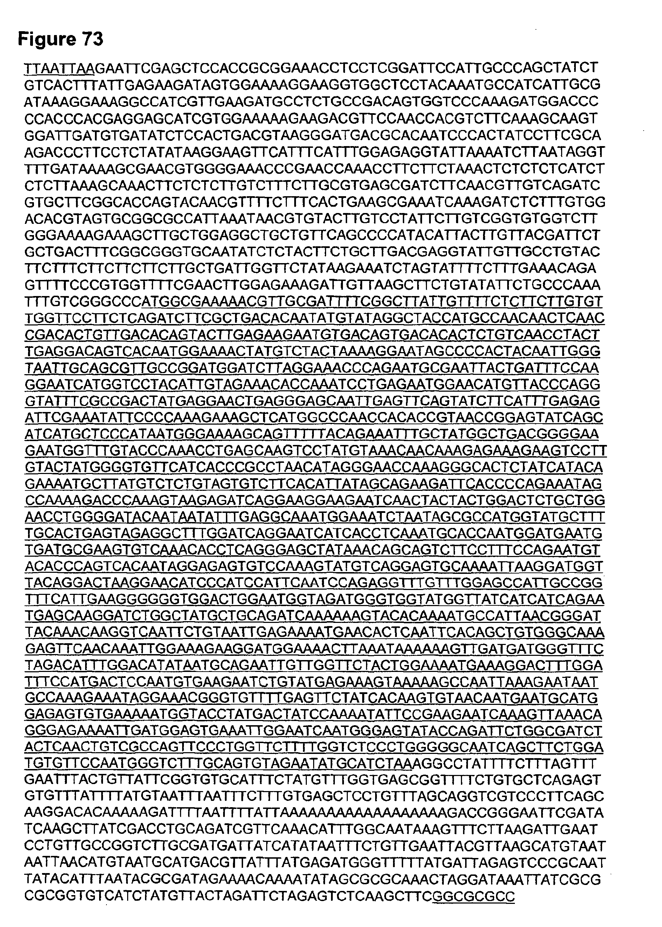

**FIGURE 73** shows the nucleic acid sequence of a portion of construct number 580, from PacI (upstream of the 35S promoter) to AscI (immediately downstream of the NOS terminator). Coding sequence of H1 (from A/New Caledonia/20/1999) in fusion with PDI SP is underlined.

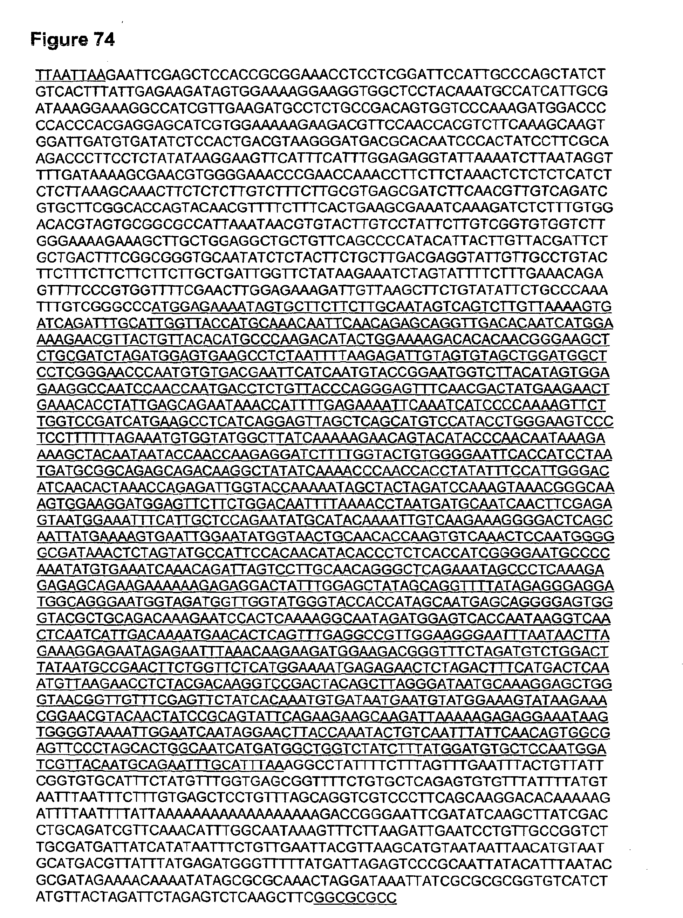

**FIGURE 74** shows the nucleic acid sequence of a portion of construct number 685, from PacI (upstream of the 35S promoter) to AscI (immediately downstream of the NOS terminator). Coding sequence of H5 from A/Indonesia/5/2005 is underlined.

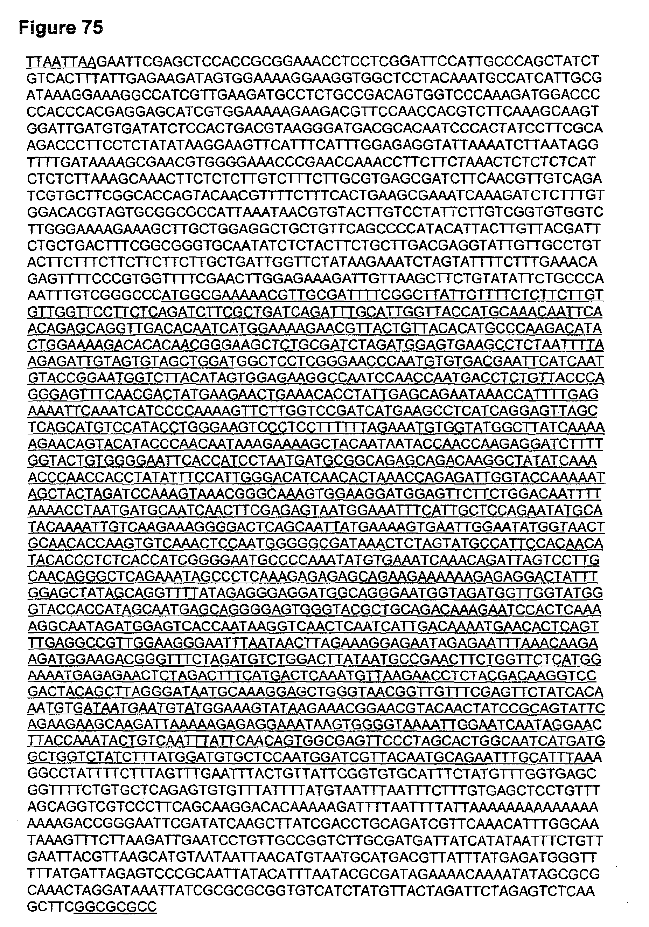

**FIGURE 75** shows the nucleic acid sequence of a portion of construct number 686, from PacI (upstream of the 35S promoter) to AscI (immediately downstream of the NOS terminator). Coding sequence of H5 from A/Indonesia/5/2005 in fusion with PDI SP is underlined.

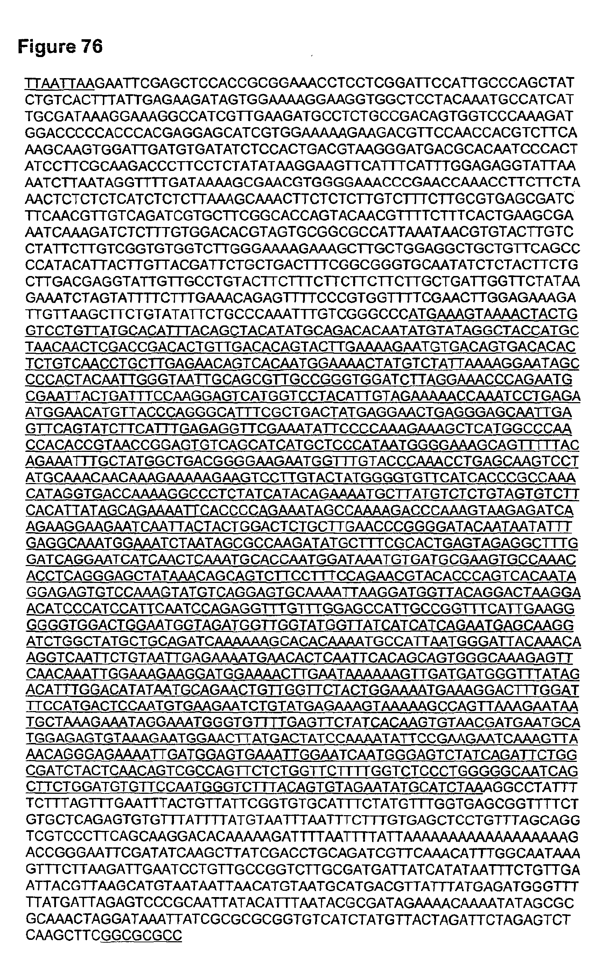

**FIGURE 76** shows the nucleic acid sequence of a portion of construct number 732, from PacI (upstream of the 35S promoter) to AscI (immediately downstream of the NOS terminator). Coding sequence of H1 from A/Brisbane/59/2007 is underlined.

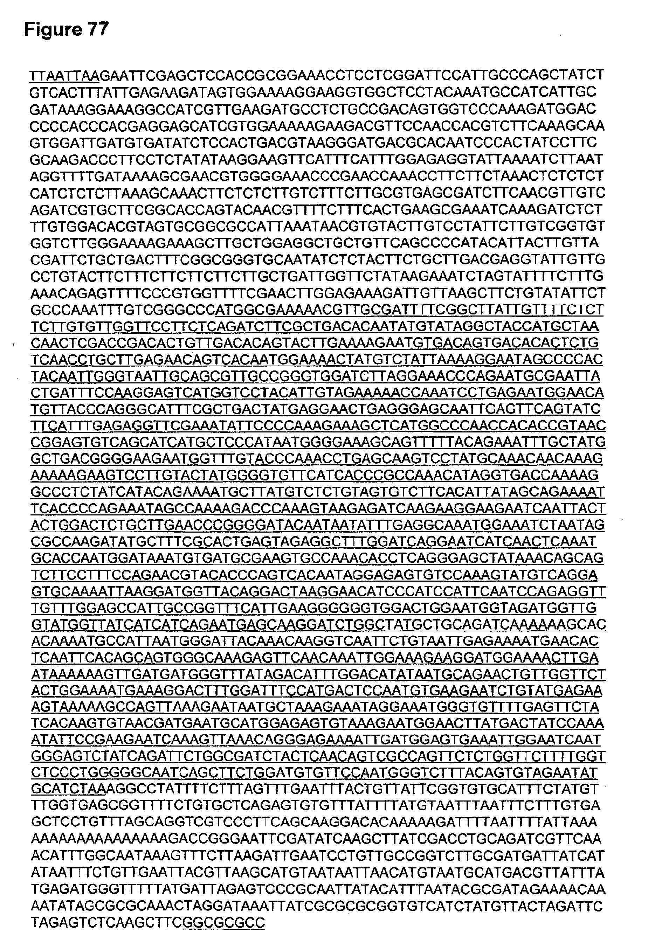

**FIGURE 77** shows the nucleic acid sequence of a portion of construct number 733, from PacI (upstream of the 35S promoter) to AscI (immediately downstream of the NOS terminator). Coding sequence of H1 from A/Brisbane/59/2007 in fusion with PDI SP is underlined.

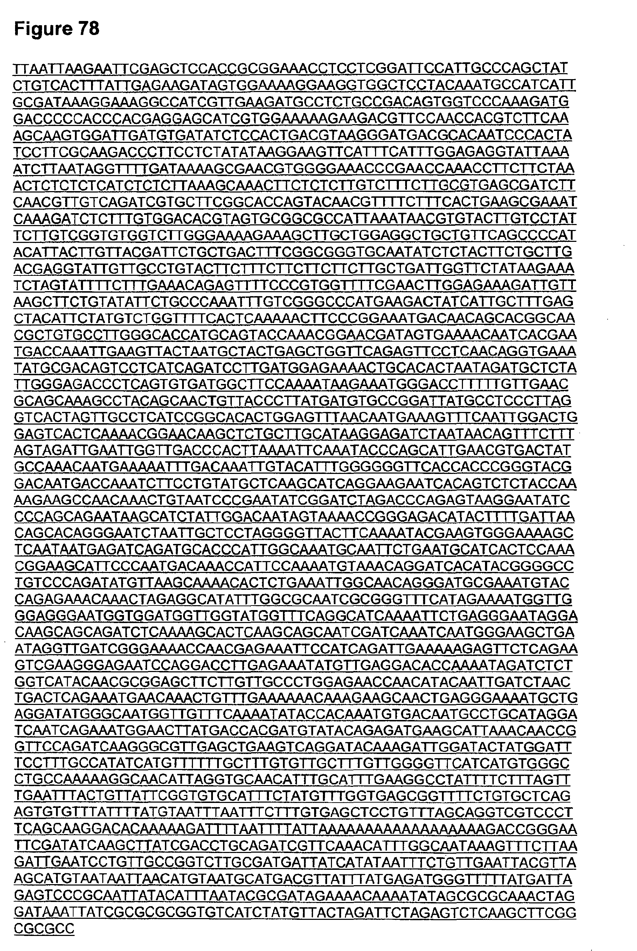

**FIGURE 78** shows the nucleic acid sequence of a portion of construct number 735, from PacI (upstream of the 35S promoter) to AscI (immediately downstream of the NOS terminator). Coding sequence of H3 from A/Brisbane/10/2007 is underlined.

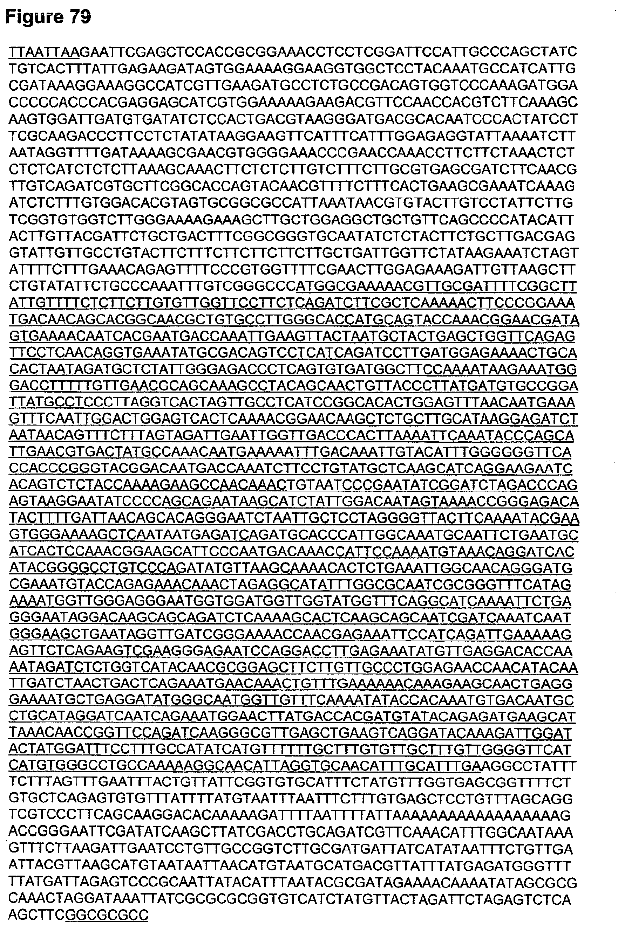

**FIGURE 79** shows the nucleic acid sequence of a portion of construct number 736, from PacI (upstream of the 35S promoter) to AscI (immediately downstream of the NOS terminator). Coding sequence of H3 from A/BUsbane/10/2007 in fusion with PDI SP is underlined

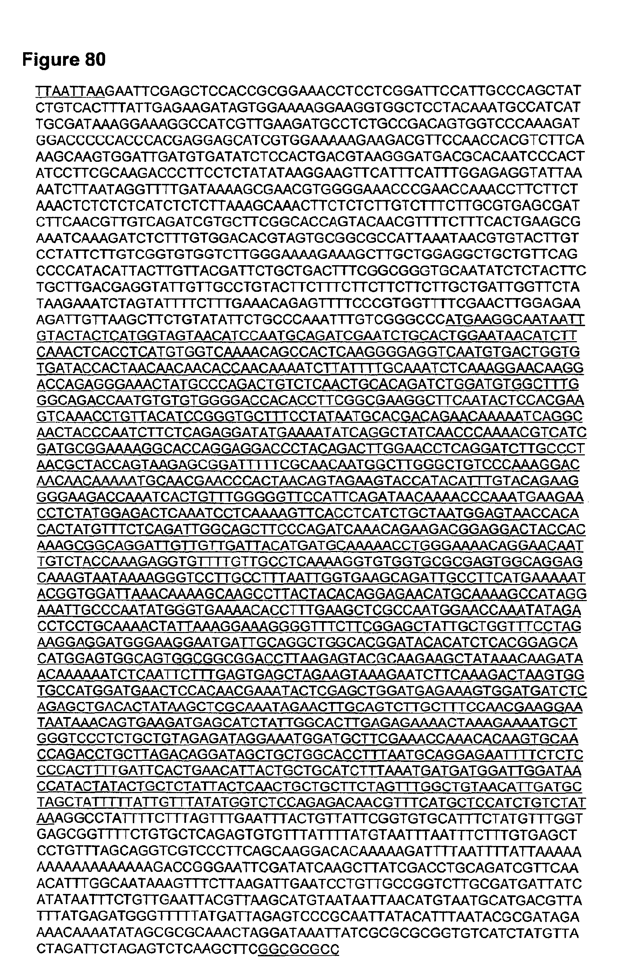

**FIGURE 80** shows the nucleic acid sequence of a portion of construct number 738, from PacI (upstream of the 35S promoter) to AscI (immediately downstream of the NOS terminator). Coding sequence of HA from B/Florida/4/2006 is underlined.

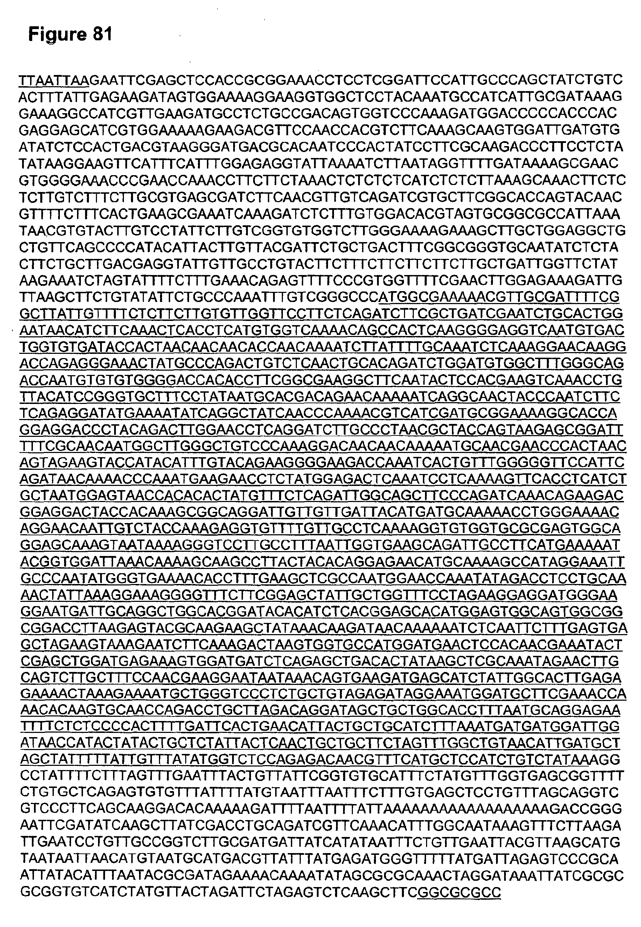

**FIGURE 81** shows the nucleic acid sequence of a portion of construct number 739, from PacI (upstream of the 35S promoter) to AscI (immediately downstream of the NOS terminator). Coding sequence of HA from B/Florida/4/2006 in fusion with PDI SP is underlined.

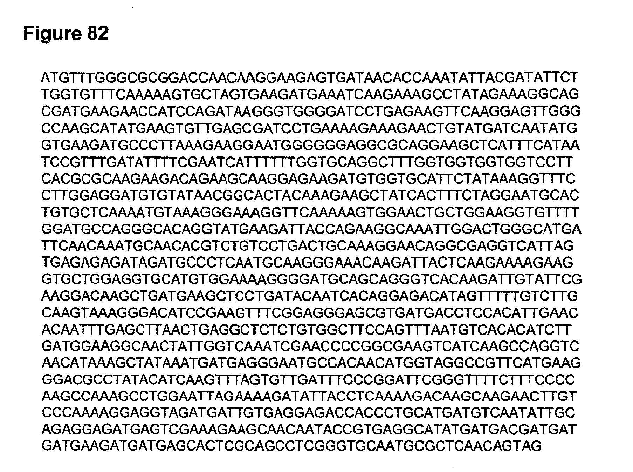

**FIGURE 82** shows a nucleic acid sequence encoding Msj1 (SEQ ID NO: 114).

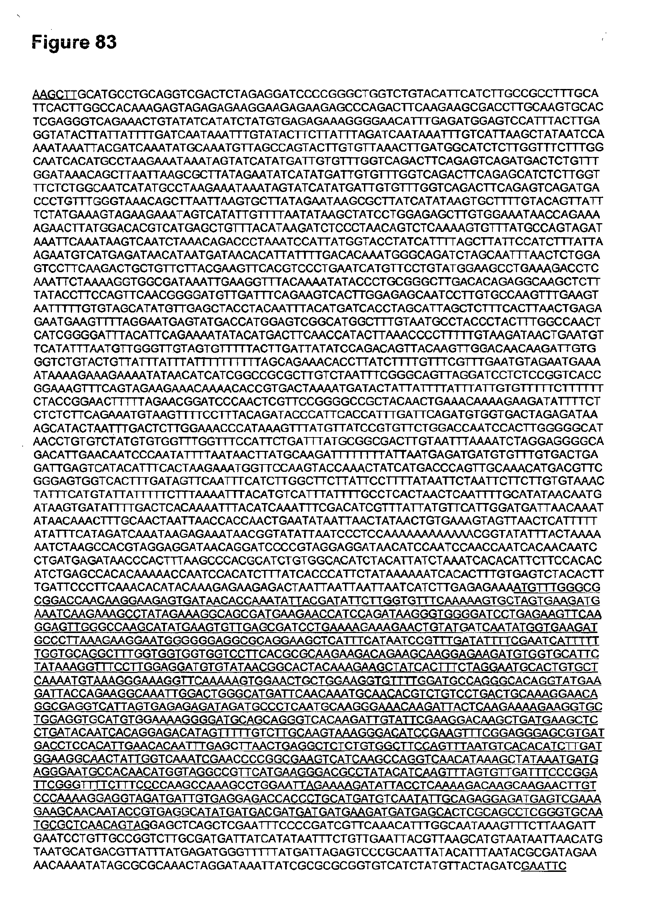

**FIGURE 83** shows the nucleic acid sequence of a portion of construct number R850, from HindIII (in the multiple cloning site, upstream of the promoter) to EcoRI (immediately downstream of the NOS terminator). HSP40 coding sequence is underlined.

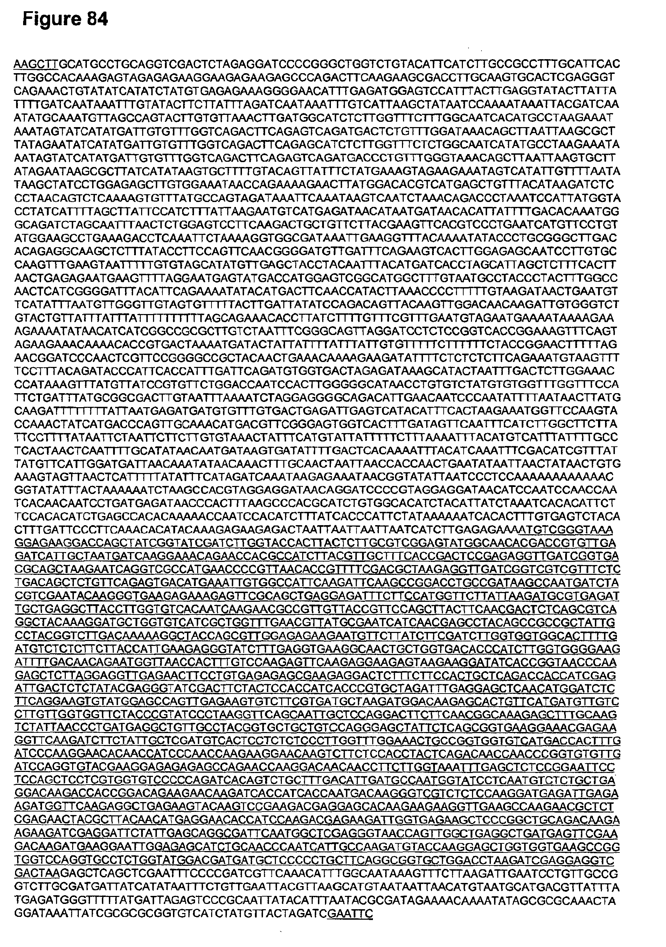

**FIGURE 84** shows the nucleic acid sequence of a portion of construct number R860, from HindIII (in the multiple cloning site, upstream of the promoter) to EcoRI (immediately downstream of the NOS terminator). HSP70 coding sequence is underlined.

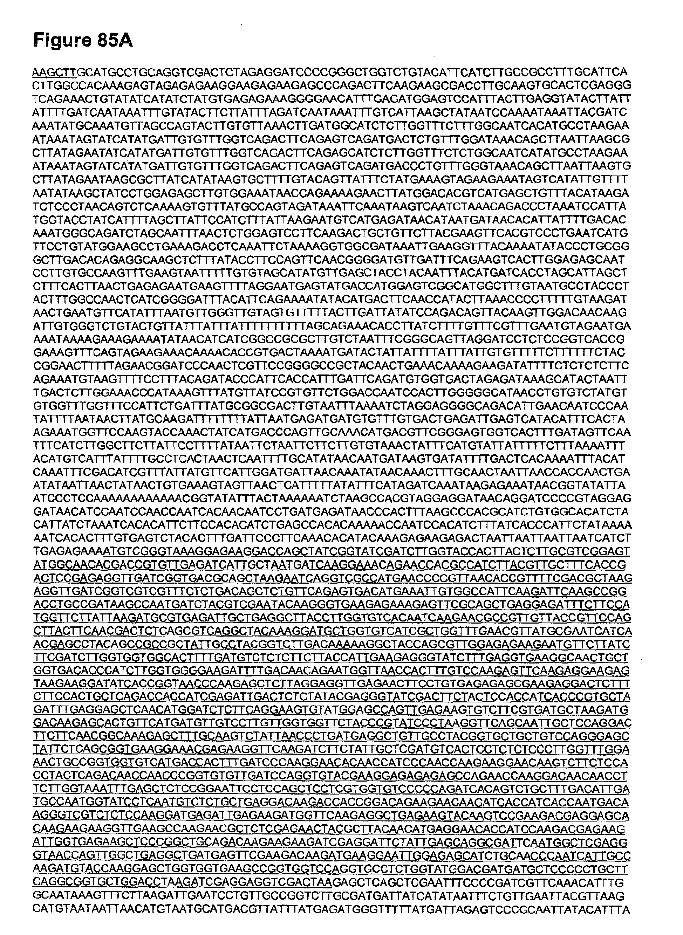

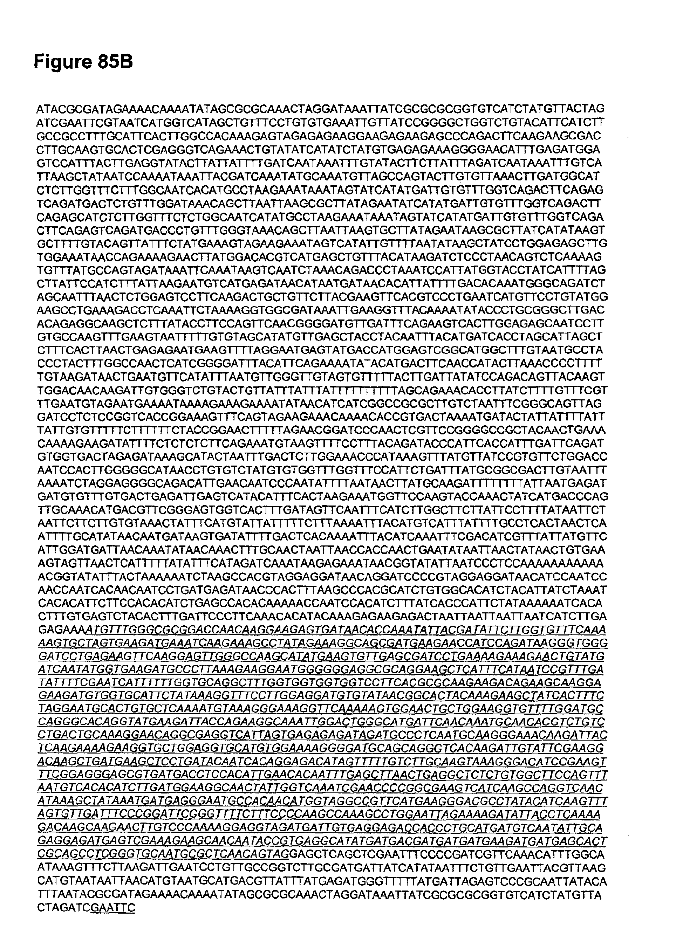

**FIGURE 85** shows the nucleic acid sequence of a portion of construct number R870, from HindIII (in the multiple cloning site, upstream of the promoter) to EcoRI (immediately downstream of the NOS terminator). HSP40 coding sequence is in underlined italic and HSP70 coding sequence is underlined. A) nucleotides 1-5003; B) nucleotides 5004-9493.

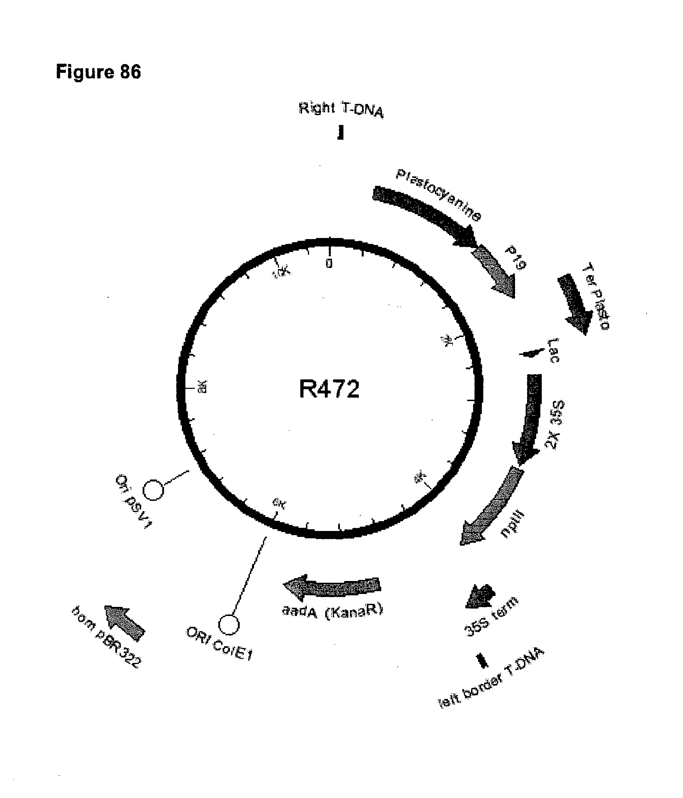

**FIGURE 86** shows a schematic representation of construct R472.

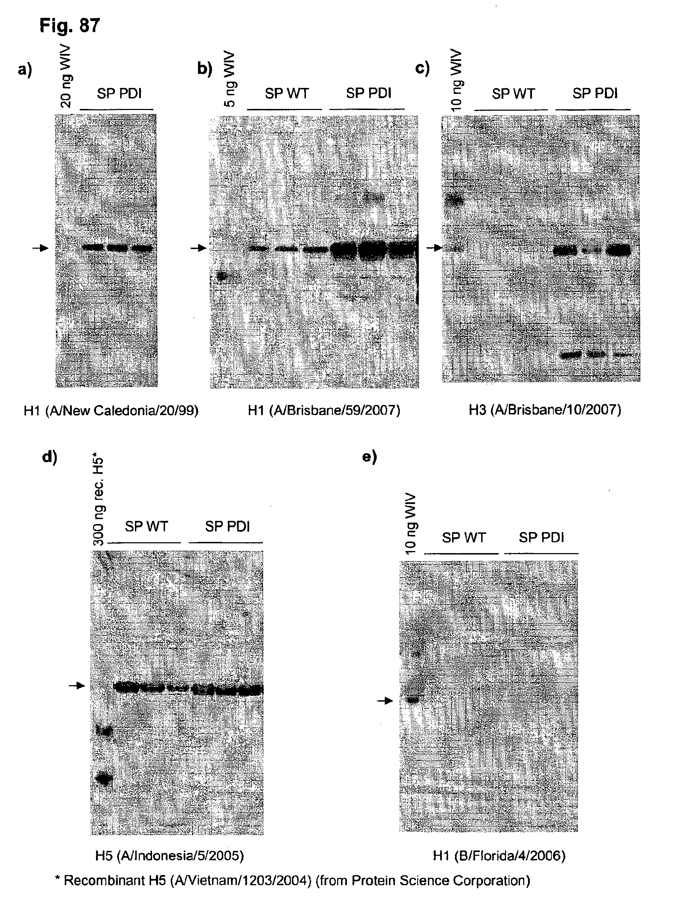

**FIGURE 87** shows an immunoblot analysis of expression of HA using a signal peptide from alfalfa protein disulfide isomerase. Twenty micrograms of leaf protein extract obtained from 3 separate plants were loaded on the SDS-PAGE except for the H1 (A/New Caledonia/20/99 (H1N1)) where five micrograms were used. The indicated controls (whole inactivated virus (WIV) of homologous strain)were spiked in five or twenty micrograms of mock-infiltrated plants. a) Expression of H1 from A/New Caledonia/20/99), b) expression of H1 from A/Brisbane/59/2007, c) expression of H3 from A/Brisbane/10/2007, d) expression of H5 from A/Indonesia/5/2005, e) expression of HA from B/Florida/4/2006. The arrows indicate the immunoband corresponding to HA0. SP WT: native signal peptide, PS PDI: alfalfa PDI signal peptide.

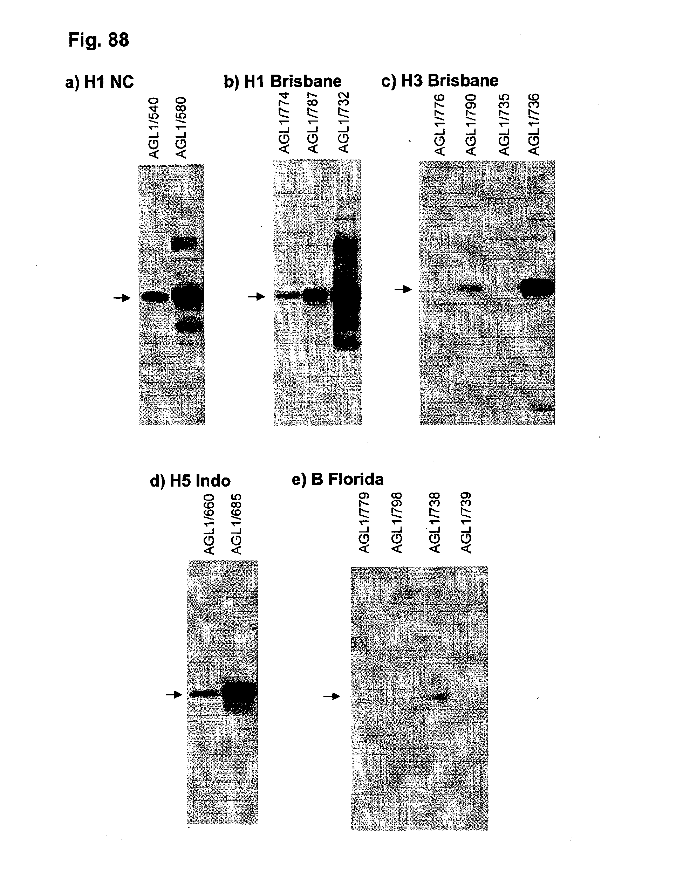

**FIGURE 88** shows a comparison of HA expression strategies by immunoblot analysis of leaf protein extracts. HA was produced using plastocyanin- or CPMV-HT-based cassettes. For CPMV-HT, the wild-type HA signal peptide and the signal peptide from alfalfa PDI were also compared. Twenty micrograms of protein extract were loaded on the SDS-PAGE for HA subtype analyzed except for the H1 New Caledonia for which five micrograms of proteins were loaded. a) Expression of H1 from A/New Caledonia/20/1999, b) expression of H1 from A/Brisbane/59/2007, c) expression of H3 from A/Brisbane/10/2007, d) expression of H5 from A/Indonesia/5/2005, and e) expression of B from B/Florida/4/2006. The arrows indicate the immunoband corresponding to HA0; specific *Agrobacterium* strains comprising the specific vectors used for HA expression are indicated at the top of the lanes.

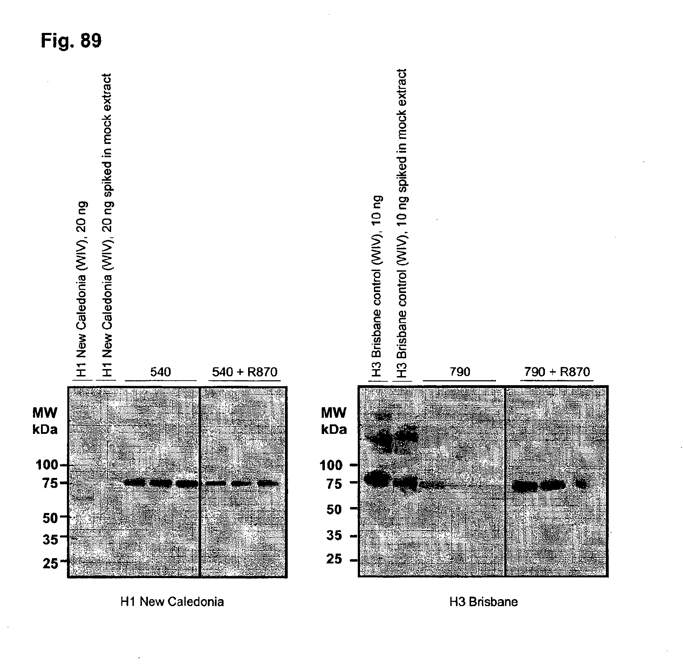

**FIGURE 89** shows an immunoblot of HA accumulation when co-expressed with Hsp 40 and Hsp70. H1 New Caledonia (AGL1/540) and H3 Brisbane (AGL1/790) were expressed alone or co-expressed with AGL1/R870. HA accumulation level was evaluated by immunoblot analysis of protein extracts from infiltrated leaves. Whole inactivated virus (WIV) of strain A/New Caledonia/20/99 or Brisbane/10/2007 were used as controls.

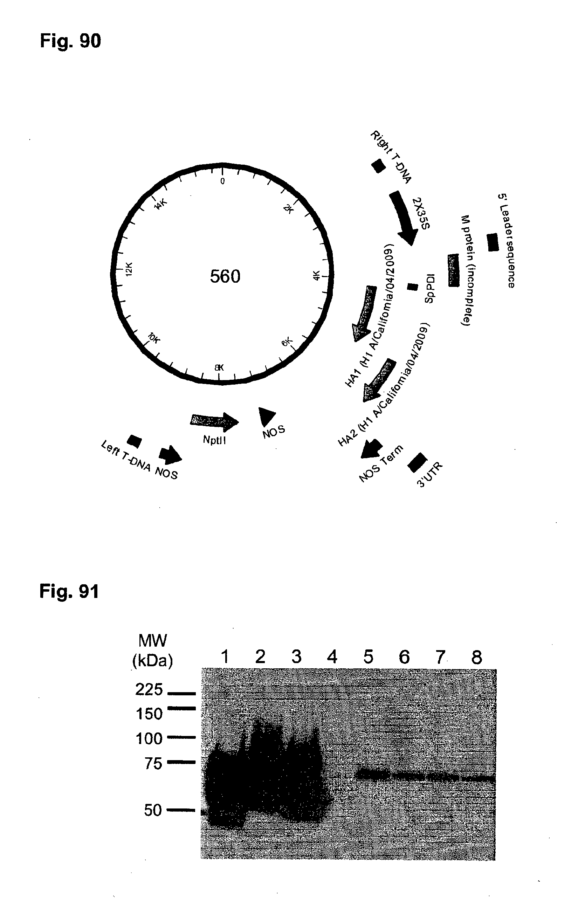

**FIGURE 90** shows a CPMV-HT based expression cassette for H1 from A/California/04/09 (construct # 560).
**FIGURE 91** shows Western blot analysis of H1 from A/California/04/09 in protein extracts from agroinfiltrated plants 2 days post infiltration. Samples run in each lane are laid out in Table 20.

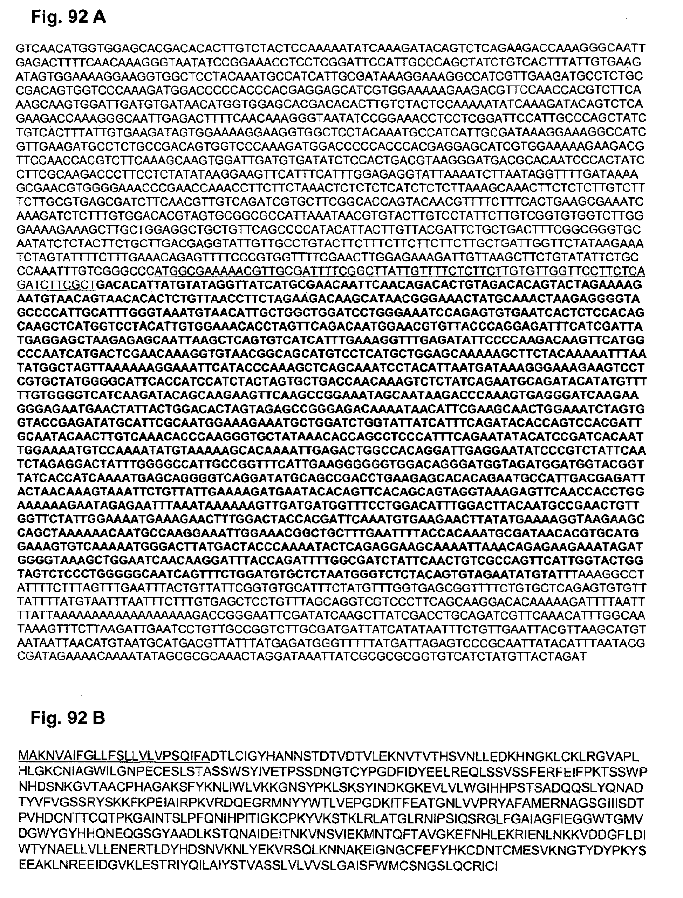

**FIGURE 92A** shows the nucleotide sequence (SEQ ID NO: 127) of the CPMV-HT-based expression cassette for H1 from A/California/04/09 (cassette number 560). Alfalfa protein disulfide isomerase signal peptide coding sequence is underlined and mature H1 coding sequence is highlighted in bold. Figure 92B shows amino acid sequence (SEQ ID NO: 128) of the H1 from A/California/04/09 (as encoded by SEQ ID NO: 127). Alfalfa protein disulfide isomerase signal peptide is underlined.

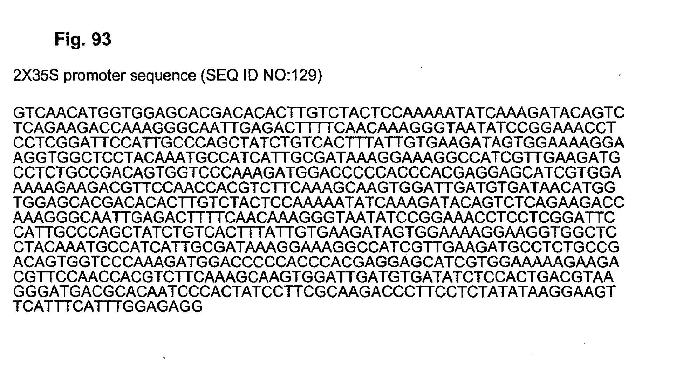

FIGURE 93 shows the nulcoetide sequence of the 2X35S promoter (SEQ ID NO: 129).

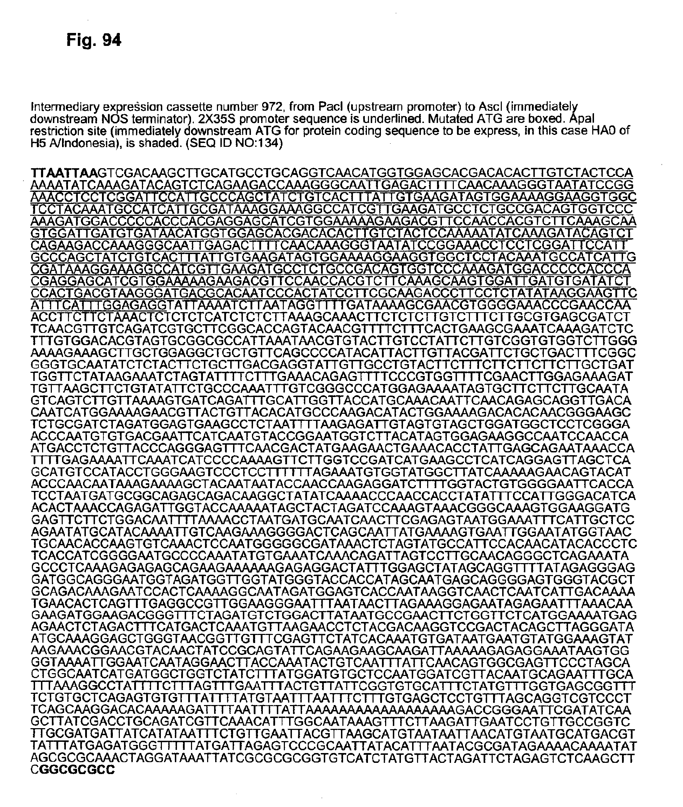

FIGURE 94 shows the nucleotide sequence of intermediary expression cassette number 972 (SEQ ID NO: 134), from PacI (upstream promoter) to AscI (immediately downstream NOS terminator). 2X35S promoter sequence is underlined. Mutated ATG are boxed. ApaI restriction site (immediately downstream ATG for protein coding sequence to be express, in this case HA0 of H5 A/Indonesia), is shaded.

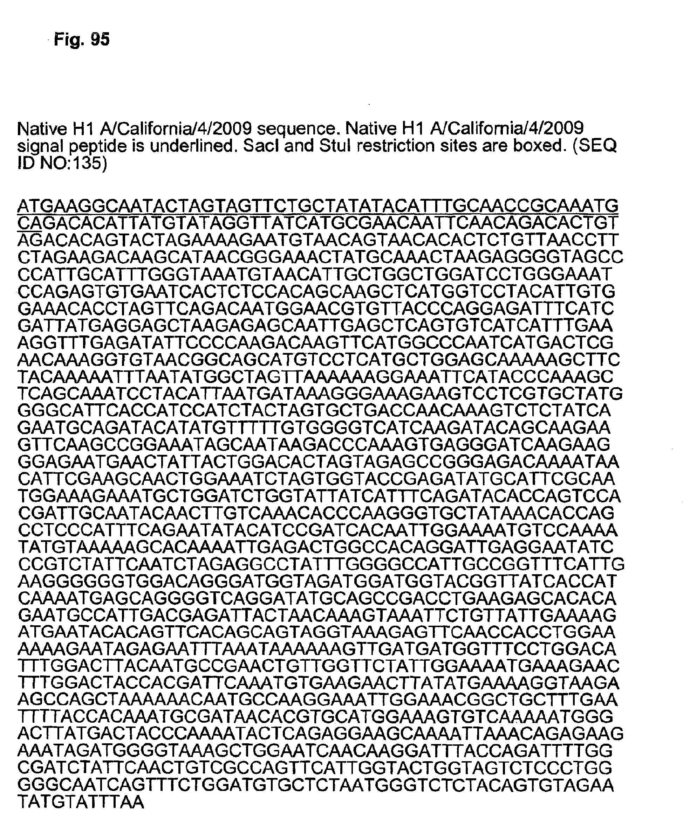

FIGURE 95 shows the nucleotide sequence of Native H1 A/California/4/2009 sequence (SEQ ID NO: 135). Native H1 A/California/4/2009 signal peptide is underlined. SacI and StuI restriction sites are boxed.

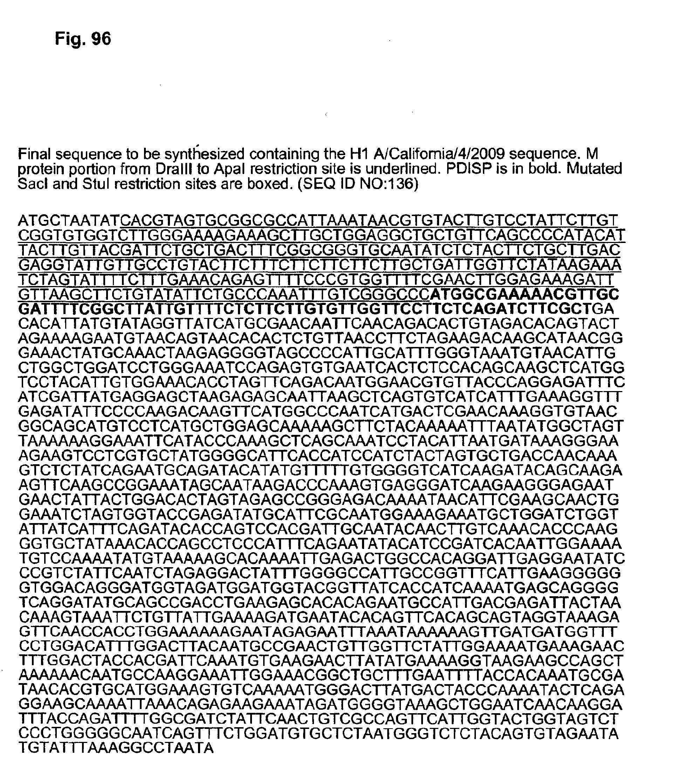

FIGURE 96 shows the nucleotide sequences of the final sequence (SEQ ID NO: 136) synthesized containing H1 A/California/4/2009 sequence. M protein portion from DraIII to ApaI restriction site is underlined. PDISP is in bold. Mutated SacI and StuI restriction sites are boxed.

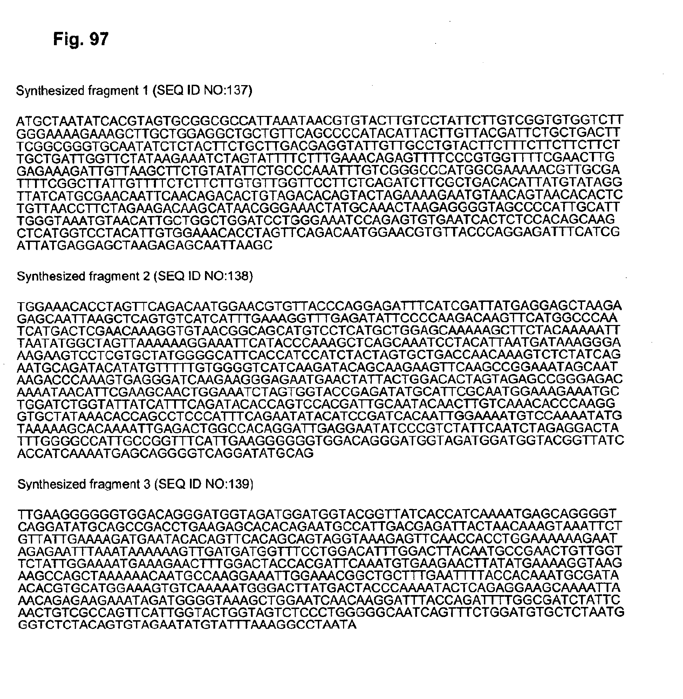

FIGURE 97 shows the nucleotide sequence of Fragments 1 (SEQ ID NO: 137), 2 (SEQ ID NO: 138) and 3 (SEQ ID NO: 139) used to synthesize the H1 A/California/4/2009 sequence using PCR-based ligation.

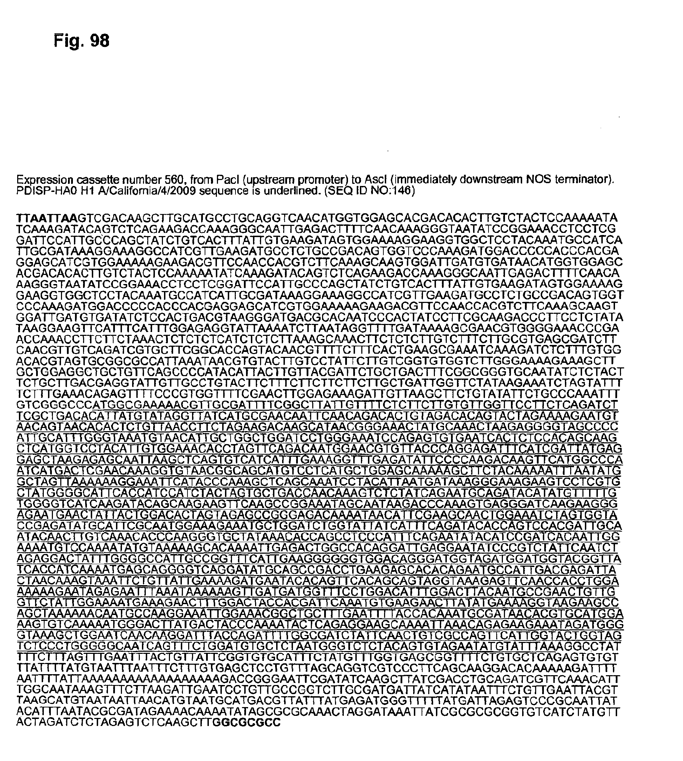

FIGURE 98 shows the nucleotide sequence of expression cassette number 560 (SEQ ID NO:146), from PacI (upstream promoter) to AscI (immediately downstream NOS terminator). PDISP-HA0 H1 A/California/4/2009 sequence is underlined.

### DETAILED DESCRIPTION

The present invention relates to the production of virus-like particles. More specifically, the present invention is directed to the production of virus-like particles comprising influenza antigens.

The following description is of a preferred embodiment.

The present invention provides a nucleic acid comprising a nucleotide sequence encoding an antigen from an enveloped virus, for example, the influenza hemagglutinin (HA), operatively linked to a regulatory region active in a plant.

Furthermore, the present invention provides a method of producing virus like particles (VLPs) in a plant. The method involves introducing a nucleic acid encoding an antigen operatively linked to a regulatory region active in the plant, into the plant, or portion of the plant, and incubating the plant or a portion of the plant under conditions that permit the expression of the nucleic acid, thereby producing the VLPs.

VLPs may be produced from influenza virus, however, VLPs may also be produced from other plasma membrane derived virus including but not limited to Measles, Ebola, Marburg, and HIV.

The invention includes VLPs of all types of influenza virus which may infect humans, including for example, but not limited to the very prevalent A (H1N1) sub-type (e.g. A/New Caledonia/20/99 (H1N1)), the A/Indonesia/5/05 sub-type (H5N1) (SEQ ID NO: 60) and the less common B type (for example SEQ ID NO:26, Figure 10O), and C type (SEQ ID NO:27, Figure 10P), and to HAs obtained from other influenza subtypes. VLPs of other influenza subtypes are also included in the present invention, for example, ABrisbane/59/2007 (H1N1; SEQ ID NO:48), A/Solomon Islands/3/2006 (H1N1; SEQ ID NO:49), A/Singapore/1/57 (H2N2; SEQ ID NO:54), A/Anhui/1/2005 (H5N1; SEQ ID NO:55), A/Vietnam/1194/2004 (H5N1; SEQ ID NO:56), A/Teal/Hong Kong/W312/97 (H6N1; SEQ ID NO:57), A/Hong Kong/1073/99 (H9N2; SEQ ID NO:59), A/Brisbane/10/2007 (H3N2; SEQ ID NO:50), A/Wisconsin/67/2005 (H3N2; SEQ ID NO:51), A/Equine/Prague/56 (H7N7; SEQ ID NO:58), B/Malaysia/2506/2004 (SEQ ID NO:52), B/Florida/4/2006 (SEQ ID NO:53) or A/California/04/09 (H1N1) (SEQ ID NO: 127).

The present invention also pertains to influenza viruses which infect other mammals or host animals, for example humans, primates, horses, pigs, birds, avian water fowl, migratory birds, quail, duck, geese, poultry, chicken, camel, canine, dogs, feline, cats, tiger, leopard, civet, mink, stone marten, ferrets, house pets, livestock, mice, rats, seal, whale and the like.

Non limiting examples of other antigens that may be expressed in plasma membrane derived viruses include, the Capsid protein of HIV - p24; gp120, gp41 - envelope proteins, the structural proteins VP30 and VP35; Gp/SGP (a glycosylated integral membrane protein) of Filoviruses, for example Ebola or Marburg, or the H protein, and F protein of Paramyxoviruses, for example, Measles.

The invention also includes, but is not limited to, influenza derived VLPs that obtain a lipid envelope from the plasma membrane of the cell in which the VLP proteins are expressed. For example, if the VLP is expressed in a plant-based system, the VLP may obtain a lipid envelope from the plasma membrane of the cell.

Generally, the term "lipid" refers to a fat-soluble (lipophilic), naturally-occurring molecules. The term is also used more specifically to refer to fatty-acids and their derivatives (including tri-, di-, and monoglycerides and phospholipids), as well as other fat-soluble sterol-containing metabolites or sterols. Phospholipids are a major component of all biological membranes, along with glycolipids, sterols and proteins. Examples of phospholipids include phosphatidylethanolamine, phosphatidylcholine, phosphatidylinositol, phosphatidylserine, phosphatidylglycerol and the like. Examples of sterols include zoosterols (for example, cholesterol) and phytosterols (for example, sitosterol) and steryl-glucoside. Over 200 phytosterols have been identified in various plant species, the most common being campesterol, stigmasterol, ergosterol, brassicasterol, delta-7-stigmasterol, delta-7-avenasterol, daunosterol, sitosterol, 24-methylcholesterol, cholesterol or beta-sitosterol. As one of skill in the art would understand, the lipid composition of the plasma membrane of a cell may vary with the culture or growth conditions of the cell or organism from which the cell is obtained.

Cell membranes generally comprise lipid bilayers, as well as proteins for various functions. Localized concentrations of particular lipids may be found in the lipid bilayer, referred to as 'lipid rafts'. Without wishing to be bound by theory, lipid rafts may have significant roles in endo and exocytosis, entry or egress of viruses or other infectious agents, inter-cell signal transduction, interaction with other structural components of the cell or organism, such as intracellular and extracellular matrices.

With reference to influenza virus, the term "hemagglutinin" or "HA" as used herein refers to a glycoprotein found on the outside of influenza viral particles. HA is a homotrimeric membrane type I glycoprotein, generally comprising a signal peptide, an HA1 domain, and an HA2 domain comprising a membrane-spanning anchor site at the C-terminus and a small cytoplasmic tail (Figure 1B). Nucleotide sequences encoding HA are well known and are available - see, for example, the BioDefence Public Health base (Influenza Virus; see URL: biohealthbase.org) or National Center for Biotechnology Information (see URL: ncbi.nlm.nih.gov), both of which are incorporated herein by reference.

The term "homotrimer" or "homotrimeric" indicates that an oligomer is formed by three HA protein molecules. Without wishing to be bound by theory, HA protein is synthesized as monomeric precursor protein (HA0) of about 75 kDa, which assembles at the surface into an elongated trimeric protein. Before trimerization occurs, the precursor protein is cleaved at a conserved activation cleavage site (also referred to as fusion peptide) into 2 polypeptide chains, HA1 and HA2 (comprising the transmembrane region), linked by a disulfide bond. The HA1 segment may be 328 amino acids in length, and the HA2 segment may be 221 amino acids in length. Although this cleavage may be important for virus infectivity, it may not be essential for the trimerization of the protein. Insertion of HA within the endoplasmic reticulum (ER) membrane of the host cell, signal peptide cleavage and protein glycosylation are co-translational events. Correct refolding of HA requires glycosylation of the protein and formation of 6 intra-chain disulfide bonds. The HA trimer assembles within the cis- and trans-Golgi complex, the transmembrane domain playing a role in the trimerization process. The crystal structures of bromelain-treated HA proteins, which lack the transmembrane domain, have shown a highly conserved structure amongst influenza strains. It has also been established that HA undergoes major conformational changes during the infection process, which requires the precursor HA0 to be cleaved into the 2 polypeptide chains HA1 and HA2. The HA protein may be processed (i.e., comprise HA1 and HA2 domains), or may be unprocessed (i.e. comprise the HA0 domain).

The present invention pertains to the use of an HA protein comprising the transmembrane domain and includes HA1 and HA2 domains, for example the HA protein may be HA0, or processed HA comprising HA1 and HA2. The HA protein may be used in the production or formation of VLPs using a plant, or plant cell, expression system.

The HA of the present invention may be obtained from any subtype. For example, the HA may be of subtype H1, H2, H3, H4, H5, H6, H7, H8, H9, H10, H11, H12, H13, H14, H15, H16 or of influenza type B. The recombinant HA of the present invention may also comprise an amino acid sequence based on the sequence any hemagglutinin known in the art-see, for example, the BioDefence Public Health base (Influenza Virus; see URL: biohealthbase.org) or National Center for Biotechnology Information (see URL: ncbi.nlm.nih.gov). Furthermore, the HA may be based on the sequence of a hemagglutinin that is isolated from one or more emerging or newly-identified influenza viruses.

The present invention also includes VLPs that comprise HAs obtained from one or more than one influenza subtype. For example, VLPs may comprise one or more than one HA from the subtype H1 (encoded by SEQ ID NO:28), H2 (encoded by SEQ ID NO:12), H3 (encoded by SEQ ID NO: 13), H4 (encoded by SEQ ID NO:14), H5 (encoded by SEQ ID NO: 15), H6 (encoded by SEQ ID NO:16), H7 (encoded by SEQ ID NO: 11), H8 (encoded by SEQ ID NO: 17), H9 (encoded by SEQ ID NO:18), H10 (encoded by SEQ ID NO:19), H11 (encoded by SEQ ID NO:20), H12 (encoded by SEQ ID NO:21), H13 (encoded by SEQ ID NO:27), H14 (encoded by SEQ ID NO:23), H15 (encoded by SEQ ID NO:24), H16 (encoded by SEQ ID NO:25), or influenza type B (encoded by SEQ ID NO: 26), or a combination thereof. One or more that one HA from the one or more than one influenza subtypes may be co-expressed within a plant or insect cell to ensure that the synthesis of the one or more than one HA results in the formation of VLPs comprising a combination of HAs obtained from one or more than one influenza subtype. Selection of the combination of HAs may be determined by the intended use of the vaccine prepared from the VLP. For example a vaccine for use in inoculating birds may comprise any combination of HA subtypes, while VLPs useful for inoculating humans may comprise subtypes one or more than one of subtypes H1, H2, H3, H5, H7, H9, H10, N1, N2, N3 and N7. However, other HA subtype combinations may be prepared depending upon the use of the inoculum.

Therefore, the present invention is directed to a VLP comprising one or more than one HA subtype, for example two, three, four, five, six, or more HA subtypes.

The present invention also provides for nucleic acids encoding hemagglutinins that form VLPs when expressed in plants.

Exemplary nucleic acids may comprise nucleotide sequences of hemagglutinin from selected strains of influenza subtypes. For example, an A (H1N1) sub-type such as A/New Caledonia/20/99 (H1N1) (SEQ ID NO: 33), the A/Indonesia/5/05 sub-type (H5N1) (comprising construct #660; SEQ ID NO: 60) and the less common B type (for example SEQ ID NO:26, Figure 10O), and C type (SEQ ID NO:27, Figure 10P), and to HAs obtained from other influenza subtypes. VLPs of other influenza subtypes are also included in the present invention, for example, A/Brisbane/59/2007 (H1N1; SEQ ID NO:36), A/Solomon Islands/3/2006 (H1N1; SEQ ID NO:37), A/Singapore/1/57 (H2N2; SEQ ID NO:42), A/Anhui/1/2005 (H5N1; SEQ ID NO:43), A/Vietnarn/1194/2004 (H5N1; SEQ ID NO:44), A/Teal/Hong Kong/W312/97 (H6N1; SEQ ID NO:45), A/Hong Kong/1073/99 (H9N2; SEQ ID NO:47), A/Brisbane/10/2007 (H3N2; SEQ ID NO:38), A/Wisconsin/67/2005 (H3N2; SEQ ID NO:39), A/Equine/Prague/56 (H7N7; SEQ ID NO:46), B/Malaysia/2506/2004 (SEQ ID NO:40), B/Florida/4/2006 (SEQ ID NO:41) or A/California/04/09 (H1N1) (SEQ ID NO: 127).

Correct folding of the hemagglutinins may be important for stability of the protein, formation of multimers, formation of VLPs and function of the HA (ability to hemagglutinate), among other characteristics of influenza hemagglutinins. Folding of a protein may be influenced by one or more factors, including, but not limited to, the sequence of the protein, the relative abundance of the protein, the degree of intracellular crowding, the availability of cofactors that may bind or be transiently associated with the folded, partially folded or unfolded protein, the presence of one or more chaperone proteins, or the like.

.Heat shock proteins (Hsp) or stress proteins are examples of chaperone proteins, which may participate in various cellular processes including protein synthesis, intracellular trafficking, prevention of misfolding, prevention of protein aggregation, assembly and disassembly of protein complexes, protein folding, and protein disaggregation. Examples of such chaperone proteins include, but are not limited to, Hsp60, Hsp65, Hsp 70, Hsp90, Hsp100, Hsp20-30, Hsp10, Hsp100-200, Hsp100, Hsp90, Lon, TF55, FKBPs, cyclophilins, CIpP, GrpE, ubiquitin, calnexin, and protein disulfide isomerases. See, for example, Macario, A.J.L., Cold Spring Harbor Laboratory Res. 25:59-70. 1995; Parsell, D.A. & Lindquist, S. Ann. Rev. Genet. 27:437-496 (1993); U.S. Patent No. 5,232,833. In some examples, a particular group of chaperone proteins includes Hsp40 and Hsp70.

Examples of Hsp70 include Hsp72 and Hsc73 from mammalian cells, DnaK from bacteria, particularly mycobacteria such as *Mycobacterium* leprae, *Mycobacterium tuberculosis,* and *Mycobacterium bovis* (such as Bacille-Calmette Guerin: referred to herein as Hsp71). DnaK from *Escherichia coli,* yeast. and other prokaryotes, and BiP and Grp78 from eukaryotes, such as *A. thaliana* (Lin et al. 2001 (Cell Stress and Chaperones 6:201-208). A particular example of an Hsp70 is A. *thaliana* Hsp70 (encoded by SEQ ID NO: 122, or SEQ ID NO: 123). Hsp70 is capable of specifically binding ATP as well as unfolded polypeptides and peptides, thereby participating in protein folding and unfolding as well as in the assembly and disassembly of protein complexes.

Examples of Hsp40 include DnaJ from prokaryotes such as *E. coli* and mycobacteria and HSJ1, HDJ1 and Hsp40 from eukaryotes, such as alfalfa (Frugis et al., 1999. Plant Molecular Biology 40:397-408). A particular example of an Hsp40 is M. sativa MsJ1 (encoded by SEQ ID NO: 121, 123 or 114). Hsp40 plays a role as a molecular chaperone in protein folding, thermotolerance and DNA replication, among other cellular activities.

Among Hsps, Hsp70 and its co-chaperone, Hsp40, are involved in the stabilization of translating and newly synthesized polypeptides before the synthesis is complete. Without wishing to be bound by theory, Hsp40 binds to the hydrophobic patches of unfolded (nascent or newly transferred) polypeptides, thus facilitating the interaction of Hsp70-ATP complex with the polypeptide. ATP hydrolysis leads to the formation of a stable complex between the polypeptide, Hsp70 and ADP, and release of Hsp40. The association of Hsp70-ADP complex with the hydrophobic patches of the polypeptide prevents their interaction with other hydrophobic patches, preventing the incorrect folding and the formation of aggregates with other proteins (reviewed in Hartl, FU. 1996. Nature 381:571-579).

Again, without wishing to be bound by theory, as protein production increases in a recombinant protein expression system, the effects of crowding on recombinant protein expression may result in aggregation and/or reduced accumulation of the recombinant protein resulting from degradation of misfolded polypeptide. Native chaperone proteins may be able to facilitate correct folding of low levels of recombinant protein, but as the expression levels increase, native chaperones may become a limiting factor. High levels of expression of hemagglutinin in the agroinfiltrated leaves may lead to the accumulation of hemagglutinin polypeptides in the cytosol, and co-expression of one or more than one chaperone proteins such as Hsp70, Hsp40 or both Hsp70 and Hsp40 may increase stability in the cytosol of the cells expressing the polypeptides cells, thus reducing the level of misfolded or aggregated hemagglutinin polypeptides, and increasing the number of polypeptides accumulate as stable hemagglutinin, exhibiting tertiary and quaternary structural characteristics that allow for hemagglutination and/or formation of virus-like particles.

Therefore, the present invention also provides for a method of producing influenza VLPs in a plant, wherein a first nucleic acid encoding an influenza HA is co-expressed with a second nucleic acid encoding a chaperone. The first and second nucleic acids may be introduced to the plant in the same step, or may be introduced to the plant sequentially. The present invention also provides for a method of producing influenza VLPs in a plant, where the plant comprises the first nucleic acid, and the second nucleic acid is subsequently introduced.

The present invention also provides for a plant comprising a nucleic acid encoding one, or more than one influenza hemagglutinin and a nucleic acid encoding one or more than one chaperones.

Processing of an N-terminal signal peptide (SP) sequence during expression and/or secretion of influenza hemagglutinins has been proposed to have a role in the folding process. The term "signal peptide" refers generally to a short (about 5-30 amino acids) sequence of amino acids, found generally at the N-terminus of a hemagglutinin polypeptide that may direct translocation of the newly-translated polypeptide to a particular organelle, or aid in positioning of specific domains of the polypeptide. The signal peptide of hemagglutinins target the translocation of the protein into the endoplasmic reticulum and have been proposed to aid in positioning of the N-terminus proximal domain relative to a membrane-anchor domain of the nascent hemagglutinin polypeptide to aid in cleavage and folding of the mature hemagglutinin. Removal of a signal peptide (for example, by a signal peptidase), may require precise cleavage and removal of the signal peptide to provide the mature hemagglutinin - this precise cleavage may be dependent on any of several factors, including a portion or all of the signal peptide, amino acid sequence flanking the cleavage site, the length of the signal peptide, or a combination of these, and not all factors may apply to any given sequence.

A signal peptide may be native to the hemagglutinin being expressed, or a recombinant hemagglutinin comprising a signal peptide from a first influenza type, subtype or strain with the balance of the hemagglutinin from a second influenza type, subtype or strain. For example the native SP of HA subtypes H1, H2, H3, H5, H6, H7, H9 or influenza type B may be used to express the HA in a plant system.

A signal peptide may also be non-native, for example, from a structural protein or hemagglutinin of a virus other than influenza, or from a plant, animal or bacterial polypeptide. An exemplary signal peptide is that of alfalfa protein disulfide isomerase (PDISP) (nucleotides 32-103 of Accession No. Z11499; SEQ ID NO: 34; Figure 17; amino acid sequence MAKNVAIFGLLFSLLLLVPSQIFAEE).

The present invention also provides for an influenza hemagglutinin comprising a native, or a non-native signal peptide, and nucleic acids encoding such hemagglutinins.

Influenza HA proteins exhibit a range of similarities and differences with respect to molecular weight, isoelectric point, size, glycan complement and the like. The physicochemical properties of the various hemagglutinins may be useful to allow for differentiation between the HAs expressed in a plant, insect cell or yeast system, and may be of particular use when more than one HA is co-expressed in a single system. Examples of such physicochemical properties are provided in Table 1.

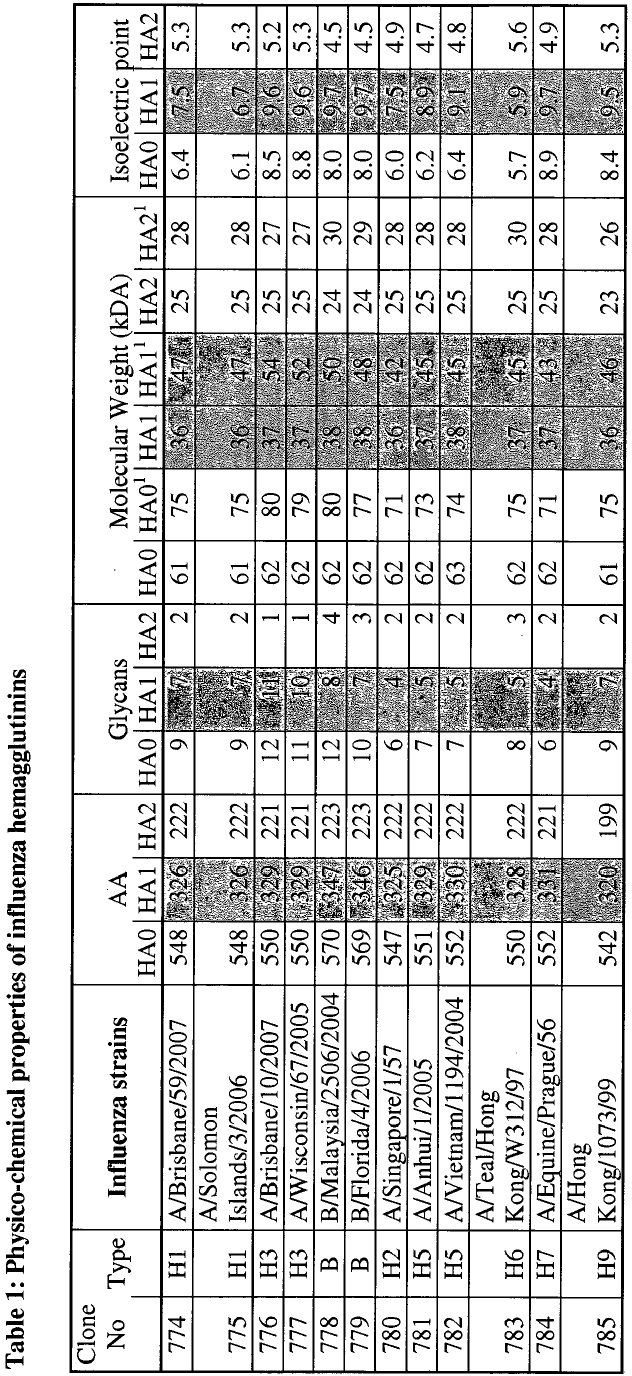

The present invention also includes nucleotide sequences SEQ ID NO:28; SEQ ID NO:3; SEQ ID NO:11, encoding HA from H1, H5 or H7, respectively. The present invention also includes a nucleotide sequence that hybridizes under stringent hybridisation conditions to SEQ ID NO:28; SEQ ID NO:3; SEQ ID NO: 11. The present invention also includes a nucleotide sequence that hybridizes under stringent hybridisation conditions to a compliment of SEQ ID NO:28; SEQ ID NO:3; SEQ ID NO:1. These nucleotide sequences that hybridize to SEQ ID or a complement of SEQ ID encode a hemagglutinin protein that, when expressed forms a VLP, and the VLP induces production of an antibody when administered to a subject. For example, expression of the nucleotide sequence within a plant cell forms a VLP, and the VLP may be used to produce an antibody that is capable of binding HA, including mature HA, HA0, HA1, or HA2 of one or more influenza types or subtypes. The VLP, when administered to a subject, induces an immune response.

The present invention also includes nucleotide sequences SEQ ID NO: 12 SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO:27, SEQ ID NO: 33, SEQ ID NO: 35, SEQ ID NO: 36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO: 127 or SEQ ID NO:47. The present invention also includes a nucleotide sequence that hybridizes under stringent hybridisation conditions to SEQ ID NO: 12 SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO:27, SEQ ID NO: 33, SEQ ID NO: 35, SEQ ID NO: 36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO: 127 or SEQ ID NO:47. The present invention also includes a nucleotide sequence that hybridizes under stringent hybridisation conditions to a compliment of SEQ ID NO: 12 SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO:27, SEQ ID NO: 33, SEQ ID NO: 35, SEQ ID NO: 36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO: 127 or SEQ ID NO:47. These nucleotide sequences that hybridize to SEQ ID NO: 12 SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO:27, SEQ ID NO: 33, SEQ ID NO: 35, SEQ ID NO: 36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO: 127 or SEQ ID NO:47or a complement of SEQ ID NO:12 SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO:27, SEQ ID NO: 33, SEQ ID NO: 35, SEQ ID NO: 36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO: 127 or SEQ ID NO:47 encode a hemagglutinin protein that, when expressed forms a VLP, and the VLP induces production of an antibody when administered to a subject. For example, expression of the nucleotide sequence within a plant cell forms a VLP, and the VLP may be used to produce an antibody that is capable of binding HA, including mature HA, HA0, HA1, or HA2 of one or more influenza types or subtypes. The VLP, when administered to a subject, induces an immune response.

In some embodiments, the present invention also includes nucleotide sequences SEQ ID NO: 33, SEQ ID NO: 35, SEQ ID NO: 36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO: 127 or SEQ ID NO:47, encoding HA from H1, H2, H3, H5, H7 or H9 subtypes of influenza A, or HA from type B influenza. The present invention also includes a nucleotide sequence that hybridizes under stringent hybridisation conditions to SEQ ID NO: 33, SEQ ID NO: 35, SEQ ID NO: 36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO: 127 or SEQ ID NO:47. The present invention also includes a nucleotide sequence that hybridizes under stringent hybridisation conditions to a compliment of SEQ ID NO: 33, SEQ ID NO: 35, SEQ ID NO: 36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO: 127 or SEQ ID NO:47. These nucleotide sequences that hybridize to SEQ ID NO: 33, SEQ ID NO: 35, SEQ ID NO: 36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO: 127 or SEQ ID NO:47or a complement of SEQ ID NO: 33, SEQ ID NO: 35, SEQ ID NO: 36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO: 127 or SEQ ID NO:47 encode a hemagglutinin protein that, when expressed forms a VLP, and the VLP induces production of an antibody when administered to a subject. For example, expression of the nucleotide sequence within a plant cell forms a VLP, and the VLP may be used to produce an antibody that is capable of binding HA, including mature HA, HA0, HA1, or HA2 of one or more influenza types or subtypes. The VLP, when administered to a subject, induces an immune response.

Hybridization under stringent hybridization conditions is known in the art (see for example Current Protocols in Molecular Biology, Ausubel et al., eds. 1995 and supplements; Maniatis et al., in Molecular Cloning (A Laboratory Manual), Cold Spring Harbor Laboratory, 1982; Sambrook and Russell, in Molecular Cloning: A Laboratory Manual, 3rd edition 2001; each of which is incorporated herein by reference). An example of one such stringent hybridization conditions may be about 16-20 hours hybridization in 4 X SSC at 65°C, followed by washing in 0.1 X SSC at 65°C for an hour, or 2 washes in 0.1 X SSC at 65°C each for 20 or 30 minutes. Alternatively, an exemplary stringent hybridization condition could be overnight (16-20 hours) in 50% formamide, 4 X SSC at 42°C, followed by washing in 0.1 X SSC at 65°C for an hour, or 2 washes in 0.1 X SSC at 65°C each for 20 or 30 minutes, or overnight (16-20 hours), or hybridization in Church aqueous phosphate buffer (7% SDS; 0.5M NaPO₄ buffer pH 7.2; 10 mM EDTA) at 65°C, with 2 washes either at 50°C in 0.1 X SSC, 0.1% SDS for 20 or 30 minutes each, or 2 washes at 65°C in 2 X SSC, 0.1% SDS for 20 or 30 minutes each.

Additionally, the present invention includes nucleotide sequences that are characterized as having about 70, 75, 80, 85, 87, 90, 91, 92, 93 94, 95, 96, 97, 98, 99, 100% or any amount therebetween, sequence identity, or sequence similarity, with the nucleotide sequence encoding HA from H1 (SEQ ID NO:28 or SEQ ID NO: 127), H5 (SEQ ID NO:3) or H7 (SEQ ID NO: 11), wherein the nucleotide sequence encodes a hemagglutinin protein that when expressed forms a VLP, and that the VLP induces the production of an antibody. For example, expression of the nucleotide sequence within a plant cell forms a VLP, and the VLP may be used to produce an antibody that is capable of binding HA, including mature HA, HA0, HA1, or HA2. The VLP, when administered to a subject, induces an immune response.

Additionally, the present invention includes nucleotide sequences that are characterized as having about 70, 75, 80, 85, 87, 90, 91, 92, 93 94, 95, 96, 97, 98, 99, 100% or any amount therebetween, sequence identity, or sequence similarity, with the nucleotide sequence of SEQ ID NO:12 SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO:27, SEQ ID NO: 33, SEQ ID NO: 35, SEQ ID NO: 36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO: 127 or SEQ ID NO:47, wherein the nucleotide sequence encodes a hemagglutinin protein that when expressed forms a VLP, and that the VLP induces the production of an antibody. For example, expression of the nucleotide sequence within a plant cell forms a VLP, and the VLP may be used to produce an antibody that is capable of binding HA, including mature HA, HA0, HA1, or HA2. The VLP, when administered to a subject, induces an immune response.

Additionally, the present invention includes nucleotide sequences that are characterized as having about 70, 75, 80, 85, 87, 90, 91, 92, 93 94, 95, 96, 97, 98, 99, 100% or any amount therebetween, sequence identity, or sequence similarity, with the nucleotide sequence of SEQ ID NO: 33, SEQ ID NO: 35, SEQ ID NO: 36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO: 127 or SEQ ID NO:47, wherein the nucleotide sequence encodes a hemagglutinin protein that when expressed forms a VLP, and that the VLP induces the production of an antibody. For example, expression of the nucleotide sequence within a plant cell forms a VLP, and the VLP may be used to produce an antibody that is capable of binding HA, including mature HA, HA0, HA1, or HA2. The VLP, when administered to a subject, induces an immune response.

Similarly, the present invention includes HAs associated with the following subtypes H1 (encoded by SEQ ID NO:28 or SEQ ID NO:127), H2 (encoded by SEQ ID NO:12), H3 (encoded by SEQ ID NO: 13), H4 (encoded by SEQ ID NO:14), H5 (encoded by SEQ ID NO:15), H6 (encoded by SEQ ID NO:16), H7 (encoded by SEQ ID NO: 11), H8 (encoded by SEQ ID NO:17), H9 (encoded by SEQ ID NO:18), H10 (encoded by SEQ ID NO:19), H11 (encoded by SEQ ID NO:20), H12 (encoded by SEQ ID NO:21), H13 (encoded by SEQ ID NO:27), H14 (encoded by SEQ ID NO:23), H15 (encoded by SEQ ID NO:24), H16 (encoded by SEQ ID NO:25), or influenza type B (encoded by SEQ ID NO: 26); see Figures 10A to 10O), and nucleotide sequences that are characterized as having from about 70 to 100% or any amount therebetween, 80 to 100% or any amount there between, 90-100% or any amount therebetween, or 95-100% or any amount therebetween, sequence identity with H1 (SEQ ID NO:28 or SEQ ID NO:127), H2 (SEQ ID NO: 12), H3 (SEQ ID NO: 13), H4 (SEQ ID NO:14), H5 (SEQ ID NO:15), H6 (SEQ ID NO:16), H7 (SEQ ID NO:11), H8 (SEQ ID NO:17), H9 (SEQ ID NO:18), H10 (SEQ ID NO:19), H11 (SEQ ID NO:20), H12 (SEQ ID NO:21), H13 (SEQ ID NO:27), H14 (SEQ ID NO:23), H15 (SEQ ID NO:24), H16 (SEQ ID NO:25), wherein the nucleotide sequence encodes a hemagglutinin protein that when expressed forms a VLP, and that the VLP induces the production of an antibody. For example, expression of the nucleotide sequence within a plant cell forms a VLP, and the VLP may be used to produce an antibody that is capable of binding HA, including mature HA, HA0, HA1, or HA2. The VLP, when administered to a subject, induces an immune response.

An "immune response" generally refers to a response of the adaptive immune system. The adaptive immune system generally comprises a humoral response, and a cell-mediated response. The humoral response is the aspect of immunity that is mediated by secreted antibodies, produced in the cells of the B lymphocyte lineage (B cell). Secreted antibodies bind to antigens on the surfaces of invading microbes (such as viruses or bacteria), which flags them for destruction. Humoral immunity is used generally to refer to antibody production and the processes that accompany it, as well as the effector functions of antibodies, including Th2 cell activation and cytokine production, memory cell generation, opsonin promotion of phagocytosis, pathogen elimination and the like. The terms "modulate" or "modulation" or the like refer to an increase or decrease in a particular response or parameter, as determined by any of several assays generally known or used, some of which are exemplified herein.

A cell-mediated response is an immune response that does not involve antibodies but rather involves the activation of macrophages, natural killer cells (NK), antigen-specific cytotoxic T-lymphocytes, and the release of various cytokines in response to an antigen. Cell-mediated immunity is used generally to refer to some Th cell activation, Tc cell activation and T-cell mediated responses. Cell mediated immunity is of particular importance in responding to viral infections.

For example, the induction of antigen specific CD8 positive T lymphocytes may be measured using an ELISPOT assay; stimulation of CD4 positive T-lymphocytes may be measured using a proliferation assay. Anti-influenza antibody titres may be quantified using an ELISA assay; isotypes of antigen-specific or cross reactive antibodies may also be measured using anti-isotype antibodies (e.g. anti -IgG, IgA, IgE or IgM)..Methods and techniques for performing such assays are well-known in the art.

A hemagglutination inhibition (HI, or HAI) assay may also be used to demonstrate the efficacy of antibodies induced by a vaccine, or vaccine composition can inhibit the agglutination of red blood cells (RBC) by recombinant HA. Hemagglutination inhibitory antibody titers of serum samples may be evaluated by microtiter HAI (Aymard et al 1973). Erythrocytes from any of several species may be used - e.g. horse, turkey, chicken or the like. This assay gives indirect information on assembly of the HA trimer on the surface of VLP, confirming the proper presentation of antigenic sites on HAs.

Cross-reactivity HAI titres may also be used to demonstrate the efficacy of an immune response to other strains of virus related to the vaccine subtype. For example, serum from a subject immunized with a vaccine composition of a first strain (e.g. VLPs of A/Indonesia 5/05) may be used in an HAI assay with a second strain of whole virus or virus particles (e.g. A/Vietnam/1194/2004), and the HAI titer determined.

Cytokine presence or levels may also be quantified. For example a T-helper cell response (Th1/Th2) will be characterized by the measurement of IFN-y and IL-4 secreting cells using by ELISA (e.g. BD Biosciences OptEIA kits). Peripheral blood mononuclear cells (PBMC) or splenocytes obtained from a subject may be cultured, and the supernatant analyzed. T lymphocytes may also be quantified by fluorescence-activated cell sorting (FACS), using marker specific fluorescent labels and methods as are known in the art.

A microneutralization assay may also be conducted to characterize an immune response in a subject, see for example the methods of Rowe et al., 1973. Virus neutralization titers may be obtained several ways, including: 1) enumeration of lysis plaques (plaque assay) following crystal violet fixation/coloration of cells; 2) microscopic observation of cell lysis in culture; 3) ELISA and spectrophotometric detection of NP virus protein (correlate with virus infection of host cells)

Sequence identity or sequence similarity may be determined using a nucleotide sequence comparison program, such as that provided within DNASIS (for example, using, but not limited to, the following parameters: GAP penalty 5, #of top diagonals 5, fixed GAP penalty 10, k-tuple 2, floating gap 10, and window size 5). However, other methods of alignment of sequences for comparison are well-known in the art for example the algorithms of Smith & Waterman (1981, Adv. Appl. Math. 2:482), Needleman & Wunsch (J. Mol. Biol. 48:443, 1970), Pearson & Lipman (1988, Proc. Nat'1. Acad. Sci. USA 85:2444), and by computerized implementations of these algorithms (e.g. GAP, BESTFIT, FASTA, and BLAST), or by manual alignment and visual inspection.

The term "hemagglutinin domain" refers to a peptide comprising either the HA0 domain, or the HA1 and HA2 domains (alternately referred to as HA1 and HA2 fragments). HA0 is a precursor of the HA1 and HA2 fragments. The HA monomer may be generally subdivided in 2 functional domains - the stem domain and the globular head, or head domain. The stem domain is involved in infectivity and pathogenicity of the virus via the conformational change it may undergo when exposed to acidic pH. The stem domain may be be further subdivided into 4 subdomains or fragments - the fusion sub-domain or peptide (a hydrophobic stretch of amino acids involved in fusion with the host membrane in the acidic pH conformational state); the stem sub-domain (may accommodate the two or more conformations), the transmembrane domain or sub-domain (TmD) (involved in the affinity of the HA for lipid rafts), and the cytoplasmic tail (cytoplasmic tail sub-domain) (Ctail) (involved in secretion of HA). The globular head is divided in 2 subdomains, the RB subdomain and the vestigial esterase domain (E). The E subdomain may be partially or fully buried and not exposed at the surface of the globular head, thus some antibodies raised against HA bind to the RB subdomain.

The term "virus like particle" (VLP), or "virus-like particles" or "VLPs" refers to structures that self-assemble and comprise structural proteins such as influenza HA protein. VLPs are generally morphologically and antigenically similar to virions produced in an infection, but lack genetic information sufficient to replicate and thus are non-infectious. In some examples, VLPs may comprise a single protein species, or more than one protein species. For VLPs comprising more than one protein species, the protein species may be from the same species of virus, or may comprise a protein from a different species, genus, subfamily or family of virus (as designated by the ICTV nomenclature). In other examples, one or more of the protein species comprising a VLP may be modified from the naturally occurring sequence. VLPs may be produced in suitable host cells including plant and insect host cells. Following extraction from the host cell and upon isolation and further purification under suitable conditions, VLPs may be purified as intact structures.

The VLPs produced from influenza derived proteins, in accordance with the present invention do not comprise M1 protein. The M1 protein is known to bind RNA (Wakefield and Brownlee, 1989) which is a contaminant of the VLP preparation. The presence of RNA is undesired when obtaining regulatory approval for the VLP product, therefore a VLP preparation lacking RNA may be advantageous.

The VLPs of the present invention may be produced in a host cell that is characterized by lacking the ability to sialylate proteins, for example a plant cell, an insect cell, fungi, and other organisms including sponge, coelenterara, annelida, arthoropoda, mollusca, nemathelminthea, trochelmintes, plathelminthes, chaetognatha, tentaculate, chlamydia, spirochetes, gram-positive bacteria, cyanobacteria, archaebacteria, or the like. See, for example Glycoforum (URL: glycoforum.gr.jp/science/word/evolution/ES-A03E.html) or Gupta et al., 1999. Nucleic Acids Research 27:370-372; or Toukach et al., 2007. Nucleic Acids Research 35:D280-D286; or URL:glycostructures.jp (Nakahara et al., 2008. Nucleic Acids Research 36:D368-D371; published online October 11, 2007 doi:10.1093/NAR/gkm833). The VLPs produced as described herein do not typically comprise neuramindase (NA). However, NA may be co-expressed with HA should VLPs comprising HA and NA be desired.

A VLP produced in a plant according to some aspects of the invention may be complexed with plant-derived lipids. The VLP may comprise an HA0, HA1 or HA2 peptide. The plant-derived lipids may be in the form of a lipid bilayer, and may further comprise an envelope surrounding the VLP. The plant derived lipids may comprise lipid components of the plasma membrane of the plant where the VLP is produced, including, but not limited to, phosphatidylcholine (PC), phosphatidylethanolamine (PE), glycosphingolipids, phytosterols or a combination thereof. A plant-derived lipid may alternately be referred to as a 'plant lipid'. Examples of phytosterols are known in the art, and include, for example, stigmasterol, sitosterol, 24-methylcholesterol and cholesterol - see, for example, Mongrand et al., 2004.

VLPs may be assessed for structure and size by, for example, hemagglutination assay, electron microscopy, or by size exclusion chromatography.

For size exclusion chromatography, total soluble proteins may be extracted from plant tissue by homogenizing (Polytron) sample of frozen-crushed plant material in extraction buffer, and insoluble material removed by centrifugation. Precipitation with PEG may also be of benefit. The soluble protein is quantified, and the extract passed through a Sephacryl^{™} column. Blue Dextran 2000 may be used as a calibration standard. Following chromatography, fractions may be further analyzed by immunoblot to determine the protein complement of the fraction.

Without wishing to be bound by theory, the capacity of HA to bind to RBC from different animals is driven by the affinity of HA for sialic acids α2,3 or α2,3 and the presence of these sialic acids on the surface of RBC. Equine and avian HA from influenza viruses agglutinate erythrocytes from all several species, including turkeys, chickens, ducks, guinea pigs, humans, sheep, horses and cows; whereas human HAs will bind to erythrocytes of turkey, chickens, ducks, guina pigs, humans and sheep (see also Ito T. et al, 1997, Virology, vol 227, p493-499; and Medeiros R et al, 2001, Virology, vol 289 p.74-85). Examples of the species reactivity of HAs of different influenza strains is shown in Tables 2A and 2B.

**Table 2A: Species of RBC bound by HAs of selected seasonal influenza strains.**

| Seasonal | Strain | No | Origin | Horse | Turkey |
|---|---|---|---|---|---|
| H1 | A/Brisbane/59/2007 (H1N1) | 774 | Human | + | ++ |
| | A/Solomon Islands/3/2006 (H1N1) | 775 | Human | + | ++ |
| H3 | A/Brisbane/10/2007 (H3N2) | 776 | Human | + | ++ |
| | A/Wisconsin/67/2005 (H3N2) | 777 | Human | + | ++ |
| B | B/Malaysia/2506/2004 | 778 | Human | + | ++ |
| | B/Florida/4/2006 | 779 | Human | + | ++ |

**Table 2B: Species of RBC bound by HAs of selected pandemic influenza strains**

| Pandemic | Strain | No | Origin | Horse | Turkey |
|---|---|---|---|---|---|
| H2 | A/Singapore/1/57 (H2N2) | 780 | Human | + | ++ |
| H5 | A/Anhui/1/2005 (H5N1) | 781 | Hu-Av | ++ | + |
| | A/Vietnam/1194/2004 (H5N1) | 782 | Hu-Av | ++ | + |
| H6 | A/Teal/Hong Kong/W312/97 (H6N1) | 783 | Avian | ++ | + |
| H7 | A/Equine/Prague/56 (H7N7) | 784 | Equine | ++ | ++ |
| H9 | A/Hong Kong/1073/99 (H9N2) | 785 | Human | ++ | + |

A fragment or portion of a protein, fusion protein or polypeptide includes a peptide or polypeptide comprising a subset of the amino acid complement of a particular protein or polypeptide, provided that the fragment can form a VLP when expressed. The fragment may, for example, comprise an antigenic region, a stress-response-inducing region, or a region comprising a functional domain of the protein or polypeptide. The fragment may also comprise a region or domain common to proteins of the same general family, or the fragment may include sufficient amino acid sequence to specifically identify the full-length protein from which it is derived.

For example, a fragment or portion may comprise from about 60% to about 100%, of the length of the full length of the protein, or any amount therebetween, provided that the fragment can form a VLP when expressed. For example, from about 60% to about 100%, from about 70% to about 100%, from about 80% to about 100%, from about 90% to about 100%, from about 95% to about 100%, of the length of the full length of the protein, or any amount therebetween. Alternately, a fragment or portion may be from about 150 to about 500 amino acids, or any amount therebetween, depending upon the HA, and provided that the fragment can form a VLP when expressed. For example, a fragment may be from 150 to about 500 amino acids, or any amount therebetween, from about 200 to about 500 amino acids, or any amount therebetween, from about 250 to about 500 amino acids, or any amount therebetween, from about 300 to about 500 or any amount therebetween, from about 350 to about 500 amino acids, or any amount therebetween, from about 400 to about 500 or any amount therebetween, from about 450 to about 500 or any amount therebetween, depending upon the HA, and provided that the fragment can form a VLP when expressed. For example, about 5, 10, 20, 30, 40 or 50 amino acids, or any amount therebetween may be removed from the C terminus, the N terminus or both the N and C terminus of an HA protein, provided that the fragment can form a VLP when expressed.

Numbering of amino acids in any given sequence are relative to the particular sequence, however one of skill can readily determine the 'equivalency' of a particular amino acid in a sequence based on structure and/or sequence. For example, if 6 N terminal amino acids were removed when constructing a clone for crystallography, this would change the specific numerical identity of the amino acid (e.g. relative to the full length of the protein), but would not alter the relative position of the amino acid in the structure.

Comparisons of a sequence or sequences may be done using a BLAST algorithm (Altschul et al., 1990. J. Mol Biol 215:403-410). A BLAST search allows for comparison of a query sequence with a specific sequence or group of sequences, or with a larger library or database (e.g. GenBank or GenPept) of sequences, and identify not only sequences that exhibit 100% identity, but also those with lesser degrees of identity. Nucleic acid or amino acid sequences may be compared using a BLAST algorithm. Furthermore the identity between two or more sequences may be determined by aligning the sequences together and determining the % identity between the sequences. Alignment may be carried out using the BLAST Algorithm (for example as available through GenBank; URL: ncbi.nlm.nih.gov/cgi-bin/BLAST/ using default parameters: Program: blastn; Database: nr; Expect 10; filter: default; Alignment: pairwise; Query genetic Codes: Standards(1)), or BLAST2 through EMBL URL: emb1-heidelberg.de/Services/ index.html using default parameters: Matrix BLOSUM62; Filter: default, echofilter: on, Expect: 10, cutoff: default; Strand: both; Descriptions: 50, Alignments: 50; or FASTA, using default parameters), or by manually comparing the sequences and calculating the % identity.

The present invention describes, but is not limited to, the cloning of a nucleic acid encoding HA into a plant expression vector, and the production of influenza VLPs from the plant, suitable for vaccine production. Examples of such nucleic acids include, for example, but are not limited to, an influenza A/New Caledonia/20/99 (H1N1) virus HA (e.g. SEQ ID NO: 61), an HA from A/California/04/09 (SEQ ID NO: 127), an HA from A/Indonesia/5/05 sub-type (H5N1) (e.g. SEQ ID NO: 60), A/Brisbane/59/2007 (H1N1) (e.g. SEQ ID NO: 36, 48,62), A/Solomon Islands/3/2006 (H1N1) (e.g. SEQ ID NO: 37, 49, 63), A/Singapore/1/57 (H2N2) (e.g. SEQ ID NO: 42, 54, 64), A/Anhui/1/2005 (H5N1) (e.g. SEQ ID NO: 43, 55, 65), A/Vietnam/1194/2004 (H5N1) (e.g. SEQ ID NO: 44, 56, 66), A/Teal/Hong Kong/W312/97 (H6N1) (e.g. SEQ ID NO: 45, 57, 67), A/Hong Kong/1073/99 (H9N2) (e.g. SEQ ID NO: 47, 59, 68), A/Brisbane/10/2007 (H3N2) (e.g. SEQ ID NO: 38, 50, 69), A/Wisconsin/67/2005 (H3N2) (e.g. SEQ ID NO: 39, 51, 70), A/Equine/Prague/56 (H7N7) (e.g. SEQ ID NO: 46, 58, 71), B/Malaysia/2506/2004 (e.g. SEQ ID NO: 40, 52, 72), B/Florida/4/2006 (e.g. SEQ ID NO: 41, 53, 73). The corresponding clone or construct numbers for these strains is provided in Table 1. Nucleic acid sequences corresponding to SEQ ID NOs: 36-47 comprise a plastocyanin upstream and operatively linked to the coding sequence of the HA for each of the types or subtypes, as illustrated in Figures 28-39. Nucleic acid sequences corresponding to SEQ ID NO: 60-73 comprise an HA expression cassette comprising alfalfa plastocyanin promoter and 5' UTR, hemagglutinin coding sequence of an HA, alfalfa plastocyanin 3' UTR and terminator sequences, as illustrated in Figures 51-64.

The VLPs may also be used to produce reagents comprised of recombinant influenza structural proteins that self-assemble into functional and immunogenic homotypic macromolecular protein structures, including subviral influenza particles and influenza VLP, in transformed hosts cells, for example plant cells or insect cells.

Therefore, the invention provides for VLPs, and a method for producing viral VLPs in a plant expression system, from the expression of a single envelope protein. The VLPs may be influenza VLPs, or VLPs produced from other plasma membrane-derived virus including, but not limited to, Measles, Ebola, Marburg, and HIV.

Proteins from other enveloped viruses, for example but not limited to Filoviridae (e.g. Ebola virus, Marburg virus, or the like), Paramyxoviridae (e.g. Measles virus, Mumps virus, Respiratory syncytial virus, pneumoviruses, or the like), Retroviridae (e.g. Human Immunodeficiency Virus-1, Human Immunodeficiency Virus-2, Human T-Cell Leukemia Virus-1, or the like), Flaviviridae (e.g. West Nile Encephalitis, Dengue virus, Hepatitis C virus, yellow fever virus, or the like), Bunyaviridae (e.g. Hantavirus or the like), Coronaviridae (e.g. coronavirus, SARS, or the like), as would be known to those of skill in the art, may also be used. Non limiting examples of antigens that may be expressed in plasma membrane derived viruses include, the capsid protein of HIV - p24; HIV glycoproteins gp120 or gp41, Filovirus proteins including VP30 or VP35 of Ebolavirus or Gp/SGP of Marburg virus or the H protein or F protein of the Measles paramyxovirus. For example, P24 of HIV (e.g. GenBank reference gi: 19172948) is the protein obtained by translation and cleavage of the *gag* sequence of the HIV virus genome (e.g. GenBank reference gi:9629357); gp 120 and gp41 of HIV are glycoproteins obtained by translation and cleavage of the gp160 protein (e.g. GenBank reference gi:9629363), encoded by *env* of the HIV virus genome. VP30 of Ebolavirus (GenPept Reference gi: 55770813) is the protein obtained by translation of the *vp30* sequence of the Ebolavirus genome (e.g. GenBank Reference gi:55770807) ; VP35 of Ebolavirus (GenPept Reference gi:55770809) is the protein obtained by translation of the *vp35* sequence of the Ebolavirus genome. Gp/SGP of Marburg virus (GenPept Reference gi:296965) is the protein obtained by translation of the (sequence) of the Marburg virus genome (GenBank Reference gi:158539108). H protein (GenPept Reference gi: 9626951) is the protein of the H sequence of the Measles virus genome (GenBank Reference gi: 9626945); F protein (GenPept reference gi: 9626950) is the protein of the F sequence of the Measles virus genome.

However, other envelope proteins may be used within the methods of the present invention as would be know to one of skill in the art.

The invention, therefore, provides for a nucleic acid molecule comprising a sequence encoding HIV-p24, HIV-gp120, HIV-gp41, Ebolavirus-VP30, Ebolavirus-VP35, Marburg virus Gp/SGP, Measles virus-H protein or -F protein. The nucleic acid molecule may be operatively linked to a regulatory region active in an insect, yeast or plant cell, or in a particular plant tissue.

The present invention further provides the cloning of a nucleic acid encoding an HA, for example but not limited to, human influenza A/Indonesia/5/05 virus HA (HSN1) or an HA from influenza strain A/California/04/09 into a plant or insect expression vector (e.g. baculovirus expression vector) and production of influenza vaccine candidates or reagents comprised of recombinant influenza structural proteins that self-assemble into functional and immunogenic homotypic macromolecular protein structures, including subviral influenza particles and influenza VLP, in transformed plant cells or transformed insect cells.

The nucleic acid encoding the HA of influenza subtypes, for example but not limited to, A/New Caledonia/20/99 (H1N1), A/California/04/09 (H1N1) A/Indonesia/5/05 sub-type (H5N1), A/Brisbane/59/2007 (H1N1), A/Solomon Islands/3/2006 (H1N1), A/Singapore/1/57 (H2N2), A/Anhui/1/2005 (H5N1), A/Vietnam/1194/2004 (H5N1), A/Teal/Hong Kong/W312/97 (H6N1), A/Hong Kong/1073/99 (H9N2), A/Brisbane/10/2007 (H3N2), A/Wisconsin/67/2005 (H3N2), A/Equine/Prague/56 (H7N7), B/Malaysia/2506/2004, B/Florida/4/2006 may be expressed, for example, using a Baculovirus Expression System in an appropriate cell line, for example, *Spodoptera frugiperda* cells (e.g. Sf-9 cell line; ATCC PTA-4047). Other insect cell lines may also be used.

The nucleic acid encoding the HA may, alternately, be expressed in a plant cell, or in a plant. The nucleic acid encoding HA may be synthesized by reverse transcription and polymerase chain reaction (PCR) using HA RNA. As an example, the RNA may be isolated from human influenza A/New Caledonia/20/99 (H1N1) virus or human influenza A/Indonesia/5/05 (H5N1) virus, or other influenza viruses e.g. A/California/04/09 (H1N1), A/Brisbane/59/2007 (H1N1), A/Solomon Islands/3/2006 (H1N1), A/Singapore/1/57 (H2N2), A/Anhui/1/2005 (H5N1), A/Vietnam/1194/2004 (H5N1), A/Teal/Hong Kong/W312/97 (H6N1), A/Hong Kong/1073/99 (H9N2), A/Brisbane/10/2007 (H3N2), A/Wisconsin/67/2005 (H3N2), A/Equine/Prague/56 (H7N7), B/Malaysia/2506/2004, B/Florida/4/2006, or from cells infected with an influenza virus. For reverse transcription and PCR, oligonucleotide primers specific for HA RNA, for example but not limited to, human influenza A/New Caledonia/20/99 (H1N1) virus HA sequences or human influenza A/Indonesia/5/05 (H5N1) virus HA0 sequences, or HA sequences from influenza subtypes A/California/04/09 (H1N1), A/Brisbane/59/2007 (H1N1), A/Solomon Islands/3/2006 (H1N1), A/Singapore/1/57 (H2N2), A/Anhui/1/2005 (H5N1), A/Vietnam/1194/2004 (H5N1), A/Teal/Hong Kong/W312/97 (H6N1), A/Hong Kong/1073/99 (H9N2), A/Brisbane/10/2007 (H3N2), A/Wisconsin/67/2005 (H3N2), A/Equine/Prague/56 (H7N7), B/Malaysia/2506/2004, B/Florida/4/2006 may be used. Additionally, a nucleic acid encoding HA may be chemically synthesized using methods as would known to one of skill in the art.

The resulting cDNA copies of these genes may be cloned in a suitable expression vector as required by the host expression system. Examples of appropriate expression vectors for plants are described below, alternatively, baculovirus expression vector, for example, pFastBacl (InVitrogen), resulting in pFastBacl-based plasmids, using known methods, and information provided by the manufacturer's instructions nay be used.

The present invention is further directed to a gene construct comprising a nucleic acid encoding HA, as described above, operatively linked to a regulatory element that is operative in a plant. Examples of regulatory elements operative in a plant cell and that may be used in accordance with the present invention include but are not limited to a plastocyanin regulatory region (US 7,125,978; which is incorporated herein by reference), or a regulatory region of Ribulose 1,5-bisphosphate carboxylase/oxygenase (RuBisCO; US 4,962,028; which is incorporated herein by reference), chlorophyll a/b binding protein (CAB; Leutwiler et al; 1986; which is incorporated herein by reference), ST-LS1 (associated with the oxygen-evolving complex of photosystem II and described by Stockhaus et al.1987, 1989; which is incorporated herein by reference). An example of a plastocyanin regulatory region is a sequence comprising nucleotides 10-85 of SEQ ID NO: 36, or a similar region of any one of SEQ ID NOS: 37-47. A regulatory element or regulatory region may enhance translation of a nucleotide sequence to which is it operatively linked - the nucleotide sequence may encode a protein or polypeptide. Another example of a regulatory region is that derived from the untranslated regions of the Cowpea Mosaic Virus (CPMV), which may be used to preferentially translate the nucleotide sequence to which it is operatively linked. This CPMV regulatory region comprises a CMPV-HT system - see, for example, Sainsbury et al, 2008, Plant Physiology 148: 1212-1218.

If the construct is expressed in an insect cell, examples of regulatory elements operative in an insect cell include but are not limited to the polyhedrin promoter (Possee and Howard 1987. Nucleic Acids Research 15:10233-10248), the gp64 promoter (Kogan et al, 1995. J Virology 69:1452-1461) and the like.

Therefore, an aspect of the invention provides for a nucleic acid comprising a regulatory region and a sequence encoding an influenza HA. The regulatory region may be a plastocyanin regulatory element, and the influenza HA may be selected from a group of influenza strains or subtypes, comprising A/California/04/09 (H1N1), A/New Caledonia/20/99 (H1N1), A/Indonesia/5/05 sub-type (H5N1), A/Brisbane/59/2007 (H1N1), A/Solomon Islands/3/2006 (H1N1), A/Singapore/1/57 (H2N2), A/Anhui/1/2005 (H5N1), A/Vietnam/1194/2004 (H5N1), A/Teal/Hong Kong/W312/97 (H6N1), A/Hong Kong/1073/99 (H9N2), A/Brisbane/10/2007 (H3N2), A/Wisconsin/67/2005 (H3N2), A/Equine/Prague/56 (H7N7), B/Malaysia/2506/2004, B/Florida/4/2006. Nucleic acid sequences comprising a plastocyanin regulatory element and an influenza HA are exemplified herein by SEQ ID NOs: 36-47.

It is known that there may be sequence differences in the sequence of influenza hemagglutinin amino acids sequences, or the nucleic acids encoding them, when influenza virus is cultured in eggs, or mammalian cells, (e.g. MDCK cells) or when isolated from an infected subject. Non-limiting examples of such differences are illustrated herein, including Example 18. Furthermore, as one of skill in the art would realize, additional variation may be observed within influenza hemagglutinins obtained from new strains as additional mutations continue to occur. Due to the known sequence variability between different influenza hemagglutinins, the present invention includes VLPs that may be made using any influenza hemagglutin provided that when expressed in a host as described herein, the influenza hemagglutin forms a VLP.

Sequence alignments and consensus sequences may be determined using any of several software packages known in the art, for example MULTALIN (F. CORPET, 1988, Nucl. Acids Res., 16 (22), 10881-10890), or sequences may be aligned manually and similarities and differences between the sequences determined.

The structure of hemagglutinins is well-studied and the structures are known to be highly conserved. When hemagglutinin structures are superimposed, a high degree of structural conservation is observed (rmsd <2A). This structural conservation is observed even though the amino acid sequence may vary in some positions (see, for example, Skehel and Wiley, 2000 Ann Rev Biochem 69:531-69; Vaccaro et al 2005). Regions of hemagglutinins are also well-conserved, for example:
- Structural domains: The HA0 polyprotein is cleaved to provide mature HA. HA is a homotrimer with each monomer comprising a receptor binding domain (HA1) and a membrane-anchoring domain (HA2) linked by a single disulphide bond; the N-terminal 20 residues of the HA2 subunit may also be referred to as the HA fusion domain or sequence. A 'tail' region (internal to the membrane envelope) is also present. Each hemagglutinin comprises these regions or domains. Individual regions or domains are typically conserved in length.
- All hemagglutinins contain the same number and position of intra- and inter-molecular disulfide bridges. The quantity and position on the amino acid sequence of the cysteines that participate in disulfide bridge network is conserved among the HAs. Examples of structures illustrating the characteristic intra- and intermolecular disulfide bridges and other conserved amino acids and their relative positions are described in, for example, Gamblin et al 2004 (Science 303:1838-1842). Exemplary structures and sequences include 1RVZ, 1RVX, 1RVT, 1RV0, 1RUY, 1RU7, available from the Protein Data Bank (Berman et al. 2003. Nature Structural Biology 10:980; URL: rcsb.org)
- Cytoplasmic tail - the majority of hemagglutinins comprise 3 cysteines at conserved positions. One or more of these cysteines may be palmitoylated as a post-translational modification.

Amino acid variation is tolerated in hemagglutinins of influenza viruses. This variation provides for new strains that are continually identified. Infectivity between the new strains may vary. However, formation of hemagglutinin trimers, which subsequently form VLPs is maintained. The present invention, therefore, provides for a hemagglutinin amino acid sequence, or a nucleic acid encoding a hemagglutinin amino acid sequence, that forms VLPs in a plant, and includes known sequences and variant sequences that may develop.

Figure 65 illustrates an example of such known variation. This figure shows a consensus amino acid sequence (SEQ ID NO: 74) for HA of the following H1N1 strains:
A/New Caledonia/20/99 (H1N1) (encoded by SEQ ID NO: 33),
A/Brisbane/59/2007 (H1N1) (SEQ ID NO: 48),
A/Solomon Islands/3/2006 (H1N1) (SEQ ID NO: 49) and

SEQ ID NO: 9. X1 (position 3) is A or V; X2 (position 52) is D or N; X3 (position 90) is K or R; X4 (position 99) is K or T; X5 (position 111) is Y or H; X6 (position 145) is V or T; X7 (position 154) is E or K; X8 (position 161) is R or K; X9 (position 181) is V or A; X10 (position 203) is D or N; X11 (position 2o5) is R or K; X12 (position 210) is T or K; X13 (position 225) is R or K; X14 (position 268) is W or R; X15 (position 283) is T or N; X16 (position 290) is E or K; X17 (position 432) is I or L; X18 (position 489) is N or D.

As another example of such variation, a sequence alignment and consensus sequence for HA of A/New Caledonia/20/99 (H1N1) (encoded by SEQ ID NO: 33), ABrisbane/59/2007 (H1N1) (SEQ ID NO: 48), A/Solomon Islands/3/2006 (H1N1) (SEQ ID NO: 49), A/PuertoRico/8/34 (H1N1) and SEQ ID NO: 9 is shown below in Table 3.

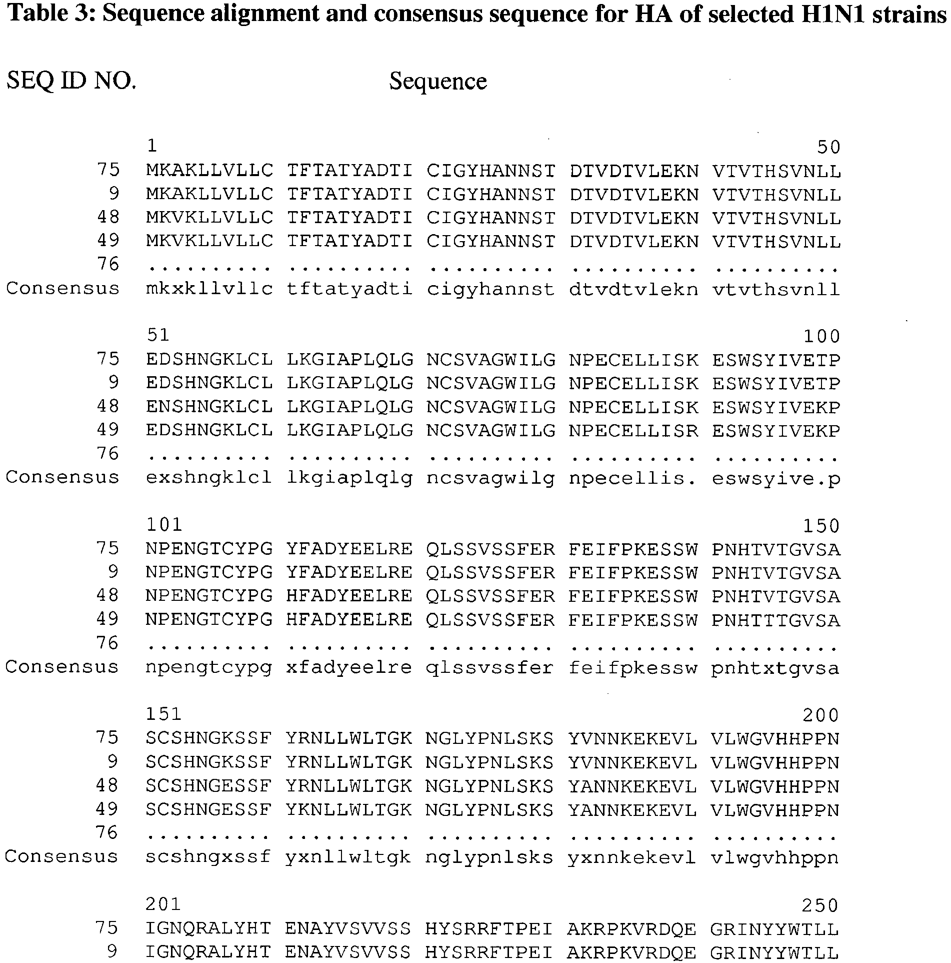

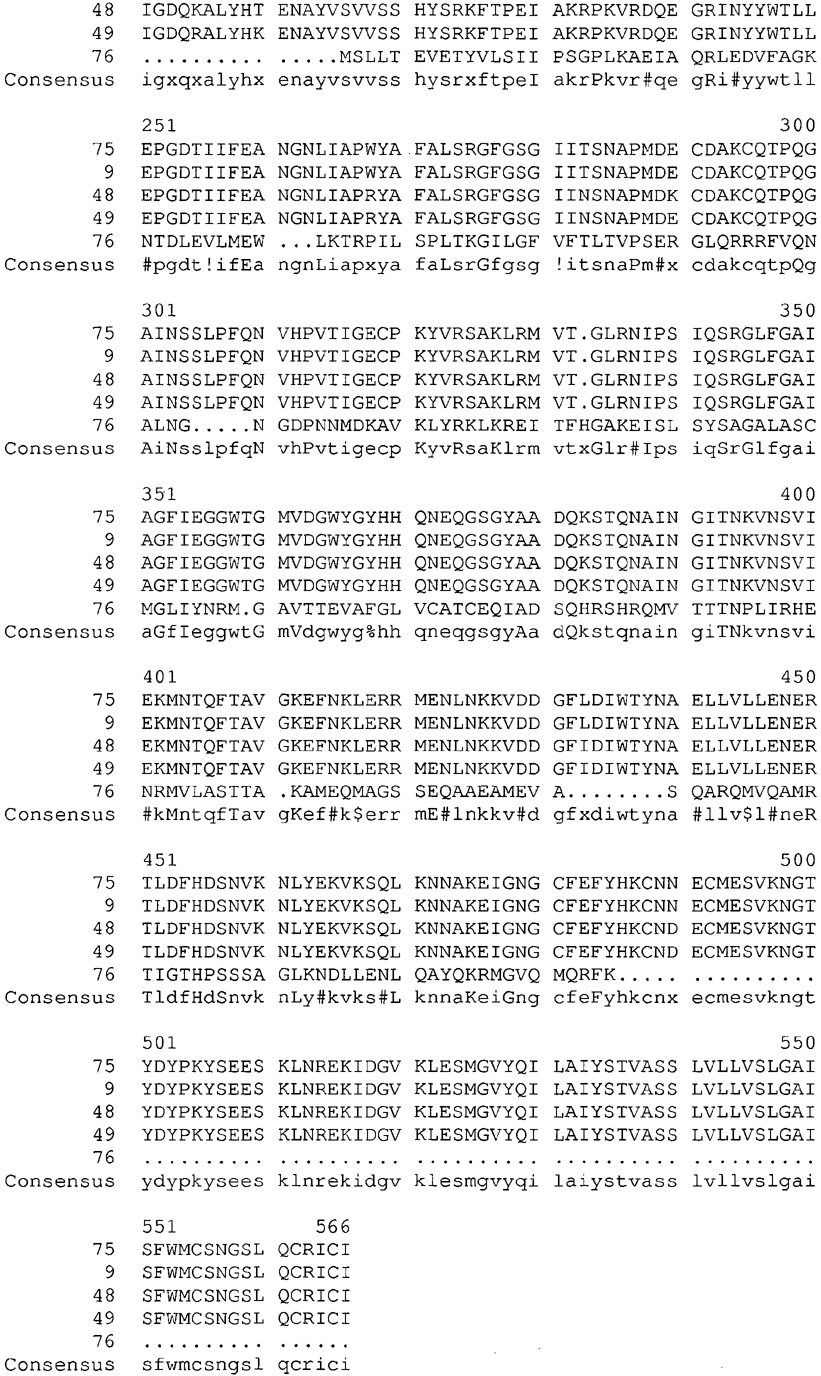

The consensus sequence indicates in upper case letters amino acids common to all sequences at a designated position; lower case letters indicate amino acids common to at least half, or a majority of the sequences; the symbol ! is any one of I or V; the symbol $ is any one of L or M; the symbol % is any one of F or Y; the symbol # is any one of N, D, Q, E,B or Z; the symbol "." is no amino acid (e.g. a deletion); X at position 3 is any one of A or V; X at position 52 is any one of E or N; X at position 90 is K or R; X at position 99 is T or K; X at position 111 is any one of Y or H; X at position 145 is any one of V or T; X at position 157 is K or E;X at position 162 is R or K; X at position 182 is V or A; X at position 203 is N or D; X at position 205 is R or K; X at position 210 is T or K; X at position 225 is K or Y; X at position 333 is H or a deletion; X at position 433 is I or L; X at position 49) is N or D.

As another example of such variation, a sequence alignment and consensus sequence for HA of A/Anhui/1/2005 (H5N1) (SEQ ID NO: 55), A/Vietnam/1194/2004 (H5N1) and A/Indonesia/5/2006 (H5N1) (SEQ ID NO: 10) is shown below in Table 4.

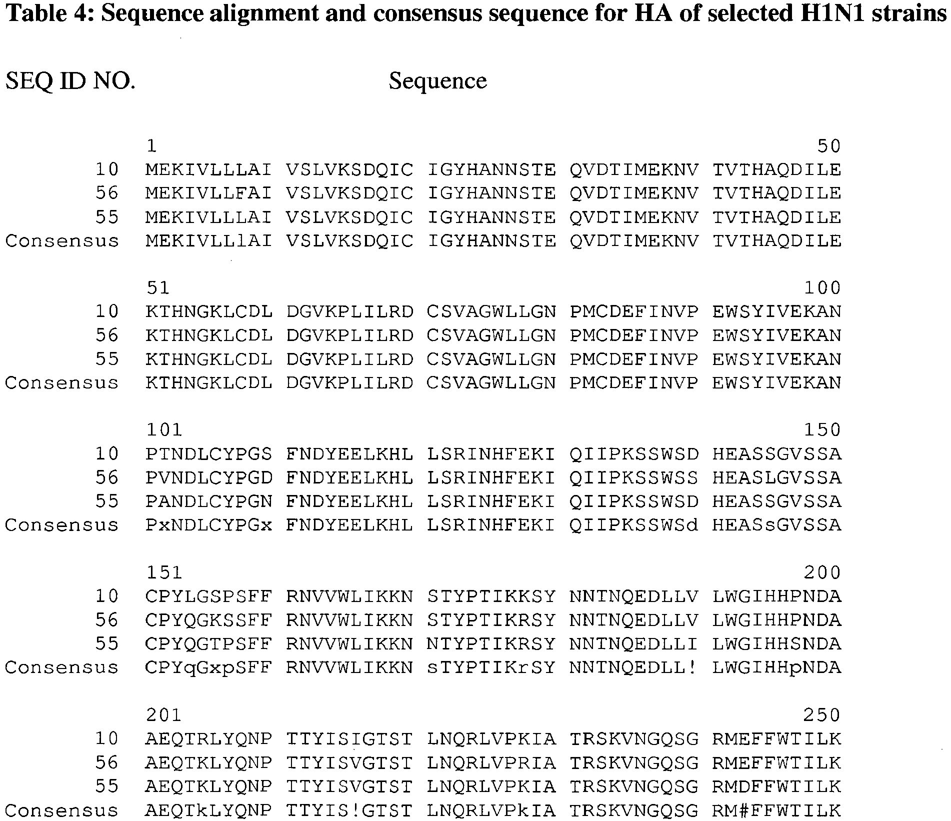

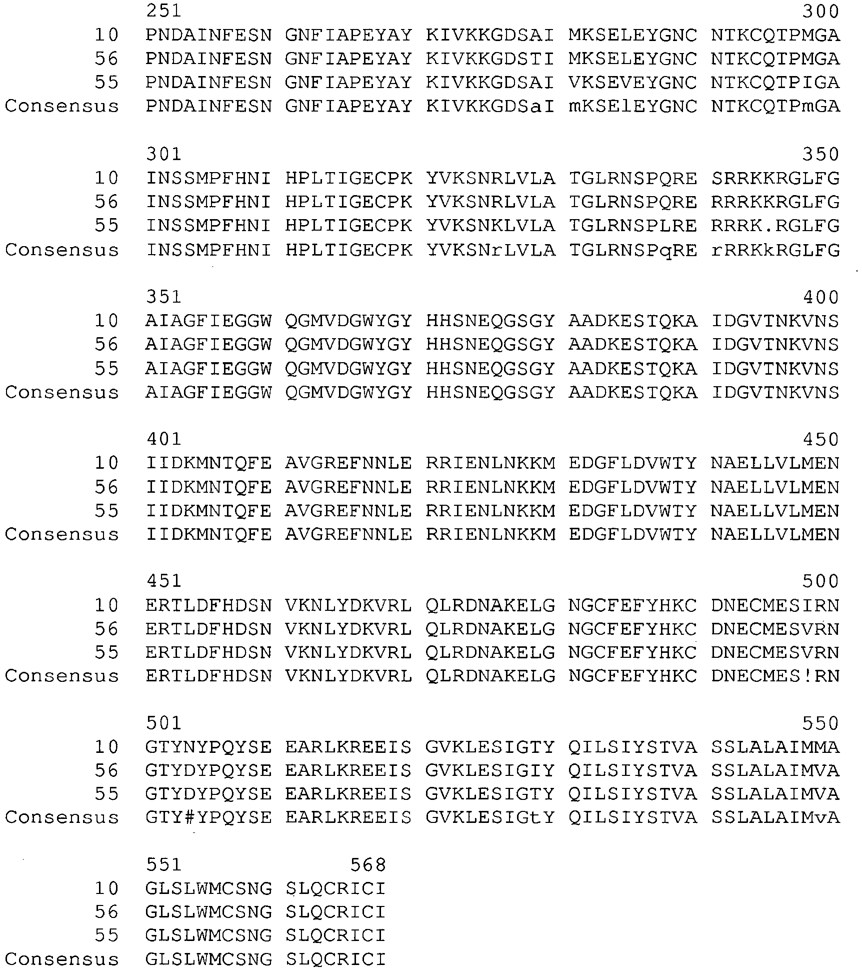

The consensus sequence indicates in upper case letters amino acids common to all sequences at a designated position; lower case letters indicate amino acids common to at least half, or a majority of the sequences; the symbol ! is any one of I or V; the symbol $ is any one of L or M; the symbol % is any one of F or Y; the symbol # is any one of N, D, Q, E,B or Z; X at position 102 is any of T, V or A; X t position 110 is any of S, D or N; X at position 156 is any of S, K or T.

The above-illustrated and described alignments and consensus sequences are non-limiting examples of variants in hemagglutinin amino acid sequences that may be used in various embodiments of the invention for the production of VLPs in a plant.

A nucleic acid encoding an amino acid sequence may be easily determined, as the codons for each amino acid are known in the art. Provision of an amino acid sequence, therefore, teaches the degenerate nucleic acid sequences that encode it. The present invention, therefore, provides for a nucleic acid sequence encoding the hemagglutinin of those influenza strains and subtypes disclosed herein (e.g. A/California/04/09 (H1N1), A/New Caledonia/20/99 (HIN1)A/Indonesia/5/2006 (H5N1), A/chicken/New York/1995, A/herring gull/DE/677/88 (H2N8), A/Texas/32/2003, A/mallard/MN/33/00, A/duck/Shanghai/1/2000, A/northern pintail/TX/828189/02, A/Turkey/Ontario/6118/68(H8N4), A/shoveler/Iran/G54/03, A/chicken/Germany/N/1949(H10N7), A/duck/England/56(H11N6), A/duck/Alberta/60/76(H12N5), A/Gull/Maryland/704/77(H13N6), A/Mallard/Gurjev/263/82, A/duck/Australia/341/83 (H15N8), A/black-headed gull/Sweden/5/99(H16N3), B/Lee/40, C/Johannesburg/66, A/PuertoRico/8/34 (H1N1), ABrisbane/59/2007 (H1N1), A/Solomon Islands 3/2006 (H1N1), A/Brisbane 10/2007 (H3N2), A/Wisconsin/67/2005 (H3N2), B/Malaysia/2506/2004, B/Florida/4/2006, A/Singapore/1/57 (H2N2), A/Anhui/1/2005 (H5N1), A/Vietnam/1194/2004 (H5N1), A/Teal/HongKong/W312/97 (H6N1), A/Equine/Prague/56 (H7N7), A/HongKong/1073/99 (H9N2)), as well as the degerenate sequences that encode the above hemagglutinins.

Further, an amino acid sequence encoded by a nucleic acid may be easily determined, as the codon or codons for each amino acid are known. Provision of a nucleic acid, therefore, teaches an amino acid sequence encoded by it. The invention, therefore, provides for amino acid sequences of the hemagglutinin of those influenza strains and subtypes disclosed herein those disclosed herein (e.g. A/California/04/09 (H1N1), A/New Caledonia/20/99 (H1N1)A/Indonesia/5/2006 (H5N1), A/chicken/New York/1995, A/herring gull/DE/677/88 (H2N8), A/Texas/32/2003, A/mallard/MN/33/00, A/duck/Shanghai/1/2000, A/northern pintail/TX/828189/02, A/Turkey/Ontario/6118/68(H8N4), A/shoveler/Iran/G54/03, A/chicken/Germany/N/1949(H10N7), A/duck/England/56(H11N6), A/duck/Alberta/60/76(H12N5); A/Gull/Maryland/704/77(H13N6), A/Mallard/Gurjev/263/82, A/duck/Australia/341/83 (H15N8), A/black-headed gull/Sweden/5/99(H16N3), B/Lee/40, C/Johannesburg/66, A/PuertoRico/8/34 (H1N1), A/Brisbane/59/2007 (H1N1), A/Solomon Islands 3/2006 (H1N1), A/Brisbane 10/2007 (H3N2), A/Wisconsin/67/2005 (H3N2), B/Malaysia/2506/2004, B/Florida/4/2006, A/Singapore/1/57 (H2N2), A/Anhui/1/2005 (H5N1), A/Vietnam/1194/2004 (H5N1), A/Teal/HongKong/W312/97 (H6N1), A/Equine/Prague/56 (H7N7), A/HongKong/1073/99 (H9N2)).

In plants, influenza VLPs bud from the plasma membrane (see Example 5, and Figure 19) therefore the lipid composition of the VLPs reflects their origin. The VLPs produced according to the present invention comprise HA of one or more than one type or subtype of influenza, complexed with plant derived lipids. Plant lipids can stimulate specific immune cells and enhance the immune response induced. Plant membranes are made of lipids, phosphatidylcholine (PC) and phosphatidylethanolamine (PE), and also contain glycosphingolipids, saponins, and phytosterols. Additionally, lipid rafts are also found in plant plasma membranes - these microdomains are enriched in sphingolipids and sterols. In plants, a variety of phytosterols are known to occur, including stigmasterol, sitosterol, 24-methylcholesterol and cholesterol (Mongrand et al., 2004).

PC and PE, as well as glycosphingolipids can bind to CD1 molecules expressed by mammalian immune cells such as antigen-presenting cells (APCs) like dendritic cells and macrophages and other cells including B and T lymphocytes in the thymus and liver (Tsuji M,. 2006). CD1 molecules are structurally similar to major histocompatibility complex (MHC) molecules of class I and their role is to present glycolipid antigens to NKT cells (Natural Killer T cells). Upon activation, NKT cells activate innate immune cells such as NK cells and dendritic cells and also activate adaptive immune cells like the antibody-producing B cells and T-cells.

A variety of phytosterols may be found in a plasma membrane - the specific complement may vary depending on the species, growth conditions, nutrient resources or pathogen state, to name a few factors. Generally, beta-sitosterol is the most abundant phytosterol.

The phytosterols present in an influenza VLP complexed with a lipid bilayer, such as an plasma-membrane derived envelope may provide for an advantageous vaccine composition. Without wishing to be bound by theory, plant-made VLPs complexed with a lipid bilayer, such as a plasma-membrane derived envelope, may induce a stronger immune reaction than VLPs made in other expression systems, and may be similar to the immune reaction induced by live or attenuated whole virus vaccines.

Therefore, in some embodiments, the invention provides for a VLP complexed with a plant-derived lipid bilayer. In some embodiments the plant-derived lipid bilayer may comprise the envelope of the VLP.

The VLP produced within a plant may include an HA comprising plant-specific N-glycans. Therefore, this invention also provides for a VLP comprising HA having plant specific N-glycans.

Furthermore, modification of N-glycan in plants is known (see for example U.S. 60/944,344; which is incorporated herein by reference) and HA having modified N-glycans may be produced. HA comprising a modified glycosylation pattern, for example with reduced fucosylated, xylosylated, or both, fucosylated and xylosylated, N-glycans may be obtained, or HA having a modified glycosylation pattern may be obtained, wherein the protein lacks fucosylation, xylosylation, or both, and comprises increased galatosylation. Furthermore, modulation of post-translational modifications, for example, the addition of terminal galactose may result in a reduction of fucosylation and xylosylation of the expressed HA when compared to a wild-type plant expressing HA.

For example, which is not to be considered limiting, the synthesis of HA having a modified glycosylation pattern may be achieved by co-expressing the protein of interest along with a nucleotide sequence encoding beta-1.4galactosyltransferase (GalT), for example, but not limited to mammalian GalT, or human GalT however GalT from another sources may also be used. The catalytic domain of GalT may also be fused to a CTS domain (i.e. the cytoplasmic tail, transmembrane domain, stem region) of N-acetylglucosaminyl transferase (GNT1), to produce a GNT1-GalT hybrid enzyme, and the hybrid enzyme may be co-expressed with HA. The HA may also be co-expressed along with a nucleotide sequence encoding N-acetylglucosaminyltrasnferase III (GnT-III), for example but not limited to mammalian GnT-III or human GnT-III, GnT-III from other sources may also be used. Additionally, a GNT1-GnT-III hybrid enzyme, comprising the CTS of GNT1 fused to GnT-III may also be used.

Therefore the present invention also includes VLP's comprising HA having modified N-glycans.

Without wishing to be bound by theory, the presence of plant N-glycans on HA may stimulate the immune response by promoting the binding of HA by antigen presenting cells. Stimulation of the immune response using plant N glycan has been proposed by Saint-Jore-Dupas et al. (2007). Furthermore, the conformation of the VLP may be advantageous for the presentation of the antigen, and enhance the adjuvant effect of VLP when complexed with a plant derived lipid layer.

By "regulatory region", "regulatory element" or "promoter" it is meant a portion of nucleic acid typically, but not always, upstream of the protein coding region of a gene, which may be comprised of either DNA or RNA, or both DNA and RNA. When a regulatory region is active, and in operative association, or operatively linked, with a gene of interest, this may result in expression of the gene of interest. A regulatory element may be capable of mediating organ specificity, or controlling developmental or temporal gene activation. A "regulatory region" includes promoter elements, core promoter elements exhibiting a basal promoter activity, elements that are inducible in response to an external stimulus, elements that mediate promoter activity such as negative regulatory elements or transcriptional enhancers. "Regulatory region", as used herein, also includes elements that are active following transcription, for example, regulatory elements that modulate gene expression such as translational and transcriptional enhancers, translational and transcriptional repressors, upstream activating sequences, and mRNA instability determinants. Several of these latter elements may be located proximal to the coding region.

In the context of this disclosure, the term "regulatory element" or "regulatory region" typically refers to a sequence of DNA, usually, but not always, upstream (5') to the coding sequence of a structural gene, which controls the expression of the coding region by providing the recognition for RNA polymerase and/or other factors required for transcription to start at a particular site. However, it is to be understood that other nucleotide sequences, located within introns, or 3' of the sequence may also contribute to the regulation of expression of a coding region of interest. An example of a regulatory element that provides for the recognition for RNA polymerase or other transcriptional factors to ensure initiation at a particular site is a promoter element. Most, but not all, eukaryotic promoter elements contain a TATA box, a conserved nucleic acid sequence comprised of adenosine and thymidine nucleotide base pairs usually situated approximately 25 base pairs upstream of a transcriptional start site. A promoter element comprises a basal promoter element, responsible for the initiation of transcription, as well as other regulatory elements (as listed above) that modify gene expression.

There are several types of regulatory regions, including those that are developmentally regulated, inducible or constitutive. A regulatory region that is developmentally regulated, or controls the differential expression of a gene under its control, is activated within certain organs or tissues of an organ at specific times during the development of that organ or tissue. However, some regulatory regions that are developmentally regulated may preferentially be active within certain organs or tissues at specific developmental stages, they may also be active in a developmentally regulated manner, or at a basal level in other organs or tissues within the plant as well. Examples of tissue-specific regulatory regions, for example see-specific a regulatory region, include the napin promoter, and the cruciferin promoter (Rask et al., 1998, J. Plant Physiol. 152: 595-599; Bilodeau et al., 1994, Plant Cell 14: 125-130). An example of a leaf-specific promoter includes the plastocyanin promoter (Figure 1b; US 7,125,978, which is incorporated herein by reference).

An inducible regulatory region is one that is capable of directly or indirectly activating transcription of one or more DNA sequences or genes in response to an inducer. In the absence of an inducer the DNA sequences or genes will not be transcribed. Typically the protein factor that binds specifically to an inducible regulatory region to activate transcription may be present in an inactive form, which is then directly or indirectly converted to the active form by the inducer. However, the protein factor may also be absent. The inducer can be a chemical agent such as a protein, metabolite, growth regulator, herbicide or phenolic compound or a physiological stress imposed directly by heat, cold, salt, or toxic elements or indirectly through the action of a pathogen or disease agent such as a virus. A plant cell containing an inducible regulatory region may be exposed to an inducer by externally applying the inducer to the cell or plant such as by spraying, watering, heating or similar methods. Inducible regulatory elements may be derived from either plant or non-plant genes (e.g. Gatz, C. and Lenk, I.R.P., 1998, Trends Plant Sci. 3, 352-358; which is incorporated by reference). Examples, of potential inducible promoters include, but not limited to, tetracycline-inducible promoter (Gatz, C.,1997, Ann. Rev. Plant Physiol. Plant Mol. Biol. 48, 89-108; which is incorporated by reference), steroid inducible promoter (Aoyama, T. and Chua, N.H.,1997, Plant J. 2, 397-404; which is incorporated by reference) and ethanol-inducible promoter (Salter, M.G., et al, 1998, Plant Journal 16, 127-132; Caddick, M.X., et al.,1998, Nature Biotech. 16, 177-180, which are incorporated by reference) cytokinin inducible IB6 and CKI1 genes (Brandstatter, I. and Kieber, J.J.,1998, Plant Cell 10, 1009-1019; Kakimoto, T., 1996, Science 274, 982-985; which are incorporated by reference) and the auxin inducible element, DR5 (Ulmasov, T., et al., 1997, Plant Cell 9, 1963-1971; which is incorporated by reference).

A constitutive regulatory region directs the expression of a gene throughout the various parts of a plant and continuously throughout plant development. Examples of known constitutive regulatory elements include promoters associated with the CaMV 35S transcript. (Odell et al., 1985, Nature, 313: 810-812), the rice actin 1 (Zhang et al, 1991, Plant Cell, 3: 1155-1165), actin 2 (An et al., 1996, Plant J., 10: 107-121), or tms 2 (U.S. 5,428,147, which is incorporated herein by reference), and triosephosphate isomerase 1 (Xu et. al., 1994, Plant Physiol. 106: 459-467) genes, the maize ubiquitin 1 gene (Cornejo et al, 1993, Plant Mol. Biol. 29: 637-646), the *Arabidopsis* ubiquitin 1 and 6 genes (Holtorf et al, 1995, Plant Mol. Biol. 29: 637-646), and the tobacco translational initiation factor 4A gene (Mandel et al, 1995 Plant Mol. Biol. 29: 995-1004). The term "constitutive" as used herein does not necessarily indicate that a gene under control of the constitutive regulatory region is expressed at the same level in all cell types, but that the gene is expressed in a wide range of cell types even though variation in abundance is often observed. Constitutive regulatory elements may be coupled with other sequences to further enhance the transcription and/or translation of the nucleotide sequence to which they are operatively linked. For example, the CMPV-HT system (Sainsbury et al, 2008, Plant Physiology 148: 1212-1218) is derived from the untranslated regions of the Cowpea mosaic virus (COMV) and demonstrates enhanced translation of the associated coding sequence.

By "native" it is meant that the nucleic acid or amino acid sequence is naturally occurring, or "wild type".

By "operatively linked" it is meant that the particular sequences, for example a regulatory element and a coding region of interest, interact either directly or indirectly to carry out an intended function, such as mediation or modulation of gene expression. The interaction of operatively linked sequences may, for example, be mediated by proteins that interact with the operatively linked sequences.

The one or more than one nucleotide sequence of the present invention may be expressed in any suitable plant host that is transformed by the nucleotide sequence, or constructs, or vectors of the present invention. Examples of suitable hosts include, but are not limited to, agricultural crops including alfalfa, canola, *Brassica* spp., maize, *Nicotiana* spp., alfalfa, potato, ginseng, pea, oat, rice, soybean, wheat, barley, sunflower, cotton and the like.

The one or more chimeric genetic constructs of the present invention can further comprise a 3' untranslated region. A 3' untranslated region refers to that portion of a gene comprising a DNA segment that contains a polyadenylation signal and any other regulatory signals capable of effecting mRNA processing or gene expression. The polyadenylation signal is usually characterized by effecting the addition of polyadenylic acid tracks to the 3' end of the mRNA precursor. Polyadenylation signals are commonly recognized by the presence of homology to the canonical form 5' AATAAA-3' although variations are not uncommon. One or more of the chimeric genetic constructs of the present invention can also include further enhancers, either translation or transcription enhancers, as may be required. These enhancer regions are well known to persons skilled in the art, and can include the ATG initiation codon and adjacent sequences. The initiation codon must be in phase with the reading frame of the coding sequence to ensure translation of the entire sequence.

Non-limiting examples of suitable 3' regions are the 3' transcribed non-translated regions containing a polyadenylation signal of *Agrobacterium* tumor inducing (Ti) plasmid genes, such as the nopaline synthase (Nos gene) and plant genes such as the soybean storage protein genes, the small subunit of the ribulose-1, 5-bisphosphate carboxylase (ssRUBISCO; US 4,962,028; which is incorporated herein by reference) gene, the promoter used in regulating plastocyanin expression (Pwee and Gray 1993; which is incorporated herein by reference). An example of a plastocyanin promoter is described in US 7,125,978 (which is incorporated herein by reference)

As described herein, promoters comprising enhancer sequences with demonstrated efficiency in leaf expression, have been found to be effective in transient expression. Without wishing to be bound by theory, attachment of upstream regulatory elements of a photosynthetic gene by attachment to the nuclear matrix may mediate strong expression. For example up to -784 from the translation start site of the pea plastocyanin gene may be used mediate strong reporter gene expression.

To aid in identification of transformed plant cells, the constructs of this invention may be further manipulated to include plant selectable markers. Useful selectable markers include enzymes that provide for resistance to chemicals such as an antibiotic for example, gentamycin, hygromycin, kanamycin, or herbicides such as phosphinothrycin, glyphosate, chlorosulfuron, and the like. Similarly, enzymes providing for production of a compound identifiable by colour change such as GUS (beta-glucuronidase), or luminescence, such as luciferase or GFP, may be used.

Also considered part of this invention are transgenic plants, plant cells or seeds containing the chimeric gene construct of the present invention. Methods of regenerating whole plants from plant cells are also known in the art. In general, transformed plant cells are cultured in an appropriate medium, which may contain selective agents such as antibiotics, where selectable markers are used to facilitate identification of transformed plant cells. Once callus forms, shoot formation can be encouraged by employing the appropriate plant hormones in accordance with known methods and the shoots transferred to rooting medium for regeneration of plants. The plants may then be used to establish repetitive generations, either from seeds or using vegetative propagation techniques. Transgenic plants can also be generated without using tissue cultures.

Also considered part of this invention are transgenic plants, trees, yeast, bacteria, fungi, insect and animal cells containing the chimeric gene construct comprising a nucleic acid encoding recombinant HA0 for VLP production, in accordance with the present invention.

The regulatory elements of the present invention may also be combined with coding region of interest for expression within a range of host organisms that are amenable to transformation, or transient expression. Such organisms include, but are not limited to plants, both monocots and dicots, for example but not limited to corn, cereal plants, wheat, barley, oat, *Nicotiana* spp, *Brassica* spp, soybean, bean, pea, alfalfa, potato, tomato, ginseng, and *Arabidopsis.*

Methods for stable transformation, and regeneration of these organisms are established in the art and known to one of skill in the art. The method of obtaining transformed and regenerated plants is not critical to the present invention.

By "transformation" it is meant the stable interspecific transfer of genetic information (nucleotide sequence) that is manifested genotypically, phenotypically or both. The interspecific transfer of genetic information from a chimeric construct to a host may be heritable and the transfer of genetic information considered stable, or the transfer may be transient and the transfer of genetic information is not inheritable.

By the term "plant matter", it is meant any material derived from a plant. Plant matter may comprise an entire plant, tissue, cells, or any fraction thereof. Further, plant matter may comprise intracellular plant components, extracellular plant components, liquid or solid extracts of plants, or a combination thereof. Further, plant matter may comprise plants, plant cells, tissue, a liquid extract, or a combination thereof, from plant leaves, stems, fruit, roots or a combination thereof. Plant matter may comprise a plant or portion thereof which has not been subjected to any processing steps. A portion of a plant may comprise plant matter. However, it is also contemplated that the plant material may be subjected to minimal processing steps as defined below, or more rigorous processing, including partial or substantial protein purification using techniques commonly known within the art including, but not limited to chromatography, electrophoresis and the like.

By the term "minimal processing" it is meant plant matter, for example, a plant or portion thereof comprising a protein of interest which is partially purified to yield a plant extract, homogenate, fraction of plant homogenate or the like (i.e. minimally processed). Partial purification may comprise, but is not limited to disrupting plant cellular structures thereby creating a composition comprising soluble plant components, and insoluble plant components which may be separated for example, but not limited to, by centrifugation, filtration or a combination thereof. In this regard, proteins secreted within the extracellular space of leaf or other tissues could be readily obtained using vacuum or centrifugal extraction, or tissues could be extracted under pressure by passage through rollers or grinding or the like to squeeze or liberate the protein free from within the extracellular space. Minimal processing could also involve preparation of crude extracts of soluble proteins, since these preparations would have negligible contamination from secondary plant products. Further, minimal processing may involve aqueous extraction of soluble protein from leaves, followed by precipitation with any suitable salt. Other methods may include large scale maceration and juice extraction in order to permit the direct use of the extract.

The plant matter, in the form of plant material or tissue may be orally delivered to a subject. The plant matter may be administered as part of a dietary supplement, along with other foods, or encapsulated. The plant matter or tissue may also be concentrated to improve or increase palatability, or provided along with other materials, ingredients, or pharmaceutical excipients, as required.

Examples of a subject or target organism that the VLPs of the present invention may be administered to include, but are not limited to, humans, primates, birds, water fowl, migratory birds, quail, duck, geese, poultry, chicken, swine, sheep, equine, horse, camel, canine, dogs, feline, cats, tiger, leopard, civet, mink, stone marten, ferrets, house pets, livestock, rabbits, mice, rats, guinea pigs or other rodents, seal, whale and the like. Such target organisms are exemplary, and are not to be considered limiting to the applications and uses of the present invention.

It is contemplated that a plant comprising the protein of interest, or expressing the VLP comprising the protein of interest may be administered to a subject or target organism, in a variety of ways depending upon the need and the situation. For example, the protein of interest obtained from the plant may be extracted prior to its use in either a crude, partially purified, or purified form. If the protein is to be purified, then it may be produced in either edible or non-edible plants. Furthermore, if the protein is orally administered, the plant tissue may be harvested and directly feed to the subject, or the harvested tissue may be dried prior to feeding, or an animal may be permitted to graze on the plant with no prior harvest taking place. It is also considered within the scope of this invention for the harvested plant tissues to be provided as a food supplement within animal feed. If the plant tissue is being feed to an animal with little or not further processing it is preferred that the plant tissue being administered is edible.

Post-transcriptional gene silencing (PTGS) may be involved in limiting expression of transgenes in plants, and co-expression of a suppressor of silencing from the potato virus Y (HcPro) may be used to counteract the specific degradation of transgene mRNAs (Brigneti et al., 1998). Alternate suppressors of silencing are well known in the art and may be used as described herein (Chiba et al., 2006, Virology 346:7-14; which is incorporated herein by reference), for example but not limited to, TEV-p1/HC-Pro (Tobacco etch virus-p1/HC-Pro), BYV -p21, p19 of Tomato bushy stunt virus (TBSV p19), capsid protein of Tomato crinkle virus (TCV -CP), 2b of Cucumber mosaic virus; CMV-2b), p25 of Potato virus X (PVX-p25), p11 of Potato virus M (PVM-p11), p11 of Potato virus S (PVS-p11), p16 of Blueberry scorch virus, (BScV -p16), p23 of Citrus tristeza virus (CTV-p23), p24 of Grapevine leafroll-associated virus-2, (GLRaV-2 p24), p10 of Grapevine virus A, (GVA-p10), p14 of Grapevine virus B (GVB-p14), p10 of Heracleum latent virus (HLV-p10), or p16 of Garlic common latent virus (GCLV-p16). Therefore, a suppressor of silencing, for example, but not limited to, HcPro, TEV -pl/HC-Pro, BYV-p21, TBSVp19, TCV-CP, CMV-2b, PVX-p25, PVM-p11, PVS-p11, BScV-p16, CTV-p23, GLRaV-2 p24, GBV-p14, HLV-p10, GCLV-p16 or GVA-p10, may be co-expressed along with the nucleic acid sequence encoding the protein of interest to further ensure high levels of protein production within a plant.

Furthermore, VLPs may be produced that comprise a combination of HA subtypes. For example, VLPs may comprise one or more than one HA from the subtype H1, H2, H3, H4, H5, H6, H7, H8, H9, H10, H11, H12, H13, H14, H15, H16, type B, or a combination thereof. Selection of the combination of HAs may be determined by the intended use of the vaccine prepared from the VLP. For example a vaccine for use in inoculating birds may comprise any combination of HA subtypes, while VLPs useful for inoculating humans may comprise subtypes one or more than one of subtypes H1, H2, H3, H5, H6, H7, H9 or B. However, other HA subtype combinations may be prepared depending upon the use of the VLP. In order to produce VLPs comprising combinations of HA subtypes, the desired HA subtype may be co-expressed within the same cell, for example a plant cell.

Furthermore, VLPs produced as described herein do not comprise neuraminidase (NA). However, NA may be co-expressed with HA should VLPs comprising HA and NA be desired.

Therefore, the present invention further includes a suitable vector comprising the chimeric construct suitable for use with either stable or transient expression systems. The genetic information may be also provided within one or more than one construct. For example, a nucleotide sequence encoding a protein of interest may be introduced in one construct, and a second nucleotide sequence encoding a protein that modifies glycosylation of the protein of interest may be introduced using a separate construct. These nucleotide sequences may then be co-expressed within a plant. However, a construct comprising a nucleotide sequence encoding both the protein of interest and the protein that modifies glycosylation profile of the protein of interest may also be used. In this case the nucleotide sequence would comprise a first sequence comprising a first nucleic acid sequence encoding the protein of interest operatively linked to a promoter or regulatory region, and a second sequence comprising a second nucleic acid sequence encoding the protein that modifies the glycosylation profile of the protein of interest, the second sequence operatively linked to a promoter or regulatory region.

By "co-expressed" it is meant that two, or more than two, nucleotide sequences are expressed at about the same time within the plant, and within the same tissue of the plant. However, the nucleotide sequences need not be expressed at exactly the same time. Rather, the two or more nucleotide sequences are expressed in a manner such that the encoded products have a chance to interact. For example, the protein that modifies glycosylation of the protein of interest may be expressed either before or during the period when the protein of interest is expressed so that modification of the glycosylation of the protein of interest takes place. The two or more than two nucleotide sequences can be co-expressed using a transient expression system, where the two or more sequences are introduced within the plant at about the same time under conditions that both sequences are expressed. Alternatively, a platform plant comprising one of the nucleotide sequences, for example the sequence encoding the protein that modifies the glycosylation profile of the protein of interest, may be transformed, either transiently or in a stable manner, with an additional sequence encoding the protein of interest. In this case, the sequence encoding the protein that modifies the glycosylation profile of the protein of interest may be expressed within a desired tissue, during a desired stage of development, or its expression may be induced using an inducible promoter, and the additional sequence encoding the protein of interest may be expressed under similar conditions and in the same tissue, to ensure that the nucleotide sequences are co-expressed.

The constructs of the present invention can be introduced into plant cells using Ti plasmids, Ri plasmids, plant virus vectors, direct DNA transformation, micro-injection, electroporation, infiltration, and the like. For reviews of such techniques see for example Weissbach and Weissbach, Methods for Plant Molecular Biology, Academy Press, New York VIII, pp. 421-463 (1988); Geierson and Corey, Plant Molecular Biology, 2d Ed. (1988); and Miki and Iyer, Fundamentals of Gene Transfer in Plants. In Plant Metabolism, 2d Ed. DT. Dennis, DH Turpin, DD Lefebrve, DB Layzell (eds), Addison-Wesley, Langmans Ltd. London, pp. 561-579 (1997). Other methods include direct DNA uptake, the use of liposomes, electroporation, for example using protoplasts, micro-injection, microprojectiles or whiskers, and vacuum infiltration. See, for example, Bilang, et al. (Gene 100: 247-250 (1991), Scheid et al. (Mol. Gen. Genet. 228: 104-112, 1991), Guerche et al. (Plant Science 52: 111-116, 1987), Neuhause et al. (Theor. Appl Genet. 75: 30-36, 1987), Klein et al., Nature 327: 70-73 (1987); Howell et al. (Science 208: 1265, 1980), Horsch et al. (Science 227: 1229-1231, 1985), DeBlock et al., Plant Physiology 91: 694-701, 1989), Methods for Plant Molecular Biology (Weissbach and Weissbach, eds., Academic Press Inc., 1988), Methods in Plant Molecular Biology (Schuler and Zielinski, eds., Academic Press Inc., 1989), Liu and Lomonossoff (J. Virol Meth, 105:343-348, 2002,), U.S. Pat. Nos. 4,945,050; 5,036,006; 5,100,792; 6,403,865; 5,625,136, (all of which are hereby incorporated by reference).

Transient expression methods may be used to express the constructs of the present invention (see Liu and Lomonossoff, 2002, Journal of Virological Methods, 105:343-348; which is incorporated herein by reference). Alternatively, a vacuum-based transient expression method, as described by Kapila et al. 1997 (incorporated herein by reference) may be used. These methods may include, for example, but are not limited to, a method of Agro-inoculation or Agro-infiltration, however, other transient methods may also be used as noted above. With either Agro-inoculation or Agro-infiltration, a mixture of *Agrobacteria* comprising the desired nucleic acid enter the intercellular spaces of a tissue, for example the leaves, aerial portion of the plant (including stem, leaves and flower), other portion of the plant (stem, root, flower), or the whole plant. After crossing the epidermis the *Agrobacterium* infect and transfer t-DNA copies into the cells. The t-DNA is episomally transcribed and the mRNA translated, leading to the production of the protein of interest in infected cells, however, the passage of t-DNA inside the nucleus is transient.

If the nucleotide sequence of interest encodes a product that is directly or indirectly toxic to the plant, then by using the method of the present invention, such toxicity may be reduced throughout the plant by selectively expressing the nucleotide sequence of interest within a desired tissue or at a desired stage of plant development. In addition, the limited period of expression resulting from transient expression may reduce the effect when producing a toxic product in the plant. An inducible promoter, a tissue-specific promoter, or a cell specific promoter, may be used to selectively direct expression of the sequence of interest.

The recombinant HA VLPs of the present invention can be used in conjunction with existing influenza vaccines, to supplement the vaccines, render them more efficacious, and to reduce the administration dosages necessary. As would be known to a person of skill in the art, the vaccine may be directed against one or more than one influenza virus. Examples of suitable vaccines include, but are not limited to, those commercially available from Sanofi-Pasteur, ID Biomedical, Merial, Sinovac, Chiron, Roche, MedImmune, GlaxoSmithKline, Novartis, Sanofi-Aventis, Serono, Shire Pharmaceuticals and the like.

If desired, the VLPs of the present invention may be admixed with a suitable adjuvant as would be known to one of skill in the art. Furthermore, the VLP may be used in a vaccine composition comprising an effective dose of the VLP for the treatment of a target organism, as defined above. Furthermore, the VLP produced according to the present invention may be combined with VLPs obtained using different influenza proteins, for example, neuraminidase (NA).

Therefore, the present invention provides a method for inducing immunity to influenza virus infection in an animal or target organism comprising administering an effective dose of a vaccine comprising one or more than one VLP. The vaccine may be administered orally, intradermally, intranasally, intramuscularly, intraperitoneally, intravenously, or subcutaneously.

Administration of VLPs produced according to the present invention is described in Example 6. Adminstration of plant-made H5 VLP resulted in a significantly higher response when compared to administration of soluble HA (see Figures 21A and 21B).

As shown in Figures 26A and 26 B a subject administered A/Indonesia/5/05 H5 VLPs is provided cross-protection to a challenge with influenza A/Turkey/582/06 (H5N1; "Turkey H5N1"). Administration of Indonesia H5 VLPs before challenge did not result in any loss of body mass. However in subject not administered H5 VLPs, but challenged with Turkey H5N1, exhibited significant loss of body mass, and several subject died.

These data, therefore, demonstrate that plant-made influenza VLPs comprising the H5 hemagglutinin viral protein induce an immune response specific for pathogenic influenza strains, and that virus-like particles may bud from a plant plasma membrane.

Therefore, the present invention provides a composition comprising an effective dose of a VLP comprising an influenza virus HA protein, one or more than one plant lipid, and a pharmaceutically acceptable carrier. The influenza virus HA protein may be H5 Indonesia/5/2006, A/Brisbane/50/2007, A/Solomon Islands 3/2006, A/Brisbane/10/2007, A/Wisconsin/67/2005, B/Malaysia/2506/2005, B/Florida/4/2006, A/Singapore/1/57, A/Anhui/1/2005, A/Vietnam/1194/2004, A/Teal/HongKong/W312/97, A/Equine/Prague/56, A/California/04/09 (H1N1) or A/ HongKong/1073/99. Also provided is a method of inducing immunity to an influenza virus infection in a subject. The method comprising administering the virus like particle comprising an influenza virus HA protein, one or more than one plant lipid, and a pharmaceutically acceptable carrier. The virus like particle may be administered to a subject orally, intradermally, intranasally, intramusclarly, intraperitoneally, intravenously, or subcutaneously.

Compositions according to various embodiments of the invention may comprise VLPs of two or more influenza strains or subtypes. "Two or more" refers to two, three, four, five, six, seven, eight, nine, 10 or more strains or subtypes. The strains or subtypes represented may be of a single subtype (e.g. all H1N1, or all H5N1), or may be a combination of subtypes. Exemplary subtype and strains include, but are not limited to, those disclosed herein (e.g. A/New Caledonia/20/99 (H1N1)A/Indonesia/5/2006 (H5N1), A/chicken/New York/1995, A/herring gull/DE/677/88 (H2N8), A/Texas/32/2003, A/mallard/MN/33/00, A/duck/Shanghai/1/2000, A/northem pintail/TX/828189/02, A/Turkey/Ontario/6118/68(H8N4), A/shoveler/Iran/G54/03, A/chicken/Germany/N/1949(H10N7), A/duck/England/56(H11N6), A/duck/Alberta/60/76(H12N5), A/Gull/Maryland/704/77(H13N6), A/Mallard/Gurjev/263/82, A/duck/Australia/341/83 (H15N8), A/black-headed gull/Sweden/5/99(H16N3), B/Lee/40, C/Johannesburg/66, A/PuertoRico/8/34 (H1N1), A/Brisbane/59/2007 (H1N1), A/Solomon Islands 3/2006 (H1N1), A/Brisbane 10/2007 (H3N2), A/Wisconsin/67/2005 (H3N2), B/Malaysia/2506/2004, B/Florida/4/2006, A/Singapore/1/57 (H2N2), A/Anhui/1/2005 (H5N1), A/Vietnam/1194/2004 (H5N1), A/Teal/HongKong/W312/97 (H6N1), A/Equine/Prague/56 (H7N7), A/California/04/09 (H1N1) or A/HongKong/1073/99 (H9N2)).

The choice of combination of strains and subtypes may depend on the geographical area of the subjects likely to be exposed to influenza, proximity of animal species to a human population to be immunized (e.g. species of waterfowl, agricultural animals such as swine, etc) and the strains they carry, are exposed to or are likely to be exposed to, predictions of antigenic drift within subtypes or strains, or combinations of these factors. Examples of combinations used in past years are available (see URL: who.int/csr/dieease/influenza/vaccine recommendations 1/en). Some or all of these strains may be employed in the combinations shown, or in other combinations, in the production of a vaccine composition.

More particularly, exemplary combinations may include VLPs from two or more strains or subtypes selected from the group comprising: A/California/04/09 (H1N1), A/Brisbane/59/2007 (H1N1), an A/Brisbane/59/2007 (H1N1)-like virus, A/Brisbane/10/2007 (H3N2), an A/Brisbane/10/2007 (H3N2)-like virus, B/Florida/4/2006 or an B/Florida/4/2006-like virus.

Another exemplary combination may include VLPs from two or more strains or subtypes selected from the group comprising A/Indonesia/5/2005, an A/Indonesia/5/2005-like virus, A/Vietnam/1194/2004, an A/Vietnam/1194/2004-like virus, A/Anhui/1/05, an A/Anhui/1/05-like virus, A/goose/Guiyang/337/2006, A/goose/Guiyang/337/2006 - like virus, A/chicken/Shanxi/2/2006, A/chicken/Shanxi/2/2006-like virus, A/California/04/09 (H1N1) or A/California/04/09 (H1N1) -like virus.

Another exemplary combination may include VLPs of A/Chicken/Italy/13474/99 (H7 type) or A/Chicken/British Columbia/04 (H7N3) strains of influenza.

Another exemplary combination may include VLPs of A/Chicken/HongKong/G9/97 or A/HongKong/1073/99.Another exemplary combination may comprise VLPs of A/Solomon Islands/3/2006. Another exemplary combination may comprise VLPs of A/Brisbane/10/2007. Another exemplary combination may comprise VLPs of A/Wisconsin/67/2005. Another exemplary combination may comprise VLPs of the B/Malaysia/2506/2004, B/Florida/4/2006 or B/Brisbane/3/2007 strains or subtypes.

The two or more VLPs may be expressed individually, and the purified or semipurified VLPs subsequently combined. Alternately, the VLPs may be co-expressed in the same host, for example a plant. The VLPs may be combined or produced in a desired ratio, for example about equivalent ratios, or may be combined in such a manner that one subtype or strain comprises the majority of the VLPs in the composition.

Therefore, the invention provides for compositions comprising VLPs of two or more strains or subtypes.

VLPs of enveloped viruses generally acquire their envelope from the membrane they bud through. Plant plasma membranes have a phytosterol complement that may have immunostimulatory effects. To investigate this possibility, plant-made H5 VLPs were administered to animals in the presence or absence of an adjuvant, and the HAI (hemagglutination inhibition antibody response) determined (Figures 22A, 22B). In the absence of an added adjuvant plant-made H5 VLPs demonstrate a significant HAI, indicative of a systemic immune response to administration of the antigen. Furthermore, the antibody isotype profiles of VLPs administered in the present or absence of adjuvant are similar (Figure 23A).
Table 5 lists sequences provided in various embodiments of the invention..

**Table 5: Sequence description for sequence identifiers.**

| **SEQ ID No** | **Sequence Description** | **In Disclosure** |
|---|---|---|
| 1 | N terminal H1 fragment | Figure 4a |
| 2 | C terminal H1 fragment | Figure 4b |
| 3 | H5 coding sequence | Figure 6 |
| 4 | primer Plato-443c | Figure 7a |
| 5 | primer SpHA(Ind)-Plasto.r | Figure 7b |
| 6 | primer Plasto-SpHA(Ind).c | Figure 7c |
| 7 | primer HA(Ind)-Sac.r | Figure 7d |
| 8 | Sequence of the alfalfa plastocyanin-based expression cassette used for the expression of H1 | Figure 1 |
| 9 | HA1 peptide sequence (A/New Caledonia/20/99) | Figure 8a |
| 10 | HA5 peptide sequence (A/Indonesia/5/2006) | Figure 8b |
| 11 | Influenza A Subtype H7 coding sequence (A/chicken/New York/1995) | Figure 9 |
| 12 | Influenza A Subtype H2 coding sequence (A/herring gull/DE/677/88 (H2N8)) | Figure 10a |
| 13 | Influenza A Subtype H3 coding sequence (A/Texas/32/2003) | Figure 10b |
| 14 | Influenza A Subtype H4 coding sequence (A/mallard/MN/33/00) | Figure 10c |
| 15 | Influenza A Subtype H5 coding sequence (A/duck/Shanghai/1/2000) | Figure 10d |
| 16 | Influenza A Subtype H6 coding sequence (A/northern pintail/TX/828189/02) | Figure 10e |
| 17 | Influenza A Subtype H8 coding sequence (A/Turkey/Ontario/6118/68(H8N4)) | Figure 10f |
| 18 | Influenza A Subtype H9 coding sequence (Alshoveler/Iran/G54/03) | Figure 10g |
| 19 | Influenza A Subtype H10 coding sequence (A/chicken/Germany/N/1949(H10N 7)) | Figure 10h |
| 20 | Influenza A Subtype H11 coding sequence (A/duck/England/56(H11N6)) | Figure 10i |
| 21 | Influenza A Subtype H12 coding sequence (A/duck/Alberta/60/76(H12N5)) | Figure 10j |
| 22 | Influenza A Subtype H13 coding sequence (A/Gull/Maryland/704/77(H13N6) ) | Figure 10k |
| 23 | Influenza A Subtype H14 coding sequence (A/Mallard/Gurjev/263/82) | Figure 10l |
| 24 | Influenza A Subtype H15 coding sequence (A/duck/Australia/341/83 (H15N8)) | Figure 10m |
| 25 | Influenza A Subtype H16 coding sequence (A/black-headed gull/Sweden/5/99(H16N3)) | Figure 10n |
| 26 | Influenza B HA coding sequence (B/Lee/40) | Figure 10o |
| 27 | Influenza C HA coding sequence (C/Johannesburg/66) | Figure 10p |
| 28 | Complete HAO H1 sequence | Figure 5 |
| 29 | Primer XmaI-pPlas.c | Figure 10q |
| 30 | Primer SacI-ATG-pPlas.r | Figure 10r |
| 31 | Primer SacI-PlasTer.c | Figure 10s |
| 32 | Primer EcoRI-PlasTer.r | Figure 10t |
| 33 | A/New Caledonia/20/99 (H1N1) GenBank Accession No. AY289929 | Figure 16 |
| 34 | *M. Sativa* protein disulfide isomerase GenBank Accession No. Z11499 | Figure 17 |
| 35 | A/.PuertoRico/8/34 (H1N1) GenBank Accession No. NC_002016.1 | Figure 18 |
| 36 | Clone 774: DNA from DraIII to Sac1 comprising plastocyanin regulatory region operatively linked to sequence encoding HA of ABrisbane/59/2007 (H1N1) | Figure 28 |
| 37 | Clone 775: DNA from DraIII to Saclcomprising plastocyanin regulatory region operatively linked to sequence encoding HA of A/Solomon Islands 3/2006 (H1N1) | Figure 29 |
| 38 | Clone 776: DNA from DraIII to Saclcomprising plastocyanin regulatory region operatively linked to sequence encoding HA of A/Brisbane 10/2007 (H3N2) | Figure 30 |
| 39 | Clone 777: DNA from DraIII to Saclcomprising plastocyanin regulatory region operatively linked to sequence encoding HA of A/Wisconsin/67/2005 (H3N2) | Figure 31 |
| 40 | Clone 778: DNA from DraIII to Saclcomprising plastocyanin regulatory region operatively linked to sequence encoding HA of B/Malaysia/2506/2004 | Figure 32 |
| 41 | Clone 779: DNA from DraIII to Saclcomprising plastocyanin regulatory region operatively linked to sequence encoding HA of B/Florida/4/2006 | Figure 33 |
| 42 | Clone 780: DNA from DraIII to Saclcomprising plastocyanin regulatory region operatively linked to sequence encoding HA of A/Singapore/1/57 (H2N2) | Figure 34 |
| 43 | Clone 781: DNA from DraIII to Saclcomprising plastocyanin regulatory region operatively linked to sequence encoding HA of A/Anhui/1/2005 (H5N1) | Figure 35 |
| 44 | Clone 782: DNA from DraIII to Saclcomprising plastocyanin regulatory region operatively linked to sequence encoding HA of A/Vietnam/1194/2004 (H5N1) | Figure 36 |
| 45 | Clone 783: DNA from DraIII to Saclcomprising plastocyanin regulatory region operatively linked to sequence encoding HA of A/Teal/HongKong/W312/97 (H6N1) | Figure 37 |
| 46 | Clone 784: DNA from DraIII to Saclcomprising plastocyanin regulatory region operatively linked to sequence encoding HA of A/Equine/Prague/56 (H7N7) | Figure 38 |
| 47 | Clone 785: DNA from DraIII to Saclcomprising plastocyanin regulatory region operatively linked to sequence encoding HA of A/HongKong/1073/99 (H9N2) | Figure 39 |
| 48 | Clone 774 HA amino acid sequence A/Brisbane/59/2007 (H1N1) | Figure 40A |
| 49 | Clone 775 HA amino acid sequence A/Solomon Islands 3/2006 (H1N1) | Figure 40B |
| 50 | Clone 776 HA amino acid sequence A/Brisbane 10/2007 (H3N2) | Figure 41A |
| 51 | Clone 777 HA amino acid sequence A/Wisconsin/67/2005 (H3N2) | Figure 41B |
| 52 | Clone 778 HA amino acid sequence B/Malaysia/2506/2004 | Figure 42A |
| 53 | Clone 779 HA amino acid sequence B/Florida/4/2006 | Figure 42B |
| 54 | Clone 780 HA amino acid sequence A/Singapore/1/57 (H2N2) | Figure 43A |
| 55 | Clone 781 HA amino acid sequence A/Anhui/1/2005 (H5N1) | Figure 43B |
| 56 | Clone 782 HA amino acid sequence A/Vietnam/1194/2004 (H5N1) | Figure 44A |
| 57 | Clone 783 HA amino acid sequence A/Teal/HongKong/W312/97 (H6N1) | Figure 44B |
| 58 | Clone 784 HA amino acid sequence A/Equine/Prague/56 (H7N7) | Figure 45A |
| 59 | Clone 785 HA amino acid sequence A/HongKong/1073/99 (H9N2) | Figure 45B |
| 60 | HA expression cassette comprising alfalfa plastocyanin promoter and 5' UTR, hemagglutinin coding sequence of **H5 from A/Indonesia/5/2005 (Construct # 660),** alfalfa plastocyanin 3' UTR and terminator sequences | Figure 51 |
| 61 | HA expression cassette comprising alfalfa plastocyanin promoter and 5' UTR, hemagglutinin coding sequence of **H1 from A/New Caledonia/20/1999 (Construct # 540),** alfalfa plastocyanin 3' UTR and terminator sequences | Figure 52 |
| 62 | HA expression cassette comprising alfalfa plastocyanin promoter and 5' UTR, hemagglutinin coding sequence of **H1 from A/Brisbane/59/2007 (construct #774),** alfalfa plastocyanin 3' UTR and terminator sequences | Figure 53 |
| 63 | HA expression cassette comprising alfalfa plastocyanin promoter and 5' UTR, hemagglutinin coding sequence of **H1 from A/Solomon Islands/3/2006 (H1N1) (construct #775),** alfalfa plastocyanin 3' UTR and terminator sequences | Figure 54 |
| 64 | HA expression cassette comprising alfalfa plastocyanin promoter and 5' UTR, hemagglutinin coding sequence of **H2 from A/Singapore/1/57 (H2N2) (construct # 780),** alfalfa plastocyanin 3' UTR and terminator sequences | Figure 55 |
| 65 | HA expression cassette comprising alfalfa plastocyanin promoter and 5' UTR, hemagglutinin coding sequence of **H5 from A/Anhui/1/2005 (H5N1) (Construct# 781),** alfalfa plastocyanin 3' UTR and terminator sequences | Figure 56 |
| 66 | HA expression cassette comprising alfalfa plastocyanin promoter and 5' UTR, hemagglutinin coding sequence of **H5 from A/Vietnam/1194/2004 (H5N1) (Construct** # **782),** alfalfa plastocyanin 3' UTR and terminator sequences | Figure 57 |
| 67 | HA expression cassette comprising alfalfa plastocyanin promoter and 5' UTR, hemagglutinin coding sequence of **H6 from A/Teal/Hong Kong/W312/97 (H6N1) (Construct** # **783),** alfalfa plastocyanin 3' UTR and terminator sequences | Figure 58 |
| 68 | HA expression cassette comprising alfalfa plastocyanin promoter and 5' UTR, hemagglutinin coding sequence of **H9 from A/Hong Kong/1073/99 (H9N2) (Construct** # **785),** alfalfa plastocyanin 3' UTR and terminator sequences | Figure 59 |
| 69 | HA expression cassette comprising alfalfa plastocyanin promoter and 5' UTR, hemagglutinin coding sequence of **H3 from ABrisbane/10/2007 (H3N2),** alfalfa plastocyanin 3' UTR and terminator sequences | Figure 60 |
| 70 | HA expression cassette comprising alfalfa plastocyanin promoter and 5' UTR, hemagglutinin coding sequence of **H3 from A/Wisconsin/67/2005 (H3N2),** alfalfa plastocyanin 3' UTR and terminator sequences | Figure 61 |
| 71 | HA expression cassette comprising alfalfa plastocyanin promoter and 5' UTR, hemagglutinin coding sequence of **H7 from A/Equine/Prague/56 (H7N7),** alfalfa plastocyanin 3' UTR and terminator sequences | Figure 62 |
| 72 | HA expression cassette comprising alfalfa plastocyanin promoter and 5' UTR, hemagglutinin coding sequence of **HA from B/Malaysia/2506/2004,** alfalfa plastocyanin 3' UTR and terminator sequences | Figure 63 |
| 73 | HA expression cassette comprising alfalfa plastocyanin promoter and 5' UTR, hemagglutinin coding sequence of **HA from B/Florida/4/2006,** alfalfa plastocyanin 3' UTR and terminator sequences | Figure 64 |
| 74 | Consensus amino acid sequence of SEQ ID NO: 49, 48, 33 and 9 | Figure 65 |
| 75 | Amino acid sequence of H1 New Caledonia (AAP34324.1) encoded by SEQ ID NO: 33 | Figure 66 |
| 76 | Amino acid sequence of H1 Puerto Rico (NC_0409878.1) encoded by SEQ ID NO: 35 | Figure 67 |
| 77 | pBinPlus.2613c | |
| 78 | Mut-ATG115.r | |
| 79 | Mut-ATG161.c | |
| 80 | LC-C5-1.110r | |
| 81 | Expression cassette number 828, from PacI (upstream promoter) to AscI (immediately downstream NOS terminator). | Figure 68 |
| 82 | SpPDI-HA(Ind).c | |
| 83 | Construct number 663, from HindIII (in the multiple cloning site, upstream Plastocyanine promoter) to EcoRI (immediately downstream Plastocynine terminator). | Figure 69 |
| 84 | SpPDI-H1B.c | |
| 85 | SacI-H1B.r | |
| 86 | Construct number 787, from HindIII (in the multiple cloning site, upstream Plastocyanine promoter) to EcoRI (immediately downstream Plastocynine terminator) | Figure 70 |
| 87 | H3B-SpPDI.r | |
| 88 | SpPDI-H3B.c | |
| 89 | H3(A-Bri).982r | |
| 90 | Construct number 790, from HindIII (in the multiple cloning site, upstream Plastocyanine promoter) to EcoRI (immediately downstream Plastocynine terminator). | Figure 7 |
| 91 | HBF-SpPDI.r | |
| 92 | SpPDI-HBF.c | |
| 93 | Plaster80r | |
| 94 | Construct number 798, from HindIII (in the multiple cloning site, upstream Plastocyanine promoter) to EcoRI (immediately downstream Plastocynine terminator). | Figure 72 |
| 95 | Apal-SpPDI.c | |
| 96 | StuI-H1(A-NC).r | |
| 97 | Construct number 580, from PacI (upstream 35S promoter) to AscI (immediately downstream NOS terminator). | Figure 73 |
| 98 | ApaI-H5 (A-Indo).1c | |
| 99 | H5 (A-Indo)-StuI.1707r | |
| 100 | Construct number 685, from PacI (upstream 35S promoter) to AscI (immediately downstream NOS terminator). | Figure 74 |
| 101 | Construct number 686, from PacI (upstream 35S promoter) to AscI (immediately downstream NOS terminator) | Figure 75 |
| 102 | ApaI-H1B.c | |
| 103 | StuI-H2B.r | |
| 104 | Construct 732, from PacI (upstream 35S promoter) to AscI (immediately downstream NOS terminator). | Figure 76 |
| 105 | Construct number 733, from PacI (upstream 35S promoter) to AscI (immediately downstream NOS terminator). | Figure 77 |
| 106 | ApaI-H3B.c | |
| 107 | StuI-H3B.r | |
| 108 | Construct number 735, from PacI (upstream 35S promoter) to AscI (immediately downstream NOS terminator). | Figure 78 |
| 109 | Construct number 736, from PacI (upstream 35S promoter) to AscI (immediately downstream NOS terminator). | Figure 79 |
| 110 | ApI-HBF.c | |
| 111 | StuI-HBF.r | |
| 112 | Construct number 738, from PacI (upstream 35S promoter) to AscI (immediately downstream NOS terminator). | Figure 80 |
| 113 | Construct number 739, from PacI (upstream 35S promoter) to AscI (immediately downstream NOS terminator). | Figure 81 |
| 114 | *M*. *sativa* Msj1 coding sequence | Figure 82 |
| 115 | Hsp-40Luz.lc | |
| 116 | Hsp40Luz-SacI.1272r | |
| 117 | Hsp40Luz-Plasto.r | |
| 118 | Hsp70Ara.1c | |
| 119 | Hsp70Ara-SacI.1956r | |
| 120 | Hsp70Ara-Plasto.r | |
| 121 | Construct number R850, from HindIII (in the multiple cloning site, upstream promoter) to EcoRI (immediately downstream NOS terminator). | Figure 83 |
| 122 | Construct number R860, from HindIII (in the multiple cloning site, upstream promoter) to EcoRI (immediately downstream NOS terminator) | Figure 84 |
| 123 | Construct number R870, from HindIII (in the multiple cloning site, upstream promoter) to EcoRI (immediately downstream NOS terminator). | Figure 85 |
| 124 | supP19-plasto.r | |
| 125 | supP19-1c | |
| 126 | SupP19-SacI.r | |
| 127 | Nucleotide sequence of the CPMV-HT-based expression cassette for H1 A/California/04/09 (cassette number 560). | Figure 92A |
| 128 | Amino acid sequence of H1 A/California/04/09 | Figure 92B |
| 129 | 2x35S promomter | Figure 93 |
| 130 | primer **PacI**-MCS-2X35S.c | |
| 131 | primer CPMV 5'UTR-**2X35S**.r | |
| 132 | primer **2X35S**-CPMV 5'UTR.c | |
| 133 | primer **ApaI**-M prot.r | |
| 134 | intermediary vector 972 | Figure 94 |
| 135 | H1 A/California/4/2009 | Figure 95 |
| 136 | portion of CPMV M (from DraIII to ApaI restriction site), signal peptide of alfalfa PDISP, HA0 of A/California/4/2009 hemagglutinin with mutated SacI and StuI restriction site | Figure 96 |
| 137 | Fragment 1 | Figure 97 |
| 138 | Fragment 2 | Figure 97 |
| 139 | Fragment 3 | Figure 97 |
| 140 | primer **DraIII**-MProt#2.c | |
| 141 | primer H1 Cal.390r | |
| 142 | primer H1 Cal.310c | |
| 143 | primer H1 Cal.1159r | |
| 144 | primer H1 Cal.1081c | |
| 145 | primer **StuI**-H1 Cal.r | |
| 146 | SpPDI-H1 A/California/4/2009 (in 2X35S/ CPMV-*HT* expression cassette) construct 560 | Figure 98 |

The invention will now be described in detail by way of reference only to the following non-limiting examples.

### Methods and Materials

### 1. Assembly of plastocyanin-based expression cassettes for native HA

All manipulations were done using the general molecular biology protocols of Sambrook and Russell (2001; which is incorporated herein by reference). The first cloning step consisted in assembling a receptor plasmid containing upstream and downstream regulatory elements of the alfalfa plastocyanin gene. The plastocyanin promoter and 5'UTR sequences were amplified from alfalfa genomic DNA using oligonucleotide primers XmaI-pPlas.c (SEQ ID NO: 29; Figure 10Q) and SacI-ATG-pPlas.r (SEQ ID NO: 30; Figure 10R). The resulting amplification product was digested with XmaI and SacI and ligated into pCAMBIA2300 (Cambia, Canberra, Australia), previously digested with the same enzymes, to create pCAMBIApromo Plasto. Similarly, the 3'UTR sequences and terminator of the plastocyanin gene was amplified from alfalfa genomic DNA using the following primers: SacI-PlasTer.c (SEQ ID NO: 31; Figure 10S) and EcoRI-PlasTer.r (SEQ ID NO: 32; Figure 10T), and the product was digested with SacI and EcoRI before being inserted into the same sites of pCAMBIApromoPlasto to create pCAMBIAPlasto.

A fragment encoding hemagglutinin from influenza strain A/Indonesia/5/05 (H5N1; Acc. No. LANL ISDN125873) was synthesized by Epoch Biolabs (Sugar Land, TX, USA). The fragment produced, containing the complete H5 coding region including the native signal peptide flanked by a HindIII site immediately upstream of the initial ATG, and a SacI site immediately downstream of the stop (TAA) codon, is presented in SEQ ID NO: 3 (Figure 6). The H5 coding region was cloned into a plastocyanin-based expression cassette by the PCR-based ligation method presented in Darveau et al. (1995). Briefly, a first PCR amplification was obtained using primers Plato-443c (SEQ ID NO: 4; Figure 7A) and SpHA(Ind)-Plasto.r (SEQ ID NO:5; Figure 7B) and pCAMBIA promoPlasto as template. In parallel, a second amplification was performed with primers Plasto-SpHA(Ind).c (SEQ ID NO: 6; Figure 7C) and HA(Ind)-Sac.r (SEQ ID NO:7; Figure 7D) with H5 coding fragment as template. The amplification obtained from both reactions were mixed together and the mixture served as template for a third reaction (assembling reaction) using Plato-443c (SEQ ID NO: 4; Figure 7A) and HA(Ind)-Sac.r (SEQ ID NO: 7; Figure 7D) as primers. The resulting fragment was digested with BamHI (in the plastocyanin promoter) and SacI (at the 3'end of the fragment) and cloned into pCAMBIAPlasto previously digested with the same enzymes. The resulting plasmid, named 660, is presented in figure 2B (also see Figure 11).

Hemagglutinin expression cassettes number 774 to 785 were assembled as follows. A synthetic fragment was synthesized comprising the complete hemagglutinin coding sequence (from ATG to stop) flanked in 3' by alfalfa plastocyanin gene sequences corresponding to the first 84 nucleotides upstream of the plastocyanin ATG and ending with a DraIII restriction site. The synthetic fragments also comprised a SacI site immediately after the stop codon.

Synthetic hemagglutinin fragments were synthesized by Top Gene Technologies (Montreal, QC, Canada), Epoch Biolabs (Sugar Land, TX, USA). The fragment synthesized are presented in figures 28 to 39 and correspond to SEQ ID NO:36 to SEQ ID NO:47. For the assembly of the complete expression cassettes, the synthetic fragments were digested with DraIII and SacI and cloned into pCAMBIAPlasto previously digested with the same enzymes. Table 6 presents the cassettes produced with the corresponding HA and other references in the text.

**Table 6: Hemagglutinin expression cassettes assembled from DraIII-SacI synthetic fragments.**

| Cassette number | Corresponding HA | Synthetic fragment | | Complete cassette | |
|---|---|---|---|---|---|
| | | Figure | Synthetic fragment SEQ ID NO | Figure | Final cassette SEQ ID NO |
| 774 | HA of A/Brisbane/59/2007 (H1N1) | 28 | 36 | 53 | 62 |
| 775 | HA of A/Solomon Islands 3/2006 (H1N1) | 29 | 37 | 54 | 63 |
| 776 | HA of A/Brisbane 10/2007 (H3N2) | 30 | 38 | 60 | 69 |
| 777 | HA of A/Wisconsin/67/2005 (H3N2) | 31 | 39 | 61 | 70 |
| 778 | HA of B/Malaysia/2506/2004 | 32 | 40 | 63 | 72 |
| 779 | HA of B/Florida/4/2006 | 33 | 41 | 64 | 73 |
| 780 | HA of A/Singapore/1/57 (H2N2) | 34 | 42 | 55 | 64 |
| 781 | HA of A/Anhui/1/2005 (H5N1) | 35 | 43 | 56 | 65 |
| 782 | HA of A/Vietnam/1194/2004 (H5N1) | 36 | 44 | 57 | 66 |
| 783 | HA of A/Teal/HongKong/W312/97 (H6N1) | 37 | 45 | 58 | 67 |
| 784 | HA of A/Equine/Prague/56 (H7N7) | 38 | 46 | 62 | 71 |
| 785 | HA of A/HongKong/1073/99 (H9N2) | 39 | 47 | 59 | 68 |

### Assembly of plastocyanin-based PDISP/HA-fusion expression cassettes

### H1 A/New Caledonia/20/99 (construct number 540)

The open reading frame from the H1 gene of influenza strain A/New Caledonia/20/99 (H1N1) was synthesized in two fragments (Plant Biotechnology Institute, National Research Council, Saskatoon, Canada). A first fragment synthesized corresponds to the wild-type H1 coding sequence (GenBank acc. No. AY289929; SEQ ID NO: 33; Figure 16) lacking the signal peptide coding sequence at the 5'end and the transmembrane domain coding sequence at the 3'end. A BglII restriction site was added at the 5' end of the coding sequence and a dual SacI/StuI site was added immediately downstream of the stop codon at the 3' terminal end of the fragment, to yield SEQ ID NO: 1 (Figure 5A). A second fragment encoding the C-terminal end of the H1 protein (comprising a transmembrane domain and cytoplasmic tail) from the KpnI site to the stop codon, and flanked in 3' by SacI and StuI restriction sites was also synthesized (SEQ ID NO. 2; Figure 5B).

The first H1 fragment was digested with BglII and SacI and cloned into the same sites of a binary vector (pCAMBIAPlasto) containing the plastocyanin promoter and 5'UTR fused to the signal peptide of alfalfa protein disulfide isomerase (PDI) gene (nucleotides 32-103; Accession No. Z11499; SEQ ID NO: 34; Figure 17) resulting in a PDI-H1 chimeric gene downstream of the plastocyanin regulatory elements. The sequence of the plastocyanin-based cassette containing the PDI signal peptide is presented in Figure 1 (SEQ ID NO:8). The resulting plasmid contained H1 coding region fused to the PDI signal peptide and flanked by plastocyanin regulatory elements. The addition of the C-terminal end coding region (encoding the transmembrane domain and the cytoplasmic tail) was obtained by inserting the synthesized fragment (SEQ ID NO: 2; Figure 5B) previously digested with KpnI and SacI, into the H1 expression plasmid. The resulting plasmid, named 540, is presented in Figure 11 (also see Figure 2A).

### H5 A/Indonesia/5/2005 (construct number 663)

The signal peptide of alfalfa protein disulfide isomerase (PDISP) (nucleotides 32-103; Accession No. Z11499; SEQ ID NO: 34; Figure 17) was linked to the HA0 coding sequence of H5 from A/Indonesia/5/2005 as follows. The H5 coding sequence was amplified with primers SpPDI-HA(Ind).c (SEQ ID NO:82) and HA(Ind)-SacI.r (SEQ ID NO: 7; Figure 7D) using construct number 660 (SEQ ID NO:60; Figure 51) as template. The resulting fragment consisted in the H5 coding sequence flanked, in 5', by the last nucleotides encoding PDISP (including a BglII restriction site) and, in 3', by a SacI restriction site. The fragment was digested with BglII and SacI and cloned into construct number 540 (SEQ ID NO:61; Figure 52) previously digested with the same restriction enzymes. The final cassette, named construct number 663 (SEQ ID NO:83), is presented in Figure 69.

### H1 A/Brisbane/59/2007 (Construct 787)

The signal peptide of alfalfa protein disulfide isomerase (PDISP) (nucleotides 32-103; Accession No. Z11499; SEQ ID NO: 34; Figure 17) was linked to the HA0 coding sequence of H1 from A/Brisbane/59/2007 as follows. The H1 coding sequence was amplified with primers SpPDI-H1B.c (SEQ ID NO: 84) and **SacI**-H1B.r (SEQ ID NO:85) using construct 774 (SEQ ID NO:62; Figure 53) as template. The resulting fragment consisted in the H1 coding sequence flanked, in 5', by the last nucleotides encoding PDISP (including a BglII restriction site) and, in 3', by a SacI restriction site. The fragment was digested with BglII and SacI and cloned into construct number 540 (SEQ ID NO:61; Figure 52) previously digested with the same restriction enzymes. The final cassette, named construct number 787 (SEQ ID NO:86), is presented in Figure 70.

### H3 A/Brisbane/10/2007 (construct number 790)

The signal peptide of alfalfa protein disulfide isomerase (PDISP) (nucleotides 32-103; Accession No. Z11499; SEQ ID NO: 34; Figure 17) was linked to the HA0 coding sequence of H3 from A/Brisbane/10/2007 as follows. PDISP was linked to the H3 coding sequence by the PCR-based ligation method presented in Darveau et al. (Methods in Neuroscience 26: 77-85(1995)). In a first round of PCR, a segment of the plastocyanine promoter fused to PDISP was amplified using primers Plasto-443c (SEQ ID NO: 4; Figure 7A) and H3B-**SpPDI**.r (SEQ ID NO:87) with construct 540 (SEQ ID NO:61; Figure 52) as template. In parallel, another fragment containing a portion of the coding sequence of H3 A/Brisbane/10/2007 (from codon 17 to the SpeI restriction site) was amplified with primers **SpPDI**-H3B.c (SEQ ID NO:88) and H3(A-Bri).982r (SEQ ID NO:89) using construct 776 (SEQ ID NO:69; Figure 60) as template. Amplification products were then mixed and used as template for a second round of amplification (assembling reaction) with primers Plasto-443c (SEQ ID NO: 4; Figure 7A) and H3(A-Bri).982r (SEQ ID NO:89). The resulting fragment was digested with BamHI (in the plastocyanin promoter) and SpeI (in the H3 coding sequence) and cloned into construct number 776 (SEQ ID NO:69; Figure 60), previously digested with the same restriction enzymes to give construct number 790 (SEQ ID NO:90). The construct is presented in Figure 71.

### HA B/Florida/4/2006 (construct number 798)

The signal peptide of alfalfa protein disulfide isomerase (PDISP) (nucleotides 32-103; Accession No. Z11499; SEQ ID NO: 34; Figure 17) was linked to the HA0 coding sequence of HA from HA B/Florida/4/2006 by the PCR-based ligation method presented in Darveau et al. (Methods in Neuroscience 26: 77-85(1995)). In a first round of amplification, a portion of the plastocyanin promoter fused to the PDISP was amplified using primers Plasto-443c (SEQ ID NO: 4; Figure 7A) and HBF-SpPDI.r (SEQ ID NO:91) with construct number 540 (SEQ ID NO:61; Figure 52) as template. In parallel, another fragment containing a portion of the coding sequence of HB B/Flo fused to the plastocyanin terminator was amplified with primers SpPDI-HBF.c (SEQ ID NO:92) and Plaster80r (SEQ ID NO:93) using construct number 779 (SEQ ID NO:73; Figure 64) as template. PCR products were then mixed and used as template for a second round of amplification (assembling reaction) with primers Plasto-443c (SEQ ID NO: 4; Figure 7A) and Plaster80r (SEQ ID NO:93). The resulting fragment was digested with BamHI (in the plastocyanin promoter) and AflII (in the HA B/Florida/4/2006 coding sequence) and cloned into construct number 779 (SEQ ID NO:73; Figure 64), previously digested with the same restriction enzymes to give construct number 798 (SEQ ID NO:94). The resulting expression cassette is presented in Figure 72.

### Assembly of CPMV-HT-based expression cassettes

CPMV-*HT* expression cassettes use the 35S promoter to control the expression of an mRNA comprising a coding sequence of interest flanked, in 5' by nucleotides 1-512 from the Cowpea mosaic virus (CPMV) RNA2 with mutated ATG at positions 115 and 161 and in 3', by nucleotides 3330-3481 from the CPMV RNA2 (corresponding to the 3' UTR) followed by the NOS terminator. Plasmid pBD-C5-1LC, (Sainsbury et al. 2008; Plant Biotechnology Journal 6: 82-92 and PCT Publication WO 2007/135480), was used for the assembly of CPMV-*HT*-based hemagglutinin expression cassettes. The mutation of ATGs at position 115 and 161 of the CPMV RNA2 was done using a PCR-based ligation method presented in Darveau et al. (Methods in Neuroscience 26: 77-85 (1995)). Two separate PCRs were performed using pBD-C5-1LC as template. The primers for the first amplification are pBinPlus.2613c (SEQ ID NO: 77) and Mut-**ATG115**.r (SEQ ID NO: 78). The primers for the second amplification were Mut-**ATG161**.c (SEQ ID NO: 79) and LC-C5-1.110r (SEQ ID NO: 80). The two obtained fragments are then mixed and used as template for a third amplification using pBinPlus.2613c (SEQ ID NO: 77) and LC-C5-1.110r (SEQ ID NO: 80) as primers. Resulting fragment is digested with PacI and ApaI and cloned into pBD-C5-1LC digested with the same enzyme. The sequence of the expression cassette generated, named 828, is presented in Figure 68 (SEQ ID NO: 81).

### Assembly of SpPDI-H1 A/New Caledonia/20/99 in CPMV-HT expression cassette (construct number 580).

A sequence encoding alfalfa PDI signal peptide fused to HA0 from H1 A/New Caledonia/20/99 was cloned into CPMV-*HT* as follows. Restriction sites ApaI (immediately upstream of intial ATG) and StuI (immediately downstream stop codon) were added to the hemagglutinin coding sequence by performing a PCR amplification with primers **ApaI**-SpPDI.c (SEQ ID NO: 95) and **StuI**-H1(A-NC).r (SEQ ID NO: 96) using construct number 540 (SEQ ID NO:61; Figure 52) as template. Resulting fragment was digested with ApaI and StuI restriction enzymes and cloned into construct number 828 (SEQ ID NO: 81) digested with the same enzymes. Resulting cassette was named construct number 580 (SEQ ID NO: 97).

### Assembly of SpPDI-H1 A/California/4/2009 in 2X35S/CPMV-HT expression cassette (construct number 560)

A fragment encoding alfalfa PDI signal peptide fused to HA0 from H1 A/California/4/2009 was cloned into 2X35S-CPMV-*HT* as follows.

We first created an intermediary vector containing the 2X35S promoter in the CPMV-HT expression cassette in replacement of the 35S promoter previously used. The change of promoter was performed using the PCR-based ligation method presented in Darveau et al. (Methods in Neuroscience 26: 77-85 (1995)). A first fragment containing 2X35S promoter SEQ ID NO: 129 (Figure 93) was amplified by PCR with primers:
**PacI**-MCS-2X35S.c (SEQ ID NO130):
   AATTG**TTAATTAA**GTCGACAAGCTTGCATGCCTGCAGGTCAAC
and CPMV 5'UTR-**2X35S**.r: (SEQ ID NO131):
   TCAAAACCTATTAAGATTTTAATA**CCTCTCCAAATGAAATGAACTTCC**
using a plasmid containing 2X35S promoter as template. In parallel, a second PCR was performed with primers:
**2X35S**-CPMV 5'UTR.c (SEQ ID NO132):
   **TTGGAGAGG**TATTAAAATCTTAATAGGTTTTGATAAAAGCGAACGTGGG
and **ApaI**-M prot.r (SEQ ID NO:133):
   TCTCCAT**GGGCCC**GACAAATTTGGGCAGAATATACAGAAGCTTA
using construct 685 (SEQ ID NO 100; Figure 74) as template. The two fragments obtained were then mixed and used as template for a second round of PCR (assembling reaction) using primers **PacI**-MCS-2X35S.c (SEQ ID NO:130) and **ApaI**-M prot.r (SEQ ID NO:133). The resulting fragment was then digested with PacI and ApaI and cloned into construct 685 (SEQ ID NO 100; Figure 74) digested with the same restriction enzymes. The sequence of the intermediary vector, named 972 (SEQ ID NO: 134), is presented in Figure 94.

Native H1 A/California/4/2009 sequence was obtained from GISAID database (accession number EPI176470) and is presented in Figure 95 (SEQ ID NO: 135). A nucleotide sequence comprising a portion of the CPMV M protein (in the CPMV-*HT* expression cassette) from DraIII to ApaI restriction site along with the signal peptide of alfalfa protein disulfide isomerase (PDISP) (nucleotides 32-103; Accession No. Z11499; SEQ ID NO: 34; Figure 17) and HA0 of A/California/4/2009 hemagglutinin with mutated SacI and StuI restriction site is presented in Figure 96 (SEQ ID NO:136). The sequence was synthesized by DNA2.0 (Menlo Park, CA, USA) in three different fragments. Fragment 1 (SEQ ID NO:137; Figure 97), 2 (SEQ ID NO: 138; Figure 97) and 3 (SEQ ID NO: 139; Figure 97) were assembled using the PCR-based ligation method presented in Darveau et al. (Methods in Neuroscience 26: 77-85 (1995)). In a first round of amplification, three different PCR were done. The first fragment was amplified using primer:
**DraIII**-MProt#2.c (SEQ ID NO:140)
   ATGCTAATAT**CACGTAGTG**CGGCGCCATTAAATAACGTGTACTTGTCC
and H1 Cal.390r (SEQ ID NO:141)
   GCTTAATTGCTCTCTTAGCTCCTCATAATCGATGAAATCTCC
using pJ201 vector (DNA2.0 proprietary vector) containing Fragment 1 (SEQ ID NO: 139) as template. The second fragment was amplified using primers:
H1 Cal.310c (SEQ ID NO:142)
   TGGAAACACCTAGTTCAGACAATGGAACGTGTTACCCAGGAG
and H1 Cal.1159r (SEQ ID NO:143)
   CTGCATATCCTGACCCCTGCTCATTTTGATGGTGATAACCGT
using pJ201 vector (DNA2.0 proprietary vector) containing Fragment 2 (SEQ ID NO: 138) as template. Finally, the last fragment was amplified using primers:
H1 Cal.1081c (SEQ ID NO:144)
   TTGAAGGGGGGTGGACAGGGATGGTAGATGGATGGTACGGTT
and **StuI**-H1 Cal.r (SEQ ID NO:145)
   TATT**AGGCCT**TTAAATACATATTCTACACTGTAGAGACCCATTAG
using pJ201 vector (DNA2.0 proprietary vector) containing Fragment 3 (SEQ ID NO139) as template. In a second round of PCR (assembly reaction), the three amplification fragments were then mixed and used as template with primers **DraIII**-MProt#2.c (SEQ ID NO:140) and **StuI**-H1 Cal.r (SEQ ID NO: 145). The resulting fragment was digested with DraIII and StuI and inserted into construct 972 (SEQ ID NO: 134) digested with the same restriction enzymes. The nucleotide sequence of the resulting construct was named 560 (SEQ ID NO: 146; Figure 98).

### Assembly of H5 A/Indonesia/5/2005 in CPMV-HT expression cassette (construct number 685).

The coding sequence of H5 from A/Indonesia/5/2005 was cloned into CPMV-*HT* as follows. Restriction sites ApaI (immediately upstream ATG) and StuI (immediately downstream stop codon) were added to the hemagglutinin coding sequence by performing a PCR amplification with primers **ApaI**-H5 (A-Indo).1c (SEQ ID NO: 98) and H5 (A-Indo)-**StuI**.1707r (SEQ ID NO: 99) using construct number 660 (SEQ ID NO:60; Figure 51) as template. Resulting fragment was digested with ApaI and StuI restriction enzymes and cloned into construct number 828 (SEQ ID NO: 81) digested with the same enzymes. Resulting cassette was named construct number 685 (SEQ ID NO: 100).

### Assembly of SpPDI-H5 A/Indonesia/5/2005 in CPMV-HT expression cassette (construct number 686).

A sequence encoding alfalfa PDI signal peptide fused to HA0 from H5 A/Indonesia/5/2005 was cloned into CPMV-*HT* as follows. Restriction sites ApaI (immediately upstream ATG) and StuI (immediately downstream stop codon) were added to the hemagglutinin coding sequence by performing a PCR amplification with primers **ApaI**-SpPDI.c (SEQ ID NO: 95) and H5 (A-Indo)-**StuI**.1707r (SEQ ID NO: 99) using construct number 663 (SEQ ID NO: 83) as template. Resulting fragment was digested with ApaI and StuI restriction enzymes and cloned into construct number 828 (SEQ ID NO: 81) digested with the same enzymes. Resulting cassette was named construct number 686 (SEQ ID NO: 101).

### Assembly of H1 A/Brisbane/59/2007 in CPMV-HT expression cassette (construct number 732).

The coding sequence of HA from H1 A/Brisbane/59/2007 was cloned into CPMV-*HT* as follows. Restriction sites ApaI (immediately upstream ATG) and StuI (immediately downstream stop codon) were added to the hemagglutinin coding sequence by performing a PCR amplification with primers **ApaI**-H1B.c (SEQ ID NO: 102) and **StuI**-H1B.r (SEQ ID NO: 103) using construct number 774 (SEQ ID NO:62; Figure 53) as template. Resulting fragment was digested with ApaI and StuI restriction enzymes and cloned into construct number 828 (SEQ ID NO: 81) digested with the same enzymes. Resulting cassette was named construct number 732 (SEQ ID NO: 104).

### Assembly of SpPDI-H1 A/Brisbane/59/2007 in CPMV-HT expression cassette (construct number 733).

A sequence encoding alfalfa PDI signal peptide fused to HA0 from H1 A/Brisbane/59/2007 was cloned into CPMV-*HT* as follows. Restriction sites ApaI (immediately upstream ATG) and StuI (immediately downstream stop codon) were added to the hemagglutinin coding sequence by performing a PCR amplification with primers **ApaI**-SpPDI.c (SEQ ID NO: 95) and **StuI**-H1B.r (SEQ ID NO: 103) using construct number 787 (SEQ ID NO: 86) as template. Resulting fragment was digested with ApaI and StuI restriction enzymes and cloned into construct number 828 (SEQ ID NO: 81) digested with the same enzymes. Resulting cassette was named construct number 733 (SEQ ID NO: 105).

### Assembly of H3 A/Brisbane/10/2007 in CPMV-HT expression cassette (construct number 735).

The coding sequence of HA from H3 A/Brisbane/10/2007 was cloned into CPMV-*HT* as follows. Restriction sites ApaI (immediately upstream ATG) and StuI (immediately downstream stop codon) were added to the hemagglutinin coding sequence by performing a PCR amplification with primers **ApaI**-H3B.c (SEQ ID NO: 106) and **StuI**-H3B.r (SEQ ID NO: 107) using construct number 776 (SEQ ID NO:69) as template. Resulting fragment was digested with ApaI and StuI restriction enzymes and cloned into construct number 828 (SEQ ID NO: 81) digested with the same enzymes. Resulting cassette was named construct number 735 (SEQ ID NO: 108).

### Assembly of SpPDI-H3 A/Brisbane/10/2007 in CPMV-HT expression cassette (construct number 736).

A sequence encoding alfalfa PDI signal peptide fused to HA0 from H3 A/Brisbane/10/2007 was cloned into CPMV-*HT* as follows. Restriction sites ApaI (immediately upstream ATG) and StuI (immediately downstream stop codon) were added to the hemagglutinin coding sequence by performing a PCR amplification with primers **ApaI**-SpPDI.c (SEQ ID NO:95) and **StuI**-H3B.r (SEQ ID NO: 107) using construct number 790 (SEQ ID NO:90) as template. Resulting fragment was digested with ApaI and StuI restriction enzymes and cloned into construct number 828 (SEQ ID NO: 81) digested with the same enzymes. Resulting cassette was named construct number 736 (SEQ ID NO: 109).

### Assembly of HA B/Florida/4/2006 in CPMV-HT expression cassette (construct number 738).

The coding sequence of HA from B/Florida/4/2006 was cloned into CPMV-*HT* as follows. Restriction sites ApaI (immediately upstream ATG) and StuI (immediately downstream stop codon) were added to the hemagglutinin coding sequence by performing a PCR apmlification with primers **ApaI**-HBF.c (SEQ ID NO: 110) and **StuI**-HBF.r (SEQ ID NO: 111) using construct number 779 (SEQ ID NO:73; Figure 64) as template. Resulting fragment was digested with ApaI and StuI restriction enzymes and cloned into construct number 828 (SEQ ID NO: 81) digested with the same enzymes. Resulting cassette was named construct number 738 (SEQ ID NO: 112).

### Assembly of SpPDI-HA B/Florida/4/2006 in CPMV-HT expression cassette (construct number 739).

A sequence encoding alfalfa PDI signal peptide fused to HA0 from B/Florida/4/2006 was cloned into CPMV-*HT* as follows. Restriction sites ApaI (immediately upstream ATG) and StuI (immediately downstream stop codon) were added to the hemagglutinin coding sequence by performing a PCR amplification with primers **ApaI**-SpPDI.c (SEQ ID NO: 95) and **StuI**-HBF.r (SEQ ID NO: 111) using construct number 798 (SEQ ID NO: 94) as template. Resulting fragment was digested with ApaI and StuI restriction enzymes and cloned into construct number 828 (SEQ ID NO: 81) digested with the same enzymes. Resulting cassette was named construct number 739 (SEQ ID NO: 113).

### Assembly of chaperone expression cassettes

Two heat shock protein (Hsp) expression cassettes were assembled. In a first cassette, expression of the *Arabidopsis thaliana* (ecotype Columbia) cytosolic HSP70 (Athsp70-1 in Lin et al. (2001) Cell Stress and Chaperones 6: 201-208) is controlled by a chimeric promoter combining elments of the alfalfa Nitrite reductase (Nir) and alfalfa Plastocyanin promoters (Nir/Plasto). A second cassette comprising the coding region of the alfalfa cytosolic HSP40 (MsJ1; Frugis et al. (1999) Plant Molecular Biology 40: 397-408) under the control of the chimeric Nir/Plasto promoter was also assembled.

An acceptor plasmid containing the alfalfa Nitrite reductase promoter (Nir), the GUS reporter gene and NOS terminator in plant binary vector was first assembled. Plasmid pNir3K51 (previously described in US Patent No. 6,420,548) was digested with HindIII and EcoRI. The resulting fragment was cloned into pCAMBIA2300 (Cambia, Canberra, Australia) digested by the same restriction enzyme to give pCAMBIA-Nir3K51.

Coding sequences for Hsp70 and Hsp40 were cloned separately in the acceptor plasmid pCAMBIANir3K51 by the PCR-based ligation method presented in Darveau et al. (Methods in Neuroscience 26:77-85 (1995)).

For Hsp40, Msj1 coding sequence (SEQ ID NO: 114) was amplified by RT-PCR from alfalfa (ecotype Rangelander) leaf total RNA using primers Hsp40Luz.1c (SEQ ID NO: 115) and Hsp40Luz-SacI.1272r (SEQ ID NO: 116). A second amplification was performed with primers Plasto-443c (SEQ ID NO: 4; Figure 7A) and Hsp40Luz-Plasto.r (SEQ ID NO: 117) with construct 660 (SEQ ID NO: 60; Figure 51) as template. PCR products were then mixed and used as template for a third amplification (assembling reaction) with primers Plasto-443c (SEQ ID NO: 4; Figure 7A) and Hsp40Luz-SacI.1272r (SEQ ID NO: 116). The resulting fragment was digested with HpaI (in the plastocyanin promoter) and cloned into pCAMBIANir3K51, previously digested with HpaI (in the Nir promoter) and SacI, and filed with T4 DNA polymerase to generate blunt ends. Clones obtained were screened for correct orientation and sequenced for sequence integrity. The resulting plasmid, named R850, is presented in Figure 83 (SEQ ID NO: 121). The coding region of the Athsp70-1 was amplified by RT-PCR from Arabidopsis leaf RNA using primers Hsp70Ara.1c (SEQ ID NO: 118) and Hsp70Ara-SacI.1956r (SEQ ID NO: 119). A second amplification was performed with primers Plato-443c (SEQ ID NO: 4; Figure 7A) and Hsp70Ara-Plasto.r (SEQ ID NO: 120) with construct 660 (SEQ ID NO: 60; Figure 51) as template. PCR products were then mixed and used as template for a third amplification (assembling reaction) with primers Plasto-443c (SEQ ID NO: 4; Figure 7A) and Hsp70ARA-SacI.1956r (SEQ ID NO: 119). The resulting fragment was digested with HpaI (in the plastocyanin promoter) and cloned into pCAMBIANir3K51 digested with HpaI (in the Nir promoter) and SacI and filed with T4 DNA polymerase to generate blunt ends. Clones obtained were screened for correct orientation and sequenced for sequence integrity. The resulting plasmid, named R860, is presented in Figure 84 (SEQ ID NO: 122).

A dual Hsp expression plasmid was assembled as follows. R860 was digested with BsrBI (downstream the NOS terminator), treated with T4 DNA polymerase to generate a blunt end, and digested with SbfI (upstream the chimeric NIR/Plasto promoter). The resulting fragment (Chimeric Nir/Plasto promoter-HSP70 coding sequence-Nos terminator) was cloned into R850 previously digested with SbfI and SmaI (both located in the multiple cloning site upstream chimeric Nir/Plasto promoter). The resulting plasmid, named R870, is presented in Figure 85 (SEQ ID NO: 123).

### Assembly of other expression cassettes

### Soluble H1 expression cassette

The cassette encoding the soluble form of H1 was prepared by replacing the region coding for the transmembrane domain and the cytoplasmic tail in 540 by a fragment encoding the leucine zipper GCN4 pII variant (Harbury et al, 1993, Science 1993; 262: 1401-1407). This fragment was synthesized with flanking KpnI and SacI sites to facilitate cloning. The plasmid resulting from this replacement was named 544 and the expression cassette is illustrated in Figure 11.

### M1 A/Puerto Rico/8/34 expression cassette

A fusion between the tobacco etch virus (TEV) 5'UTR and the open reading frame of the influenza A/PR/8/34 M1 gene (Acc. # NC_002016) was synthesized with a flanking SacI site added downstream of the stop codon. The fragment was digested with SwaI (in the TEV 5'UTR) and SacI, and cloned into a 2X35S/TEV based expression cassette in a pCAMBIA binary plasmid. The resulting plasmid bore the M1 coding region under the control of a 2X35S/TEV promoter and 5'UTR and the NOS terminator (construct 750; figure 11).

### HcPro expression cassette

An HcPro construct (35HcPro) was prepared as described in Hamilton et al. (2002). All clones were sequenced to confirm the integrity of the constructs. The plasmids were used to transform *Agrobacteium tumefaciens* (AGL1; ATCC, Manassas, VA 20108, USA) by electroporation (Mattanovich et al., 1989). The integrity of all *A*. *tumefaciens* strains were confirmed by restriction mapping.

### P19 expression cassette

The coding sequence of p19 protein of tomato bushy stunt virus (TBSV) was linked to the alfalfa plastocyanin expression cassette by the PCR-based ligation method presented in Darveau et al. (Methods in Neuroscience 26: 77-85(1995)). In a first round of PCR, a segment of the plastocyanin promoter was amplified using primers Plasto-443c (SEQ ID NO: 4; Figure 7A) and supP19-plasto.r (SEQ ID NO:124) with construct 660 (SEQ ID NO:60; Figure 51) as template. In parallel, another fragment containing the coding sequence of p19 was amplified with primers supP19-1c (SEQ ID NO:125) and SupP19-SacI.r (SEQ ID NO: 126) using construct 35S:p19 as described in Voinnet et al. (The Plant Journal 33: 949-956 (2003)) as template. Amplification products were then mixed and used as template for a second round of amplification (assembling reaction) with primers Plasto-443c (SEQ ID NO: 4; Figure 7A) and SupP19-SacI.r (SEQ ID NO: 126). The resulting fragment was digested with BamHI (in the plastocyanin promoter) and SacI (at the end of the p19 coding sequence) and cloned into construct number 660 (SEQ ID NO:60; Figure 51), previously digested with the same restriction enzymes to give construct number R472. Plasmid R472 is presented in Figure 86.

### 3. Preparation of plant biomass, inoculum, agroinfiltration, and harvesting

*Nicotiana benthamiana* or *Nicotiana tabacum* plants were grown from seeds in flats filled with a commercial peat moss substrate. The plants were allowed to grow in the greenhouse under a 16/8 photoperiod and a temperature regime of 25°C day/20°C night. Three weeks after seeding, individual plantlets were picked out, transplanted in pots and left to grow in the greenhouse for three additional weeks under the same environmental conditions. Prior to transformation, apical and axillary buds were removed at various times as indicated below, either by pinching the buds from the plant, or by chemically treating the plant

*Agrobacteria* transfected with each construct were grown in a YEB medium supplemented with 10 mM 2-[N-morpholino]ethanesulfonic acid (MES), 20 µM acetosyringone, 50 µg/ml kanamycin and 25 µg/ml of carbenicillin pH5.6 until they reached an OD₆₀₀ between 0.6 and 1.6. *Agrobacterium* suspensions were centrifuged before use and resuspended in infiltration medium (10 mM MgCl₂ and 10 mM MES pH 5.6). Syringe-infiltration was performed as described by Liu and Lomonossoff (2002, Journal of Virological Methods, 105:343-348). For vacuum-infiltration, *A*. *tumefaciens* suspensions were centrifuged, resuspended in the infiltration medium and stored overnight at 4°C. On the day of infiltration, culture batches were diluted in 2.5 culture volumes and allowed to warm before use. Whole plants of *N. benthamiana* or *N. tabacumwere* placed upside down in the bacterial suspension in an air-tight stainless steel tank under a vacuum of 20-40 Torr for 2-min. Following syringe or vacuum infiltration, plants were returned to the greenhouse for a 2-6 day incubation period until harvest. Unless otherwise specified, all infiltrations were performed as co-infiltration with AGL1/35S-HcPro in a 1:1 ratio, except for CPMV-HT cassette-bearing strains which were co-infiltrated with strain AGL1/R472 in a 1:1 ratio.

### 4. Leaf sampling and total protein extraction

Following incubation, the aerial part of plants was harvested, frozen at -80ºC, crushed into pieces. Total soluble proteins were extracted by homogenizing (Polytron) each sample of frozen-crushed plant material in 3 volumes of cold 50 mM Tris pH 7.4, 0.15 M NaCl, and 1 mM phenylmethanesulfonyl fluoride. After homogenization, the slurries were centrifuged at 20,000 g for 20 min at 4ºC and these clarified crude extracts (supernatant) kept for analyses. The total protein content of clarified crude extracts was determined by the Bradford assay (Bio-Rad, Hercules, CA) using bovine serum albumin as the reference standard.

### 5. Size exclusion chromatography ofprotein extract

Size exclusion chromatography (SEC) columns of 32 ml Sephacryl™ S-500 high resolution beads (S-500 HR : GE Healthcare, Uppsala, Sweden, Cat. No. 17-0613-10) were packed and equilibrated with equilibration/elution buffer (50 mM Tris pH8, 150 mM NaCl). One and a half millilitre of crude protein extract was loaded onto the column followed by an elution step with 45 mL of equilibration/elution buffer. The elution was collected in fractions of 1.5 mL relative protein content of eluted fractions was monitored by mixing 10 µL of the fraction with 200 µL of diluted Bio-Rad protein dye reagent (Bio-Rad, Hercules, CA The column was washed with 2 column volumes of 0.2N NaOH followed by 10 column volumes of 50 mM Tris pH8, 150 mM NaCl, 20% ethanol. Each separation was followed by a calibration of the column with Blue Dextran 2000 (GE Healthcare Bio-Science Corp., Piscataway, NJ, USA). Elution profiles of Blue Dextran 2000 and host soluble proteins were compared between each separation to ensure uniformity of the elution profiles between the columns used.

### 6. Protein Analysis and Immunoblotting

Protein concentrations were determined by the BCA protein assay (Pierce Biochemicals, Rockport IL). Proteins were separated by SDS-PAGE under reducing conditions and stained with Coomassie Blue. Stained gels were scanned and densitometry analysis performed using ImageJ Software (NIH).

Proteins from elution fraction from SEC were precipitated with acetone (Bollag et al., 1996), resuspended in 1/5 volume in equilibration/elution buffer and separated by SDS-PAGE under reducing conditions and electrotransferred onto polyvinylene difluoride (PVDF) membranes (Roche Diagnostics Corporation, Indianapolis, IN) for immunodetection. Prior to immunoblotting, the membranes were blocked with 5% skim milk and 0.1% Tween-20 in Tris-buffered saline (TBS-T) for 16-18h at 4ºC.

Immunoblotting was performed by incubation with a suitable antibody (Table 6), in 2 µg/ml in 2% skim milk in TBS-Tween 20 0.1 %. Secondary antibodies used for chemiluminescence detection were as indicated in Table 4, diluted as indicated in 2% skim milk in TBS-Tween 20 0.1%. Immunoreactive complexes were detected by chemiluminescence using luminol as the substrate (Roche Diagnostics Corporation). Horseradish peroxidase-enzyme conjugation of human IgG antibody was carried out by using the EZ-Link Plus^{®} Activated Peroxidase conjugation kit (Pierce, Rockford, IL). Whole, inactivated virus (WIV), used as controls of detection for H1, H3 and B subtypes, were purchased from National Institute for Biological Standards and Control (NIBSC).

**Table 6: Electrophoresis conditions, antibodies, and dilutions for immunoblotting of expressed proteins.**

| HA subtype | Influenza strain | Electrophoresis condition | Primary antibody | Dilution | Secondary antibody | Dilution |
|---|---|---|---|---|---|---|
| H1 | A/California/0 4/09 (H1N1) | Reducing | FII 10-I50F | 4 µg/ml | Goat antimouse (JIR 115-035-146) | 1:10 000 |
| H1 | A/Brisbane/59 /2007 (H1N1) | Reducing | FII 10-I50 | 4 µg/ml | Goat antimouse (JIR 115-035-146) | 1:10 000 |
| H1 | A/Solomon Islands/3/2006 (H1N1) | Reducing | NIBSC 07/104 | 1:2000 | Rabbit anti-sheep (JIR 313-035-045) | 1:10 000 |
| H1 | A/New Caledonia/20/ 99 (H1N1) | Reducing | FII 10-I50 | 4 µg/ml | Goat antimouse (JIR 115-035-146) | 1:10 000 |
| H2 | A/Singapore/1 /57 (H2N2) | Non-reducing | NIBSC 00/440 | 1:1000 | Rabbit anti-sheep (JIR 313-035-045) | 1:10 000 |
| H3 | A/Brisbane/10 /2007 (H3N2) | Non-Reducing | TGA AS393 | 1:4000 | Rabbit anti-sheep (JIR 313-035-045) | 1:10 000 |
| H3 | A/Brisbane/10 /2007 (H3N2) | Non-Reducing | NIBSC 08/136 | 1:1000 | Rabbit anti-sheep (JIR 313-035-045) | 1:10 000 |
| H3 | A/Wisconsin/6 7/2005 (H3N2) | Non-Reducing | NIBSC 05/236 | 1:1000 | Rabbit anti-sheep (JIR 313-035-045) | 1:10 000 |
| H5 | A/Indonesia/5/ 2005 (H5N1) | Reducing | ITC IT-003-005V | 1:4000 | Goat anti-rabbit (JIR 111-035-144) | 1:10 000 |
| H5 | A/Anhui/1/200 5 (H5N1) | Reducing | NIBSC 07/338 | 1:750 | Rabbit anti-sheep (JIR 313-035-045) | 1:10 000 |
| H5 | A/Vietnam/11 94/2004 (H5N1) | Non-reducing | ITC IT-003-005 | 1:2000 | Goat anti-rabbit (JIR 111-035-144) | 1:10 000 |
| H6 | A/Teal/Hong Kong/W312/9 7 (H6N1) | Non-reducing | BEI NR 663 | 1:500 | Rabbit anti-sheep (JIR 313-035-045) | 1:10 000 |
| H7 | A/Equine/Prag ue/56 (H7N7) | Non-reducing | NIBSC 02/294 | 1:1000 | Rabbit anti-sheep (JIR 313-035-045) | 1:10 000 |
| H9 | A/Hong Kong/1073/99 (H9N2) | Reducing | NIBSC 07/146 | 1:1000 | Rabbit anti-sheep (JIR 313-035-045) | 1:10 000 |
| B | B/Malaysia/25 06/2004 | Non-Reducing | NIBSC 07/184 | 1:2000 | Rabbit anti-sheep (JIR 313-035-045) | 1:10 000 |
| B | B/Florida/4/20 06 | Non-Reducing | NIBSC 07/356 | 1:2000 | Rabbit anti-sheep (JIR 313-035-045) | 1:10 000 |

| | | | | | | |
|---|---|---|---|---|---|---|
| FII: Fitzgerald Industries International, Concord, MA, USA; NIBSC: National Institute for Biological Standards and Control; JIR: Jackson ImmunoResearch, West Grove, PA, USA; BEI NR: Biodefense and emerging infections research resources repository; ITC: Immune Technology Corporation, Woodside, NY, USA; TGA: Therapeutic Goods Administration, Australia. | | | | | | |

Hemagglutination assay for H5 was based on a method described by Nayak and Reichl (2004). Briefly, serial double dilutions of the test samples (100 µL) were made in V-bottomed 96-well microtiter plates containing 100 µL PBS, leaving 100 µL of diluted sample per well. One hundred microliters of a 0.25% turkey red blood cells suspension (Bio Link Inc., Syracuse, NY) were added to each well, and plates were incubated for 2h at room temperature. The reciprocal of the highest dilution showing complete hemagglutination was recorded as HA activity. In parallel, a recombinant HA standard was diluted in PBS and run as a control on each plate.

### 7. Sucrose gradient ultracentrifugation

One milliliter of fractions 9, 10 and 11 eluted from the gel filtration chromatography on H5-containing biomass were pooled, loaded onto a 20-60% (w/v) discontinuous sucrose density gradient, and centrifuged 17,5 h at 125 000 g (4°C). The gradient was fractionated in 19 3-mL fractions starting from the top, and dialyzed to remove sucrose prior to immunological analysis and hemagglutination assays.

### 8. Electron microscopy

One hundred microliters of the samples to be examined were placed in an Airfuge ultracentrifugation tube (Beckman Instruments, Palo Alto, CA, USA). A grid was placed at the bottom of the tube which was then centrifuged 5 min at 120 000 g. The grid was removed, gently dried, and placed on a drop of 3% phosphotungstic acid at pH 6 for staining. Grids were examined on a Hitachi 7100 transmission electron microscope (TEM) (for images in Figures 14B, 15B and 15C).

For images in Figure 19, Leaf blocks of approximately 1 mm³ were fixed in PBS containing 2.5% glutaraldehyde and washed in PBS containing 3% sucrose before a post-fixation step in 1.33% osmium tetroxide. Fixed samples were imbedded in Spurr resin and ultrathin layers were laid on a grid. Samples were positively stained with 5% uranyl acetate and 0,2% lead citrate before observation. Grids were examined on a Hitachi 7100 transmission electron microscope (TEM).

### 9. Plasma membrane lipid analysis

Plasma membranes (PM) were obtained from tobacco leaves and cultured BY2 cells after cell fractionation according to Mongrand *et al*.by partitioning in an aqueous polymer two-phase system with polyethylene glycol 3350/dextran T-500 (6.6% each). All steps were performed at 4 °C.

Lipids were extracted and purified from the different fractions according to Bligh and Dyer. Polar and neutral lipids were separated by monodimensional HP-TLC using the solvent systems described in Lefebvre *et al*.. Lipids of PM fractions were detected after staining with copper acetate as described by Macala *et al.* Lipids were identified by comparison of their migration time with those of standards (all standards were obtained from Sigma-Aldrich, St-Louis, MO, USA, except for SG which was obtained from Matreya, Pleasant Gap, PA, USA).

### 10. H5 VLP (A/Indonesia/5/2005) purification

Frozen 660-infiltrated leaves of *N. benthamiana* were homogenized in 1.5 volumes of 50 mM Tris pH 8, NaCl 150 mM and 0.04% sodium meta-bisulfite using a commercial blender. The resulting extract was supplemented with 1 mM PMSF and adjusted to pH 6 with 1 M acetic acid before being heated at 42°C for 5 min. Diatomaceous earth (DE) was added to the heat-treated extract to adsorb the contaminants precipitated by the pH shift and heat treatment, and the slurry was filtered through a Whatman paper filter. The resulting clarified extract was centrifuged at 10,000 x g for 10 minutes at RT to remove residual DE, passed through 0.8/0.2 µm Acropack 20 filters and loaded onto a fetuin-agarose affinity column (Sigma-Aldrich, St-Louis, MO, USA). Following a wash step in 400 mM NaCl, 25 mM Tris pH 6, bound proteins were eluted with 1.5 M NaCl, 50 mM MES pH 6. Eluted VLP were supplemented with Tween-80 to a final concentration of 0.0005% (v/v). VLP were concentrated on a 100 kDa MWCO Amicon membrane, centrifuged at 10,000 x g for 30 minutes at 4°C and resuspended in PBS pH 7.4 with 0.01% Tween-80 and 0.01% thimerosal. Suspended VLPs were filter-sterilized before use.

### 11. Animal studies

### Mice

Studies on the immune response to influenza VLP administration were performed with 6-8 week old female BALB/c mice (Charles River Laboratories). Seventy mice were randomly divided into fourteen groups of five animals. Eight groups were used for intramuscular immunization and six groups were used to test intranasal route of administration. All groups were immunized in a two-dose regiment, the boost immunization being done 3 weeks following the first immunization.

For intramuscular administration in hind legs, unanaesthetized mice were immunized with either the plant-made H5 VLP (A/Indonesia/5/2005 (H5N1) vaccine (0.1, 1, 5 or 12 µg), or a control hemagglutinin (H5) antigen. The control H5 comprised recombinant soluble hemagglutinin produced based on strain A/Indonesia/5/05 H5N1 and purified from 293 cell culture (Immune Technology Corp., New York, USA) (used at 5 µg per injection unless otherwise indicated). Buffer control was PBS. This antigen consists of amino acids 18-530 of the HA protein, and has a His-tag and a modified cleavage site. Electron microscopy confirmed that this commercial product is not in the form of VLPs.

To measure the effect of adjuvant, two groups of animals were immunized with 5 µg plant-made VLP H5 vaccine plus one volume Alhydrogel 2% (alum, Accurate Chemical & Scientific Corporation, Westbury, NY, US) or with 5 µg recombinant hemagglutinin purified from 293 cell culture plus 1 volume alum. Seventy mice were randomly divided into fourteen groups of five animals. Eight groups were used for intramuscular immunization and six groups were used to test intranasal route of administration. All groups were immunized according to a prime-boost regimen, the boost immunization performed 3 weeks following the first immunization.

For intramuscular administration in hind legs, unanaesthetized mice were immunized with the plant-made H5 VLP (0.1, 1, 5 or 12 µg), or the control hemagglutinin (HA) antigen (5 µg) or PBS. All antigen preparations were mixed with Alhydrogel 1% (alum, Accurate Chemical & Scientific Corporation, Westbury, NY, US) in a 1:1 volume ratio prior to immunizations. To measure the effect of adjuvant, two groups of animals were immunized with either 5 µg plant-made VLP H5 vaccine or with 5 µg of control HA antigen without any adjuvant.

For intranasal administration, mice were briefly anaesthetized by inhalation of isoflurane using an automated induction chamber. They were then immunized by addition of 4 µl drop/nostril with the plant-made VLP vaccine (0.1 or 1 µg), or with control HA antigen (1 µg) or with PBS. All antigen preparations were mixed with chitosan glutamate 1% (Protosan, Novamatrix/ FMC BioPolymer, Norway) prior to immunizations. The mice then breathed in the solutions. To verify the effect of adjuvant with the intranasal route of administration, two groups of animals were immunized with 1 µg plant-made VLP H5 vaccine or with 1 µg control HA antigen.

### Ferrets

Ten groups of 5 ferrets (male, 18-24 weeks old, mass of approx 1 kg) were used. Treatment for each group is as described in Table 7. The adjuvant used was Alhydrogel (alum) (Superfos Biosector, Denmark) 2% (final=1%). Vaccine composition was membrane-associated A/Indonesia/5/05 (H5N1) VLPs produced as described. The vaccine control (positive control) was a fully glycosylated membrane-bound recombinant H5 from Indonesia strain produced using adenovirus in 293 cell culture by Immune Technology Corporation (ITC).

**Table 7. Treatment groups**

| **Group** | **n** | **Product injected to animals** | **Route of administration** | **Adjuvant** |
|---|---|---|---|---|
| 1 | 5 | PBS (negative control) | i.m.* | - |
| 2 | 5 | Vaccine-plant, 1 µg | i.m. | - |
| 3 | 5 | Vaccine-plant, 1 µg | i.m. | Alum |
| 4 | 5 | Vaccine-plant, 5 µg | i.m. | - |
| 5 | 5 | Vaccine-plant, 5 µg | i.m. | Alum |
| 6 | 5 | Vaccine-plant, 7.5 µg | i.m. | - |
| 7 | 5 | Vaccine-plant, 15 µg | i.m. | - |
| 8 | 5 | Vaccine-plant, 15 µg | i.m. | Alum |
| 9 | 5 | Vaccine-plant, 30 µg | i.m. | - |
| 10 | 5 | Vaccine-control, 5 µg | i.m. | - |

| | | | | |
|---|---|---|---|---|
| *i.m.: intramuscular | | | | |

Ferrets were assessed for overall health and appearance (body weight, rectal temperature, posture, fur, movement patterns, breathing, excrement) regularly during the study. Animals were immunized by intramuscular injection (0.5-1.0 total volume) in quadriceps at day 0, 14 and 28; for protocols incorporating adjuvant, the vaccine composition was combined with Alhydrogel immediately prior to immunization in a 1:1 volume ratio). Serum samples were obtained on day 0 before immunizing, and on day 21 and 35. Animals were sacrificed (exsanguination/cardiac puncture) on days 40-45, and , spleens were collected and necropsy performed.

Anti-influenza antibody titres may be quantified in ELISA assays using homologous or heterologous inactivated H5N1 viruses.

Hemagglutination inhibitory antibody titers of serum samples (pre-immune, day 21 and day 35) were evaluated by microtiter HAI as described (Aymard et al 1973). Briefly, sera were pretreated with receptor-destroying enzyme, heat-inactivated and mixed with a suspension of erythrocytes (washed red blood cells-RBC). Horse washed RBC (10%) from Lampire are recommended and considering that the assay may vary depending of the source of the RBC (horse-dependant), washed RBCs from 10 horses have been tested to select the most sensitive batch. Alternately, turkey RBC may be used. Antibody titer was expressed as the reciprocal of the highest dilution which completely inhibits hemagglutination.

Cross-reactive HAI titers: HAI titers of ferrets immunized with a vaccine for the A/Indonesia/5/05 (clade 2.1) were measured using inactivated H5N1 influenza strains from another subclade or clade such as the clade 1 Vietnam strains A/Vietnam/1203/2004 and A/Vietnam/1194/2004 or the A/Anhui/01/2005 (subclade 2.3) or the A/turkey/Turkey/1/05 (subclade 2.2). All analyses were performed on individual samples.

Data analysis: Statistical analysis (ANOVA) were performed on all data to establish if differences between groups are statistically significant.

### Experimental design for lethal challenge(mice)

One hundred twenty eight mice were randomly divided into sixteen groups of eight animals, one group being unimmunized and not challenged (negative control). All groups were immunized via intramuscular administration in a two-dose regimen, the second immunization being done 2 weeks following the first immunization.

For intramuscular administration in hind legs, unanaesthetized mice were immunized with the plant-made H5 VLP (1, 5 or 15 µg), or 15 µg of control HA antigen or PBS. All antigen preparations were mixed with one volume of Alhydrogel 1% prior to immunizations (alum, Accurate Chemical & Scientific Corporation, Westbury, NY, US).

During the immunization period, mice were weighted once a week and observation and monitored for local reactions at the injection site.

Twenty two days following the second immunization, anesthetized mice were challenged intranasally (i.n.) into a BL4 containment laboratory (P4-Jean Mérieux-INSERM, Lyon, France) with 4.09 x 10⁶ 50% cell culture infective dose (CCID50) of influenza A/Turkey/582/06 virus (kindly provided by Dr. Bruno Lina, Lyon University, Lyon, France). Following challenge, mice were observed for ill clinical symptoms and weighed daily, over a fourteen day period. Mice with severe infection symptoms and weight loss of ≥25% were euthanized after anaesthesia.

### Blood collection, lung and nasal washes and spleen collection

Lateral saphenous vein blood collection was performed fourteen days after the first immunization and fourteen days after second immunization on unanaesthetized animal. Serum was collected by centrifugation at 8000 g for 10 min.

Four weeks after second immunisation, mice were anaesthetized with CO₂ gas and immediately upon termination, cardiac puncture was used to collect blood.

After final bleeding, a catheter was inserted into the trachea towards the lungs and one ml of cold PBS-protease inhibitor cocktail solution was put into a 1cc syringe attached to the catheter and injected into the lungs and then removed for analysis. This wash procedure was performed two times. The lung washes were centrifuged to remove cellular debris. For nasal washes, a catheter was inserted towards the nasal area and 0.5 ml of the PBS-protease inhibitor cocktail solution was pushed through the catheter into the nasal passages and then collected. The nasal washes were centrifuged to remove cellular debris. Spleen collection was performed on mice immunized intramuscularly with 5 µg of adjuvanted plant-made vaccine or 5 µg adjuvanted recombinant H5 antigen as well as on mice immunized intranasaly with 1 µg of adjuvanted plant-made vaccine or 1 µg adjuvanted recombinant H5 antigen. Collected spleens were placed in RPMI supplemented with gentamycin and mashed in a 50 ml conical tube with plunger from a 10 ml syringe. Mashed spleens were rinsed 2 times and centrifuged at 2000 rpm for 5 min and resuspended in ACK lysing buffer for 5 min at room temperature. The splenocytes were washed in PBS-gentamycin, resuspended in 5% RPMI and counted. Splenocytes were used for proliferation assay.

### Antibody titers

Anti-influenza antibody titers of sera were measured at 14 days after the first immunization as well as 14 and 28 days after the second immunisation. The titer were determined by enzyme-linked immunosorbent assay (ELISA) using the inactivated virus A/Indonesia/5/05 as the coating antigen. The end-point titers were expressed as the reciprocal value of the highest dilution that reached an OD value of at least 0.1 higher than that of negative control samples.

For antibody class determination (IgG1, IgG2a, IgG2b, IgG3, IgM), the titers were evaluated by ELISA as previously described.

### Hemagglutination inhibition (HI) titers

Hemagglutination inhibition (HI) titers of sera were measured at 14 and 28 days after the second immunisation as previously described (WHO 2002; Kendal 1982). Inactivated virus preparations from strains A/Indonesia/5/05 or A/Vietnam/1203/2004 were used to test mouse serum samples for HI activity. Sera were pre-treated with receptor-destroying enzyme II (RDE II) (Denka Seiken Co., Tokyo, Japan) prepared from *Vibrio cholerae* (Kendal 1982). HI assays were performed with 0.5% turkey red blood cells. HI antibody titres were defined as the reciprocal of the highest dilution causing complete inhibition of agglutination.

### Examples

### Example 1:Transient expression of influenza virus A/Indonesia/5/05 (H5N1) hemagglutinin by agroinfiltration in N. benthamiana plants

The ability of the transient expression system to produce influenza hemagglutinin was determined through the expression of the H5 subtype from strain A/Indonesia/5/05 (H5N1). As presented in Figure 11, the hemagglutinin gene coding sequence (GenBank Accession No. EF541394), with its native signal peptide and transmembrane domain, was first assembled in the plastocyanin expression cassette - promoter, 5'UTR, 3'UTR and transcription termination sequences from the alfalfa plastocyanin gene - and the assembled cassette (660) was inserted into to a pCAMBIA binary plasmid. This plasmid was then transfected into *Agrobacterium* (AGL1), creating the recombinant strain AGL1/660, which was used for transient expression.

*N. benthamiana* plants were infiltrated with AGL1/660, and the leaves were harvested after a six-day incubation period. To determine whether H5 accumulated in the agroinfiltrated leaves, protein were first extracted from infiltrated leaf tissue and analyzed by Western blotting using anti-H5 (Vietnam) polyclonal antibodies. A unique band of approximately 72 kDa was detected in extracts (Figure 12), corresponding in size to the uncleaved HA0 form of influenza hemagglutinin. The commercial H5 used as positive control (A/Vietnam/1203/2004; Protein Science Corp., Meriden, CT, USA) was detected as two bands of approximately 48 and 28 kDa, corresponding to the molecular weight of HA1 and HA2 fragments, respectively. This demonstrated that expression of H5 in infiltrated leaves results in the accumulation of the uncleaved translation product.

The formation of active HA trimers was demonstrated by the capacity of crude protein extracts from AGL1/660-transformed leaves to agglutinate turkey red blood cells (data not shown).

### Example 2: Characterization of hemagglutinin-containing structures in plant extracts using size exclusion chromatography

The assembly of plant-produced influenza hemagglutinin into high molecular weight structures was assessed by gel filtration. Crude protein extracts from AGL1/660-infiltrated plants (1.5 mL) were fractionated by size exclusion chromatography (SEC) on Sephacryl™ S-500 HR columns (GE Healthcare Bio-Science Corp., Piscataway, NJ, USA). Elution fractions were assayed for their total protein content and for HA abundance using immunodetection with anti-HA antibodies (Figure 13A).As shown in Figure 13A, Blue Dextran (2 MDa) elution peaked early in fraction 10 while the bulk of host proteins was retained in the column and eluted between fractions 14 and 22. When proteins from 200 µL of each SEC elution fraction were concentrated (5-fold) by acetone-precipitation and analyzed by Western blotting (Figure 15A, H5), hemagglutinin (H5) was primarily found in fractions 9 to 14 (Figure 13B). Without wishing to be bound by theory, this suggests that the HA protein had either assembled into a large superstructure or that it has attached to a high molecular weight structure.

A second expression cassette was assembled with the H1 nucleic acid sequence from A/New Caledonia/20/99 (H1N1) (SEQ ID NO: 33; Figure 16; GenBank Accession No. AY289929) to produce construct 540 (Figure 11). A chimeric gene construct was designed so as to produce a soluble trimeric form of H1 in which the signal peptide originated from a plant protein disulfide isomerase gene, and the transmembrane domain of H1 was replaced by the pII variant of the GCN4 leucine zipper, a peptide shown to self-assemble into trimers (Harbury et al., 1993) (cassette 544, figure 11). Although lacking the transmembrane domain, this soluble trimeric form was capable of hemagglutination (data not shown).

Protein extracts from plants infiltrated with AGL1/540 or AGL1/544 were fractionated by SEC and the presence of H1 eluted fractions was examined by Western blotting with anti-influenza A antibodies (Fitzgerald, Concord, MA, USA). In AGL1/540-infiltrated leaves, H1 accumulated mainly as a very high molecular weight structure, with the peak was skewed toward smaller size structures (H1; Figure 13C). In AGL1/544-infiltrated leaves, the soluble form of H1 accumulated as isolated trimers as demonstrated by the elution pattern from gel filtration which parallels the host protein elution profile (soluble H1; Figure 13D). In comparison, H1 rosettes (Protein Science Corp., Meriden, CT, USA), consisting in micelles of 5-6 trimers of hemagglutinin eluted at fractions 12 to 16 (Figure 13E), earlier than the soluble form of H1 (Figure 13D) and later than the native H1 (Figure 13C).

To evaluate the impact of M1 co-expression on hemagglutinin assembly into structure, a M1 expression cassette was assembled using the nucleic acid corresponding to the coding sequence of the A/PR/8/34 (H1N1) M1 (SEQ ID NO: 35; Figure 18; GenBank Accession No. NC_002016). The construct was named 750 and is presented in Figure 11. For the co-expression of M1 and H1, suspensions of AGL1/540 and AGL1/750 were mixed in equal volume before infiltration. Co-infiltration of multiple *Agrobacterium* suspensions permits co-expression of multiple transgenes. The Western blot analysis of SEC elution fractions shows that the co-expression of M1 did not modify the elution profile of the H1 structures, but resulted in a decrease in H1 accumulation in the agroinfiltrated leaves (see Figure 13F).

### Example 3: Isolation of H5 structures by centrifugation in sucrose gradient and observation under electron microscopy

The observation of hemagglutinin structure under electron microscopy (EM) required a higher concentration and purity level than that obtained from SEC on crude leaf protein extracts. To allow EM observation of H5 structures, a crude leaf protein extract was first concentrated by PEG precipitation (20% PEG) followed by resuspension in 1/10 volumes of extraction buffer. The concentrated protein extract was fractionated by S-500 HR gel filtration and elution fractions 9, 10, and 11 (corresponding to the void volume of the column) were pooled and further isolated from host proteins by ultracentrifugation on a 20-60% sucrose density gradient. The sucrose gradient was fractionated starting from the top and the fractions were dialysed and concentrated on a 100 NMWL centrifugal filter unit prior to analysis. As shown on the Western blots and hemagglutination results(Figure 14A), H5 accumulated mainly in fractions 16 to 19 which contained ≈60% sucrose, whereas most of the host proteins peaked at fraction 13. Fractions 17, 18, and 19 were pooled, negatively stained, and observed under EM. Examination of the sample clearly demonstrated the presence of spiked spheric structures ranging in size from 80 to 300 nm which matched the morphological characteristics of influenza VLPs (Figure 14B).

### Example 4: Purification of influenza H5 VLPs from plant biomass

In addition to an abundant content of soluble proteins, plant leaf extracts contain a complex mixture of soluble sugars, nucleic acids and lipids. The crude extract was clarified by a pH shift and heat treatment followed by filtration on diatomaceous earth (see *Material and method* section for a detailed description of the clarification method). Figure 15A (lanes 1-4) presents a Coomassie Blue stained gel comparing protein content at the various steps of clarification. A comparison of protein content in the crude extract (lane 1) and in the clarified extract (lane 4) reveals the capacity of the clarification steps to reduce the global protein content and remove most of the major contaminant visible at 50 kDa in crude leaf extracts. The 50 kDa band corresponds to the RuBisCO large subunit, representing up to 30% of total leaf proteins.

Influenza H5 VLPs were purified from these clarified extracts by affinity chromatography on a fetuin column. A comparison of the load fraction (Figure 15A, lane 5) with the flowthrough (Figure 15A, lane 6) and the eluted VLPs (Figure 15A, lane 7) demonstrates the specificity of the fetuin affinity column for influenza H5 VLPs in plant clarified extract.

The purification procedure resulted in over 75% purity in H5, as determined by densitometry on the Coomassie Blue stained SDS-PAGE gel (Figure 15A, lane 7). In order to assess the structural quality of the purified product, the purified H5 was concentrated on a 100 NMWL (nominal molecular weight limit) centrifugal filter unit and examined under EM after negative staining. Figure 15B shows a representative sector showing the presence of profuse VLPs. A closer examination confirmed the presence of spikes on the VLPs (figure 15C).

As shown in Figure 15D, H5 VLPs were purified to approx. 89% purity from clarified leaf extract by affinity chromatography on a fetuin column, based on the density of the Coomassie Blue stained H5 hemagglutinin and on total protein content determination by the BCA method.

The bioactivity of HA VLPs was confirmed by their capacity to agglutinate turkey red blood cells (data not shown).

Figure 15D also confirms the identity of the purified VLP visualized by Western blotting and immunodetection with an anti-H5 polyclonal serum (A/Vietnam/1203/2004). A unique band of approximately 72 kDa is detected and corresponds in size to the uncleaved HA0 form of influenza hemagglutinin. Figure 15c shows the VLP structure of the vaccine with the hemagglutinin spikes covering its structure.

VLPs were formulated for immunization of mice by filtering through a 0.22 µm filter; endotoxin content was measured using the endotoxin LAL (Limulus Amebocyte Lysate) detection kit (Lonza, Walkserville, MS, USA). The filtered vaccine contained 105.8 ±11.6% EU/ml (endotoxin units/ml).

### Example 5: Localization of influenza VLPs in plants

To localize the VLPs and confirm their plasma membrane origin, thin leaf sections of H5-producing plants were fixed and examined under TEM after positive staining. Observation of leaf cells indicated the presence of VLPs in extracellular cavities formed by the invagination of the plasma membrane (Figure 19). The shape and position of the VLPs observed demonstrated that despite the apposition of their plasma membranes on the cell wall, plant cells have the plasticity required to produce influenza VLPs derived from their plasma membrane and accumulate them in the apoplastic space.

### Example 6: Plasma Membrane Lipid analysis

Further confirmation of the composition and origin of the plant influenza VLPs was obtained from analyses of the lipid content. Lipids were extracted from purified VLPs and their composition was compared to that of highly purified tobacco plasma membranes by high performance thin layer chromatography (HP-TLC). The migration patterns of polar and neutral lipids from VLPs and control plasma membranes were similar. Purified VLPs contained the major phospholipids (phosphatidylcholine and phosphatidylethanolamine) and sphingolipids (glucosyl-ceramide) found in the plasma membrane (Figure 27A), and both contained free sterols as the sole neutral lipids (Figure 27B). However, immunodetection of a plasma membrane protein marker (ATPase) in purified VLP extracts showed that the VLP lipid bilayer does not contain one of the major proteins associated with plant plasma membranes, suggesting that host proteins may have been excluded from the membranes during the process of VLPs budding from the plant cells (Figure 27C).

### Example 7: Immunogenicity of the H5 VLPs and effect of route of administration

Mice were administered plant-made H5 VLPs by intramuscular injection, or intranasal (inhalation). 0.1 to 12 ug of VLPs were injected intramuscularly into mice, with alum as an adjuvant, according to the described methods. Peak antibody titers were observed with the lowest antigen quantity, in a similar magnitude to that of 5 ug recombinant, soluble hemagglutinin (H5) (Figure 20A).

0.1 to 1 ug plant-made H5 VLPs were administered intranasally with a chitosan adjuvant provided for an antibody response greater than that of the recombinant soluble H5 with an alum adjuvant (Figure 20B).

For both administration routes, and over a range of antigen quantities, seroconversion was observed in all of the mice tested. Recombinant H5 soluble antigen conferred low (<1/40) or negligible (1<1/10 for the non-adjuvanted recombinant H5) HI titres.

### Example 8:Hemagglutination-inhibition antibody titer (HAI) H5 VLP

Figure 21 A, B illustrates the hemagglutination inhibition (HAI) antibody response 14 days following a "boost" with plant-made H5 VLP, or recombinant soluble H5. The lowest dose of antigen (0.1 ug) when administered intramuscularly produced a superior HAI response to a 10-fold greater administration (5 ug) of recombinant soluble H5. Increasing doses of H5 VLP provided a modest increase in HAI over the lowest dose.

HAI response following intranasal administration was significantly increased in mice administered plant-made H5 VLPs (1.0 or 0.1 ug) compared to those administered 1 ug recombinant soluble H5, which was similar to the negative control. All mice immunized by intramuscular injection of H5 VLPs (from 0.1 to 12 µg) had higher HAI titers than mice immunised with the control H5 antigen (Figure 21A). For the same dose of 5 µg, VLPs induced HAI titers 20 times higher than the corresponding dose of the control H5 antigen. VLPs also induced significantly higher HAI titers than the control HA antigen when delivered through the intranasal route (Figure 21b). For a given dose of H5 VLP the levels of HAI titers were lower in mice immunised intranasally than for mice immunised intramuscularly; 1 µg VLP induced a mean HAI titer of 210 when administered i.m. while the same dose induced a mean HAI titer of 34 administered i.n..

When administered intramuscularly, all doses of VLPs induced high level of antibodies capable of binding homologous whole inactivated viruses (Figures 20a and 24). No significant difference was found between the plant-made VLP vaccine and the control H5 antigen (except the 12 µg VLP group 14 days after boost), as both antigen preparations induce high binding antibody titers against the homologous strain. However, when administered intranasally, VLPs induced higher binding antibody titers in than did the control H5 antigen (Figure 20b). When mixed with Chitosan, immunization with one microgram VLP induced a reciprocal mean Ab titer of 5 500, 8.6 times higher than the level found in mice immunized with 1 µg of the control HA antigen (reciprocal mean Ab titer of 920).

The immunogenicity of the plant-derived influenza VLPs was then investigated through a dose-ranging study in mice. Groups of five BALB/c mice were immunized intramuscularly twice at 3-week intervals with 0.1 µg to 12 µg of VLPs containing HA from influenza A/Indonesia/5/05 (H5N1) formulated in alum (1:1 ratio). Hemagglutination-inhibition titers (HI or HAI), using whole inactivated virus antigen (A/Indonesia/5/05 (H5N1)), were measured on sera collected 14 days after the second immunization. Immunization with doses of VLP as low as 0.1 µg induced the production of antibodies that inhibited viruses from agglutinating erythrocytes at high dilutions (Figure 21A). Parallel immunization of mice with 5µg of non-VLP alum-adjuvanted control H5 antigen (also from A/Indonesia/5/05) induce an HI response that was 2-3 logs lower than that achieved with the lowest VLP dose.

For both administration routes, and over a range of antigen quantities, the HAI response is superior in mice administered VLPs.

### Example 9: Effect of adjuvant on immunogenicity of H5 VLPs

Plant-made H5 VLPs have a plasma membrane origin (Figure 19, Example 5). Without wishing to be bound by theory, enveloped viruses or VLPs of enveloped viruses generally acquire their envelope from the membrane they bud through. Plant plasma membranes have a phytosterol complement that is rarely, if ever found in animal cells, and several of these sterols have been demonstrated to exhibit immunostimulatory effects.

Plant-made H5 VLPs were administered intramuscularly (Figure 22A) or intranasally (Figure 22B) to mice in the presence or absence of an adjuvant, and the HAI (hemagglutination inhibition antibody response) determined. VLPs, in the presence or absence of an added adjuvant (alum or chitosan, as in these examples) in either system of administration demonstrated a significantly greater HAI hemagglutinin inhibition than recombinant soluble H5. Even in the absence of an added adjuvant (i.e. alum or chitosan), plant-made H5 VLPs demonstrate a significant HAI, indicative of a systemic immune response to administration of the antigen.

Alum enhanced the mean level of HAI titers by a factor of 5 for intramuscular administration of VLP (Figure 22a) and by a factor of 3.7 for the control H5 antigen. When administered i.m., 5 µg VLPs induced a mean HAI titer 12 times higher than the corresponding dose of control H5 antigen. Chitosan did not boost the mean HAI level of the control H5 antigen (Figure 22b) while it increased the mean HAI level of mice immunised with 1 µg VLP administered i.n. by a factor of 5-fold.

### Example 10: Antibody isotypes

Mice administered plant-made H5 VLPs or recombinant soluble H5 in the presence or absence of alum as an added adjuvant demonstrate a variety of immunoglobulin isotypes (Figure 23A).

In the presence of an added adjuvant, the antibody isotype profiles of VLPs and the recombinant H5 are similar, with IgG1 being the dominant isotype. When VLPs or recombinant H5 are administered without an added adjuvant, IgG1 response is reduced, but remains the dominant isotype response to VLPs, with IgM, IgG2a, IgG2B and IgG3 maintaining similar titers as in the presence of an added adjuvant. IgG1, IgG2a, and IgG2b titers are markedly reduced when recombinant H5 is administered without an added adjuvant (Figure 23A).

These data, therefore, demonstrate that plant-made VLPs do not require an added adjuvant to elicit a antibody response in a host.

Antibody titers against whole inactivated influenza virus strains (A/Indonesia/5/05; A/Vietnam/1203/04)I in mice administered plant-made VLPs or soluble recombinant HA intramuscularly in the presence of an added antigen are illustrated in Figure 23B. No significant difference is observed in the antibody titers for these influenza strains in mice administered 1 ug or 5 ug of VLPs or 5 ug of soluble HA.

### Example 11: Cross-reactivity of serum antibodies induced by the H5 VLP vaccine

Cross-reactivity of serum antibodies induced by H5 VLP was assessed against whole inactivated influenza viruses of different strains. All VLP doses (from 0.1 to 12 µg) as well as 5 µg of control HA antigen induced high binding antibody titers against a clade 1 strain (A/Vietnam/1194/04), the homologous strain A/Indonesia/5/05 of clade 2.1, and a clade 2.2 strain A/turkey/Turkey/1/05 (Figure 25A).

However, only the plant-made VLP induced HAI titer against the A/turkey/Turkey/1/05 strain (Figure 25b). HAI titers for the A/Indonesia/5/05 were high for VLPs.

### Example 12: Cross-protection conferred by immunization with plant-made H5 VLP

Mice that previously had been administered a two-dose regimen of A/Indonesia/5/05 H5 VLPs as described, were subsequently challenged intranasally with influenza A/Turkey/582/06 (H5N1) ("Turkey H5N1") infectious virus, and observed. The dose administered, per animal, was 10 LD₅₀ (4.09 X 10⁵ CCID₅₀).

By 7 days post-challenge, only 37.5% of the mice administered the PBS vaccine control had survived exposure to Turkey H5N1 (Figure 26A). 100% of animals administered the control antigen (HA) or 1, 5 or 15 ug of Indonesia H5 VLPs survived up to 17 days post-challenge, when the experiment was terminated.

Body mass of the mice was also monitored during the experiment, and the average mass of the surviving mice plotted (Figure 26B). Mice administered 1, 5 or 15 ug of the Indonesia H5 VLPs before challenge did not lose any appreciable mass during the course of the experiment, and in particular mice administered 5 ug of the VLPs appear to have gained significant mass. Negative control mice (no Turkey H5N1 challenge) did not appreciably gain or lose body mass. Positive control mice (not administered VLPs, but challenged with Turkey H5N1) exhibited significant loss of body mass during the course of the experiment, and three of these mice died. As body mass is an average of all mice in the cohort, removal of the 'sickest' mice (the 3 that died) may lead to an apparent overall increase in mass, however note that the average body mass of the positive control cohort is still significantly below that of the negative or the VLP-treated cohorts.

These data, therefore, demonstrate that plant-made influenza VLPs comprising the H5 hemagglutinin viral protein induce an immune response specific for pathogenic influenza strains, and that virus-like particles may bud from a plant plasma membrane.

These data, therefore, demonstrate that plants are capable of producing influenza virus-like particles, and also for the first time, that virus-like particles can bud from a plant plasma membrane.

Further, using the current transient expression technology, a first antigen lot was produced only 16 days after the sequence of the target HA was obtained. Under the current yields for H5 VLPs, and at an exemplary dose of 5 µg per subject, each kg of infiltrated leaf may produce ~20,000 vaccine doses. This unique combination of platform simplicity, surge capacity and powerful immunogenicity provides for, among other embodiments, a new method response in the context of a pandemic.

### Example 13: Characterization of hemagglutinin-containing (H1, H2, H3, H5, H6 and H9) structures in plant extracts using size exclusion chromatography

The assembly of plant-produced influenza hemagglutinin of different subtypes into high molecular weight structures was assessed by gel filtration. Crude or concentrated protein extracts from AGL1/660-, AGL1/540-, AGL1/783-, AGL1/780-, AGL1/785- and AGL1/790-infiltrated plants (1.5 mL) were fractionated by size exclusion chromatography (SEC) on Sephacryl™ S-500 HR columns (GE Healthcare Bio-Science Corp., Piscataway, NJ, USA). As shown in Figure 46, Blue Dextran (2 MDa) elution peaked early in fraction 10. When proteins from 200 µL of each SEC elution fraction were concentrated (5-fold) by acetone-precipitation and analyzed by Western blotting (Figure 46), hemagglutinins were primarily found in fractions 7 to 14, indicating the incorporation of HA into VLPs. Without wishing to be bound by theory, this suggests that the HA protein had either assembled into a large superstructure or that it has attached to a high molecular weight structure, irrespectively of the subtype produced. In Figure 46, H1 from strain A/New Caledonia/20/1999 and H3 from strain A/Brisbane/10/2007 were produced using PDI signal peptide-containing cassettes. The results obtained indicate that replacement of the native signal peptide by that of alfalfa PDI does not affect the abiity of HA to assemble into particles.

### Example 14: Transient expression of seasonal influenza virus hemagglutinin by agroinfiltration in N. benthamiana plants using the wild-type nucleotide sequence

The ability of the transient expression system to produce seasonal influenza hemagglutinins was determined through the expression of the H1 subtype from strains A/Brisbane/59/2007 (H1N1) (plasmid #774), A/New Caledonia/20/1999 (H1N1) (plasmid #540) and A/Solomon Islands/3/2006 (H1N1) (plasmid #775), of the H3 subtype from strains A/Brisbane/10/2007 (plasmid #776) and A/Wisconsin/67/2005 (plasmid #777) and of the B type from strains B/Malaysia/2506/2004 (Victoria lineage) (plasmid #778) and B/Florida/4/2006 (Yamagata lineage) (plasmid #779). The hemagglutinin gene coding sequences were first assembled in the plastocyanin expression cassette - promoter, 5'UTR, 3'UTR and transcription termination sequences from the alfalfa plastocyanin gene - and the assembled cassettes were inserted into to a pCAMBIA binary plasmid. The plasmids were then transfected into *Agrobacterium* (AGL1), producing *Agrobacterium* strains AGL1/774, AGL1/540, AGL1/775, AGL1/776, AGL1/777, AGL1/778 and AGL1/779, respectively.

*N. benthamiana* plants were infiltrated with AGL1/774, AGL1/540, AGL1/775, AGL1/776, AGL1/777, AGL1/778 and AGL1/779 and the leaves were harvested after a six-day incubation period. To determine whether H1 accumulated in the agroinfiltrated leaves, protein was first extracted from infiltrated leaf tissue and analyzed by Western blotting using anti-HA antibodies (see Table 6 for the antibodies and conditions used for the detection of each HA subtype). For the HA from H1 strains, a unique band of approximately 72 kDa was detected in extracts (Figure 47), corresponding in size to the uncleaved HA0 form of influenza hemagglutinin. This demonstrated that expression of different annual epidemic strains of hemagglutinin in infiltrated leaves results in the accumulation of the uncleaved translation product. Using these expression and immunodetection strategies, the expression of influenza HA from H3 subtype or B type was not detected in the crude protein extracts (Figure 47).

### Example 15: Transient expression of potential pandemic influenza virus hemagglutinin by agroinfiltration in N. benthamiana plants using the wild-type nucleotide sequence

The ability of the transient expression system to produce potential influenza hemagglutinins was determined through the expression of the H5 subtype from strains A/Anhui/1/2005 (H5N1) (plasmid #781), A/Indonesia/5/2005 (H5N1) (plasmid #660) and A/Vietnam/1194/2004 (H5N1) (plasmid #782), the H2 subtype from strain A/Singapore/1/1957 (H2N2) (plasmid #780), the H6 from strain A/Teal/Hong Kong/W312/1997 (H6N1) (plasmid # 783), the H7 for strain A/Equipe/Prague/1956 (H7N7) (plasmid #784) and finally H9 from strain A/Hong Kong/1073/1999 (H9N2) (plasmid # 785). The hemagglutinin gene coding sequences were first assembled in the plastocyanin expression cassette - promoter, 5'UTR, 3'UTR and transcription termination sequences from the alfalfa plastocyanin gene - and the assembled cassettes were inserted into to a pCAMBIA binary plasmid. The plasmids were then transfected into *Agrobacterium* (AGL1), producing *Agrobacterium* strains AGL1/781, AGL1/660, AGL1/782, AGL1/780, AGL1/783, AGL1/784 and AGL1/785.

*N. benthamiana* plants were infiltrated with AGL1/781, AGL1/660, AGL1/782, AGL1/780, AGL1/784 and AGL1/785, and the leaves were harvested after a six-day incubation period. To determine whether H5 accumulated in the agroinfiltrated leaves, protein was first extracted from infiltrated leaf tissue and analyzed by Western blotting using appropriate anti-HA antibodies (see Table 6 for the antibodies and conditions used for the detection of each HA subtype). A unique band of approximately 72 kDa was detected in extracts of plants transformed with H5 and H2 expression constructs (Figure 48a and b), corresponding in size to the uncleaved HA0 form of influenza hemagglutinin. This demonstrated that expression of different potential pandemic strains of hemagglutinin in infiltrated leaves results in the accumulation of the uncleaved translation product. Using these expression and immunodetection strategies, the expression of influenza HA from H7 and H9 was not detected in the crude protein extracts (Figure 48b).

### Example 16: Transient expression of H5 by agroinfiltration in N. tabacum plants

The ability of the transient expression system to produce influenza hemagglutinin in leaves of *Nicotiana tabacum* was analysed through the expression of the H5 subtype from strain A/Indonesia/5/2005 (H5N1) (plasmid #660). The hemagglutinin gene coding sequences were first assembled in the plastocyanin expression cassette - promoter, 5'UTR, 3'UTR and transcription termination sequences from the alfalfa plastocyanin gene - and the assembled cassettes were inserted into to a pCAMBIA binary plasmid. The plasmids was then transfected into *Agrobacterium* (AGL1), producing strain AGL1/660.

*N. tabacum* plants were infiltrated with AGL1/660 and the leaves were harvested after a six-day incubation period. To determine whether H5 accumulated in the agroinfiltrated leaves, proteins were first extracted from infiltrated leaves and analyzed by Western blot using anti-H5 antibodies. A unique band of approximately 72 kDa was detected in extracts (Figure 49), corresponding in size to the uncleaved HA0 form of influenza hemagglutinin. This demonstrated that expression of hemagglutinin in infiltrated *N. tabacum* leaves results in the accumulation of the uncleaved HA0 precursor.

### Example 17: Immunogenicity of plant-made H5N1 VLP vaccine from A/Indonesia/5/05 (H5N1) in ferrets

A dose escalation study in ferrets was performed to evaluate the immunogenicity of plant derived VLPs. In vitro cross-reactivity of serum antibody induced by the H5 VLP vaccine at 3 doses (1, 5 and 15 ug) was assessed by hemagglutination inhibition of three other H5N1 strains - A/turkey/Turkey/1/05 (clade 2.2), A/Vietnam/1194/04 (clade 1) and A/Anhui/5/05 (all whole, inactivated virus), using serum taken 14 days after the first dose of vaccine (Figure 50A), and 14 days after the 2^{nd} dose (Figure 50 B). For all 3 dose concentrations, cross-reactivity is observed

### Example 18: Analysis of the immunogenicity results according to CHMP criteria.

The EMEA's Committee for Medicinal Products for Human Use (CHMP) (http://www.emea.europa.eu/htms/general/contacts/CHMP/CHMP.html) sets out three criteria (applied following the second dose) for vaccine efficacy: 1 - Number of seroconversion or significant increase in HI titers (4-fold) >40%; 2 - Mean geometric increase of at least 2.5; 3 - proportion of subjects achieving an HI titer of 1/40 should be at least 70%. Analysis of these criteria in the ferret model is shown in Tables 8-11. (*) is indicative of meeting or exceeding the CHMP criteria. A summary of cross-immunogenicity analysis in relation to CHMP criteria for licensure is shown in Table 12.

Animals were assessed daily for body weight, temperature and overall condition. No sign of sickness or discomfort was recorded during the study. Body weight and temperature was within normal ranges during the study. The vaccine was safe and tolerated by the animals.

**Table 8: Data for homologous strain (A/Indonesia/5/05)**

| **Day** | **Criteria** | **Study group** | | | | | | | | |
|---|---|---|---|---|---|---|---|---|---|---|
| | | 1 µg | 1 µg adjuvanted | 5 µg | 5 µg adjuvanted | 7.5 µg | 15 µg | 15 µg adjuvanted | 30 µg | 5 µg ITC |
| 14 (post 1st inj.) | % 4-fold increase in HI titer | 0% | 100% | 0% | **100 % *** | 20% | 20% | **80% *** | 0% | 0% |
| | Mean geometric increase | 0% | 7.6 | 0% | **15.6 *** | 1.3 | 1.2 | **11.2 *** | 0% | 0% |
| | % of HI titer of 1/40 | 0% | 60% | 0% | **100% *** | 20% | 0% | **80% *** | 0% | 0% |
| | *Mean HI titer* | | *38* | | *78* | | | *56* | | |
| 35(14 days post boost) | % 4-fold increase in HI titer | 0% | **100% *** | 0% | **60% *** | 0% | 0% | **40% *** | 0% | 0% |
| | Mean geometric increase | 0% | **10.8 *** | 0% | **5.9 *** | 0.7 | 0% | **4 *** | 0% | 0% |
| | % of HI titer of 1/40 | 0% | **100** % ***** | 0% | **100% *** | 0% | 0% | **100 % *** | 0% | 0% |
| | *Mean HI titer* | | *411* | | *465* | | | *217* | | |

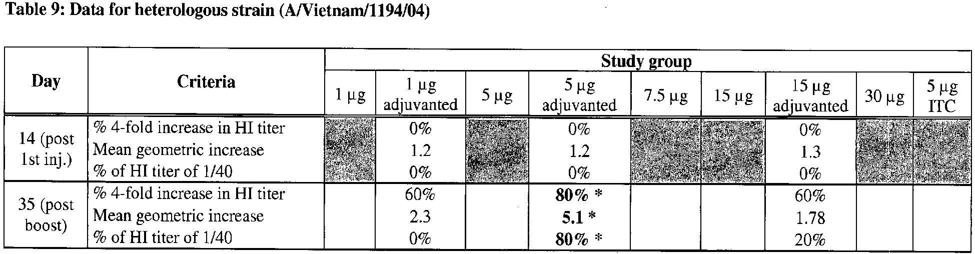

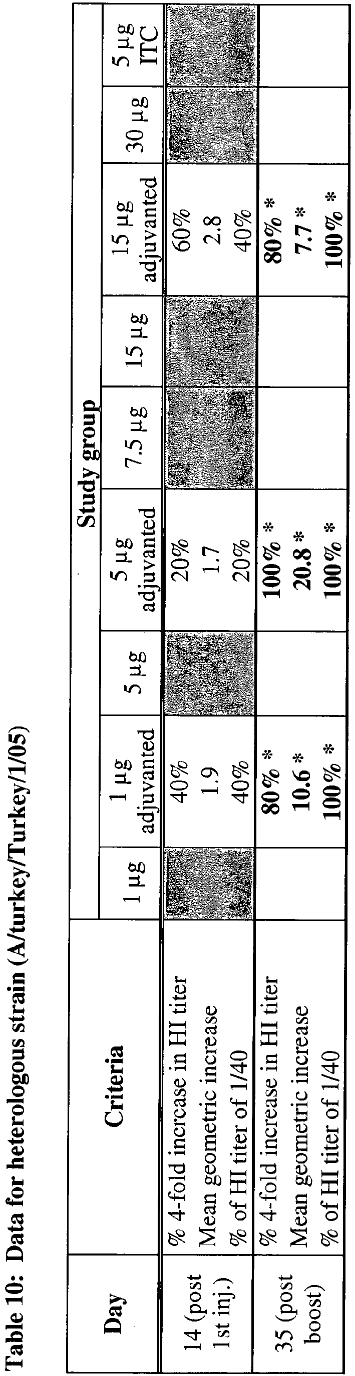

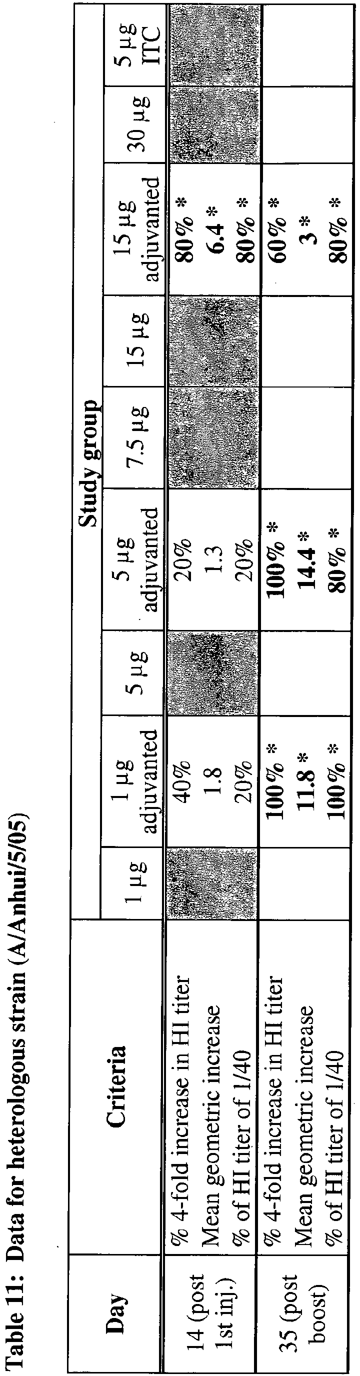

**Table 12: Summary of cross-immunogenicity analysis in relation to CHMP criteria for licensure.**

| **Strain** | **Criteria** | **Study group** | | |
|---|---|---|---|---|
| | | 1 µg adjuvanted | 5 µg adjuvanted | 15 µg adjuvanted |
| A/turkey/Turkey/1/05 (clade 2.2 | % 4-fold increase in HI titer | **80% *** | **100% *** | **80% *** |
| | Mean geometric increase | **10.6 *** | **20.8 *** | **7.7 *** |
| | % of HI titer of 1/40 | **100% *** | **100% *** | **100% *** |
| A/Anhui/1/05 (clade 2.3) | % 4-fold increase in HI titer | **100% *** | **100% *** | **60% *** |
| | Mean geometric increase | **11.8 *** | **14.4 *** | **3 *** |
| | % of HI titer of 1/40 | **100% *** | **80% *** | **80% *** |
| A/Vietnam/1194/04 (clade 1) | % 4-fold increase in HI titer | 60% | **80% *** | 60% |
| | Mean geometric increase | 2.3 | **7.1 *** | 1.78 |
| | % of HI titer of 1/40 | 0% | **80% *** | 20% |

### Example 19: Selection of hemagglutinin nucleotide sequences

The nucleotide sequences of the HA were retrieved from an influenza sequence database (see URL: flu.lanl.gov), or the NCBI influenza virus resource (Bao et al., 2008. J. Virology 82(2): 596-601; see URL: ncbi.nlm.nih.gov/genomes/FLU/FLU.html). For several of the HA nucleic acid sequences, multiple entries are listed in the databases (Table 13). Some variation is associated primarily with the culture system (Origin - MDCK, egg, unknown, viral RNA/clinical isolate); for example, the glycosylation site at position 194 (mature protein numbering) of the HA is absent when type B influenza virus is expressed in allantoic fluid of eggs (see also Chen et al., 2008). For some sequences, domains may be lacking (e.g. incomplete clones, sequencing artifacts, etc.). Domains and sub-domains of influenza hemagglutinin are discussed generally in the Descrition. Domains or subdomains of a first sequence may be combined with a domain from a second existing sequence e.g. the signal peptide of a first strain sequence may be combined with the balance of the hemagglutinin coding sequence from a second strain to provide a complete coding sequence.

**Table 13: Variation in Influenza subtypes for selected HA coding sequences**

| | **Strain** | **Sequence database reference No.** | **Origin** | **SP** | **HA1** | **HA2** | **DTm** | **Divergence** |
|---|---|---|---|---|---|---|---|---|
| H1 | A/Solomon Islands/3/20 06 | ISDN2315 58 (Vaccine rec.) | MDCK | Y | Y | Y | Y | 189: R ou G, 220: K (MDCK) T(Egg), 249: Q (MDCK) R(Egg), 550: L (MDCK) R (Egg) |
| | A/Solomon Islands/3/20 06 | ISDN2381 90 | Egg | Y | Y | Y | Y | 189: R ou G, 220: K (MDCK) T(Egg), 249: Q (MDCK) R(Egg), 550: L (MDCK) R (Egg) |
| | A/Solomon Islands/3/20 06 | EU100724 | ? | Y | Y | Y | Y | 189: R ou G, 220: K (MDCK) T(Egg), 249: Q (MDCK) R(Egg), 550: L (MDCK) R (Egg) |
| | A/Solomon Islands/3/20 06 | ISDN2209 51 | MDCK | Y | Y | N | N | 189: R ou G, 220: K (MDCK) T(Egg), 249: Q (MDCK) R(Egg), 550: L (MDCK) R (Egg) |
| | A/Solomon Islands/3/20 06 | ISDN2209 53 | Egg | Y | Y | N | N | 189: R ou G, 220: K (MDCK) T(Egg), 249: Q (MDCK) R(Egg), 550: L (MDCK) R (Egg) |
| | A/Solomon Islands/3/20 06 | EU124137 | Egg | Y | Y | N | N | 189: R ou G, 220: K (MDCK) T(Egg), 249: Q (MDCK) R(Egg), 550: L (MDCK) R (Egg) |
| | A/Solomon Islands/3/20 06 | EU124135 | MDCK | Y | Y | N | N | 189: R ou G, 220: K (MDCK) T(Egg), 249: Q (MDCK) R(Egg), 550: L (MDCK) R (Egg) |
| | A/Solomon Islands/3/20 06 | EU124177 | MDCK | Y | Y | Y | Y | 189: R ou G, 220: K (MDCK) T(Egg), 249: Q (MDCK) R(Egg), 550: L (MDCK) R (Egg) |
| | | | | | | | | |
| H1 | A/Brisbane/5 9/2007 | ISDN2826 76 | MDCK | Y | Y | Y | | 203: D/I/N D est le plus abondant chez les H1 |
| | A/Brisbane/ 59/2007 | ISDN2851 01 | Egg | Y | Y | N | N | 203: D/I/N D est le plus abondant chez les H1 |
| | A/Brisbane/ 59/2007 | ISDN2857 77 | Egg | Y | Y | Y | Y | 203: D/I/N D est le plus abondant chez les H1 |
| | A/Brisbane/ 59/2007 | ISDN2826 77 | Egg | Y | Y | Y | Y | 203: D/I/N D est le plus abondant chez les H1 |
| | | | | | | | | |
| H3 | A/Brisbane/1 0/2007 | ISDN2748 93 | Egg | Y | Y | Y | Y | 202: V/G, 210:L/P, 215: del Ala, 242: S/I |
| | A/Brisbane/ 10/2007 | ISDN2576 48 | MDCK | N | Y | Y | Y | 202: V/G, 210:L/P, 215: del Ala, 242: S/I |
| | A/Brisbane/ 10/2007 | ISDN2567 51 | Egg | Y | Y | Y | Y | 202: V/G, 210:L/P, 215: del Ala, 242: S/I |
| | A/Brisbane/ 10/2007 | ISDN2737 57 | Egg | Y | Y | Y | Y | 202: V/G, 210:L/P, 215: del Ala, 242: S/I |
| | A/Brisbane/ 10/2007 | ISDN2737 59 | Egg | Y | Y | Y | Y | 202: V/G, 210:L/P, 215: del Ala, 242: S/I |
| | A/Brisbane/ 10/2007 | EU199248 | Egg | N | Y | Y | Y | 202: V/G, 210:L/P, 215: del Ala, 242: S/I |
| | A/Brisbane/ 10/2007 | EU199366 | Egg | Y | Y | Y | Y | 202: V/G, 210:L/P, 215: del Ala, 242: S/I |
| | A/Brisbane/ 10/2007 | ISDN2570 43 | Egg | N | Y | Y | Y | 202: V/G, 210:L/P, 215: del Ala, 242: S/I |
| | A/Brisbane/ 10/2007 | EU199250 | MDCK | N | Y | Y | Y | 202: V/G, 210:L/P, 215: del Ala, 242: S/I |
| | A/Brisbane/ 10/2007 | ISDN2753 57 | Egg | N | Y | N | N | 202: V/G, 210:L/P, 215: del Ala, 242: S/I |
| | A/Brisbane/ 10/2007 | ISDN2604 30 | Egg | N | Y | Y | Y | 202: V/G, 210:L/P, 215: del Ala, 242: S/I |
| | | | | | | | | |
| H3 | A/Wisconsin /67/2005 | ISDN1314 64 (vaccine rec.) | ? | N | Y | Y | N | 138: A/S |
| | | | | | | | | 156: H/Q |
| | | | | | | | | 186: G/V |
| | | | | | | | | 196: H/Y |
| | A/Wisconsi n/67/2005 | DQ865947 | ? | N | Y | parti el | N | 138: A/S |
| | | | | | | | | 156: H/Q |
| | | | | | | | | 186: G/V |
| | | | | | | | | 196: H/Y |
| | A/Wisconsi n/67/2005 | EF473424 | ? | N | Y | Y | N | 138: A/S |
| | | | | | | | | 156: H/Q |
| | | | | | | | | 186: G/V |
| | | | | | | | | 196: H/Y |
| | AlWisconsi n/67/2005 | ISDN1387 23 | Egg | N | Y | Y | Y | 138: A/S |
| | | | | | | | | 156: H/Q |
| | | | | | | | | 186: G/V |
| | | | | | | | | 196: H/Y |
| | A/Wisconsi n/67/2005 | E473455 | Egg | N | Y | Y | Y | 138: A/S |
| | | | | | | | | 156: H/Q |
| | | | | | | | | 186: GN |
| | | | | | | | | 196: H/Y |
| | A/Wisconsi n/67/2005 | ISDN1387 24 | ? | N | Y | Y | Y | 138: A/S |
| | | | | | | | | 156: H/Q |
| | | | | | | | | 186: G/V |
| | | | | | | | | 196: H/Y |
| | | | | | | | | |
| B | B/Malaysia/ 2506/2004 | ISDN1266 72 (vaccine rec.) | Egg | Y | Y | N | N | 120 K/N |
| | | | | | | | | 210 T/A |
| | B/Malaysia/ 2506/2004 | EF566433 | Egg | Y | Y | N | N | 120 K/N |
| | | | | | | | | 210 T/A |
| | B/Malaysia/ 2506/2004 | ISDN2312 65 | Egg | Y | Y | Y | Y | 120 K/N |
| | | | | | | | | 210 T/A |
| | B/Malaysia/ 2506/2004 | ISDN2315 57 | MDCK | Y | Y | Y | Y | 120 K/N |
| | | | | | | | | 210 T/A |
| | B/Malaysia/ 2506/2004 | EF566394 | MDCK | Y | Y | N | N | 120 K/N |
| | | | | | | | | 210 T/A |
| | B/Malaysia/ 2506/2004 | EU124274 | Egg | Y | Y | Y | Y | 120 K/N |
| | | | | | | | | 210 T/A |
| | B/Malaysia/ 2506/2004 | EU 124275 | MDCK | Y | Y | Y | Y | 120 K/N |
| | | | | | | | | 210 T/A |
| | B/Malaysia/ 2506/2004 | ISDN1247 76 | MDCK | Y | Y | N | N | 120 K/N |
| | | | | | | | | 210 T/A |
| | | | | | | | | |
| B | B/Florida/4/ 2006 | ISDN2616 49 | Egg | Y | Y | Y | N | lacking glycosylation site at position 211; 10 amino acids of DTm/cytoplasmic tail |
| | B/Florida/4/ 2006 | EU100604 | MDCK | N | Y | N | N | |
| | B/Florida/4/ 2006 | ISDN2180 61 | MDCK | N | Y | N | N | |
| | B/Florida/4/ 2006 | ISDN2857 78 | Egg | Y | Y | Y | Y | Includes cytoplasmic tail |
| | | | | | | | | |
| B | B/Brisbane/3 /2007 | ISDN2566 28 | Egg | N | Y | N | N | lacking glycosylation site at position 211 |
| | B/Brisbane/ 3/2007 | ISDN2637 82 | Egg | Y | Y | Y | Y | lacking glycosylation site at position 211 |
| | B/Brisbane/ 3/2007 | ISDN2637 83 | MDCK | Y | Y | Y | Y | |
| | | | | | | | | |
| H5 | A/Viet Nam/1194/2 004 | ISDN3868 6 (Vaccine rec.) | ? | Y | Y | Y | Y | |
| | A/Viet Nam/1194/2 004 | AY651333 | ? | Y | Y | Y | Y | |
| | A/Viet Nam/1194/2 004 | EF541402 | ? | Y | Y | Y | Y | |
| | | | | | | | | |
| H5 | A/Anhui1/1/ 2005 | DQ37928 (vaccine rec.) | ? | Y | Y | Y | Y | |
| | A/Anhui1/1/ 2005 | ISDN1314 65 | Egg | Y | Y | Y | Y | |
| | | | | | | | | |
| H7 | A/Chicken/It aly/13474/19 99 | AJ91720 | ARN gen | Y | Y | Y | Y | |
| | | | | | | | | |
| H7 | A/Equine/Pr ague/56 | AB298277 (Lab reassortant) | ? | Y | Y | Y | Y | 152 (R/G) |
| | | | | | | | | 169 (T/I) |
| | | | | | | | | 208 (N/D) (glycosylation site abolished) |
| | A/Eguine/Pr ague/56 | X62552 | ? | Y | Y | Y | Y | |
| | | | | | | | | |
| H9 | A/Hong Kong/1073/1 999 | AJ404626 | ? | Y | Y | Y | Y | |
| | A/Hong Kong/1073/1 999 | AB080226 | ? | N | Y | N | N | |
| | | | | | | | | |
| H2 | A/Singapore/ 1/1957 | AB296074 | ? | Y | Y | Y | Y | |
| | A/Singapore /1/1957 | L20410 | RNA | Y | Y | Y | Y | |
| | A/Singapore /1/1957 | L11142 | ? | Y | Y | Y | Y | |
| | | | | | | | | |
| H2 | A/Japan/305/ 1957 | L20406 | ? | Y | Y | Y | Y | |
| | A/Japan/305 /1957 | L20407 | ? | Y | Y | Y | Y | |
| | A/Japan/305 /1957 | CY014976 | ? | Y | Y | Y | Y | |
| | A/Japan/305 /1957 | AY209953 | ? | Y | Y | N | N | |
| | A/Japan/305 /1957 | J02127 | ? | Y | Y | Y | Y | |
| | A/Japan/305 /1957 | DQ508841 | ? | Y | Y | Y | Y | |
| | A/Japan/305 /1957 | AY643086 | ? | Y | Y | Y | N | |
| | A/Japan/305 /1957 | AB289337 | ? | Y | Y | Y | Y | |
| | A/Japan/305 /1957 | AY643085 | ? | Y | Y | Y | Y | |
| | A/Japan/305 /1957 | AY643087 | Drug resistan t | Y | Y | Y | N | |
| | | | | | | | | |
| H6 | A/Teal/Hong Kong/W312/ 1997 (H6N1) | AF250479 | Egg | Y | Y | Y | Y | |

| | | | | | | | | |
|---|---|---|---|---|---|---|---|---|
| Y, N - Yes, No, respectively SP - presence of signal peptide sequence Y/N HA1 - complete HA1 domain Y/N HA2 - complete HA2 domain Y/N DTm - complete transmembrane domain Y/N | | | | | | | | |

### Strain: H1 from A/Solomon Islands/3/2006

Eight amino acid sequences were compared, and variations identified. (Table 14). Position 171 exhibited a variation of glycine (G) or arginine (R) in some sequences.

**Table 14: A/Solomon Islands/3/2006 amino acid variation**

| Amino acid #* | MDCK | Egg |
|---|---|---|
| 212 | K | T |
| 241 | Q | R |
| 542 | L | R |

| | | |
|---|---|---|
| Numbering from the starting M | | |

### Strain: H1 from A/Brisbane/59/2007

Position 203 exhibited a variation of aspartic acid (D), isoleucine (I) or asparagine (N).

### Strain: H3 from A/Brisbane/10/2007

Sequence variations were observed at 5 positions (Table 15). In position 215, a deletion is observed in two sampled sequences.

**Table 15: H3 from A/Brisbane/10/2007 amino acid variation**

| | Origin | 202, 210, 215, 235 242* | | | | |
|---|---|---|---|---|---|---|
| ISDN274893 | Egg | V | L | - | Y | I |
| ISDN273759 | Egg | G | P | A | S | I |
| EU 199248 | Egg | G | P | A | S | I |
| EU199366 | Egg | G | P | A | S | I |
| ISDN273757 | Egg | V | L | - | S | S |
| ISDN257043 | Egg | G | P | A | S | I |
| EU199250 | MDCK | G | L | A | S | I |
| ISDN375357 | Egg | G | P | A | S | I |
| ISDN260430 | Egg | G | P | A | S | I |
| ISDN256751 | Egg | G | P | A | S | I |
| ISDN257648 | MDCK | G | L | A | S | I |

| | | | | | | |
|---|---|---|---|---|---|---|
| *Numbering from the starting M | | | | | | |

### Strain: H3 from A/Wisconsin/67/2005

Sequence variations in this strain were observed at 4 positions (Table 16).

**Table 16: H3 from A/Wisconsin/67/2005 amino acid variation**

| | Origin | 138, 156, 186, 196 | | | |
|---|---|---|---|---|---|
| ISDN138724 | Unknown | A | H | G | H |
| DQ865947 | Unknown | S | H | V | Y |
| EF473424 | Unknown | A | H | G | H |
| ISDN138723 | Egg | S | Q | V | Y |
| ISDN 131464 | Unknown | A | H | G | H |
| EF473455 | Egg | A | H | G | H |

| | | | | | |
|---|---|---|---|---|---|
| *Numbering from the mature protein | | | | | |

### Strain: B from B/Malaysia/2506/2004

Variation at two positions is observed (Table 17). Position 120 is not a glycosylation site; position 210 is involved in glycosylation; this glycosylation is abolished following culture in eggs.

**Table 17: Hemagglutinin from B/Malaysia/2506/2004 amino acid variation**

| Amino acid #* | MDCK | Egg |
|---|---|---|
| 120 | K | N |
| 210 | T | A |

| | | |
|---|---|---|
| * Numbering from the middle of SP | | |

### Strain: hemagglutinin from B/Florida/4/2006; ISDN261649

Obseved variations include amino acid sequence variation at position 211, depending on the culture system. Asparatine (N) is found in sequences isolated from MDCK cells, while glutamic acid (D) is found in sequence isolated from eggs. Position 211 is a glycosylation site, and is abolished following culture in eggs.

### Strain: H2 from A/Singapore/1/1957

Sequence variations were observed in 6 position s (Table 18).

**Table 18: H2 from A/Singapore/1/1957 amino acid variation**

| | Origin | Amino acid No. | | | | | |
|---|---|---|---|---|---|---|---|
| | | 166 168 199\236 238 358 | | | | | |
| L20410 | Viral RNA | K | E | T | L | S | V |
| L11142 | Unknown | E | G | K | L | S | I |
| AB296074 | Unknown | K | G | T | Q | G | V |
| Consensus A/Japan/305/1957 | | K | G | T | Q/L | G | V |

| | | | | | | | |
|---|---|---|---|---|---|---|---|
| ¹ Numbering from the mature protein | | | | | | | |

### Straits: H5 from A/Vietnam/1194/2004 and H5 from A/Anhui/1/2005

There were no variations observed in the amino acid sequence upon aligning the primary sequences of either of these H5 strains.

### Strain: H6 from A/Teal/Hong Kong/W312/1997

Only one entry was available for strain (AF250179).

### Strain: H7 from A/Equine/Prague/56

A total of 2 sequence entries were found in the databases. The entry AB298877 was excluded as it is a laboratory reassortant.

### Strain: H9 from A/Hong Kong/1073/1999; AJ404626

A total of 2 sequence entries were found in the databases. Only one was complete.

### Example 20. Transient expression of influenza virus hemagglutinin fused to a signal peptide from a plant secreted protein.

The effect of signal peptide modification on HA accumulation level for other hemagglutinins was also investigated through the expression of the A subtype HAs from strains A/Brisbane/59/2007 (H1N1) (plasmid #787), A/New Caledonia/20/1999 (H1N1) (plasmid #540), from strains ABrisbane/10/2007 (H3N2) (plasmid 790) and A/Indonesia/5/2005 (H5N1) (plasmid #663) and of the B type from strains B/Florida/4/2006 (plasmid #798) fused to the signal peptide (SP; nucleotides 32-103) from of alfalfa protein disulfide isomerase (PDI; accession No. Z11499; SEQ. ID. NO: 34; Figure 17). The PDI SP-hemagglutinin gene fusions were assembled in the plastocyanin expression cassette - promoter, 5'UTR, 3'UTR and transcription termination sequences from the alfalfa plastocyanin gene - and the assembled cassettes were inserted into to a pCAMBIA binary plasmid. The plasmids were then transfected into *Agrobacterium* (AGL1), producing *Agrobacterium* strains AGL1/787, AGL1/540, AGL1/790, AGL1/663 and AGL1/798, respectively.

*N. benthamiana* plants were infiltrated with AGL1/787, AGL1/540, AGL1/790, AGL1/663 and AGL1/798. In parallel, a series of plants was infiltrated with AGL1/774, AGL776, AGL1/660 and AGL1/779 for comparison purposes. Leaves were harvested after a six-day incubation period and proteins were extracted from infiltrated leaves and analyzed by Western blot using the appropriate anti-HA antibodies. The expression of HA from H1/Brisbane and H3/Brisbane were considerably improved using the SP from PDI compared to the expression observed for the same HAs with their native signal peptide (Fig. 87b and c, respectively). The expression of a third HA from subtype H1 (strain A/New Caledonia/20/1999) was confirmed using this SP replacement strategy (Fig. 87a). The modification of sognal peptide did not lead to substantial increase in HA accumulation for H5 (A/Indonesia/5/2005) (Figure 87d), and no signal was detected for HA from strain B/Florida/4/2006, irrespectively of the signal peptide used for expression (Figure 87e). For all the conditions where the expression of HA was detected , a unique immunoreactive band was observed at a molecular weight of approximately 72 kDa (Fig. 87a to d), corresponding in size to the uncleaved HA0 precursor.

### Example 21. HA expression under the control of CPMV-HT expression cassette.

An expression cassette CPMV-HT (Sainsbury et al. 2008 Plant Physiology 148: 1212-1218; see also WO 2007/135480) comprising untranslated sequences from the Cowpea mosaic virus (CPMV) RNA2 was used for expression of some hemagglutinins in transgenic plants. HA from A/New Caledonia/20/1999 (H1), ABrisbane/59/2007 (H1), A/Brisbane/10/2007 (H3), A/Indonesia/5/2005 (H5) and B/Florida/4/2006 (B) were expressed under the control of CPMV-*HT* in *N. benthamiana* plants, agroinfiltrated as described. After incubation, leaves were harvested, extracted and HA contents in protein extracts were compared by Western blot. As shown in Figure 88, the CPMV-HT expression cassette led to higher HA expression level than the plastocyanin cassette, irrespectively of the signal peptide used. Furthermore, for strain B from B/Florida/4/2006, the use of CPMV-HT expression cassette allowed the detection of HA accumulation which remained undetectable under these immunodetection conditions when expressed under the plastocyanin cassette.

**Table 19: Expression cassette used for expression of influenza hemagglutinins with native or PDI signal peptides.**

| Agro strain | HA expressed | Signal Peptide | Expression Cassette |
|---|---|---|---|
| AGL1/560 | H1 (A/California/04/09) | PDI | 2X35S/CPMV-HT |
| AGL1/540 | H1 (A/New Caledonia/20/99) | PDI | Plastocyanin |
| AGL1/580 | H1 (A/New Caledonia/20/99) | PDI | CPMV-HT |
| AGL1/774 | H1 (A/Brisbane/59/2007) | native | Plastocyanin |
| AGL1/787 | H1 (A/Brisbane/59/2007) | PDI | Plastocyanin |
| AGL1/732 | H1 (A/Brisbane/59/2007) | native | CPMV-HT |
| AGL1/776 | H3 (ABrisbane/10/2007) | native | Plastocyanin |
| AGL1/790 | H3 (A/Brisbane/10/2007) | PDI | Plastocyanin |
| AGL1/735 | H3 (A/Brisbane/10/2007) | native | CPMV-HT |
| AGL1/736 | H3 (A/Brisbane/10/2007) | PDI | CPMV-HT |
| AGL1/660 | H5 (A/Indonesia/5/2005) | native | Plastocyanin |
| AGL1/685 | H5 (A/Indonesia/5/2005) | native | CPMV-HT |
| AGL1/779 | B (B/Florida/4/2006) | native | Plastocyanin |
| AGL1/798 | B (B/Florida/4/2006) | PDI | Plastocyanin |
| AGL1/738 | B (B/Florida/4/2006) | native | CPMV-HT |
| AGL1/739 | B (B/Florida/4/2006) | PDI | CPMV-HT |

### Example 22. Co-expression with Hsp70 and Hsp40 in combination with signal peptide modification.

Cytosolic Hsp70 and Hsp40 (construct number R870) of plant origin were co-expressed with H1 New Caledonia (construct number 540) or H3 Brisbane (construct number 790), both bearing a signal peptide of plant origin (alfalfa PDI signal peptide). The co-expression was performed by agroinfiltration of *N. benthamiana* plants with a bacterial suspension containing a mixture (1:1:1 ratio) of AGL1/540, AGL1/R870, AGL1/35SHcPro (For H1) or AGL1/790, AGL1/R870 and AGL1/35SHcPro (for H3). Control plants were agroinfiltrated with a mixture (1:2 ratio) of AGL1/540, AGL1/35SHcPro (for H1) or AGL1/790, AGL1/35SHcPro (for H3). After incubation, leaves were harvest, extracted and HA contents in protein extracts were compared by Western blot (Figure 89). In the conditions tested the results obtained indicate that the co-expression of Hsp70 and Hsp40 did not increase hemagglutinin accumulation level for H1 New Caledonia. However, for H3 Brisbane, the Western blot clearly indicated that the co-expression of cytosolic Hsp70 and Hsp40 resulted in a significant increase in hemagglutinin accumulation level.

### Example 23. Expression of H1 A/California 04/09 under control of 2X35S/CPMV-HT expression cassette.

A CPMV-HT expression cassette was also used for expression of H1 A/California 04/09 (construct #560, Figures 90, 98) in *N. benthamiana* plants, agroinfiltrated as described. After 2 days of incubation, leaves were harvested, extracted and HA contents in protein extracts were compared by Western blot. As shown in Figure 91, the CPMV-HT expression cassette led to significant expression of HA at 2 days post infiltration. VLPs produced form expression of HA in plants demonstrate agglutination of red blood cells.

**Table 20: samples for eachlane of the Western blot illustrated in Figure 91.**

| Lane # | Description | |
|---|---|---|
| 1 | 10 ng H1 (ABri/59/07) | Positive control (ITC, IT-003-0052p) |
| 2 | 40 ng H1N1 (A/NC/20/99) | Positive control (NISBC, 06/170) |
| 3 | 40 ng H1 (A/Bri/59/07) | Positive control (NISBC, 08/100) |
| 4 | Mock infiltrated plant | Negative control |
| 5 | BW09-I001-560-1 | 2X35S-CPMV HT H1 A/California/4/09 |
| 6 | BW09-I001-560-2 | 2X35S-CPMV HT H1 A/California/4/09 |
| 7 | BW09-I001-560-3 | 2X35S-CPMV HT H1 A/California/4/09 |
| 8 | BW09-I001-560-6 | 2X35S-CPMV HT H1 A/California/4/09 |
| | | |

All citations are hereby incorporated by reference.

The present invention has been described with regard to one or more embodiments.

### References:

Aymard, H. M., M. T. Coleman, W. R. Dowdle, W. G. Laver, G. C. Schild, and R. G. Webster. 1973. Influenza virus neuraminidase-inhibition test procedures. Bull. W.H.O. 48: 199-202
Bollag, D.M., Rozycki, M.D., and Edelstein, S.J. (1996) Protein methods (2nd edition). Wiley-Liss, New York, USA.
Bligh, E.G., & Dyer, W.J. Can. J. Med. Sci. 37, 911-917 (1959).
Chen, B.J., Leser, G.P., Morita, E., and Lamb R.A. (2007) Influenza virus hemagglutinin and neuraminidase, but not the matrix protein, are required for assembly and budding of plasmid-derived virus-like particles. J. Virol. 81, 7111-7123.
Chen Z, Aspelund A, Jin H. 2008 Stabilizing the glycosylation pattern of influenza B hemagglutinin following adaptation to growth in eggs.Vaccine vol 26 p 361-371
Crawford, J. , Wilkinson, B. , Vosnesensky, A. , Smith, G. , Garcia, M. , Stone, H. , and Perdue, M. L. (1999). Baculovirus-derived hemagglutinin vaccines protect against lethal influenza infections by avian H5 and H7 subtypes. Vaccine 17,2265-2274.
Darveau, A., Pelletier, A. & Perreault, J. PCR-mediated synthesis of chimeric molecules. Methods Neurosc. 26, 77-85 (1995).
Grgacic EVL, Anderson DA. Virus-like particles: passport to immune recognition. Methods 2006; 40: 60-65.
Gillim-Ross, L., and Subbarao, K. (2006) Emerging respiratory viruses: chanllenges and vaccine strategies. Clin. Microbiol. Rev. 19, 614-636.
Gomez-Puertas, P., Mena, I., Castillo, M., Vivo, A., Perez-Pastrana, E. and Portela, A. (1999) Efficient formation of influenza virus-like particles: dependence on the expression level of viral proteins. J. Gen. Virol. 80, 1635-1645.
Gomez-Puertas, P., Albo, C., Perez-Pastrana, E., Vivo, A., and Portela, A. (2000) Influenza Virus protein is the major driving force in virus budding. J Virol. 74, 11538-11547.
Hamilton, A., Voinnet, O., Chappell, L. & Baulcombe, D. Two classes of short interfering RNA in RNA silencing. EMBO J. 21, 4671-4679 (2002).
Höfgen, R. & Willmitzer, L. Storage of competent cells for Agrobacterium transformation. Nucleic Acid Res. 16, 9877 (1988).
Harbury PB, Zhang T, Kim PS, Alber T. (1993) A switch between two-, three-, and four-stranded coiled coils in GCN4 leucine zipper mutants. Science; 262: 1401-1407)
Horimoto T., Kawaoka Y. Strategies for developing vaccines against h5N1 influenza a viruses. Trends in Mol. Med. 2006; 12(11):506-514.
Huang Z, Elkin G, Maloney BJ, Beuhner N, Arntzen CJ, Thanavala Y, Mason HS. Virus-like particle expression and assembly in plants: hepatitis B and Norwalk viruses. Vaccine. 2005 Mar 7;23(15):1851-8.
Johansson, B. E. (1999). Immunization with influenza A virus hemagglutinin and neuraminidase produced in recombinant baculovirus results in a balanced and broadened immune response superior to conventional vaccine. Vaccine 17, 2073-2080.
Latham, T. , and Galarza, J. M. (2001). Formation of wild-type and chimeric influenza virus-like particles following simultaneous expression of only four structural proteins. J. Virol. 75,6154-6165.
Lefebvre, B. et al. Plant Physiol. 144, 402-418 (2007).
Leutwiler LS et al 1986. Nucleic Acid Sresearch 14910):4051-64
Liu, L & Lomonossoff, G.P. Agroinfection as a rapid method for propagating Cowpea mosaic virus-based constructs. J. Virol. Methods 105, 343-348 (2002).
Macala, L.J., Yo, R.K. & Ando, S. J Lipid Res. 24, 1243-1250 (1983)
Mattanovich, D., Rüker, F., da Câmara Machado, A., Laimer, M., Regner, F., Steinkellner, H., Himmler, G., and Katinger, H. (1989) Efficient transformation of Agrobacterium spp. By electroporation. Nucl. Ac. Res. 17, 6747.
Mena, I., Vivo, A., Perez, E., and Portela, A. (1996) Rescue of synthetic chloramphenicol acetyltransferase RNA into influenza virus-like particles obtained from recombinant plasmids. J. Virol. 70, 5016-5024.
Mongrand S, Morel J, Laroche J, Claverol S, Carde JP, Hartmann MA et al.. Lipid rafts in higher plant cells. The Journal of Biological Chemistry 2004; 279(35): 36277-36286.
Neumann, G., Watanabe, T., and Kawaoka, Y. (2000) Plasmid-driven formation of virus-like particles. J. Virol. 74, 547-551.
Nayak DP, Reichl U. (2004) Neuraminidase activity assays for monitoring MDCK cell culture derived influenza virus. J Virol Methods 122(1):9-15.
Olsen, C. W. , McGregor, M. W. , Dybdahl-Sissoko, N. , Schram, B. R. , Nelson, K. M. , Lunn, D., Macklin, M. D. , and Swain, W. F. (1997). Immunogenicity and efficacy of baculovirus-expressed and DNA-based equine influenza virus hemagglutinin vaccines in mice. Vaccine 15, 1149-1156.
Quan FS, Huang C, Compans RW, Kang SM. Virus-like particle vaccine induces protective immunity against homologous and heterologous strains of influenza virus. Journal of Virology 2007; 81(7): 3514-3524.
Rowe, T. et al. 1999. Detection of antibody to avian influenza a (h5N1) virus in human serum by using a cmbiation of serologic assays. J. Clin Microbiol 37(4):937-43
Saint-Jore-Dupas C et al. 2007. From planta to pharma with glycosylation in the toolbox. Trends in Biotechnology 25(7) :317-23
Sambrook J, and Russell DW. Molecular cloning: a laboratory manual. Cold Spring Harbor, N.Y. Cold Spring Harbor Laboratory Press, 2001.
Stockhaus J et al 1987. Analysis of cis-active sequences involved in the leaf-specific expression of a potato gene in transgenic plants. Proceedings of the National Academy of Sciences U.S.S. 84(22):7943-7947.
Stockhaus J et al 1989. Identification of enhancer elements in the upstream region of the nuclear photosynthetic gene ST-LS1. Plant Cell. 1(8):805-13.
Suzuki, Y. (2005) Sialobiology of influenza. Molecular mechanism of host range variation of influenza viruses. Biol. Pharm. Bull 28, 399-408.
Tsuji M., Cell. Mol. Life Sci., 63 (2006); 1889-1898
Wakefield L., G.G. Brownlee Nuc Acid Res. 17 (1989); 8569-8580.
Kendal, AP, Pereira MS, Skehel J. Concepts and procedures for laboratory-based influenza surveillance. Atlanta:CDC; 1982. p.B17-B35
WHO. Manual on animal influenza diagnosis and surveillance. Department of communicable disease surveillance and response. World Health Organisation Global Influenza Program. 2002.
Skehel JJ and Wildy DC Ann Rev Biochem 2000 69:531-69
Vaccaro L et al 2005. Biophysical J. 88:25-36.
Gamblin, S.J., Haire, L.F., Russell, R.J., Stevens, D.J., Xiao, B., Ha, Y., Vasisht, N., Steinhauer, D.A., Daniels, R.S., Elliot, A., Wiley, D.C., Skehel, J.J. (2004) The structure and receptor binding properties of the 1918 influenza hemagglutinin. Science 303: 1838-1842

## Claims

1. A nucleic acid comprising a nucleotide sequence encoding an influenza hemagglutinin (HA) having at least 80% sequence identity to (HA) of type A/California/04/09, wherein the nucleotide sequence comprises a regulatory element that is operative in a plant, the regulatory element comprising a Cowpea Mosaic Virus (CPMV)-HT.

2. The nucleic acid of claim 1, wherein the HA comprises a native or a non-native signal peptide and/or optionally
wherein the non-native signal peptide is a protein disulfide isomerase signal peptide.

3. A nucleic acid comprising a nucleotide sequence encoding an influenza hemagglutinin (HA) having at least 80% sequence identity to (HA) of type A/California/04/09, wherein the HA comprises a protein disulfide isomerase signal peptide, operatively linked to a regulatory region active in a plant.

4. The nucleic acid of any one of claims 1-3, wherein the influenza hemagglutinin sequence is selected from SEQ ID NO: 127 and 135.

5. A method of producing influenza virus like particles (VLPs) in a plant comprising:
a) introducing the nucleic acid of any one of claims 1-5 into the plant, or portion of the plant, and
b) incubating the plant or portion of the plant under conditions that permit the expression of the nucleic acid, thereby producing the VLPs.

6. The method of claim 5, further comprising the steps of
c) harvesting the plant, and
d) purifying the VLPs, wherein the VLPs range in size from 80-300 nm.

7. The method of any one of claims 5-6, wherein in the step of introducing (step a), the nucleic acid is introduced in the plant in a transient manner or the nucleic acid is introduced in the plant so that it is stable.

8. The method of any one of claims 5-7 wherein, in the step of introducing (step a), a second nucleic acid comprising a nucleotide sequence encoding one or more than one chaperone proteins is introduced to the plant, wherein the one or more than one chaperon proteins is preferably selected from the group consisting of Hsp40 and Hsp70.

9. A method of producing influenza virus like particles (VLPs) in a plant comprising:
a) providing a plant, or a portion of a plant, comprising the nucleic acid of any one of claims 1-4, and
b) incubating the plant or portion of the plant under conditions that permit the expression of the nucleic acid, thereby producing the VLPs

10. A plant comprising the nucleic acid of any one of claims 1-4.

11. The plant of claim 10, further comprising a second nucleic acid comprising a nucleotide sequence encoding one or more than one chaperone proteins operatively linked to a regulatory region active in the plant, wherein the one or more than one chaperon proteins is preferably selected from the group consisting of Hsp40 and Hsp70.

12. A virus like particle (VLP) produced in a plant, the VLP comprising an influenza virus hemagglutinin (HA) encoded by the nucleic acid of any one of claims 1-4, and one or more than one lipid derived from a plant and/or optionally bearing plant-specific N-glycans or modified N-glycans.

13. A virus like particle (VLP) produced by the method of any one of claims 5-9 and/or optionally bearing plant-specific N-glycans, or modified N-glycans.

14. A composition comprising an effective dose of the VLP of any one of claims 12-13 for inducing an immune response, and a pharmaceutically acceptable carrier.

15. A vaccine comprising an effective dose of the VLP of any one of claims 12-13 for inducing an immune response.

16. The virus like particle of any one of claims 12-13, the composition of claim 14 or the vaccine of claim 15 for use in inducing immunity to an influenza virus infection in a subject.

## Patentansprüche

1. Nukleinsäure, umfassend eine Nukleotidsequenz, die für ein Influenza-Hämagglutinin (HA) mit einer mindestens 80%igen Sequenzidentität zu (HA) vom Typ A/California/04/09 kodiert, wobei die Nukleotidsequenz ein regulatorisches Element umfasst, das in einer Pflanze funktionsfähig ist, wobei das regulatorische Element ein Cowpea Mosaic-Virus (CPMV)-HT umfasst.

2. Nukleinsäure nach Anspruch 1, wobei das HA ein natives oder nicht-natives Signalpeptid umfasst und/oder gegebenenfalls
wobei das nicht-native Signalpeptid ein Proteindisulfidisomerase-Signalpeptid ist.

3. Nukleinsäure, umfassend eine Nukleotidsequenz, die für ein Influenza-Hämagglutinin (HA) mit einer mindestens 80% Sequenzidentität zu (HA) vom Typ A/California/04/09 kodiert, wobei das HA ein Proteindisulfidisomerase-Signalpeptid umfasst, das funktionell mit einer in einer Pflanze aktiven, regulatorischen Region verknüpft ist.

4. Nukleinsäure nach einem der Ansprüche 1-3, wobei die Influenza-Hämagglutinin-Sequenz ausgewählt ist aus den SEQ ID NO: 127 und 135.

5. Verfahren zur Herstellung von Influenzavirus-ähnlichen Partikeln (VLP) in einer Pflanze, umfassend:
a) Einführen der Nukleinsäure nach einem der Ansprüche 1-4 in die Pflanze oder einen Teil der Pflanze; und
b) Inkubieren der Pflanze oder des Teils der Pflanze unter Bedingungen, die die Expression der Nukleinsäure erlauben, wodurch die VLP produziert werden.

6. Verfahren nach Anspruch 5, zusätzlich umfassend die Schritte:
c) Ernten der Pflanze, und
d) Reinigen der VLP, wobei die VLP in einem Größenbereich von 80-300 nm liegen.

7. Verfahren nach einem der Ansprüche 5-6, wobei die Nukleinsäure in dem Schritt des Einführens (Schritt a) auf eine transiente Weise in die Pflanze eingeführt wird oder die Nukleinsäure so in die Pflanze eingeführt wird, dass sie stabil ist.

8. Verfahren nach einem der Ansprüche 5-7, wobei im Schritt des Einführens (Schritt a) eine zweite Nukleinsäure, die eine für ein oder mehrere Chaperonproteine kodierende Nukleotidsequenz umfasst, in die Pflanze eingeführt wird, wobei das eine oder die mehreren Chaperonproteine vorzugsweise aus der Gruppe bestehend aus Hsp40 und Hsp70 ausgewählt sind.

9. Verfahren zur Herstellung von Influenzavirus-ähnlichen Partikeln (VLP) in einer Pflanze, umfassend:
a) Bereitstellen einer Pflanze oder eines Teils einer Pflanze, umfassend die Nukleinsäure nach einem der Ansprüche 1-4, und
b) Inkubieren der Pflanze oder des Teils der Pflanze unter Bedingungen, die die Expression der Nukleinsäure erlauben, wodurch die VLP produziert werden.

10. Pflanze, umfassend die Nukleinsäure nach einem der Ansprüche 1-4.

11. Pflanze nach Anspruch 10, zusätzlich umfassend eine zweite Nukleinsäure, die eine Nukleotidsequenz umfasst, die für ein oder mehrere, funktionell mit einer in der Pflanze aktiven regulatorischen Region verknüpfte Chaperonproteine kodiert, wobei das eine oder die mehreren Chaperonproteine vorzugsweise aus der Gruppe bestehend aus Hsp40 und Hsp70 ausgewählt sind.

12. Virusähnliches Partikel (VLP), produziert in einer Pflanze, wobei das VLP ein Influenzavirus-Hämagglutinin (HA), das durch die Nukleinsäure nach einem der Ansprüche 1-4 kodiert wird, und ein oder mehr als ein von einer Pflanze abstammendes Lipid umfasst und/oder gegebenenfalls pflanzenspezifische N-Glykane oder modifizierte N-Glykane trägt.

13. Virusähnliches Partikel (VLP), produziert mit dem Verfahren nach einem der Ansprüche 5-9 und/oder gegebenenfalls pflanzenspezifische N-Glykane oder modifizierte N-Glykane tragend.

14. Zusammensetzung, umfassend eine wirksame Dosis der VLP nach einem der Ansprüche 12-13 zur Induzierung einer Immunantwort sowie einen pharmazeutisch zulässigen Träger.

15. Impfstoff, umfassend eine wirksame Dosis der VLP nach einem der Ansprüche 12-13 zur Induzierung einer Immunantwort.

16. Virusähnliches Partikel nach einem der Ansprüche 12-13, Zusammensetzung nach Anspruch 14 oder Impfstoff nach Anspruch 15 zur Verwendung bei der Induzierung der Immunität gegenüber einer Influenzavirusinfektion in einem Subjekt.

## Revendications

1. Acide nucléique comprenant une séquence de nucléotides codant pour une hémagglutinine (HA) de la grippe ayant une identité de séquence d'au moins 80 % avec la (HA) de type A/California/04/09, dans lequel la séquence de nucléotides comprend un élément de régulation qui est opérationnel dans une plante, l'élément de régulation comprenant un virus de la mosaïque du niébé (CPMV)-HT.

2. Acide nucléique selon la revendication 1, dans lequel la HA comprend un peptide signal natif ou non natif et/ou facultativement
dans lequel le peptide signal non natif est un peptide signal de la protéine disulfure isomérase.

3. Acide nucléique comprenant une séquence de nucléotides codant pour une hémagglutinine (HA) de la grippe ayant une identité de séquence d'au moins 80 % avec la (HA) de type A/California/04/09, dans lequel la HA comprend un peptide signal de la protéine disulfure isomérase, lié de façon opérationnelle à une région de régulation active dans une plante.

4. Acide nucléique selon l'une quelconque des revendications 1 à 3, dans lequel la séquence de l'hémagglutinine de la grippe est sélectionnée parmi SEQ ID NO : 127 et 135.

5. Procédé de production de pseudo-particules du virus de la grippe (VLP) dans une plante, comprenant :
a) l'introduction de l'acide nucléique selon l'une quelconque des revendications 1 à 5 dans la plante, ou dans une partie de la plante, et
b) l'incubation de la plante ou de la partie de la plante dans des conditions qui permettent l'expression de l'acide nucléique, permettant ainsi la production des VLP.

6. Procédé selon la revendication 5, comprenant en outre les étapes de
c) récolte de la plante, et
d) purification des VLP, dans lequel la taille des VLP se situe dans la plage de 80 à 300 nm.

7. Procédé selon l'une quelconque des revendications 5 et 6, dans lequel dans l'étape d'introduction (étape a), l'acide nucléique est introduit dans la plante de manière transitoire ou l'acide nucléique est introduit dans la plante de manière à ce qu'il soit stable.

8. Procédé selon l'une quelconque des revendications 5 à 7, dans lequel dans l'étape d'introduction (étape a), un second acide nucléique comprenant une séquence de nucléotides codant pour une ou plusieurs protéines chaperons est introduit dans la plante, dans lequel les une ou plusieurs protéines chaperons sont de préférence sélectionnées dans le groupe constitué de Hsp40 et Hsp70.

9. Procédé de production de pseudo-particules du virus de la grippe (VLP) dans une plante, comprenant :
a) la fourniture d'une plante, ou d'une partie d'une plante, comprenant l'acide nucléique selon l'une quelconque des revendications 1 à 4, et
b) l'incubation de la plante ou de la partie de la plante dans des conditions qui permettent l'expression de l'acide nucléique, permettant ainsi la production des VLP.

10. Plante comprenant l'acide nucléique selon l'une quelconque des revendications 1 à 4.

11. Plante selon la revendication 10, comprenant en outre un second acide nucléique comprenant une séquence de nucléotides codant pour au moins une protéine chaperon liée de manière opérationnelle à une région de régulation active dans la plante, dans laquelle la ou les protéines chaperons sont de préférence sélectionnées dans le groupe constitué de Hsp40 et Hsp70.

12. Pseudo-particule virale (VLP) produite dans une plante, la VLP comprenant une hémagglutinine (HA) du virus influenza codée par l'acide nucléique selon l'une quelconque des revendications 1 à 4, et au moins un lipide dérivé d'une plante et/ou portant facultativement des N-glycanes spécifiques de la plante ou des N-glycanes modifiés.

13. Pseudo-particule virale (VLP) produite par le procédé selon l'une quelconque des revendications 5 à 9 et/ou portant facultativement des N-glycanes spécifiques de la plante ou des N-glycanes modifiés.

14. Composition comprenant une dose efficace de la VLP selon l'une quelconque des revendications 12 et 13 pour l'induction d'une réponse immunitaire, et un vecteur pharmaceutiquement acceptable.

15. Vaccin comprenant une dose efficace de la VLP selon l'une quelconque des revendications 12 et 13 pour l'induction d'une réponse immunitaire.

16. Pseudo-particule virale selon l'une quelconque des revendications 12 et 13, composition selon la revendication 14 ou vaccin selon la revendication 15 pour leur utilisation dans l'induction d'une réponse immunitaire à une infection par un virus de la grippe chez un sujet.


### SEQUENCE LISTING

<110> Medicago Inc.
<120> Influenza Virus-Like Particles (VLPS) Comprising Hemagglutinin
<130> 1088-0013
<140> EP
   <141> 2009-07-02
<150> PCT/CA2008/001281
   <151> 2008-07-11
<150> PCT/CA2008/000032
   <151> 2009-01-12
<160> 146
<170> PatentIn version 3.4
<210> 1
   <211> 1556
   <212> DNA
   <213> Influenza virus
<400> 1
   agatcttcgc tgacacaata tgtataggct accatgccaa caactcaacc gacactgttg      60
   acacagtact tgagaagaat gtgacagtga cacactctgt caacctactt gaggacagtc     120
   acaatggaaa actatgtcta ctaaaaggaa tagccccact acaattgggt aattgcagcg     180
   ttgccggatg gatcttagga aacccagaat gcgaattact gatttccaag gaatcatggt     240
   cctacattgt agaaacacca aatcctgaga atggaacatg ttacccaggg tatttcgccg     300
   actatgagga actgagggag caattgagtt cagtatcttc atttgagaga ttcgaaatat     360
   tccccaaaga aagctcatgg cccaaccaca ccgtaaccgg agtatcagca tcatgctccc     420
   ataatgggaa aagcagtttt tacagaaatt tgctatggct gacggggaag aatggtttgt     480
   acccaaacct gagcaagtcc tatgtaaaca acaaagagaa agaagtcctt gtactatggg     540
   gtgttcatca cccgcctaac atagggaacc aaagggcact ctatcataca gaaaatgctt     600
   atgtctctgt agtgtcttca cattatagca gaagattcac cccagaaata gccaaaagac     660
   ccaaagtaag agatcaggaa ggaagaatca actactactg gactctgctg gaacctgggg     720
   atacaataat atttgaggca aatggaaatc taatagcgcc atggtatgct tttgcactga     780
   gtagaggctt tggatcagga atcatcacct caaatgcacc aatggatgaa tgtgatgcga     840
   agtgtcaaac acctcaggga gctataaaca gcagtcttcc tttccagaat gtacacccag     900
   tcacaatagg agagtgtcca aagtatgtca ggagtgcaaa attaaggatg gttacaggac     960
   taaggaacat cccatccatt caatccagag gtttgtttgg agccattgcc ggtttcattg    1020
   aaggggggtg gactggaatg gtagatgggt ggtatggtta tcatcatcag aatgagcaag    1080
   gatctggcta tgctgcagat caaaaaagta cacaaaatgc cattaacggg attacaaaca    1140
   aggtcaattc tgtaattgag aaaatgaaca ctcaattcac agctgtgggc aaagagttca    1200
   acaaattgga aagaaggatg gaaaacttaa ataaaaaagt tgatgatggg tttctagaca    1260
   tttggacata taatgcagaa ttgttggttc tactggaaaa tgaaaggact ttggatttcc    1320
   atgactccaa tgtgaagaat ctgtatgaga aagtaaaaag ccaattaaag aataatgcca    1380
   aagaaatagg aaacgggtgt tttgagttct atcacaagtg taacaatgaa tgcatggaga    1440
   gtgtgaaaaa tggtacctat gactatccaa aatattccga agaatcaaag ttaaacaggg    1500
   agaaaattga tggagtgaaa ttggaatcaa tgggagtata ctaagagctc aggcct         1556
<210> 2
   <211> 219
   <212> DNA
   <213> Influenza virus
<400> 2
   ggtacctatg actatccaaa atattccgaa gaatcaaagt taaacaggga gaaaattgat 60
   ggagtgaaat tggaatcaat gggagtatac cagattctgg cgatctactc aactgtcgcc 120
   agttccctgg ttcttttggt ctccctgggg gcaatcagct tctggatgtg ttccaatggg 180
   tctttgcagt gtagaatatg catctaagag ctcaggcct 219
<210> 3
   <211> 1719
   <212> DNA
   <213> Influenza virus
<400> 3
   aagcttatgg agaaaatagt gcttcttctt gcaatagtca gtcttgttaa aagtgatcag 60
   atttgcattg gttaccatgc aaacaattca acagagcagg ttgacacaat catggaaaag 120
   aacgttactg ttacacatgc ccaagacata ctggaaaaga cacacaacgg gaagctctgc 180
   gatctagatg gagtgaagcc tctaatttta agagattgta gtgtagctgg atggctcctc 240
   gggaacccaa tgtgtgacga attcatcaat gtaccggaat ggtcttacat agtggagaag 300
   gccaatccaa ccaatgacct ctgttaccca gggagtttca acgactatga agaactgaaa 360
   cacctattga gcagaataaa ccattttgag aaaattcaaa tcatccccaa aagttcttgg 420
   tccgatcatg aagcctcatc aggagttagc tcagcatgtc catacctggg aagtccctcc 480
   ttttttagaa atgtggtatg gcttatcaaa aagaacagta catacccaac aataaagaaa 540
   agctacaata ataccaacca agaggatctt ttggtactgt ggggaattca ccatcctaat 600
   gatgcggcag agcagacaag gctatatcaa aacccaacca cctatatttc cattgggaca 660
   tcaacactaa accagagatt ggtaccaaaa atagctacta gatccaaagt aaacgggcaa 720
   agtggaagga tggagttctt ctggacaatt ttaaaaccta atgatgcaat caacttcgag 780
   agtaatggaa atttcattgc tccagaatat gcatacaaaa ttgtcaagaa aggggactca 840
   gcaattatga aaagtgaatt ggaatatggt aactgcaaca ccaagtgtca aactccaatg 900
   ggggcgataa actctagtat gccattccac aacatacacc ctctcaccat cggggaatgc     960
   cccaaatatg tgaaatcaaa cagattagtc cttgcaacag ggctcagaaa tagccctcaa    1020
   agagagagca gaagaaaaaa gagaggacta tttggagcta tagcaggttt tatagaggga    1080
   ggatggcagg gaatggtaga tggttggtat gggtaccacc atagcaatga gcaggggagt    1140
   gggtacgctg cagacaaaga atccactcaa aaggcaatag atggagtcac caataaggtc    1200
   aactcaatca ttgacaaaat gaacactcag tttgaggccg ttggaaggga atttaataac    1260
   ttagaaagga gaatagagaa tttaaacaag aagatggaag acgggtttct agatgtctgg    1320
   acttataatg ccgaacttct ggttctcatg gaaaatgaga gaactctaga ctttcatgac    1380
   tcaaatgtta agaacctcta cgacaaggtc cgactacagc ttagggataa tgcaaaggag    1440
   ctgggtaacg gttgtttcga gttctatcac aaatgtgata atgaatgtat ggaaagtata    1500
   agaaacggaa cgtacaacta tccgcagtat tcagaagaag caagattaaa aagagaggaa    1560
   ataagtgggg taaaattgga atcaatagga acttaccaaa tactgtcaat ttattcaaca    1620
   gtggcgagtt ccctagcact ggcaatcatg atggctggtc tatctttatg gatgtgctcc    1680
   aatggatcgt tacaatgcag aatttgcatt taagagctc                           1719
<210> 4
   <211> 25
   <212> DNA
   <213> Artificial sequence
<220>
   <223> primer Plasto-443c
<400> 4
   gtattagtaa ttagaatttg gtgtc 25
<210> 5
   <211> 44
   <212> DNA
   <213> Artificial sequence
<220>
   <223> primer SpHA(Ind)-Plasto.r
<400> 5
   gcaagaagaa gcactatttt ctccattttc tctcaagatg atta 44
<210> 6
   <211> 45
   <212> DNA
   <213> Artificial sequence
<220>
   <223> primer SpHA(Ind)-Plasto.r
<400> 6
   ttaatcatct tgagagaaaa tggagaaaat agtgcttctt cttgc 45
<210> 7
   <211> 38
   <212> DNA
   <213> Artificial sequence
<220>
   <223> primer HA(Ind)-Sac.r
<400> 7
   actttgagct cttaaatgca aattctgcat tgtaacga 38
<210> 8
   <211> 1471
   <212> DNA
   <213> Artificial sequence
<220>
   <223> alfalfa plastocyanin-based expression cassette
<400> 8
   agaggtaccc cgggctggta tatttatatg ttgtcaaata actcaaaaac cataaaagtt      60
   taagttagca agtgtgtaca tttttacttg aacaaaaata ttcacctact actgttataa     120
   atcattatta aacattagag taaagaaata tggatgataa gaacaagagt agtgatattt     180
   tgacaacaat tttgttgcaa catttgagaa aattttgttg ttctctcttt tcattggtca     240
   aaaacaatag agagagaaaa aggaagaggg agaataaaaa cataatgtga gtatgagaga     300
   gaaagttgta caaaagttgt accaaaatag ttgtacaaat atcattgagg aatttgacaa     360
   aagctacaca aataagggtt aattgctgta aataaataag gatgacgcat tagagagatg     420
   taccattaga gaatttttgg caagtcatta aaaagaaaga ataaattatt tttaaaatta     480
   aaagttgagt catttgatta aacatgtgat tatttaatga attgatgaaa gagttggatt     540
   aaagttgtat tagtaattag aatttggtgt caaatttaat ttgacatttg atcttttcct     600
   atatattgcc ccatagagtc agttaactca tttttatatt tcatagatca aataagagaa     660
   ataacggtat attaatccct ccaaaaaaaa aaaacggtat atttactaaa aaatctaagc     720
   cacgtaggag gataacagga tccccgtagg aggataacat ccaatccaac caatcacaac     780
   aatcctgatg agataaccca ctttaagccc acgcatctgt ggcacatcta cattatctaa     840
   atcacacatt cttccacaca tctgagccac acaaaaacca atccacatct ttatcaccca     900
   ttctataaaa aatcacactt tgtgagtcta cactttgatt cccttcaaac acatacaaag     960
   agaagagact aattaattaa ttaatcatct tgagagaaaa tggcgaaaaa cgttgcgatt    1020
   ttcggcttat tgttttctct tcttgtgttg gttccttctc agatctgagc tctaagttaa    1080
   aatgcttctt cgtctcctat ttataatatg gtttgttatt gttaattttg ttcttgtaga    1140
   agagcttaat taatcgttgt tgttatgaaa tactatttgt atgagatgaa ctggtgtaat    1200
   gtaattcatt tacataagtg gagtcagaat cagaatgttt cctccataac taactagaca    1260
   tgaagacctg ccgcgtacaa ttgtcttata tttgaacaac taaaattgaa catcttttgc    1320
   cacaacttta taagtggtta atatagctca aatatatggt caagttcaat agattaataa    1380
   tggaaatatc agttatcgaa attcattaac aatcaactta acgttattaa ctactaattt    1440
   tatatcatcc cctttgataa atgatagtac a                                   1471
<210> 9
   <211> 565
   <212> PRT
   <213> Influenza virus
<400> 9
   Met Lys Ala Lys Leu Leu Val Leu Leu Cys Thr Phe Thr Ala Thr Tyr
   1               5                   10                  15
   Ala Asp Thr Ile Cys Ile Gly Tyr His Ala Asn Asn Ser Thr Asp Thr
               20                  25                  30
   Val Asp Thr Val Leu Glu Lys Asn Val Thr Val Thr His Ser Val Asn
           35                  40                  45
   Leu Leu Glu Asp Ser His Asn Gly Lys Leu Cys Leu Leu Lys Gly Ile
       50                  55                  60
   Ala Pro Leu Gln Leu Gly Asn Cys Ser Val Ala Gly Trp Ile Leu Gly
   65                  70                  75                  80
   Asn Pro Glu Cys Glu Leu Leu Ile Ser Lys Glu Ser Trp Ser Tyr Ile
                   85                  90                  95
   Val Glu Thr Pro Asn Pro Glu Asn Gly Thr Cys Tyr Pro Gly Tyr Phe
               100                 105                 110
   Ala Asp Tyr Glu Glu Leu Arg Glu Gln Leu Ser Ser Val Ser Ser Phe
           115                 120                 125
   Glu Arg Phe Glu Ile Phe Pro Lys Glu Ser Ser Trp Pro Asn His Thr
       130                 135                 140
   Val Thr Gly Val Ser Ala Ser Cys Ser His Asn Gly Lys Ser Ser Phe
   145                 150                 155                 160
   Tyr Arg Asn Leu Leu Trp Leu Thr Gly Lys Asn Gly Leu Tyr Pro Asn
                   165                 170                 175
   Leu Ser Lys Ser Tyr Val Asn Asn Lys Glu Lys Glu Val Leu Val Leu
               180                 185                 190
   Trp Gly Val His His Pro Pro Asn Ile Gly Asn Gln Arg Ala Leu Tyr
           195                 200                 205
   His Thr Glu Asn Ala Tyr Val Ser Val Val Ser Ser His Tyr Ser Arg
   210 215 220
   Arg Phe Thr Pro Glu Ile Ala Lys Arg Pro Lys Val Arg Asp Gln Glu
   225 230 235 240
   Gly Arg Ile Asn Tyr Tyr Trp Thr Leu Leu Glu Pro Gly Asp Thr Ile
   245 250 255
   Ile Phe Glu Ala Asn Gly Asn Leu Ile Ala Pro Trp Tyr Ala Phe Ala
   260 265 270
   Leu Ser Arg Gly Phe Gly Ser Gly Ile Ile Thr Ser Asn Ala Pro Met
   275 280 285
   Asp Glu Cys Asp Ala Lys Cys Gln Thr Pro Gln Gly Ala Ile Asn Ser
   290 295 300
   Ser Leu Pro Phe Gln Asn Val His Pro Val Thr Ile Gly Glu Cys Pro
   305 310 315 320
   Lys Tyr Val Arg Ser Ala Lys Leu Arg Met Val Thr Gly Leu Arg Asn
   325 330 335
   Ile Pro Ser Ile Gln Ser Arg Gly Leu Phe Gly Ala Ile Ala Gly Phe
   340 345 350
   Ile Glu Gly Gly Trp Thr Gly Met Val Asp Gly Trp Tyr Gly Tyr His
   355 360 365
   His Gln Asn Glu Gln Gly Ser Gly Tyr Ala Ala Asp Gln Lys Ser Thr
   370 375 380
   Gln Asn Ala Ile Asn Gly Ile Thr Asn Lys Val Asn Ser Val Ile Glu
   385 390 395 400
   Lys Met Asn Thr Gln Phe Thr Ala Val Gly Lys Glu Phe Asn Lys Leu
   405 410 415
   Glu Arg Arg Met Glu Asn Leu Asn Lys Lys Val Asp Asp Gly Phe Leu
   420 425 430
   Asp Ile Trp Thr Tyr Asn Ala Glu Leu Leu Val Leu Leu Glu Asn Glu
   435 440 445
   Arg Thr Leu Asp Phe His Asp Ser Asn Val Lys Asn Leu Tyr Glu Lys
   450 455 460
   Val Lys Ser Gln Leu Lys Asn Asn Ala Lys Glu Ile Gly Asn Gly Cys
   465 470 475 480
   Phe Glu Phe Tyr His Lys Cys Asn Asn Glu Cys Met Glu Ser Val Lys
   485 490 495
   Asn Gly Thr Tyr Asp Tyr Pro Lys Tyr Ser Glu Glu Ser Lys Leu Asn
   500 505 510
   Arg Glu Lys Ile Asp Gly Val Lys Leu Glu Ser Met Gly Val Tyr Gln
   515 520 525
   Ile Leu Ala Ile Tyr Ser Thr Val Ala Ser Ser Leu Val Leu Leu Val
   530 535 540
   Ser Leu Gly Ala Ile Ser Phe Trp Met Cys Ser Asn Gly Ser Leu Gln
   545 550 555 560
   Cys Arg Ile Cys Ile
   565
<210> 10
   <211> 568
   <212> PRT
   <213> Influenza virus
<400> 10
   Met Glu Lys Ile Val Leu Leu Leu Ala Ile Val Ser Leu Val Lys Ser
   1               5                   10                  15
   Asp Gln Ile Cys Ile Gly Tyr His Ala Asn Asn Ser Thr Glu Gln Val
               20                  25                  30
   Asp Thr Ile Met Glu Lys Asn Val Thr Val Thr His Ala Gln Asp Ile
           35                  40                  45
   Leu Glu Lys Thr His Asn Gly Lys Leu Cys Asp Leu Asp Gly Val Lys
       50                  55                  60
   Pro Leu Ile Leu Arg Asp Cys Ser Val Ala Gly Trp Leu Leu Gly Asn
   65                  70                  75                  80
   Pro Met Cys Asp Glu Phe Ile Asn Val Pro Glu Trp Ser Tyr Ile Val
                   85                  90                  95
   Glu Lys Ala Asn Pro Thr Asn Asp Leu Cys Tyr Pro Gly Ser Phe Asn
               100                 105                 110
   Asp Tyr Glu Glu Leu Lys His Leu Leu Ser Arg Ile Asn His Phe Glu
   115 120 125
   Lys Ile Gln Ile Ile Pro Lys Ser Ser Trp Ser Asp His Glu Ala Ser
   130 135 140
   Ser Gly Val Ser Ser Ala Cys Pro Tyr Leu Gly Ser Pro Ser Phe Phe
   145 150 155 160
   Arg Asn Val Val Trp Leu Ile Lys Lys Asn Ser Thr Tyr Pro Thr Ile
   165 170 175
   Lys Lys Ser Tyr Asn Asn Thr Asn Gln Glu Asp Leu Leu Val Leu Trp
   180 185 190
   Gly Ile His His Pro Asn Asp Ala Ala Glu Gln Thr Arg Leu Tyr Gln
   195 200 205
   Asn Pro Thr Thr Tyr Ile Ser Ile Gly Thr Ser Thr Leu Asn Gln Arg
   210 215 220
   Leu Val Pro Lys Ile Ala Thr Arg Ser Lys Val Asn Gly Gln Ser Gly
   225 230 235 240
   Arg Met Glu Phe Phe Trp Thr Ile Leu Lys Pro Asn Asp Ala Ile Asn
   245 250 255
   Phe Glu Ser Asn Gly Asn Phe Ile Ala Pro Glu Tyr Ala Tyr Lys Ile
   260 265 270
   Val Lys Lys Gly Asp Ser Ala Ile Met Lys Ser Glu Leu Glu Tyr Gly
   275 280 285
   Asn Cys Asn Thr Lys Cys Gln Thr Pro Met Gly Ala Ile Asn Ser Ser
   290 295 300
   Met Pro Phe His Asn Ile His Pro Leu Thr Ile Gly Glu Cys Pro Lys
   305 310 315 320
   Tyr Val Lys Ser Asn Arg Leu Val Leu Ala Thr Gly Leu Arg Asn Ser
   325 330 335
   Pro Gln Arg Glu Ser Arg Arg Lys Lys Arg Gly Leu Phe Gly Ala Ile
   340 345 350
   Ala Gly Phe Ile Glu Gly Gly Trp Gln Gly Met Val Asp Gly Trp Tyr
   355 360 365
   Gly Tyr His His Ser Asn Glu Gln Gly Ser Gly Tyr Ala Ala Asp Lys
   370 375 380
   Glu Ser Thr Gln Lys Ala Ile Asp Gly Val Thr Asn Lys Val Asn Ser
   385                 390                 395                 400
   Ile Ile Asp Lys Met Asn Thr Gln Phe Glu Ala Val Gly Arg Glu Phe
                   405                 410                 415
   Asn Asn Leu Glu Arg Arg Ile Glu Asn Leu Asn Lys Lys Met Glu Asp
               420                 425                 430
   Gly Phe Leu Asp Val Trp Thr Tyr Asn Ala Glu Leu Leu Val Leu Met
           435                 440                 445
   Glu Asn Glu Arg Thr Leu Asp Phe His Asp Ser Asn Val Lys Asn Leu
       450                 455                 460
   Tyr Asp Lys Val Arg Leu Gln Leu Arg Asp Asn Ala Lys Glu Leu Gly
   465                 470                 475                 480
   Asn Gly Cys Phe Glu Phe Tyr His Lys Cys Asp Asn Glu Cys Met Glu
                   485                 490                 495
   Ser Ile Arg Asn Gly Thr Tyr Asn Tyr Pro Gln Tyr Ser Glu Glu Ala
               500                 505                 510
   Arg Leu Lys Arg Glu Glu Ile Ser Gly Val Lys Leu Glu Ser Ile Gly
           515                 520                 525
   Thr Tyr Gln Ile Leu Ser Ile Tyr Ser Thr Val Ala Ser Ser Leu Ala
       530                 535                 540
   Leu Ala Ile Met Met Ala Gly Leu Ser Leu Trp Met Cys Ser Asn Gly
   545                 550                 555                 560
   Ser Leu Gln Cys Arg Ile Cys Ile
                   565
<210> 11
   <211> 1629
   <212> DNA
   <213> Influenza virus
<400> 11
   gacaaaatat gtcttgggca ccatgctgtg gcaaatggaa caaaagtgaa cacattaaca 60
   gagaggggga ttgaagtagt gaacgccaca gagacggtgg aaactgcgaa tatcaagaaa 120
   atatgtattc aagggaaaag gccaacagat ctgggacaat gtggacttct aggaacccta 180
   ataggacctc cccaatgtga tcaattcctg gagttttact ctgatttgat aattgagcga 240
   agagaaggaa ccgatgtgtg ctatcccggt aaattcacaa atgaagaatc actgaggcag 300
   atccttcgag ggtcaggagg aattgataag gagtcaatgg gtttcaccta tagtggaata 360
   agaaccaatg gagcgacaag tgcctgcaaa agatcaggtt cttctttcta tgcagagatg 420
   aagtggttgc tgtcgaattc agacaatgcg gcattccctc aaatgacaaa gtcgtataga 480
   aatcccagaa acaaaccagc tctgataatt tggggagttc atcactctgg atcggttagc 540
   gagcagacca aactctatgg aagtggaaac aagttgataa cagtaggaag ctcaaaatac 600
   cagcaatcat tcaccccaag tccgggagca cggccacaag tgaatggaca atcagggaga 660
   atcgattttc actggctact ccttgatccc aatgacacag tgaccttcac tttcaatggg 720
   gcattcatag cccctgacag ggcaagtttc tttagaggag aatcactagg agtccagagt 780
   gatgttcctc tggattctag ttgtggaggg gattgctttc acagtggggg tacgatagtc 840
   agttccctgc cattccaaaa catcaaccct agaactgtgg ggagatgccc tcggtatgtc 900
   aaacagacaa gcctcctttt ggctacagga atgagaaatg ttccagagaa tccaaagccc 960
   agaggccttt ttggagcaat tgctggattc atagagaatg gatgggaggg tctcatcgat 1020
   ggatggtatg gtttcagaca tcaaaatgca caaggggaag gaactgcagc tgactacaaa 1080
   agcacccaat ctgcaataga tcagatcaca ggcaaattga atcgtctgat tgacaaaaca 1140
   aatcagcagt ttgagctgat agacaatgag ttcaatgaga tagaacaaca aataggaaat 1200
   gtcattaatt ggacacgaga cgcaatgact gaggtatggt cgtataatgc tgagctgttg 1260
   gtggcaatgg aaaatcagca tacaatagat cttgcggact cagaaatgaa caaactttat 1320
   gagcgtgtca gaaaacaact aagggagaat gctgaagaag atggaactgg atgttttgag 1380
   atattccata agtgtgatga tcagtgcatg gagagcataa ggaacaacac ttatgaccat 1440
   actcaataca gaacagagtc attgcagaat agaatacaga tagacccagt gaaattgagt 1500
   agtggataca aagacataat cttatggttt agcttcgggg catcatgttt tcttcttcta 1560
   gccgttgtaa tgggattggt tttcatttgc ataaagaatg gaaacatgcg gtgcaccatt 1620
   tgtatataa 1629
<210> 12
   <211> 1773
   <212> DNA
   <213> Influenza virus
<400> 12
   agcaaaagca ggggttatac catagacaac caaaggcaag acaatggcca tcatttatct     60
   aattcttctg ttcacagcag tgagagggga ccaaatatgc attggatacc attccaacaa    120
   ttccacagaa aaggttgaca caatcctaga gagaaatgtc actgtgactc acgctgagga    180
   cattcttgag aagactcaca atgggaagtt atgcaaacta aatggaatcc ctccacttga    240
   attaagggat tgcagcattg ccggatggct ccttgggaat ccagaatgtg atatacttct    300
   aactgtgcca gaatggtcat acataataga aaaagaaaat ccaaggaacg gcttgtgcta    360
   cccaggcagt ttcaatgatt atgaagaatt gaagcatctt atcagcagcg tgacacattt 420
   tgagaaagta aagattctgc ccagaaatga atggacacag catacaacaa ctggaggttc 480
   acaggcttgc gcagactatg gtggtccgtc attcttccgg aacatggtct ggttgacaaa 540
   gaaagggtcg aattatccaa ttgccaaaag atcttacaac aatacaagtg gggaacaaat 600
   gctgatcatt tgggggatac atcaccccaa tgatgaaagt gaacaaagag cattgtatca 660
   gaatgtgggg acctatgtgt cagtaggaac atcaacactg aacaaaagat catccccaga 720
   aatagcaaca agacctaaag tgaatggaca aggaggcaga atggaattct cgtggactat 780
   cttagatata tgggacacaa taaattttga gagtactggc aatctaattg caccagaata 840
   tggtttcaaa atatccaaac gaggtagttc agggatcatg aaaacagaag gaaaacttga 900
   aaactgcgag accaagtgcc aaactccttt gggagcaata aatacaacat taccctttca 960
   caatatccac ccactgacca ttggtgagtg ccccaaatat gtaaaatcgg aaagattagt 1020
   cttagcaaca ggactaagaa acgtccctca gattgagtca aggggattgt ttggggcaat 1080
   agctggtttt atagagggtg gatggcaagg aatggttgat ggttggtatg ggtatcatca 1140
   cagcaatgac cagggatctg ggtatgcagc agacaaagaa tccactcaaa aggcaattga 1200
   tggaatcacc aacaaggtaa attctgtgat cgaaaagatg aacacccaat tcggagctgt 1260
   tggaaaagaa ttcagtaact tggagagaag actggagaac ttgaataaaa agatggagga 1320
   cggatttcta gatgtgtgga catacaatgc cgagctccta gttctaatgg aaaatgagag 1380
   gacacttgac tttcatgatt ctaatgtcaa gaatctatat gataaagtca gaatgcaact 1440
   gagagacaat gcaaaagaac tagggaatgg atgttttgaa ttttatcaca aatgtgatga 1500
   tgaatgcatg aacagtgtga agaatgggac atatgattat tccaagtatg aagaggagtc 1560
   taaactaaac aggactgaaa tcaaaggggt taaattgagc aatatggggg tttatcaaat 1620
   ccttgccatc tatgctacag tagcaggttc cctgtcactg gcaatcatga tagctgggat 1680
   ttctatatgg atgtgctcca acgggtctct gcaatgcaga atctgcatat gatcatcagt 1740
   cattttgtaa ttaaaaacac ccttgtttct act 1773
<210> 13
   <211> 1086
   <212> DNA
   <213> Influenza virus
<400> 13
   caaaaacttc ccggaaatga caacagcacg gcaacgctgt gccttgggca ccatgcagta 60
   ccaaacggaa cgatagtgaa aacaatcacg aatgaccaaa ttgaagttac taatgctact 120
   gagctggtac agagttcctc aacaggtgga atatgcgaca gtcctcatca gatccttgat 180
   ggagaaaact gcacactaat agatgctcta ttgggagacc ctcagtgtga tggcttccaa 240
   aataagaaat gggacctttt tgttgaacgc agcaaagcct acagcaactg ttacccttat 300
   gatgtgccgg attatgcctc ccttaggtca ctagttgcct catccggcac actggagttt 360
   aacaatgaaa gcttcgattg gactggagtc actcagaatg gaacaagctc tgcttgcaaa 420
   aggagatcta ataaaagttt ctttagtaga ttgaattggt tgacccactt aaaatacaaa 480
   tacccagcat tgaacgtgac tatgccaaac aatgaaaaat ttgacaaatt gtacatttgg 540
   ggggttcacc acccgggtac ggacagtgac caaatcagcc tatatgctca agcatcagga 600
   agaatcacag tctctaccaa aagaagccaa caaactgtaa tcccgaatat cggatctaga 660
   cccagggtaa gggatgtctc cagccgaata agcatctatt ggacaatagt aaaaccggga 720
   gacatacttt tgattaacag cacagggaat ctaattgctc ctcggggtta cttcaaaata 780
   cgaagtggga aaagctcaat aatgagatca gatgcaccca ttggcaaatg caattccgaa 840
   tgcatcactc caaatggaag cattcccaat gacaaaccat ttcaaaatgt aaacaggatc 900
   acatatgggg cctgtcccag atatgttaag caaaacactc tgaaattggc aacagggatg 960
   cgaaatgtac cagagaaaca aactagaggc atatttggcg caatcgcggg tttcatagaa 1020
   aatggttggg agggaatggt ggacggttgg tacggtttca ggcatcaaaa ttctgagggc 1080
   acagga 1086
<210> 14
   <211> 1048
   <212> DNA
   <213> Influenza virus
<400> 14
   atgctatcaa tcacgattct gtttctgctc atagcagagg gttcctctca gaattacaca 60
   gggaatcccg tgatatgcct gggacatcat gccgtatcca atgggacaat ggtgaaaacc 120
   ctgactgatg accaagtaga agttgtcact gcccaagaat tagtggaatc gcaacatcta 180
   ccggagttgt gtcctagccc tttaagatta gtagatggac aaacttgtga catcgtcaat 240
   ggtgccttgg ggagtccagg ctgtgatcac ttgaatggtg cagaatggga tgtcttcata 300
   gaacgaccca ctgctgtgga cacttgttat ccatttgatg tgccggatta ccagagccta 360
   cggagtatcc tagcaaacaa tgggaaattt gagttcattg ctgaggaatt ccaatggaac 420
   acagtcaaac aaaatgggaa atccggagca tgcaaaagag caaatgtgaa tgactttttc 480
   aacagattga actggctgac caaatctgat gggaatgcat acccacttca aaacctgaca 540
   aaggttaaca acggggacta tgcaagactt tacatatggg gagttcatca tccttcaact 600
   gacacagaac aaaccaactt gtataagaac aaccctggga gagtaactgt ttccaccaaa 660
   accagtcaaa caagtgtggt accaaacatt ggcagtagac catgggtaag aggccaaagc 720
   ggcaggatta gcttctattg gacaattgtg gagccaggag acctcatagt cttcaacacc 780
   atagggaatt taattgctcc gagaggtcat tacaagctta acagtcaaaa gaagagcaca 840
   attctgaata ctgcaattcc cataggatct tgtgttagta aatgtcacac agataggggt 900
   tcaatctcta caaccaaacc ctttcagaac atctcaagaa tatcaattgg ggactgtccc 960
   aagtatgtca aacagggatc cttgaaacta gctacaggaa tgaggaatat ccctgagaaa 1020
   gcaaccagag gcctgtttgg tgcaattg 1048
<210> 15
   <211> 1707
   <212> DNA
   <213> Influenza virus
<400> 15
   atggagaaaa tagtgcttct tcttgcaata gtcagtcttg ttaaaagtga tcagatttgc      60
   attggttacc atgcaaacaa ctcgacagag caggttgaca caataatgga aaagaacgtt     120
   actgttacac atgcccaaga catactggaa aagacacaca acgggaaact ctgcgatcta     180
   gatggagtga agcctctaat tttgagagat tgtagtgtag ctggatggct cctcggaaac     240
   cctatgtgtg acgaattcat caatgtgccg gaatggtctt acatagtgga gaaggccagt     300
   ccagccaatg acctctgtta cccaggggat ttcaacgact atgaagaact gaaacaccta     360
   ttgagcagaa taaaccactt tgagaaaatt cagatcatcc ccaaaagttc ttggtccaat     420
   catgaagcct catcaggggt gagcgcagca tgtccatacc atgggaagcc ctcctttttc     480
   agaaatgtgg tatggcttat caaaaagaac agtgcatacc caacaataaa gaggagctac     540
   aataatacca accaagaaga tcttttggta ctgtggggga ttcaccatcc taatgatgcg     600
   gcagagcaga caaagctcta tcaaaaccca accacctata tttccgttgg aacatcaaca     660
   ctaaaccaga gattggtccc aaaaatagct actagatcca aagtaaacgg gcaaagtgga     720
   agaatggagt tcttctggac aattttaaag ccgaatgatg ccataaattt cgagagtaat     780
   ggaaatttca ttgctccaga atatgcatac aaaattgtca agaaagggga ctcagcaatt     840
   atgaaaagtg aattggaata tggtaactgc aacaccaagt gtcaaactcc aatgggggcg     900
   ataaactcta gtatgccatt ccacaacata caccctctca caatcgggga atgccccaaa     960
   tatgtgaaat caaacagatt agtccttgcg actggactca gaaatacccc tcaaagagat    1020
   agaagaagaa aaaagagagg actatttgga gctatagcag gttttataga gggaggatgg    1080
   caaggaatgg tagatggttg gtatgggtac caccatagca atgagcaggg gagtggatac    1140
   gctgcagaca aagaatccac tcaaaaggca atagatggag tcaccaataa ggtcaactcg    1200
   atcattgaca aaatgaacac tcagtttgag gccgttggaa gggaatttaa taacttagaa    1260
   aggaggatag aaaatttaaa caagaagatg gaagacggat tcctagatgt ctggacttat    1320
   aatgctgaac ttctggttct catggaaaat gagagaactc tagactttca tgattcaaat    1380
   gtcaagaacc tttacaacaa ggtccgacta cagcttaggg ataatgcaaa ggagctgggt    1440
   aatggttgtt tcgagttcta tcacaaatgt gataatgaat gtatggaaag tgtaaaaaac    1500
   gggacgtatg actacccgca gtattcagaa gaagcaagac taaacagaga ggaaataagt    1560
   ggagtaaaat tggaatcaat gggaacttac caaatactgt caatttattc aacagtggcg    1620
   agttccctag cactggcaat catggtagct ggtctatctt tatggatgtg ctccaatggg    1680
   tcgttacaat gcagaatttg catttaa                                        1707
<210> 16
   <211> 1050
   <212> DNA
   <213> Influenza virus
<400> 16
   atgattgcaa tcattgtaat agcgatactg gcagcagccg gaaagtcaga caagatctgc      60
   attgggtatc atgccaacaa ttcaacaaca caggtggata cgatacttga gaagaatgta     120
   accgtcacac actcagttga attgctggag aatcagaagg aagaaagatt ctgcaagatc     180
   ttgaacaagg cccctctcga cctaaaggga tgcaccatag agggttggat cttggggaat     240
   ccccaatgcg atctgttgct tggtgaccaa agctggtcat atatagtgga aagacctact     300
   gcccaaaatg ggatatgcta cccaggagct ttgaatgagg tagaagaact gaaagcattt     360
   atcggatcag gagaaagggt agagagattt gagatgtttc ccaaaagcac atgggcaggg     420
   gtagacacca gcagtggggt aacaaaagct tgtccttata atagtggttc atctttctac     480
   agaaacctcc tatggataat aaagaccaag tcagcagcgt atccagtaat taagggaact     540
   tacagcaaca ctggaaacca gccaatcctc tatttctggg gtgtgcacca tcctcctgac     600
   accaatgagc aaaatactct gtatggctct ggcgatcggt atgttaggat gggaactgag     660
   agcatgaatt ttgccaagag cccagaaatt gcggcaagac ccgctgtgaa tggccaaaga     720
   ggtcgaattg attattactg gtctgtttta aaaccaggag aaaccttgaa tgtggaatct     780
   aatggaaatc taatcgctcc ttggtatgca tacaaatttg tcaacacaaa taataaggga     840
   gccgtcttca agtcaaattt accaatcgag aattgcgatg ccacatgcca gactattgca     900
   ggagtcctaa ggaccaataa aacatttcag aatgtgagcc ctctgtggat aggagaatgc     960
   cccaagtatg tgaaaagtga aagtctaagg cttgctactg gactaagaaa tgttccacag    1020
   attgaaacca gagggctttt cggagctatc                                    1050
<210> 17
   <211> 1698
   <212> DNA
   <213> Influenza virus
<400> 17
   atggaaaaat tcatcgcaat agcaaccttg gcgagcacaa atgcatacga taggatatgc      60
   attgggtacc aatcaaacaa ctccacagac acagtgaaca ctctcataga acagaatgta     120
   ccagtcaccc aaacaatgga gctcgtggaa acagagaaac atcccgctta ttgtaacact     180
   gatttaggtg ccccattgga actgcgagac tgcaagattg aggcagtaat ctatgggaac     240
   cccaagtgtg acatccatct gaaggatcaa ggttggtcat acatagtgga gaggcccagc    300
   gcaccagaag ggatgtgtta ccctggatct gtggaaaatc tagaagaact gaggtttgtc    360
   ttctccagtg ctgcatctta caagagaata agactatttg actattccag gtggaatgtg    420
   actagatctg gaacgagtaa agcatgcaat gcatcaacag gtggccaatc cttctatagg    480
   agcatcaatt ggttgaccaa aaaggaacca gacacttatg acttcaatga aggagcttat    540
   gttaataatg aagatggaga catcattttc ttatggggga tccatcatcc gccggacaca    600
   aaagagcaga caacactata taaaaatgca aacactttga gtagtgttac tactaacact    660
   ataaacagaa gctttcaacc aaatattggt cccagaccat tagtaagagg acagcaaggg    720
   aggatggatt actattgggg cattctgaaa agaggggaga ctctgaagat caggaccaac    780
   ggaaatttaa tcgcacctga atttggctat ctgctcaaag gtgaaagcta cggcagaata    840
   attcaaaatg aggatatacc catcgggaac tgtaacacaa aatgtcaaac atatgcggga    900
   gcaatcaata gcagcaaacc ctttcagaat gcaagtaggc attacatggg agaatgtccc    960
   aaatatgtga agaaggcaag cttgcgactt gcagttgggc ttaggaatac gccttctgtt   1020
   gaacccagag gactgtttgg agccattgct ggtttcattg aaggaggatg gtctggaatg   1080
   attgatgggt ggtatggatt tcatcacagc aattcagagg gaacaggaat ggcagctgac   1140
   cagaaatcaa cacaagaagc catcgataag atcaccaata aagtcaacaa tatagttgac   1200
   aagatgaaca gggagtttga agttgtgaat catgagttct ctgaagttga aaaaagaata   1260
   aacatgataa acgataaaat agatgaccaa attgaagatc tttgggctta caatgcagag   1320
   ctccttgtgc tcttagagaa ccagaaaacg ctagacgaac atgattccaa tgtcaaaaac   1380
   ctttttgatg aagtgaaaag gagactgtca gccaatgcaa tagatgctgg gaacggttgc   1440
   tttgacatac ttcacaaatg cgacaatgag tgtatggaaa ctataaagaa cggaacttac   1500
   gatcataagg aatatgaaga ggaggctaaa ctagaaagga gcaagataaa tggagtaaaa   1560
   ctagaagaga acaccactta caaaattctt agcatttaca gtacagtggc ggccagtctt   1620
   tgcttggcaa tcctgattgc tggaggttta atcctgggca tgcaaaatgg atcttgtaga   1680
   tgcatgttct gtatttga                                                1698
<210> 18
   <211> 1363
   <212> DNA
   <213> Influenza virus
<400> 18
   atggaaacag tatcactaat gactatacta ctagtagcaa cagcaagcaa tgcagacaaa      60
   atctgcatcg gccaccagtc aacaaactcc acagaaactg tggacacgct aacagaaacc     120
   aatgttcctg tgacacatgc caaagaattg ctccacacag agcacaatgg aatgctgtgt     180
   gcaacaaatc tgggacatcc cctaatctta gacacgtgca ctattgaagg actgatctat     240
   ggtaaccctt cttgtgactt gctgttggga ggaagagaat ggtcctacat cgtcgaaagg     300
   tcatcagctg taaatggaac gtgttaccct gggaatgtag agaacctaga ggaactcagg     360
   acacttttta gttccgctag ttcctaccga agaatccaaa tcttcccaga cacaatctgg     420
   aatgtgactt acactggaac aagcaaagca tgttcagatt cattctacag gagtatgaga     480
   tggctgactc aaaaaagcgg gtcttaccct gttcaagacg ctcaatacac aaataatatg     540
   ggaaagagca ttcttttcgt gtggggcata catcacccac ccactgaagc tgcacagaca     600
   aatttgtaca caagaaccga cacaacaaca agcgtgacaa cagaagactt aaataggatc     660
   ttcaaaccga tggtagggcc aaggcccctt gtcaatggtc tgcagggaag aattaattat     720
   tattggtcgg tactaaaacc aggccagaca ctgcgagtaa gatccaatgg gaatctaatt     780
   gctccatggt atggacacat tctttcggga gggagccatg gaagaatcct gaagactgat     840
   ttaaaaagta gtaattgcgt agtgcaatgt cagactgaaa aaggcggctt aaacagtaca     900
   ttgccgttcc acaatatcag taaatatgca tttggaaact gtcccaaata tgttagagtt     960
   aaaagtctca aactggcagt agggttgagg aacgtgcctg ctagatcaag tagaggacta    1020
   ttcggagcca tagctggatt catagaagga ggttggccag gactagtcgc tggttggtat    1080
   ggtttccagc attcaaatga tcaaggggtt ggtattgcgg cagataggga ttcaactcaa    1140
   aaggcaattg atagaataac aaccaaggtg aataatatag tcgacaaaat gaacaaacaa    1200
   tatgaaataa ttgatcatga attcagtgag gttgaaacta ggctcaacat gatcaataat    1260
   aagattgatg accaaataca agacatatgg gcatataatg cagagttgct agtactactt    1320
   gaaaaccaga aaacactcga tgagcatgac gcaaatgtga aga                      1363
<210> 19
   <211> 1727
   <212> DNA
   <213> Influenza virus
<400> 19
   agcaaaagca ggggtcacaa tgtacaaagt agtagtaata attgcgctcc ttggagcagt      60
   gaaaggtctt gacagaatct gcctaggaca ccatgcggtt gccaatggaa ccattgtgaa     120
   gacccttaca aatgaacaag aggaagtgac caatgctact gagacggtag agagcacaaa     180
   tttgaataaa ttgtgtatga aaggaagaag ctacaaggac ttgggcaatt gtcacccggt     240
   aggaatgttg ataggaacac ctgtttgtga tccgcacttg accgggacct gggacactct     300
   cattgagcga gagaatgcca ttgcccactg ttatccaggg gcaaccataa atgaagaagc     360
   attgaggcag aaaataatgg aaagtggagg aatcagcaag atgagcactg gcttcactta     420
   tgggtcttcc atcacctcag ctgggaccac taaggcatgc atgagaaatg gaggagatag     480
   tttctatgca gagctcaaat ggctagtgtc aaagacaaag ggacaaaatt tccctcagac     540
   aacaaacacc tatcggaata cggacacagc agaacatctc ataatatggg gaattcatca     600
   cccttccagc acacaggaaa agaatgactt atacggaact cagtcactat ctatatcagt     660
   tgagagttct acatatcaga acaactttgt tccagttgtt ggggcaagac ctcaggtcaa     720
   tggacaaagt gggcgaattg actttcactg gacactagta cagccgggtg acaacataac     780
   cttctcagac aatggaggtc taatagcacc aagtcgagtt agcaaattaa ctggaaggga     840
   tttgggaatc caatcagaag cgttgataga caacagttgt gaatccaaat gcttttggag     900
   agggggttct ataaatacaa agctcccttt tcaaaatctg tcacccagaa cagtaggtca     960
   atgccccaaa tacgtaaatc agaggagttt actgcttgca acagggatga ggaatgtgcc    1020
   agaagtggtg cagggaaggg gtctgtttgg tgcaatagca gggttcatag aaaacggatg    1080
   ggaaggaatg gtagacggct ggtatggttt cagacaccaa aatgcccagg gcacaggcca    1140
   agctgctgat tacaagagta ctcaagcagc tattgaccaa atcacaggga aactgaacag    1200
   gttgattgag aagaccaaca ctgagtttga gtcaatagaa tctgaattca gtgagactga    1260
   gcatcaaatt ggtaacgtca ttaattggac caaagattca ataaccgaca tttggactta    1320
   caacgcagag ctattagtgg caatggagaa tcagcacaca attgacatgg ctgattcaga    1380
   gatgctaaat ctgtatgaaa gggtaagaaa gcaactcaga cagaatgcag aagaagacgg    1440
   aaagggatgt tttgagatat atcatacttg tgatgattcg tgcatggaga gtataaggaa    1500
   caatacttat gaccattcac aatacagaga ggaggctctt ctgaatagac tgaacatcaa    1560
   cccagtgaaa ctttcttcgg ggtacaaaga catcatactt tggtttagct tcggggaatc    1620
   atgctttgtt cttctagccg ttgttatggg tcttgttttc ttctgcctga aaatggaaaa    1680
   catgcgatgc acaatctgta tttagttaaa aacaccttgt ttctact                 1727
<210> 20
   <211> 1698
   <212> DNA
   <213> Influenza virus
<400> 20
   atggagaaaa cactgctatt tgcagctatt ttcctttgtg tgaaagcaga tgagatctgt      60
   atcgggtatt taagcaacaa ctcgacagac aaagttgaca caataattga gaacaatgtc     120
   acggtcacta gctcagtgga actggttgag acagaacaca ctggatcatt ctgttcaatc     180
   aatggaaaac aaccaataag ccttggagat tgttcatttg ctggatggat attaggaaac     240
   cctatgtgtg atgaactaat tggaaagact tcatggtctt acattgtgga aaaacccaat     300
   ccaacaaatg gaatctgtta cccaggaact ttagagagtg aagaagaact aagactgaaa     360
   ttcagtggag ttttagaatt taacaaattc gaagtattca catcaaatgg atggggtgct     420
   gtaaattcag gagtaggagt aaccgctgca tgcaaattcg ggggttctaa ttctttcttt      480
   cgaaacatgg tatggctgat acaccaatca ggaacatatc ctgtaataaa gagaaccttt     540
   aacaacacca aagggagaga tgtactgatt gtttggggaa ttcatcatcc tgctacactg     600
   acagaacatc aagatctgta taaaaaggac agctcctatg tagcagtggg ttcagagacc 660
   tacaacagaa gattcactcc agaaatcaac actaggccca gagtcaatgg acaggccgga 720
   cggatgacat tctactggaa gatagtcaaa ccaggagaat caataacatt cgaatctaat 780
   ggggcgttcc tagctcctag atatgctttt gagattgtct ctgttggaaa tgggaaactg 840
   ttcaggagcg aactgaacat tgaatcatgc tctaccaaat gtcaaacaga aataggagga 900
   attaatacga acaaaagctt ccacaatgtt cacagaaaca ctatcgggga ttgccccaag 960
   tatgtgaatg tcaaatcctt aaagcttgca acaggaccta gaaatgtccc agcaatagca 1020
   tcgagaggct tgtttggagc aatagctgga ttcatagaag ggggatggcc tggactgatc 1080
   aatggatggt atgggttcca acacagggac gaagaaggaa caggcattgc agcagacaag 1140
   gagtcaactc aaaaggcaat agaccagata acatccaagg taaataacat cgttgacagg 1200
   atgaatacaa actttgagtc tgtgcaacac gaattcagtg aaatagagga aagaataaat 1260
   caattatcaa aacacgtaga tgattctgtg gttgacatct ggtcatataa tgcacagctt 1320
   ctcgttttac ttgaaaatga gaagacactg gacctccatg actcaaatgt caggaacctc 1380
   catgagaaag tcagaagaat gctaaaggac aatgccaaag atgaggggaa cggatgcttc 1440
   accttttacc ataagtgtga caataaatgc attgaacgag ttagaaacgg aacatatgat 1500
   cataaagaat tcgaggagga atcaaaaatc aatcgccagg agattgaagg ggtgaaacta 1560
   gattctagtg ggaatgtgta taaaatactg tcaatttaca gctgcattgc aagcagtctt 1620
   gtattggcag cactcatcat ggggttcatg ttttgggcat gcagtaatgg atcatgtaga 1680
   tgtaccattt gcatttag 1698
<210> 21
   <211> 1695
   <212> DNA
   <213> Influenza virus
<400> 21
   atggaaaaat tcatcatttt gagtactgtc ttggcagcaa gctttgcata tgacaaaatt 60
   tgcattggat accaaacaaa caactcgact gaaacggtaa acacactaag tgaacaaaac 120
   gttccggtga cgcaggtgga agaacttgta catcgtggga ttgatccgat cctgtgtgga 180
   acggaactag gatcaccact agtgcttgat gactgttcat tagagggtct aatcctaggc 240
   aatcccaaat gtgatcttta tttgaatggc agggaatggt catacatagt agagaggccc 300
   aaagagatgg aaggagtttg ctatccaggg tcaattgaaa accaggaaga gctaagatct 360
   ctgttttctt ccatcaaaaa atatgaaaga gtgaagatgt ttgatttcac caaatggaat 420
   gtcacataca ctgggaccag caaggcctgc aataatacat caaaccaagg ctcattctat 480
   aggagcatga gatggttgac cttaaaatca ggacaatttc cagtccaaac agatgagtac 540
   aagaacacca gagattcaga cattgtattc acctgggcca ttcaccaccc accaacatct 600
   gatgaacaag taaaattata caaaaatcct gatactctct cttcagtcac caccgtagaa 660
   atcaatagga gcttcaagcc taatataggg ccaagaccac tcgtgagagg acaacaaggg 720
   agaatggatt actactgggc tgttcttaaa cctggacaaa cagtcaaaat acaaaccaat 780
   ggtaatctta ttgcacctga atatggtcac ttaatcacag ggaaatcaca tggcaggata 840
   ctcaagaata atttgcccat gggacagtgt gtgactgaat gtcaattgaa cgagggtgta 900
   atgaacacaa gcaaaccttt ccagaacact agtaagcact atattgggaa atgccccaaa 960
   tacataccat cagggagttt aaaattggca atagggctca ggaatgtccc acaagttcaa 1020
   gatcgggggc tctttggagc aattgcaggt ttcatagaag gcggatggcc agggctagtg 1080
   gctggttggt acggatttca gcatcaaaat gcggagggga caggcatagc tgcagacaga 1140
   gacagcaccc aaagggcaat agacaatatg caaaacaaac tcaacaatgt catcgacaaa 1200
   atgaataaac aatttgaagt ggtgaatcat gagttttcag aagtggaaag cagaataaac 1260
   atgattaatt ccaaaattga tgatcagata actgacatat gggcatacaa tgctgaattg 1320
   cttgtcctat tggaaaatca gaagacatta gatgagcatg acgctaatgt aaggaatcta 1380
   catgatcggg tcagaagagt cctgagggaa aatgcaattg acacaggaga cggctgcttt 1440
   gagattttac ataaatgtga caacaattgt atggacacga ttagaaacgg gacatacaat 1500
   cacaaagagt atgaggaaga aagcaaaatc gaacgacaga aagtcaatgg tgtgaaactt 1560
   gaggagaatt ctacatataa aattctgagc atctacagca gtgttgcctc aagcttagtt 1620
   ctactgctca tgattattgg gggtttcatt ttcgggtgtc aaaatggaaa tgttcgttgt 1680
   actttctgta tttaa 1695
<210> 22
   <211> 1701
   <212> DNA
   <213> Influenza virus
<400> 22
   atggctctaa atgtcattgc aactttgaca cttataagtg tatgtgtaca tgcagacaga 60
   atatgcgtgg ggtatctgag caccaattca tcagaaaggg tcgacacgct ccttgaaaat 120
   ggggtcccag tcaccagctc cattgatctg attgagacaa accacacagg aacatactgt 180
   tctctaaatg gagtcagtcc agtgcatttg ggagattgca gctttgaagg atggattgta 240
   ggaaacccag cctgcaccag caactttggg atcagagagt ggtcatacct gattgaggac 300
   cccgcggccc ctcatgggct ttgctaccct ggagaattaa acaacaatgg tgaactcaga 360
   cacttgttca gtggaatcag gtcattcagt agaacggaat tgatcccacc tacctcctgg 420
   ggggaagtac ttgacggtac aacatctgct tgcagagata acacgggaac caacagcttc 480
   tatcgaaatt tagtttggtt tataaagaag aatactagat atccagttat cagtaagacc 540
   tacaacaata caacgggaag ggatgtttta gttttatggg gaatacatca cccagtgtct 600
   gtggatgaga caaagactct gtatgtcaat agtgatccat acacactggt ttccaccaag 660
   tcttggagcg agaaatataa actagaaacg ggagtccgac ctggctataa tggacagagg 720
   agctggatga aaatttattg gtctttgata catccagggg agatgattac tttcgagagt 780
   aatggtggat ttttagcccc aagatatggg tacataattg aagaatatgg aaaaggaagg 840
   attttccaga gtcgcatcag aatgtctagg tgcaacacca agtgccagac ttcggttgga 900
   gggataaaca caaacagaac gttccaaaac atcgataaga atgctcttgg tgactgtccc 960
   aaatacataa agtctggcca actcaagcta gccactggac tcagaaatgt gccagctata 1020
   tcgaatagag gattgttcgg agcaattgca gggttcatag aaggaggctg gccaggttta 1080
   atcaatggtt ggtacggttt tcagcatcaa aatgaacagg gaacaggaat agctgcagac 1140
   aaagaatcaa cacagaaagc tatagaccag ataacaacca aaataaataa cattattgat 1200
   aaaatgaatg ggaactatga ttcaattagg ggtgaattca atcaagttga gaagcgtata 1260
   aacatgcttg cagacagaat agatgatgcc gtgacggaca tttggtcata caatgccaaa 1320
   cttcttgtat tgctggaaaa tgataaaact ttagatatgc atgatgctaa tgtaaagaat 1380
   ttacatgagc aagtacgaag agaattgaag gacaatgcaa ttgacgaagg aaatggctgt 1440
   tttgaactcc ttcataaatg caatgactcc tgcatggaaa ctataagaaa tggaacgtat 1500
   gaccacactg agtatgcaga ggagtcaaag ttaaagaggc aagaaatcga tgggatcaaa 1560
   ctcaaatcag aagacaacgt ttacaaagca ttatcaatat acagttgcat tgcaagtagt 1620
   gttgtactag taggactcat actctctttc atcatgtggg cctgtagtag tgggaattgc 1680
   cgattcaatg tttgtatata a 1701
<210> 23
   <211> 1749
   <212> DNA
   <213> Influenza virus
<400> 23
   agcaaaagca ggggaaaatg attgcactca tattggttgc actggctctg agccacactg 60
   cttattctca gatcacaaat gggacaacag gaaaccccat tatatgcttg gggcatcatg 120
   cagtggaaaa cggcacatct gttaaaacac taacagacaa tcacgtagaa gttgtgtcag 180
   ctaaagaatt agttgagacg aaccacactg atgaactgtg cccaagcccc ttgaagcttg 240
   tcgacgggca agactgccac ctcatcaatg gtgcattggg gagtccaggc tgtgaccgtt 300
   tgcaggacac cacttgggat gtcttcattg aaaggcccac tgcagtagac acatgttatc 360
   cattcgacgt cccagattac cagagtctca gaagcatcct agcaagcagt gggagtttgg 420
   agttcatcgc cgaacaattc acctggaatg gtgtcaaagt tgacggatca agcagtgctt 480
   gtttgagggg cggtcgcaac agcttcttct cccgactaaa ctggctaacc aaagcaacaa 540
   atggaaacta tggacctatt aacgtcacta aagaaaatac gggctcttat gtcaggctct 600
   atctctgggg agtgcatcac ccatcaagcg ataatgagca aacggatctc tacaaggtgg 660
   caacagggag agtaacagta tctacccgct cggaccaaat cagtattgtt cccaatatag 720
   gaagtagacc gagggtaagg aatcagagcg gcaggataag catctactgg accctagtaa 780
   acccagggga ctccatcatt ttcaacagta ttgggaattt gattgcacca agaggccact 840
   acaaaataag caaatctact aagagcacag tgcttaaaag tgacaaaagg attgggtcat 900
   gcacaagccc ttgcttaact gataaaggtt cgatccaaag tgacaaacct tttcagaatg 960
   tatcaaggat tgctatagga aactgcccga aatatgtaaa gcaagggtcc ctgatgttag 1020
   caactggaat gcgcaacatc cctggcaaac aggcaaaggg cttatttggg gcaattgctg 1080
   gattcattga aaatggttgg caaggcctga ttgatgggtg gtatggattc aggcaccaaa 1140
   atgctgaagg aacaggaact gctgcagacc tgaagtcaac tcaggcagcc attgatcaga 1200
   taaatggcaa gctgaacaga ttgatagaga agacaaatga aaaatatcac caaatagaaa 1260
   aggaattcga acaggtggaa ggaagaatac aagaccttga gaagtacgtt gaggacacta 1320
   agattgattt gtggtcatac aatgctgaat tgctagtagc actagagaat cagcacacaa 1380
   tagatgtcac agactccgaa atgaacaagc tttttgaaag agtaagaagg caattaagag 1440
   agaatgcaga agatcaaggc aacggttgtt tcgagatatt ccatcagtgt gacaacaatt 1500
   gtatagaaag cattagaaac ggaacttatg accacaacat ctacagggat gaagccatca 1560
   acaatcgaat caaaataaat cctgtcactt tgacgatggg gtacaaggac ataatcctgt 1620
   ggatttcttt ctccatgtca tgctttgtct tcgtggcact gattctggga tttgttctat 1680
   gggcttgtca aaacgggaat atccgatgcc aaatctgtat ataaagaaaa aacacccttg 1740
   tttctactc 1749
<210> 24
   <211> 1762
   <212> DNA
   <213> Influenza virus
<400> 24
   agcaaaagca ggggatacaa aatgaacact caaatcatcg tcattctagt cctcggactg     60
   tcgatggtga gatctgacaa gatttgtctc gggcaccatg ccgtagcaaa tgggacaaaa    120
   gtcaacacac taactgagaa aggagtggaa gtggtcaatg ccacggagac agtggagatt    180
   acaggaataa ataaagtgtg cacaaaaggg aagaaagcgg tggacttggg atcttgtgga    240
   atactgggaa ctatcattgg gcctccacaa tgtgactctc atcttaaatt caaagctgat    300
   ctgataatag aaagaagaaa ttcaagtgac atctgttacc cagggaaatt cactaatgag    360
   gaagcactga gacaaataat cagagaatct ggtggaattg acaaagagcc aatgggattt    420
   agatattcag gaataaaaac agacggggca accagtgcgt gtaagagaac agtgtcctct    480
   ttctactcag aaatgaaatg gcttttatcc agcaaggcta accaggtgtt cccacaactg    540
   aatcagacat acaggaacaa cagaaaagaa ccagccctaa ttgtttgggg agtacatcat 600
   tcaagttcct tggatgagca aaataagcta tatggagctg ggaacaagct gataacagta 660
   ggaagctcaa aataccaaca atcgttttca ccaagtccag gggacaggcc caaagtgaat 720
   ggtcaggccg ggaggatcga ctttcattgg atgctattgg acccagggga tacagtcact 780
   tttaccttca atggtgcatt catagcccca gatagagcca cctttctccg ctctaatgcc 840
   ccatcgggag ttgagtacaa tgggaagtca ctgggaatac agagtgatgc acaaattgat 900
   gaatcatgtg aaggggaatg cttctacagt ggagggacaa taaacagccc tttgccattt 960
   caaaacatcg atagttgggc tgtcggaagg tgccccagat atgtaaagca atcaagcctg 1020
   ccgctggcct taggaatgaa aaatgtacca gagaaaatac atactagggg actgttcggt 1080
   gcaattgcag gattcatcga gaatggatgg gaaggactca ttgatggatg gtatggattt 1140
   aggcatcaaa atgcacaggg gcagggaaca gctgctgact acaagagtac tcaggctgca 1200
   attgaccaga taacagggaa acttaataga ttaattgaaa aaaccaacac acagtttgaa 1260
   ctcatagaca atgagttcac tgaagtggag cagcagatag gcaatgtaat aaactggaca 1320
   agggactcct tgactgagat ctggtcatac aatgctgaac ttctagtagc aatggaaaat 1380
   cagcatacaa ttgaccttgc agattctgaa atgaacaaac tctatgagag agtgagaaga 1440
   cagctaaggg agaatgccga ggaggatgga actggatgtt ttgagatttt ccaccgatgt 1500
   gacgatcaat gtatggagag catacgaaat aatacttaca atcacactga atatcgacag 1560
   gaagccttac agaataggat aatgatcaat ccggtaaagc ttagtggtgg gtacaaagat 1620
   gtgatactat ggtttagctt cggggcatca tgtgtaatgc ttctagccat tgctatgggt 1680
   cttattttca tgtgtgtgaa aaacgggaat ctgcggtgca ctatctgtat ataattattt 1740
   gaaaaacacc cttgtttcta ct 1762
<210> 25
   <211> 1760
   <212> DNA
   <213> Influenza virus
<400> 25
   agcaaaagca ggggatattg tcaaaacaac agaatggtga tcaaagtgct ctactttctc 60
   atcgtattgt taagtaggta ttcgaaagca gacaaaatat gcataggata tctaagcaac 120
   aacgccacag acacagtaga cacactgaca gagaacggag ttccagtgac cagctcagtt 180
   gatctcgttg aaacaaacca cacaggaaca tactgctcac tgaatggaat cagcccaatt 240
   catcttggtg actgcagctt tgagggatgg atcgtaggaa acccttcctg tgccaccaac    300
   atcaacatca gagagtggtc gtatctaatt gaggacccca atgcccccaa caaactctgc    360
   ttcccaggag agttagataa taatggagaa ttacgacatc tcttcagcgg agtgaactct 420
   tttagcagaa cagaattaat aagtcccaac aaatggggag acattctgga tggagtcacc 480
   gcttcttgcc gcgataatgg ggcaagcagt ttttacagaa atttggtctg gatagtgaag 540
   aataaaaatg gaaaataccc tgtcataaag ggggattaca ataacacaac aggcagagat 600
   gttctagtac tctggggcat tcaccatccg gatacagaaa caacagccat aaacttgtac 660
   gcaagcaaaa acccctacac attagtatca acaaaggaat ggagcaaaag atatgaacta 720
   gaaattggca ccagaatagg tgatggacag agaagttgga tgaaactata ttggcacctc 780
   atgcgccctg gagagaggat aatgtttgaa agcaacgggg gccttatagc gcccagatac 840
   ggatacatca ttgagaagta cggtacagga cgaattttcc aaagtggagt gagaatggcc 900
   aaatgcaaca caaagtgtca aacatcatta ggtgggataa acaccaacaa aactttccaa 960
   aacatagaga gaaatgctct tggagattgc ccaaagtaca taaagtctgg acagctgaag 1020
   cttgcaactg ggctgagaaa tgtcccatcc gttggtgaaa gaggtttgtt tggtgcaatt 1080
   gcaggcttca tagaaggagg gtggcctggg ctaattaatg gatggtatgg tttccagcat 1140
   cagaatgaac aggggactgg cattgctgca gacaaagcct ccactcagaa agcgatagat 1200
   gaaataacaa caaaaattaa caatataata gagaagatga acggaaacta tgattcaata 1260
   agaggggaat tcaatcaagt agaaaagagg atcaacatgc tcgctgatcg agttgatgat 1320
   gcagtaactg acatatggtc gtacaatgct aaacttcttg tactgcttga aaatgggaga 1380
   acattggact tacacgacgc aaatgtcagg aacttacacg atcaggtcaa gagaatattg 1440
   aaaagtaatg ctattgatga aggagatggt tgcttcaatc ttcttcacaa atgtaatgac 1500
   tcatgcatgg aaactattag aaatgggacc tacaatcatg aagattacag ggaagaatca 1560
   caactgaaaa ggcaggaaat tgagggaata aaattgaagt ctgaagacaa tgtgtataaa 1620
   gtactgtcga tttatagctg cattgcaagc agtattgtgc tggtaggtct catacttgcg 1680
   ttcataatgt gggcatgcag caatggaaat tgccggttta atgtttgtat atagtcggaa 1740
   aaaataccct tgtttctact 1760
<210> 26
   <211> 1882
   <212> DNA
   <213> Influenza virus
<400> 26
   agcagaagcg ttgcattttc taatatccac aaaatgaagg caataattgt actactcatg      60
   gtagtaacat ccaatgcaga tcgaatctgc actgggataa catcgtcaaa ctcacctcat     120
   gtggttaaaa ctgccactca aggggaagtc aatgtgactg gtgtgatacc actaacaaca     180
   acacctacca aatctcattt tgcaaatctc aaaggaacac agaccagagg aaaactatgc     240
   ccaaactgtt ttaactgcac agatctggac gtggccctag gcagaccaaa atgcatgggg     300
   aacacaccct ccgcaaaagt ctcaatactc catgaagtca aacctgctac atctggatgc     360
   tttcctataa tgcacgacag aacaaaaatc agacaactac ctaatcttct cagaggatat     420
   gaaaacatca ggttatcaac cagtaatgtt atcaatacag agacggcacc aggaggaccc 480
   tacaaggtgg ggacctcagg atcttgccct aacgttgcta atgggaacgg cttcttcaac 540
   acaatggctt gggttatccc aaaagacaac aacaagacag caataaatcc agtaacagta 600
   gaagtaccat acatttgttc agaaggggaa gaccaaatta ctgtttgggg gttccactct 660
   gatgacaaaa cccaaatgga aagactctat ggagactcaa atcctcaaaa gttcacctca 720
   tctgccaatg gagtaaccac acattatgtt tctcagattg gtggcttccc aaatcaaaca 780
   gaagacgaag ggctaaaaca aagcggcaga attgttgttg attacatggt acaaaaacct 840
   ggaaaaacag gaacaattgt ttatcaaaga ggcattttat tgcctcaaaa agtgtggtgc 900
   gcaagtggca ggagcaaggt aataaaaggg tccttgcctt taattggtga agcagattgc 960
   ctccacgaaa agtacggtgg attaaataaa agcaagcctt actacacagg agagcatgca 1020
   aaggccatag gaaattgccc aatatgggtg aaaacaccct tgaagctggc caatggaacc 1080
   aaatatagac cgcctgcaaa actattaaag gaaagaggtt tcttcggagc tattgctggt 1140
   ttcttggaag gaggatggga aggaatgatt gcaggttggc acggatacac atctcatgga 1200
   gcacatggag tggcagtggc agcagacctt aagagtacac aagaagctat aaacaagata 1260
   acaaaaaatc tcaactattt aagtgagcta gaagtaaaaa accttcaaag actaagcgga 1320
   gcaatgaatg agcttcacga cgaaatactc gagctagacg aaaaagtgga tgatctaaga 1380
   gctgatacaa taagctcaca aatagagctt gcagtcttgc tttccaacga agggataata 1440
   aacagtgaag atgagcatct cttggcactt gaaagaaaac tgaagaaaat gcttggcccc 1500
   tctgctgtag aaatagggaa tgggtgcttt gaaaccaaac acaaatgcaa ccagacttgc 1560
   ctagacagga tagctgctgg cacctttaat gcaggagatt tttctcttcc cacttttgat 1620
   tcattaaaca ttactgctgc atctttaaat gatgatggct tggataatca tactatactg 1680
   ctctactact caactgctgc ttctagcttg gctgtaacat taatgatagc tatcttcatt 1740
   gtctacatgg tctccagaga caatgtttct tgttccatct gtctgtgagg gagattaagc 1800
   cctgtgtttt cctttactgt agtgctcatt tgcttgtcac cattacaaag aaacgttatt 1860
   gaaaaatgct cttgttacta ct 1882
<210> 27
   <211> 2073
   <212> DNA
   <213> Influenza virus
<400> 27
   agcagaagca gggggttaat aatgtttttc tcattactct tggtgttggg cctcacagag 60
   gctgaaaaaa taaagatatg ccttcaaaag caagtgaaca gtagcttcag cctacacaat 120
   ggcttcggag gaaatttgta tgccacagaa gaaaaaagaa tgtttgagct tgttaagccc 180
   aaagctggag cctctgtctt gaatcaaagt acatggattg gctttggaga ttcaaggact 240
   gacaaaagca attcagcttt tcctaggtct gctgatgttt cagcaaaaac tgctgataag 300
   tttcgttttt tgtctggtgg atccttaatg ttgagtatgt ttggcccacc tgggaaggta 360
   gactaccttt accaaggatg tggaaaacat aaagtttttt atgaaggagt taactggagt 420
   ccacatgctg ctataaattg ttacagaaaa aattggactg atatcaaact gaatttccag 480
   aaaaacattt atgaattggc ttcacaatca cattgcatga gcttggtgaa tgccttggac 540
   aaaactattc ctttacaagt gactgctggg actgcaggaa attgcaacaa cagcttctta 600
   aaaaatccag cattgtacac acaagaagtc aagccttcag aaaacaaatg tgggaaagaa 660
   aatcttgctt tcttcacact tccaacccaa tttggaacct atgagtgcaa actgcatctt 720
   gtggcttctt gctatttcat ctatgatagt aaagaagtgt acaataaaag aggatgtgac 780
   aactactttc aagtgatcta tgattcattt ggaaaagtcg ttggaggact agataacagg 840
   gtatcacctt acacagggaa ttctggagac accccaacaa tgcaatgtga catgctccag 900
   ctgaaacctg gaagatattc agtaagaagc tctccaagat tccttttaat gcctgaaaga 960
   agttattgct ttgacatgaa agaaaaagga ccagtcactg ctgtccaatc catttgggga 1020
   aaaggcagag aatctgacta tgcagtggat caagcttgct tgagcactcc agggtgcatg 1080
   ttgatccaaa agcaaaagcc atacattgga gaagctgatg atcaccatgg agatcaagaa 1140
   atgagggagt tgctgtcagg actggactat gaagctagat gcatatcaca atcagggtgg 1200
   gtgaatgaaa ccagtccttt tacggagaaa tacctccttc ctcccaaatt tggaagatgc 1260
   cctttggctg caaaggaaga atccattcca aaaatcccag atggccttct aattcccacc 1320
   agtggaaccg ataccactgt aaccaaacct aagagcagaa tttttggaat cgatgacctc 1380
   attattggtg tgctctttgt tgcaatcgtt gaaacaggaa ttggaggcta tctgcttgga 1440
   agtagaaaag aatcaggagg aggtgtgaca aaagaatcag ctgaaaaagg gtttgagaaa 1500
   attggaaatg acatacaaat tttaaaatct tctataaata tcgcaataga aaaactaaat 1560
   gacagaattt ctcatgatga gcaagccatc agagatctaa ctttagaaat tgaaaatgca 1620
   agatctgaag ctttattggg agaattggga ataataagag ccttattggt aggaaatata 1680
   agcataggat tacaggaatc tttatgggaa ctagcttcag aaataacaaa tagagcagga 1740
   gatctagcag ttgaagtctc cccaggttgc tggataattg acaataacat ttgtgatcaa 1800
   agctgtcaaa attttatttt caagttcaac gaaactgcac ctgttccaac cattccccct 1860
   cttgacacaa aaattgatct gcaatcagat cctttttact ggggaagcag cttgggctta 1920
   gcaataactg ctactatttc attggcagct ttggtgatct ctgggatcgc catctgcaga 1980
   actaaatgat tgagacaatt ttgaaaaatg gataatgtgt tggtcaatat tttgtacagt 2040
   tttataaaaa acaaaaatcc ccttgctact gct 2073
<210> 28
   <211> 1670
   <212> DNA
   <213> Artificial sequence
<220>
   <223> sequence encoding HA0 of H1 (A/New Caledonia/20/99 (H1N1)
<400> 28
   agatcttcgc tgacacaata tgtataggct accatgccaa caactcaacc gacactgttg 60
   acacagtact tgagaagaat gtgacagtga cacactctgt caacctactt gaggacagtc 120
   acaatggaaa actatgtcta ctaaaaggaa tagccccact acaattgggt aattgcagcg 180
   ttgccggatg gatcttagga aacccagaat gcgaattact gatttccaag gaatcatggt 240
   cctacattgt agaaacacca aatcctgaga atggaacatg ttacccaggg tatttcgccg 300
   actatgagga actgagggag caattgagtt cagtatcttc atttgagaga ttcgaaatat 360
   tccccaaaga aagctcatgg cccaaccaca ccgtaaccgg agtatcagca tcatgctccc 420
   ataatgggaa aagcagtttt tacagaaatt tgctatggct gacggggaag aatggtttgt 480
   acccaaacct gagcaagtcc tatgtaaaca acaaagagaa agaagtcctt gtactatggg 540
   gtgttcatca cccgcctaac atagggaacc aaagggcact ctatcataca gaaaatgctt 600
   atgtctctgt agtgtcttca cattatagca gaagattcac cccagaaata gccaaaagac 660
   ccaaagtaag agatcaggaa ggaagaatca actactactg gactctgctg gaacctgggg 720
   atacaataat atttgaggca aatggaaatc taatagcgcc atggtatgct tttgcactga 780
   gtagaggctt tggatcagga atcatcacct caaatgcacc aatggatgaa tgtgatgcga 840
   agtgtcaaac acctcaggga gctataaaca gcagtcttcc tttccagaat gtacacccag 900
   tcacaatagg agagtgtcca aagtatgtca ggagtgcaaa attaaggatg gttacaggac 960
   taaggaacat cccatccatt caatccagag gtttgtttgg agccattgcc ggtttcattg 1020
   aaggggggtg gactggaatg gtagatgggt ggtatggtta tcatcatcag aatgagcaag 1080
   gatctggcta tgctgcagat caaaaaagta cacaaaatgc cattaacggg attacaaaca 1140
   aggtcaattc tgtaattgag aaaatgaaca ctcaattcac agctgtgggc aaagagttca 1200
   acaaattgga aagaaggatg gaaaacttaa ataaaaaagt tgatgatggg tttctagaca 1260
   tttggacata taatgcagaa ttgttggttc tactggaaaa tgaaaggact ttggatttcc 1320
   atgactccaa tgtgaagaat ctgtatgaga aagtaaaaag ccaattaaag aataatgcca 1380
   aagaaatagg aaacgggtgt tttgagttct atcacaagtg taacaatgaa tgcatggaga 1440
   gtgtgaaaaa tggtacctat gactatccaa aatattccga agaatcaaag ttaaacaggg 1500
   agaaaattga tggagtgaaa ttggaatcaa tgggagtata ccagattctg gcgatctact 1560
   caactgtcgc cagttccctg gttcttttgg tctccctggg ggcaatcagc ttctggatgt 1620
   gttccaatgg gtctttgcag tgtagaatat gcatctaaga gctcaggcct 1670
<210> 29
   <211> 32
   <212> DNA
   <213> Artificial sequence
<220>
   <223> primer XmaI-pPlas.c
<400> 29
   agttccccgg gctggtatat ttatatgttg tc 32
<210> 30
   <211> 46
   <212> DNA
   <213> Artificial sequence
<220>
   <223> primer SacI-ATG-pPlas.r
<400> 30
   aatagagctc cattttctct caagatgatt aattaattaa ttagtc 46
<210> 31
   <211> 46
   <212> DNA
   <213> Artificial sequence
<220>
   <223> primer SacI-PlasTer.c
<400> 31
   aatagagctc gttaaaatgc ttcttcgtct cctatttata atatgg 46
<210> 32
   <211> 48
   <212> DNA
   <213> Artificial sequence
<220>
   <223> primer EcoRI-PlasTer.r
<400> 32
   ttacgaattc tccttcctaa ttggtgtact atcatttatc aaagggga 48
<210> 33
   <211> 1711
   <212> DNA
   <213> Influenza virus
<400> 33
   atgaaagcaa aactactggt cctgttatgt acatttacag ctacatatgc agacacaata 60
   tgtataggct accatgccaa caactcaacc gacactgttg acacagtact tgagaagaat 120
   gtgacagtga cacactctgt caacctactt gaggacagtc acaatggaaa actatgtcta 180
   ctaaaaggaa tagccccact acaattgggt aattgcagcg ttgccggatg gatcttagga 240
   aacccagaat gcgaattact gatttccaag gaatcatggt cctacattgt agaaacacca 300
   aatcctgaga atggaacatg ttacccaggg tatttcgccg actatgagga actgagggag 360
   caattgagtt cagtatcttc atttgagaga ttcgaaatat tccccaaaga aagctcatgg     420
   cccaaccaca ccgtaaccgg agtatcagca tcatgctccc ataatgggaa aagcagtttt     480
   tacagaaatt tgctatggct gacggggaag aatggtttgt acccaaacct gagcaagtcc     540
   tatgtaaaca acaaagagaa agaagtcctt gtactatggg gtgttcatca cccgcctaac     600
   atagggaacc aaagggccct ctatcataca gaaaatgctt atgtctctgt agtgtcttca     660
   cattatagca gaagattcac cccagaaata gccaaaagac ccaaagtaag agatcaggaa     720
   ggaagaatca actactactg gactctgctg gaacctgggg atacaataat atttgaggca     780
   aatggaaatc taatagcgcc atggtatgct tttgcactga gtagaggctt tggatcagga     840
   atcatcacct caaatgcacc aatggatgaa tgtgatgcga agtgtcaaac acctcaggga     900
   gctataaaca gcagtcttcc tttccagaat gtacacccag tcacaatagg agagtgtcca     960
   aagtatgtca ggagtgcaaa attaaggatg gttacaggac taaggaacat cccatccatt    1020
   caatccagag gtttgtttgg agccattgcc ggtttcattg aaggggggtg gactggaatg    1080
   gtagatgggt ggtatggtta tcatcatcag aatgagcaag gatctggcta tgctgcagat    1140
   caaaaaagta cacaaaatgc cattaacggg attacaaaca aggtgaattc tgtaattgag    1200
   aaaatgaaca ctcaattcac agctgtgggc aaagaattca acaaattgga aagaaggatg    1260
   gaaaacttaa ataaaaaagt tgatgatggg tttctagaca tttggacata taatgcagaa    1320
   ttgttggttc tactggaaaa tgaaaggact ttggatttcc atgactccaa tgtgaagaat    1380
   ctgtatgaga aagtaaaaag ccaattaaag aataatgcca aagaaatagg aaacgggtgt    1440
   tttgaattct atcacaagtg taacaatgaa tgcatggaga gtgtgaaaaa tggaacttat    1500
   gactatccaa aatattccga agaatcaaag ttaaacaggg agaaaattga tggagtgaaa    1560
   ttggaatcaa tgggagtcta tcagattctg gcgatctact caactgtcgc cagttccctg    1620
   gttcttttgg tctccctggg ggcaatcagc ttctggatgt gttccaatgg gtctttgcag    1680
   tgtagaatat gcatctgaga ccagaatttc a                                   1711
<210> 34
   <211> 1781
   <212> DNA
   <213> Medicago sativa
<400> 34
   ccaaatcctt aacattcttt caacaccaac aatggcgaaa aacgttgcga ttttcggttt 60
   attgttttct cttcttctgt tggttccttc tcagatcttc gctgaggaat catcaactga 120
   cgctaaggaa tttgttctta cattggataa cactaatttc catgacactg ttaagaagca 180
   cgatttcatc gtcgttgaat tctacgcacc ttggtgtgga cactgtaaga agctagcccc 240
   agagtatgag aaggctgctt ctatcttgag cactcacgag ccaccagttg ttttggctaa 300
   agttgatgcc aatgaggagc acaacaaaga cctcgcatcg gaaaatgatg ttaagggatt 360
   cccaaccatt aagattttta ggaatggtgg aaagaacatt caagaataca aaggtccccg 420
   tgaagctgaa ggtattgttg agtatttgaa aaaacaaagt ggccctgcat ccacagaaat 480
   taaatctgct gatgatgcga ccgcttttgt tggtgacaac aaagttgtta ttgtcggagt 540
   tttccctaaa ttttctggtg aggagtacga taacttcatt gcattagcag agaagttgcg 600
   ttctgactat gactttgctc acactttgaa tgccaaacac cttccaaagg gagactcatc 660
   agtgtctggg cctgtggtta ggttatttaa gccatttgac gagctctttg ttgactcaaa 720
   ggatttcaat gtagaagctc tagagaaatt cattgaagaa tccagtaccc caattgtgac 780
   tgtcttcaac aatgagccta gcaatcaccc ttttgttgtc aaattcttta actctcccaa 840
   cgcaaaggct atgttgttca tcaactttac taccgaaggt gctgaatctt tcaaaacaaa 900
   ataccatgaa gtggctgagc aatacaaaca acagggagtt agctttcttg ttggagatgt 960
   tgagtctagt caaggtgcct tccagtattt tggactgaag gaagaacaag tacctctaat 1020
   tattattcag cataatgatg gcaagaagtt tttcaaaccc aatttggaac ttgatcaact 1080
   cccaacttgg ttgaaggcat acaaggatgg caaggttgaa ccatttgtca agtctgaacc 1140
   tattcctgaa actaacaacg agcctgttaa agtggtggtt gggcaaactc ttgaggacgt 1200
   tgttttcaag tctgggaaga atgttttgat agagttttat gctccttggt gtggtcactg 1260
   caagcagttg gctccaatct tggatgaagt tgctgtctca ttccaaagcg atgctgatgt 1320
   tgttattgca aaactggatg caactgccaa cgatatccca accgacacct ttgatgtcca 1380
   aggctatcca accttgtact tcaggtcagc aagtggaaaa ctatcacaat acgacggtgg 1440
   taggacaaag gaagacatca tagaattcat tgaaaagaac aaggataaaa ctggtgctgc 1500
   tcatcaagaa gtagaacaac caaaagctgc tgctcagcca gaagcagaac aaccaaaaga 1560
   tgagctttga aaagttccgc ttggaggata tcggcacaca gtcatctgcg ggctttacaa 1620
   ctcttttgta tctcagaatc agaagttagg aaatcttagt gccaatctat ctatttttgc 1680
   gtttcatttt atctttttgg tttactctaa tgtattactg aataatgtga gttttggcgg 1740
   agtttagtac tggaactttt gtttctgtaa aaaaaaaaaa a 1781
<210> 35
   <211> 1027
   <212> DNA
   <213> Influenza virus
<400> 35
   agcgaaagca ggtagatatt gaaagatgag tcttctaacc gaggtcgaaa cgtacgttct      60
   ctctatcatc ccgtcaggcc ccctcaaagc cgagatcgca cagagacttg aagatgtctt     120
   tgcagggaag aacaccgatc ttgaggttct catggaatgg ctaaagacaa gaccaatcct     180
   gtcacctctg actaagggga ttttaggatt tgtgttcacg ctcaccgtgc ccagtgagcg     240
   aggactgcag cgtagacgct ttgtccaaaa tgcccttaat gggaacgggg atccaaataa     300
   catggacaaa gcagttaaac tgtataggaa gctcaagagg gagataacat tccatggggc     360
   caaagaaatc tcactcagtt attctgctgg tgcacttgcc agttgtatgg gcctcatata     420
   caacaggatg ggggctgtga ccactgaagt ggcatttggc ctggtatgtg caacctgtga     480
   acagattgct gactcccagc atcggtctca taggcaaatg gtgacaacaa ccaacccact     540
   aatcagacat gagaacagaa tggttttagc cagcactaca gctaaggcta tggagcaaat     600
   ggctggatcg agtgagcaag cagcagaggc catggaggtt gctagtcagg ctaggcaaat     660
   ggtgcaagcg atgagaacca ttgggactca tcctagctcc agtgctggtc tgaaaaatga     720
   tcttcttgaa aatttgcagg cctatcagaa acgaatgggg gtgcagatgc aacggttcaa     780
   gtgatcctct cgctattgcc gcaaatatca ttgggatctt gcacttgata ttgtggattc     840
   ttgatcgtct ttttttcaaa tgcatttacc gtcgctttaa atacggactg aaaggagggc     900
   cttctacgga aggagtgcca aagtctatga gggaagaata tcgaaaggaa cagcagagtg     960
   ctgtggatgc tgacgatggt cattttgtca gcatagagct ggagtaaaaa actaccttgt    1020
   ttctact                                                              1027
<210> 36
   <211> 1788
   <212> DNA
   <213> Artificial sequence
<220>
   <223> clone 774 - nucleotide sequence of A/Brisbane/59/2007 (H1N1)
<400> 36
   cactttgtga gtctacactt tgattccctt caaacacata caaagagaag agactaatta      60
   attaattaat catcttgaga gaaaatgaaa gtaaaactac tggtcctgtt atgcacattt     120
   acagctacat atgcagacac aatatgtata ggctaccatg ctaacaactc gaccgacact     180
   gttgacacag tacttgaaaa gaatgtgaca gtgacacact ctgtcaacct gcttgagaac     240
   agtcacaatg gaaaactatg tctattaaaa ggaatagccc cactacaatt gggtaattgc     300
   agcgttgccg ggtggatctt aggaaaccca gaatgcgaat tactgatttc caaggagtca     360
   tggtcctaca ttgtagaaaa accaaatcct gagaatggaa catgttaccc agggcatttc     420
   gctgactatg aggaactgag ggagcaattg agttcagtat cttcatttga gaggttcgaa     480
   atattcccca aagaaagctc atggcccaac cacaccgtaa ccggagtgtc agcatcatgc     540
   tcccataatg gggaaagcag tttttacaga aatttgctat ggctgacggg gaagaatggt     600
   ttgtacccaa acctgagcaa gtcctatgca aacaacaaag aaaaagaagt ccttgtacta     660
   tggggtgttc atcacccgcc aaacataggt gaccaaaagg ccctctatca tacagaaaat     720
   gcttatgtct ctgtagtgtc ttcacattat agcagaaaat tcaccccaga aatagccaaa     780
   agacccaaag taagagatca agaaggaaga atcaattact actggactct gcttgaaccc     840
   ggggatacaa taatatttga ggcaaatgga aatctaatag cgccaagata tgctttcgca     900
   ctgagtagag gctttggatc aggaatcatc aactcaaatg caccaatgga taaatgtgat     960
   gcgaagtgcc aaacacctca gggagctata aacagcagtc ttcctttcca gaacgtacac    1020
   ccagtcacaa taggagagtg tccaaagtat gtcaggagtg caaaattaag gatggttaca    1080
   ggactaagga acatcccatc cattcaatcc agaggtttgt ttggagccat tgccggtttc    1140
   attgaagggg ggtggactgg aatggtagat ggttggtatg gttatcatca tcagaatgag    1200
   caaggatctg gctatgctgc agatcaaaaa agcacacaaa atgccattaa tgggattaca    1260
   aacaaggtca attctgtaat tgagaaaatg aacactcaat tcacagcagt gggcaaagag    1320
   ttcaacaaat tggaaagaag gatggaaaac ttgaataaaa aagttgatga tgggtttata    1380
   gacatttgga catataatgc agaactgttg gttctactgg aaaatgaaag gactttggat    1440
   ttccatgact ccaatgtgaa gaatctgtat gagaaagtaa aaagccagtt aaagaataat    1500
   gctaaagaaa taggaaatgg gtgttttgag ttctatcaca agtgtaacga tgaatgcatg    1560
   gagagtgtaa agaatggaac ttatgactat ccaaaatatt ccgaagaatc aaagttaaac    1620
   agggagaaaa ttgatggagt gaaattggaa tcaatgggag tctatcagat tctggcgatc    1680
   tactcaacag tcgccagttc tctggttctt ttggtctccc tgggggcaat cagcttctgg    1740
   atgtgttcca atgggtcttt acagtgtaga atatgcatct aagagctc                 1788
<210> 37
   <211> 1788
   <212> DNA
   <213> Artificial sequence
<220>
   <223> clone 775 - nucleotide sequence of A/Solomon Islands 3/2006 (H1N1)
<400> 37
   cactttgtga gtctacactt tgattccctt caaacacata caaagagaag agactaatta     60
   attaattaat catcttgaga gaaaatgaaa gtaaaactac tggtcctgtt atgcacattt    120
   acagctacat atgcagacac aatatgtata ggctaccatg ccaacaactc aaccgacact    180
   gttgacacag tacttgagaa gaatgtgaca gtgacacact ctgtcaacct gcttgaggac    240
   agtcacaatg gaaaattatg tctattaaaa ggaatagccc cactacaatt gggtaattgc    300
   agcgttgccg gatggatctt aggaaaccca gaatgcgaat tactgatttc cagggaatca    360
   tggtcctaca ttgtagaaaa accaaatcct gagaatggaa catgttaccc agggcatttc    420
   gccgactatg aggaactgag ggagcaattg agttcagtat cttcatttga gagattcgaa    480
   atattcccca aagaaagctc atggcccaac cacaccacaa ccggagtatc agcatcatgc    540
   tcccataatg gggaaagcag tttttacaaa aatttgctat ggctgacggg gaagaatggt    600
   ttgtacccaa acctgagcaa gtcctatgca aacaacaaag agaaagaagt ccttgtacta    660
   tggggtgttc atcacccgcc taacataggt gaccaaaggg ctctctatca taaagaaaat    720
   gcttatgtct ctgtagtgtc ttcacattat agcagaaaat tcaccccaga aatagccaaa 780
   agacccaaag taagagatca agaaggaaga atcaactact actggactct acttgaaccc 840
   ggggatacaa taatatttga ggcaaatgga aatctaatag cgccaagata tgctttcgca 900
   ctgagtagag gctttggatc aggaatcatc aactcaaatg caccaatgga tgaatgtgat 960
   gcgaagtgcc aaacacctca gggagctata aacagcagtc ttcctttcca gaatgtacac 1020
   cctgtcacaa taggagagtg tccaaagtat gtcaggagtg caaaattaag gatggttaca 1080
   ggactaagga acatcccatc cattcaatcc agaggtttgt ttggagccat tgccggtttc 1140
   attgaagggg ggtggactgg aatggtagat ggttggtatg gttatcatca tcagaatgag 1200
   caaggatctg gctatgctgc agatcaaaaa agcacacaaa atgccattaa tgggattaca 1260
   aacaaggtca attctgtaat tgagaaaatg aacactcaat tcacagctgt gggcaaagag 1320
   ttcaacaaat tggaaagaag gatggaaaac ttaaataaaa aagttgatga tgggtttata 1380
   gacatttgga catataatgc agaattgttg gttctactgg aaaatgaaag gactttggat 1440
   ttccatgact ccaatgtgaa gaatctgtat gagaaagtaa aaagccaatt aaagaataat 1500
   gccaaagaaa taggaaatgg gtgttttgag ttctatcata agtgtaacga tgaatgcatg 1560
   gagagtgtaa aaaatggaac ttatgactat ccaaaatatt ccgaagaatc aaagttaaac 1620
   agggagaaaa ttgatggagt gaaattggaa tcaatgggag tctatcagat tctggcgatc 1680
   tactcaacag tcgccagttc tctggttctt ttggtctccc tgggggcaat cagcttctgg 1740
   atgtgttcca atgggtcttt gcagtgtaga atatgcatct gagagctc 1788
<210> 38
   <211> 1791
   <212> DNA
   <213> Artificial sequence
<220>
   <223> clone 776 - nucleotide sequence of A/Brisbane 10/2007 (H3N2)
<400> 38
   cactttgtga gtctacactt tgattccctt caaacacata caaagagaag agactaatta 60
   attaattaat catcttgaga gaaaatgaag actatcattg ctttgagcta cattctatgt 120
   ctggttttca ctcaaaaact tcccggaaat gacaacagca cggcaacgct gtgccttggg 180
   caccatgcag taccaaacgg aacgatagtg aaaacaatca cgaatgacca aattgaagtt 240
   actaatgcta ctgagctggt tcagagttcc tcaacaggtg aaatatgcga cagtcctcat 300
   cagatccttg atggagaaaa ctgcacacta atagatgctc tattgggaga ccctcagtgt 360
   gatggcttcc aaaataagaa atgggacctt tttgttgaac gcagcaaagc ctacagcaac 420
   tgttaccctt atgatgtgcc ggattatgcc tcccttaggt cactagttgc ctcatccggc 480
   acactggagt ttaacaatga aagtttcaat tggactggag tcactcaaaa cggaacaagc 540
   tctgcttgca taaggagatc taataacagt ttctttagta gattgaattg gttgacccac 600
   ttaaaattca aatacccagc attgaacgtg actatgccaa acaatgaaaa atttgacaaa 660
   ttgtacattt ggggggttca ccacccgggt acggacaatg accaaatctt cctgtatgct 720
   caagcatcag gaagaatcac agtctctacc aaaagaagcc aacaaactgt aatcccgaat 780
   atcggatcta gacccagagt aaggaatatc cccagcagaa taagcatcta ttggacaata 840
   gtaaaaccgg gagacatact tttgattaac agcacaggga atctaattgc tcctaggggt 900
   tacttcaaaa tacgaagtgg gaaaagctca ataatgagat cagatgcacc cattggcaaa 960
   tgcaattctg aatgcatcac tccaaacgga agcattccca atgacaaacc attccaaaat 1020
   gtaaacagga tcacatacgg ggcctgtccc agatatgtta agcaaaacac tctgaaattg 1080
   gcaacaggga tgcgaaatgt accagagaaa caaactagag gcatatttgg cgcaatcgcg 1140
   ggtttcatag aaaatggttg ggagggaatg gtggatggtt ggtatggttt caggcatcaa 1200
   aattctgagg gaataggaca agcagcagat ctcaaaagca ctcaagcagc aatcgatcaa 1260
   atcaatggga agctgaatag gttgatcggg aaaaccaacg agaaattcca tcagattgaa 1320
   aaagagttct cagaagtcga agggagaatc caggaccttg agaaatatgt tgaggacacc 1380
   aaaatagatc tctggtcata caacgcggag cttcttgttg ccctggagaa ccaacataca 1440
   attgatctaa ctgactcaga aatgaacaaa ctgtttgaaa aaacaaagaa gcaactgagg 1500
   gaaaatgctg aggatatggg caatggttgt ttcaaaatat accacaaatg tgacaatgcc 1560
   tgcataggat caatcagaaa tggaacttat gaccacgatg tatacagaga tgaagcatta 1620
   aacaaccggt tccagatcaa gggcgttgag ctgaagtcag gatacaaaga ttggatacta 1680
   tggatttcct ttgccatatc atgttttttg ctttgtgttg ctttgttggg gttcatcatg 1740
   tgggcctgcc aaaaaggcaa cattaggtgc aacatttgca tttgagagct c 1791
<210> 39
   <211> 1791
   <212> DNA
   <213> Artificial sequence
<220>
   <223> clone 777 - nucleotide sequence of A/Wisconsin/67/2005 (H3N2)
<400> 39
   cactttgtga gtctacactt tgattccctt caaacacata caaagagaag agactaatta      60
   attaattaat catcttgaga gaaaatgaag actatcattg ctttgagcta cattctatgt     120
   ctggttttca ctcaaaaact tcccggaaat gacaacagca cggcaacgct gtgccttggg     180
   caccatgcag taccaaacgg aacgatagtg aaaacaatca cgaatgacca aattgaagtt     240
   actaatgcta ctgagctggt tcagagttcc tcaacaggtg gaatatgcga cagtcctcat     300
   cagatccttg atggagaaaa ctgcacacta atagatgctc tattgggaga ccctcagtgt     360
   gatggcttcc aaaataagaa atgggacctt tttgttgaac gcagcaaagc ctacagcaac     420
   tgttaccctt atgatgtgcc ggattatgcc tcccttaggt cactagttgc ctcatccggc 480
   acactggagt ttaacgatga aagtttcaat tggactggag tcactcaaaa tggaacaagc 540
   tctgcttgca aaaggagatc taataacagt ttctttagta gattgaattg gttgacccac 600
   ttaaaattca aatacccagc attgaacgtg actatgccaa acaatgaaaa atttgacaaa 660
   ttgtacattt ggggggttca ccacccgggt acggacaatg accaaatctt cctgcatgct 720
   caagcatcag gaagaatcac agtctctacc aaaagaagcc aacaaactgt aatcccgaat 780
   atcggatcta gacccagaat aaggaatatc cccagcagaa taagcatcta ttggacaata 840
   gtaaaaccgg gagacatact tttgattaac agcacaggga atctaattgc tcctaggggt 900
   tacttcaaaa tacgaagtgg gaaaagctca ataatgagat cagatgcacc cattggcaaa 960
   tgcaattctg aatgcatcac tccaaatgga agcattccca atgacaaacc atttcaaaat 1020
   gtaaacagga tcacatatgg ggcctgtccc agatatgtta agcaaaacac tctgaaattg 1080
   gcaacaggga tgcgaaatgt accagagaaa caaactagag gcatatttgg cgcaatcgcg 1140
   ggtttcatag aaaatggttg ggagggaatg gtggatggtt ggtacggttt caggcatcaa 1200
   aattctgagg gaataggaca agcagcagat ctcaaaagca ctcaagcagc aatcaatcaa 1260
   atcaatggga agctgaatag gttgatcggg aaaaccaacg agaaattcca tcagattgaa 1320
   aaagagttct cagaagtaga agggagaatc caggacctcg agaaatatgt tgaggacact 1380
   aaaatagatc tctggtcata caacgcggag cttcttgttg ccctggagaa ccaacataca 1440
   attgatctaa ctgactcaga aatgaacaaa ctgtttgaaa gaacaaagaa gcaactgagg 1500
   gaaaatgctg aggatatggg caatggttgt ttcaaaatat accacaaatg tgacaatgcc 1560
   tgcataggat caatcagaaa tggaacttat gaccatgatg tatacagaga tgaagcatta 1620
   aacaaccggt tccagatcaa aggcgttgag ctgaagtcag gatacaaaga ttggatacta 1680
   tggatttcct ttgccatatc atgttttttg ctttgtgttg ctttgttggg gttcatcatg 1740
   tgggcctgcc aaaaaggcaa cattaggtgc aacatttgca tttgagagct c 1791
<210> 40
   <211> 1848
   <212> DNA
   <213> Artificial sequence
<220>
   <223> clone 778 - nucleotide sequence of B/Malaysia/2506/2004
<400> 40
   cactttgtga gtctacactt tgattccctt caaacacata caaagagaag agactaatta 60
   attaattaat catcttgaga gaaaatgaag gcaataattg tactactcat ggtagtaaca 120
   tccaatgcag atcgaatctg cactgggata acatcgtcaa actcaccaca tgttgtcaaa 180
   actgctactc aaggggaggt caatgtgact ggtgtaatac cactgacaac aacacccacc 240
   aaatctcatt ttgcaaatct caaaggaaca gaaaccagag ggaaactatg cccaaaatgc 300
   ctcaactgca cagatctgga cgtggccttg ggcagaccaa aatgcacggg gaacataccc 360
   tcggcaagag tttcaatact ccatgaagtc agacctgtta catctgggtg ctttcctata 420
   atgcacgaca gaacaaaaat tagacagctg cctaaacttc tcagaggata cgaacatatc 480
   aggttatcaa ctcataacgt tatcaatgca gaaaatgcac caggaggacc ctacaaaatt 540
   ggaacctcag ggtcttgccc taacgttacc aatggaaacg gatttttcgc aacaatggct 600
   tgggccgtcc caaaaaacga caacaacaaa acagcaacaa attcattaac aatagaagta 660
   ccatacattt gtacagaagg agaagaccaa attaccgttt gggggttcca ctctgataac 720
   gaaacccaaa tggcaaagct ctatggggac tcaaagcccc agaagttcac ctcatctgcc 780
   aacggagtga ccacacatta cgtttcacag attggtggct tcccaaatca aacagaagac 840
   ggaggactac cacaaagcgg tagaattgtt gttgattaca tggtgcaaaa atctgggaaa 900
   acaggaacaa ttacctatca aagaggtatt ttattgcctc aaaaagtgtg gtgcgcaagt 960
   ggcaggagca aggtaataaa aggatcgttg cctttaattg gagaagcaga ttgcctccac 1020
   gaaaaatacg gtggattaaa caaaagcaag ccttactaca caggggaaca tgcaaaggcc 1080
   ataggaaatt gcccaatatg ggtgaaaaca cccttgaagc tggccaatgg aaccaaatat 1140
   agacctcctg caaaactatt aaaggaaagg ggtttcttcg gagctattgc tggtttctta 1200
   gaaggaggat gggaaggaat gattgcaggt tggcacggat acacatccca tggggcacat 1260
   ggagtagcgg tggcagcaga ccttaagagc actcaagagg ccataaacaa gataacaaaa 1320
   aatctcaact ctttgagtga gctggaagta aagaatcttc aaagactaag cggtgccatg 1380
   gatgaactcc acaacgaaat actagaacta gacgagaaag tggatgatct cagagctgat 1440
   acaataagct cacaaataga actcgcagtc ctgctttcca atgaaggaat aataaacagt 1500
   gaagatgagc atctcttggc gcttgaaaga aagctgaaga aaatgctggg cccctctgct 1560
   gtagagatag ggaatggatg ctttgaaacc aaacacaagt gcaaccagac ctgtctcgac 1620
   agaatagctg ctggtacctt tgatgcagga gaattttctc tccccacttt tgattcactg 1680
   aatattactg ctgcatcttt aaatgacgat ggattggata atcatactat actgctttac 1740
   tactcaactg ctgcctccag tttggctgta acattgatga tagctatctt tgttgtttat 1800
   atggtctcca gagacaatgt ttcttgctcc atctgtctat aagagctc 1848
<210> 41
   <211> 1845
   <212> DNA
   <213> Artificial sequence
<220>
   <223> clone 779 - nucleotide sequence of B/Florida/4/2006
<400> 41
   cactttgtga gtctacactt tgattccctt caaacacata caaagagaag agactaatta 60
   attaattaat catcttgaga gaaaatgaag gcaataattg tactactcat ggtagtaaca 120
   tccaatgcag atcgaatctg cactggaata acatcttcaa actcacctca tgtggtcaaa 180
   acagccactc aaggggaggt caatgtgact ggtgtgatac cactaacaac aacaccaaca 240
   aaatcttatt ttgcaaatct caaaggaaca aggaccagag ggaaactatg cccagactgt 300
   ctcaactgca cagatctgga tgtggctttg ggcagaccaa tgtgtgtggg gaccacacct 360
   tcggcgaagg cttcaatact ccacgaagtc aaacctgtta catccgggtg ctttcctata 420
   atgcacgaca gaacaaaaat caggcaacta cccaatcttc tcagaggata tgaaaatatc 480
   aggctatcaa cccaaaacgt catcgatgcg gaaaaggcac caggaggacc ctacagactt 540
   ggaacctcag gatcttgccc taacgctacc agtaagagcg gatttttcgc aacaatggct 600
   tgggctgtcc caaaggacaa caacaaaaat gcaacgaacc cactaacagt agaagtacca 660
   tacatttgta cagaagggga agaccaaatc actgtttggg ggttccattc agataacaaa 720
   acccaaatga agaacctcta tggagactca aatcctcaaa agttcacctc atctgctaat 780
   ggagtaacca cacactatgt ttctcagatt ggcagcttcc cagatcaaac agaagacgga 840
   ggactaccac aaagcggcag gattgttgtt gattacatga tgcaaaaacc tgggaaaaca 900
   ggaacaattg tctaccaaag aggtgttttg ttgcctcaaa aggtgtggtg cgcgagtggc 960
   aggagcaaag taataaaagg gtccttgcct ttaattggtg aagcagattg ccttcatgaa 1020
   aaatacggtg gattaaacaa aagcaagcct tactacacag gagaacatgc aaaagccata 1080
   ggaaattgcc caatatgggt gaaaacacct ttgaagctcg ccaatggaac caaatataga 1140
   cctcctgcaa aactattaaa ggaaaggggt ttcttcggag ctattgctgg tttcctagaa 1200
   ggaggatggg aaggaatgat tgcaggctgg cacggataca catctcacgg agcacatgga 1260
   gtggcagtgg cggcggacct taagagtacg caagaagcta taaacaagat aacaaaaaat 1320
   ctcaattctt tgagtgagct agaagtaaag aatcttcaaa gactaagtgg tgccatggat 1380
   gaactccaca acgaaatact cgagctggat gagaaagtgg atgatctcag agctgacact 1440
   ataagctcgc aaatagaact tgcagtcttg ctttccaacg aaggaataat aaacagtgaa 1500
   gatgagcatc tattggcact tgagagaaaa ctaaagaaaa tgctgggtcc ctctgctgta 1560
   gagataggaa atggatgctt cgaaaccaaa cacaagtgca accagacctg cttagacagg 1620
   atagctgctg gcacctttaa tgcaggagaa ttttctctcc ccacttttga ttcactgaac 1680
   attactgctg catctttaaa tgatgatgga ttggataacc atactatact gctctattac 1740
   tcaactgctg cttctagttt ggctgtaaca ttgatgctag ctatttttat tgtttatatg 1800
   gtctccagag acaacgtttc atgctccatc tgtctataag agctc 1845
<210> 42
   <211> 1779
   <212> DNA
   <213> Artificial sequence
<220>
   <223> clone 780 - nucleotide sequence of A/Singapore/1/57 (H2N2)
<400> 42
   cactttgtga gtctacactt tgattccctt caaacacata caaagagaag agactaatta 60
   attaattaat catcttgaga gaaaatggcc atcatttatc taattctcct gttcacagca 120
   gtgagagggg accaaatatg cattggatac catgccaata attccacaga gaaggtcgac 180
   acaattctag agcggaacgt cactgtgact catgccaagg acattcttga gaagacccat 240
   aacggaaagt tatgcaaact aaacggaatc cctccacttg aactagggga ctgtagcatt 300
   gccggatggc tccttggaaa tccagaatgt gataggcttc taagtgtgcc agaatggtcc 360
   tatataatgg agaaagaaaa cccgagagac ggtttgtgtt atccaggcag cttcaatgat 420
   tatgaagaat tgaaacatct cctcagcagc gtgaaacatt tcgagaaagt aaagattctg 480
   cccaaagata gatggacaca gcatacaaca actggaggtt cacgggcctg cgcggtgtct 540
   ggtaatccat cattcttcag gaacatggtc tggctgacaa agaaagaatc aaattatccg 600
   gttgccaaag gatcgtacaa caatacaagc ggagaacaaa tgctaataat ttggggggtg 660
   caccatccca atgatgagac agaacaaaga acattgtacc agaatgtggg aacctatgtt 720
   tccgtaggca catcaacatt gaacaaaagg tcaaccccag acatagcaac aaggcctaaa 780
   gtgaatggac taggaagtag aatggagttc tcttggaccc tattggatat gtgggacacc 840
   ataaattttg agagtactgg taatctaatt gcaccagagt atggattcaa aatatcgaaa 900
   agaggtagtt cagggatcat gaaaacagaa ggaacacttg agaactgtga gaccaaatgc 960
   caaactcctt tgggagcaat aaatacaaca ttgccttttc acaatgtcca cccactgaca 1020
   ataggtgagt gccccaaata tgtaaaatcg gagaagttgg tcttagcaac aggactaagg 1080
   aatgttcccc agattgaatc aagaggattg tttggggcaa tagctggttt tatagaagga 1140
   ggatggcaag gaatggttga tggttggtat ggataccatc acagcaatga ccagggatca 1200
   gggtatgcag cagacaaaga atccactcaa aaggcatttg atggaatcac caacaaggta 1260
   aattctgtga ttgaaaagat gaacacccaa tttgaagctg ttgggaaaga gttcagtaac 1320
   ttagagagaa gactggagaa cttgaacaaa aagatggaag acgggtttct agatgtgtgg 1380
   acatacaatg ctgagcttct agttctgatg gaaaatgaga ggacacttga ctttcatgat 1440
   tctaatgtca agaatctgta tgataaagtc agaatgcagc tgagagacaa cgtcaaagaa 1500
   ctaggaaatg gatgttttga attttatcac aaatgtgatg atgaatgcat gaatagtgtg 1560
   aaaaacggga cgtatgatta tcccaagtat gaagaagagt ctaaactaaa tagaaatgaa 1620
   atcaaagggg taaaattgag cagcatgggg gtttatcaaa tccttgccat ttatgctaca 1680
   gtagcaggtt ctctgtcact ggcaatcatg atggctggga tctctttctg gatgtgctcc 1740
   aacgggtctc tgcagtgcag gatctgcata tgagagctc 1779
<210> 43
   <211> 1794
   <212> DNA
   <213> Artificial sequence
<220>
   <223> clone 781 - nucleotide sequence of A/Anhui/1/2005 (H5N1)
<400> 43
   cactttgtga gtctacactt tgattccctt caaacacata caaagagaag agactaatta      60
   attaattaat catcttgaga gaaaatggag aaaatagtgc ttcttcttgc aatagtcagc     120
   cttgttaaaa gtgatcagat ttgcattggt taccatgcaa acaactcgac agagcaggtt     180
   gacacaataa tggaaaagaa cgttactgtt acacatgccc aagacatact ggaaaagaca     240
   cacaacggga agctctgcga tctagatgga gtgaagcctc tgattttaag agattgtagt     300
   gtagctggat ggctcctcgg aaacccaatg tgtgacgagt tcatcaatgt gccggaatgg     360
   tcttacatag tggagaaggc caacccagcc aatgacctct gttacccagg gaatttcaac     420
   gactatgaag aactgaaaca cctattgagc agaataaacc attttgagaa aattcagatc     480
   atccccaaaa gttcttggtc cgatcatgaa gcctcatcag gggtcagctc agcatgtcca     540
   taccagggaa cgccctcctt tttcagaaat gtggtatggc ttatcaaaaa gaacaataca     600
   tacccaacaa taaagagaag ctacaataat accaaccagg aagatctttt gatactgtgg     660
   gggattcatc attctaatga tgcggcagag cagacaaagc tctatcaaaa cccaaccacc     720
   tatatttccg ttgggacatc aacactaaac cagagattgg taccaaaaat agctactaga     780
   tccaaagtaa acgggcaaag tggaaggatg gatttcttct ggacaatttt aaaaccgaat     840
   gatgcaatca acttcgagag taatggaaat ttcattgctc cagaatatgc atacaaaatt     900
   gtcaagaaag gggactcagc aattgttaaa agtgaagtgg aatatggtaa ctgcaataca     960
   aagtgtcaaa ctccaatagg ggcgataaac tctagtatgc cattccacaa catacaccct    1020
   ctcaccatcg ggaatgcccc caaatatgtg aaatcaaaca aattagtcct tgcgactggg    1080
   ctcagaaata gtcctctaag agaaagaaga agaaaaagag gactatttgg agctatagca    1140
   gggtttatag agggaggatg gcagggaatg gtagatggtt ggtatgggta ccaccatagc    1200
   aatgagcagg ggagtgggta cgctgcagac aaagaatcca ctcaaaaggc aatagatgga    1260
   gtcaccaata aggtcaactc gatcattgac aaaatgaaca ctcagtttga ggccgttgga    1320
   agggaattta ataacttaga aaggagaata gagaatttaa acaagaaaat ggaagacgga    1380
   ttcctagatg tctggactta taatgctgaa cttctggttc tcatggaaaa tgagagaact    1440
   ctagacttcc atgattcaaa tgtcaagaac ctttacgaca aggtccgact acagcttagg    1500
   gataatgcaa aggagctggg taacggttgt ttcgagttct atcacaaatg tgataatgaa    1560
   tgtatggaaa gtgtaagaaa cggaacgtat gactacccgc agtattcaga agaagcaaga    1620
   ttaaaaagag aggaaataag tggagtaaaa ttggaatcaa taggaactta ccaaatactg    1680
   tcaatttatt caacagttgc gagttctcta gcactggcaa tcatggtggc tggtctatct    1740
   ttgtggatgt gctccaatgg gtcgttacaa tgcagaattt gcatttaaga gctc          1794
<210> 44
   <211> 1797
   <212> DNA
   <213> Artificial sequence
<220>
   <223> clone 782 - nucleotide sequence of A/Vietnam/1194/2004 (H5N1)
<400> 44
   cactttgtga gtctacactt tgattccctt caaacacata caaagagaag agactaatta      60
   attaattaat catcttgaga gaaaatggag aaaatagtgc ttctttttgc aatagtcagt     120
   cttgttaaaa gtgatcagat ttgcattggt taccatgcaa acaactcgac agagcaggtt     180
   gacacaataa tggaaaagaa cgttactgtt acacatgccc aagacatact ggaaaagaca     240
   cacaatggga agctctgcga tctagatgga gtgaagcctc taattttgag agattgtagt     300
   gtagctggat ggctcctcgg aaacccaatg tgtgacgagt tcatcaatgt gccggaatgg     360
   tcttacatag tggagaaggc caatccagtc aatgacctct gttacccagg ggatttcaat     420
   gactatgaag aattgaaaca cctattgagc agaataaacc attttgagaa aattcagatc     480
   atccccaaaa gttcttggtc cagtcatgaa gcctcattgg gggtcagctc agcatgtcca     540
   taccagggaa agtcctcctt tttcagaaat gtggtatggc ttatcaaaaa gaacagtaca     600
   tacccaacaa taaagaggag ctacaataat accaaccaag aagatctttt ggtactgtgg     660
   gggattcacc atcctaatga tgcggcagag cagacaaagc tctatcaaaa cccaaccacc     720
   tatatttccg ttgggacatc tacactaaac cagagattgg taccaagaat agctactaga     780
   tccaaagtaa acgggcaaag tggaaggatg gagttcttct ggacaatttt aaaaccgaat     840
   gatgcaatca acttcgagag taatggaaat ttcattgctc cagaatatgc atacaaaatt     900
   gtcaagaaag gggactcaac aattatgaaa agtgaattgg aatatggtaa ctgcaatacc     960
   aagtgtcaaa ctccaatggg ggcgataaac tctagcatgc cattccacaa tatacaccct    1020
   ctcaccatcg gggaatgccc caaatatgtg aaatcaaaca gattagtcct tgcgactggg    1080
   ctcagaaata gccctcaaag agagagaaga agaaaaaaga gaggattatt tggagctata    1140
   gcaggtttta tagagggagg atggcaggga atggtagatg gttggtatgg gtaccaccat    1200
   agcaacgagc aggggagtgg gtacgctgca gacaaagaat ccactcaaaa ggcaatagat    1260
   ggagtcacca ataaggtcaa ctcgattatt gacaaaatga acactcagtt tgaggccgtt    1320
   ggaagggaat ttaacaactt agaaaggaga atagagaatt taaacaagaa gatggaagac    1380
   gggttcctag atgtctggac ttataatgct gaacttctag ttctcatgga aaacgagaga    1440
   actctagact ttcatgactc aaatgtcaag aacctttacg acaaggtccg actacagctt    1500
   agggataatg caaaggagct gggtaacggt tgtttcgagt tctatcataa atgtgataat    1560
   gaatgtatgg aaagtgtaag aaacggaacg tatgactacc cgcagtattc agaagaagca    1620
   agactaaaaa gagaggaaat aagtggagta aaattggaat caataggaat ttaccaaata    1680
   ttgtcaattt attctacagt ggccagctcc ctagcactgg caatcatggt agctggtcta    1740
   tccttatgga tgtgctccaa tgggtcgtta caatgcagaa tttgcattta agagctc       1797
<210> 45
   <211> 1791
   <212> DNA
   <213> Artificial sequence
<220>
   <223> clone 783 - nucleotide sequence of A/Teal/HongKong/W312/97 (H6N1)
<400> 45
   cactttgtga gtctacactt tgattccctt caaacacata caaagagaag agactaatta 60
   attaattaat catcttgaga gaaaatgatt gcaatcattg taatagcaat actggcagca 120
   gccggaaagt cagacaagat ctgcattggg tatcatgcca acaattcaac aacacaggta 180
   gatacgatac ttgagaagaa tgtgactgtc acacactcaa ttgaattgct ggaaaatcag 240
   aaggaagaaa gattctgcaa gatattgaac aaggcccctc tcgacttaag ggaatgtacc 300
   atagagggtt ggatcttggg gaatccccaa tgcgacctat tgcttggtga tcaaagctgg 360
   tcatacattg tggaaagacc tactgctcaa aacgggatct gctacccagg aaccttaaat 420
   gaggtagaag aactgagggc acttattgga tcaggagaaa gggtagagag atttgagatg 480
   tttccccaaa gcacctggca aggagttgac accaacagtg gaacaacaag atcctgccct 540
   tattctactg gtgcgtcttt ctacagaaac ctcctatgga taataaaaac caagacagca 600
   gaatatccag taattaaggg aatttacaac aacactggaa cccagccaat cctctatttc 660
   tggggtgtgc atcatcctcc taacaccgac gagcaagata ctctgtatgg ctctggtgat 720
   cgatacgtta gaatgggaac tgaaagcatg aattttgcca agagtccgga aattgcggca 780
   aggcctgctg tgaatggaca aagaggcaga attgattatt attggtcggt tttaaaacca 840
   ggggaaacct tgaatgtgga atctaatgga aatctaatcg ccccttggta tgcatacaaa 900
   tttgtcaaca caaatagtaa aggagccgtc ttcaggtcag atttaccaat cgagaactgc 960
   gatgccacat gccagactat tgcaggggtt ctaaggacca ataaaacatt tcagaatgtg 1020
   agtcccctgt ggataggaga atgtcccaaa tacgtgaaaa gtgaaagtct gaggcttgca 1080
   actggactaa gaaatgttcc acagattgaa actagaggac tcttcggagc tattgcaggg 1140
   tttattgaag gaggatggac tgggatgata gatgggtggt atggctatca ccatgaaaat 1200
   tctcaagggt caggatatgc agcagacaga gaaagcactc aaaaggctgt aaacagaatt 1260
   acaaataagg tcaattccat catcaacaaa atgaacacac aatttgaagc tgtcgatcac 1320
   gaattttcaa atctggagag gagaattgac aatctgaaca aaagaatgca agatggattt 1380
   ctggatgttt ggacatacaa tgctgaactg ttggttcttc ttgaaaacga aagaacacta 1440
   gacatgcatg acgcaaatgt gaagaaccta catgaaaagg tcaaatcaca actaagggac 1500
   aatgctacga tcttagggaa tggttgcttt gaattttggc ataagtgtga caatgaatgc 1560
   atagagtctg tcaaaaatgg tacatatgac tatcccaaat accagactga aagcaaatta 1620
   aacaggctaa aaatagaatc agtaaagcta gagaaccttg gtgtgtatca aattcttgcc 1680
   atttatagta cggtatcgag cagcctagtg ttggtagggc tgatcatggc aatgggtctt 1740
   tggatgtgtt caaatggttc aatgcagtgc aggatatgta tataagagct c 1791
<210> 46
   <211> 1803
   <212> DNA
   <213> Artificial sequence
<220>
   <223> clone 784 - nucleotide sequence of A/Equine/Prague/56 (H7N7)
<400> 46
   cactttgtga gtctacactt tgattccctt caaacacata caaagagaag agactaatta     60
   attaattaat catcttgaga gaaaatgaac actcaaattc taatattagc cacttcggca    120
   ttcttctatg tacgtgcaga taaaatctgc ctaggacatc atgctgtgtc taatggaacc    180
   aaagtagaca cccttactga aaaaggaata gaagttgtca atgcaacaga aacagttgaa    240
   caaacaaaca tccctaagat ctgctcaaaa ggaaaacaga ctgttgacct tggtcaatgt    300
   ggattactag ggaccgttat tggtcctccc caatgtgacc aatttcttga gttctctgct    360
   aatttaatag ttgaaagaag ggaaggtaat gacatttgtt atccaggcaa atttgacaat    420
   gaagaaacat tgagaaaaat actcagaaaa tccggaggaa ttaaaaagga gaatatggga    480
   ttcacatata ccggagtgag aaccaatgga gagactagcg catgtagaag gtcaagatct    540
   tccttttatg cagagatgaa atggcttcta tccagcacag acaatgggac atttccacaa    600
   atgacaaagt cctacaagaa cactaagaag gtaccagctc tgataatctg gggaatccac    660
   cactcaggat caactactga acagactaga ttatatggaa gtgggaataa attgataaca    720
   gtttggagtt ccaaatacca acaatctttt gtcccaaatc ctggaccaag accgcaaatg    780
   aatggtcaat caggaagaat tgactttcac tggctgatgc tagatcccaa tgatactgtc    840
   actttcagtt ttaatggggc ctttatagca cctgaccgcg ccagttttct aagaggtaaa    900
   tctctaggaa tccaaagtga tgcacaactt gacaataatt gtgaaggtga atgctatcat    960
   attggaggta ctataattag caacttgccc tttcaaaaca ttaatagtag ggcaatcgga   1020
   aaatgcccca gatacgtgaa gcagaagagc ttaatgctag caacaggaat gaaaaatgtt   1080
   cctgaagctc ctgcacataa acaactaact catcacatgc gcaaaaaaag aggtttattt   1140
   ggtgcaatag caggattcat tgaaaatggg tgggaaggat taatagacgg atggtatgga   1200
   tataagcatc agaatgcaca aggagaaggg actgctgcag actacaaaag tacacaatct 1260
   gctatcaacc aaataaccgg aaaattgaac agactaatag aaaaaaccaa ccagcaattc 1320
   gaactaatag ataatgagtt caatgaaata gaaaaacaaa ttggcaatgt tattaactgg 1380
   actagagatt ctatcatcga agtatggtca tataatgcag agttcctcgt agcagtggag 1440
   aatcaacaca ctattgattt aactgactca gaaatgaaca aactatatga aaaggtaaga 1500
   agacaactga gagaaaatgc tgaggaagat ggtaatggct gttttgaaat attccaccaa 1560
   tgtgacaatg attgcatggc cagcattaga aacaacacat atgaccataa aaaatacaga 1620
   aaagaggcaa tacaaaacag aatccagatt gacgcagtaa agttgagcag tggttacaaa 1680
   gatataatac tttggtttag cttcggggca tcatgtttct tatttcttgc cattgcaatg 1740
   ggtcttgttt tcatatgtat aaaaaatgga aacatgcggt gcactatttg tatataagag 1800
   ctc 1803
<210> 47
   <211> 1773
   <212> DNA
   <213> Artificial sequence
<220>
   <223> clone 785 - nucleotide sequence of A/HongKong/1073/99 (H9N2)
<400> 47
   cactttgtga gtctacactt tgattccctt caaacacata caaagagaag agactaatta      60
   attaattaat catcttgaga gaaaatggaa acaatatcac taataactat actactagta     120
   gtaacagcaa gcaatgcaga taaaatctgc atcggccacc agtcaacaaa ctccacagaa     180
   actgtggaca cgctaacaga aaccaatgtt cctgtgacac atgccaaaga attgctccac     240
   acagagcata atggaatgct gtgtgcaaca agcctgggac atcccctcat tctagacaca     300
   tgcactattg aaggactagt ctatggcaac ccttcttgtg acctgctgtt gggaggaaga     360
   gaatggtcct acatcgtcga aagatcatca gctgtaaatg gaacgtgtta ccctgggaat     420
   gtagaaaacc tagaggaact caggacactt tttagttccg ctagttccta ccaaagaatc     480
   caaatcttcc cagacacaac ctggaatgtg acttacactg gaacaagcag agcatgttca     540
   ggttcattct acaggagtat gagatggctg actcaaaaga gcggttttta ccctgttcaa     600
   gacgcccaat acacaaataa caggggaaag agcattcttt tcgtgtgggg catacatcac     660
   ccacccacct ataccgagca aacaaatttg tacataagaa acgacacaac aacaagcgtg     720
   acaacagaag atttgaatag gaccttcaaa ccagtgatag ggccaaggcc ccttgtcaat     780
   ggtctgcagg gaagaattga ttattattgg tcggtactaa aaccaggcca aacattgcga     840
   gtacgatcca atgggaatct aattgctcca tggtatggac acgttctttc aggagggagc     900
   catggaagaa tcctgaagac tgatttaaaa ggtggtaatt gtgtagtgca atgtcagact     960
   gaaaaaggtg gcttaaacag tacattgcca ttccacaata tcagtaaata tgcatttgga    1020
   acctgcccca aatatgtaag agttaatagt ctcaaactgg cagtcggtct gaggaacgtg    1080
   cctgctagat caagtagagg actatttgga gccatagctg gattcataga aggaggttgg    1140
   ccaggactag tcgctggctg gtatggtttc cagcattcaa atgatcaagg ggttggtatg    1200
   gctgcagata gggattcaac tcaaaaggca attgataaaa taacatccaa ggtgaataat    1260
   atagtcgaca agatgaacaa gcaatatgaa ataattgatc atgaatttag tgaggttgaa    1320
   actagactca atatgatcaa taataagatt gatgaccaaa tacaagacgt atgggcatat    1380
   aatgcagaat tgctagtact acttgaaaat caaaaaacac tcgatgagca tgatgcgaac    1440
   gtgaacaatc tatataacaa ggtgaagagg gcactgggct ccaatgctat ggaagatggg    1500
   aaaggctgtt tcgagctata ccataaatgt gatgatcagt gcatggaaac aattcggaac    1560
   gggacctata ataggagaaa gtatagagag gaatcaagac tagaaaggca gaaaatagag    1620
   ggggttaagc tggaatctga gggaacttac aaaatcctca ccatttattc gactgtcgcc    1680
   tcatctcttg tgcttgcaat ggggtttgct gccttcctgt tctgggccat gtccaatgga    1740
   tcttgcagat gcaacatttg tatataagag ctc                                 1773
<210> 48
   <211> 565
   <212> PRT
   <213> Artificial sequence
<220>
   <223> clone 774 (A/Brisbane/59/2007 (H1N1)
<400> 48
   Met Lys Val Lys Leu Leu Val Leu Leu Cys Thr Phe Thr Ala Thr Tyr
   1               5                   10                  15
   Ala Asp Thr Ile Cys Ile Gly Tyr His Ala Asn Asn Ser Thr Asp Thr
               20                  25                  30
   Val Asp Thr Val Leu Glu Lys Asn Val Thr Val Thr His Ser Val Asn
           35                  40                  45
   Leu Leu Glu Asn Ser His Asn Gly Lys Leu Cys Leu Leu Lys Gly Ile
       50                  55                  60
   Ala Pro Leu Gln Leu Gly Asn Cys Ser Val Ala Gly Trp Ile Leu Gly
   65                  70                  75                  80
   Asn Pro Glu Cys Glu Leu Leu Ile Ser Lys Glu Ser Trp Ser Tyr Ile
                   85                  90                  95
   Val Glu Lys Pro Asn Pro Glu Asn Gly Thr Cys Tyr Pro Gly His Phe
               100                 105                 110
   Ala Asp Tyr Glu Glu Leu Arg Glu Gln Leu Ser Ser Val Ser Ser Phe
   115 120 125
   Glu Arg Phe Glu Ile Phe Pro Lys Glu Ser Ser Trp Pro Asn His Thr
   130 135 140
   Val Thr Gly Val Ser Ala Ser Cys Ser His Asn Gly Glu Ser Ser Phe
   145 150 155 160
   Tyr Arg Asn Leu Leu Trp Leu Thr Gly Lys Asn Gly Leu Tyr Pro Asn
   165 170 175
   Leu Ser Lys Ser Tyr Ala Asn Asn Lys Glu Lys Glu Val Leu Val Leu
   180 185 190
   Trp Gly Val His His Pro Pro Asn Ile Gly Asp Gln Lys Ala Leu Tyr
   195 200 205
   His Thr Glu Asn Ala Tyr Val Ser Val Val Ser Ser His Tyr Ser Arg
   210 215 220
   Lys Phe Thr Pro Glu Ile Ala Lys Arg Pro Lys Val Arg Asp Gln Glu
   225 230 235 240
   Gly Arg Ile Asn Tyr Tyr Trp Thr Leu Leu Glu Pro Gly Asp Thr Ile
   245 250 255
   Ile Phe Glu Ala Asn Gly Asn Leu Ile Ala Pro Arg Tyr Ala Phe Ala
   260 265 270
   Leu Ser Arg Gly Phe Gly Ser Gly Ile Ile Asn Ser Asn Ala Pro Met
   275 280 285
   Asp Lys Cys Asp Ala Lys Cys Gln Thr Pro Gln Gly Ala Ile Asn Ser
   290 295 300
   Ser Leu Pro Phe Gln Asn Val His Pro Val Thr Ile Gly Glu Cys Pro
   305 310 315 320
   Lys Tyr Val Arg Ser Ala Lys Leu Arg Met Val Thr Gly Leu Arg Asn
   325 330 335
   Ile Pro Ser Ile Gln Ser Arg Gly Leu Phe Gly Ala Ile Ala Gly Phe
   340 345 350
   Ile Glu Gly Gly Trp Thr Gly Met Val Asp Gly Trp Tyr Gly Tyr His
   355 360 365
   His Gln Asn Glu Gln Gly Ser Gly Tyr Ala Ala Asp Gln Lys Ser Thr
       370                 375                 380
   Gln Asn Ala Ile Asn Gly Ile Thr Asn Lys Val Asn Ser Val Ile Glu
   385                 390                 395                 400
   Lys Met Asn Thr Gln Phe Thr Ala Val Gly Lys Glu Phe Asn Lys Leu
                   405                 410                 415
   Glu Arg Arg Met Glu Asn Leu Asn Lys Lys Val Asp Asp Gly Phe Ile
               420                 425                 430
   Asp Ile Trp Thr Tyr Asn Ala Glu Leu Leu Val Leu Leu Glu Asn Glu
           435                 440                 445
   Arg Thr Leu Asp Phe His Asp Ser Asn Val Lys Asn Leu Tyr Glu Lys
       450                 455                 460
   Val Lys Ser Gln Leu Lys Asn Asn Ala Lys Glu Ile Gly Asn Gly Cys
   465                 470                 475                 480
   Phe Glu Phe Tyr His Lys Cys Asn Asp Glu Cys Met Glu Ser Val Lys
                   485                 490                 495
   Asn Gly Thr Tyr Asp Tyr Pro Lys Tyr Ser Glu Glu Ser Lys Leu Asn
               500                 505                 510
   Arg Glu Lys Ile Asp Gly Val Lys Leu Glu Ser Met Gly Val Tyr Gln
           515                 520                 525
   Ile Leu Ala Ile Tyr Ser Thr Val Ala Ser Ser Leu Val Leu Leu Val
       530                 535                 540
   Ser Leu Gly Ala Ile Ser Phe Trp Met Cys Ser Asn Gly Ser Leu Gln
   545                 550                 555                 560
   Cys Arg Ile Cys Ile
                   565
<210> 49
   <211> 565
   <212> PRT
   <213> Artificial sequence
<220>
   <223> clone 775 (A/Solomon Islands 3/2006 (H1N1)
<400> 49
   Met Lys Val Lys Leu Leu Val Leu Leu Cys Thr Phe Thr Ala Thr Tyr
   1               5                   10                  15
   Ala Asp Thr Ile Cys Ile Gly Tyr His Ala Asn Asn Ser Thr Asp Thr
               20                  25                  30
   Val Asp Thr Val Leu Glu Lys Asn Val Thr Val Thr His Ser Val Asn
           35                  40                  45
   Leu Leu Glu Asp Ser His Asn Gly Lys Leu Cys Leu Leu Lys Gly Ile
       50                  55                  60
   Ala Pro Leu Gln Leu Gly Asn Cys Ser Val Ala Gly Trp Ile Leu Gly
   65                  70                  75                  80
   Asn Pro Glu Cys Glu Leu Leu Ile Ser Arg Glu Ser Trp Ser Tyr Ile
                   85                  90                  95
   Val Glu Lys Pro Asn Pro Glu Asn Gly Thr Cys Tyr Pro Gly His Phe
               100                 105                 110
   Ala Asp Tyr Glu Glu Leu Arg Glu Gln Leu Ser Ser Val Ser Ser Phe
           115                 120                 125
   Glu Arg Phe Glu Ile Phe Pro Lys Glu Ser Ser Trp Pro Asn His Thr
       130                 135                 140
   Thr Thr Gly Val Ser Ala Ser Cys Ser His Asn Gly Glu Ser Ser Phe
   145                 150                 155                 160
   Tyr Lys Asn Leu Leu Trp Leu Thr Gly Lys Asn Gly Leu Tyr Pro Asn
                   165                 170                 175
   Leu Ser Lys Ser Tyr Ala Asn Asn Lys Glu Lys Glu Val Leu Val Leu
               180                 185                 190
   Trp Gly Val His His Pro Pro Asn Ile Gly Asp Gln Arg Ala Leu Tyr
           195                 200                 205
   His Lys Glu Asn Ala Tyr Val Ser Val Val Ser Ser His Tyr Ser Arg
       210                 215                 220
   Lys Phe Thr Pro Glu Ile Ala Lys Arg Pro Lys Val Arg Asp Gln Glu
   225                 230                 235                 240
   Gly Arg Ile Asn Tyr Tyr Trp Thr Leu Leu Glu Pro Gly Asp Thr Ile
                   245                 250                 255
   Ile Phe Glu Ala Asn Gly Asn Leu Ile Ala Pro Arg Tyr Ala Phe Ala
               260                 265                 270
   Leu Ser Arg Gly Phe Gly Ser Gly Ile Ile Asn Ser Asn Ala Pro Met
   275 280 285
   Asp Glu Cys Asp Ala Lys Cys Gln Thr Pro Gln Gly Ala Ile Asn Ser
   290 295 300
   Ser Leu Pro Phe Gln Asn Val His Pro Val Thr Ile Gly Glu Cys Pro
   305 310 315 320
   Lys Tyr Val Arg Ser Ala Lys Leu Arg Met Val Thr Gly Leu Arg Asn
   325 330 335
   Ile Pro Ser Ile Gln Ser Arg Gly Leu Phe Gly Ala Ile Ala Gly Phe
   340 345 350
   Ile Glu Gly Gly Trp Thr Gly Met Val Asp Gly Trp Tyr Gly Tyr His
   355 360 365
   His Gln Asn Glu Gln Gly Ser Gly Tyr Ala Ala Asp Gln Lys Ser Thr
   370 375 380
   Gln Asn Ala Ile Asn Gly Ile Thr Asn Lys Val Asn Ser Val Ile Glu
   385 390 395 400
   Lys Met Asn Thr Gln Phe Thr Ala Val Gly Lys Glu Phe Asn Lys Leu
   405 410 415
   Glu Arg Arg Met Glu Asn Leu Asn Lys Lys Val Asp Asp Gly Phe Ile
   420 425 430
   Asp Ile Trp Thr Tyr Asn Ala Glu Leu Leu Val Leu Leu Glu Asn Glu
   435 440 445
   Arg Thr Leu Asp Phe His Asp Ser Asn Val Lys Asn Leu Tyr Glu Lys
   450 455 460
   Val Lys Ser Gln Leu Lys Asn Asn Ala Lys Glu Ile Gly Asn Gly Cys
   465 470 475 480
   Phe Glu Phe Tyr His Lys Cys Asn Asp Glu Cys Met Glu Ser Val Lys
   485 490 495
   Asn Gly Thr Tyr Asp Tyr Pro Lys Tyr Ser Glu Glu Ser Lys Leu Asn
   500 505 510
   Arg Glu Lys Ile Asp Gly Val Lys Leu Glu Ser Met Gly Val Tyr Gln
   515 520 525
   Ile Leu Ala Ile Tyr Ser Thr Val Ala Ser Ser Leu Val Leu Leu Val
   530 535 540
   Ser Leu Gly Ala Ile Ser Phe Trp Met Cys Ser Asn Gly Ser Leu Gln
   545 550 555 560
   Cys Arg Ile Cys Ile
   565
<210> 50
   <211> 566
   <212> PRT
   <213> Artificial sequence
<220>
   <223> clone 776 (A/Brisbane/10/2007 (H3N2)
<400> 50
   Met Lys Thr Ile Ile Ala Leu Ser Tyr Ile Leu Cys Leu Val Phe Thr
   1               5                   10                  15
   Gln Lys Leu Pro Gly Asn Asp Asn Ser Thr Ala Thr Leu Cys Leu Gly
               20                  25                  30
   His His Ala Val Pro Asn Gly Thr Ile Val Lys Thr Ile Thr Asn Asp
           35                  40                  45
   Gln Ile Glu Val Thr Asn Ala Thr Glu Leu Val Gln Ser Ser Ser Thr
       50                  55                  60
   Gly Glu Ile Cys Asp Ser Pro His Gln Ile Leu Asp Gly Glu Asn Cys
   65                  70                  75                      80
   Thr Leu Ile Asp Ala Leu Leu Gly Asp Pro Gln Cys Asp Gly Phe Gln
                   85                  90                  95
   Asn Lys Lys Trp Asp Leu Phe Val Glu Arg Ser Lys Ala Tyr Ser Asn
               100                 105                 110
   Cys Tyr Pro Tyr Asp Val Pro Asp Tyr Ala Ser Leu Arg Ser Leu Val
           115                 120                 125
   Ala Ser Ser Gly Thr Leu Glu Phe Asn Asn Glu Ser Phe Asn Trp Thr
       130                 135                 140
   Gly Val Thr Gln Asn Gly Thr Ser Ser Ala Cys Ile Arg Arg Ser Asn
   145                 150                 155                     160
   Asn Ser Phe Phe Ser Arg Leu Asn Trp Leu Thr His Leu Lys Phe Lys
                   165                 170                 175
   Tyr Pro Ala Leu Asn Val Thr Met Pro Asn Asn Glu Lys Phe Asp Lys
                   180                 185                 190
   Leu Tyr Ile Trp Gly Val His His Pro Gly Thr Asp Asn Asp Gln Ile
               195                 200                 205
   Phe Leu Tyr Ala Gln Ala Ser Gly Arg Ile Thr Val Ser Thr Lys Arg
       210                 215                 220
   Ser Gln Gln Thr Val Ile Pro Asn Ile Gly Ser Arg Pro Arg Val Arg
   225                 230                 235                 240
   Asn Ile Pro Ser Arg Ile Ser Ile Tyr Trp Thr Ile Val Lys Pro Gly
                   245                 250                 255
   Asp Ile Leu Leu Ile Asn Ser Thr Gly Asn Leu Ile Ala Pro Arg Gly
                   260                 265                 270
   Tyr Phe Lys Ile Arg Ser Gly Lys Ser Ser Ile Met Arg Ser Asp Ala
               275                 280                 285
   Pro Ile Gly Lys Cys Asn Ser Glu Cys Ile Thr Pro Asn Gly Ser Ile
       290                 295                 300
   Pro Asn Asp Lys Pro Phe Gln Asn Val Asn Arg Ile Thr Tyr Gly Ala
   305                 310                 315                 320
   Cys Pro Arg Tyr Val Lys Gln Asn Thr Leu Lys Leu Ala Thr Gly Met
                   325                 330                 335
   Arg Asn Val Pro Glu Lys Gln Thr Arg Gly Ile Phe Gly Ala Ile Ala
                   340                 345                 350
   Gly Phe Ile Glu Asn Gly Trp Glu Gly Met Val Asp Gly Trp Tyr Gly
               355                 360                 365
   Phe Arg His Gln Asn Ser Glu Gly Ile Gly Gln Ala Ala Asp Leu Lys
       370                 375                 380
   Ser Thr Gln Ala Ala Ile Asp Gln Ile Asn Gly Lys Leu Asn Arg Leu
   385                 390                 395                 400
   Ile Gly Lys Thr Asn Glu Lys Phe His Gln Ile Glu Lys Glu Phe Ser
                   405                 410                 415
   Glu Val Glu Gly Arg Ile Gln Asp Leu Glu Lys Tyr Val Glu Asp Thr
                   420                 425                 430
   Lys Ile Asp Leu Trp Ser Tyr Asn Ala Glu Leu Leu Val Ala Leu Glu
   435 440 445
   Asn Gln His Thr Ile Asp Leu Thr Asp Ser Glu Met Asn Lys Leu Phe
   450 455 460
   Glu Lys Thr Lys Lys Gln Leu Arg Glu Asn Ala Glu Asp Met Gly Asn
   465 470 475 480
   Gly Cys Phe Lys Ile Tyr His Lys Cys Asp Asn Ala Cys Ile Gly Ser
   485 490 495
   Ile Arg Asn Gly Thr Tyr Asp His Asp Val Tyr Arg Asp Glu Ala Leu
   500 505 510
   Asn Asn Arg Phe Gln Ile Lys Gly Val Glu Leu Lys Ser Gly Tyr Lys
   515 520 525
   Asp Trp Ile Leu Trp Ile Ser Phe Ala Ile Ser Cys Phe Leu Leu Cys
   530 535 540
   Val Ala Leu Leu Gly Phe Ile Met Trp Ala Cys Gln Lys Gly Asn Ile
   545 550 555 560
   Arg Cys Asn Ile Cys Ile
   565
<210> 51
   <211> 566
   <212> PRT
   <213> Artificial sequence
<220>
   <223> clone 777 (A/Wisconsin/67/2005 (H3N2)
<400> 51
   Met Lys Thr Ile Ile Ala Leu Ser Tyr Ile Leu Cys Leu Val Phe Thr
   1               5                   10                  15
   Gln Lys Leu Pro Gly Asn Asp Asn Ser Thr Ala Thr Leu Cys Leu Gly
               20                  25                  30
   His His Ala Val Pro Asn Gly Thr Ile Val Lys Thr Ile Thr Asn Asp
           35                  40                  45
   Gln Ile Glu Val Thr Asn Ala Thr Glu Leu Val Gln Ser Ser Ser Thr
       50                  55                  60
   Gly Gly Ile Cys Asp Ser Pro His Gln Ile Leu Asp Gly Glu Asn Cys
   65                  70                  75                  80
   Thr Leu Ile Asp Ala Leu Leu Gly Asp Pro Gln Cys Asp Gly Phe Gln
                   85                  90                  95
   Asn Lys Lys Trp Asp Leu Phe Val Glu Arg Ser Lys Ala Tyr Ser Asn
               100                 105                 110
   Cys Tyr Pro Tyr Asp Val Pro Asp Tyr Ala Ser Leu Arg Ser Leu Val
           115                 120                 125
   Ala Ser Ser Gly Thr Leu Glu Phe Asn Asp Glu Ser Phe Asn Trp Thr
       130                 135                 140
   Gly Val Thr Gln Asn Gly Thr Ser Ser Ala Cys Lys Arg Arg Ser Asn
   145                 150                 155                 160
   Asn Ser Phe Phe Ser Arg Leu Asn Trp Leu Thr His Leu Lys Phe Lys
                   165                 170                 175
   Tyr Pro Ala Leu Asn Val Thr Met Pro Asn Asn Glu Lys Phe Asp Lys
               180                 185                 190
   Leu Tyr Ile Trp Gly Val His His Pro Gly Thr Asp Asn Asp Gln Ile
           195                 200                 205
   Phe Leu His Ala Gln Ala Ser Gly Arg Ile Thr Val Ser Thr Lys Arg
       210                 215                 220
   Ser Gln Gln Thr Val Ile Pro Asn Ile Gly Ser Arg Pro Arg Ile Arg
   225                 230                 235                 240
   Asn Ile Pro Ser Arg Ile Ser Ile Tyr Trp Thr Ile Val Lys Pro Gly
                   245                 250                 255
   Asp Ile Leu Leu Ile Asn Ser Thr Gly Asn Leu Ile Ala Pro Arg Gly
               260                 265                 270
   Tyr Phe Lys Ile Arg Ser Gly Lys Ser Ser Ile Met Arg Ser Asp Ala
           275                 280                 285
   Pro Ile Gly Lys Cys Asn Ser Glu Cys Ile Thr Pro Asn Gly Ser Ile
       290                 295                 300
   Pro Asn Asp Lys Pro Phe Gln Asn Val Asn Arg Ile Thr Tyr Gly Ala
   305                 310                 315                 320
   Cys Pro Arg Tyr Val Lys Gln Asn Thr Leu Lys Leu Ala Thr Gly Met
                   325                 330                 335
   Arg Asn Val Pro Glu Lys Gln Thr Arg Gly Ile Phe Gly Ala Ile Ala
   340 345 350
   Gly Phe Ile Glu Asn Gly Trp Glu Gly Met Val Asp Gly Trp Tyr Gly
   355 360 365
   Phe Arg His Gln Asn Ser Glu Gly Ile Gly Gln Ala Ala Asp Leu Lys
   370 375 380
   Ser Thr Gln Ala Ala Ile Asn Gln Ile Asn Gly Lys Leu Asn Arg Leu
   385 390 395 400
   Ile Gly Lys Thr Asn Glu Lys Phe His Gln Ile Glu Lys Glu Phe Ser
   405 410 415
   Glu Val Glu Gly Arg Ile Gln Asp Leu Glu Lys Tyr Val Glu Asp Thr
   420 425 430
   Lys Ile Asp Leu Trp Ser Tyr Asn Ala Glu Leu Leu Val Ala Leu Glu
   435 440 445
   Asn Gln His Thr Ile Asp Leu Thr Asp Ser Glu Met Asn Lys Leu Phe
   450 455 460
   Glu Arg Thr Lys Lys Gln Leu Arg Glu Asn Ala Glu Asp Met Gly Asn
   465 470 475 480
   Gly Cys Phe Lys Ile Tyr His Lys Cys Asp Asn Ala Cys Ile Gly Ser
   485 490 495
   Ile Arg Asn Gly Thr Tyr Asp His Asp Val Tyr Arg Asp Glu Ala Leu
   500 505 510
   Asn Asn Arg Phe Gln Ile Lys Gly Val Glu Leu Lys Ser Gly Tyr Lys
   515 520 525
   Asp Trp Ile Leu Trp Ile Ser Phe Ala Ile Ser Cys Phe Leu Leu Cys
   530 535 540
   Val Ala Leu Leu Gly Phe Ile Met Trp Ala Cys Gln Lys Gly Asn Ile
   545 550 555 560
   Arg Cys Asn Ile Cys Ile
   565
<210> 52
   <211> 585
   <212> PRT
   <213> Artificial sequence
<220>
   <223> clone 778 (B/Malaysia/2506/2004)
<400> 52
   Met Lys Ala Ile Ile Val Leu Leu Met Val Val Thr Ser Asn Ala Asp
   1               5                   10                  15
   Arg Ile Cys Thr Gly Ile Thr Ser Ser Asn Ser Pro His Val Val Lys
               20                  25                  30
   Thr Ala Thr Gln Gly Glu Val Asn Val Thr Gly Val Ile Pro Leu Thr
           35                  40                  45
   Thr Thr Pro Thr Lys Ser His Phe Ala Asn Leu Lys Gly Thr Glu Thr
       50                  55                  60
   Arg Gly Lys Leu Cys Pro Lys Cys Leu Asn Cys Thr Asp Leu Asp Val
   65                  70                  75                  80
   Ala Leu Gly Arg Pro Lys Cys Thr Gly Asn Ile Pro Ser Ala Arg Val
                   85                  90                  95
   Ser Ile Leu His Glu Val Arg Pro Val Thr Ser Gly Cys Phe Pro Ile
               100                 105                 110
   Met His Asp Arg Thr Lys Ile Arg Gln Leu Pro Lys Leu Leu Arg Gly
           115                 120                 125
   Tyr Glu His Ile Arg Leu Ser Thr His Asn Val Ile Asn Ala Glu Asn
       130                 135                 140
   Ala Pro Gly Gly Pro Tyr Lys Ile Gly Thr Ser Gly Ser Cys Pro Asn
   145                 150                 155                 160
   Val Thr Asn Gly Asn Gly Phe Phe Ala Thr Met Ala Trp Ala Val Pro
                   165                 170                 175
   Lys Asn Asp Asn Asn Lys Thr Ala Thr Asn Ser Leu Thr Ile Glu Val
               180                 185                 190
   Pro Tyr Ile Cys Thr Glu Gly Glu Asp Gln Ile Thr Val Trp Gly Phe
           195                 200                 205
   His Ser Asp Asn Glu Thr Gln Met Ala Lys Leu Tyr Gly Asp Ser Lys
       210                 215                 220
   Pro Gln Lys Phe Thr Ser Ser Ala Asn Gly Val Thr Thr His Tyr Val
   225                 230                 235                 240
   Ser Gln Ile Gly Gly Phe Pro Asn Gln Thr Glu Asp Gly Gly Leu Pro
   245 250 255
   Gln Ser Gly Arg Ile Val Val Asp Tyr Met Val Gln Lys Ser Gly Lys
   260 265 270
   Thr Gly Thr Ile Thr Tyr Gln Arg Gly Ile Leu Leu Pro Gln Lys Val
   275 280 285
   Trp Cys Ala Ser Gly Arg Ser Lys Val Ile Lys Gly Ser Leu Pro Leu
   290 295 300
   Ile Gly Glu Ala Asp Cys Leu His Glu Lys Tyr Gly Gly Leu Asn Lys
   305 310 315 320
   Ser Lys Pro Tyr Tyr Thr Gly Glu His Ala Lys Ala Ile Gly Asn Cys
   325 330 335
   Pro Ile Trp Val Lys Thr Pro Leu Lys Leu Ala Asn Gly Thr Lys Tyr
   340 345 350
   Arg Pro Pro Ala Lys Leu Leu Lys Glu Arg Gly Phe Phe Gly Ala Ile
   355 360 365
   Ala Gly Phe Leu Glu Gly Gly Trp Glu Gly Met Ile Ala Gly Trp His
   370 375 380
   Gly Tyr Thr Ser His Gly Ala His Gly Val Ala Val Ala Ala Asp Leu
   385 390 395 400
   Lys Ser Thr Gln Glu Ala Ile Asn Lys Ile Thr Lys Asn Leu Asn Ser
   405 410 415
   Leu Ser Glu Leu Glu Val Lys Asn Leu Gln Arg Leu Ser Gly Ala Met
   420 425 430
   Asp Glu Leu His Asn Glu Ile Leu Glu Leu Asp Glu Lys Val Asp Asp
   435 440 445
   Leu Arg Ala Asp Thr Ile Ser Ser Gln Ile Glu Leu Ala Val Leu Leu
   450 455 460
   Ser Asn Glu Gly Ile Ile Asn Ser Glu Asp Glu His Leu Leu Ala Leu
   465 470 475 480
   Glu Arg Lys Leu Lys Lys Met Leu Gly Pro Ser Ala Val Glu Ile Gly
   485 490 495
   Asn Gly Cys Phe Glu Thr Lys His Lys Cys Asn Gln Thr Cys Leu Asp
   500 505 510
   Arg Ile Ala Ala Gly Thr Phe Asp Ala Gly Glu Phe Ser Leu Pro Thr
   515 520 525
   Phe Asp Ser Leu Asn Ile Thr Ala Ala Ser Leu Asn Asp Asp Gly Leu
   530 535 540
   Asp Asn His Thr Ile Leu Leu Tyr Tyr Ser Thr Ala Ala Ser Ser Leu
   545 550 555 560
   Ala Val Thr Leu Met Ile Ala Ile Phe Val Val Tyr Met Val Ser Arg
   565 570 575
   Asp Asn Val Ser Cys Ser Ile Cys Leu
   580 585
<210> 53
   <211> 584
   <212> PRT
   <213> Artificial
<220>
   <223> clone 779 (B/Florida/4/2006)
<400> 53
   Met Lys Ala Ile Ile Val Leu Leu Met Val Val Thr Ser Asn Ala Asp
   1               5                   10                  15
   Arg Ile Cys Thr Gly Ile Thr Ser Ser Asn Ser Pro His Val Val Lys
               20                  25                  30
   Thr Ala Thr Gln Gly Glu Val Asn Val Thr Gly Val Ile Pro Leu Thr
           35                  40                  45
   Thr Thr Pro Thr Lys Ser Tyr Phe Ala Asn Leu Lys Gly Thr Arg Thr
       50                  55                  60
   Arg Gly Lys Leu Cys Pro Asp Cys Leu Asn Cys Thr Asp Leu Asp Val
   65                  70                  75                  80
   Ala Leu Gly Arg Pro Met Cys Val Gly Thr Thr Pro Ser Ala Lys Ala
                   85                  90                  95
   Ser Ile Leu His Glu Val Lys Pro Val Thr Ser Gly Cys Phe Pro Ile
               100                 105                 110
   Met His Asp Arg Thr Lys Ile Arg Gln Leu Pro Asn Leu Leu Arg Gly
           115                 120                 125
   Tyr Glu Asn Ile Arg Leu Ser Thr Gln Asn Val Ile Asp Ala Glu Lys
   130 135 140
   Ala Pro Gly Gly Pro Tyr Arg Leu Gly Thr Ser Gly Ser Cys Pro Asn
   145 150 155 160
   Ala Thr Ser Lys Ser Gly Phe Phe Ala Thr Met Ala Trp Ala Val Pro
   165 170 175
   Lys Asp Asn Asn Lys Asn Ala Thr Asn Pro Leu Thr Val Glu Val Pro
   180 185 190
   Tyr Ile Cys Thr Glu Gly Glu Asp Gln Ile Thr Val Trp Gly Phe His
   195 200 205
   Ser Asp Asn Lys Thr Gln Met Lys Asn Leu Tyr Gly Asp Ser Asn Pro
   210 215 220
   Gln Lys Phe Thr Ser Ser Ala Asn Gly Val Thr Thr His Tyr Val Ser
   225 230 235 240
   Gln Ile Gly Ser Phe Pro Asp Gln Thr Glu Asp Gly Gly Leu Pro Gln
   245 250 255
   Ser Gly Arg Ile Val Val Asp Tyr Met Met Gln Lys Pro Gly Lys Thr
   260 265 270
   Gly Thr Ile Val Tyr Gln Arg Gly Val Leu Leu Pro Gln Lys Val Trp
   275 280 285
   Cys Ala Ser Gly Arg Ser Lys Val Ile Lys Gly Ser Leu Pro Leu Ile
   290 295 300
   Gly Glu Ala Asp Cys Leu His Glu Lys Tyr Gly Gly Leu Asn Lys Ser
   305 310 315 320
   Lys Pro Tyr Tyr Thr Gly Glu His Ala Lys Ala Ile Gly Asn Cys Pro
   325 330 335
   Ile Trp Val Lys Thr Pro Leu Lys Leu Ala Asn Gly Thr Lys Tyr Arg
   340 345 350
   Pro Pro Ala Lys Leu Leu Lys Glu Arg Gly Phe Phe Gly Ala Ile Ala
   355 360 365
   Gly Phe Leu Glu Gly Gly Trp Glu Gly Met Ile Ala Gly Trp His Gly
   370 375 380
   Tyr Thr Ser His Gly Ala His Gly Val Ala Val Ala Ala Asp Leu Lys
   385 390 395 400
   Ser Thr Gln Glu Ala Ile Asn Lys Ile Thr Lys Asn Leu Asn Ser Leu
   405 410 415
   Ser Glu Leu Glu Val Lys Asn Leu Gln Arg Leu Ser Gly Ala Met Asp
   420 425 430
   Glu Leu His Asn Glu Ile Leu Glu Leu Asp Glu Lys Val Asp Asp Leu
   435 440 445
   Arg Ala Asp Thr Ile Ser Ser Gln Ile Glu Leu Ala Val Leu Leu Ser
   450 455 460
   Asn Glu Gly Ile Ile Asn Ser Glu Asp Glu His Leu Leu Ala Leu Glu
   465 470 475 480
   Arg Lys Leu Lys Lys Met Leu Gly Pro Ser Ala Val Glu Ile Gly Asn
   485 490 495
   Gly Cys Phe Glu Thr Lys His Lys Cys Asn Gln Thr Cys Leu Asp Arg
   500 505 510
   Ile Ala Ala Gly Thr Phe Asn Ala Gly Glu Phe Ser Leu Pro Thr Phe
   515 520 525
   Asp Ser Leu Asn Ile Thr Ala Ala Ser Leu Asn Asp Asp Gly Leu Asp
   530 535 540
   Asn His Thr Ile Leu Leu Tyr Tyr Ser Thr Ala Ala Ser Ser Leu Ala
   545 550 555 560
   Val Thr Leu Met Leu Ala Ile Phe Ile Val Tyr Met Val Ser Arg Asp
   565 570 575
   Asn Val Ser Cys Ser Ile Cys Leu
   580
<210> 54
   <211> 562
   <212> PRT
   <213> Artificial sequence
<220>
   <223> clone 780 (A/Singapore/1/57 (H2N2))
<400> 54
   Met Ala Ile Ile Tyr Leu Ile Leu Leu Phe Thr Ala Val Arg Gly Asp
   1               5                   10                  15
   Gln Ile Cys Ile Gly Tyr His Ala Asn Asn Ser Thr Glu Lys Val Asp
   20 25 30
   Thr Ile Leu Glu Arg Asn Val Thr Val Thr His Ala Lys Asp Ile Leu
   35 40 45
   Glu Lys Thr His Asn Gly Lys Leu Cys Lys Leu Asn Gly Ile Pro Pro
   50 55 60
   Leu Glu Leu Gly Asp Cys Ser Ile Ala Gly Trp Leu Leu Gly Asn Pro
   65 70 75 80
   Glu Cys Asp Arg Leu Leu Ser Val Pro Glu Trp Ser Tyr Ile Met Glu
   85 90 95
   Lys Glu Asn Pro Arg Asp Gly Leu Cys Tyr Pro Gly Ser Phe Asn Asp
   100 105 110
   Tyr Glu Glu Leu Lys His Leu Leu Ser Ser Val Lys His Phe Glu Lys
   115 120 125
   Val Lys Ile Leu Pro Lys Asp Arg Trp Thr Gln His Thr Thr Thr Gly
   130 135 140
   Gly Ser Arg Ala Cys Ala Val Ser Gly Asn Pro Ser Phe Phe Arg Asn
   145 150 155 160
   Met Val Trp Leu Thr Lys Lys Glu Ser Asn Tyr Pro Val Ala Lys Gly
   165 170 175
   Ser Tyr Asn Asn Thr Ser Gly Glu Gln Met Leu Ile Ile Trp Gly Val
   180 185 190
   His His Pro Asn Asp Glu Thr Glu Gln Arg Thr Leu Tyr Gln Asn Val
   195 200 205
   Gly Thr Tyr Val Ser Val Gly Thr Ser Thr Leu Asn Lys Arg Ser Thr
   210 215 220
   Pro Asp Ile Ala Thr Arg Pro Lys Val Asn Gly Leu Gly Ser Arg Met
   225 230 235 240
   Glu Phe Ser Trp Thr Leu Leu Asp Met Trp Asp Thr Ile Asn Phe Glu
   245 250 255
   Ser Thr Gly Asn Leu Ile Ala Pro Glu Tyr Gly Phe Lys Ile Ser Lys
   260 265 270
   Arg Gly Ser Ser Gly Ile Met Lys Thr Glu Gly Thr Leu Glu Asn Cys
           275                 280                 285
   Glu Thr Lys Cys Gln Thr Pro Leu Gly Ala Ile Asn Thr Thr Leu Pro
       290                 295                 300
   Phe His Asn Val His Pro Leu Thr Ile Gly Glu Cys Pro Lys Tyr Val
   305                 310                 315                 320
   Lys Ser Glu Lys Leu Val Leu Ala Thr Gly Leu Arg Asn Val Pro Gln
                   325                 330                 335
   Ile Glu Ser Arg Gly Leu Phe Gly Ala Ile Ala Gly Phe Ile Glu Gly
               340                 345                 350
   Gly Trp Gln Gly Met Val Asp Gly Trp Tyr Gly Tyr His His Ser Asn
           355                 360                 365
   Asp Gln Gly Ser Gly Tyr Ala Ala Asp Lys Glu Ser Thr Gln Lys Ala
       370                 375                 380
   Phe Asp Gly Ile Thr Asn Lys Val Asn Ser Val Ile Glu Lys Met Asn
   385                 390                 395                 400
   Thr Gln Phe Glu Ala Val Gly Lys Glu Phe Ser Asn Leu Glu Arg Arg
                   405                 410                 415
   Leu Glu Asn Leu Asn Lys Lys Met Glu Asp Gly Phe Leu Asp Val Trp
               420                 425                 430
   Thr Tyr Asn Ala Glu Leu Leu Val Leu Met Glu Asn Glu Arg Thr Leu
           435                 440                 445
   Asp Phe His Asp Ser Asn Val Lys Asn Leu Tyr Asp Lys Val Arg Met
       450                 455                 460
   Gln Leu Arg Asp Asn Val Lys Glu Leu Gly Asn Gly Cys Phe Glu Phe
   465                 470                 475                 480
   Tyr His Lys Cys Asp Asp Glu Cys Met Asn Ser Val Lys Asn Gly Thr
                   485                 490                 495
   Tyr Asp Tyr Pro Lys Tyr Glu Glu Glu Ser Lys Leu Asn Arg Asn Glu
               500                 505                 510
   Ile Lys Gly Val Lys Leu Ser Ser Met Gly Val Tyr Gln Ile Leu Ala
           515                 520                 525
   Ile Tyr Ala Thr Val Ala Gly Ser Leu Ser Leu Ala Ile Met Met Ala
       530                 535                 540
   Gly Ile Ser Phe Trp Met Cys Ser Asn Gly Ser Leu Gln Cys Arg Ile
   545 550 555 560
   Cys Ile
<210> 55
   <211> 567
   <212> PRT
   <213> Artificial sequence
<220>
   <223> clone 781 (A/Anhui/1/2005 (H5N1))
<400> 55
   Met Glu Lys Ile Val Leu Leu Leu Ala Ile Val Ser Leu Val Lys Ser
   1               5                   10                  15
   Asp Gln Ile Cys Ile Gly Tyr His Ala Asn Asn Ser Thr Glu Gln Val
               20                  25                  30
   Asp Thr Ile Met Glu Lys Asn Val Thr Val Thr His Ala Gln Asp Ile
           35                  40                  45
   Leu Glu Lys Thr His Asn Gly Lys Leu Cys Asp Leu Asp Gly Val Lys
       50                  55                  60
   Pro Leu Ile Leu Arg Asp Cys Ser Val Ala Gly Trp Leu Leu Gly Asn
   65                  70                  75                  80
   Pro Met Cys Asp Glu Phe Ile Asn Val Pro Glu Trp Ser Tyr Ile Val
                   85                  90                  95
   Glu Lys Ala Asn Pro Ala Asn Asp Leu Cys Tyr Pro Gly Asn Phe Asn
               100                 105                 110
   Asp Tyr Glu Glu Leu Lys His Leu Leu Ser Arg Ile Asn His Phe Glu
           115                 120                 125
   Lys Ile Gln Ile Ile Pro Lys Ser Ser Trp Ser Asp His Glu Ala Ser
       130                 135                 140
   Ser Gly Val Ser Ser Ala Cys Pro Tyr Gln Gly Thr Pro Ser Phe Phe
   145                 150                 155                 160
   Arg Asn Val Val Trp Leu Ile Lys Lys Asn Asn Thr Tyr Pro Thr Ile
                   165                 170                 175
   Lys Arg Ser Tyr Asn Asn Thr Asn Gln Glu Asp Leu Leu Ile Leu Trp
   180 185 190
   Gly Ile His His Ser Asn Asp Ala Ala Glu Gln Thr Lys Leu Tyr Gln
   195 200 205
   Asn Pro Thr Thr Tyr Ile Ser Val Gly Thr Ser Thr Leu Asn Gln Arg
   210 215 220
   Leu Val Pro Lys Ile Ala Thr Arg Ser Lys Val Asn Gly Gln Ser Gly
   225 230 235 240
   Arg Met Asp Phe Phe Trp Thr Ile Leu Lys Pro Asn Asp Ala Ile Asn
   245 250 255
   Phe Glu Ser Asn Gly Asn Phe Ile Ala Pro Glu Tyr Ala Tyr Lys Ile
   260 265 270
   Val Lys Lys Gly Asp Ser Ala Ile Val Lys Ser Glu Val Glu Tyr Gly
   275 280 285
   Asn Cys Asn Thr Lys Cys Gln Thr Pro Ile Gly Ala Ile Asn Ser Ser
   290 295 300
   Met Pro Phe His Asn Ile His Pro Leu Thr Ile Gly Glu Cys Pro Lys
   305 310 315 320
   Tyr Val Lys Ser Asn Lys Leu Val Leu Ala Thr Gly Leu Arg Asn Ser
   325 330 335
   Pro Leu Arg Glu Arg Arg Arg Lys Arg Gly Leu Phe Gly Ala Ile Ala
   340 345 350
   Gly Phe Ile Glu Gly Gly Trp Gln Gly Met Val Asp Gly Trp Tyr Gly
   355 360 365
   Tyr His His Ser Asn Glu Gln Gly Ser Gly Tyr Ala Ala Asp Lys Glu
   370 375 380
   Ser Thr Gln Lys Ala Ile Asp Gly Val Thr Asn Lys Val Asn Ser Ile
   385 390 395 400
   Ile Asp Lys Met Asn Thr Gln Phe Glu Ala Val Gly Arg Glu Phe Asn
   405 410 415
   Asn Leu Glu Arg Arg Ile Glu Asn Leu Asn Lys Lys Met Glu Asp Gly
   420 425 430
   Phe Leu Asp Val Trp Thr Tyr Asn Ala Glu Leu Leu Val Leu Met Glu
   435 440 445
   Asn Glu Arg Thr Leu Asp Phe His Asp Ser Asn Val Lys Asn Leu Tyr
   450 455 460
   Asp Lys Val Arg Leu Gln Leu Arg Asp Asn Ala Lys Glu Leu Gly Asn
   465 470 475 480
   Gly Cys Phe Glu Phe Tyr His Lys Cys Asp Asn Glu Cys Met Glu Ser
   485 490 495
   Val Arg Asn Gly Thr Tyr Asp Tyr Pro Gln Tyr Ser Glu Glu Ala Arg
   500 505 510
   Leu Lys Arg Glu Glu Ile Ser Gly Val Lys Leu Glu Ser Ile Gly Thr
   515 520 525
   Tyr Gln Ile Leu Ser Ile Tyr Ser Thr Val Ala Ser Ser Leu Ala Leu
   530 535 540
   Ala Ile Met Val Ala Gly Leu Ser Leu Trp Met Cys Ser Asn Gly Ser
   545 550 555 560
   Leu Gln Cys Arg Ile Cys Ile
   565
<210> 56
   <211> 568
   <212> PRT
   <213> Artificial sequence
<220>
   <223> clone 782 (A/Vietnam/1194/2004 (H5N1))
<400> 56
   Met Glu Lys Ile Val Leu Leu Phe Ala Ile Val Ser Leu Val Lys Ser
   1               5                   10                  15
   Asp Gln Ile Cys Ile Gly Tyr His Ala Asn Asn Ser Thr Glu Gln Val
               20                  25                  30
   Asp Thr Ile Met Glu Lys Asn Val Thr Val Thr His Ala Gln Asp Ile
           35                  40                  45
   Leu Glu Lys Thr His Asn Gly Lys Leu Cys Asp Leu Asp Gly Val Lys
       50                  55                  60
   Pro Leu Ile Leu Arg Asp Cys Ser Val Ala Gly Trp Leu Leu Gly Asn
   65                  70                  75                  80
   Pro Met Cys Asp Glu Phe Ile Asn Val Pro Glu Trp Ser Tyr Ile Val
   85 90 95
   Glu Lys Ala Asn Pro Val Asn Asp Leu Cys Tyr Pro Gly Asp Phe Asn
   100 105 110
   Asp Tyr Glu Glu Leu Lys His Leu Leu Ser Arg Ile Asn His Phe Glu
   115 120 125
   Lys Ile Gln Ile Ile Pro Lys Ser Ser Trp Ser Ser His Glu Ala Ser
   130 135 140
   Leu Gly Val Ser Ser Ala Cys Pro Tyr Gln Gly Lys Ser Ser Phe Phe
   145 150 155 160
   Arg Asn Val Val Trp Leu Ile Lys Lys Asn Ser Thr Tyr Pro Thr Ile
   165 170 175
   Lys Arg Ser Tyr Asn Asn Thr Asn Gln Glu Asp Leu Leu Val Leu Trp
   180 185 190
   Gly Ile His His Pro Asn Asp Ala Ala Glu Gln Thr Lys Leu Tyr Gln
   195 200 205
   Asn Pro Thr Thr Tyr Ile Ser Val Gly Thr Ser Thr Leu Asn Gln Arg
   210 215 220
   Leu Val Pro Arg Ile Ala Thr Arg Ser Lys Val Asn Gly Gln Ser Gly
   225 230 235 240
   Arg Met Glu Phe Phe Trp Thr Ile Leu Lys Pro Asn Asp Ala Ile Asn
   245 250 255
   Phe Glu Ser Asn Gly Asn Phe Ile Ala Pro Glu Tyr Ala Tyr Lys Ile
   260 265 270
   Val Lys Lys Gly Asp Ser Thr Ile Met Lys Ser Glu Leu Glu Tyr Gly
   275 280 285
   Asn Cys Asn Thr Lys Cys Gln Thr Pro Met Gly Ala Ile Asn Ser Ser
   290 295 300
   Met Pro Phe His Asn Ile His Pro Leu Thr Ile Gly Glu Cys Pro Lys
   305 310 315 320
   Tyr Val Lys Ser Asn Arg Leu Val Leu Ala Thr Gly Leu Arg Asn Ser
   325 330 335
   Pro Gln Arg Glu Arg Arg Arg Lys Lys Arg Gly Leu Phe Gly Ala Ile
   340 345 350
   Ala Gly Phe Ile Glu Gly Gly Trp Gln Gly Met Val Asp Gly Trp Tyr
   355 360 365
   Gly Tyr His His Ser Asn Glu Gln Gly Ser Gly Tyr Ala Ala Asp Lys
   370 375 380
   Glu Ser Thr Gln Lys Ala Ile Asp Gly Val Thr Asn Lys Val Asn Ser
   385 390 395 400
   Ile Ile Asp Lys Met Asn Thr Gln Phe Glu Ala Val Gly Arg Glu Phe
   405 410 415
   Asn Asn Leu Glu Arg Arg Ile Glu Asn Leu Asn Lys Lys Met Glu Asp
   420 425 430
   Gly Phe Leu Asp Val Trp Thr Tyr Asn Ala Glu Leu Leu Val Leu Met
   435 440 445
   Glu Asn Glu Arg Thr Leu Asp Phe His Asp Ser Asn Val Lys Asn Leu
   450 455 460
   Tyr Asp Lys Val Arg Leu Gln Leu Arg Asp Asn Ala Lys Glu Leu Gly
   465 470 475 480
   Asn Gly Cys Phe Glu Phe Tyr His Lys Cys Asp Asn Glu Cys Met Glu
   485 490 495
   Ser Val Arg Asn Gly Thr Tyr Asp Tyr Pro Gln Tyr Ser Glu Glu Ala
   500 505 510
   Arg Leu Lys Arg Glu Glu Ile Ser Gly Val Lys Leu Glu Ser Ile Gly
   515 520 525
   Ile Tyr Gln Ile Leu Ser Ile Tyr Ser Thr Val Ala Ser Ser Leu Ala
   530 535 540
   Leu Ala Ile Met Val Ala Gly Leu Ser Leu Trp Met Cys Ser Asn Gly
   545 550 555 560
   Ser Leu Gln Cys Arg Ile Cys Ile
   565
<210> 57
   <211> 566
   <212> PRT
   <213> Artificial sequence
<220>
   <223> clone 783 (A/Teal/HongKong/W312/97 (H6N1))
<400> 57
   Met Ile Ala Ile Ile Val Ile Ala Ile Leu Ala Ala Ala Gly Lys Ser
   1               5                   10                  15
   Asp Lys Ile Cys Ile Gly Tyr His Ala Asn Asn Ser Thr Thr Gln Val
               20                  25                  30
   Asp Thr Ile Leu Glu Lys Asn Val Thr Val Thr His Ser Ile Glu Leu
           35                  40                  45
   Leu Glu Asn Gln Lys Glu Glu Arg Phe Cys Lys Ile Leu Asn Lys Ala
       50                  55                  60
   Pro Leu Asp Leu Arg Glu Cys Thr Ile Glu Gly Trp Ile Leu Gly Asn
   65                  70                  75                  80
   Pro Gln Cys Asp Leu Leu Leu Gly Asp Gln Ser Trp Ser Tyr Ile Val
                   85                  90                  95
   Glu Arg Pro Thr Ala Gln Asn Gly Ile Cys Tyr Pro Gly Thr Leu Asn
               100                 105                 110
   Glu Val Glu Glu Leu Arg Ala Leu Ile Gly Ser Gly Glu Arg Val Glu
           115                 120                 125
   Arg Phe Glu Met Phe Pro Gln Ser Thr Trp Gln Gly Val Asp Thr Asn
       130                 135                 140
   Ser Gly Thr Thr Arg Ser Cys Pro Tyr Ser Thr Gly Ala Ser Phe Tyr
   145                 150                 155                 160
   Arg Asn Leu Leu Trp Ile Ile Lys Thr Lys Thr Ala Glu Tyr Pro Val
                   165                 170                 175
   Ile Lys Gly Ile Tyr Asn Asn Thr Gly Thr Gln Pro Ile Leu Tyr Phe
               180                 185                 190
   Trp Gly Val His His Pro Pro Asn Thr Asp Glu Gln Asp Thr Leu Tyr
           195                 200                 205
   Gly Ser Gly Asp Arg Tyr Val Arg Met Gly Thr Glu Ser Met Asn Phe
       210                 215                 220
   Ala Lys Ser Pro Glu Ile Ala Ala Arg Pro Ala Val Asn Gly Gln Arg
   225                 230                 235                 240
   Gly Arg Ile Asp Tyr Tyr Trp Ser Val Leu Lys Pro Gly Glu Thr Leu
                   245                 250                 255
   Asn Val Glu Ser Asn Gly Asn Leu Ile Ala Pro Trp Tyr Ala Tyr Lys
   260 265 270
   Phe Val Asn Thr Asn Ser Lys Gly Ala Val Phe Arg Ser Asp Leu Pro
   275 280 285
   Ile Glu Asn Cys Asp Ala Thr Cys Gln Thr Ile Ala Gly Val Leu Arg
   290 295 300
   Thr Asn Lys Thr Phe Gln Asn Val Ser Pro Leu Trp Ile Gly Glu Cys
   305 310 315 320
   Pro Lys Tyr Val Lys Ser Glu Ser Leu Arg Leu Ala Thr Gly Leu Arg
   325 330 335
   Asn Val Pro Gln Ile Glu Thr Arg Gly Leu Phe Gly Ala Ile Ala Gly
   340 345 350
   Phe Ile Glu Gly Gly Trp Thr Gly Met Ile Asp Gly Trp Tyr Gly Tyr
   355 360 365
   His His Glu Asn Ser Gln Gly Ser Gly Tyr Ala Ala Asp Arg Glu Ser
   370 375 380
   Thr Gln Lys Ala Val Asn Arg Ile Thr Asn Lys Val Asn Ser Ile Ile
   385 390 395 400
   Asn Lys Met Asn Thr Gln Phe Glu Ala Val Asp His Glu Phe Ser Asn
   405 410 415
   Leu Glu Arg Arg Ile Asp Asn Leu Asn Lys Arg Met Gln Asp Gly Phe
   420 425 430
   Leu Asp Val Trp Thr Tyr Asn Ala Glu Leu Leu Val Leu Leu Glu Asn
   435 440 445
   Glu Arg Thr Leu Asp Met His Asp Ala Asn Val Lys Asn Leu His Glu
   450 455 460
   Lys Val Lys Ser Gln Leu Arg Asp Asn Ala Thr Ile Leu Gly Asn Gly
   465 470 475 480
   Cys Phe Glu Phe Trp His Lys Cys Asp Asn Glu Cys Ile Glu Ser Val
   485 490 495
   Lys Asn Gly Thr Tyr Asp Tyr Pro Lys Tyr Gln Thr Glu Ser Lys Leu
   500 505 510
   Asn Arg Leu Lys Ile Glu Ser Val Lys Leu Glu Asn Leu Gly Val Tyr
   515 520 525
   Gln Ile Leu Ala Ile Tyr Ser Thr Val Ser Ser Ser Leu Val Leu Val
   530 535 540
   Gly Leu Ile Met Ala Met Gly Leu Trp Met Cys Ser Asn Gly Ser Met
   545 550 555 560
   Gln Cys Arg Ile Cys Ile
   565
<210> 58
   <211> 570
   <212> PRT
   <213> Artificial sequence
<220>
   <223> clone 784 (A/Equine/Prague/56 (H7N7))
<400> 58
   Met Asn Thr Gln Ile Leu Ile Leu Ala Thr Ser Ala Phe Phe Tyr Val
   1               5                   10                  15
   Arg Ala Asp Lys Ile Cys Leu Gly His His Ala Val Ser Asn Gly Thr
               20                  25                  30
   Lys Val Asp Thr Leu Thr Glu Lys Gly Ile Glu Val Val Asn Ala Thr
           35                  40                  45
   Glu Thr Val Glu Gln Thr Asn Ile Pro Lys Ile Cys Ser Lys Gly Lys
       50                  55                  60
   Gln Thr Val Asp Leu Gly Gln Cys Gly Leu Leu Gly Thr Val Ile Gly
   65                  70                  75                  80
   Pro Pro Gln Cys Asp Gln Phe Leu Glu Phe Ser Ala Asn Leu Ile Val
                   85                  90                  95
   Glu Arg Arg Glu Gly Asn Asp Ile Cys Tyr Pro Gly Lys Phe Asp Asn
               100                 105                 110
   Glu Glu Thr Leu Arg Lys Ile Leu Arg Lys Ser Gly Gly Ile Lys Lys
           115                 120                 125
   Glu Asn Met Gly Phe Thr Tyr Thr Gly Val Arg Thr Asn Gly Glu Thr
       130                 135                 140
   Ser Ala Cys Arg Arg Ser Arg Ser Ser Phe Tyr Ala Glu Met Lys Trp
   145                 150                 155                 160
   Leu Leu Ser Ser Thr Asp Asn Gly Thr Phe Pro Gln Met Thr Lys Ser
                   165                 170                 175
   Tyr Lys Asn Thr Lys Lys Val Pro Ala Leu Ile Ile Trp Gly Ile His
               180                 185                 190
   His Ser Gly Ser Thr Thr Glu Gln Thr Arg Leu Tyr Gly Ser Gly Asn
           195                 200                 205
   Lys Leu Ile Thr Val Trp Ser Ser Lys Tyr Gln Gln Ser Phe Val Pro
       210                 215                 220
   Asn Pro Gly Pro Arg Pro Gln Met Asn Gly Gln Ser Gly Arg Ile Asp
   225                 230                 235                 240
   Phe His Trp Leu Met Leu Asp Pro Asn Asp Thr Val Thr Phe Ser Phe
                   245                 250                 255
   Asn Gly Ala Phe Ile Ala Pro Asp Arg Ala Ser Phe Leu Arg Gly Lys
               260                 265                 270
   Ser Leu Gly Ile Gln Ser Asp Ala Gln Leu Asp Asn Asn Cys Glu Gly
           275                 280                 285
   Glu Cys Tyr His Ile Gly Gly Thr Ile Ile Ser Asn Leu Pro Phe Gln
       290                 295                 300
   Asn Ile Asn Ser Arg Ala Ile Gly Lys Cys Pro Arg Tyr Val Lys Gln
   305                 310                 315                 320
   Lys Ser Leu Met Leu Ala Thr Gly Met Lys Asn Val Pro Glu Ala Pro
                   325                 330                 335
   Ala His Lys Gln Leu Thr His His Met Arg Lys Lys Arg Gly Leu Phe
               340                 345                 350
   Gly Ala Ile Ala Gly Phe Ile Glu Asn Gly Trp Glu Gly Leu Ile Asp
           355                 360                 365
   Gly Trp Tyr Gly Tyr Lys His Gln Asn Ala Gln Gly Glu Gly Thr Ala
       370                 375                 380
   Ala Asp Tyr Lys Ser Thr Gln Ser Ala Ile Asn Gln Ile Thr Gly Lys
   385                 390                 395                 400
   Leu Asn Arg Leu Ile Glu Lys Thr Asn Gln Gln Phe Glu Leu Ile Asp
                   405                 410                 415
   Asn Glu Phe Asn Glu Ile Glu Lys Gln Ile Gly Asn Val Ile Asn Trp
   420 425 430
   Thr Arg Asp Ser Ile Ile Glu Val Trp Ser Tyr Asn Ala Glu Phe Leu
   435 440 445
   Val Ala Val Glu Asn Gln His Thr Ile Asp Leu Thr Asp Ser Glu Met
   450 455 460
   Asn Lys Leu Tyr Glu Lys Val Arg Arg Gln Leu Arg Glu Asn Ala Glu
   465 470 475 480
   Glu Asp Gly Asn Gly Cys Phe Glu Ile Phe His Gln Cys Asp Asn Asp
   485 490 495
   Cys Met Ala Ser Ile Arg Asn Asn Thr Tyr Asp His Lys Lys Tyr Arg
   500 505 510
   Lys Glu Ala Ile Gln Asn Arg Ile Gln Ile Asp Ala Val Lys Leu Ser
   515 520 525
   Ser Gly Tyr Lys Asp Ile Ile Leu Trp Phe Ser Phe Gly Ala Ser Cys
   530 535 540
   Phe Leu Phe Leu Ala Ile Ala Met Gly Leu Val Phe Ile Cys Ile Lys
   545 550 555 560
   Asn Gly Asn Met Arg Cys Thr Ile Cys Ile
   565 570
<210> 59
   <211> 560
   <212> PRT
   <213> Artificial sequence
<220>
   <223> clone 785 (A/HongKong/1073/99 (H9N2))
<400> 59
   Met Glu Thr Ile Ser Leu Ile Thr Ile Leu Leu Val Val Thr Ala Ser
   1               5                   10                  15
   Asn Ala Asp Lys Ile Cys Ile Gly His Gln Ser Thr Asn Ser Thr Glu
               20                  25                  30
   Thr Val Asp Thr Leu Thr Glu Thr Asn Val Pro Val Thr His Ala Lys
           35                  40                  45
   Glu Leu Leu His Thr Glu His Asn Gly Met Leu Cys Ala Thr Ser Leu
       50                  55                  60
   Gly His Pro Leu Ile Leu Asp Thr Cys Thr Ile Glu Gly Leu Val Tyr
   65                  70                  75                  80
   Gly Asn Pro Ser Cys Asp Leu Leu Leu Gly Gly Arg Glu Trp Ser Tyr
                   85                  90                  95
   Ile Val Glu Arg Ser Ser Ala Val Asn Gly Thr Cys Tyr Pro Gly Asn
               100                 105                 110
   Val Glu Asn Leu Glu Glu Leu Arg Thr Leu Phe Ser Ser Ala Ser Ser
           115                 120                 125
   Tyr Gln Arg Ile Gln Ile Phe Pro Asp Thr Thr Trp Asn Val Thr Tyr
       130                 135                 140
   Thr Gly Thr Ser Arg Ala Cys Ser Gly Ser Phe Tyr Arg Ser Met Arg
   145                 150                 155                 160
   Trp Leu Thr Gln Lys Ser Gly Phe Tyr Pro Val Gln Asp Ala Gln Tyr
                   165                 170                 175
   Thr Asn Asn Arg Gly Lys Ser Ile Leu Phe Val Trp Gly Ile His His
               180                 185                 190
   Pro Pro Thr Tyr Thr Glu Gln Thr Asn Leu Tyr Ile Arg Asn Asp Thr
           195                 200                 205
   Thr Thr Ser Val Thr Thr Glu Asp Leu Asn Arg Thr Phe Lys Pro Val
       210                 215                 220
   Ile Gly Pro Arg Pro Leu Val Asn Gly Leu Gln Gly Arg Ile Asp Tyr
   225                 230                 235                 240
   Tyr Trp Ser Val Leu Lys Pro Gly Gln Thr Leu Arg Val Arg Ser Asn
                   245                 250                 255
   Gly Asn Leu Ile Ala Pro Trp Tyr Gly His Val Leu Ser Gly Gly Ser
               260                 265                 270
   His Gly Arg Ile Leu Lys Thr Asp Leu Lys Gly Gly Asn Cys Val Val
           275                 280                 285
   Gln Cys Gln Thr Glu Lys Gly Gly Leu Asn Ser Thr Leu Pro Phe His
       290                 295                 300
   Asn Ile Ser Lys Tyr Ala Phe Gly Thr Cys Pro Lys Tyr Val Arg Val
   305                 310                 315                 320
   Asn Ser Leu Lys Leu Ala Val Gly Leu Arg Asn Val Pro Ala Arg Ser
                   325                 330                 335
   Ser Arg Gly Leu Phe Gly Ala Ile Ala Gly Phe Ile Glu Gly Gly Trp
               340                 345                 350
   Pro Gly Leu Val Ala Gly Trp Tyr Gly Phe Gln His Ser Asn Asp Gln
           355                 360                 365
   Gly Val Gly Met Ala Ala Asp Arg Asp Ser Thr Gln Lys Ala Ile Asp
       370                 375                 380
   Lys Ile Thr Ser Lys Val Asn Asn Ile Val Asp Lys Met Asn Lys Gln
   385                 390                 395                 400
   Tyr Glu Ile Ile Asp His Glu Phe Ser Glu Val Glu Thr Arg Leu Asn
                   405                 410                 415
   Met Ile Asn Asn Lys Ile Asp Asp Gln Ile Gln Asp Val Trp Ala Tyr
               420                 425                 430
   Asn Ala Glu Leu Leu Val Leu Leu Glu Asn Gln Lys Thr Leu Asp Glu
           435                 440                 445
   His Asp Ala Asn Val Asn Asn Leu Tyr Asn Lys Val Lys Arg Ala Leu
       450                 455                 460
   Gly Ser Asn Ala Met Glu Asp Gly Lys Gly Cys Phe Glu Leu Tyr His
   465                 470                 475                 480
   Lys Cys Asp Asp Gln Cys Met Glu Thr Ile Arg Asn Gly Thr Tyr Asn
                   485                 490                 495
   Arg Arg Lys Tyr Arg Glu Glu Ser Arg Leu Glu Arg Gln Lys Ile Glu
               500                 505                 510
   Gly Val Lys Leu Glu Ser Glu Gly Thr Tyr Lys Ile Leu Thr Ile Tyr
           515                 520                 525
   Ser Thr Val Ala Ser Ser Leu Val Leu Ala Met Gly Phe Ala Ala Phe
       530                 535                 540
   Leu Phe Trp Ala Met Ser Asn Gly Ser Cys Arg Cys Asn Ile Cys Ile
   545                 550                 555                 560
<210> 60
   <211> 3111
   <212> DNA
   <213> Artificial sequence
<220>
   <223> H5 from A/Indonesia/5/2005 (Construct # 660)
<400> 60
   agaggtaccc cgggctggta tatttatatg ttgtcaaata actcaaaaac cataaaagtt 60
   taagttagca agtgtgtaca tttttacttg aacaaaaata ttcacctact actgttataa 120
   atcattatta aacattagag taaagaaata tggatgataa gaacaagagt agtgatattt 180
   tgacaacaat tttgttgcaa catttgagaa aattttgttg ttctctcttt tcattggtca 240
   aaaacaatag agagagaaaa aggaagaggg agaataaaaa cataatgtga gtatgagaga 300
   gaaagttgta caaaagttgt accaaaatag ttgtacaaat atcattgagg aatttgacaa 360
   aagctacaca aataagggtt aattgctgta aataaataag gatgacgcat tagagagatg 420
   taccattaga gaatttttgg caagtcatta aaaagaaaga ataaattatt tttaaaatta 480
   aaagttgagt catttgatta aacatgtgat tatttaatga attgatgaaa gagttggatt 540
   aaagttgtat tagtaattag aatttggtgt caaatttaat ttgacatttg atcttttcct 600
   atatattgcc ccatagagtc agttaactca tttttatatt tcatagatca aataagagaa 660
   ataacggtat attaatccct ccaaaaaaaa aaaacggtat atttactaaa aaatctaagc 720
   cacgtaggag gataacagga tccccgtagg aggataacat ccaatccaac caatcacaac 780
   aatcctgatg agataaccca ctttaagccc acgcatctgt ggcacatcta cattatctaa 840
   atcacacatt cttccacaca tctgagccac acaaaaacca atccacatct ttatcaccca 900
   ttctataaaa aatcacactt tgtgagtcta cactttgatt cccttcaaac acatacaaag 960
   agaagagact aattaattaa ttaatcatct tgagagaaaa tggagaaaat agtgcttctt 1020
   cttgcaatag tcagtcttgt taaaagtgat cagatttgca ttggttacca tgcaaacaat 1080
   tcaacagagc aggttgacac aatcatggaa aagaacgtta ctgttacaca tgcccaagac 1140
   atactggaaa agacacacaa cgggaagctc tgcgatctag atggagtgaa gcctctaatt 1200
   ttaagagatt gtagtgtagc tggatggctc ctcgggaacc caatgtgtga cgaattcatc 1260
   aatgtaccgg aatggtctta catagtggag aaggccaatc caaccaatga cctctgttac 1320
   ccagggagtt tcaacgacta tgaagaactg aaacacctat tgagcagaat aaaccatttt 1380
   gagaaaattc aaatcatccc caaaagttct tggtccgatc atgaagcctc atcaggagtt 1440
   agctcagcat gtccatacct gggaagtccc tcctttttta gaaatgtggt atggcttatc 1500
   aaaaagaaca gtacataccc aacaataaag aaaagctaca ataataccaa ccaagaggat 1560
   cttttggtac tgtggggaat tcaccatcct aatgatgcgg cagagcagac aaggctatat 1620
   caaaacccaa ccacctatat ttccattggg acatcaacac taaaccagag attggtacca 1680
   aaaatagcta ctagatccaa agtaaacggg caaagtggaa ggatggagtt cttctggaca 1740
   attttaaaac ctaatgatgc aatcaacttc gagagtaatg gaaatttcat tgctccagaa 1800
   tatgcataca aaattgtcaa gaaaggggac tcagcaatta tgaaaagtga attggaatat 1860
   ggtaactgca acaccaagtg tcaaactcca atgggggcga taaactctag tatgccattc 1920
   cacaacatac accctctcac catcggggaa tgccccaaat atgtgaaatc aaacagatta 1980
   gtccttgcaa cagggctcag aaatagccct caaagagaga gcagaagaaa aaagagagga 2040
   ctatttggag ctatagcagg ttttatagag ggaggatggc agggaatggt agatggttgg 2100
   tatgggtacc accatagcaa tgagcagggg agtgggtacg ctgcagacaa agaatccact 2160
   caaaaggcaa tagatggagt caccaataag gtcaactcaa tcattgacaa aatgaacact 2220
   cagtttgagg ccgttggaag ggaatttaat aacttagaaa ggagaataga gaatttaaac 2280
   aagaagatgg aagacgggtt tctagatgtc tggacttata atgccgaact tctggttctc 2340
   atggaaaatg agagaactct agactttcat gactcaaatg ttaagaacct ctacgacaag 2400
   gtccgactac agcttaggga taatgcaaag gagctgggta acggttgttt cgagttctat 2460
   cacaaatgtg ataatgaatg tatggaaagt ataagaaacg gaacgtacaa ctatccgcag 2520
   tattcagaag aagcaagatt aaaaagagag gaaataagtg gggtaaaatt ggaatcaata 2580
   ggaacttacc aaatactgtc aatttattca acagtggcga gttccctagc actggcaatc 2640
   atgatggctg gtctatcttt atggatgtgc tccaatggat cgttacaatg cagaatttgc 2700
   atttaagagc tctaagttaa aatgcttctt cgtctcctat ttataatatg gtttgttatt 2760
   gttaattttg ttcttgtaga agagcttaat taatcgttgt tgttatgaaa tactatttgt 2820
   atgagatgaa ctggtgtaat gtaattcatt tacataagtg gagtcagaat cagaatgttt 2880
   cctccataac taactagaca tgaagacctg ccgcgtacaa ttgtcttata tttgaacaac 2940
   taaaattgaa catcttttgc cacaacttta taagtggtta atatagctca aatatatggt 3000
   caagttcaat agattaataa tggaaatatc agttatcgaa attcattaac aatcaactta 3060
   acgttattaa ctactaattt tatatcatcc cctttgataa atgatagtac a 3111
<210> 61
   <211> 3123
   <212> DNA
   <213> Artificial sequence
<220>
   <223> H1 from A/New Caledonia/20/1999 (Construct # 540)
<400> 61
   agaggtaccc cgggctggta tatttatatg ttgtcaaata actcaaaaac cataaaagtt     60
   taagttagca agtgtgtaca tttttacttg aacaaaaata ttcacctact actgttataa    120
   atcattatta aacattagag taaagaaata tggatgataa gaacaagagt agtgatattt    180
   tgacaacaat tttgttgcaa catttgagaa aattttgttg ttctctcttt tcattggtca    240
   aaaacaatag agagagaaaa aggaagaggg agaataaaaa cataatgtga gtatgagaga    300
   gaaagttgta caaaagttgt accaaaatag ttgtacaaat atcattgagg aatttgacaa    360
   aagctacaca aataagggtt aattgctgta aataaataag gatgacgcat tagagagatg 420
   taccattaga gaatttttgg caagtcatta aaaagaaaga ataaattatt tttaaaatta 480
   aaagttgagt catttgatta aacatgtgat tatttaatga attgatgaaa gagttggatt 540
   aaagttgtat tagtaattag aatttggtgt caaatttaat ttgacatttg atcttttcct 600
   atatattgcc ccatagagtc agttaactca tttttatatt tcatagatca aataagagaa 660
   ataacggtat attaatccct ccaaaaaaaa aaaacggtat atttactaaa aaatctaagc 720
   cacgtaggag gataacagga tccccgtagg aggataacat ccaatccaac caatcacaac 780
   aatcctgatg agataaccca ctttaagccc acgcatctgt ggcacatcta cattatctaa 840
   atcacacatt cttccacaca tctgagccac acaaaaacca atccacatct ttatcaccca 900
   ttctataaaa aatcacactt tgtgagtcta cactttgatt cccttcaaac acatacaaag 960
   agaagagact aattaattaa ttaatcatct tgagagaaaa tggcgaaaaa cgttgcgatt 1020
   ttcggcttat tgttttctct tcttgtgttg gttccttctc agatcttcgc tgacacaata 1080
   tgtataggct accatgccaa caactcaacc gacactgttg acacagtact tgagaagaat 1140
   gtgacagtga cacactctgt caacctactt gaggacagtc acaatggaaa actatgtcta 1200
   ctaaaaggaa tagccccact acaattgggt aattgcagcg ttgccggatg gatcttagga 1260
   aacccagaat gcgaattact gatttccaag gaatcatggt cctacattgt agaaacacca 1320
   aatcctgaga atggaacatg ttacccaggg tatttcgccg actatgagga actgagggag 1380
   caattgagtt cagtatcttc atttgagaga ttcgaaatat tccccaaaga aagctcatgg 1440
   cccaaccaca ccgtaaccgg agtatcagca tcatgctccc ataatgggaa aagcagtttt 1500
   tacagaaatt tgctatggct gacggggaag aatggtttgt acccaaacct gagcaagtcc 1560
   tatgtaaaca acaaagagaa agaagtcctt gtactatggg gtgttcatca cccgcctaac 1620
   atagggaacc aaagggcact ctatcataca gaaaatgctt atgtctctgt agtgtcttca 1680
   cattatagca gaagattcac cccagaaata gccaaaagac ccaaagtaag agatcaggaa 1740
   ggaagaatca actactactg gactctgctg gaacctgggg atacaataat atttgaggca 1800
   aatggaaatc taatagcgcc atggtatgct tttgcactga gtagaggctt tggatcagga 1860
   atcatcacct caaatgcacc aatggatgaa tgtgatgcga agtgtcaaac acctcaggga 1920
   gctataaaca gcagtcttcc tttccagaat gtacacccag tcacaatagg agagtgtcca 1980
   aagtatgtca ggagtgcaaa attaaggatg gttacaggac taaggaacat cccatccatt 2040
   caatccagag gtttgtttgg agccattgcc ggtttcattg aaggggggtg gactggaatg    2100
   gtagatgggt ggtatggtta tcatcatcag aatgagcaag gatctggcta tgctgcagat 2160
   caaaaaagta cacaaaatgc cattaacggg attacaaaca aggtcaattc tgtaattgag 2220
   aaaatgaaca ctcaattcac agctgtgggc aaagagttca acaaattgga aagaaggatg 2280
   gaaaacttaa ataaaaaagt tgatgatggg tttctagaca tttggacata taatgcagaa 2340
   ttgttggttc tactggaaaa tgaaaggact ttggatttcc atgactccaa tgtgaagaat 2400
   ctgtatgaga aagtaaaaag ccaattaaag aataatgcca aagaaatagg aaacgggtgt 2460
   tttgagttct atcacaagtg taacaatgaa tgcatggaga gtgtgaaaaa tggtacctat 2520
   gactatccaa aatattccga agaatcaaag ttaaacaggg agaaaattga tggagtgaaa 2580
   ttggaatcaa tgggagtata ccagattctg gcgatctact caactgtcgc cagttccctg 2640
   gttcttttgg tctccctggg ggcaatcagc ttctggatgt gttccaatgg gtctttgcag 2700
   tgtagaatat gcatctaaga gctctaagtt aaaatgcttc ttcgtctcct atttataata 2760
   tggtttgtta ttgttaattt tgttcttgta gaagagctta attaatcgtt gttgttatga 2820
   aatactattt gtatgagatg aactggtgta atgtaattca tttacataag tggagtcaga 2880
   atcagaatgt ttcctccata actaactaga catgaagacc tgccgcgtac aattgtctta 2940
   tatttgaaca actaaaattg aacatctttt gccacaactt tataagtggt taatatagct 3000
   caaatatatg gtcaagttca atagattaat aatggaaata tcagttatcg aaattcatta 3060
   acaatcaact taacgttatt aactactaat tttatatcat cccctttgat aaatgatagt 3120
   aca 3123
<210> 62
   <211> 3088
   <212> DNA
   <213> Artificial
<220>
   <223> H1 from A/Brisbane/59/2007 (construct #774)
<400> 62
   ctggtatatt tatatgttgt caaataactc aaaaaccata aaagtttaag ttagcaagtg 60
   tgtacatttt tacttgaaca aaaatattca cctactactg ttataaatca ttattaaaca 120
   ttagagtaaa gaaatatgga tgataagaac aagagtagtg atattttgac aacaattttg 180
   ttgcaacatt tgagaaaatt ttgttgttct ctcttttcat tggtcaaaaa caatagagag 240
   agaaaaagga agagggagaa taaaaacata atgtgagtat gagagagaaa gttgtacaaa 300
   agttgtacca aaatagttgt acaaatatca ttgaggaatt tgacaaaagc tacacaaata 360
   agggttaatt gctgtaaata aataaggatg acgcattaga gagatgtacc attagagaat 420
   ttttggcaag tcattaaaaa gaaagaataa attattttta aaattaaaag ttgagtcatt 480
   tgattaaaca tgtgattatt taatgaattg atgaaagagt tggattaaag ttgtattagt 540
   aattagaatt tggtgtcaaa tttaatttga catttgatct tttcctatat attgccccat 600
   agagtcagtt aactcatttt tatatttcat agatcaaata agagaaataa cggtatatta 660
   atccctccaa aaaaaaaaaa cggtatattt actaaaaaat ctaagccacg taggaggata 720
   acaggatccc cgtaggagga taacatccaa tccaaccaat cacaacaatc ctgatgagat 780
   aacccacttt aagcccacgc atctgtggca catctacatt atctaaatca cacattcttc 840
   cacacatctg agccacacaa aaaccaatcc acatctttat cacccattct ataaaaaatc 900
   acactttgtg agtctacact ttgattccct tcaaacacat acaaagagaa gagactaatt 960
   aattaattaa tcatcttgag agaaaatgaa agtaaaacta ctggtcctgt tatgcacatt 1020
   tacagctaca tatgcagaca caatatgtat aggctaccat gctaacaact cgaccgacac 1080
   tgttgacaca gtacttgaaa agaatgtgac agtgacacac tctgtcaacc tgcttgagaa 1140
   cagtcacaat ggaaaactat gtctattaaa aggaatagcc ccactacaat tgggtaattg 1200
   cagcgttgcc gggtggatct taggaaaccc agaatgcgaa ttactgattt ccaaggagtc 1260
   atggtcctac attgtagaaa aaccaaatcc tgagaatgga acatgttacc cagggcattt 1320
   cgctgactat gaggaactga gggagcaatt gagttcagta tcttcatttg agaggttcga 1380
   aatattcccc aaagaaagct catggcccaa ccacaccgta accggagtgt cagcatcatg 1440
   ctcccataat ggggaaagca gtttttacag aaatttgcta tggctgacgg ggaagaatgg 1500
   tttgtaccca aacctgagca agtcctatgc aaacaacaaa gaaaaagaag tccttgtact 1560
   atggggtgtt catcacccgc caaacatagg tgaccaaaag gccctctatc atacagaaaa 1620
   tgcttatgtc tctgtagtgt cttcacatta tagcagaaaa ttcaccccag aaatagccaa 1680
   aagacccaaa gtaagagatc aagaaggaag aatcaattac tactggactc tgcttgaacc 1740
   cggggataca ataatatttg aggcaaatgg aaatctaata gcgccaagat atgctttcgc 1800
   actgagtaga ggctttggat caggaatcat caactcaaat gcaccaatgg ataaatgtga 1860
   tgcgaagtgc caaacacctc agggagctat aaacagcagt cttcctttcc agaacgtaca 1920
   cccagtcaca ataggagagt gtccaaagta tgtcaggagt gcaaaattaa ggatggttac 1980
   aggactaagg aacatcccat ccattcaatc cagaggtttg tttggagcca ttgccggttt 2040
   cattgaaggg gggtggactg gaatggtaga tggttggtat ggttatcatc atcagaatga 2100
   gcaaggatct ggctatgctg cagatcaaaa aagcacacaa aatgccatta atgggattac 2160
   aaacaaggtc aattctgtaa ttgagaaaat gaacactcaa ttcacagcag tgggcaaaga 2220
   gttcaacaaa ttggaaagaa ggatggaaaa cttgaataaa aaagttgatg atgggtttat 2280
   agacatttgg acatataatg cagaactgtt ggttctactg gaaaatgaaa ggactttgga 2340
   tttccatgac tccaatgtga agaatctgta tgagaaagta aaaagccagt taaagaataa 2400
   tgctaaagaa ataggaaatg ggtgttttga gttctatcac aagtgtaacg atgaatgcat 2460
   ggagagtgta aagaatggaa cttatgacta tccaaaatat tccgaagaat caaagttaaa 2520
   cagggagaaa attgatggag tgaaattgga atcaatggga gtctatcaga ttctggcgat 2580
   ctactcaaca gtcgccagtt ctctggttct tttggtctcc ctgggggcaa tcagcttctg 2640
   gatgtgttcc aatgggtctt tacagtgtag aatatgcatc taagagctct aagttaaaat 2700
   gcttcttcgt ctcctattta taatatggtt tgttattgtt aattttgttc ttgtagaaga   2760
   gcttaattaa tcgttgttgt tatgaaatac tatttgtatg agatgaactg gtgtaatgta   2820
   attcatttac ataagtggag tcagaatcag aatgtttcct ccataactaa ctagacatga   2880
   agacctgccg cgtacaattg tcttatattt gaacaactaa aattgaacat cttttgccac   2940
   aactttataa gtggttaata tagctcaaat atatggtcaa gttcaataga ttaataatgg   3000
   aaatatcagt tatcgaaatt cattaacaat caacttaacg ttattaacta ctaattttat   3060
   atcatcccct ttgataaatg atagtaca                                      3088
<210> 63
   <211> 3102
   <212> DNA
   <213> Artificial
<220>
   <223> H1 from A/Solomon Islands/3/2006 (H1N1) (Construct # 775)
<400> 63
   agaggtaccc cgggctggta tatttatatg ttgtcaaata actcaaaaac cataaaagtt 60
   taagttagca agtgtgtaca tttttacttg aacaaaaata ttcacctact actgttataa 120
   atcattatta aacattagag taaagaaata tggatgataa gaacaagagt agtgatattt 180
   tgacaacaat tttgttgcaa catttgagaa aattttgttg ttctctcttt tcattggtca 240
   aaaacaatag agagagaaaa aggaagaggg agaataaaaa cataatgtga gtatgagaga 300
   gaaagttgta caaaagttgt accaaaatag ttgtacaaat atcattgagg aatttgacaa 360
   aagctacaca aataagggtt aattgctgta aataaataag gatgacgcat tagagagatg 420
   taccattaga gaatttttgg caagtcatta aaaagaaaga ataaattatt tttaaaatta 480
   aaagttgagt catttgatta aacatgtgat tatttaatga attgatgaaa gagttggatt 540
   aaagttgtat tagtaattag aatttggtgt caaatttaat ttgacatttg atcttttcct 600
   atatattgcc ccatagagtc agttaactca tttttatatt tcatagatca aataagagaa 660
   ataacggtat attaatccct ccaaaaaaaa aaaacggtat atttactaaa aaatctaagc 720
   cacgtaggag gataacagga tccccgtagg aggataacat ccaatccaac caatcacaac 780
   aatcctgatg agataaccca ctttaagccc acgcatctgt ggcacatcta cattatctaa 840
   atcacacatt cttccacaca tctgagccac acaaaaacca atccacatct ttatcaccca 900
   ttctataaaa aatcacactt tgtgagtcta cactttgatt cccttcaaac acatacaaag 960
   agaagagact aattaattaa ttaatcatct tgagagaaaa tgaaagtaaa actactggtc 1020
   ctgttatgca catttacagc tacatatgca gacacaatat gtataggcta ccatgccaac 1080
   aactcaaccg acactgttga cacagtactt gagaagaatg tgacagtgac acactctgtc 1140
   aacctgcttg aggacagtca caatggaaaa ttatgtctat taaaaggaat agccccacta 1200
   caattgggta attgcagcgt tgccggatgg atcttaggaa acccagaatg cgaattactg 1260
   atttccaggg aatcatggtc ctacattgta gaaaaaccaa atcctgagaa tggaacatgt 1320
   tacccagggc atttcgccga ctatgaggaa ctgagggagc aattgagttc agtatcttca 1380
   tttgagagat tcgaaatatt ccccaaagaa agctcatggc ccaaccacac cacaaccgga 1440
   gtatcagcat catgctccca taatggggaa agcagttttt acaaaaattt gctatggctg 1500
   acggggaaga atggtttgta cccaaacctg agcaagtcct atgcaaacaa caaagagaaa 1560
   gaagtccttg tactatgggg tgttcatcac ccgcctaaca taggtgacca aagggctctc 1620
   tatcataaag aaaatgctta tgtctctgta gtgtcttcac attatagcag aaaattcacc 1680
   ccagaaatag ccaaaagacc caaagtaaga gatcaagaag gaagaatcaa ctactactgg 1740
   actctacttg aacccgggga tacaataata tttgaggcaa atggaaatct aatagcgcca 1800
   agatatgctt tcgcactgag tagaggcttt ggatcaggaa tcatcaactc aaatgcacca 1860
   atggatgaat gtgatgcgaa gtgccaaaca cctcagggag ctataaacag cagtcttcct 1920
   ttccagaatg tacaccctgt cacaatagga gagtgtccaa agtatgtcag gagtgcaaaa 1980
   ttaaggatgg ttacaggact aaggaacatc ccatccattc aatccagagg tttgtttgga 2040
   gccattgccg gtttcattga aggggggtgg actggaatgg tagatggttg gtatggttat 2100
   catcatcaga atgagcaagg atctggctat gctgcagatc aaaaaagcac acaaaatgcc 2160
   attaatggga ttacaaacaa ggtcaattct gtaattgaga aaatgaacac tcaattcaca 2220
   gctgtgggca aagagttcaa caaattggaa agaaggatgg aaaacttaaa taaaaaagtt 2280
   gatgatgggt ttatagacat ttggacatat aatgcagaat tgttggttct actggaaaat 2340
   gaaaggactt tggatttcca tgactccaat gtgaagaatc tgtatgagaa agtaaaaagc 2400
   caattaaaga ataatgccaa agaaatagga aatgggtgtt ttgagttcta tcataagtgt 2460
   aacgatgaat gcatggagag tgtaaaaaat ggaacttatg actatccaaa atattccgaa 2520
   gaatcaaagt taaacaggga gaaaattgat ggagtgaaat tggaatcaat gggagtctat 2580
   cagattctgg cgatctactc aacagtcgcc agttctctgg ttcttttggt ctccctgggg 2640
   gcaatcagct tctggatgtg ttccaatggg tctttgcagt gtagaatatg catctgagag 2700
   ctctaagtta aaatgcttct tcgtctccta tttataatat ggtttgttat tgttaatttt 2760
   gttcttgtag aagagcttaa ttaatcgttg ttgttatgaa atactatttg tatgagatga 2820
   actggtgtaa tgtaattcat ttacataagt ggagtcagaa tcagaatgtt tcctccataa 2880
   ctaactagac atgaagacct gccgcgtaca attgtcttat atttgaacaa ctaaaattga 2940
   acatcttttg ccacaacttt ataagtggtt aatatagctc aaatatatgg tcaagttcaa 3000
   tagattaata atggaaatat cagttatcga aattcattaa caatcaactt aacgttatta 3060
   actactaatt ttatatcatc ccctttgata aatgatagta ca 3102
<210> 64
   <211> 3093
   <212> DNA
   <213> Artificial sequence
<220>
   <223> H2 from A/Singapore/1/57 (H2N2) (construct # 780)
<400> 64
   agaggtaccc cgggctggta tatttatatg ttgtcaaata actcaaaaac cataaaagtt 60
   taagttagca agtgtgtaca tttttacttg aacaaaaata ttcacctact actgttataa 120
   atcattatta aacattagag taaagaaata tggatgataa gaacaagagt agtgatattt 180
   tgacaacaat tttgttgcaa catttgagaa aattttgttg ttctctcttt tcattggtca 240
   aaaacaatag agagagaaaa aggaagaggg agaataaaaa cataatgtga gtatgagaga 300
   gaaagttgta caaaagttgt accaaaatag ttgtacaaat atcattgagg aatttgacaa 360
   aagctacaca aataagggtt aattgctgta aataaataag gatgacgcat tagagagatg 420
   taccattaga gaatttttgg caagtcatta aaaagaaaga ataaattatt tttaaaatta 480
   aaagttgagt catttgatta aacatgtgat tatttaatga attgatgaaa gagttggatt 540
   aaagttgtat tagtaattag aatttggtgt caaatttaat ttgacatttg atcttttcct 600
   atatattgcc ccatagagtc agttaactca tttttatatt tcatagatca aataagagaa 660
   ataacggtat attaatccct ccaaaaaaaa aaaacggtat atttactaaa aaatctaagc 720
   cacgtaggag gataacagga tccccgtagg aggataacat ccaatccaac caatcacaac 780
   aatcctgatg agataaccca ctttaagccc acgcatctgt ggcacatcta cattatctaa 840
   atcacacatt cttccacaca tctgagccac acaaaaacca atccacatct ttatcaccca 900
   ttctataaaa aatcacactt tgtgagtcta cactttgatt cccttcaaac acatacaaag 960
   agaagagact aattaattaa ttaatcatct tgagagaaaa tggccatcat ttatctaatt 1020
   ctcctgttca cagcagtgag aggggaccaa atatgcattg gataccatgc caataattcc 1080
   acagagaagg tcgacacaat tctagagcgg aacgtcactg tgactcatgc caaggacatt 1140
   cttgagaaga cccataacgg aaagttatgc aaactaaacg gaatccctcc acttgaacta 1200
   ggggactgta gcattgccgg atggctcctt ggaaatccag aatgtgatag gcttctaagt 1260
   gtgccagaat ggtcctatat aatggagaaa gaaaacccga gagacggttt gtgttatcca 1320
   ggcagcttca atgattatga agaattgaaa catctcctca gcagcgtgaa acatttcgag 1380
   aaagtaaaga ttctgcccaa agatagatgg acacagcata caacaactgg aggttcacgg 1440
   gcctgcgcgg tgtctggtaa tccatcattc ttcaggaaca tggtctggct gacaaagaaa 1500
   gaatcaaatt atccggttgc caaaggatcg tacaacaata caagcggaga acaaatgcta 1560
   ataatttggg gggtgcacca tcccaatgat gagacagaac aaagaacatt gtaccagaat 1620
   gtgggaacct atgtttccgt aggcacatca acattgaaca aaaggtcaac cccagacata 1680
   gcaacaaggc ctaaagtgaa tggactagga agtagaatgg agttctcttg gaccctattg 1740
   gatatgtggg acaccataaa ttttgagagt actggtaatc taattgcacc agagtatgga 1800
   ttcaaaatat cgaaaagagg tagttcaggg atcatgaaaa cagaaggaac acttgagaac 1860
   tgtgagacca aatgccaaac tcctttggga gcaataaata caacattgcc ttttcacaat 1920
   gtccacccac tgacaatagg tgagtgcccc aaatatgtaa aatcggagaa gttggtctta 1980
   gcaacaggac taaggaatgt tccccagatt gaatcaagag gattgtttgg ggcaatagct 2040
   ggttttatag aaggaggatg gcaaggaatg gttgatggtt ggtatggata ccatcacagc 2100
   aatgaccagg gatcagggta tgcagcagac aaagaatcca ctcaaaaggc atttgatgga 2160
   atcaccaaca aggtaaattc tgtgattgaa aagatgaaca cccaatttga agctgttggg 2220
   aaagagttca gtaacttaga gagaagactg gagaacttga acaaaaagat ggaagacggg 2280
   tttctagatg tgtggacata caatgctgag cttctagttc tgatggaaaa tgagaggaca 2340
   cttgactttc atgattctaa tgtcaagaat ctgtatgata aagtcagaat gcagctgaga 2400
   gacaacgtca aagaactagg aaatggatgt tttgaatttt atcacaaatg tgatgatgaa 2460
   tgcatgaata gtgtgaaaaa cgggacgtat gattatccca agtatgaaga agagtctaaa 2520
   ctaaatagaa atgaaatcaa aggggtaaaa ttgagcagca tgggggttta tcaaatcctt 2580
   gccatttatg ctacagtagc aggttctctg tcactggcaa tcatgatggc tgggatctct 2640
   ttctggatgt gctccaacgg gtctctgcag tgcaggatct gcatatgaga gctctaagtt 2700
   aaaatgcttc ttcgtctcct atttataata tggtttgtta ttgttaattt tgttcttgta 2760
   gaagagctta attaatcgtt gttgttatga aatactattt gtatgagatg aactggtgta 2820
   atgtaattca tttacataag tggagtcaga atcagaatgt ttcctccata actaactaga 2880
   catgaagacc tgccgcgtac aattgtctta tatttgaaca actaaaattg aacatctttt 2940
   gccacaactt tataagtggt taatatagct caaatatatg gtcaagttca atagattaat 3000
   aatggaaata tcagttatcg aaattcatta acaatcaact taacgttatt aactactaat 3060
   tttatatcat cccctttgat aaatgatagt aca 3093
<210> 65
   <211> 3108
   <212> DNA
   <213> Artificial sequence
<220>
   <223> H5 from A/Anhui/1/2005 (H5N1) (Construct# 781)
<400> 65
   agaggtaccc cgggctggta tatttatatg ttgtcaaata actcaaaaac cataaaagtt 60
   taagttagca agtgtgtaca tttttacttg aacaaaaata ttcacctact actgttataa 120
   atcattatta aacattagag taaagaaata tggatgataa gaacaagagt agtgatattt 180
   tgacaacaat tttgttgcaa catttgagaa aattttgttg ttctctcttt tcattggtca 240
   aaaacaatag agagagaaaa aggaagaggg agaataaaaa cataatgtga gtatgagaga 300
   gaaagttgta caaaagttgt accaaaatag ttgtacaaat atcattgagg aatttgacaa 360
   aagctacaca aataagggtt aattgctgta aataaataag gatgacgcat tagagagatg 420
   taccattaga gaatttttgg caagtcatta aaaagaaaga ataaattatt tttaaaatta 480
   aaagttgagt catttgatta aacatgtgat tatttaatga attgatgaaa gagttggatt 540
   aaagttgtat tagtaattag aatttggtgt caaatttaat ttgacatttg atcttttcct 600
   atatattgcc ccatagagtc agttaactca tttttatatt tcatagatca aataagagaa 660
   ataacggtat attaatccct ccaaaaaaaa aaaacggtat atttactaaa aaatctaagc 720
   cacgtaggag gataacagga tccccgtagg aggataacat ccaatccaac caatcacaac 780
   aatcctgatg agataaccca ctttaagccc acgcatctgt ggcacatcta cattatctaa 840
   atcacacatt cttccacaca tctgagccac acaaaaacca atccacatct ttatcaccca 900
   ttctataaaa aatcacactt tgtgagtcta cactttgatt cccttcaaac acatacaaag 960
   agaagagact aattaattaa ttaatcatct tgagagaaaa tggagaaaat agtgcttctt 1020
   cttgcaatag tcagccttgt taaaagtgat cagatttgca ttggttacca tgcaaacaac 1080
   tcgacagagc aggttgacac aataatggaa aagaacgtta ctgttacaca tgcccaagac 1140
   atactggaaa agacacacaa cgggaagctc tgcgatctag atggagtgaa gcctctgatt 1200
   ttaagagatt gtagtgtagc tggatggctc ctcggaaacc caatgtgtga cgagttcatc 1260
   aatgtgccgg aatggtctta catagtggag aaggccaacc cagccaatga cctctgttac 1320
   ccagggaatt tcaacgacta tgaagaactg aaacacctat tgagcagaat aaaccatttt 1380
   gagaaaattc agatcatccc caaaagttct tggtccgatc atgaagcctc atcaggggtc 1440
   agctcagcat gtccatacca gggaacgccc tcctttttca gaaatgtggt atggcttatc 1500
   aaaaagaaca atacataccc aacaataaag agaagctaca ataataccaa ccaggaagat 1560
   cttttgatac tgtgggggat tcatcattct aatgatgcgg cagagcagac aaagctctat 1620
   caaaacccaa ccacctatat ttccgttggg acatcaacac taaaccagag attggtacca 1680
   aaaatagcta ctagatccaa agtaaacggg caaagtggaa ggatggattt cttctggaca 1740
   attttaaaac cgaatgatgc aatcaacttc gagagtaatg gaaatttcat tgctccagaa 1800
   tatgcataca aaattgtcaa gaaaggggac tcagcaattg ttaaaagtga agtggaatat 1860
   ggtaactgca atacaaagtg tcaaactcca ataggggcga taaactctag tatgccattc 1920
   cacaacatac accctctcac catcggggaa tgccccaaat atgtgaaatc aaacaaatta 1980
   gtccttgcga ctgggctcag aaatagtcct ctaagagaaa gaagaagaaa aagaggacta 2040
   tttggagcta tagcagggtt tatagaggga ggatggcagg gaatggtaga tggttggtat 2100
   gggtaccacc atagcaatga gcaggggagt gggtacgctg cagacaaaga atccactcaa 2160
   aaggcaatag atggagtcac caataaggtc aactcgatca ttgacaaaat gaacactcag 2220
   tttgaggccg ttggaaggga atttaataac ttagaaagga gaatagagaa tttaaacaag 2280
   aaaatggaag acggattcct agatgtctgg acttataatg ctgaacttct ggttctcatg 2340
   gaaaatgaga gaactctaga cttccatgat tcaaatgtca agaaccttta cgacaaggtc 2400
   cgactacagc ttagggataa tgcaaaggag ctgggtaacg gttgtttcga gttctatcac 2460
   aaatgtgata atgaatgtat ggaaagtgta agaaacggaa cgtatgacta cccgcagtat 2520
   tcagaagaag caagattaaa aagagaggaa ataagtggag taaaattgga atcaatagga 2580
   acttaccaaa tactgtcaat ttattcaaca gttgcgagtt ctctagcact ggcaatcatg 2640
   gtggctggtc tatctttgtg gatgtgctcc aatgggtcgt tacaatgcag aatttgcatt 2700
   taagagctct aagttaaaat gcttcttcgt ctcctattta taatatggtt tgttattgtt 2760
   aattttgttc ttgtagaaga gcttaattaa tcgttgttgt tatgaaatac tatttgtatg 2820
   agatgaactg gtgtaatgta attcatttac ataagtggag tcagaatcag aatgtttcct 2880
   ccataactaa ctagacatga agacctgccg cgtacaattg tcttatattt gaacaactaa 2940
   aattgaacat cttttgccac aactttataa gtggttaata tagctcaaat atatggtcaa 3000
   gttcaataga ttaataatgg aaatatcagt tatcgaaatt cattaacaat caacttaacg 3060
   ttattaacta ctaattttat atcatcccct ttgataaatg atagtaca 3108
<210> 66
   <211> 3111
   <212> DNA
   <213> Artificial sequence
<220>
   <223> H5 from A/Vietnam/1194/2004 (H5N1) (Construct # 782)
<400> 66
   agaggtaccc cgggctggta tatttatatg ttgtcaaata actcaaaaac cataaaagtt      60
   taagttagca agtgtgtaca tttttacttg aacaaaaata ttcacctact actgttataa     120
   atcattatta aacattagag taaagaaata tggatgataa gaacaagagt agtgatattt     180
   tgacaacaat tttgttgcaa catttgagaa aattttgttg ttctctcttt tcattggtca     240
   aaaacaatag agagagaaaa aggaagaggg agaataaaaa cataatgtga gtatgagaga     300
   gaaagttgta caaaagttgt accaaaatag ttgtacaaat atcattgagg aatttgacaa     360
   aagctacaca aataagggtt aattgctgta aataaataag gatgacgcat tagagagatg     420
   taccattaga gaatttttgg caagtcatta aaaagaaaga ataaattatt tttaaaatta     480
   aaagttgagt catttgatta aacatgtgat tatttaatga attgatgaaa gagttggatt     540
   aaagttgtat tagtaattag aatttggtgt caaatttaat ttgacatttg atcttttcct     600
   atatattgcc ccatagagtc agttaactca tttttatatt tcatagatca aataagagaa     660
   ataacggtat attaatccct ccaaaaaaaa aaaacggtat atttactaaa aaatctaagc     720
   cacgtaggag gataacagga tccccgtagg aggataacat ccaatccaac caatcacaac 780
   aatcctgatg agataaccca ctttaagccc acgcatctgt ggcacatcta cattatctaa 840
   atcacacatt cttccacaca tctgagccac acaaaaacca atccacatct ttatcaccca 900
   ttctataaaa aatcacactt tgtgagtcta cactttgatt cccttcaaac acatacaaag 960
   agaagagact aattaattaa ttaatcatct tgagagaaaa tggagaaaat agtgcttctt 1020
   tttgcaatag tcagtcttgt taaaagtgat cagatttgca ttggttacca tgcaaacaac 1080
   tcgacagagc aggttgacac aataatggaa aagaacgtta ctgttacaca tgcccaagac 1140
   atactggaaa agacacacaa tgggaagctc tgcgatctag atggagtgaa gcctctaatt 1200
   ttgagagatt gtagtgtagc tggatggctc ctcggaaacc caatgtgtga cgagttcatc 1260
   aatgtgccgg aatggtctta catagtggag aaggccaatc cagtcaatga cctctgttac 1320
   ccaggggatt tcaatgacta tgaagaattg aaacacctat tgagcagaat aaaccatttt 1380
   gagaaaattc agatcatccc caaaagttct tggtccagtc atgaagcctc attgggggtc 1440
   agctcagcat gtccatacca gggaaagtcc tcctttttca gaaatgtggt atggcttatc 1500
   aaaaagaaca gtacataccc aacaataaag aggagctaca ataataccaa ccaagaagat 1560
   cttttggtac tgtgggggat tcaccatcct aatgatgcgg cagagcagac aaagctctat 1620
   caaaacccaa ccacctatat ttccgttggg acatctacac taaaccagag attggtacca 1680
   agaatagcta ctagatccaa agtaaacggg caaagtggaa ggatggagtt cttctggaca 1740
   attttaaaac cgaatgatgc aatcaacttc gagagtaatg gaaatttcat tgctccagaa 1800
   tatgcataca aaattgtcaa gaaaggggac tcaacaatta tgaaaagtga attggaatat 1860
   ggtaactgca ataccaagtg tcaaactcca atgggggcga taaactctag catgccattc 1920
   cacaatatac accctctcac catcggggaa tgccccaaat atgtgaaatc aaacagatta 1980
   gtccttgcga ctgggctcag aaatagccct caaagagaga gaagaagaaa aaagagagga 2040
   ttatttggag ctatagcagg ttttatagag ggaggatggc agggaatggt agatggttgg 2100
   tatgggtacc accatagcaa cgagcagggg agtgggtacg ctgcagacaa agaatccact 2160
   caaaaggcaa tagatggagt caccaataag gtcaactcga ttattgacaa aatgaacact 2220
   cagtttgagg ccgttggaag ggaatttaac aacttagaaa ggagaataga gaatttaaac 2280
   aagaagatgg aagacgggtt cctagatgtc tggacttata atgctgaact tctagttctc 2340
   atggaaaacg agagaactct agactttcat gactcaaatg tcaagaacct ttacgacaag 2400
   gtccgactac agcttaggga taatgcaaag gagctgggta acggttgttt cgagttctat 2460
   cataaatgtg ataatgaatg tatggaaagt gtaagaaacg gaacgtatga ctacccgcag 2520
   tattcagaag aagcaagact aaaaagagag gaaataagtg gagtaaaatt ggaatcaata 2580
   ggaatttacc aaatattgtc aatttattct acagtggcca gctccctagc actggcaatc 2640
   atggtagctg gtctatcctt atggatgtgc tccaatgggt cgttacaatg cagaatttgc 2700
   atttaagagc tctaagttaa aatgcttctt cgtctcctat ttataatatg gtttgttatt   2760
   gttaattttg ttcttgtaga agagcttaat taatcgttgt tgttatgaaa tactatttgt   2820
   atgagatgaa ctggtgtaat gtaattcatt tacataagtg gagtcagaat cagaatgttt   2880
   cctccataac taactagaca tgaagacctg ccgcgtacaa ttgtcttata tttgaacaac   2940
   taaaattgaa catcttttgc cacaacttta taagtggtta atatagctca aatatatggt   3000
   caagttcaat agattaataa tggaaatatc agttatcgaa attcattaac aatcaactta   3060
   acgttattaa ctactaattt tatatcatcc cctttgataa atgatagtac a            3111
<210> 67
   <211> 3105
   <212> DNA
   <213> Artificial
<220>
   <223> H6 from A/Teal/Hong Kong/W312/97 (H6N1) (Construct # 783)
<400> 67
   agaggtaccc cgggctggta tatttatatg ttgtcaaata actcaaaaac cataaaagtt 60
   taagttagca agtgtgtaca tttttacttg aacaaaaata ttcacctact actgttataa 120
   atcattatta aacattagag taaagaaata tggatgataa gaacaagagt agtgatattt 180
   tgacaacaat tttgttgcaa catttgagaa aattttgttg ttctctcttt tcattggtca 240
   aaaacaatag agagagaaaa aggaagaggg agaataaaaa cataatgtga gtatgagaga 300
   gaaagttgta caaaagttgt accaaaatag ttgtacaaat atcattgagg aatttgacaa 360
   aagctacaca aataagggtt aattgctgta aataaataag gatgacgcat tagagagatg 420
   taccattaga gaatttttgg caagtcatta aaaagaaaga ataaattatt tttaaaatta 480
   aaagttgagt catttgatta aacatgtgat tatttaatga attgatgaaa gagttggatt 540
   aaagttgtat tagtaattag aatttggtgt caaatttaat ttgacatttg atcttttcct 600
   atatattgcc ccatagagtc agttaactca tttttatatt tcatagatca aataagagaa 660
   ataacggtat attaatccct ccaaaaaaaa aaaacggtat atttactaaa aaatctaagc 720
   cacgtaggag gataacagga tccccgtagg aggataacat ccaatccaac caatcacaac 780
   aatcctgatg agataaccca ctttaagccc acgcatctgt ggcacatcta cattatctaa 840
   atcacacatt cttccacaca tctgagccac acaaaaacca atccacatct ttatcaccca 900
   ttctataaaa aatcacactt tgtgagtcta cactttgatt cccttcaaac acatacaaag 960
   agaagagact aattaattaa ttaatcatct tgagagaaaa tgattgcaat cattgtaata 1020
   gcaatactgg cagcagccgg aaagtcagac aagatctgca ttgggtatca tgccaacaat 1080
   tcaacaacac aggtagatac gatacttgag aagaatgtga ctgtcacaca ctcaattgaa 1140
   ttgctggaaa atcagaagga agaaagattc tgcaagatat tgaacaaggc ccctctcgac 1200
   ttaagggaat gtaccataga gggttggatc ttggggaatc cccaatgcga cctattgctt 1260
   ggtgatcaaa gctggtcata cattgtggaa agacctactg ctcaaaacgg gatctgctac 1320
   ccaggaacct taaatgaggt agaagaactg agggcactta ttggatcagg agaaagggta 1380
   gagagatttg agatgtttcc ccaaagcacc tggcaaggag ttgacaccaa cagtggaaca 1440
   acaagatcct gcccttattc tactggtgcg tctttctaca gaaacctcct atggataata 1500
   aaaaccaaga cagcagaata tccagtaatt aagggaattt acaacaacac tggaacccag 1560
   ccaatcctct atttctgggg tgtgcatcat cctcctaaca ccgacgagca agatactctg 1620
   tatggctctg gtgatcgata cgttagaatg ggaactgaaa gcatgaattt tgccaagagt 1680
   ccggaaattg cggcaaggcc tgctgtgaat ggacaaagag gcagaattga ttattattgg 1740
   tcggttttaa aaccagggga aaccttgaat gtggaatcta atggaaatct aatcgcccct 1800
   tggtatgcat acaaatttgt caacacaaat agtaaaggag ccgtcttcag gtcagattta 1860
   ccaatcgaga actgcgatgc cacatgccag actattgcag gggttctaag gaccaataaa 1920
   acatttcaga atgtgagtcc cctgtggata ggagaatgtc ccaaatacgt gaaaagtgaa 1980
   agtctgaggc ttgcaactgg actaagaaat gttccacaga ttgaaactag aggactcttc 2040
   ggagctattg cagggtttat tgaaggagga tggactggga tgatagatgg gtggtatggc 2100
   tatcaccatg aaaattctca agggtcagga tatgcagcag acagagaaag cactcaaaag 2160
   gctgtaaaca gaattacaaa taaggtcaat tccatcatca acaaaatgaa cacacaattt 2220
   gaagctgtcg atcacgaatt ttcaaatctg gagaggagaa ttgacaatct gaacaaaaga 2280
   atgcaagatg gatttctgga tgtttggaca tacaatgctg aactgttggt tcttcttgaa 2340
   aacgaaagaa cactagacat gcatgacgca aatgtgaaga acctacatga aaaggtcaaa 2400
   tcacaactaa gggacaatgc tacgatctta gggaatggtt gctttgaatt ttggcataag 2460
   tgtgacaatg aatgcataga gtctgtcaaa aatggtacat atgactatcc caaataccag 2520
   actgaaagca aattaaacag gctaaaaata gaatcagtaa agctagagaa ccttggtgtg 2580
   tatcaaattc ttgccattta tagtacggta tcgagcagcc tagtgttggt agggctgatc 2640
   atggcaatgg gtctttggat gtgttcaaat ggttcaatgc agtgcaggat atgtatataa 2700
   gagctctaag ttaaaatgct tcttcgtctc ctatttataa tatggtttgt tattgttaat 2760
   tttgttcttg tagaagagct taattaatcg ttgttgttat gaaatactat ttgtatgaga 2820
   tgaactggtg taatgtaatt catttacata agtggagtca gaatcagaat gtttcctcca 2880
   taactaacta gacatgaaga cctgccgcgt acaattgtct tatatttgaa caactaaaat 2940
   tgaacatctt ttgccacaac tttataagtg gttaatatag ctcaaatata tggtcaagtt 3000
   caatagatta ataatggaaa tatcagttat cgaaattcat taacaatcaa cttaacgtta 3060
   ttaactacta attttatatc atcccctttg ataaatgata gtaca 3105
<210> 68
   <211> 3087
   <212> DNA
   <213> Artificial sequence
<220>
   <223> H9 from A/Hong Kong/1073/99 (H9N2) (Construct # 785)
<400> 68
   agaggtaccc cgggctggta tatttatatg ttgtcaaata actcaaaaac cataaaagtt    60
   taagttagca agtgtgtaca tttttacttg aacaaaaata ttcacctact actgttataa   120
   atcattatta aacattagag taaagaaata tggatgataa gaacaagagt agtgatattt   180
   tgacaacaat tttgttgcaa catttgagaa aattttgttg ttctctcttt tcattggtca   240
   aaaacaatag agagagaaaa aggaagaggg agaataaaaa cataatgtga gtatgagaga   300
   gaaagttgta caaaagttgt accaaaatag ttgtacaaat atcattgagg aatttgacaa   360
   aagctacaca aataagggtt aattgctgta aataaataag gatgacgcat tagagagatg   420
   taccattaga gaatttttgg caagtcatta aaaagaaaga ataaattatt tttaaaatta   480
   aaagttgagt catttgatta aacatgtgat tatttaatga attgatgaaa gagttggatt   540
   aaagttgtat tagtaattag aatttggtgt caaatttaat ttgacatttg atcttttcct   600
   atatattgcc ccatagagtc agttaactca tttttatatt tcatagatca aataagagaa   660
   ataacggtat attaatccct ccaaaaaaaa aaaacggtat atttactaaa aaatctaagc   720
   cacgtaggag gataacagga tccccgtagg aggataacat ccaatccaac caatcacaac   780
   aatcctgatg agataaccca ctttaagccc acgcatctgt ggcacatcta cattatctaa   840
   atcacacatt cttccacaca tctgagccac acaaaaacca atccacatct ttatcaccca   900
   ttctataaaa aatcacactt tgtgagtcta cactttgatt cccttcaaac acatacaaag   960
   agaagagact aattaattaa ttaatcatct tgagagaaaa tggaaacaat atcactaata  1020
   actatactac tagtagtaac agcaagcaat gcagataaaa tctgcatcgg ccaccagtca  1080
   acaaactcca cagaaactgt ggacacgcta acagaaacca atgttcctgt gacacatgcc  1140
   aaagaattgc tccacacaga gcataatgga atgctgtgtg caacaagcct gggacatccc  1200
   ctcattctag acacatgcac tattgaagga ctagtctatg gcaacccttc ttgtgacctg  1260
   ctgttgggag gaagagaatg gtcctacatc gtcgaaagat catcagctgt aaatggaacg  1320
   tgttaccctg ggaatgtaga aaacctagag gaactcagga cactttttag ttccgctagt  1380
   tcctaccaaa gaatccaaat cttcccagac acaacctgga atgtgactta cactggaaca  1440
   agcagagcat gttcaggttc attctacagg agtatgagat ggctgactca aaagagcggt  1500
   ttttaccctg ttcaagacgc ccaatacaca aataacaggg gaaagagcat tcttttcgtg  1560
   tggggcatac atcacccacc cacctatacc gagcaaacaa atttgtacat aagaaacgac  1620
   acaacaacaa gcgtgacaac agaagatttg aataggacct tcaaaccagt gatagggcca  1680
   aggccccttg tcaatggtct gcagggaaga attgattatt attggtcggt actaaaacca 1740
   ggccaaacat tgcgagtacg atccaatggg aatctaattg ctccatggta tggacacgtt 1800
   ctttcaggag ggagccatgg aagaatcctg aagactgatt taaaaggtgg taattgtgta 1860
   gtgcaatgtc agactgaaaa aggtggctta aacagtacat tgccattcca caatatcagt 1920
   aaatatgcat ttggaacctg ccccaaatat gtaagagtta atagtctcaa actggcagtc 1980
   ggtctgagga acgtgcctgc tagatcaagt agaggactat ttggagccat agctggattc 2040
   atagaaggag gttggccagg actagtcgct ggctggtatg gtttccagca ttcaaatgat 2100
   caaggggttg gtatggctgc agatagggat tcaactcaaa aggcaattga taaaataaca 2160
   tccaaggtga ataatatagt cgacaagatg aacaagcaat atgaaataat tgatcatgaa 2220
   tttagtgagg ttgaaactag actcaatatg atcaataata agattgatga ccaaatacaa 2280
   gacgtatggg catataatgc agaattgcta gtactacttg aaaatcaaaa aacactcgat 2340
   gagcatgatg cgaacgtgaa caatctatat aacaaggtga agagggcact gggctccaat 2400
   gctatggaag atgggaaagg ctgtttcgag ctataccata aatgtgatga tcagtgcatg 2460
   gaaacaattc ggaacgggac ctataatagg agaaagtata gagaggaatc aagactagaa 2520
   aggcagaaaa tagagggggt taagctggaa tctgagggaa cttacaaaat cctcaccatt 2580
   tattcgactg tcgcctcatc tcttgtgctt gcaatggggt ttgctgcctt cctgttctgg 2640
   gccatgtcca atggatcttg cagatgcaac atttgtatat aagagctcta agttaaaatg 2700
   cttcttcgtc tcctatttat aatatggttt gttattgtta attttgttct tgtagaagag 2760
   cttaattaat cgttgttgtt atgaaatact atttgtatga gatgaactgg tgtaatgtaa 2820
   ttcatttaca taagtggagt cagaatcaga atgtttcctc cataactaac tagacatgaa 2880
   gacctgccgc gtacaattgt cttatatttg aacaactaaa attgaacatc ttttgccaca 2940
   actttataag tggttaatat agctcaaata tatggtcaag ttcaatagat taataatgga 3000
   aatatcagtt atcgaaattc attaacaatc aacttaacgt tattaactac taattttata 3060
   tcatcccctt tgataaatga tagtaca 3087
<210> 69
   <211> 3105
   <212> DNA
   <213> Artificial sequence
<220>
   <223> H3 from A/Brisbane/10/2007 (H3N2)
<400> 69
   agaggtaccc cgggctggta tatttatatg ttgtcaaata actcaaaaac cataaaagtt 60
   taagttagca agtgtgtaca tttttacttg aacaaaaata ttcacctact actgttataa 120
   atcattatta aacattagag taaagaaata tggatgataa gaacaagagt agtgatattt 180
   tgacaacaat tttgttgcaa catttgagaa aattttgttg ttctctcttt tcattggtca 240
   aaaacaatag agagagaaaa aggaagaggg agaataaaaa cataatgtga gtatgagaga 300
   gaaagttgta caaaagttgt accaaaatag ttgtacaaat atcattgagg aatttgacaa 360
   aagctacaca aataagggtt aattgctgta aataaataag gatgacgcat tagagagatg 420
   taccattaga gaatttttgg caagtcatta aaaagaaaga ataaattatt tttaaaatta 480
   aaagttgagt catttgatta aacatgtgat tatttaatga attgatgaaa gagttggatt 540
   aaagttgtat tagtaattag aatttggtgt caaatttaat ttgacatttg atcttttcct 600
   atatattgcc ccatagagtc agttaactca tttttatatt tcatagatca aataagagaa 660
   ataacggtat attaatccct ccaaaaaaaa aaaacggtat atttactaaa aaatctaagc 720
   cacgtaggag gataacagga tccccgtagg aggataacat ccaatccaac caatcacaac 780
   aatcctgatg agataaccca ctttaagccc acgcatctgt ggcacatcta cattatctaa 840
   atcacacatt cttccacaca tctgagccac acaaaaacca atccacatct ttatcaccca 900
   ttctataaaa aatcacactt tgtgagtcta cactttgatt cccttcaaac acatacaaag 960
   agaagagact aattaattaa ttaatcatct tgagagaaaa tgaagactat cattgctttg 1020
   agctacattc tatgtctggt tttcactcaa aaacttcccg gaaatgacaa cagcacggca 1080
   acgctgtgcc ttgggcacca tgcagtacca aacggaacga tagtgaaaac aatcacgaat 1140
   gaccaaattg aagttactaa tgctactgag ctggttcaga gttcctcaac aggtgaaata 1200
   tgcgacagtc ctcatcagat ccttgatgga gaaaactgca cactaataga tgctctattg 1260
   ggagaccctc agtgtgatgg cttccaaaat aagaaatggg acctttttgt tgaacgcagc 1320
   aaagcctaca gcaactgtta cccttatgat gtgccggatt atgcctccct taggtcacta 1380
   gttgcctcat ccggcacact ggagtttaac aatgaaagtt tcaattggac tggagtcact 1440
   caaaacggaa caagctctgc ttgcataagg agatctaata acagtttctt tagtagattg 1500
   aattggttga cccacttaaa attcaaatac ccagcattga acgtgactat gccaaacaat 1560
   gaaaaatttg acaaattgta catttggggg gttcaccacc cgggtacgga caatgaccaa 1620
   atcttcctgt atgctcaagc atcaggaaga atcacagtct ctaccaaaag aagccaacaa 1680
   actgtaatcc cgaatatcgg atctagaccc agagtaagga atatccccag cagaataagc 1740
   atctattgga caatagtaaa accgggagac atacttttga ttaacagcac agggaatcta 1800
   attgctccta ggggttactt caaaatacga agtgggaaaa gctcaataat gagatcagat 1860
   gcacccattg gcaaatgcaa ttctgaatgc atcactccaa acggaagcat tcccaatgac 1920
   aaaccattcc aaaatgtaaa caggatcaca tacggggcct gtcccagata tgttaagcaa 1980
   aacactctga aattggcaac agggatgcga aatgtaccag agaaacaaac tagaggcata 2040
   tttggcgcaa tcgcgggttt catagaaaat ggttgggagg gaatggtgga tggttggtat 2100
   ggtttcaggc atcaaaattc tgagggaata ggacaagcag cagatctcaa aagcactcaa 2160
   gcagcaatcg atcaaatcaa tgggaagctg aataggttga tcgggaaaac caacgagaaa 2220
   ttccatcaga ttgaaaaaga gttctcagaa gtcgaaggga gaatccagga ccttgagaaa 2280
   tatgttgagg acaccaaaat agatctctgg tcatacaacg cggagcttct tgttgccctg 2340
   gagaaccaac atacaattga tctaactgac tcagaaatga acaaactgtt tgaaaaaaca 2400
   aagaagcaac tgagggaaaa tgctgaggat atgggcaatg gttgtttcaa aatataccac 2460
   aaatgtgaca atgcctgcat aggatcaatc agaaatggaa cttatgacca cgatgtatac 2520
   agagatgaag cattaaacaa ccggttccag atcaagggcg ttgagctgaa gtcaggatac 2580
   aaagattgga tactatggat ttcctttgcc atatcatgtt ttttgctttg tgttgctttg 2640
   ttggggttca tcatgtgggc ctgccaaaaa ggcaacatta ggtgcaacat ttgcatttga 2700
   gagctctaag ttaaaatgct tcttcgtctc ctatttataa tatggtttgt tattgttaat 2760
   tttgttcttg tagaagagct taattaatcg ttgttgttat gaaatactat ttgtatgaga 2820
   tgaactggtg taatgtaatt catttacata agtggagtca gaatcagaat gtttcctcca 2880
   taactaacta gacatgaaga cctgccgcgt acaattgtct tatatttgaa caactaaaat 2940
   tgaacatctt ttgccacaac tttataagtg gttaatatag ctcaaatata tggtcaagtt 3000
   caatagatta ataatggaaa tatcagttat cgaaattcat taacaatcaa cttaacgtta 3060
   ttaactacta attttatatc atcccctttg ataaatgata gtaca 3105
<210> 70
   <211> 3105
   <212> DNA
   <213> Artificial sequence
<220>
   <223> H3 from A/Wisconsin/67/2005 (H3N2)
<400> 70
   agaggtaccc cgggctggta tatttatatg ttgtcaaata actcaaaaac cataaaagtt      60
   taagttagca agtgtgtaca tttttacttg aacaaaaata ttcacctact actgttataa     120
   atcattatta aacattagag taaagaaata tggatgataa gaacaagagt agtgatattt     180
   tgacaacaat tttgttgcaa catttgagaa aattttgttg ttctctcttt tcattggtca     240
   aaaacaatag agagagaaaa aggaagaggg agaataaaaa cataatgtga gtatgagaga     300
   gaaagttgta caaaagttgt accaaaatag ttgtacaaat atcattgagg aatttgacaa     360
   aagctacaca aataagggtt aattgctgta aataaataag gatgacgcat tagagagatg     420
   taccattaga gaatttttgg caagtcatta aaaagaaaga ataaattatt tttaaaatta     480
   aaagttgagt catttgatta aacatgtgat tatttaatga attgatgaaa gagttggatt     540
   aaagttgtat tagtaattag aatttggtgt caaatttaat ttgacatttg atcttttcct     600
   atatattgcc ccatagagtc agttaactca tttttatatt tcatagatca aataagagaa     660
   ataacggtat attaatccct ccaaaaaaaa aaaacggtat atttactaaa aaatctaagc     720
   cacgtaggag gataacagga tccccgtagg aggataacat ccaatccaac caatcacaac 780
   aatcctgatg agataaccca ctttaagccc acgcatctgt ggcacatcta cattatctaa 840
   atcacacatt cttccacaca tctgagccac acaaaaacca atccacatct ttatcaccca 900
   ttctataaaa aatcacactt tgtgagtcta cactttgatt cccttcaaac acatacaaag 960
   agaagagact aattaattaa ttaatcatct tgagagaaaa tgaagactat cattgctttg 1020
   agctacattc tatgtctggt tttcactcaa aaacttcccg gaaatgacaa cagcacggca 1080
   acgctgtgcc ttgggcacca tgcagtacca aacggaacga tagtgaaaac aatcacgaat 1140
   gaccaaattg aagttactaa tgctactgag ctggttcaga gttcctcaac aggtggaata 1200
   tgcgacagtc ctcatcagat ccttgatgga gaaaactgca cactaataga tgctctattg 1260
   ggagaccctc agtgtgatgg cttccaaaat aagaaatggg acctttttgt tgaacgcagc 1320
   aaagcctaca gcaactgtta cccttatgat gtgccggatt atgcctccct taggtcacta 1380
   gttgcctcat ccggcacact ggagtttaac gatgaaagtt tcaattggac tggagtcact 1440
   caaaatggaa caagctctgc ttgcaaaagg agatctaata acagtttctt tagtagattg 1500
   aattggttga cccacttaaa attcaaatac ccagcattga acgtgactat gccaaacaat 1560
   gaaaaatttg acaaattgta catttggggg gttcaccacc cgggtacgga caatgaccaa 1620
   atcttcctgc atgctcaagc atcaggaaga atcacagtct ctaccaaaag aagccaacaa 1680
   actgtaatcc cgaatatcgg atctagaccc agaataagga atatccccag cagaataagc 1740
   atctattgga caatagtaaa accgggagac atacttttga ttaacagcac agggaatcta 1800
   attgctccta ggggttactt caaaatacga agtgggaaaa gctcaataat gagatcagat 1860
   gcacccattg gcaaatgcaa ttctgaatgc atcactccaa atggaagcat tcccaatgac 1920
   aaaccatttc aaaatgtaaa caggatcaca tatggggcct gtcccagata tgttaagcaa 1980
   aacactctga aattggcaac agggatgcga aatgtaccag agaaacaaac tagaggcata 2040
   tttggcgcaa tcgcgggttt catagaaaat ggttgggagg gaatggtgga tggttggtac 2100
   ggtttcaggc atcaaaattc tgagggaata ggacaagcag cagatctcaa aagcactcaa 2160
   gcagcaatca atcaaatcaa tgggaagctg aataggttga tcgggaaaac caacgagaaa 2220
   ttccatcaga ttgaaaaaga gttctcagaa gtagaaggga gaatccagga cctcgagaaa 2280
   tatgttgagg acactaaaat agatctctgg tcatacaacg cggagcttct tgttgccctg 2340
   gagaaccaac atacaattga tctaactgac tcagaaatga acaaactgtt tgaaagaaca 2400
   aagaagcaac tgagggaaaa tgctgaggat atgggcaatg gttgtttcaa aatataccac 2460
   aaatgtgaca atgcctgcat aggatcaatc agaaatggaa cttatgacca tgatgtatac 2520
   agagatgaag cattaaacaa ccggttccag atcaaaggcg ttgagctgaa gtcaggatac 2580
   aaagattgga tactatggat ttcctttgcc atatcatgtt ttttgctttg tgttgctttg 2640
   ttggggttca tcatgtgggc ctgccaaaaa ggcaacatta ggtgcaacat ttgcatttga 2700
   gagctctaag ttaaaatgct tcttcgtctc ctatttataa tatggtttgt tattgttaat 2760
   tttgttcttg tagaagagct taattaatcg ttgttgttat gaaatactat ttgtatgaga 2820
   tgaactggtg taatgtaatt catttacata agtggagtca gaatcagaat gtttcctcca 2880
   taactaacta gacatgaaga cctgccgcgt acaattgtct tatatttgaa caactaaaat 2940
   tgaacatctt ttgccacaac tttataagtg gttaatatag ctcaaatata tggtcaagtt 3000
   caatagatta ataatggaaa tatcagttat cgaaattcat taacaatcaa cttaacgtta 3060
   ttaactacta attttatatc atcccctttg ataaatgata gtaca 3105
<210> 71
   <211> 3117
   <212> DNA
   <213> Artificial sequence
<220>
   <223> H7 from A/Equine/Prague/56 (H7N7)
<400> 71
   agaggtaccc cgggctggta tatttatatg ttgtcaaata actcaaaaac cataaaagtt 60
   taagttagca agtgtgtaca tttttacttg aacaaaaata ttcacctact actgttataa 120
   atcattatta aacattagag taaagaaata tggatgataa gaacaagagt agtgatattt 180
   tgacaacaat tttgttgcaa catttgagaa aattttgttg ttctctcttt tcattggtca 240
   aaaacaatag agagagaaaa aggaagaggg agaataaaaa cataatgtga gtatgagaga 300
   gaaagttgta caaaagttgt accaaaatag ttgtacaaat atcattgagg aatttgacaa 360
   aagctacaca aataagggtt aattgctgta aataaataag gatgacgcat tagagagatg 420
   taccattaga gaatttttgg caagtcatta aaaagaaaga ataaattatt tttaaaatta 480
   aaagttgagt catttgatta aacatgtgat tatttaatga attgatgaaa gagttggatt 540
   aaagttgtat tagtaattag aatttggtgt caaatttaat ttgacatttg atcttttcct 600
   atatattgcc ccatagagtc agttaactca tttttatatt tcatagatca aataagagaa 660
   ataacggtat attaatccct ccaaaaaaaa aaaacggtat atttactaaa aaatctaagc 720
   cacgtaggag gataacagga tccccgtagg aggataacat ccaatccaac caatcacaac 780
   aatcctgatg agataaccca ctttaagccc acgcatctgt ggcacatcta cattatctaa 840
   atcacacatt cttccacaca tctgagccac acaaaaacca atccacatct ttatcaccca 900
   ttctataaaa aatcacactt tgtgagtcta cactttgatt cccttcaaac acatacaaag 960
   agaagagact aattaattaa ttaatcatct tgagagaaaa tgaacactca aattctaata 1020
   ttagccactt cggcattctt ctatgtacgt gcagataaaa tctgcctagg acatcatgct 1080
   gtgtctaatg gaaccaaagt agacaccctt actgaaaaag gaatagaagt tgtcaatgca 1140
   acagaaacag ttgaacaaac aaacatccct aagatctgct caaaaggaaa acagactgtt 1200
   gaccttggtc aatgtggatt actagggacc gttattggtc ctccccaatg tgaccaattt 1260
   cttgagttct ctgctaattt aatagttgaa agaagggaag gtaatgacat ttgttatcca 1320
   ggcaaatttg acaatgaaga aacattgaga aaaatactca gaaaatccgg aggaattaaa 1380
   aaggagaata tgggattcac atataccgga gtgagaacca atggagagac tagcgcatgt 1440
   agaaggtcaa gatcttcctt ttatgcagag atgaaatggc ttctatccag cacagacaat 1500
   gggacatttc cacaaatgac aaagtcctac aagaacacta agaaggtacc agctctgata 1560
   atctggggaa tccaccactc aggatcaact actgaacaga ctagattata tggaagtggg 1620
   aataaattga taacagtttg gagttccaaa taccaacaat cttttgtccc aaatcctgga 1680
   ccaagaccgc aaatgaatgg tcaatcagga agaattgact ttcactggct gatgctagat 1740
   cccaatgata ctgtcacttt cagttttaat ggggccttta tagcacctga ccgcgccagt 1800
   tttctaagag gtaaatctct aggaatccaa agtgatgcac aacttgacaa taattgtgaa 1860
   ggtgaatgct atcatattgg aggtactata attagcaact tgccctttca aaacattaat 1920
   agtagggcaa tcggaaaatg ccccagatac gtgaagcaga agagcttaat gctagcaaca 1980
   ggaatgaaaa atgttcctga agctcctgca cataaacaac taactcatca catgcgcaaa 2040
   aaaagaggtt tatttggtgc aatagcagga ttcattgaaa atgggtggga aggattaata 2100
   gacggatggt atggatataa gcatcagaat gcacaaggag aagggactgc tgcagactac 2160
   aaaagtacac aatctgctat caaccaaata accggaaaat tgaacagact aatagaaaaa 2220
   accaaccagc aattcgaact aatagataat gagttcaatg aaatagaaaa acaaattggc 2280
   aatgttatta actggactag agattctatc atcgaagtat ggtcatataa tgcagagttc 2340
   ctcgtagcag tggagaatca acacactatt gatttaactg actcagaaat gaacaaacta 2400
   tatgaaaagg taagaagaca actgagagaa aatgctgagg aagatggtaa tggctgtttt 2460
   gaaatattcc accaatgtga caatgattgc atggccagca ttagaaacaa cacatatgac 2520
   cataaaaaat acagaaaaga ggcaatacaa aacagaatcc agattgacgc agtaaagttg 2580
   agcagtggtt acaaagatat aatactttgg tttagcttcg gggcatcatg tttcttattt 2640
   cttgccattg caatgggtct tgttttcata tgtataaaaa atggaaacat gcggtgcact 2700
   atttgtatat aagagctcta agttaaaatg cttcttcgtc tcctatttat aatatggttt 2760
   gttattgtta attttgttct tgtagaagag cttaattaat cgttgttgtt atgaaatact 2820
   atttgtatga gatgaactgg tgtaatgtaa ttcatttaca taagtggagt cagaatcaga 2880
   atgtttcctc cataactaac tagacatgaa gacctgccgc gtacaattgt cttatatttg 2940
   aacaactaaa attgaacatc ttttgccaca actttataag tggttaatat agctcaaata 3000
   tatggtcaag ttcaatagat taataatgga aatatcagtt atcgaaattc attaacaatc 3060
   aacttaacgt tattaactac taattttata tcatcccctt tgataaatga tagtaca 3117
<210> 72
   <211> 3162
   <212> DNA
   <213> Artificial sequence
<220>
   <223> HA from B/Malaysia/2506/2004
<400> 72
   agaggtaccc cgggctggta tatttatatg ttgtcaaata actcaaaaac cataaaagtt 60
   taagttagca agtgtgtaca tttttacttg aacaaaaata ttcacctact actgttataa 120
   atcattatta aacattagag taaagaaata tggatgataa gaacaagagt agtgatattt 180
   tgacaacaat tttgttgcaa catttgagaa aattttgttg ttctctcttt tcattggtca 240
   aaaacaatag agagagaaaa aggaagaggg agaataaaaa cataatgtga gtatgagaga 300
   gaaagttgta caaaagttgt accaaaatag ttgtacaaat atcattgagg aatttgacaa 360
   aagctacaca aataagggtt aattgctgta aataaataag gatgacgcat tagagagatg 420
   taccattaga gaatttttgg caagtcatta aaaagaaaga ataaattatt tttaaaatta 480
   aaagttgagt catttgatta aacatgtgat tatttaatga attgatgaaa gagttggatt 540
   aaagttgtat tagtaattag aatttggtgt caaatttaat ttgacatttg atcttttcct 600
   atatattgcc ccatagagtc agttaactca tttttatatt tcatagatca aataagagaa 660
   ataacggtat attaatccct ccaaaaaaaa aaaacggtat atttactaaa aaatctaagc 720
   cacgtaggag gataacagga tccccgtagg aggataacat ccaatccaac caatcacaac 780
   aatcctgatg agataaccca ctttaagccc acgcatctgt ggcacatcta cattatctaa 840
   atcacacatt cttccacaca tctgagccac acaaaaacca atccacatct ttatcaccca 900
   ttctataaaa aatcacactt tgtgagtcta cactttgatt cccttcaaac acatacaaag 960
   agaagagact aattaattaa ttaatcatct tgagagaaaa tgaaggcaat aattgtacta 1020
   ctcatggtag taacatccaa tgcagatcga atctgcactg ggataacatc gtcaaactca 1080
   ccacatgttg tcaaaactgc tactcaaggg gaggtcaatg tgactggtgt aataccactg 1140
   acaacaacac ccaccaaatc tcattttgca aatctcaaag gaacagaaac cagagggaaa 1200
   ctatgcccaa aatgcctcaa ctgcacagat ctggacgtgg ccttgggcag accaaaatgc 1260
   acggggaaca taccctcggc aagagtttca atactccatg aagtcagacc tgttacatct 1320
   gggtgctttc ctataatgca cgacagaaca aaaattagac agctgcctaa acttctcaga 1380
   ggatacgaac atatcaggtt atcaactcat aacgttatca atgcagaaaa tgcaccagga 1440
   ggaccctaca aaattggaac ctcagggtct tgccctaacg ttaccaatgg aaacggattt 1500
   ttcgcaacaa tggcttgggc cgtcccaaaa aacgacaaca acaaaacagc aacaaattca 1560
   ttaacaatag aagtaccata catttgtaca gaaggagaag accaaattac cgtttggggg 1620
   ttccactctg ataacgaaac ccaaatggca aagctctatg gggactcaaa gccccagaag 1680
   ttcacctcat ctgccaacgg agtgaccaca cattacgttt cacagattgg tggcttccca   1740
   aatcaaacag aagacggagg actaccacaa agcggtagaa ttgttgttga ttacatggtg   1800
   caaaaatctg ggaaaacagg aacaattacc tatcaaagag gtattttatt gcctcaaaaa   1860
   gtgtggtgcg caagtggcag gagcaaggta ataaaaggat cgttgccttt aattggagaa   1920
   gcagattgcc tccacgaaaa atacggtgga ttaaacaaaa gcaagcctta ctacacaggg   1980
   gaacatgcaa aggccatagg aaattgccca atatgggtga aaacaccctt gaagctggcc   2040
   aatggaacca aatatagacc tcctgcaaaa ctattaaagg aaaggggttt cttcggagct   2100
   attgctggtt tcttagaagg aggatgggaa ggaatgattg caggttggca cggatacaca   2160
   tcccatgggg cacatggagt agcggtggca gcagacctta agagcactca agaggccata   2220
   aacaagataa caaaaaatct caactctttg agtgagctgg aagtaaagaa tcttcaaaga   2280
   ctaagcggtg ccatggatga actccacaac gaaatactag aactagacga gaaagtggat   2340
   gatctcagag ctgatacaat aagctcacaa atagaactcg cagtcctgct ttccaatgaa   2400
   ggaataataa acagtgaaga tgagcatctc ttggcgcttg aaagaaagct gaagaaaatg   2460
   ctgggcccct ctgctgtaga gatagggaat ggatgctttg aaaccaaaca caagtgcaac   2520
   cagacctgtc tcgacagaat agctgctggt acctttgatg caggagaatt ttctctcccc   2580
   acttttgatt cactgaatat tactgctgca tctttaaatg acgatggatt ggataatcat   2640
   actatactgc tttactactc aactgctgcc tccagtttgg ctgtaacatt gatgatagct   2700
   atctttgttg tttatatggt ctccagagac aatgtttctt gctccatctg tctataagag   2760
   ctctaagtta aaatgcttct tcgtctccta tttataatat ggtttgttat tgttaatttt   2820
   gttcttgtag aagagcttaa ttaatcgttg ttgttatgaa atactatttg tatgagatga   2880
   actggtgtaa tgtaattcat ttacataagt ggagtcagaa tcagaatgtt tcctccataa   2940
   ctaactagac atgaagacct gccgcgtaca attgtcttat atttgaacaa ctaaaattga   3000
   acatcttttg ccacaacttt ataagtggtt aatatagctc aaatatatgg tcaagttcaa   3060
   tagattaata atggaaatat cagttatcga aattcattaa caatcaactt aacgttatta   3120
   actactaatt ttatatcatc ccctttgata aatgatagta ca                      3162
<210> 73
   <211> 3159
   <212> DNA
   <213> Artificial sequence
<220>
   <223> HA from B/Florida/4/2006
<400> 73
   agaggtaccc cgggctggta tatttatatg ttgtcaaata actcaaaaac cataaaagtt 60
   taagttagca agtgtgtaca tttttacttg aacaaaaata ttcacctact actgttataa 120
   atcattatta aacattagag taaagaaata tggatgataa gaacaagagt agtgatattt 180
   tgacaacaat tttgttgcaa catttgagaa aattttgttg ttctctcttt tcattggtca 240
   aaaacaatag agagagaaaa aggaagaggg agaataaaaa cataatgtga gtatgagaga 300
   gaaagttgta caaaagttgt accaaaatag ttgtacaaat atcattgagg aatttgacaa 360
   aagctacaca aataagggtt aattgctgta aataaataag gatgacgcat tagagagatg 420
   taccattaga gaatttttgg caagtcatta aaaagaaaga ataaattatt tttaaaatta 480
   aaagttgagt catttgatta aacatgtgat tatttaatga attgatgaaa gagttggatt 540
   aaagttgtat tagtaattag aatttggtgt caaatttaat ttgacatttg atcttttcct 600
   atatattgcc ccatagagtc agttaactca tttttatatt tcatagatca aataagagaa 660
   ataacggtat attaatccct ccaaaaaaaa aaaacggtat atttactaaa aaatctaagc 720
   cacgtaggag gataacagga tccccgtagg aggataacat ccaatccaac caatcacaac 780
   aatcctgatg agataaccca ctttaagccc acgcatctgt ggcacatcta cattatctaa 840
   atcacacatt cttccacaca tctgagccac acaaaaacca atccacatct ttatcaccca 900
   ttctataaaa aatcacactt tgtgagtcta cactttgatt cccttcaaac acatacaaag 960
   agaagagact aattaattaa ttaatcatct tgagagaaaa tgaaggcaat aattgtacta 1020
   ctcatggtag taacatccaa tgcagatcga atctgcactg gaataacatc ttcaaactca 1080
   cctcatgtgg tcaaaacagc cactcaaggg gaggtcaatg tgactggtgt gataccacta 1140
   acaacaacac caacaaaatc ttattttgca aatctcaaag gaacaaggac cagagggaaa 1200
   ctatgcccag actgtctcaa ctgcacagat ctggatgtgg ctttgggcag accaatgtgt 1260
   gtggggacca caccttcggc gaaggcttca atactccacg aagtcaaacc tgttacatcc 1320
   gggtgctttc ctataatgca cgacagaaca aaaatcaggc aactacccaa tcttctcaga 1380
   ggatatgaaa atatcaggct atcaacccaa aacgtcatcg atgcggaaaa ggcaccagga 1440
   ggaccctaca gacttggaac ctcaggatct tgccctaacg ctaccagtaa gagcggattt 1500
   ttcgcaacaa tggcttgggc tgtcccaaag gacaacaaca aaaatgcaac gaacccacta 1560
   acagtagaag taccatacat ttgtacagaa ggggaagacc aaatcactgt ttgggggttc 1620
   cattcagata acaaaaccca aatgaagaac ctctatggag actcaaatcc tcaaaagttc 1680
   acctcatctg ctaatggagt aaccacacac tatgtttctc agattggcag cttcccagat 1740
   caaacagaag acggaggact accacaaagc ggcaggattg ttgttgatta catgatgcaa 1800
   aaacctggga aaacaggaac aattgtctac caaagaggtg ttttgttgcc tcaaaaggtg 1860
   tggtgcgcga gtggcaggag caaagtaata aaagggtcct tgcctttaat tggtgaagca 1920
   gattgccttc atgaaaaata cggtggatta aacaaaagca agccttacta cacaggagaa 1980
   catgcaaaag ccataggaaa ttgcccaata tgggtgaaaa cacctttgaa gctcgccaat 2040
   ggaaccaaat atagacctcc tgcaaaacta ttaaaggaaa ggggtttctt cggagctatt 2100
   gctggtttcc tagaaggagg atgggaagga atgattgcag gctggcacgg atacacatct 2160
   cacggagcac atggagtggc agtggcggcg gaccttaaga gtacgcaaga agctataaac 2220
   aagataacaa aaaatctcaa ttctttgagt gagctagaag taaagaatct tcaaagacta 2280
   agtggtgcca tggatgaact ccacaacgaa atactcgagc tggatgagaa agtggatgat 2340
   ctcagagctg acactataag ctcgcaaata gaacttgcag tcttgctttc caacgaagga 2400
   ataataaaca gtgaagatga gcatctattg gcacttgaga gaaaactaaa gaaaatgctg 2460
   ggtccctctg ctgtagagat aggaaatgga tgcttcgaaa ccaaacacaa gtgcaaccag 2520
   acctgcttag acaggatagc tgctggcacc tttaatgcag gagaattttc tctccccact 2580
   tttgattcac tgaacattac tgctgcatct ttaaatgatg atggattgga taaccatact 2640
   atactgctct attactcaac tgctgcttct agtttggctg taacattgat gctagctatt 2700
   tttattgttt atatggtctc cagagacaac gtttcatgct ccatctgtct ataagagctc 2760
   taagttaaaa tgcttcttcg tctcctattt ataatatggt ttgttattgt taattttgtt 2820
   cttgtagaag agcttaatta atcgttgttg ttatgaaata ctatttgtat gagatgaact 2880
   ggtgtaatgt aattcattta cataagtgga gtcagaatca gaatgtttcc tccataacta 2940
   actagacatg aagacctgcc gcgtacaatt gtcttatatt tgaacaacta aaattgaaca 3000
   tcttttgcca caactttata agtggttaat atagctcaaa tatatggtca agttcaatag 3060
   attaataatg gaaatatcag ttatcgaaat tcattaacaa tcaacttaac gttattaact 3120
   actaatttta tatcatcccc tttgataaat gatagtaca 3159
<210> 74
   <211> 565
   <212> PRT
   <213> Influenza virus
<220>
   <221> misc_feature
   <222> (3)..(3)
   <223> Xaa can be Ala or Val
<220>
   <221> misc_feature
   <222> (52)..(52)
   <223> Xaa can be Asp or Asn
<220>
   <221> misc_feature
   <222> (90)..(90)
   <223> Xaa can be Lys or Arg
<220>
   <221> misc_feature
   <222> (99)..(99)
   <223> Xaa can be Lys or Thr
<220>
   <221> misc_feature
   <222> (111)..(111)
   <223> Xaa can be Tyr or His
<220>
   <221> misc_feature
   <222> (145)..(145)
   <223> Xaa can be Val or Thr
<220>
   <221> misc_feature
   <222> (157)..(157)
   <223> Xaa can be Glu Lys
<220>
   <221> misc_feature
   <222> (162)..(162)
   <223> Xaa can be Arg or Lys
<220>
   <221> misc_feature
   <222> (182)..(182)
   <223> Xaa can be Val or Ala
<220>
   <221> misc_feature
   <222> (203)..(203)
   <223> Xaa can be Asp or Asn
<220>
   <221> misc_feature
   <222> (205)..(205)
   <223> Xaa can be Arg or Lys
<220>
   <221> misc_feature
   <222> (210)..(210)
   <223> Xaa can be Thr or Lys
<220>
   <221> misc_feature
   <222> (225)..(225)
   <223> Xaa can be Arg or Lys
<220>
   <221> misc_feature
   <222> (268)..(268)
   <223> Xaa can be Trp or Arg
<220>
   <221> misc_feature
   <222> (283)..(283)
   <223> Xaa can be Thr or Asn
<220>
   <221> misc_feature
   <222> (290)..(290)
   <223> Xaa can be Glu or Gly
<220>
   <221> misc_feature
   <222> (432)..(432)
   <223> Xaa can be Ile or Leu
<220>
   <221> misc_feature
   <222> (489)..(489)
   <223> Xaa can be Asn or Asp
<400> 74
   Met Lys Xaa Lys Leu Leu Val Leu Leu Cys Thr Phe Thr Ala Thr Tyr
   1               5                   10                  15
   Ala Asp Thr Ile Cys Ile Gly Tyr His Ala Asn Asn Ser Thr Asp Thr
               20                  25                  30
   Val Asp Thr Val Leu Glu Lys Asn Val Thr Val Thr His Ser Val Asn
           35                  40                  45
   Leu Leu Glu Xaa Ser His Asn Gly Lys Leu Cys Leu Leu Lys Gly Ile
       50                  55                  60
   Ala Pro Leu Gln Leu Gly Asn Cys Ser Val Ala Gly Trp Ile Leu Gly
   65                  70                  75                  80
   Asn Pro Glu Cys Glu Leu Leu Ile Ser Xaa Glu Ser Trp Ser Tyr Ile
                   85                  90                  95
   Val Glu Xaa Pro Asn Pro Glu Asn Gly Thr Cys Tyr Pro Gly Xaa Phe
               100                 105                 110
   Ala Asp Tyr Glu Glu Leu Arg Glu Gln Leu Ser Ser Val Ser Ser Phe
           115                 120                 125
   Glu Arg Phe Glu Ile Phe Pro Lys Glu Ser Ser Trp Pro Asn His Thr
       130                 135                 140
   Xaa Thr Gly Val Ser Ala Ser Cys Ser His Asn Gly Xaa Ser Ser Phe
   145                 150                 155                 160
   Tyr Xaa Asn Leu Leu Trp Leu Thr Gly Lys Asn Gly Leu Tyr Pro Asn
                   165                 170                 175
   Leu Ser Lys Ser Tyr Xaa Asn Asn Lys Glu Lys Glu Val Leu Val Leu
               180                 185                 190
   Trp Gly Val His His Pro Pro Asn Ile Gly Xaa Gln Xaa Ala Leu Tyr
           195                 200                 205
   His Xaa Glu Asn Ala Tyr Val Ser Val Val Ser Ser His Tyr Ser Arg
       210                 215                 220
   Xaa Phe Thr Pro Glu Ile Ala Lys Arg Pro Lys Val Arg Asp Gln Glu
   225                 230                 235                 240
   Gly Arg Ile Asn Tyr Tyr Trp Thr Leu Leu Glu Pro Gly Asp Thr Ile
   245 250 255
   Ile Phe Glu Ala Asn Gly Asn Leu Ile Ala Pro Xaa Tyr Ala Phe Ala
   260 265 270
   Leu Ser Arg Gly Phe Gly Ser Gly Ile Ile Xaa Ser Asn Ala Pro Met
   275 280 285
   Asp Xaa Cys Asp Ala Lys Cys Gln Thr Pro Gln Gly Ala Ile Asn Ser
   290 295 300
   Ser Leu Pro Phe Gln Asn Val His Pro Val Thr Ile Gly Glu Cys Pro
   305 310 315 320
   Lys Tyr Val Arg Ser Ala Lys Leu Arg Met Val Thr Gly Leu Arg Asn
   325 330 335
   Ile Pro Ser Ile Gln Ser Arg Gly Leu Phe Gly Ala Ile Ala Gly Phe
   340 345 350
   Ile Glu Gly Gly Trp Thr Gly Met Val Asp Gly Trp Tyr Gly Tyr His
   355 360 365
   His Gln Asn Glu Gln Gly Ser Gly Tyr Ala Ala Asp Gln Lys Ser Thr
   370 375 380
   Gln Asn Ala Ile Asn Gly Ile Thr Asn Lys Val Asn Ser Val Ile Glu
   385 390 395 400
   Lys Met Asn Thr Gln Phe Thr Ala Val Gly Lys Glu Phe Asn Lys Leu
   405 410 415
   Glu Arg Arg Met Glu Asn Leu Asn Lys Lys Val Asp Asp Gly Phe Xaa
   420 425 430
   Asp Ile Trp Thr Tyr Asn Ala Glu Leu Leu Val Leu Leu Glu Asn Glu
   435 440 445
   Arg Thr Leu Asp Phe His Asp Ser Asn Val Lys Asn Leu Tyr Glu Lys
   450 455 460
   Val Lys Ser Gln Leu Lys Asn Asn Ala Lys Glu Ile Gly Asn Gly Cys
   465 470 475 480
   Phe Glu Phe Tyr His Lys Cys Asn Xaa Glu Cys Met Glu Ser Val Lys
   485 490 495
   Asn Gly Thr Tyr Asp Tyr Pro Lys Tyr Ser Glu Glu Ser Lys Leu Asn
   500 505 510
   Arg Glu Lys Ile Asp Gly Val Lys Leu Glu Ser Met Gly Val Tyr Gln
   515 520 525
   Ile Leu Ala Ile Tyr Ser Thr Val Ala Ser Ser Leu Val Leu Leu Val
   530 535 540
   Ser Leu Gly Ala Ile Ser Phe Trp Met Cys Ser Asn Gly Ser Leu Gln
   545 550 555 560
   Cys Arg Ile Cys Ile
   565
<210> 75
   <211> 565
   <212> PRT
   <213> Influenza virus
<400> 75
   Met Lys Ala Lys Leu Leu Val Leu Leu Cys Thr Phe Thr Ala Thr Tyr
   1               5                   10                  15
   Ala Asp Thr Ile Cys Ile Gly Tyr His Ala Asn Asn Ser Thr Asp Thr
               20                  25                  30
   Val Asp Thr Val Leu Glu Lys Asn Val Thr Val Thr His Ser Val Asn
           35                  40                  45
   Leu Leu Glu Asp Ser His Asn Gly Lys Leu Cys Leu Leu Lys Gly Ile
       50                  55                  60
   Ala Pro Leu Gln Leu Gly Asn Cys Ser Val Ala Gly Trp Ile Leu Gly
   65                  70                  75                  80
   Asn Pro Glu Cys Glu Leu Leu Ile Ser Lys Glu Ser Trp Ser Tyr Ile
                   85                  90                  95
   Val Glu Thr Pro Asn Pro Glu Asn Gly Thr Cys Tyr Pro Gly Tyr Phe
               100                 105                 110
   Ala Asp Tyr Glu Glu Leu Arg Glu Gln Leu Ser Ser Val Ser Ser Phe
           115                 120                 125
   Glu Arg Phe Glu Ile Phe Pro Lys Glu Ser Ser Trp Pro Asn His Thr
       130                 135                 140
   Val Thr Gly Val Ser Ala Ser Cys Ser His Asn Gly Lys Ser Ser Phe
   145                 150                 155                 160
   Tyr Arg Asn Leu Leu Trp Leu Thr Gly Lys Asn Gly Leu Tyr Pro Asn
                   165                 170                 175
   Leu Ser Lys Ser Tyr Val Asn Asn Lys Glu Lys Glu Val Leu Val Leu
               180                 185                 190
   Trp Gly Val His His Pro Pro Asn Ile Gly Asn Gln Arg Ala Leu Tyr
           195                 200                 205
   His Thr Glu Asn Ala Tyr Val Ser Val Val Ser Ser His Tyr Ser Arg
       210                 215                 220
   Arg Phe Thr Pro Glu Ile Ala Lys Arg Pro Lys Val Arg Asp Gln Glu
   225                 230                 235                 240
   Gly Arg Ile Asn Tyr Tyr Trp Thr Leu Leu Glu Pro Gly Asp Thr Ile
                   245                 250                 255
   Ile Phe Glu Ala Asn Gly Asn Leu Ile Ala Pro Trp Tyr Ala Phe Ala
               260                 265                 270
   Leu Ser Arg Gly Phe Gly Ser Gly Ile Ile Thr Ser Asn Ala Pro Met
           275                 280                 285
   Asp Glu Cys Asp Ala Lys Cys Gln Thr Pro Gln Gly Ala Ile Asn Ser
       290                 295                 300
   Ser Leu Pro Phe Gln Asn Val His Pro Val Thr Ile Gly Glu Cys Pro
   305                 310                 315                 320
   Lys Tyr Val Arg Ser Ala Lys Leu Arg Met Val Thr Gly Leu Arg Asn
                   325                 330                 335
   Ile Pro Ser Ile Gln Ser Arg Gly Leu Phe Gly Ala Ile Ala Gly Phe
               340                 345                 350
   Ile Glu Gly Gly Trp Thr Gly Met Val Asp Gly Trp Tyr Gly Tyr His
           355                 360                 365
   His Gln Asn Glu Gln Gly Ser Gly Tyr Ala Ala Asp Gln Lys Ser Thr
       370                 375                 380
   Gln Asn Ala Ile Asn Gly Ile Thr Asn Lys Val Asn Ser Val Ile Glu
   385                 390                 395                 400
   Lys Met Asn Thr Gln Phe Thr Ala Val Gly Lys Glu Phe Asn Lys Leu
                   405                 410                 415
   Glu Arg Arg Met Glu Asn Leu Asn Lys Lys Val Asp Asp Gly Phe Leu
   420 425 430
   Asp Ile Trp Thr Tyr Asn Ala Glu Leu Leu Val Leu Leu Glu Asn Glu
   435 440 445
   Arg Thr Leu Asp Phe His Asp Ser Asn Val Lys Asn Leu Tyr Glu Lys
   450 455 460
   Val Lys Ser Gln Leu Lys Asn Asn Ala Lys Glu Ile Gly Asn Gly Cys
   465 470 475 480
   Phe Glu Phe Tyr His Lys Cys Asn Asn Glu Cys Met Glu Ser Val Lys
   485 490 495
   Asn Gly Thr Tyr Asp Tyr Pro Lys Tyr Ser Glu Glu Ser Lys Leu Asn
   500 505 510
   Arg Glu Lys Ile Asp Gly Val Lys Leu Glu Ser Met Gly Val Tyr Gln
   515 520 525
   Ile Leu Ala Ile Tyr Ser Thr Val Ala Ser Ser Leu Val Leu Leu Val
   530 535 540
   Ser Leu Gly Ala Ile Ser Phe Trp Met Cys Ser Asn Gly Ser Leu Gln
   545 550 555 560
   Cys Arg Ile Cys Ile
   565
<210> 76
   <211> 252
   <212> PRT
   <213> Influenza virus
<400> 76
   Met Ser Leu Leu Thr Glu Val Glu Thr Tyr Val Leu Ser Ile Ile Pro
   1               5                   10                  15
   Ser Gly Pro Leu Lys Ala Glu Ile Ala Gln Arg Leu Glu Asp Val Phe
               20                  25                  30
   Ala Gly Lys Asn Thr Asp Leu Glu Val Leu Met Glu Trp Leu Lys Thr
           35                  40                  45
   Arg Pro Ile Leu Ser Pro Leu Thr Lys Gly Ile Leu Gly Phe Val Phe
       50                  55                  60
   Thr Leu Thr Val Pro Ser Glu Arg Gly Leu Gln Arg Arg Arg Phe Val
   65                  70                  75                  80
   Gln Asn Ala Leu Asn Gly Asn Gly Asp Pro Asn Asn Met Asp Lys Ala
                   85                  90                  95
   Val Lys Leu Tyr Arg Lys Leu Lys Arg Glu Ile Thr Phe His Gly Ala
               100                 105                 110
   Lys Glu Ile Ser Leu Ser Tyr Ser Ala Gly Ala Leu Ala Ser Cys Met
           115                 120                 125
   Gly Leu Ile Tyr Asn Arg Met Gly Ala Val Thr Thr Glu Val Ala Phe
       130                 135                 140
   Gly Leu Val Cys Ala Thr Cys Glu Gln Ile Ala Asp Ser Gln His Arg
   145                 150                 155                 160
   Ser His Arg Gln Met Val Thr Thr Thr Asn Pro Leu Ile Arg His Glu
                   165                 170                 175
   Asn Arg Met Val Leu Ala Ser Thr Thr Ala Lys Ala Met Glu Gln Met
               180                 185                 190
   Ala Gly Ser Ser Glu Gln Ala Ala Glu Ala Met Glu Val Ala Ser Gln
           195                 200                 205
   Ala Arg Gln Met Val Gln Ala Met Arg Thr Ile Gly Thr His Pro Ser
       210                 215                 220
   Ser Ser Ala Gly Leu Lys Asn Asp Leu Leu Glu Asn Leu Gln Ala Tyr
   225                 230                 235                 240
   Gln Lys Arg Met Gly Val Gln Met Gln Arg Phe Lys
                   245                 250
<210> 77
   <211> 24
   <212> DNA
   <213> Artificial sequence
<220>
   <223> pBinPlus.2613c
<400> 77
   aggaagggaa gaaagcgaaa ggag 24
<210> 78
   <211> 56
   <212> DNA
   <213> Artificial sequence
<220>
   <223> Mut-ATG115.r
<400> 78
   gtgccgaagc acgatctgac aacgttgaag atcgctcacg caagaaagac aagaga 56
<210> 79
   <211> 52
   <212> DNA
   <213> Artificial sequence
<220>
   <223> Mut-ATG161.c
<400> 79
   gttgtcagat cgtgcttcgg caccagtaca acgttttctt tcactgaagc ga 52
<210> 80
   <211> 25
   <212> DNA
   <213> Artificial sequence
<220>
   <223> LC-C5-1.110r
<400> 80
   tctcctggag tcacagacag ggtgg 25
<210> 81
   <211> 2065
   <212> DNA
   <213> Artificial sequence
<220>
   <223> Expression cassette number 828
<400> 81
   ttaattaaga attcgagctc caccgcggaa acctcctcgg attccattgc ccagctatct 60
   gtcactttat tgagaagata gtggaaaagg aaggtggctc ctacaaatgc catcattgcg 120
   ataaaggaaa ggccatcgtt gaagatgcct ctgccgacag tggtcccaaa gatggacccc 180
   cacccacgag gagcatcgtg gaaaaagaag acgttccaac cacgtcttca aagcaagtgg 240
   attgatgtga tatctccact gacgtaaggg atgacgcaca atcccactat ccttcgcaag 300
   acccttcctc tatataagga agttcatttc atttggagag gtattaaaat cttaataggt 360
   tttgataaaa gcgaacgtgg ggaaacccga accaaacctt cttctaaact ctctctcatc 420
   tctcttaaag caaacttctc tcttgtcttt cttgcgtgag cgatcttcaa cgttgtcaga 480
   tcgtgcttcg gcaccagtac aacgttttct ttcactgaag cgaaatcaaa gatctctttg 540
   tggacacgta gtgcggcgcc attaaataac gtgtacttgt cctattcttg tcggtgtggt 600
   cttgggaaaa gaaagcttgc tggaggctgc tgttcagccc catacattac ttgttacgat 660
   tctgctgact ttcggcgggt gcaatatctc tacttctgct tgacgaggta ttgttgcctg 720
   tacttctttc ttcttcttct tgctgattgg ttctataaga aatctagtat tttctttgaa 780
   acagagtttt cccgtggttt tcgaacttgg agaaagattg ttaagcttct gtatattctg 840
   cccaaatttg tcgggcccat ggttttcaca cctcagatac ttggacttat gcttttttgg 900
   atttcagcct ccagaggtga tattgtgcta actcagtctc cagccaccct gtctgtgact 960
   ccaggagata gtgtcagtct ttcctgcagg gccagccaaa gtattagcaa caacctacac 1020
   tggtttcaac aaaaatcgca tgagtctcca aggcttctca tcaagtatgc ttcccagtcc 1080
   atatctggga tcccctccag gttcagtggc agtggatctg ggacagattt cactctcagt 1140
   atcaacagtg tgaagactga agattttgga atgtttttct gtcaacagag taacagctgg 1200
   cctctcacgt tcggtgatgg gacaaagctg gagctgaaac gggctgatgc tgcaccaact 1260
   gtatccatct tcccaccatc cagtgagcag ttaacatctg gaggtgcctc agtcgtgtgc 1320
   ttcttgaaca acttctaccc caaagacatc aatgtcaagt ggaagattga tggcagtgaa 1380
   cgacaaaatg gcgtcctgaa cagttggact gatcaggaca gcaaagacag cacctacagc 1440
   atgagcagca ccctcacgtt gaccaaggac gagtatgaac gacataacag ctatacctgt 1500
   gaggccactc acaagacatc aacttcaccc attgtcaaga gcttcaacag gaatgagtgt 1560
   tagaggccta ttttctttag tttgaattta ctgttattcg gtgtgcattt ctatgtttgg 1620
   tgagcggttt tctgtgctca gagtgtgttt attttatgta atttaatttc tttgtgagct 1680
   cctgtttagc aggtcgtccc ttcagcaagg acacaaaaag attttaattt tattaaaaaa 1740
   aaaaaaaaaa aagaccggga attcgatatc aagcttatcg acctgcagat cgttcaaaca 1800
   tttggcaata aagtttctta agattgaatc ctgttgccgg tcttgcgatg attatcatat 1860
   aatttctgtt gaattacgtt aagcatgtaa taattaacat gtaatgcatg acgttattta 1920
   tgagatgggt ttttatgatt agagtcccgc aattatacat ttaatacgcg atagaaaaca 1980
   aaatatagcg cgcaaactag gataaattat cgcgcgcggt gtcatctatg ttactagatt 2040
   ctagagtctc aagcttcggc gcgcc 2065
<210> 82
   <211> 48
   <212> DNA
   <213> Artificial sequence
<220>
   <223> SpPDI-HA(Ind).c
<400> 82
   gttccttctc agatcttcgc tgatcagatt tgcattggtt accatgca 48
<210> 83
   <211> 3218
   <212> DNA
   <213> Artificial sequence
<220>
   <223> Construct number 663, from HindIII
<400> 83
   aagcttgcta gcggcctcaa tggccctgca ggtcgactct agaggtaccc cgggctggta 60
   tatttatatg ttgtcaaata actcaaaaac cataaaagtt taagttagca agtgtgtaca 120
   tttttacttg aacaaaaata ttcacctact actgttataa atcattatta aacattagag 180
   taaagaaata tggatgataa gaacaagagt agtgatattt tgacaacaat tttgttgcaa 240
   catttgagaa aattttgttg ttctctcttt tcattggtca aaaacaatag agagagaaaa 300
   aggaagaggg agaataaaaa cataatgtga gtatgagaga gaaagttgta caaaagttgt 360
   accaaaatag ttgtacaaat atcattgagg aatttgacaa aagctacaca aataagggtt 420
   aattgctgta aataaataag gatgacgcat tagagagatg taccattaga gaatttttgg 480
   caagtcatta aaaagaaaga ataaattatt tttaaaatta aaagttgagt catttgatta 540
   aacatgtgat tatttaatga attgatgaaa gagttggatt aaagttgtat tagtaattag 600
   aatttggtgt caaatttaat ttgacatttg atcttttcct atatattgcc ccatagagtc 660
   agttaactca tttttatatt tcatagatca aataagagaa ataacggtat attaatccct 720
   ccaaaaaaaa aaaacggtat atttactaaa aaatctaagc cacgtaggag gataacagga 780
   tccccgtagg aggataacat ccaatccaac caatcacaac aatcctgatg agataaccca 840
   ctttaagccc acgcatctgt ggcacatcta cattatctaa atcacacatt cttccacaca 900
   tctgagccac acaaaaacca atccacatct ttatcaccca ttctataaaa aatcacactt 960
   tgtgagtcta cactttgatt cccttcaaac acatacaaag agaagagact aattaattaa 1020
   ttaatcatct tgagagaaaa tggcgaaaaa cgttgcgatt ttcggcttat tgttttctct 1080
   tcttgtgttg gttccttctc agatcttcgc tgatcagatt tgcattggtt accatgcaaa 1140
   caattcaaca gagcaggttg acacaatcat ggaaaagaac gttactgtta cacatgccca 1200
   agacatactg gaaaagacac acaacgggaa gctctgcgat ctagatggag tgaagcctct 1260
   aattttaaga gattgtagtg tagctggatg gctcctcggg aacccaatgt gtgacgaatt 1320
   catcaatgta ccggaatggt cttacatagt ggagaaggcc aatccaacca atgacctctg 1380
   ttacccaggg agtttcaacg actatgaaga actgaaacac ctattgagca gaataaacca 1440
   ttttgagaaa attcaaatca tccccaaaag ttcttggtcc gatcatgaag cctcatcagg 1500
   agttagctca gcatgtccat acctgggaag tccctccttt tttagaaatg tggtatggct 1560
   tatcaaaaag aacagtacat acccaacaat aaagaaaagc tacaataata ccaaccaaga 1620
   ggatcttttg gtactgtggg gaattcacca tcctaatgat gcggcagagc agacaaggct 1680
   atatcaaaac ccaaccacct atatttccat tgggacatca acactaaacc agagattggt 1740
   accaaaaata gctactagat ccaaagtaaa cgggcaaagt ggaaggatgg agttcttctg 1800
   gacaatttta aaacctaatg atgcaatcaa cttcgagagt aatggaaatt tcattgctcc 1860
   agaatatgca tacaaaattg tcaagaaagg ggactcagca attatgaaaa gtgaattgga 1920
   atatggtaac tgcaacacca agtgtcaaac tccaatgggg gcgataaact ctagtatgcc 1980
   attccacaac atacaccctc tcaccatcgg ggaatgcccc aaatatgtga aatcaaacag 2040
   attagtcctt gcaacagggc tcagaaatag ccctcaaaga gagagcagaa gaaaaaagag 2100
   aggactattt ggagctatag caggttttat agagggagga tggcagggaa tggtagatgg 2160
   ttggtatggg taccaccata gcaatgagca ggggagtggg tacgctgcag acaaagaatc 2220
   cactcaaaag gcaatagatg gagtcaccaa taaggtcaac tcaatcattg acaaaatgaa 2280
   cactcagttt gaggccgttg gaagggaatt taataactta gaaaggagaa tagagaattt 2340
   aaacaagaag atggaagacg ggtttctaga tgtctggact tataatgccg aacttctggt 2400
   tctcatggaa aatgagagaa ctctagactt tcatgactca aatgttaaga acctctacga 2460
   caaggtccga ctacagctta gggataatgc aaaggagctg ggtaacggtt gtttcgagtt 2520
   ctatcacaaa tgtgataatg aatgtatgga aagtataaga aacggaacgt acaactatcc 2580
   gcagtattca gaagaagcaa gattaaaaag agaggaaata agtggggtaa aattggaatc 2640
   aataggaact taccaaatac tgtcaattta ttcaacagtg gcgagttccc tagcactggc 2700
   aatcatgatg gctggtctat ctttatggat gtgctccaat ggatcgttac aatgcagaat 2760
   ttgcatttaa gagctctaag ttaaaatgct tcttcgtctc ctatttataa tatggtttgt 2820
   tattgttaat tttgttcttg tagaagagct taattaatcg ttgttgttat gaaatactat 2880
   ttgtatgaga tgaactggtg taatgtaatt catttacata agtggagtca gaatcagaat 2940
   gtttcctcca taactaacta gacatgaaga cctgccgcgt acaattgtct tatatttgaa 3000
   caactaaaat tgaacatctt ttgccacaac tttataagtg gttaatatag ctcaaatata 3060
   tggtcaagtt caatagatta ataatggaaa tatcagttat cgaaattcat taacaatcaa 3120
   cttaacgtta ttaactacta attttatatc atcccctttg ataaatgata gtacaccaat 3180
   taggaaggag catgctcgag gcctggctgg ccgaattc 3218
<210> 84
   <211> 49
   <212> DNA
   <213> Artificial sequence
<220>
   <223> SpPDI-H1B.c
<400> 84
   ttctcagatc ttcgctgaca caatatgtat aggctaccat gctaacaac 49
<210> 85
   <211> 47
   <212> DNA
   <213> Artificial sequence
<220>
   <223> SacI-H1B.r
<400> 85
   cttagagctc ttagatgcat attctacact gtaaagaccc attggaa 47
<210> 86
   <211> 3206
   <212> DNA
   <213> Artificial sequence
<220>
   <223> Construct number 787, from HindIII
<400> 86
   aagcttgcta gcggcctcaa tggccctgca ggtcgactct agaggtaccc cgggctggta 60
   tatttatatg ttgtcaaata actcaaaaac cataaaagtt taagttagca agtgtgtaca 120
   tttttacttg aacaaaaata ttcacctact actgttataa atcattatta aacattagag 180
   taaagaaata tggatgataa gaacaagagt agtgatattt tgacaacaat tttgttgcaa 240
   catttgagaa aattttgttg ttctctcttt tcattggtca aaaacaatag agagagaaaa 300
   aggaagaggg agaataaaaa cataatgtga gtatgagaga gaaagttgta caaaagttgt 360
   accaaaatag ttgtacaaat atcattgagg aatttgacaa aagctacaca aataagggtt 420
   aattgctgta aataaataag gatgacgcat tagagagatg taccattaga gaatttttgg 480
   caagtcatta aaaagaaaga ataaattatt tttaaaatta aaagttgagt catttgatta 540
   aacatgtgat tatttaatga attgatgaaa gagttggatt aaagttgtat tagtaattag 600
   aatttggtgt caaatttaat ttgacatttg atcttttcct atatattgcc ccatagagtc 660
   agttaactca tttttatatt tcatagatca aataagagaa ataacggtat attaatccct 720
   ccaaaaaaaa aaaacggtat atttactaaa aaatctaagc cacgtaggag gataacagga 780
   tccccgtagg aggataacat ccaatccaac caatcacaac aatcctgatg agataaccca 840
   ctttaagccc acgcatctgt ggcacatcta cattatctaa atcacacatt cttccacaca 900
   tctgagccac acaaaaacca atccacatct ttatcaccca ttctataaaa aatcacactt 960
   tgtgagtcta cactttgatt cccttcaaac acatacaaag agaagagact aattaattaa 1020
   ttaatcatct tgagagaaaa tggcgaaaaa cgttgcgatt ttcggcttat tgttttctct 1080
   tcttgtgttg gttccttctc agatcttcgc tgacacaata tgtataggct accatgctaa 1140
   caactcgacc gacactgttg acacagtact tgaaaagaat gtgacagtga cacactctgt 1200
   caacctgctt gagaacagtc acaatggaaa actatgtcta ttaaaaggaa tagccccact 1260
   acaattgggt aattgcagcg ttgccgggtg gatcttagga aacccagaat gcgaattact 1320
   gatttccaag gagtcatggt cctacattgt agaaaaacca aatcctgaga atggaacatg 1380
   ttacccaggg catttcgctg actatgagga actgagggag caattgagtt cagtatcttc 1440
   atttgagagg ttcgaaatat tccccaaaga aagctcatgg cccaaccaca ccgtaaccgg 1500
   agtgtcagca tcatgctccc ataatgggga aagcagtttt tacagaaatt tgctatggct 1560
   gacggggaag aatggtttgt acccaaacct gagcaagtcc tatgcaaaca acaaagaaaa 1620
   agaagtcctt gtactatggg gtgttcatca cccgccaaac ataggtgacc aaaaggccct 1680
   ctatcataca gaaaatgctt atgtctctgt agtgtcttca cattatagca gaaaattcac 1740
   cccagaaata gccaaaagac ccaaagtaag agatcaagaa ggaagaatca attactactg 1800
   gactctgctt gaacccgggg atacaataat atttgaggca aatggaaatc taatagcgcc 1860
   aagatatgct ttcgcactga gtagaggctt tggatcagga atcatcaact caaatgcacc 1920
   aatggataaa tgtgatgcga agtgccaaac acctcaggga gctataaaca gcagtcttcc 1980
   tttccagaac gtacacccag tcacaatagg agagtgtcca aagtatgtca ggagtgcaaa 2040
   attaaggatg gttacaggac taaggaacat cccatccatt caatccagag gtttgtttgg 2100
   agccattgcc ggtttcattg aaggggggtg gactggaatg gtagatggtt ggtatggtta 2160
   tcatcatcag aatgagcaag gatctggcta tgctgcagat caaaaaagca cacaaaatgc 2220
   cattaatggg attacaaaca aggtcaattc tgtaattgag aaaatgaaca ctcaattcac 2280
   agcagtgggc aaagagttca acaaattgga aagaaggatg gaaaacttga ataaaaaagt 2340
   tgatgatggg tttatagaca tttggacata taatgcagaa ctgttggttc tactggaaaa 2400
   tgaaaggact ttggatttcc atgactccaa tgtgaagaat ctgtatgaga aagtaaaaag 2460
   ccagttaaag aataatgcta aagaaatagg aaatgggtgt tttgagttct atcacaagtg 2520
   taacgatgaa tgcatggaga gtgtaaagaa tggaacttat gactatccaa aatattccga 2580
   agaatcaaag ttaaacaggg agaaaattga tggagtgaaa ttggaatcaa tgggagtcta 2640
   tcagattctg gcgatctact caacagtcgc cagttctctg gttcttttgg tctccctggg 2700
   ggcaatcagc ttctggatgt gttccaatgg gtctttacag tgtagaatat gcatctaaga 2760
   gctctaagtt aaaatgcttc ttcgtctcct atttataata tggtttgtta ttgttaattt 2820
   tgttcttgta gaagagctta attaatcgtt gttgttatga aatactattt gtatgagatg 2880
   aactggtgta atgtaattca tttacataag tggagtcaga atcagaatgt ttcctccata 2940
   actaactaga catgaagacc tgccgcgtac aattgtctta tatttgaaca actaaaattg 3000
   aacatctttt gccacaactt tataagtggt taatatagct caaatatatg gtcaagttca 3060
   atagattaat aatggaaata tcagttatcg aaattcatta acaatcaact taacgttatt 3120
   aactactaat tttatatcat cccctttgat aaatgatagt acaccaatta ggaaggagca 3180
   tgctcgaggc ctggctggcc gaattc 3206
<210> 87
   <211> 45
   <212> DNA
   <213> Artificial sequence
<220>
   <223> H3B-SpPDI.r
<400> 87
   tgtcatttcc gggaagtttt tgagcgaaga tctgagaagg aacca 45
<210> 88
   <211> 45
   <212> DNA
   <213> Artificial sequence
<220>
   <223> SpPDI-H3B.c
<400> 88
   tctcagatct tcgctcaaaa acttcccgga aatgacaaca gcacg 45
<210> 89
   <211> 23
   <212> DNA
   <213> Artificial sequence
<220>
   <223> H3(A-Bri).982r
<400> 89
   ttgcttaaca tatctgggac agg 23
<210> 90
   <211> 3212
   <212> DNA
   <213> Artificial sequence
<220>
   <223> Construct number 790, from HindIII
<400> 90
   aagcttgcta gcggcctcaa tggccctgca ggtcgactct agaggtaccc cgggctggta     60
   tatttatatg ttgtcaaata actcaaaaac cataaaagtt taagttagca agtgtgtaca    120
   tttttacttg aacaaaaata ttcacctact actgttataa atcattatta aacattagag    180
   taaagaaata tggatgataa gaacaagagt agtgatattt tgacaacaat tttgttgcaa    240
   catttgagaa aattttgttg ttctctcttt tcattggtca aaaacaatag agagagaaaa    300
   aggaagaggg agaataaaaa cataatgtga gtatgagaga gaaagttgta caaaagttgt    360
   accaaaatag ttgtacaaat atcattgagg aatttgacaa aagctacaca aataagggtt    420
   aattgctgta aataaataag gatgacgcat tagagagatg taccattaga gaatttttgg    480
   caagtcatta aaaagaaaga ataaattatt tttaaaatta aaagttgagt catttgatta    540
   aacatgtgat tatttaatga attgatgaaa gagttggatt aaagttgtat tagtaattag    600
   aatttggtgt caaatttaat ttgacatttg atcttttcct atatattgcc ccatagagtc    660
   agttaactca tttttatatt tcatagatca aataagagaa ataacggtat attaatccct    720
   ccaaaaaaaa aaaacggtat atttactaaa aaatctaagc cacgtaggag gataacagga    780
   tccccgtagg aggataacat ccaatccaac caatcacaac aatcctgatg agataaccca    840
   ctttaagccc acgcatctgt ggcacatcta cattatctaa atcacacatt cttccacaca 900
   tctgagccac acaaaaacca atccacatct ttatcaccca ttctataaaa aatcacactt 960
   tgtgagtcta cactttgatt cccttcaaac acatacaaag agaagagact aattaattaa 1020
   ttaatcatct tgagagaaaa tggcgaaaaa cgttgcgatt ttcggcttat tgttttctct 1080
   tcttgtgttg gttccttctc agatcttcgc tcaaaaactt cccggaaatg acaacagcac 1140
   ggcaacgctg tgccttgggc accatgcagt accaaacgga acgatagtga aaacaatcac 1200
   gaatgaccaa attgaagtta ctaatgctac tgagctggtt cagagttcct caacaggtga 1260
   aatatgcgac agtcctcatc agatccttga tggagaaaac tgcacactaa tagatgctct 1320
   attgggagac cctcagtgtg atggcttcca aaataagaaa tgggaccttt ttgttgaacg 1380
   cagcaaagcc tacagcaact gttaccctta tgatgtgccg gattatgcct cccttaggtc 1440
   actagttgcc tcatccggca cactggagtt taacaatgaa agtttcaatt ggactggagt 1500
   cactcaaaac ggaacaagct ctgcttgcat aaggagatct aataacagtt tctttagtag 1560
   attgaattgg ttgacccact taaaattcaa atacccagca ttgaacgtga ctatgccaaa 1620
   caatgaaaaa tttgacaaat tgtacatttg gggggttcac cacccgggta cggacaatga 1680
   ccaaatcttc ctgtatgctc aagcatcagg aagaatcaca gtctctacca aaagaagcca 1740
   acaaactgta atcccgaata tcggatctag acccagagta aggaatatcc ccagcagaat 1800
   aagcatctat tggacaatag taaaaccggg agacatactt ttgattaaca gcacagggaa 1860
   tctaattgct cctaggggtt acttcaaaat acgaagtggg aaaagctcaa taatgagatc 1920
   agatgcaccc attggcaaat gcaattctga atgcatcact ccaaacggaa gcattcccaa 1980
   tgacaaacca ttccaaaatg taaacaggat cacatacggg gcctgtccca gatatgttaa 2040
   gcaaaacact ctgaaattgg caacagggat gcgaaatgta ccagagaaac aaactagagg 2100
   catatttggc gcaatcgcgg gtttcataga aaatggttgg gagggaatgg tggatggttg 2160
   gtatggtttc aggcatcaaa attctgaggg aataggacaa gcagcagatc tcaaaagcac 2220
   tcaagcagca atcgatcaaa tcaatgggaa gctgaatagg ttgatcggga aaaccaacga 2280
   gaaattccat cagattgaaa aagagttctc agaagtcgaa gggagaatcc aggaccttga 2340
   gaaatatgtt gaggacacca aaatagatct ctggtcatac aacgcggagc ttcttgttgc 2400
   cctggagaac caacatacaa ttgatctaac tgactcagaa atgaacaaac tgtttgaaaa 2460
   aacaaagaag caactgaggg aaaatgctga ggatatgggc aatggttgtt tcaaaatata 2520
   ccacaaatgt gacaatgcct gcataggatc aatcagaaat ggaacttatg accacgatgt 2580
   atacagagat gaagcattaa acaaccggtt ccagatcaag ggcgttgagc tgaagtcagg 2640
   atacaaagat tggatactat ggatttcctt tgccatatca tgttttttgc tttgtgttgc 2700
   tttgttgggg ttcatcatgt gggcctgcca aaaaggcaac attaggtgca acatttgcat 2760
   ttgagagctc taagttaaaa tgcttcttcg tctcctattt ataatatggt ttgttattgt 2820
   taattttgtt cttgtagaag agcttaatta atcgttgttg ttatgaaata ctatttgtat 2880
   gagatgaact ggtgtaatgt aattcattta cataagtgga gtcagaatca gaatgtttcc 2940
   tccataacta actagacatg aagacctgcc gcgtacaatt gtcttatatt tgaacaacta 3000
   aaattgaaca tcttttgcca caactttata agtggttaat atagctcaaa tatatggtca 3060
   agttcaatag attaataatg gaaatatcag ttatcgaaat tcattaacaa tcaacttaac 3120
   gttattaact actaatttta tatcatcccc tttgataaat gatagtacac caattaggaa 3180
   ggagcatgct cgaggcctgg ctggccgaat tc 3212
<210> 91
   <211> 50
   <212> DNA
   <213> Artificial sequence
<220>
   <223> HBF-SpPDI.r
<400> 91
   gttattccag tgcagattcg atcagcgaag atctgagaag gaaccaacac 50
<210> 92
   <211> 50
   <212> DNA
   <213> Artificial sequence
<220>
   <223> SpPDI-HBF.c
<400> 92
   cagatcttcg ctgatcgaat ctgcactgga ataacatctt caaactcacc 50
<210> 93
   <211> 28
   <212> DNA
   <213> Artificial sequence
<220>
   <223> Plaster80r
<400> 93
   caaatagtat ttcataacaa caacgatt 28
<210> 94
   <211> 3269
   <212> DNA
   <213> Artificial sequence
<220>
   <223> Construct number 798, from HindIII
<400> 94
   aagcttgcta gcggcctcaa tggccctgca ggtcgactct agaggtaccc cgggctggta 60
   tatttatatg ttgtcaaata actcaaaaac cataaaagtt taagttagca agtgtgtaca 120
   tttttacttg aacaaaaata ttcacctact actgttataa atcattatta aacattagag 180
   taaagaaata tggatgataa gaacaagagt agtgatattt tgacaacaat tttgttgcaa 240
   catttgagaa aattttgttg ttctctcttt tcattggtca aaaacaatag agagagaaaa 300
   aggaagaggg agaataaaaa cataatgtga gtatgagaga gaaagttgta caaaagttgt 360
   accaaaatag ttgtacaaat atcattgagg aatttgacaa aagctacaca aataagggtt 420
   aattgctgta aataaataag gatgacgcat tagagagatg taccattaga gaatttttgg 480
   caagtcatta aaaagaaaga ataaattatt tttaaaatta aaagttgagt catttgatta 540
   aacatgtgat tatttaatga attgatgaaa gagttggatt aaagttgtat tagtaattag 600
   aatttggtgt caaatttaat ttgacatttg atcttttcct atatattgcc ccatagagtc 660
   agttaactca tttttatatt tcatagatca aataagagaa ataacggtat attaatccct 720
   ccaaaaaaaa aaaacggtat atttactaaa aaatctaagc cacgtaggag gataacagga 780
   tccccgtagg aggataacat ccaatccaac caatcacaac aatcctgatg agataaccca 840
   ctttaagccc acgcatctgt ggcacatcta cattatctaa atcacacatt cttccacaca 900
   tctgagccac acaaaaacca atccacatct ttatcaccca ttctataaaa aatcacactt 960
   tgtgagtcta cactttgatt cccttcaaac acatacaaag agaagagact aattaattaa 1020
   ttaatcatct tgagagaaaa tggcgaaaaa cgttgcgatt ttcggcttat tgttttctct 1080
   tcttgtgttg gttccttctc agatcttcgc tgatcgaatc tgcactggaa taacatcttc 1140
   aaactcacct catgtggtca aaacagccac tcaaggggag gtcaatgtga ctggtgtgat 1200
   accactaaca acaacaccaa caaaatctta ttttgcaaat ctcaaaggaa caaggaccag 1260
   agggaaacta tgcccagact gtctcaactg cacagatctg gatgtggctt tgggcagacc 1320
   aatgtgtgtg gggaccacac cttcggcgaa ggcttcaata ctccacgaag tcaaacctgt 1380
   tacatccggg tgctttccta taatgcacga cagaacaaaa atcaggcaac tacccaatct 1440
   tctcagagga tatgaaaata tcaggctatc aacccaaaac gtcatcgatg cggaaaaggc 1500
   accaggagga ccctacagac ttggaacctc aggatcttgc cctaacgcta ccagtaagag 1560
   cggatttttc gcaacaatgg cttgggctgt cccaaaggac aacaacaaaa atgcaacgaa 1620
   cccactaaca gtagaagtac catacatttg tacagaaggg gaagaccaaa tcactgtttg 1680
   ggggttccat tcagataaca aaacccaaat gaagaacctc tatggagact caaatcctca 1740
   aaagttcacc tcatctgcta atggagtaac cacacactat gtttctcaga ttggcagctt 1800
   cccagatcaa acagaagacg gaggactacc acaaagcggc aggattgttg ttgattacat 1860
   gatgcaaaaa cctgggaaaa caggaacaat tgtctaccaa agaggtgttt tgttgcctca 1920
   aaaggtgtgg tgcgcgagtg gcaggagcaa agtaataaaa gggtccttgc ctttaattgg 1980
   tgaagcagat tgccttcatg aaaaatacgg tggattaaac aaaagcaagc cttactacac 2040
   aggagaacat gcaaaagcca taggaaattg cccaatatgg gtgaaaacac ctttgaagct 2100
   cgccaatgga accaaatata gacctcctgc aaaactatta aaggaaaggg gtttcttcgg 2160
   agctattgct ggtttcctag aaggaggatg ggaaggaatg attgcaggct ggcacggata 2220
   cacatctcac ggagcacatg gagtggcagt ggcggcggac cttaagagta cgcaagaagc 2280
   tataaacaag ataacaaaaa atctcaattc tttgagtgag ctagaagtaa agaatcttca 2340
   aagactaagt ggtgccatgg atgaactcca caacgaaata ctcgagctgg atgagaaagt 2400
   ggatgatctc agagctgaca ctataagctc gcaaatagaa cttgcagtct tgctttccaa 2460
   cgaaggaata ataaacagtg aagatgagca tctattggca cttgagagaa aactaaagaa 2520
   aatgctgggt ccctctgctg tagagatagg aaatggatgc ttcgaaacca aacacaagtg 2580
   caaccagacc tgcttagaca ggatagctgc tggcaccttt aatgcaggag aattttctct 2640
   ccccactttt gattcactga acattactgc tgcatcttta aatgatgatg gattggataa 2700
   ccatactata ctgctctatt actcaactgc tgcttctagt ttggctgtaa cattgatgct 2760
   agctattttt attgtttata tggtctccag agacaacgtt tcatgctcca tctgtctata 2820
   agagctctaa gttaaaatgc ttcttcgtct cctatttata atatggtttg ttattgttaa 2880
   ttttgttctt gtagaagagc ttaattaatc gttgttgtta tgaaatacta tttgtatgag 2940
   atgaactggt gtaatgtaat tcatttacat aagtggagtc agaatcagaa tgtttcctcc 3000
   ataactaact agacatgaag acctgccgcg tacaattgtc ttatatttga acaactaaaa 3060
   ttgaacatct tttgccacaa ctttataagt ggttaatata gctcaaatat atggtcaagt 3120
   tcaatagatt aataatggaa atatcagtta tcgaaattca ttaacaatca acttaacgtt 3180
   attaactact aattttatat catccccttt gataaatgat agtacaccaa ttaggaagga 3240
   gcatgctcga ggcctggctg gccgaattc 3269
<210> 95
   <211> 45
   <212> DNA
   <213> Artificial sequence
<220>
   <223> ApaI-SpPDI.c
<400> 95
   ttgtcgggcc catggcgaaa aacgttgcga ttttcggctt attgt 45
<210> 96
   <211> 42
   <212> DNA
   <213> Artificial sequence
<220>
   <223> StuI-H1(A-NC).r
<400> 96
   aaaataggcc tttagatgca tattctacac tgcaaagacc ca 42
<210> 97
   <211> 3079
   <212> DNA
   <213> Artificial sequence
<220>
   <223> Construct number 580, from PacI
<400> 97
   ttaattaaga attcgagctc caccgcggaa acctcctcgg attccattgc ccagctatct 60
   gtcactttat tgagaagata gtggaaaagg aaggtggctc ctacaaatgc catcattgcg 120
   ataaaggaaa ggccatcgtt gaagatgcct ctgccgacag tggtcccaaa gatggacccc 180
   cacccacgag gagcatcgtg gaaaaagaag acgttccaac cacgtcttca aagcaagtgg 240
   attgatgtga tatctccact gacgtaaggg atgacgcaca atcccactat ccttcgcaag 300
   acccttcctc tatataagga agttcatttc atttggagag gtattaaaat cttaataggt 360
   tttgataaaa gcgaacgtgg ggaaacccga accaaacctt cttctaaact ctctctcatc 420
   tctcttaaag caaacttctc tcttgtcttt cttgcgtgag cgatcttcaa cgttgtcaga 480
   tcgtgcttcg gcaccagtac aacgttttct ttcactgaag cgaaatcaaa gatctctttg 540
   tggacacgta gtgcggcgcc attaaataac gtgtacttgt cctattcttg tcggtgtggt 600
   cttgggaaaa gaaagcttgc tggaggctgc tgttcagccc catacattac ttgttacgat 660
   tctgctgact ttcggcgggt gcaatatctc tacttctgct tgacgaggta ttgttgcctg 720
   tacttctttc ttcttcttct tgctgattgg ttctataaga aatctagtat tttctttgaa 780
   acagagtttt cccgtggttt tcgaacttgg agaaagattg ttaagcttct gtatattctg 840
   cccaaatttg tcgggcccat ggcgaaaaac gttgcgattt tcggcttatt gttttctctt 900
   cttgtgttgg ttccttctca gatcttcgct gacacaatat gtataggcta ccatgccaac 960
   aactcaaccg acactgttga cacagtactt gagaagaatg tgacagtgac acactctgtc 1020
   aacctacttg aggacagtca caatggaaaa ctatgtctac taaaaggaat agccccacta 1080
   caattgggta attgcagcgt tgccggatgg atcttaggaa acccagaatg cgaattactg 1140
   atttccaagg aatcatggtc ctacattgta gaaacaccaa atcctgagaa tggaacatgt 1200
   tacccagggt atttcgccga ctatgaggaa ctgagggagc aattgagttc agtatcttca 1260
   tttgagagat tcgaaatatt ccccaaagaa agctcatggc ccaaccacac cgtaaccgga 1320
   gtatcagcat catgctccca taatgggaaa agcagttttt acagaaattt gctatggctg 1380
   acggggaaga atggtttgta cccaaacctg agcaagtcct atgtaaacaa caaagagaaa 1440
   gaagtccttg tactatgggg tgttcatcac ccgcctaaca tagggaacca aagggcactc 1500
   tatcatacag aaaatgctta tgtctctgta gtgtcttcac attatagcag aagattcacc 1560
   ccagaaatag ccaaaagacc caaagtaaga gatcaggaag gaagaatcaa ctactactgg 1620
   actctgctgg aacctgggga tacaataata tttgaggcaa atggaaatct aatagcgcca 1680
   tggtatgctt ttgcactgag tagaggcttt ggatcaggaa tcatcacctc aaatgcacca 1740
   atggatgaat gtgatgcgaa gtgtcaaaca cctcagggag ctataaacag cagtcttcct 1800
   ttccagaatg tacacccagt cacaatagga gagtgtccaa agtatgtcag gagtgcaaaa 1860
   ttaaggatgg ttacaggact aaggaacatc ccatccattc aatccagagg tttgtttgga 1920
   gccattgccg gtttcattga aggggggtgg actggaatgg tagatgggtg gtatggttat 1980
   catcatcaga atgagcaagg atctggctat gctgcagatc aaaaaagtac acaaaatgcc 2040
   attaacggga ttacaaacaa ggtcaattct gtaattgaga aaatgaacac tcaattcaca 2100
   gctgtgggca aagagttcaa caaattggaa agaaggatgg aaaacttaaa taaaaaagtt 2160
   gatgatgggt ttctagacat ttggacatat aatgcagaat tgttggttct actggaaaat 2220
   gaaaggactt tggatttcca tgactccaat gtgaagaatc tgtatgagaa agtaaaaagc 2280
   caattaaaga ataatgccaa agaaatagga aacgggtgtt ttgagttcta tcacaagtgt 2340
   aacaatgaat gcatggagag tgtgaaaaat ggtacctatg actatccaaa atattccgaa 2400
   gaatcaaagt taaacaggga gaaaattgat ggagtgaaat tggaatcaat gggagtatac 2460
   cagattctgg cgatctactc aactgtcgcc agttccctgg ttcttttggt ctccctgggg 2520
   gcaatcagct tctggatgtg ttccaatggg tctttgcagt gtagaatatg catctaaagg 2580
   cctattttct ttagtttgaa tttactgtta ttcggtgtgc atttctatgt ttggtgagcg 2640
   gttttctgtg ctcagagtgt gtttatttta tgtaatttaa tttctttgtg agctcctgtt 2700
   tagcaggtcg tcccttcagc aaggacacaa aaagatttta attttattaa aaaaaaaaaa 2760
   aaaaaagacc gggaattcga tatcaagctt atcgacctgc agatcgttca aacatttggc 2820
   aataaagttt cttaagattg aatcctgttg ccggtcttgc gatgattatc atataatttc 2880
   tgttgaatta cgttaagcat gtaataatta acatgtaatg catgacgtta tttatgagat 2940
   gggtttttat gattagagtc ccgcaattat acatttaata cgcgatagaa aacaaaatat 3000
   agcgcgcaaa ctaggataaa ttatcgcgcg cggtgtcatc tatgttacta gattctagag 3060
   tctcaagctt cggcgcgcc 3079
<210> 98
   <211> 39
   <212> DNA
   <213> Artificial sequence
<220>
   <223> ApaI-H5 (A-Indo).1c
<400> 98
   tgtcgggccc atggagaaaa tagtgcttct tcttgcaat 39
<210> 99
   <211> 37
   <212> DNA
   <213> Artificial sequence
<220>
   <223> H5 (A-Indo)-StuI.1707r
<400> 99
   aaataggcct ttaaatgcaa attctgcatt gtaacga 37
<210> 100
   <211> 3067
   <212> DNA
   <213> Artificial sequence
<220>
   <223> Construct number 685, from PacI
<400> 100
   ttaattaaga attcgagctc caccgcggaa acctcctcgg attccattgc ccagctatct 60
   gtcactttat tgagaagata gtggaaaagg aaggtggctc ctacaaatgc catcattgcg 120
   ataaaggaaa ggccatcgtt gaagatgcct ctgccgacag tggtcccaaa gatggacccc 180
   cacccacgag gagcatcgtg gaaaaagaag acgttccaac cacgtcttca aagcaagtgg 240
   attgatgtga tatctccact gacgtaaggg atgacgcaca atcccactat ccttcgcaag 300
   acccttcctc tatataagga agttcatttc atttggagag gtattaaaat cttaataggt 360
   tttgataaaa gcgaacgtgg ggaaacccga accaaacctt cttctaaact ctctctcatc 420
   tctcttaaag caaacttctc tcttgtcttt cttgcgtgag cgatcttcaa cgttgtcaga 480
   tcgtgcttcg gcaccagtac aacgttttct ttcactgaag cgaaatcaaa gatctctttg 540
   tggacacgta gtgcggcgcc attaaataac gtgtacttgt cctattcttg tcggtgtggt 600
   cttgggaaaa gaaagcttgc tggaggctgc tgttcagccc catacattac ttgttacgat 660
   tctgctgact ttcggcgggt gcaatatctc tacttctgct tgacgaggta ttgttgcctg 720
   tacttctttc ttcttcttct tgctgattgg ttctataaga aatctagtat tttctttgaa 780
   acagagtttt cccgtggttt tcgaacttgg agaaagattg ttaagcttct gtatattctg 840
   cccaaatttg tcgggcccat ggagaaaata gtgcttcttc ttgcaatagt cagtcttgtt 900
   aaaagtgatc agatttgcat tggttaccat gcaaacaatt caacagagca ggttgacaca 960
   atcatggaaa agaacgttac tgttacacat gcccaagaca tactggaaaa gacacacaac 1020
   gggaagctct gcgatctaga tggagtgaag cctctaattt taagagattg tagtgtagct 1080
   ggatggctcc tcgggaaccc aatgtgtgac gaattcatca atgtaccgga atggtcttac 1140
   atagtggaga aggccaatcc aaccaatgac ctctgttacc cagggagttt caacgactat 1200
   gaagaactga aacacctatt gagcagaata aaccattttg agaaaattca aatcatcccc 1260
   aaaagttctt ggtccgatca tgaagcctca tcaggagtta gctcagcatg tccatacctg 1320
   ggaagtccct ccttttttag aaatgtggta tggcttatca aaaagaacag tacataccca 1380
   acaataaaga aaagctacaa taataccaac caagaggatc ttttggtact gtggggaatt 1440
   caccatccta atgatgcggc agagcagaca aggctatatc aaaacccaac cacctatatt 1500
   tccattggga catcaacact aaaccagaga ttggtaccaa aaatagctac tagatccaaa 1560
   gtaaacgggc aaagtggaag gatggagttc ttctggacaa ttttaaaacc taatgatgca 1620
   atcaacttcg agagtaatgg aaatttcatt gctccagaat atgcatacaa aattgtcaag 1680
   aaaggggact cagcaattat gaaaagtgaa ttggaatatg gtaactgcaa caccaagtgt 1740
   caaactccaa tgggggcgat aaactctagt atgccattcc acaacataca ccctctcacc 1800
   atcggggaat gccccaaata tgtgaaatca aacagattag tccttgcaac agggctcaga 1860
   aatagccctc aaagagagag cagaagaaaa aagagaggac tatttggagc tatagcaggt 1920
   tttatagagg gaggatggca gggaatggta gatggttggt atgggtacca ccatagcaat 1980
   gagcagggga gtgggtacgc tgcagacaaa gaatccactc aaaaggcaat agatggagtc 2040
   accaataagg tcaactcaat cattgacaaa atgaacactc agtttgaggc cgttggaagg 2100
   gaatttaata acttagaaag gagaatagag aatttaaaca agaagatgga agacgggttt 2160
   ctagatgtct ggacttataa tgccgaactt ctggttctca tggaaaatga gagaactcta 2220
   gactttcatg actcaaatgt taagaacctc tacgacaagg tccgactaca gcttagggat 2280
   aatgcaaagg agctgggtaa cggttgtttc gagttctatc acaaatgtga taatgaatgt 2340
   atggaaagta taagaaacgg aacgtacaac tatccgcagt attcagaaga agcaagatta 2400
   aaaagagagg aaataagtgg ggtaaaattg gaatcaatag gaacttacca aatactgtca 2460
   atttattcaa cagtggcgag ttccctagca ctggcaatca tgatggctgg tctatcttta 2520
   tggatgtgct ccaatggatc gttacaatgc agaatttgca tttaaaggcc tattttcttt 2580
   agtttgaatt tactgttatt cggtgtgcat ttctatgttt ggtgagcggt tttctgtgct 2640
   cagagtgtgt ttattttatg taatttaatt tctttgtgag ctcctgttta gcaggtcgtc 2700
   ccttcagcaa ggacacaaaa agattttaat tttattaaaa aaaaaaaaaa aaaagaccgg 2760
   gaattcgata tcaagcttat cgacctgcag atcgttcaaa catttggcaa taaagtttct 2820
   taagattgaa tcctgttgcc ggtcttgcga tgattatcat ataatttctg ttgaattacg 2880
   ttaagcatgt aataattaac atgtaatgca tgacgttatt tatgagatgg gtttttatga 2940
   ttagagtccc gcaattatac atttaatacg cgatagaaaa caaaatatag cgcgcaaact 3000
   aggataaatt atcgcgcgcg gtgtcatcta tgttactaga ttctagagtc tcaagcttcg 3060
   gcgcgcc 3067
<210> 101
   <211> 3091
   <212> DNA
   <213> Artificial sequence
<220>
   <223> Construct number 686, from PacI
<400> 101
   ttaattaaga attcgagctc caccgcggaa acctcctcgg attccattgc ccagctatct 60
   gtcactttat tgagaagata gtggaaaagg aaggtggctc ctacaaatgc catcattgcg 120
   ataaaggaaa ggccatcgtt gaagatgcct ctgccgacag tggtcccaaa gatggacccc 180
   cacccacgag gagcatcgtg gaaaaagaag acgttccaac cacgtcttca aagcaagtgg 240
   attgatgtga tatctccact gacgtaaggg atgacgcaca atcccactat ccttcgcaag 300
   acccttcctc tatataagga agttcatttc atttggagag gtattaaaat cttaataggt 360
   tttgataaaa gcgaacgtgg ggaaacccga accaaacctt cttctaaact ctctctcatc 420
   tctcttaaag caaacttctc tcttgtcttt cttgcgtgag cgatcttcaa cgttgtcaga 480
   tcgtgcttcg gcaccagtac aacgttttct ttcactgaag cgaaatcaaa gatctctttg 540
   tggacacgta gtgcggcgcc attaaataac gtgtacttgt cctattcttg tcggtgtggt 600
   cttgggaaaa gaaagcttgc tggaggctgc tgttcagccc catacattac ttgttacgat 660
   tctgctgact ttcggcgggt gcaatatctc tacttctgct tgacgaggta ttgttgcctg 720
   tacttctttc ttcttcttct tgctgattgg ttctataaga aatctagtat tttctttgaa 780
   acagagtttt cccgtggttt tcgaacttgg agaaagattg ttaagcttct gtatattctg 840
   cccaaatttg tcgggcccat ggcgaaaaac gttgcgattt tcggcttatt gttttctctt 900
   cttgtgttgg ttccttctca gatcttcgct gatcagattt gcattggtta ccatgcaaac 960
   aattcaacag agcaggttga cacaatcatg gaaaagaacg ttactgttac acatgcccaa 1020
   gacatactgg aaaagacaca caacgggaag ctctgcgatc tagatggagt gaagcctcta 1080
   attttaagag attgtagtgt agctggatgg ctcctcggga acccaatgtg tgacgaattc 1140
   atcaatgtac cggaatggtc ttacatagtg gagaaggcca atccaaccaa tgacctctgt 1200
   tacccaggga gtttcaacga ctatgaagaa ctgaaacacc tattgagcag aataaaccat 1260
   tttgagaaaa ttcaaatcat ccccaaaagt tcttggtccg atcatgaagc ctcatcagga 1320
   gttagctcag catgtccata cctgggaagt ccctcctttt ttagaaatgt ggtatggctt 1380
   atcaaaaaga acagtacata cccaacaata aagaaaagct acaataatac caaccaagag 1440
   gatcttttgg tactgtgggg aattcaccat cctaatgatg cggcagagca gacaaggcta 1500
   tatcaaaacc caaccaccta tatttccatt gggacatcaa cactaaacca gagattggta 1560
   ccaaaaatag ctactagatc caaagtaaac gggcaaagtg gaaggatgga gttcttctgg 1620
   acaattttaa aacctaatga tgcaatcaac ttcgagagta atggaaattt cattgctcca 1680
   gaatatgcat acaaaattgt caagaaaggg gactcagcaa ttatgaaaag tgaattggaa 1740
   tatggtaact gcaacaccaa gtgtcaaact ccaatggggg cgataaactc tagtatgcca 1800
   ttccacaaca tacaccctct caccatcggg gaatgcccca aatatgtgaa atcaaacaga 1860
   ttagtccttg caacagggct cagaaatagc cctcaaagag agagcagaag aaaaaagaga 1920
   ggactatttg gagctatagc aggttttata gagggaggat ggcagggaat ggtagatggt 1980
   tggtatgggt accaccatag caatgagcag gggagtgggt acgctgcaga caaagaatcc 2040
   actcaaaagg caatagatgg agtcaccaat aaggtcaact caatcattga caaaatgaac 2100
   actcagtttg aggccgttgg aagggaattt aataacttag aaaggagaat agagaattta 2160
   aacaagaaga tggaagacgg gtttctagat gtctggactt ataatgccga acttctggtt 2220
   ctcatggaaa atgagagaac tctagacttt catgactcaa atgttaagaa cctctacgac 2280
   aaggtccgac tacagcttag ggataatgca aaggagctgg gtaacggttg tttcgagttc 2340
   tatcacaaat gtgataatga atgtatggaa agtataagaa acggaacgta caactatccg 2400
   cagtattcag aagaagcaag attaaaaaga gaggaaataa gtggggtaaa attggaatca 2460
   ataggaactt accaaatact gtcaatttat tcaacagtgg cgagttccct agcactggca 2520
   atcatgatgg ctggtctatc tttatggatg tgctccaatg gatcgttaca atgcagaatt 2580
   tgcatttaaa ggcctatttt ctttagtttg aatttactgt tattcggtgt gcatttctat 2640
   gtttggtgag cggttttctg tgctcagagt gtgtttattt tatgtaattt aatttctttg 2700
   tgagctcctg tttagcaggt cgtcccttca gcaaggacac aaaaagattt taattttatt 2760
   aaaaaaaaaa aaaaaaaaga ccgggaattc gatatcaagc ttatcgacct gcagatcgtt 2820
   caaacatttg gcaataaagt ttcttaagat tgaatcctgt tgccggtctt gcgatgatta 2880
   tcatataatt tctgttgaat tacgttaagc atgtaataat taacatgtaa tgcatgacgt 2940
   tatttatgag atgggttttt atgattagag tcccgcaatt atacatttaa tacgcgatag 3000
   aaaacaaaat atagcgcgca aactaggata aattatcgcg cgcggtgtca tctatgttac 3060
   tagattctag agtctcaagc ttcggcgcgc c 3091
<210> 102
   <211> 45
   <212> DNA
   <213> Artificial sequence
<220>
   <223> ApaI-H1B.c
<400> 102
   tgtcgggccc atgaaagtaa aactactggt cctgttatgc acatt 45
<210> 103
   <211> 46
   <212> DNA
   <213> Artificial sequence
<220>
   <223> StuI-H2B.r
<400> 103
   aaataggcct ttagatgcat attctacact gtaaagaccc attgga 46
<210> 104
   <211> 3058
   <212> DNA
   <213> Artificial sequence
<220>
   <223> Construct 732, from PacI
<400> 104
   ttaattaaga attcgagctc caccgcggaa acctcctcgg attccattgc ccagctatct 60
   gtcactttat tgagaagata gtggaaaagg aaggtggctc ctacaaatgc catcattgcg 120
   ataaaggaaa ggccatcgtt gaagatgcct ctgccgacag tggtcccaaa gatggacccc 180
   cacccacgag gagcatcgtg gaaaaagaag acgttccaac cacgtcttca aagcaagtgg 240
   attgatgtga tatctccact gacgtaaggg atgacgcaca atcccactat ccttcgcaag 300
   acccttcctc tatataagga agttcatttc atttggagag gtattaaaat cttaataggt 360
   tttgataaaa gcgaacgtgg ggaaacccga accaaacctt cttctaaact ctctctcatc 420
   tctcttaaag caaacttctc tcttgtcttt cttgcgtgag cgatcttcaa cgttgtcaga 480
   tcgtgcttcg gcaccagtac aacgttttct ttcactgaag cgaaatcaaa gatctctttg 540
   tggacacgta gtgcggcgcc attaaataac gtgtacttgt cctattcttg tcggtgtggt 600
   cttgggaaaa gaaagcttgc tggaggctgc tgttcagccc catacattac ttgttacgat 660
   tctgctgact ttcggcgggt gcaatatctc tacttctgct tgacgaggta ttgttgcctg 720
   tacttctttc ttcttcttct tgctgattgg ttctataaga aatctagtat tttctttgaa 780
   acagagtttt cccgtggttt tcgaacttgg agaaagattg ttaagcttct gtatattctg 840
   cccaaatttg tcgggcccat gaaagtaaaa ctactggtcc tgttatgcac atttacagct 900
   acatatgcag acacaatatg tataggctac catgctaaca actcgaccga cactgttgac 960
   acagtacttg aaaagaatgt gacagtgaca cactctgtca acctgcttga gaacagtcac 1020
   aatggaaaac tatgtctatt aaaaggaata gccccactac aattgggtaa ttgcagcgtt 1080
   gccgggtgga tcttaggaaa cccagaatgc gaattactga tttccaagga gtcatggtcc 1140
   tacattgtag aaaaaccaaa tcctgagaat ggaacatgtt acccagggca tttcgctgac 1200
   tatgaggaac tgagggagca attgagttca gtatcttcat ttgagaggtt cgaaatattc 1260
   cccaaagaaa gctcatggcc caaccacacc gtaaccggag tgtcagcatc atgctcccat 1320
   aatggggaaa gcagttttta cagaaatttg ctatggctga cggggaagaa tggtttgtac 1380
   ccaaacctga gcaagtccta tgcaaacaac aaagaaaaag aagtccttgt actatggggt 1440
   gttcatcacc cgccaaacat aggtgaccaa aaggccctct atcatacaga aaatgcttat 1500
   gtctctgtag tgtcttcaca ttatagcaga aaattcaccc cagaaatagc caaaagaccc 1560
   aaagtaagag atcaagaagg aagaatcaat tactactgga ctctgcttga acccggggat 1620
   acaataatat ttgaggcaaa tggaaatcta atagcgccaa gatatgcttt cgcactgagt 1680
   agaggctttg gatcaggaat catcaactca aatgcaccaa tggataaatg tgatgcgaag 1740
   tgccaaacac ctcagggagc tataaacagc agtcttcctt tccagaacgt acacccagtc 1800
   acaataggag agtgtccaaa gtatgtcagg agtgcaaaat taaggatggt tacaggacta 1860
   aggaacatcc catccattca atccagaggt ttgtttggag ccattgccgg tttcattgaa 1920
   ggggggtgga ctggaatggt agatggttgg tatggttatc atcatcagaa tgagcaagga 1980
   tctggctatg ctgcagatca aaaaagcaca caaaatgcca ttaatgggat tacaaacaag 2040
   gtcaattctg taattgagaa aatgaacact caattcacag cagtgggcaa agagttcaac 2100
   aaattggaaa gaaggatgga aaacttgaat aaaaaagttg atgatgggtt tatagacatt 2160
   tggacatata atgcagaact gttggttcta ctggaaaatg aaaggacttt ggatttccat 2220
   gactccaatg tgaagaatct gtatgagaaa gtaaaaagcc agttaaagaa taatgctaaa 2280
   gaaataggaa atgggtgttt tgagttctat cacaagtgta acgatgaatg catggagagt 2340
   gtaaagaatg gaacttatga ctatccaaaa tattccgaag aatcaaagtt aaacagggag 2400
   aaaattgatg gagtgaaatt ggaatcaatg ggagtctatc agattctggc gatctactca 2460
   acagtcgcca gttctctggt tcttttggtc tccctggggg caatcagctt ctggatgtgt 2520
   tccaatgggt ctttacagtg tagaatatgc atctaaaggc ctattttctt tagtttgaat 2580
   ttactgttat tcggtgtgca tttctatgtt tggtgagcgg ttttctgtgc tcagagtgtg 2640
   tttattttat gtaatttaat ttctttgtga gctcctgttt agcaggtcgt cccttcagca 2700
   aggacacaaa aagattttaa ttttattaaa aaaaaaaaaa aaaaagaccg ggaattcgat 2760
   atcaagctta tcgacctgca gatcgttcaa acatttggca ataaagtttc ttaagattga 2820
   atcctgttgc cggtcttgcg atgattatca tataatttct gttgaattac gttaagcatg 2880
   taataattaa catgtaatgc atgacgttat ttatgagatg ggtttttatg attagagtcc 2940
   cgcaattata catttaatac gcgatagaaa acaaaatata gcgcgcaaac taggataaat 3000
   tatcgcgcgc ggtgtcatct atgttactag attctagagt ctcaagcttc ggcgcgcc 3058
<210> 105
   <211> 3079
   <212> DNA
   <213> Artificial sequence
<220>
   <223> Construct number 733, from PacI
<400> 105
   ttaattaaga attcgagctc caccgcggaa acctcctcgg attccattgc ccagctatct     60
   gtcactttat tgagaagata gtggaaaagg aaggtggctc ctacaaatgc catcattgcg    120
   ataaaggaaa ggccatcgtt gaagatgcct ctgccgacag tggtcccaaa gatggacccc    180
   cacccacgag gagcatcgtg gaaaaagaag acgttccaac cacgtcttca aagcaagtgg    240
   attgatgtga tatctccact gacgtaaggg atgacgcaca atcccactat ccttcgcaag 300
   acccttcctc tatataagga agttcatttc atttggagag gtattaaaat cttaataggt 360
   tttgataaaa gcgaacgtgg ggaaacccga accaaacctt cttctaaact ctctctcatc 420
   tctcttaaag caaacttctc tcttgtcttt cttgcgtgag cgatcttcaa cgttgtcaga 480
   tcgtgcttcg gcaccagtac aacgttttct ttcactgaag cgaaatcaaa gatctctttg 540
   tggacacgta gtgcggcgcc attaaataac gtgtacttgt cctattcttg tcggtgtggt 600
   cttgggaaaa gaaagcttgc tggaggctgc tgttcagccc catacattac ttgttacgat 660
   tctgctgact ttcggcgggt gcaatatctc tacttctgct tgacgaggta ttgttgcctg 720
   tacttctttc ttcttcttct tgctgattgg ttctataaga aatctagtat tttctttgaa 780
   acagagtttt cccgtggttt tcgaacttgg agaaagattg ttaagcttct gtatattctg 840
   cccaaatttg tcgggcccat ggcgaaaaac gttgcgattt tcggcttatt gttttctctt 900
   cttgtgttgg ttccttctca gatcttcgct gacacaatat gtataggcta ccatgctaac 960
   aactcgaccg acactgttga cacagtactt gaaaagaatg tgacagtgac acactctgtc 1020
   aacctgcttg agaacagtca caatggaaaa ctatgtctat taaaaggaat agccccacta 1080
   caattgggta attgcagcgt tgccgggtgg atcttaggaa acccagaatg cgaattactg 1140
   atttccaagg agtcatggtc ctacattgta gaaaaaccaa atcctgagaa tggaacatgt 1200
   tacccagggc atttcgctga ctatgaggaa ctgagggagc aattgagttc agtatcttca 1260
   tttgagaggt tcgaaatatt ccccaaagaa agctcatggc ccaaccacac cgtaaccgga 1320
   gtgtcagcat catgctccca taatggggaa agcagttttt acagaaattt gctatggctg 1380
   acggggaaga atggtttgta cccaaacctg agcaagtcct atgcaaacaa caaagaaaaa 1440
   gaagtccttg tactatgggg tgttcatcac ccgccaaaca taggtgacca aaaggccctc 1500
   tatcatacag aaaatgctta tgtctctgta gtgtcttcac attatagcag aaaattcacc 1560
   ccagaaatag ccaaaagacc caaagtaaga gatcaagaag gaagaatcaa ttactactgg 1620
   actctgcttg aacccgggga tacaataata tttgaggcaa atggaaatct aatagcgcca 1680
   agatatgctt tcgcactgag tagaggcttt ggatcaggaa tcatcaactc aaatgcacca 1740
   atggataaat gtgatgcgaa gtgccaaaca cctcagggag ctataaacag cagtcttcct 1800
   ttccagaacg tacacccagt cacaatagga gagtgtccaa agtatgtcag gagtgcaaaa 1860
   ttaaggatgg ttacaggact aaggaacatc ccatccattc aatccagagg tttgtttgga 1920
   gccattgccg gtttcattga aggggggtgg actggaatgg tagatggttg gtatggttat 1980
   catcatcaga atgagcaagg atctggctat gctgcagatc aaaaaagcac acaaaatgcc 2040
   attaatggga ttacaaacaa ggtcaattct gtaattgaga aaatgaacac tcaattcaca 2100
   gcagtgggca aagagttcaa caaattggaa agaaggatgg aaaacttgaa taaaaaagtt 2160
   gatgatgggt ttatagacat ttggacatat aatgcagaac tgttggttct actggaaaat 2220
   gaaaggactt tggatttcca tgactccaat gtgaagaatc tgtatgagaa agtaaaaagc   2280
   cagttaaaga ataatgctaa agaaatagga aatgggtgtt ttgagttcta tcacaagtgt   2340
   aacgatgaat gcatggagag tgtaaagaat ggaacttatg actatccaaa atattccgaa   2400
   gaatcaaagt taaacaggga gaaaattgat ggagtgaaat tggaatcaat gggagtctat   2460
   cagattctgg cgatctactc aacagtcgcc agttctctgg ttcttttggt ctccctgggg   2520
   gcaatcagct tctggatgtg ttccaatggg tctttacagt gtagaatatg catctaaagg   2580
   cctattttct ttagtttgaa tttactgtta ttcggtgtgc atttctatgt ttggtgagcg   2640
   gttttctgtg ctcagagtgt gtttatttta tgtaatttaa tttctttgtg agctcctgtt   2700
   tagcaggtcg tcccttcagc aaggacacaa aaagatttta attttattaa aaaaaaaaaa   2760
   aaaaaagacc gggaattcga tatcaagctt atcgacctgc agatcgttca aacatttggc   2820
   aataaagttt cttaagattg aatcctgttg ccggtcttgc gatgattatc atataatttc   2880
   tgttgaatta cgttaagcat gtaataatta acatgtaatg catgacgtta tttatgagat   2940
   gggtttttat gattagagtc ccgcaattat acatttaata cgcgatagaa aacaaaatat   3000
   agcgcgcaaa ctaggataaa ttatcgcgcg cggtgtcatc tatgttacta gattctagag   3060
   tctcaagctt cggcgcgcc                                                3079
<210> 106
   <211> 48
   <212> DNA
   <213> Artificial sequence
<220>
   <223> ApaI-H3B.c
<400> 106
   ttgtcgggcc catgaagact atcattgctt tgagctacat tctatgtc 48
<210> 107
   <211> 44
   <212> DNA
   <213> Artificial sequence
<220>
   <223> StuI-H3B.r
<400> 107
   aaaataggcc ttcaaatgca aatgttgcac ctaatgttgc cttt 44
<210> 108
   <211> 3061
   <212> DNA
   <213> Artificial sequence
<220>
   <223> Construct number 735, from PacI
<400> 108
   ttaattaaga attcgagctc caccgcggaa acctcctcgg attccattgc ccagctatct 60
   gtcactttat tgagaagata gtggaaaagg aaggtggctc ctacaaatgc catcattgcg 120
   ataaaggaaa ggccatcgtt gaagatgcct ctgccgacag tggtcccaaa gatggacccc 180
   cacccacgag gagcatcgtg gaaaaagaag acgttccaac cacgtcttca aagcaagtgg 240
   attgatgtga tatctccact gacgtaaggg atgacgcaca atcccactat ccttcgcaag 300
   acccttcctc tatataagga agttcatttc atttggagag gtattaaaat cttaataggt 360
   tttgataaaa gcgaacgtgg ggaaacccga accaaacctt cttctaaact ctctctcatc 420
   tctcttaaag caaacttctc tcttgtcttt cttgcgtgag cgatcttcaa cgttgtcaga 480
   tcgtgcttcg gcaccagtac aacgttttct ttcactgaag cgaaatcaaa gatctctttg 540
   tggacacgta gtgcggcgcc attaaataac gtgtacttgt cctattcttg tcggtgtggt 600
   cttgggaaaa gaaagcttgc tggaggctgc tgttcagccc catacattac ttgttacgat 660
   tctgctgact ttcggcgggt gcaatatctc tacttctgct tgacgaggta ttgttgcctg 720
   tacttctttc ttcttcttct tgctgattgg ttctataaga aatctagtat tttctttgaa 780
   acagagtttt cccgtggttt tcgaacttgg agaaagattg ttaagcttct gtatattctg 840
   cccaaatttg tcgggcccat gaagactatc attgctttga gctacattct atgtctggtt 900
   ttcactcaaa aacttcccgg aaatgacaac agcacggcaa cgctgtgcct tgggcaccat 960
   gcagtaccaa acggaacgat agtgaaaaca atcacgaatg accaaattga agttactaat 1020
   gctactgagc tggttcagag ttcctcaaca ggtgaaatat gcgacagtcc tcatcagatc 1080
   cttgatggag aaaactgcac actaatagat gctctattgg gagaccctca gtgtgatggc 1140
   ttccaaaata agaaatggga cctttttgtt gaacgcagca aagcctacag caactgttac 1200
   ccttatgatg tgccggatta tgcctccctt aggtcactag ttgcctcatc cggcacactg 1260
   gagtttaaca atgaaagttt caattggact ggagtcactc aaaacggaac aagctctgct 1320
   tgcataagga gatctaataa cagtttcttt agtagattga attggttgac ccacttaaaa 1380
   ttcaaatacc cagcattgaa cgtgactatg ccaaacaatg aaaaatttga caaattgtac 1440
   atttgggggg ttcaccaccc gggtacggac aatgaccaaa tcttcctgta tgctcaagca 1500
   tcaggaagaa tcacagtctc taccaaaaga agccaacaaa ctgtaatccc gaatatcgga 1560
   tctagaccca gagtaaggaa tatccccagc agaataagca tctattggac aatagtaaaa 1620
   ccgggagaca tacttttgat taacagcaca gggaatctaa ttgctcctag gggttacttc 1680
   aaaatacgaa gtgggaaaag ctcaataatg agatcagatg cacccattgg caaatgcaat 1740
   tctgaatgca tcactccaaa cggaagcatt cccaatgaca aaccattcca aaatgtaaac 1800
   aggatcacat acggggcctg tcccagatat gttaagcaaa acactctgaa attggcaaca 1860
   gggatgcgaa atgtaccaga gaaacaaact agaggcatat ttggcgcaat cgcgggtttc 1920
   atagaaaatg gttgggaggg aatggtggat ggttggtatg gtttcaggca tcaaaattct 1980
   gagggaatag gacaagcagc agatctcaaa agcactcaag cagcaatcga tcaaatcaat 2040
   gggaagctga ataggttgat cgggaaaacc aacgagaaat tccatcagat tgaaaaagag 2100
   ttctcagaag tcgaagggag aatccaggac cttgagaaat atgttgagga caccaaaata 2160
   gatctctggt catacaacgc ggagcttctt gttgccctgg agaaccaaca tacaattgat 2220
   ctaactgact cagaaatgaa caaactgttt gaaaaaacaa agaagcaact gagggaaaat 2280
   gctgaggata tgggcaatgg ttgtttcaaa atataccaca aatgtgacaa tgcctgcata 2340
   ggatcaatca gaaatggaac ttatgaccac gatgtataca gagatgaagc attaaacaac 2400
   cggttccaga tcaagggcgt tgagctgaag tcaggataca aagattggat actatggatt 2460
   tcctttgcca tatcatgttt tttgctttgt gttgctttgt tggggttcat catgtgggcc 2520
   tgccaaaaag gcaacattag gtgcaacatt tgcatttgaa ggcctatttt ctttagtttg 2580
   aatttactgt tattcggtgt gcatttctat gtttggtgag cggttttctg tgctcagagt 2640
   gtgtttattt tatgtaattt aatttctttg tgagctcctg tttagcaggt cgtcccttca 2700
   gcaaggacac aaaaagattt taattttatt aaaaaaaaaa aaaaaaaaga ccgggaattc 2760
   gatatcaagc ttatcgacct gcagatcgtt caaacatttg gcaataaagt ttcttaagat 2820
   tgaatcctgt tgccggtctt gcgatgatta tcatataatt tctgttgaat tacgttaagc 2880
   atgtaataat taacatgtaa tgcatgacgt tatttatgag atgggttttt atgattagag 2940
   tcccgcaatt atacatttaa tacgcgatag aaaacaaaat atagcgcgca aactaggata 3000
   aattatcgcg cgcggtgtca tctatgttac tagattctag agtctcaagc ttcggcgcgc 3060
   c 3061
<210> 109
   <211> 3085
   <212> DNA
   <213> Artificial sequence
<220>
   <223> Construct number 736, from PacI
<400> 109
   ttaattaaga attcgagctc caccgcggaa acctcctcgg attccattgc ccagctatct 60
   gtcactttat tgagaagata gtggaaaagg aaggtggctc ctacaaatgc catcattgcg 120
   ataaaggaaa ggccatcgtt gaagatgcct ctgccgacag tggtcccaaa gatggacccc 180
   cacccacgag gagcatcgtg gaaaaagaag acgttccaac cacgtcttca aagcaagtgg 240
   attgatgtga tatctccact gacgtaaggg atgacgcaca atcccactat ccttcgcaag 300
   acccttcctc tatataagga agttcatttc atttggagag gtattaaaat cttaataggt 360
   tttgataaaa gcgaacgtgg ggaaacccga accaaacctt cttctaaact ctctctcatc 420
   tctcttaaag caaacttctc tcttgtcttt cttgcgtgag cgatcttcaa cgttgtcaga 480
   tcgtgcttcg gcaccagtac aacgttttct ttcactgaag cgaaatcaaa gatctctttg 540
   tggacacgta gtgcggcgcc attaaataac gtgtacttgt cctattcttg tcggtgtggt 600
   cttgggaaaa gaaagcttgc tggaggctgc tgttcagccc catacattac ttgttacgat 660
   tctgctgact ttcggcgggt gcaatatctc tacttctgct tgacgaggta ttgttgcctg 720
   tacttctttc ttcttcttct tgctgattgg ttctataaga aatctagtat tttctttgaa 780
   acagagtttt cccgtggttt tcgaacttgg agaaagattg ttaagcttct gtatattctg 840
   cccaaatttg tcgggcccat ggcgaaaaac gttgcgattt tcggcttatt gttttctctt 900
   cttgtgttgg ttccttctca gatcttcgct caaaaacttc ccggaaatga caacagcacg 960
   gcaacgctgt gccttgggca ccatgcagta ccaaacggaa cgatagtgaa aacaatcacg 1020
   aatgaccaaa ttgaagttac taatgctact gagctggttc agagttcctc aacaggtgaa 1080
   atatgcgaca gtcctcatca gatccttgat ggagaaaact gcacactaat agatgctcta 1140
   ttgggagacc ctcagtgtga tggcttccaa aataagaaat gggacctttt tgttgaacgc 1200
   agcaaagcct acagcaactg ttacccttat gatgtgccgg attatgcctc ccttaggtca 1260
   ctagttgcct catccggcac actggagttt aacaatgaaa gtttcaattg gactggagtc 1320
   actcaaaacg gaacaagctc tgcttgcata aggagatcta ataacagttt ctttagtaga 1380
   ttgaattggt tgacccactt aaaattcaaa tacccagcat tgaacgtgac tatgccaaac 1440
   aatgaaaaat ttgacaaatt gtacatttgg ggggttcacc acccgggtac ggacaatgac 1500
   caaatcttcc tgtatgctca agcatcagga agaatcacag tctctaccaa aagaagccaa 1560
   caaactgtaa tcccgaatat cggatctaga cccagagtaa ggaatatccc cagcagaata 1620
   agcatctatt ggacaatagt aaaaccggga gacatacttt tgattaacag cacagggaat 1680
   ctaattgctc ctaggggtta cttcaaaata cgaagtggga aaagctcaat aatgagatca 1740
   gatgcaccca ttggcaaatg caattctgaa tgcatcactc caaacggaag cattcccaat 1800
   gacaaaccat tccaaaatgt aaacaggatc acatacgggg cctgtcccag atatgttaag 1860
   caaaacactc tgaaattggc aacagggatg cgaaatgtac cagagaaaca aactagaggc 1920
   atatttggcg caatcgcggg tttcatagaa aatggttggg agggaatggt ggatggttgg 1980
   tatggtttca ggcatcaaaa ttctgaggga ataggacaag cagcagatct caaaagcact 2040
   caagcagcaa tcgatcaaat caatgggaag ctgaataggt tgatcgggaa aaccaacgag 2100
   aaattccatc agattgaaaa agagttctca gaagtcgaag ggagaatcca ggaccttgag 2160
   aaatatgttg aggacaccaa aatagatctc tggtcataca acgcggagct tcttgttgcc 2220
   ctggagaacc aacatacaat tgatctaact gactcagaaa tgaacaaact gtttgaaaaa 2280
   acaaagaagc aactgaggga aaatgctgag gatatgggca atggttgttt caaaatatac 2340
   cacaaatgtg acaatgcctg cataggatca atcagaaatg gaacttatga ccacgatgta 2400
   tacagagatg aagcattaaa caaccggttc cagatcaagg gcgttgagct gaagtcagga 2460
   tacaaagatt ggatactatg gatttccttt gccatatcat gttttttgct ttgtgttgct 2520
   ttgttggggt tcatcatgtg ggcctgccaa aaaggcaaca ttaggtgcaa catttgcatt 2580
   tgaaggccta ttttctttag tttgaattta ctgttattcg gtgtgcattt ctatgtttgg 2640
   tgagcggttt tctgtgctca gagtgtgttt attttatgta atttaatttc tttgtgagct 2700
   cctgtttagc aggtcgtccc ttcagcaagg acacaaaaag attttaattt tattaaaaaa 2760
   aaaaaaaaaa aagaccggga attcgatatc aagcttatcg acctgcagat cgttcaaaca 2820
   tttggcaata aagtttctta agattgaatc ctgttgccgg tcttgcgatg attatcatat 2880
   aatttctgtt gaattacgtt aagcatgtaa taattaacat gtaatgcatg acgttattta 2940
   tgagatgggt ttttatgatt agagtcccgc aattatacat ttaatacgcg atagaaaaca 3000
   aaatatagcg cgcaaactag gataaattat cgcgcgcggt gtcatctatg ttactagatt 3060
   ctagagtctc aagcttcggc gcgcc 3085
<210> 110
   <211> 46
   <212> DNA
   <213> Artificial sequence
<220>
   <223> ApI-HBF.c
<400> 110
   ttgtcgggcc catgaaggca ataattgtac tactcatggt agtaac 46
<210> 111
   <211> 46
   <212> DNA
   <213> Artificial sequence
<220>
   <223> StuI-HBF.r
<400> 111
   aaaataggcc tttatagaca gatggagcat gaaacgttgt ctctgg 46
<210> 112
   <211> 3115
   <212> DNA
   <213> Artificial sequence
<220>
   <223> Construct number 738, from PacI
<400> 112
   ttaattaaga attcgagctc caccgcggaa acctcctcgg attccattgc ccagctatct 60
   gtcactttat tgagaagata gtggaaaagg aaggtggctc ctacaaatgc catcattgcg 120
   ataaaggaaa ggccatcgtt gaagatgcct ctgccgacag tggtcccaaa gatggacccc 180
   cacccacgag gagcatcgtg gaaaaagaag acgttccaac cacgtcttca aagcaagtgg 240
   attgatgtga tatctccact gacgtaaggg atgacgcaca atcccactat ccttcgcaag 300
   acccttcctc tatataagga agttcatttc atttggagag gtattaaaat cttaataggt 360
   tttgataaaa gcgaacgtgg ggaaacccga accaaacctt cttctaaact ctctctcatc 420
   tctcttaaag caaacttctc tcttgtcttt cttgcgtgag cgatcttcaa cgttgtcaga 480
   tcgtgcttcg gcaccagtac aacgttttct ttcactgaag cgaaatcaaa gatctctttg 540
   tggacacgta gtgcggcgcc attaaataac gtgtacttgt cctattcttg tcggtgtggt 600
   cttgggaaaa gaaagcttgc tggaggctgc tgttcagccc catacattac ttgttacgat 660
   tctgctgact ttcggcgggt gcaatatctc tacttctgct tgacgaggta ttgttgcctg 720
   tacttctttc ttcttcttct tgctgattgg ttctataaga aatctagtat tttctttgaa 780
   acagagtttt cccgtggttt tcgaacttgg agaaagattg ttaagcttct gtatattctg 840
   cccaaatttg tcgggcccat gaaggcaata attgtactac tcatggtagt aacatccaat 900
   gcagatcgaa tctgcactgg aataacatct tcaaactcac ctcatgtggt caaaacagcc 960
   actcaagggg aggtcaatgt gactggtgtg ataccactaa caacaacacc aacaaaatct 1020
   tattttgcaa atctcaaagg aacaaggacc agagggaaac tatgcccaga ctgtctcaac 1080
   tgcacagatc tggatgtggc tttgggcaga ccaatgtgtg tggggaccac accttcggcg 1140
   aaggcttcaa tactccacga agtcaaacct gttacatccg ggtgctttcc tataatgcac 1200
   gacagaacaa aaatcaggca actacccaat cttctcagag gatatgaaaa tatcaggcta 1260
   tcaacccaaa acgtcatcga tgcggaaaag gcaccaggag gaccctacag acttggaacc 1320
   tcaggatctt gccctaacgc taccagtaag agcggatttt tcgcaacaat ggcttgggct 1380
   gtcccaaagg acaacaacaa aaatgcaacg aacccactaa cagtagaagt accatacatt 1440
   tgtacagaag gggaagacca aatcactgtt tgggggttcc attcagataa caaaacccaa 1500
   atgaagaacc tctatggaga ctcaaatcct caaaagttca cctcatctgc taatggagta 1560
   accacacact atgtttctca gattggcagc ttcccagatc aaacagaaga cggaggacta 1620
   ccacaaagcg gcaggattgt tgttgattac atgatgcaaa aacctgggaa aacaggaaca 1680
   attgtctacc aaagaggtgt tttgttgcct caaaaggtgt ggtgcgcgag tggcaggagc 1740
   aaagtaataa aagggtcctt gcctttaatt ggtgaagcag attgccttca tgaaaaatac 1800
   ggtggattaa acaaaagcaa gccttactac acaggagaac atgcaaaagc cataggaaat 1860
   tgcccaatat gggtgaaaac acctttgaag ctcgccaatg gaaccaaata tagacctcct 1920
   gcaaaactat taaaggaaag gggtttcttc ggagctattg ctggtttcct agaaggagga 1980
   tgggaaggaa tgattgcagg ctggcacgga tacacatctc acggagcaca tggagtggca 2040
   gtggcggcgg accttaagag tacgcaagaa gctataaaca agataacaaa aaatctcaat 2100
   tctttgagtg agctagaagt aaagaatctt caaagactaa gtggtgccat ggatgaactc 2160
   cacaacgaaa tactcgagct ggatgagaaa gtggatgatc tcagagctga cactataagc 2220
   tcgcaaatag aacttgcagt cttgctttcc aacgaaggaa taataaacag tgaagatgag   2280
   catctattgg cacttgagag aaaactaaag aaaatgctgg gtccctctgc tgtagagata   2340
   ggaaatggat gcttcgaaac caaacacaag tgcaaccaga cctgcttaga caggatagct   2400
   gctggcacct ttaatgcagg agaattttct ctccccactt ttgattcact gaacattact   2460
   gctgcatctt taaatgatga tggattggat aaccatacta tactgctcta ttactcaact   2520
   gctgcttcta gtttggctgt aacattgatg ctagctattt ttattgttta tatggtctcc   2580
   agagacaacg tttcatgctc catctgtcta taaaggccta ttttctttag tttgaattta   2640
   ctgttattcg gtgtgcattt ctatgtttgg tgagcggttt tctgtgctca gagtgtgttt   2700
   attttatgta atttaatttc tttgtgagct cctgtttagc aggtcgtccc ttcagcaagg   2760
   acacaaaaag attttaattt tattaaaaaa aaaaaaaaaa aagaccggga attcgatatc   2820
   aagcttatcg acctgcagat cgttcaaaca tttggcaata aagtttctta agattgaatc   2880
   ctgttgccgg tcttgcgatg attatcatat aatttctgtt gaattacgtt aagcatgtaa   2940
   taattaacat gtaatgcatg acgttattta tgagatgggt ttttatgatt agagtcccgc   3000
   aattatacat ttaatacgcg atagaaaaca aaatatagcg cgcaaactag gataaattat   3060
   cgcgcgcggt gtcatctatg ttactagatt ctagagtctc aagcttcggc gcgcc        3115
<210> 113
   <211> 3142
   <212> DNA
   <213> Artificial sequence
<220>
   <223> Construct number 739, from PacI
<400> 113
   ttaattaaga attcgagctc caccgcggaa acctcctcgg attccattgc ccagctatct    60
   gtcactttat tgagaagata gtggaaaagg aaggtggctc ctacaaatgc catcattgcg   120
   ataaaggaaa ggccatcgtt gaagatgcct ctgccgacag tggtcccaaa gatggacccc   180
   cacccacgag gagcatcgtg gaaaaagaag acgttccaac cacgtcttca aagcaagtgg   240
   attgatgtga tatctccact gacgtaaggg atgacgcaca atcccactat ccttcgcaag   300
   acccttcctc tatataagga agttcatttc atttggagag gtattaaaat cttaataggt   360
   tttgataaaa gcgaacgtgg ggaaacccga accaaacctt cttctaaact ctctctcatc   420
   tctcttaaag caaacttctc tcttgtcttt cttgcgtgag cgatcttcaa cgttgtcaga   480
   tcgtgcttcg gcaccagtac aacgttttct ttcactgaag cgaaatcaaa gatctctttg   540
   tggacacgta gtgcggcgcc attaaataac gtgtacttgt cctattcttg tcggtgtggt   600
   cttgggaaaa gaaagcttgc tggaggctgc tgttcagccc catacattac ttgttacgat   660
   tctgctgact ttcggcgggt gcaatatctc tacttctgct tgacgaggta ttgttgcctg   720
   tacttctttc ttcttcttct tgctgattgg ttctataaga aatctagtat tttctttgaa 780
   acagagtttt cccgtggttt tcgaacttgg agaaagattg ttaagcttct gtatattctg 840
   cccaaatttg tcgggcccat ggcgaaaaac gttgcgattt tcggcttatt gttttctctt 900
   cttgtgttgg ttccttctca gatcttcgct gatcgaatct gcactggaat aacatcttca 960
   aactcacctc atgtggtcaa aacagccact caaggggagg tcaatgtgac tggtgtgata 1020
   ccactaacaa caacaccaac aaaatcttat tttgcaaatc tcaaaggaac aaggaccaga 1080
   gggaaactat gcccagactg tctcaactgc acagatctgg atgtggcttt gggcagacca 1140
   atgtgtgtgg ggaccacacc ttcggcgaag gcttcaatac tccacgaagt caaacctgtt 1200
   acatccgggt gctttcctat aatgcacgac agaacaaaaa tcaggcaact acccaatctt 1260
   ctcagaggat atgaaaatat caggctatca acccaaaacg tcatcgatgc ggaaaaggca 1320
   ccaggaggac cctacagact tggaacctca ggatcttgcc ctaacgctac cagtaagagc 1380
   ggatttttcg caacaatggc ttgggctgtc ccaaaggaca acaacaaaaa tgcaacgaac 1440
   ccactaacag tagaagtacc atacatttgt acagaagggg aagaccaaat cactgtttgg 1500
   gggttccatt cagataacaa aacccaaatg aagaacctct atggagactc aaatcctcaa 1560
   aagttcacct catctgctaa tggagtaacc acacactatg tttctcagat tggcagcttc 1620
   ccagatcaaa cagaagacgg aggactacca caaagcggca ggattgttgt tgattacatg 1680
   atgcaaaaac ctgggaaaac aggaacaatt gtctaccaaa gaggtgtttt gttgcctcaa 1740
   aaggtgtggt gcgcgagtgg caggagcaaa gtaataaaag ggtccttgcc tttaattggt 1800
   gaagcagatt gccttcatga aaaatacggt ggattaaaca aaagcaagcc ttactacaca 1860
   ggagaacatg caaaagccat aggaaattgc ccaatatggg tgaaaacacc tttgaagctc 1920
   gccaatggaa ccaaatatag acctcctgca aaactattaa aggaaagggg tttcttcgga 1980
   gctattgctg gtttcctaga aggaggatgg gaaggaatga ttgcaggctg gcacggatac 2040
   acatctcacg gagcacatgg agtggcagtg gcggcggacc ttaagagtac gcaagaagct 2100
   ataaacaaga taacaaaaaa tctcaattct ttgagtgagc tagaagtaaa gaatcttcaa 2160
   agactaagtg gtgccatgga tgaactccac aacgaaatac tcgagctgga tgagaaagtg 2220
   gatgatctca gagctgacac tataagctcg caaatagaac ttgcagtctt gctttccaac 2280
   gaaggaataa taaacagtga agatgagcat ctattggcac ttgagagaaa actaaagaaa 2340
   atgctgggtc cctctgctgt agagatagga aatggatgct tcgaaaccaa acacaagtgc 2400
   aaccagacct gcttagacag gatagctgct ggcaccttta atgcaggaga attttctctc 2460
   cccacttttg attcactgaa cattactgct gcatctttaa atgatgatgg attggataac 2520
   catactatac tgctctatta ctcaactgct gcttctagtt tggctgtaac attgatgcta 2580
   gctattttta ttgtttatat ggtctccaga gacaacgttt catgctccat ctgtctataa 2640
   aggcctattt tctttagttt gaatttactg ttattcggtg tgcatttcta tgtttggtga 2700
   gcggttttct gtgctcagag tgtgtttatt ttatgtaatt taatttcttt gtgagctcct 2760
   gtttagcagg tcgtcccttc agcaaggaca caaaaagatt ttaattttat taaaaaaaaa 2820
   aaaaaaaaag accgggaatt cgatatcaag cttatcgacc tgcagatcgt tcaaacattt 2880
   ggcaataaag tttcttaaga ttgaatcctg ttgccggtct tgcgatgatt atcatataat 2940
   ttctgttgaa ttacgttaag catgtaataa ttaacatgta atgcatgacg ttatttatga 3000
   gatgggtttt tatgattaga gtcccgcaat tatacattta atacgcgata gaaaacaaaa 3060
   tatagcgcgc aaactaggat aaattatcgc gcgcggtgtc atctatgtta ctagattcta 3120
   gagtctcaag cttcggcgcg cc 3142
<210> 114
   <211> 1272
   <212> DNA
   <213> Medicago sativa
<400> 114 gctcaacagt ag 1272
<210> 115
   <211> 20
   <212> DNA
   <213> Artificial sequence
<220>
   <223> Hsp-40Luz.1c
<400> 115
   atgtttgggc gcggaccaac 20
<210> 116
   <211> 31
   <212> DNA
   <213> Artificial sequence
<220>
   <223> Hsp40Luz-SacI.1272r
<400> 116
   agctgagctc ctactgttga gcgcattgca c 31
<210> 117
   <211> 36
   <212> DNA
   <213> Artificial sequence
<220>
   <223> Hsp40Luz-Plasto.r
<400> 117
   gttggtccgc gcccaaacat tttctctcaa gatgat 36
<210> 118
   <211> 21
   <212> DNA
   <213> Artificial sequence
<220>
   <223> Hsp70Ara.1c
<400> 118
   atgtcgggta aaggagaagg a 21
<210> 119
   <211> 33
   <212> DNA
   <213> Artificial sequence
<220>
   <223> Hsp70Ara-SacI.1956r
<400> 119
   agctgagctc ttagtcgacc tcctcgatct tag 33
<210> 120
   <211> 37
   <212> DNA
   <213> Artificial sequence
<220>
   <223> Hsp70Ara-Plasto.r
<400> 120
   tccttctcct ttacccgaca ttttctctca agatgat 37
<210> 121
   <211> 4402
   <212> DNA
   <213> Artificial sequence
<220>
   <223> Construct number R850, from HindIII
<400> 121
   aagcttgcat gcctgcaggt cgactctaga ggatccccgg gctggtctgt acattcatct      60
   tgccgccttt gcattcactt ggccacaaag agtagagaga aggaagagaa gagcccagac     120
   ttcaagaagc gaccttgcaa gtgcactcga gggtcagaaa ctgtatatca tatctatgtg     180
   agagaaaggg gaacatttga gatggagtcc atttacttga ggtatactta ttattttgat     240
   caataaattt gtatacttct tatttagatc aataaatttg tcattaagct ataatccaaa     300
   ataaattacg atcaaatatg caaatgttag ccagtacttg tgttaaactt gatggcatct     360
   cttggtttct ttggcaatca catgcctaag aaataaatag tatcatatga ttgtgtttgg     420
   tcagacttca gagtcagatg actctgtttg gataaacagc ttaattaagc gcttatagaa     480
   tatcatatga ttgtgtttgg tcagacttca gagcatctct tggtttctct ggcaatcata     540
   tgcctaagaa ataaatagta tcatatgatt gtgtttggtc agacttcaga gtcagatgac     600
   cctgtttggg taaacagctt aattaagtgc ttatagaata agcgcttatc atataagtgc     660
   ttttgtacag ttatttctat gaaagtagaa gaaatagtca tattgtttta atataagcta     720
   tcctggagag cttgtggaaa taaccagaaa agaacttatg gacacgtcat gagctgttta     780
   cataagatct ccctaacagt ctcaaaagtg tttatgccag tagataaatt caaataagtc     840
   aatctaaaca gaccctaaat ccattatggt acctatcatt ttagcttatt ccatctttat     900
   taagaatgtc atgagataac ataatgataa cacattattt tgacacaaat gggcagatct     960
   agcaatttaa ctctggagtc cttcaagact gctgttctta cgaagttcac gtccctgaat    1020
   catgttcctg tatggaagcc tgaaagacct caaattctaa aaggtggcga taaattgaag    1080
   gtttacaaaa tataccctgc gggcttgaca cagaggcaag ctctttatac cttccagttc    1140
   aacggggatg ttgatttcag aagtcacttg gagagcaatc cttgtgccaa gtttgaagta    1200
   atttttgtgt agcatatgtt gagctaccta caatttacat gatcacctag cattagctct    1260
   ttcacttaac tgagagaatg aagttttagg aatgagtatg accatggagt cggcatggct    1320
   ttgtaatgcc taccctactt tggccaactc atcggggatt tacattcaga aaatatacat    1380
   gacttcaacc atacttaaac ccctttttgt aagataactg aatgttcata tttaatgttg 1440
   ggttgtagtg tttttacttg attatatcca gacagttaca agttggacaa caagattgtg 1500
   ggtctgtact gttatttatt tatttttttt ttagcagaaa caccttatct tttgtttcgt 1560
   ttgaatgtag aatgaaaata aaagaaagaa aatataacat catcggccgc gcttgtctaa 1620
   tttcgggcag ttaggatcct ctccggtcac cggaaagttt cagtagaaga aacaaaacac 1680
   cgtgactaaa atgatactat tattttattt attgtgtttt tcttttttct accggaactt 1740
   tttagaacgg atcccaactc gttccggggc cgctacaact gaaacaaaag aagatatttt 1800
   ctctctcttc agaaatgtaa gttttccttt acagataccc attcaccatt tgattcagat 1860
   gtggtgacta gagataaagc atactaattt gactcttgga aacccataaa gtttatgtta 1920
   tccgtgttct ggaccaatcc acttgggggc ataacctgtg tctatgtgtg gtttggtttc 1980
   cattctgatt tatgcggcga cttgtaattt aaaatctagg aggggcagac attgaacaat 2040
   cccaatattt taataactta tgcaagattt tttttattaa tgagatgatg tgtttgtgac 2100
   tgagattgag tcatacattt cactaagaaa tggttccaag taccaaacta tcatgaccca 2160
   gttgcaaaca tgacgttcgg gagtggtcac tttgatagtt caatttcatc ttggcttctt 2220
   attcctttta taattctaat tcttcttgtg taaactattt catgtattat ttttctttaa 2280
   aatttacatg tcatttattt tgcctcacta actcaatttt gcatataaca atgataagtg 2340
   atattttgac tcacaaaatt tacatcaaat ttcgacatcg tttattatgt tcattggatg 2400
   attaacaaat ataacaaact ttgcaactaa ttaaccacca actgaatata attaactata 2460
   actgtgaaag tagttaactc atttttatat ttcatagatc aaataagaga aataacggta 2520
   tattaatccc tccaaaaaaa aaaaacggta tatttactaa aaaatctaag ccacgtagga 2580
   ggataacagg atccccgtag gaggataaca tccaatccaa ccaatcacaa caatcctgat 2640
   gagataaccc actttaagcc cacgcatctg tggcacatct acattatcta aatcacacat 2700
   tcttccacac atctgagcca cacaaaaacc aatccacatc tttatcaccc attctataaa 2760
   aaatcacact ttgtgagtct acactttgat tcccttcaaa cacatacaaa gagaagagac 2820
   taattaatta attaatcatc ttgagagaaa atgtttgggc gcggaccaac aaggaagagt 2880
   gataacacca aatattacga tattcttggt gtttcaaaaa gtgctagtga agatgaaatc 2940
   aagaaagcct atagaaaggc agcgatgaag aaccatccag ataagggtgg ggatcctgag 3000
   aagttcaagg agttgggcca agcatatgaa gtgttgagcg atcctgaaaa gaaagaactg 3060
   tatgatcaat atggtgaaga tgcccttaaa gaaggaatgg ggggaggcgc aggaagctca 3120
   tttcataatc cgtttgatat tttcgaatca ttttttggtg caggctttgg tggtggtggt 3180
   ccttcacgcg caagaagaca gaagcaagga gaagatgtgg tgcattctat aaaggtttcc 3240
   ttggaggatg tgtataacgg cactacaaag aagctatcac tttctaggaa tgcactgtgc 3300
   tcaaaatgta aagggaaagg ttcaaaaagt ggaactgctg gaaggtgttt tggatgccag 3360
   ggcacaggta tgaagattac cagaaggcaa attggactgg gcatgattca acaaatgcaa 3420
   cacgtctgtc ctgactgcaa aggaacaggc gaggtcatta gtgagagaga tagatgccct 3480
   caatgcaagg gaaacaagat tactcaagaa aagaaggtgc tggaggtgca tgtggaaaag 3540
   gggatgcagc agggtcacaa gattgtattc gaaggacaag ctgatgaagc tcctgataca 3600
   atcacaggag acatagtttt tgtcttgcaa gtaaagggac atccgaagtt tcggagggag 3660
   cgtgatgacc tccacattga acacaatttg agcttaactg aggctctctg tggcttccag 3720
   tttaatgtca cacatcttga tggaaggcaa ctattggtca aatcgaaccc cggcgaagtc 3780
   atcaagccag gtcaacataa agctataaat gatgagggaa tgccacaaca tggtaggccg 3840
   ttcatgaagg gacgcctata catcaagttt agtgttgatt tcccggattc gggttttctt 3900
   tccccaagcc aaagcctgga attagaaaag atattacctc aaaagacaag caagaacttg 3960
   tcccaaaagg aggtagatga ttgtgaggag accaccctgc atgatgtcaa tattgcagag 4020
   gagatgagtc gaaagaagca acaataccgt gaggcatatg atgacgatga tgatgaagat 4080
   gatgagcact cgcagcctcg ggtgcaatgc gctcaacagt aggagctcag ctcgaatttc 4140
   cccgatcgtt caaacatttg gcaataaagt ttcttaagat tgaatcctgt tgccggtctt 4200
   gcgatgatta tcatataatt tctgttgaat tacgttaagc atgtaataat taacatgtaa 4260
   tgcatgacgt tatttatgag atgggttttt atgattagag tcccgcaatt atacatttaa 4320
   tacgcgatag aaaacaaaat atagcgcgca aactaggata aattatcgcg cgcggtgtca 4380
   tctatgttac tagatcgaat tc 4402
<210> 122
   <211> 5086
   <212> DNA
   <213> Artificial sequence
<220>
   <223> Construct number R860, from HindIII
<400> 122
   aagcttgcat gcctgcaggt cgactctaga ggatccccgg gctggtctgt acattcatct 60
   tgccgccttt gcattcactt ggccacaaag agtagagaga aggaagagaa gagcccagac 120
   ttcaagaagc gaccttgcaa gtgcactcga gggtcagaaa ctgtatatca tatctatgtg 180
   agagaaaggg gaacatttga gatggagtcc atttacttga ggtatactta ttattttgat 240
   caataaattt gtatacttct tatttagatc aataaatttg tcattaagct ataatccaaa 300
   ataaattacg atcaaatatg caaatgttag ccagtacttg tgttaaactt gatggcatct 360
   cttggtttct ttggcaatca catgcctaag aaataaatag tatcatatga ttgtgtttgg 420
   tcagacttca gagtcagatg actctgtttg gataaacagc ttaattaagc gcttatagaa 480
   tatcatatga ttgtgtttgg tcagacttca gagcatctct tggtttctct ggcaatcata 540
   tgcctaagaa ataaatagta tcatatgatt gtgtttggtc agacttcaga gtcagatgac 600
   cctgtttggg taaacagctt aattaagtgc ttatagaata agcgcttatc atataagtgc 660
   ttttgtacag ttatttctat gaaagtagaa gaaatagtca tattgtttta atataagcta 720
   tcctggagag cttgtggaaa taaccagaaa agaacttatg gacacgtcat gagctgttta 780
   cataagatct ccctaacagt ctcaaaagtg tttatgccag tagataaatt caaataagtc 840
   aatctaaaca gaccctaaat ccattatggt acctatcatt ttagcttatt ccatctttat 900
   taagaatgtc atgagataac ataatgataa cacattattt tgacacaaat gggcagatct 960
   agcaatttaa ctctggagtc cttcaagact gctgttctta cgaagttcac gtccctgaat 1020
   catgttcctg tatggaagcc tgaaagacct caaattctaa aaggtggcga taaattgaag 1080
   gtttacaaaa tataccctgc gggcttgaca cagaggcaag ctctttatac cttccagttc 1140
   aacggggatg ttgatttcag aagtcacttg gagagcaatc cttgtgccaa gtttgaagta 1200
   atttttgtgt agcatatgtt gagctaccta caatttacat gatcacctag cattagctct 1260
   ttcacttaac tgagagaatg aagttttagg aatgagtatg accatggagt cggcatggct 1320
   ttgtaatgcc taccctactt tggccaactc atcggggatt tacattcaga aaatatacat 1380
   gacttcaacc atacttaaac ccctttttgt aagataactg aatgttcata tttaatgttg 1440
   ggttgtagtg tttttacttg attatatcca gacagttaca agttggacaa caagattgtg 1500
   ggtctgtact gttatttatt tatttttttt ttagcagaaa caccttatct tttgtttcgt 1560
   ttgaatgtag aatgaaaata aaagaaagaa aatataacat catcggccgc gcttgtctaa 1620
   tttcgggcag ttaggatcct ctccggtcac cggaaagttt cagtagaaga aacaaaacac 1680
   cgtgactaaa atgatactat tattttattt attgtgtttt tcttttttct accggaactt 1740
   tttagaacgg atcccaactc gttccggggc cgctacaact gaaacaaaag aagatatttt 1800
   ctctctcttc agaaatgtaa gttttccttt acagataccc attcaccatt tgattcagat 1860
   gtggtgacta gagataaagc atactaattt gactcttgga aacccataaa gtttatgtta 1920
   tccgtgttct ggaccaatcc acttgggggc ataacctgtg tctatgtgtg gtttggtttc 1980
   cattctgatt tatgcggcga cttgtaattt aaaatctagg aggggcagac attgaacaat 2040
   cccaatattt taataactta tgcaagattt tttttattaa tgagatgatg tgtttgtgac 2100
   tgagattgag tcatacattt cactaagaaa tggttccaag taccaaacta tcatgaccca 2160
   gttgcaaaca tgacgttcgg gagtggtcac tttgatagtt caatttcatc ttggcttctt 2220
   attcctttta taattctaat tcttcttgtg taaactattt catgtattat ttttctttaa 2280
   aatttacatg tcatttattt tgcctcacta actcaatttt gcatataaca atgataagtg 2340
   atattttgac tcacaaaatt tacatcaaat ttcgacatcg tttattatgt tcattggatg 2400
   attaacaaat ataacaaact ttgcaactaa ttaaccacca actgaatata attaactata 2460
   actgtgaaag tagttaactc atttttatat ttcatagatc aaataagaga aataacggta 2520
   tattaatccc tccaaaaaaa aaaaacggta tatttactaa aaaatctaag ccacgtagga 2580
   ggataacagg atccccgtag gaggataaca tccaatccaa ccaatcacaa caatcctgat 2640
   gagataaccc actttaagcc cacgcatctg tggcacatct acattatcta aatcacacat 2700
   tcttccacac atctgagcca cacaaaaacc aatccacatc tttatcaccc attctataaa 2760
   aaatcacact ttgtgagtct acactttgat tcccttcaaa cacatacaaa gagaagagac 2820
   taattaatta attaatcatc ttgagagaaa atgtcgggta aaggagaagg accagctatc 2880
   ggtatcgatc ttggtaccac ttactcttgc gtcggagtat ggcaacacga ccgtgttgag 2940
   atcattgcta atgatcaagg aaacagaacc acgccatctt acgttgcttt caccgactcc 3000
   gagaggttga tcggtgacgc agctaagaat caggtcgcca tgaaccccgt taacaccgtt 3060
   ttcgacgcta agaggttgat cggtcgtcgt ttctctgaca gctctgttca gagtgacatg 3120
   aaattgtggc cattcaagat tcaagccgga cctgccgata agccaatgat ctacgtcgaa 3180
   tacaagggtg aagagaaaga gttcgcagct gaggagattt cttccatggt tcttattaag 3240
   atgcgtgaga ttgctgaggc ttaccttggt gtcacaatca agaacgccgt tgttaccgtt 3300
   ccagcttact tcaacgactc tcagcgtcag gctacaaagg atgctggtgt catcgctggt 3360
   ttgaacgtta tgcgaatcat caacgagcct acagccgccg ctattgccta cggtcttgac 3420
   aaaaaggcta ccagcgttgg agagaagaat gttcttatct tcgatcttgg tggtggcact 3480
   tttgatgtct ctcttcttac cattgaagag ggtatctttg aggtgaaggc aactgctggt 3540
   gacacccatc ttggtgggga agattttgac aacagaatgg ttaaccactt tgtccaagag 3600
   ttcaagagga agagtaagaa ggatatcacc ggtaacccaa gagctcttag gaggttgaga 3660
   acttcctgtg agagagcgaa gaggactctt tcttccactg ctcagaccac catcgagatt 3720
   gactctctat acgagggtat cgacttctac tccaccatca cccgtgctag atttgaggag 3780
   ctcaacatgg atctcttcag gaagtgtatg gagccagttg agaagtgtct tcgtgatgct 3840
   aagatggaca agagcactgt tcatgatgtt gtccttgttg gtggttctac ccgtatccct 3900
   aaggttcagc aattgctcca ggacttcttc aacggcaaag agctttgcaa gtctattaac 3960
   cctgatgagg ctgttgccta cggtgctgct gtccagggag ctattctcag cggtgaagga 4020
   aacgagaagg ttcaagatct tctattgctc gatgtcactc ctctctccct tggtttggaa 4080
   actgccggtg gtgtcatgac cactttgatc ccaaggaaca caaccatccc aaccaagaag 4140
   gaacaagtct tctccaccta ctcagacaac caacccggtg tgttgatcca ggtgtacgaa 4200
   ggagagagag ccagaaccaa ggacaacaac cttcttggta aatttgagct ctccggaatt 4260
   cctccagctc ctcgtggtgt cccccagatc acagtctgct ttgacattga tgccaatggt 4320
   atcctcaatg tctctgctga ggacaagacc accggacaga agaacaagat caccatcacc 4380
   aatgacaagg gtcgtctctc caaggatgag attgagaaga tggttcaaga ggctgagaag 4440
   tacaagtccg aagacgagga gcacaagaag aaggttgaag ccaagaacgc tctcgagaac 4500
   tacgcttaca acatgaggaa caccatccaa gacgagaaga ttggtgagaa gctcccggct 4560
   gcagacaaga agaagatcga ggattctatt gagcaggcga ttcaatggct cgagggtaac 4620
   cagttggctg aggctgatga gttcgaagac aagatgaagg aattggagag catctgcaac 4680
   ccaatcattg ccaagatgta ccaaggagct ggtggtgaag ccggtggtcc aggtgcctct 4740
   ggtatggacg atgatgctcc ccctgcttca ggcggtgctg gacctaagat cgaggaggtc 4800
   gactaagagc tcagctcgaa tttccccgat cgttcaaaca tttggcaata aagtttctta 4860
   agattgaatc ctgttgccgg tcttgcgatg attatcatat aatttctgtt gaattacgtt 4920
   aagcatgtaa taattaacat gtaatgcatg acgttattta tgagatgggt ttttatgatt 4980
   agagtcccgc aattatacat ttaatacgcg atagaaaaca aaatatagcg cgcaaactag 5040
   gataaattat cgcgcgcggt gtcatctatg ttactagatc gaattc 5086
<210> 123
   <211> 9493
   <212> DNA
   <213> Artificial sequence
<220>
   <223> Construct number R870, from HindIII
<400> 123
   aagcttgcat gcctgcaggt cgactctaga ggatccccgg gctggtctgt acattcatct      60
   tgccgccttt gcattcactt ggccacaaag agtagagaga aggaagagaa gagcccagac     120
   ttcaagaagc gaccttgcaa gtgcactcga gggtcagaaa ctgtatatca tatctatgtg     180
   agagaaaggg gaacatttga gatggagtcc atttacttga ggtatactta ttattttgat     240
   caataaattt gtatacttct tatttagatc aataaatttg tcattaagct ataatccaaa     300
   ataaattacg atcaaatatg caaatgttag ccagtacttg tgttaaactt gatggcatct     360
   cttggtttct ttggcaatca catgcctaag aaataaatag tatcatatga ttgtgtttgg     420
   tcagacttca gagtcagatg actctgtttg gataaacagc ttaattaagc gcttatagaa     480
   tatcatatga ttgtgtttgg tcagacttca gagcatctct tggtttctct ggcaatcata     540
   tgcctaagaa ataaatagta tcatatgatt gtgtttggtc agacttcaga gtcagatgac     600
   cctgtttggg taaacagctt aattaagtgc ttatagaata agcgcttatc atataagtgc     660
   ttttgtacag ttatttctat gaaagtagaa gaaatagtca tattgtttta atataagcta     720
   tcctggagag cttgtggaaa taaccagaaa agaacttatg gacacgtcat gagctgttta     780
   cataagatct ccctaacagt ctcaaaagtg tttatgccag tagataaatt caaataagtc     840
   aatctaaaca gaccctaaat ccattatggt acctatcatt ttagcttatt ccatctttat     900
   taagaatgtc atgagataac ataatgataa cacattattt tgacacaaat gggcagatct     960
   agcaatttaa ctctggagtc cttcaagact gctgttctta cgaagttcac gtccctgaat 1020
   catgttcctg tatggaagcc tgaaagacct caaattctaa aaggtggcga taaattgaag 1080
   gtttacaaaa tataccctgc gggcttgaca cagaggcaag ctctttatac cttccagttc 1140
   aacggggatg ttgatttcag aagtcacttg gagagcaatc cttgtgccaa gtttgaagta 1200
   atttttgtgt agcatatgtt gagctaccta caatttacat gatcacctag cattagctct 1260
   ttcacttaac tgagagaatg aagttttagg aatgagtatg accatggagt cggcatggct 1320
   ttgtaatgcc taccctactt tggccaactc atcggggatt tacattcaga aaatatacat 1380
   gacttcaacc atacttaaac ccctttttgt aagataactg aatgttcata tttaatgttg 1440
   ggttgtagtg tttttacttg attatatcca gacagttaca agttggacaa caagattgtg 1500
   ggtctgtact gttatttatt tatttttttt ttagcagaaa caccttatct tttgtttcgt 1560
   ttgaatgtag aatgaaaata aaagaaagaa aatataacat catcggccgc gcttgtctaa 1620
   tttcgggcag ttaggatcct ctccggtcac cggaaagttt cagtagaaga aacaaaacac 1680
   cgtgactaaa atgatactat tattttattt attgtgtttt tcttttttct accggaactt 1740
   tttagaacgg atcccaactc gttccggggc cgctacaact gaaacaaaag aagatatttt 1800
   ctctctcttc agaaatgtaa gttttccttt acagataccc attcaccatt tgattcagat 1860
   gtggtgacta gagataaagc atactaattt gactcttgga aacccataaa gtttatgtta 1920
   tccgtgttct ggaccaatcc acttgggggc ataacctgtg tctatgtgtg gtttggtttc 1980
   cattctgatt tatgcggcga cttgtaattt aaaatctagg aggggcagac attgaacaat 2040
   cccaatattt taataactta tgcaagattt tttttattaa tgagatgatg tgtttgtgac 2100
   tgagattgag tcatacattt cactaagaaa tggttccaag taccaaacta tcatgaccca 2160
   gttgcaaaca tgacgttcgg gagtggtcac tttgatagtt caatttcatc ttggcttctt 2220
   attcctttta taattctaat tcttcttgtg taaactattt catgtattat ttttctttaa 2280
   aatttacatg tcatttattt tgcctcacta actcaatttt gcatataaca atgataagtg 2340
   atattttgac tcacaaaatt tacatcaaat ttcgacatcg tttattatgt tcattggatg 2400
   attaacaaat ataacaaact ttgcaactaa ttaaccacca actgaatata attaactata 2460
   actgtgaaag tagttaactc atttttatat ttcatagatc aaataagaga aataacggta 2520
   tattaatccc tccaaaaaaa aaaaacggta tatttactaa aaaatctaag ccacgtagga 2580
   ggataacagg atccccgtag gaggataaca tccaatccaa ccaatcacaa caatcctgat 2640
   gagataaccc actttaagcc cacgcatctg tggcacatct acattatcta aatcacacat 2700
   tcttccacac atctgagcca cacaaaaacc aatccacatc tttatcaccc attctataaa 2760
   aaatcacact ttgtgagtct acactttgat tcccttcaaa cacatacaaa gagaagagac 2820
   taattaatta attaatcatc ttgagagaaa atgtcgggta aaggagaagg accagctatc 2880
   ggtatcgatc ttggtaccac ttactcttgc gtcggagtat ggcaacacga ccgtgttgag 2940
   atcattgcta atgatcaagg aaacagaacc acgccatctt acgttgcttt caccgactcc 3000
   gagaggttga tcggtgacgc agctaagaat caggtcgcca tgaaccccgt taacaccgtt 3060
   ttcgacgcta agaggttgat cggtcgtcgt ttctctgaca gctctgttca gagtgacatg 3120
   aaattgtggc cattcaagat tcaagccgga cctgccgata agccaatgat ctacgtcgaa 3180
   tacaagggtg aagagaaaga gttcgcagct gaggagattt cttccatggt tcttattaag 3240
   atgcgtgaga ttgctgaggc ttaccttggt gtcacaatca agaacgccgt tgttaccgtt 3300
   ccagcttact tcaacgactc tcagcgtcag gctacaaagg atgctggtgt catcgctggt 3360
   ttgaacgtta tgcgaatcat caacgagcct acagccgccg ctattgccta cggtcttgac 3420
   aaaaaggcta ccagcgttgg agagaagaat gttcttatct tcgatcttgg tggtggcact 3480
   tttgatgtct ctcttcttac cattgaagag ggtatctttg aggtgaaggc aactgctggt 3540
   gacacccatc ttggtgggga agattttgac aacagaatgg ttaaccactt tgtccaagag 3600
   ttcaagagga agagtaagaa ggatatcacc ggtaacccaa gagctcttag gaggttgaga 3660
   acttcctgtg agagagcgaa gaggactctt tcttccactg ctcagaccac catcgagatt 3720
   gactctctat acgagggtat cgacttctac tccaccatca cccgtgctag atttgaggag 3780
   ctcaacatgg atctcttcag gaagtgtatg gagccagttg agaagtgtct tcgtgatgct 3840
   aagatggaca agagcactgt tcatgatgtt gtccttgttg gtggttctac ccgtatccct 3900
   aaggttcagc aattgctcca ggacttcttc aacggcaaag agctttgcaa gtctattaac 3960
   cctgatgagg ctgttgccta cggtgctgct gtccagggag ctattctcag cggtgaagga 4020
   aacgagaagg ttcaagatct tctattgctc gatgtcactc ctctctccct tggtttggaa 4080
   actgccggtg gtgtcatgac cactttgatc ccaaggaaca caaccatccc aaccaagaag 4140
   gaacaagtct tctccaccta ctcagacaac caacccggtg tgttgatcca ggtgtacgaa 4200
   ggagagagag ccagaaccaa ggacaacaac cttcttggta aatttgagct ctccggaatt 4260
   cctccagctc ctcgtggtgt cccccagatc acagtctgct ttgacattga tgccaatggt 4320
   atcctcaatg tctctgctga ggacaagacc accggacaga agaacaagat caccatcacc 4380
   aatgacaagg gtcgtctctc caaggatgag attgagaaga tggttcaaga ggctgagaag 4440
   tacaagtccg aagacgagga gcacaagaag aaggttgaag ccaagaacgc tctcgagaac 4500
   tacgcttaca acatgaggaa caccatccaa gacgagaaga ttggtgagaa gctcccggct 4560
   gcagacaaga agaagatcga ggattctatt gagcaggcga ttcaatggct cgagggtaac 4620
   cagttggctg aggctgatga gttcgaagac aagatgaagg aattggagag catctgcaac 4680
   ccaatcattg ccaagatgta ccaaggagct ggtggtgaag ccggtggtcc aggtgcctct 4740
   ggtatggacg atgatgctcc ccctgcttca ggcggtgctg gacctaagat cgaggaggtc 4800
   gactaagagc tcagctcgaa tttccccgat cgttcaaaca tttggcaata aagtttctta 4860
   agattgaatc ctgttgccgg tcttgcgatg attatcatat aatttctgtt gaattacgtt 4920
   aagcatgtaa taattaacat gtaatgcatg acgttattta tgagatgggt ttttatgatt 4980
   agagtcccgc aattatacat ttaatacgcg atagaaaaca aaatatagcg cgcaaactag 5040
   gataaattat cgcgcgcggt gtcatctatg ttactagatc gaattcgtaa tcatggtcat 5100
   agctgtttcc tgtgtgaaat tgttatccgg ggctggtctg tacattcatc ttgccgcctt 5160
   tgcattcact tggccacaaa gagtagagag aaggaagaga agagcccaga cttcaagaag 5220
   cgaccttgca agtgcactcg agggtcagaa actgtatatc atatctatgt gagagaaagg 5280
   ggaacatttg agatggagtc catttacttg aggtatactt attattttga tcaataaatt 5340
   tgtatacttc ttatttagat caataaattt gtcattaagc tataatccaa aataaattac 5400
   gatcaaatat gcaaatgtta gccagtactt gtgttaaact tgatggcatc tcttggtttc 5460
   tttggcaatc acatgcctaa gaaataaata gtatcatatg attgtgtttg gtcagacttc 5520
   agagtcagat gactctgttt ggataaacag cttaattaag cgcttataga atatcatatg 5580
   attgtgtttg gtcagacttc agagcatctc ttggtttctc tggcaatcat atgcctaaga 5640
   aataaatagt atcatatgat tgtgtttggt cagacttcag agtcagatga ccctgtttgg 5700
   gtaaacagct taattaagtg cttatagaat aagcgcttat catataagtg cttttgtaca 5760
   gttatttcta tgaaagtaga agaaatagtc atattgtttt aatataagct atcctggaga 5820
   gcttgtggaa ataaccagaa aagaacttat ggacacgtca tgagctgttt acataagatc 5880
   tccctaacag tctcaaaagt gtttatgcca gtagataaat tcaaataagt caatctaaac 5940
   agaccctaaa tccattatgg tacctatcat tttagcttat tccatcttta ttaagaatgt 6000
   catgagataa cataatgata acacattatt ttgacacaaa tgggcagatc tagcaattta 6060
   actctggagt ccttcaagac tgctgttctt acgaagttca cgtccctgaa tcatgttcct 6120
   gtatggaagc ctgaaagacc tcaaattcta aaaggtggcg ataaattgaa ggtttacaaa 6180
   atataccctg cgggcttgac acagaggcaa gctctttata ccttccagtt caacggggat 6240
   gttgatttca gaagtcactt ggagagcaat ccttgtgcca agtttgaagt aatttttgtg 6300
   tagcatatgt tgagctacct acaatttaca tgatcaccta gcattagctc tttcacttaa 6360
   ctgagagaat gaagttttag gaatgagtat gaccatggag tcggcatggc tttgtaatgc 6420
   ctaccctact ttggccaact catcggggat ttacattcag aaaatataca tgacttcaac 6480
   catacttaaa cccctttttg taagataact gaatgttcat atttaatgtt gggttgtagt 6540
   gtttttactt gattatatcc agacagttac aagttggaca acaagattgt gggtctgtac 6600
   tgttatttat ttattttttt tttagcagaa acaccttatc ttttgtttcg tttgaatgta 6660
   gaatgaaaat aaaagaaaga aaatataaca tcatcggccg cgcttgtcta atttcgggca 6720
   gttaggatcc tctccggtca ccggaaagtt tcagtagaag aaacaaaaca ccgtgactaa 6780
   aatgatacta ttattttatt tattgtgttt ttcttttttc taccggaact ttttagaacg 6840
   gatcccaact cgttccgggg ccgctacaac tgaaacaaaa gaagatattt tctctctctt 6900
   cagaaatgta agttttcctt tacagatacc cattcaccat ttgattcaga tgtggtgact 6960
   agagataaag catactaatt tgactcttgg aaacccataa agtttatgtt atccgtgttc 7020
   tggaccaatc cacttggggg cataacctgt gtctatgtgt ggtttggttt ccattctgat 7080
   ttatgcggcg acttgtaatt taaaatctag gaggggcaga cattgaacaa tcccaatatt 7140
   ttaataactt atgcaagatt ttttttatta atgagatgat gtgtttgtga ctgagattga 7200
   gtcatacatt tcactaagaa atggttccaa gtaccaaact atcatgaccc agttgcaaac 7260
   atgacgttcg ggagtggtca ctttgatagt tcaatttcat cttggcttct tattcctttt 7320
   ataattctaa ttcttcttgt gtaaactatt tcatgtatta tttttcttta aaatttacat 7380
   gtcatttatt ttgcctcact aactcaattt tgcatataac aatgataagt gatattttga 7440
   ctcacaaaat ttacatcaaa tttcgacatc gtttattatg ttcattggat gattaacaaa 7500
   tataacaaac tttgcaacta attaaccacc aactgaatat aattaactat aactgtgaaa 7560
   gtagttaact catttttata tttcatagat caaataagag aaataacggt atattaatcc 7620
   ctccaaaaaa aaaaaacggt atatttacta aaaaatctaa gccacgtagg aggataacag 7680
   gatccccgta ggaggataac atccaatcca accaatcaca acaatcctga tgagataacc 7740
   cactttaagc ccacgcatct gtggcacatc tacattatct aaatcacaca ttcttccaca 7800
   catctgagcc acacaaaaac caatccacat ctttatcacc cattctataa aaaatcacac 7860
   tttgtgagtc tacactttga ttcccttcaa acacatacaa agagaagaga ctaattaatt 7920
   aattaatcat cttgagagaa aatgtttggg cgcggaccaa caaggaagag tgataacacc 7980
   aaatattacg atattcttgg tgtttcaaaa agtgctagtg aagatgaaat caagaaagcc 8040
   tatagaaagg cagcgatgaa gaaccatcca gataagggtg gggatcctga gaagttcaag 8100
   gagttgggcc aagcatatga agtgttgagc gatcctgaaa agaaagaact gtatgatcaa 8160
   tatggtgaag atgcccttaa agaaggaatg gggggaggcg caggaagctc atttcataat 8220
   ccgtttgata ttttcgaatc attttttggt gcaggctttg gtggtggtgg tccttcacgc 8280
   gcaagaagac agaagcaagg agaagatgtg gtgcattcta taaaggtttc cttggaggat 8340
   gtgtataacg gcactacaaa gaagctatca ctttctagga atgcactgtg ctcaaaatgt 8400
   aaagggaaag gttcaaaaag tggaactgct ggaaggtgtt ttggatgcca gggcacaggt 8460
   atgaagatta ccagaaggca aattggactg ggcatgattc aacaaatgca acacgtctgt 8520
   cctgactgca aaggaacagg cgaggtcatt agtgagagag atagatgccc tcaatgcaag 8580
   ggaaacaaga ttactcaaga aaagaaggtg ctggaggtgc atgtggaaaa ggggatgcag 8640
   cagggtcaca agattgtatt cgaaggacaa gctgatgaag ctcctgatac aatcacagga 8700
   gacatagttt ttgtcttgca agtaaaggga catccgaagt ttcggaggga gcgtgatgac 8760
   ctccacattg aacacaattt gagcttaact gaggctctct gtggcttcca gtttaatgtc 8820
   acacatcttg atggaaggca actattggtc aaatcgaacc ccggcgaagt catcaagcca 8880
   ggtcaacata aagctataaa tgatgaggga atgccacaac atggtaggcc gttcatgaag 8940
   ggacgcctat acatcaagtt tagtgttgat ttcccggatt cgggttttct ttccccaagc 9000
   caaagcctgg aattagaaaa gatattacct caaaagacaa gcaagaactt gtcccaaaag 9060
   gaggtagatg attgtgagga gaccaccctg catgatgtca atattgcaga ggagatgagt 9120
   cgaaagaagc aacaataccg tgaggcatat gatgacgatg atgatgaaga tgatgagcac 9180
   tcgcagcctc gggtgcaatg cgctcaacag taggagctca gctcgaattt ccccgatcgt 9240
   tcaaacattt ggcaataaag tttcttaaga ttgaatcctg ttgccggtct tgcgatgatt 9300
   atcatataat ttctgttgaa ttacgttaag catgtaataa ttaacatgta atgcatgacg 9360
   ttatttatga gatgggtttt tatgattaga gtcccgcaat tatacattta atacgcgata 9420
   gaaaacaaaa tatagcgcgc aaactaggat aaattatcgc gcgcggtgtc atctatgtta 9480
   ctagatcgaa ttc 9493
<210> 124
   <211> 34
   <212> DNA
   <213> Artificial sequence
<220>
   <223> supP19-plasto.r
<400> 124
   ccttgtatag ctcgttccat tttctctcaa gatg 34
<210> 125
   <211> 20
   <212> DNA
   <213> Artificial sequence
<220>
   <223> supP19-1c
<400> 125
   atggaacgag ctatacaagg 20
<210> 126
   <211> 32
   <212> DNA
   <213> Artificial sequence
<220>
   <223> SupP19-SacI.r
<400> 126
   agtcgagctc ttactcgctt tctttttcga ag 32
<210> 127
   <211> 3462
   <212> DNA
   <213> Artificial
<220>
   <223> A/California/04/09 (cassette number 560)
<400> 127
   gtcaacatgg tggagcacga cacacttgtc tactccaaaa atatcaaaga tacagtctca    60
   gaagaccaaa gggcaattga gacttttcaa caaagggtaa tatccggaaa cctcctcgga   120
   ttccattgcc cagctatctg tcactttatt gtgaagatag tggaaaagga aggtggctcc   180
   tacaaatgcc atcattgcga taaaggaaag gccatcgttg aagatgcctc tgccgacagt   240
   ggtcccaaag atggaccccc acccacgagg agcatcgtgg aaaaagaaga cgttccaacc   300
   acgtcttcaa agcaagtgga ttgatgtgat aacatggtgg agcacgacac acttgtctac   360
   tccaaaaata tcaaagatac agtctcagaa gaccaaaggg caattgagac ttttcaacaa   420
   agggtaatat ccggaaacct cctcggattc cattgcccag ctatctgtca ctttattgtg   480
   aagatagtgg aaaaggaagg tggctcctac aaatgccatc attgcgataa aggaaaggcc   540
   atcgttgaag atgcctctgc cgacagtggt cccaaagatg gacccccacc cacgaggagc   600
   atcgtggaaa aagaagacgt tccaaccacg tcttcaaagc aagtggattg atgtgatatc   660
   tccactgacg taagggatga cgcacaatcc cactatcctt cgcaagaccc ttcctctata   720
   taaggaagtt catttcattt ggagaggtat taaaatctta ataggttttg ataaaagcga   780
   acgtggggaa acccgaacca aaccttcttc taaactctct ctcatctctc ttaaagcaaa   840
   cttctctctt gtctttcttg cgtgagcgat cttcaacgtt gtcagatcgt gcttcggcac   900
   cagtacaacg ttttctttca ctgaagcgaa atcaaagatc tctttgtgga cacgtagtgc   960
   ggcgccatta aataacgtgt acttgtccta ttcttgtcgg tgtggtcttg ggaaaagaaa  1020
   gcttgctgga ggctgctgtt cagccccata cattacttgt tacgattctg ctgactttcg  1080
   gcgggtgcaa tatctctact tctgcttgac gaggtattgt tgcctgtact tctttcttct  1140
   tcttcttgct gattggttct ataagaaatc tagtattttc tttgaaacag agttttcccg  1200
   tggttttcga acttggagaa agattgttaa gcttctgtat attctgccca aatttgtcgg  1260
   gcccatggcg aaaaacgttg cgattttcgg cttattgttt tctcttcttg tgttggttcc  1320
   ttctcagatc ttcgctgaca cattatgtat aggttatcat gcgaacaatt caacagacac  1380
   tgtagacaca gtactagaaa agaatgtaac agtaacacac tctgttaacc ttctagaaga  1440
   caagcataac gggaaactat gcaaactaag aggggtagcc ccattgcatt tgggtaaatg  1500
   taacattgct ggctggatcc tgggaaatcc agagtgtgaa tcactctcca cagcaagctc  1560
   atggtcctac attgtggaaa cacctagttc agacaatgga acgtgttacc caggagattt  1620
   catcgattat gaggagctaa gagagcaatt aagctcagtg tcatcatttg aaaggtttga  1680
   gatattcccc aagacaagtt catggcccaa tcatgactcg aacaaaggtg taacggcagc  1740
   atgtcctcat gctggagcaa aaagcttcta caaaaattta atatggctag ttaaaaaagg 1800
   aaattcatac ccaaagctca gcaaatccta cattaatgat aaagggaaag aagtcctcgt 1860
   gctatggggc attcaccatc catctactag tgctgaccaa caaagtctct atcagaatgc 1920
   agatacatat gtttttgtgg ggtcatcaag atacagcaag aagttcaagc cggaaatagc 1980
   aataagaccc aaagtgaggg atcaagaagg gagaatgaac tattactgga cactagtaga 2040
   gccgggagac aaaataacat tcgaagcaac tggaaatcta gtggtaccga gatatgcatt 2100
   cgcaatggaa agaaatgctg gatctggtat tatcatttca gatacaccag tccacgattg 2160
   caatacaact tgtcaaacac ccaagggtgc tataaacacc agcctcccat ttcagaatat 2220
   acatccgatc acaattggaa aatgtccaaa atatgtaaaa agcacaaaat tgagactggc 2280
   cacaggattg aggaatatcc cgtctattca atctagagga ctatttgggg ccattgccgg 2340
   tttcattgaa ggggggtgga cagggatggt agatggatgg tacggttatc accatcaaaa 2400
   tgagcagggg tcaggatatg cagccgacct gaagagcaca cagaatgcca ttgacgagat 2460
   tactaacaaa gtaaattctg ttattgaaaa gatgaataca cagttcacag cagtaggtaa 2520
   agagttcaac cacctggaaa aaagaataga gaatttaaat aaaaaagttg atgatggttt 2580
   cctggacatt tggacttaca atgccgaact gttggttcta ttggaaaatg aaagaacttt 2640
   ggactaccac gattcaaatg tgaagaactt atatgaaaag gtaagaagcc agctaaaaaa 2700
   caatgccaag gaaattggaa acggctgctt tgaattttac cacaaatgcg ataacacgtg 2760
   catggaaagt gtcaaaaatg ggacttatga ctacccaaaa tactcagagg aagcaaaatt 2820
   aaacagagaa gaaatagatg gggtaaagct ggaatcaaca aggatttacc agattttggc 2880
   gatctattca actgtcgcca gttcattggt actggtagtc tccctggggg caatcagttt 2940
   ctggatgtgc tctaatgggt ctctacagtg tagaatatgt atttaaaggc ctattttctt 3000
   tagtttgaat ttactgttat tcggtgtgca tttctatgtt tggtgagcgg ttttctgtgc 3060
   tcagagtgtg tttattttat gtaatttaat ttctttgtga gctcctgttt agcaggtcgt 3120
   cccttcagca aggacacaaa aagattttaa ttttattaaa aaaaaaaaaa aaaaagaccg 3180
   ggaattcgat atcaagctta tcgacctgca gatcgttcaa acatttggca ataaagtttc 3240
   ttaagattga atcctgttgc cggtcttgcg atgattatca tataatttct gttgaattac 3300
   gttaagcatg taataattaa catgtaatgc atgacgttat ttatgagatg ggtttttatg 3360
   attagagtcc cgcaattata catttaatac gcgatagaaa acaaaatata gcgcgcaaac 3420
   taggataaat tatcgcgcgc ggtgtcatct atgttactag at 3462
<210> 128
   <211> 573
   <212> PRT
   <213> Artificial sequence
<220>
   <223> A/California/04/09
<400> 128
   Met Ala Lys Asn Val Ala Ile Phe Gly Leu Leu Phe Ser Leu Leu Val
   1 5 10 15
   Leu Val Pro Ser Gln Ile Phe Ala Asp Thr Leu Cys Ile Gly Tyr His
   20 25 30
   Ala Asn Asn Ser Thr Asp Thr Val Asp Thr Val Leu Glu Lys Asn Val
   35 40 45
   Thr Val Thr His Ser Val Asn Leu Leu Glu Asp Lys His Asn Gly Lys
   50 55 60
   Leu Cys Lys Leu Arg Gly Val Ala Pro Leu His Leu Gly Lys Cys Asn
   65 70 75 80
   Ile Ala Gly Trp Ile Leu Gly Asn Pro Glu Cys Glu Ser Leu Ser Thr
   85 90 95
   Ala Ser Ser Trp Ser Tyr Ile Val Glu Thr Pro Ser Ser Asp Asn Gly
   100 105 110
   Thr Cys Tyr Pro Gly Asp Phe Ile Asp Tyr Glu Glu Leu Arg Glu Gln
   115 120 125
   Leu Ser Ser Val Ser Ser Phe Glu Arg Phe Glu Ile Phe Pro Lys Thr
   130 135 140
   Ser Ser Trp Pro Asn His Asp Ser Asn Lys Gly Val Thr Ala Ala Cys
   145 150 155 160
   Pro His Ala Gly Ala Lys Ser Phe Tyr Lys Asn Leu Ile Trp Leu Val
   165 170 175
   Lys Lys Gly Asn Ser Tyr Pro Lys Leu Ser Lys Ser Tyr Ile Asn Asp
   180 185 190
   Lys Gly Lys Glu Val Leu Val Leu Trp Gly Ile His His Pro Ser Thr
   195 200 205
   Ser Ala Asp Gln Gln Ser Leu Tyr Gln Asn Ala Asp Thr Tyr Val Phe
   210 215 220
   Val Gly Ser Ser Arg Tyr Ser Lys Lys Phe Lys Pro Glu Ile Ala Ile
   225 230 235 240
   Arg Pro Lys Val Arg Asp Gln Glu Gly Arg Met Asn Tyr Tyr Trp Thr
   245 250 255
   Leu Val Glu Pro Gly Asp Lys Ile Thr Phe Glu Ala Thr Gly Asn Leu
   260 265 270
   Val Val Pro Arg Tyr Ala Phe Ala Met Glu Arg Asn Ala Gly Ser Gly
   275 280 285
   Ile Ile Ile Ser Asp Thr Pro Val His Asp Cys Asn Thr Thr Cys Gln
   290 295 300
   Thr Pro Lys Gly Ala Ile Asn Thr Ser Leu Pro Phe Gln Asn Ile His
   305 310 315 320
   Pro Ile Thr Ile Gly Lys Cys Pro Lys Tyr Val Lys Ser Thr Lys Leu
   325 330 335
   Arg Leu Ala Thr Gly Leu Arg Asn Ile Pro Ser Ile Gln Ser Arg Gly
   340 345 350
   Leu Phe Gly Ala Ile Ala Gly Phe Ile Glu Gly Gly Trp Thr Gly Met
   355 360 365
   Val Asp Gly Trp Tyr Gly Tyr His His Gln Asn Glu Gln Gly Ser Gly
   370 375 380
   Tyr Ala Ala Asp Leu Lys Ser Thr Gln Asn Ala Ile Asp Glu Ile Thr
   385 390 395 400
   Asn Lys Val Asn Ser Val Ile Glu Lys Met Asn Thr Gln Phe Thr Ala
   405 410 415
   Val Gly Lys Glu Phe Asn His Leu Glu Lys Arg Ile Glu Asn Leu Asn
   420 425 430
   Lys Lys Val Asp Asp Gly Phe Leu Asp Ile Trp Thr Tyr Asn Ala Glu
   435 440 445
   Leu Leu Val Leu Leu Glu Asn Glu Arg Thr Leu Asp Tyr His Asp Ser
   450 455 460
   Asn Val Lys Asn Leu Tyr Glu Lys Val Arg Ser Gln Leu Lys Asn Asn
   465 470 475 480
   Ala Lys Glu Ile Gly Asn Gly Cys Phe Glu Phe Tyr His Lys Cys Asp
   485 490 495
   Asn Thr Cys Met Glu Ser Val Lys Asn Gly Thr Tyr Asp Tyr Pro Lys
   500 505 510
   Tyr Ser Glu Glu Ala Lys Leu Asn Arg Glu Glu Ile Asp Gly Val Lys
           515                 520                 525
   Leu Glu Ser Thr Arg Ile Tyr Gln Ile Leu Ala Ile Tyr Ser Thr Val
       530                 535                 540
   Ala Ser Ser Leu Val Leu Val Val Ser Leu Gly Ala Ile Ser Phe Trp
   545                 550                 555                 560
   Met Cys Ser Asn Gly Ser Leu Gln Cys Arg Ile Cys Ile
                   565                 570
<210> 129
   <211> 747
   <212> DNA
   <213> Artificial sequence
<220>
   <223> 2X35S promoter
<400> 129
   gtcaacatgg tggagcacga cacacttgtc tactccaaaa atatcaaaga tacagtctca      60
   gaagaccaaa gggcaattga gacttttcaa caaagggtaa tatccggaaa cctcctcgga     120
   ttccattgcc cagctatctg tcactttatt gtgaagatag tggaaaagga aggtggctcc     180
   tacaaatgcc atcattgcga taaaggaaag gccatcgttg aagatgcctc tgccgacagt     240
   ggtcccaaag atggaccccc acccacgagg agcatcgtgg aaaaagaaga cgttccaacc     300
   acgtcttcaa agcaagtgga ttgatgtgat aacatggtgg agcacgacac acttgtctac     360
   tccaaaaata tcaaagatac agtctcagaa gaccaaaggg caattgagac ttttcaacaa     420
   agggtaatat ccggaaacct cctcggattc cattgcccag ctatctgtca ctttattgtg     480
   aagatagtgg aaaaggaagg tggctcctac aaatgccatc attgcgataa aggaaaggcc     540
   atcgttgaag atgcctctgc cgacagtggt cccaaagatg gacccccacc cacgaggagc     600
   atcgtggaaa aagaagacgt tccaaccacg tcttcaaagc aagtggattg atgtgatatc     660
   tccactgacg taagggatga cgcacaatcc cactatcctt cgcaagaccc ttcctctata     720
   taaggaagtt catttcattt ggagagg                                         747
<210> 130
   <211> 43
   <212> DNA
   <213> Artificial sequence
<220>
   <223> primer PacI-MCS-2X35S.c
<400> 130
   aattgttaat taagtcgaca agcttgcatg cctgcaggtc aac 43
<210> 131
   <211> 48
   <212> DNA
   <213> Artificial sequence
<220>
   <223> primer CPMV 5'UTR-2X35S.r
<400> 131
   tcaaaaccta ttaagatttt aatacctctc caaatgaaat gaacttcc 48
<210> 132
   <211> 49
   <212> DNA
   <213> Artificial sequence
<220>
   <223> primer 2X35S-CPMV 5'UTR.c
<400> 132
   ttggagaggt attaaaatct taataggttt tgataaaagc gaacgtggg 49
<210> 133
   <211> 44
   <212> DNA
   <213> Artificial sequence
<220>
   <223> primer ApaI-M prot.r
<400> 133
   tctccatggg cccgacaaat ttgggcagaa tatacagaag ctta 44
<210> 134
   <211> 3505
   <212> DNA
   <213> Artificial sequence
<220>
   <223> expression cassette number 972
<400> 134
   ttaattaagt cgacaagctt gcatgcctgc aggtcaacat ggtggagcac gacacacttg 60
   tctactccaa aaatatcaaa gatacagtct cagaagacca aagggcaatt gagacttttc 120
   aacaaagggt aatatccgga aacctcctcg gattccattg cccagctatc tgtcacttta 180
   ttgtgaagat agtggaaaag gaaggtggct cctacaaatg ccatcattgc gataaaggaa 240
   aggccatcgt tgaagatgcc tctgccgaca gtggtcccaa agatggaccc ccacccacga 300
   ggagcatcgt ggaaaaagaa gacgttccaa ccacgtcttc aaagcaagtg gattgatgtg 360
   ataacatggt ggagcacgac acacttgtct actccaaaaa tatcaaagat acagtctcag 420
   aagaccaaag ggcaattgag acttttcaac aaagggtaat atccggaaac ctcctcggat 480
   tccattgccc agctatctgt cactttattg tgaagatagt ggaaaaggaa ggtggctcct 540
   acaaatgcca tcattgcgat aaaggaaagg ccatcgttga agatgcctct gccgacagtg 600
   gtcccaaaga tggaccccca cccacgagga gcatcgtgga aaaagaagac gttccaacca 660
   cgtcttcaaa gcaagtggat tgatgtgata tctccactga cgtaagggat gacgcacaat 720
   cccactatcc ttcgcaagac ccttcctcta tataaggaag ttcatttcat ttggagaggt 780
   attaaaatct taataggttt tgataaaagc gaacgtgggg aaacccgaac caaaccttct 840
   tctaaactct ctctcatctc tcttaaagca aacttctctc ttgtctttct tgcgtgagcg 900
   atcttcaacg ttgtcagatc gtgcttcggc accagtacaa cgttttcttt cactgaagcg 960
   aaatcaaaga tctctttgtg gacacgtagt gcggcgccat taaataacgt gtacttgtcc 1020
   tattcttgtc ggtgtggtct tgggaaaaga aagcttgctg gaggctgctg ttcagcccca 1080
   tacattactt gttacgattc tgctgacttt cggcgggtgc aatatctcta cttctgcttg 1140
   acgaggtatt gttgcctgta cttctttctt cttcttcttg ctgattggtt ctataagaaa 1200
   tctagtattt tctttgaaac agagttttcc cgtggttttc gaacttggag aaagattgtt 1260
   aagcttctgt atattctgcc caaatttgtc gggcccatgg agaaaatagt gcttcttctt 1320
   gcaatagtca gtcttgttaa aagtgatcag atttgcattg gttaccatgc aaacaattca 1380
   acagagcagg ttgacacaat catggaaaag aacgttactg ttacacatgc ccaagacata 1440
   ctggaaaaga cacacaacgg gaagctctgc gatctagatg gagtgaagcc tctaatttta 1500
   agagattgta gtgtagctgg atggctcctc gggaacccaa tgtgtgacga attcatcaat 1560
   gtaccggaat ggtcttacat agtggagaag gccaatccaa ccaatgacct ctgttaccca 1620
   gggagtttca acgactatga agaactgaaa cacctattga gcagaataaa ccattttgag 1680
   aaaattcaaa tcatccccaa aagttcttgg tccgatcatg aagcctcatc aggagttagc 1740
   tcagcatgtc catacctggg aagtccctcc ttttttagaa atgtggtatg gcttatcaaa 1800
   aagaacagta catacccaac aataaagaaa agctacaata ataccaacca agaggatctt 1860
   ttggtactgt ggggaattca ccatcctaat gatgcggcag agcagacaag gctatatcaa 1920
   aacccaacca cctatatttc cattgggaca tcaacactaa accagagatt ggtaccaaaa 1980
   atagctacta gatccaaagt aaacgggcaa agtggaagga tggagttctt ctggacaatt 2040
   ttaaaaccta atgatgcaat caacttcgag agtaatggaa atttcattgc tccagaatat 2100
   gcatacaaaa ttgtcaagaa aggggactca gcaattatga aaagtgaatt ggaatatggt 2160
   aactgcaaca ccaagtgtca aactccaatg ggggcgataa actctagtat gccattccac 2220
   aacatacacc ctctcaccat cggggaatgc cccaaatatg tgaaatcaaa cagattagtc 2280
   cttgcaacag ggctcagaaa tagccctcaa agagagagca gaagaaaaaa gagaggacta 2340
   tttggagcta tagcaggttt tatagaggga ggatggcagg gaatggtaga tggttggtat 2400
   gggtaccacc atagcaatga gcaggggagt gggtacgctg cagacaaaga atccactcaa 2460
   aaggcaatag atggagtcac caataaggtc aactcaatca ttgacaaaat gaacactcag 2520
   tttgaggccg ttggaaggga atttaataac ttagaaagga gaatagagaa tttaaacaag   2580
   aagatggaag acgggtttct agatgtctgg acttataatg ccgaacttct ggttctcatg   2640
   gaaaatgaga gaactctaga ctttcatgac tcaaatgtta agaacctcta cgacaaggtc   2700
   cgactacagc ttagggataa tgcaaaggag ctgggtaacg gttgtttcga gttctatcac   2760
   aaatgtgata atgaatgtat ggaaagtata agaaacggaa cgtacaacta tccgcagtat   2820
   tcagaagaag caagattaaa aagagaggaa ataagtgggg taaaattgga atcaatagga   2880
   acttaccaaa tactgtcaat ttattcaaca gtggcgagtt ccctagcact ggcaatcatg   2940
   atggctggtc tatctttatg gatgtgctcc aatggatcgt tacaatgcag aatttgcatt   3000
   taaaggccta ttttctttag tttgaattta ctgttattcg gtgtgcattt ctatgtttgg   3060
   tgagcggttt tctgtgctca gagtgtgttt attttatgta atttaatttc tttgtgagct   3120
   cctgtttagc aggtcgtccc ttcagcaagg acacaaaaag attttaattt tattaaaaaa   3180
   aaaaaaaaaa aagaccggga attcgatatc aagcttatcg acctgcagat cgttcaaaca   3240
   tttggcaata aagtttctta agattgaatc ctgttgccgg tcttgcgatg attatcatat   3300
   aatttctgtt gaattacgtt aagcatgtaa taattaacat gtaatgcatg acgttattta   3360
   tgagatgggt ttttatgatt agagtcccgc aattatacat ttaatacgcg atagaaaaca   3420
   aaatatagcg cgcaaactag gataaattat cgcgcgcggt gtcatctatg ttactagatt   3480
   ctagagtctc aagcttcggc gcgcc                                         3505
<210> 135
   <211> 1701
   <212> DNA
   <213> Influenza virus
<400> 135
   atgaaggcaa tactagtagt tctgctatat acatttgcaa ccgcaaatgc agacacatta 60
   tgtataggtt atcatgcgaa caattcaaca gacactgtag acacagtact agaaaagaat 120
   gtaacagtaa cacactctgt taaccttcta gaagacaagc ataacgggaa actatgcaaa 180
   ctaagagggg tagccccatt gcatttgggt aaatgtaaca ttgctggctg gatcctggga 240
   aatccagagt gtgaatcact ctccacagca agctcatggt cctacattgt ggaaacacct 300
   agttcagaca atggaacgtg ttacccagga gatttcatcg attatgagga gctaagagag 360
   caattgagct cagtgtcatc atttgaaagg tttgagatat tccccaagac aagttcatgg 420
   cccaatcatg actcgaacaa aggtgtaacg gcagcatgtc ctcatgctgg agcaaaaagc 480
   ttctacaaaa atttaatatg gctagttaaa aaaggaaatt catacccaaa gctcagcaaa 540
   tcctacatta atgataaagg gaaagaagtc ctcgtgctat ggggcattca ccatccatct 600
   actagtgctg accaacaaag tctctatcag aatgcagata catatgtttt tgtggggtca 660
   tcaagataca gcaagaagtt caagccggaa atagcaataa gacccaaagt gagggatcaa 720
   gaagggagaa tgaactatta ctggacacta gtagagccgg gagacaaaat aacattcgaa 780
   gcaactggaa atctagtggt accgagatat gcattcgcaa tggaaagaaa tgctggatct 840
   ggtattatca tttcagatac accagtccac gattgcaata caacttgtca aacacccaag 900
   ggtgctataa acaccagcct cccatttcag aatatacatc cgatcacaat tggaaaatgt 960
   ccaaaatatg taaaaagcac aaaattgaga ctggccacag gattgaggaa tatcccgtct 1020
   attcaatcta gaggcctatt tggggccatt gccggtttca ttgaaggggg gtggacaggg 1080
   atggtagatg gatggtacgg ttatcaccat caaaatgagc aggggtcagg atatgcagcc 1140
   gacctgaaga gcacacagaa tgccattgac gagattacta acaaagtaaa ttctgttatt 1200
   gaaaagatga atacacagtt cacagcagta ggtaaagagt tcaaccacct ggaaaaaaga 1260
   atagagaatt taaataaaaa agttgatgat ggtttcctgg acatttggac ttacaatgcc 1320
   gaactgttgg ttctattgga aaatgaaaga actttggact accacgattc aaatgtgaag 1380
   aacttatatg aaaaggtaag aagccagcta aaaaacaatg ccaaggaaat tggaaacggc 1440
   tgctttgaat tttaccacaa atgcgataac acgtgcatgg aaagtgtcaa aaatgggact 1500
   tatgactacc caaaatactc agaggaagca aaattaaaca gagaagaaat agatggggta 1560
   aagctggaat caacaaggat ttaccagatt ttggcgatct attcaactgt cgccagttca 1620
   ttggtactgg tagtctccct gggggcaatc agtttctgga tgtgctctaa tgggtctcta 1680
   cagtgtagaa tatgtattta a 1701
<210> 136
   <211> 2056
   <212> DNA
   <213> Artificial sequence
<220>
   <223> to be synthesized containing H1 A/California/4/2009
<400> 136
   atgctaatat cacgtagtgc ggcgccatta aataacgtgt acttgtccta ttcttgtcgg      60
   tgtggtcttg ggaaaagaaa gcttgctgga ggctgctgtt cagccccata cattacttgt     120
   tacgattctg ctgactttcg gcgggtgcaa tatctctact tctgcttgac gaggtattgt     180
   tgcctgtact tctttcttct tcttcttgct gattggttct ataagaaatc tagtattttc     240
   tttgaaacag agttttcccg tggttttcga acttggagaa agattgttaa gcttctgtat     300
   attctgccca aatttgtcgg gcccatggcg aaaaacgttg cgattttcgg cttattgttt     360
   tctcttcttg tgttggttcc ttctcagatc ttcgctgaca cattatgtat aggttatcat     420
   gcgaacaatt caacagacac tgtagacaca gtactagaaa agaatgtaac agtaacacac     480
   tctgttaacc ttctagaaga caagcataac gggaaactat gcaaactaag aggggtagcc     540
   ccattgcatt tgggtaaatg taacattgct ggctggatcc tgggaaatcc agagtgtgaa     600
   tcactctcca cagcaagctc atggtcctac attgtggaaa cacctagttc agacaatgga     660
   acgtgttacc caggagattt catcgattat gaggagctaa gagagcaatt aagctcagtg     720
   tcatcatttg aaaggtttga gatattcccc aagacaagtt catggcccaa tcatgactcg     780
   aacaaaggtg taacggcagc atgtcctcat gctggagcaa aaagcttcta caaaaattta     840
   atatggctag ttaaaaaagg aaattcatac ccaaagctca gcaaatccta cattaatgat     900
   aaagggaaag aagtcctcgt gctatggggc attcaccatc catctactag tgctgaccaa     960
   caaagtctct atcagaatgc agatacatat gtttttgtgg ggtcatcaag atacagcaag    1020
   aagttcaagc cggaaatagc aataagaccc aaagtgaggg atcaagaagg gagaatgaac    1080
   tattactgga cactagtaga gccgggagac aaaataacat tcgaagcaac tggaaatcta    1140
   gtggtaccga gatatgcatt cgcaatggaa agaaatgctg gatctggtat tatcatttca    1200
   gatacaccag tccacgattg caatacaact tgtcaaacac ccaagggtgc tataaacacc    1260
   agcctcccat ttcagaatat acatccgatc acaattggaa aatgtccaaa atatgtaaaa    1320
   agcacaaaat tgagactggc cacaggattg aggaatatcc cgtctattca atctagagga    1380
   ctatttgggg ccattgccgg tttcattgaa ggggggtgga cagggatggt agatggatgg    1440
   tacggttatc accatcaaaa tgagcagggg tcaggatatg cagccgacct gaagagcaca    1500
   cagaatgcca ttgacgagat tactaacaaa gtaaattctg ttattgaaaa gatgaataca    1560
   cagttcacag cagtaggtaa agagttcaac cacctggaaa aaagaataga gaatttaaat    1620
   aaaaaagttg atgatggttt cctggacatt tggacttaca atgccgaact gttggttcta    1680
   ttggaaaatg aaagaacttt ggactaccac gattcaaatg tgaagaactt atatgaaaag    1740
   gtaagaagcc agctaaaaaa caatgccaag gaaattggaa acggctgctt tgaattttac    1800
   cacaaatgcg ataacacgtg catggaaagt gtcaaaaatg ggacttatga ctacccaaaa    1860
   tactcagagg aagcaaaatt aaacagagaa gaaatagatg gggtaaagct ggaatcaaca    1920
   aggatttacc agattttggc gatctattca actgtcgcca gttcattggt actggtagtc    1980
   tccctggggg caatcagttt ctggatgtgc tctaatgggt ctctacagtg tagaatatgt    2040
   atttaaaggc ctaata                                                    2056
<210> 137
   <211> 714
   <212> DNA
   <213> Artificial sequence
<220>
   <223> synthesized fragment 1
<400> 137
   atgctaatat cacgtagtgc ggcgccatta aataacgtgt acttgtccta ttcttgtcgg 60
   tgtggtcttg ggaaaagaaa gcttgctgga ggctgctgtt cagccccata cattacttgt 120
   tacgattctg ctgactttcg gcgggtgcaa tatctctact tctgcttgac gaggtattgt 180
   tgcctgtact tctttcttct tcttcttgct gattggttct ataagaaatc tagtattttc 240
   tttgaaacag agttttcccg tggttttcga acttggagaa agattgttaa gcttctgtat 300
   attctgccca aatttgtcgg gcccatggcg aaaaacgttg cgattttcgg cttattgttt 360
   tctcttcttg tgttggttcc ttctcagatc ttcgctgaca cattatgtat aggttatcat 420
   gcgaacaatt caacagacac tgtagacaca gtactagaaa agaatgtaac agtaacacac 480
   tctgttaacc ttctagaaga caagcataac gggaaactat gcaaactaag aggggtagcc 540
   ccattgcatt tgggtaaatg taacattgct ggctggatcc tgggaaatcc agagtgtgaa 600
   tcactctcca cagcaagctc atggtcctac attgtggaaa cacctagttc agacaatgga 660
   acgtgttacc caggagattt catcgattat gaggagctaa gagagcaatt aagc 714
<210> 138
   <211> 849
   <212> DNA
   <213> Artificial sequence
<220>
   <223> synthesized fragment 2
<400> 138
   tggaaacacc tagttcagac aatggaacgt gttacccagg agatttcatc gattatgagg     60
   agctaagaga gcaattaagc tcagtgtcat catttgaaag gtttgagata ttccccaaga    120
   caagttcatg gcccaatcat gactcgaaca aaggtgtaac ggcagcatgt cctcatgctg    180
   gagcaaaaag cttctacaaa aatttaatat ggctagttaa aaaaggaaat tcatacccaa    240
   agctcagcaa atcctacatt aatgataaag ggaaagaagt cctcgtgcta tggggcattc    300
   accatccatc tactagtgct gaccaacaaa gtctctatca gaatgcagat acatatgttt    360
   ttgtggggtc atcaagatac agcaagaagt tcaagccgga aatagcaata agacccaaag    420
   tgagggatca agaagggaga atgaactatt actggacact agtagagccg ggagacaaaa    480
   taacattcga agcaactgga aatctagtgg taccgagata tgcattcgca atggaaagaa    540
   atgctggatc tggtattatc atttcagata caccagtcca cgattgcaat acaacttgtc    600
   aaacacccaa gggtgctata aacaccagcc tcccatttca gaatatacat ccgatcacaa    660
   ttggaaaatg tccaaaatat gtaaaaagca caaaattgag actggccaca ggattgagga    720
   atatcccgtc tattcaatct agaggactat ttggggccat tgccggtttc attgaagggg    780
   ggtggacagg gatggtagat ggatggtacg gttatcacca tcaaaatgag caggggtcag    840
   gatatgcag                                                            849
<210> 139
   <211> 651
   <212> DNA
   <213> Artificial sequence
<220>
   <223> synthesized fragment 3
<400> 139
   ttgaaggggg gtggacaggg atggtagatg gatggtacgg ttatcaccat caaaatgagc      60
   aggggtcagg atatgcagcc gacctgaaga gcacacagaa tgccattgac gagattacta     120
   acaaagtaaa ttctgttatt gaaaagatga atacacagtt cacagcagta ggtaaagagt     180
   tcaaccacct ggaaaaaaga atagagaatt taaataaaaa agttgatgat ggtttcctgg     240
   acatttggac ttacaatgcc gaactgttgg ttctattgga aaatgaaaga actttggact     300
   accacgattc aaatgtgaag aacttatatg aaaaggtaag aagccagcta aaaaacaatg     360
   ccaaggaaat tggaaacggc tgctttgaat tttaccacaa atgcgataac acgtgcatgg     420
   aaagtgtcaa aaatgggact tatgactacc caaaatactc agaggaagca aaattaaaca     480
   gagaagaaat agatggggta aagctggaat caacaaggat ttaccagatt ttggcgatct     540
   attcaactgt cgccagttca ttggtactgg tagtctccct gggggcaatc agtttctgga     600
   tgtgctctaa tgggtctcta cagtgtagaa tatgtattta aaggcctaat a              651
<210> 140
   <211> 48
   <212> DNA
   <213> Artificial sequence
<220>
   <223> primer DraIII-MProt#2.c
<400> 140
   atgctaatat cacgtagtgc ggcgccatta aataacgtgt acttgtcc 48
<210> 141
   <211> 42
   <212> DNA
   <213> Artificial sequence
<220>
   <223> primer H1 Cal.390r
<400> 141
   gcttaattgc tctcttagct cctcataatc gatgaaatct cc 42
<210> 142
   <211> 42
   <212> DNA
   <213> Artificial sequence
<220>
   <223> primer H1 Cal.310c
<400> 142
   tggaaacacc tagttcagac aatggaacgt gttacccagg ag 42
<210> 143
   <211> 42
   <212> DNA
   <213> Artificial sequence
<220>
   <223> primer H1 Cal.1159r
<400> 143
   ctgcatatcc tgacccctgc tcattttgat ggtgataacc gt 42
<210> 144
   <211> 42
   <212> DNA
   <213> Artificial sequence
<220>
   <223> primer H1 Cal.1081c
<400> 144
   ttgaaggggg gtggacaggg atggtagatg gatggtacgg tt 42
<210> 145
   <211> 45
   <212> DNA
   <213> Artificial sequence
<220>
   <223> primer StuI-H1 Cal.r
<400> 145
   tattaggcct ttaaatacat attctacact gtagagaccc attag 45
<210> 146
   <211> 3520
   <212> DNA
   <213> Artificial sequence
<220>
   <223> expression cassette number 560
<400> 146
   ttaattaagt cgacaagctt gcatgcctgc aggtcaacat ggtggagcac gacacacttg      60
   tctactccaa aaatatcaaa gatacagtct cagaagacca aagggcaatt gagacttttc     120
   aacaaagggt aatatccgga aacctcctcg gattccattg cccagctatc tgtcacttta     180
   ttgtgaagat agtggaaaag gaaggtggct cctacaaatg ccatcattgc gataaaggaa     240
   aggccatcgt tgaagatgcc tctgccgaca gtggtcccaa agatggaccc ccacccacga     300
   ggagcatcgt ggaaaaagaa gacgttccaa ccacgtcttc aaagcaagtg gattgatgtg     360
   ataacatggt ggagcacgac acacttgtct actccaaaaa tatcaaagat acagtctcag     420
   aagaccaaag ggcaattgag acttttcaac aaagggtaat atccggaaac ctcctcggat     480
   tccattgccc agctatctgt cactttattg tgaagatagt ggaaaaggaa ggtggctcct     540
   acaaatgcca tcattgcgat aaaggaaagg ccatcgttga agatgcctct gccgacagtg     600
   gtcccaaaga tggaccccca cccacgagga gcatcgtgga aaaagaagac gttccaacca     660
   cgtcttcaaa gcaagtggat tgatgtgata tctccactga cgtaagggat gacgcacaat     720
   cccactatcc ttcgcaagac ccttcctcta tataaggaag ttcatttcat ttggagaggt 780
   attaaaatct taataggttt tgataaaagc gaacgtgggg aaacccgaac caaaccttct 840
   tctaaactct ctctcatctc tcttaaagca aacttctctc ttgtctttct tgcgtgagcg 900
   atcttcaacg ttgtcagatc gtgcttcggc accagtacaa cgttttcttt cactgaagcg 960
   aaatcaaaga tctctttgtg gacacgtagt gcggcgccat taaataacgt gtacttgtcc 1020
   tattcttgtc ggtgtggtct tgggaaaaga aagcttgctg gaggctgctg ttcagcccca 1080
   tacattactt gttacgattc tgctgacttt cggcgggtgc aatatctcta cttctgcttg 1140
   acgaggtatt gttgcctgta cttctttctt cttcttcttg ctgattggtt ctataagaaa 1200
   tctagtattt tctttgaaac agagttttcc cgtggttttc gaacttggag aaagattgtt 1260
   aagcttctgt atattctgcc caaatttgtc gggcccatgg cgaaaaacgt tgcgattttc 1320
   ggcttattgt tttctcttct tgtgttggtt ccttctcaga tcttcgctga cacattatgt 1380
   ataggttatc atgcgaacaa ttcaacagac actgtagaca cagtactaga aaagaatgta 1440
   acagtaacac actctgttaa ccttctagaa gacaagcata acgggaaact atgcaaacta 1500
   agaggggtag ccccattgca tttgggtaaa tgtaacattg ctggctggat cctgggaaat 1560
   ccagagtgtg aatcactctc cacagcaagc tcatggtcct acattgtgga aacacctagt 1620
   tcagacaatg gaacgtgtta cccaggagat ttcatcgatt atgaggagct aagagagcaa 1680
   ttaagctcag tgtcatcatt tgaaaggttt gagatattcc ccaagacaag ttcatggccc 1740
   aatcatgact cgaacaaagg tgtaacggca gcatgtcctc atgctggagc aaaaagcttc 1800
   tacaaaaatt taatatggct agttaaaaaa ggaaattcat acccaaagct cagcaaatcc 1860
   tacattaatg ataaagggaa agaagtcctc gtgctatggg gcattcacca tccatctact 1920
   agtgctgacc aacaaagtct ctatcagaat gcagatacat atgtttttgt ggggtcatca 1980
   agatacagca agaagttcaa gccggaaata gcaataagac ccaaagtgag ggatcaagaa 2040
   gggagaatga actattactg gacactagta gagccgggag acaaaataac attcgaagca 2100
   actggaaatc tagtggtacc gagatatgca ttcgcaatgg aaagaaatgc tggatctggt 2160
   attatcattt cagatacacc agtccacgat tgcaatacaa cttgtcaaac acccaagggt 2220
   gctataaaca ccagcctccc atttcagaat atacatccga tcacaattgg aaaatgtcca 2280
   aaatatgtaa aaagcacaaa attgagactg gccacaggat tgaggaatat cccgtctatt 2340
   caatctagag gactatttgg ggccattgcc ggtttcattg aaggggggtg gacagggatg 2400
   gtagatggat ggtacggtta tcaccatcaa aatgagcagg ggtcaggata tgcagccgac 2460
   ctgaagagca cacagaatgc cattgacgag attactaaca aagtaaattc tgttattgaa 2520
   aagatgaata cacagttcac agcagtaggt aaagagttca accacctgga aaaaagaata 2580
   gagaatttaa ataaaaaagt tgatgatggt ttcctggaca tttggactta caatgccgaa 2640
   ctgttggttc tattggaaaa tgaaagaact ttggactacc acgattcaaa tgtgaagaac   2700
   ttatatgaaa aggtaagaag ccagctaaaa aacaatgcca aggaaattgg aaacggctgc   2760
   tttgaatttt accacaaatg cgataacacg tgcatggaaa gtgtcaaaaa tgggacttat   2820
   gactacccaa aatactcaga ggaagcaaaa ttaaacagag aagaaataga tggggtaaag   2880
   ctggaatcaa caaggattta ccagattttg gcgatctatt caactgtcgc cagttcattg   2940
   gtactggtag tctccctggg ggcaatcagt ttctggatgt gctctaatgg gtctctacag   3000
   tgtagaatat gtatttaaag gcctattttc tttagtttga atttactgtt attcggtgtg   3060
   catttctatg tttggtgagc ggttttctgt gctcagagtg tgtttatttt atgtaattta   3120
   atttctttgt gagctcctgt ttagcaggtc gtcccttcag caaggacaca aaaagatttt   3180
   aattttatta aaaaaaaaaa aaaaaaagac cgggaattcg atatcaagct tatcgacctg   3240
   cagatcgttc aaacatttgg caataaagtt tcttaagatt gaatcctgtt gccggtcttg   3300
   cgatgattat catataattt ctgttgaatt acgttaagca tgtaataatt aacatgtaat   3360
   gcatgacgtt atttatgaga tgggttttta tgattagagt cccgcaatta tacatttaat   3420
   acgcgataga aaacaaaata tagcgcgcaa actaggataa attatcgcgc gcggtgtcat   3480
   ctatgttact agatctctag agtctcaagc ttggcgcgcc                         3520